# EUROPEAN PATENT SPECIFICATION

(11) **EP 2 899 545 B1**
(45) Date of publication and mention of the grant of the patent: **22.08.2018**
(21) Application number: 15151607.7
(22) Date of filing: 23.06.2011
(51) Int. Cl.: G01N 33/68

(54) **METHODS FOR DIAGNOSIS AND PROGNOSIS OF RENAL INJURY AND RENAL FAILURE**
VERFAHREN ZUR DIAGNOSE UND PROGNOSE VON NIERENLÄSION UND NIERENINSUFFIZIENZ
PROCÉDÉS POUR LE DIAGNOSTIC ET LE PRONOSTIC DE LÉSION RÉNALE ET D'INSUFFISANCE RÉNALE

(30) Priority: 23.06.2010 US 357965 P; 23.06.2010 US 357956 P; 14.07.2010 US 364300 P; 14.07.2010 US 364304 P
(43) Date of publication of application: 29.07.2015
(62) Divisional of application: 11798515.0
(73) Proprietor: Astute Medical, Inc., San Diego, CA 92121 (US)
(72) Inventor: Anderberg, Joseph, Encinitas, CA 92024 (US); Gray, Jeff, Solana Beach, CA 92075 (US); McPherson, Paul, Encinitas, CA 92024 (US); Nakamura, Kevin, Cardiff By The Sea, CA 92007 (US); Kampf, James Patrick, San Diego, CA 92130 (US)
(74) Representative: Schiweck Weinzierl Koch Patentanwälte Partnerschaft mbB

(56) References cited:
- WO-A1-2011/075744
- CN-A- 102 539 745
- CORREALE JORGE ET AL: "Activation of humoral immunity and eosinophils in neuromyelitis optica", NEUROLOGY, vol. 63, no. 12, 28 December 2004 (2004-12-28), pages 2363-2370, XP002738815, ISSN: 0028-3878
- ARTHUR JOHN M ET AL: "Diagnostic and prognostic biomarkers in acute renal failure", CONTRIBUTIONS TO NEPHROLOGY, KARGER, BASEL, CH, vol. 160, 1 January 2008 (2008-01-01), pages 53-64, XP009108671, ISSN: 0302-5144, DOI: 10.1159/000125929

## Description

### BACKGROUND OF THE INVENTION

The following discussion of the background of the invention is merely provided to aid the reader in understanding the invention and is not admitted to describe or constitute prior art to the present invention.

The kidney is responsible for water and solute excretion from the body. Its functions include maintenance of acid-base balance, regulation of electrolyte concentrations, control of blood volume, and regulation of blood pressure. As such, loss of kidney function through injury and/or disease results in substantial morbidity and mortality. A detailed discussion of renal injuries is provided in Harrison's Principles of Internal Medicine, 17th Ed., McGraw Hill, New York, pages 1741-1830. Renal disease and/or injury may be acute or chronic. Acute and chronic kidney disease are described as follows (from Current Medical Diagnosis & Treatment 2008, 47th Ed, McGraw Hill, New York, pages 785-815): "Acute renal failure is worsening of renal function over hours to days, resulting in the retention of nitrogenous wastes (such as urea nitrogen) and creatinine in the blood. Retention of these substances is called azotemia. Chronic renal failure (chronic kidney disease) results from an abnormal loss of renal function over months to years".

Acute renal failure (ARF, also known as acute kidney injury, or AKI) is an abrupt (typically detected within about 48 hours to 1 week)reduction in glomerular filtration. This loss of filtration capacity results in retention of nitrogenous (urea and creatinine) and non-nitrogenous waste products that are normally excreted by the kidney, a reduction in urine output, or both. It is reported that ARF complicates about 5% of hospital admissions, 4-15% of cardiopulmonary bypass surgeries, and up to 30% of intensive care admissions. ARF may be categorized as prerenal, intrinsic renal, or postrenal in causation. Intrinsic renal disease can be further divided into glomerular, tubular, interstitial, and vascular abnormalities. Major causes of ARF are described in the following table, which is adapted from the Merck Manual, 17th ed., Chapter 222

| **Type** | **Risk Factors** |
|---|---|
| **Prerenal** | |
| ECF volume depletion | Excessive diuresis, hemorrhage, GI losses, loss of intravascular fluid into the extravascular space (due to ascites, peritonitis, pancreatitis, or burns), loss of skin and mucus membranes, renal salt- and water-wasting states |
| Low cardiac output | Cardiomyopathy, MI, cardiac tamponade, pulmonary embolism, pulmonary hypertension, positive-pressure mechanical ventilation |
| Low systemic vascular resistance | Septic shock, liver failure, antihypertensive drugs |
| Increased renal vascular resistance | NSAIDs, cyclosporines, tacrolimus, hypercalcemia, anaphylaxis, anesthetics, renal artery obstruction, renal vein thrombosis, sepsis, hepatorenal syndrome |
| Decreased efferent arteriolar tone (leading to decreased GFR from reduced glomerular transcapillary pressure, especially in patients with bilateral renal artery stenosis) | ACE inhibitors or angiotensin II receptor blockers |

| **Intrinsic Renal** | |
|---|---|
| Acute tubular injury | Ischemia (prolonged or severe prerenal state): surgery, hemorrhage, arterial or venous obstruction; Toxins: NSAIDs, cyclosporines, tacrolimus, aminoglycosides, foscarnet, ethylene glycol, hemoglobin, myoglobin, ifosfamide, heavy metals, methotrexate, radiopaque contrast agents, streptozotocin |
| Acute glomerulonephritis | ANCA-associated: Crescentic glomerulonephritis, polyarteritis nodosa, Wegener's granulomatosis; Anti-GBM glomerulonephritis: Goodpasture's syndrome; Immune-complex: Lupus glomerulonephritis, postinfectious glomerulonephritis, cryoglobulinemic glomerulonephritis |
| Acute tubulo interstitial nephritis | Drug reaction (eg, β-lactams, NSAIDs, sulfonamides, ciprofloxacin, thiazide diuretics, furosemide, phenytoin, allopurinol, pyelonephritis, papillary necrosis |
| Acute vascular nephropathy | Vasculitis, malignant hypertension, thrombotic microangiopathies, scleroderma, atheroembolism |
| Infiltrative diseases | Lymphoma, sarcoidosis, leukemia |

| **Postrenal** | |
|---|---|
| Tubular precipitation | Uric acid (tumor lysis), sulfonamides, triamterene, acyclovir, indinavir, methotrexate, ethylene glycol ingestion, myeloma protein, myoglobin |
| Ureteral obstruction | Intrinsic: Calculi, clots, sloughed renal tissue, fungus ball, edema, malignancy, congenital defects; Extrinsic: Malignancy, retroperitoneal fibrosis, ureteral trauma during surgery or high impact injury |
| Bladder obstruction | Mechanical: Benign prostatic hyperplasia, prostate cancer, bladder cancer, urethral strictures, phimosis, |

| **Type** | **Risk Factors** |
|---|---|
| | paraphimosis, urethral valves, obstructed indwelling urinary catheter; Neurogenic: Anticholinergic drugs, upper or lower motor neuron lesion |

In the case of ischemic ARF, the course of the disease may be divided into four phases. During an initiation phase, which lasts hours to days, reduced perfusion of the kidney is evolving into injury. Glomerular ultrafiltration reduces, the flow of filtrate is reduced due to debris within the tubules, and back leakage of filtrate through injured epithelium occurs. Renal injury can be mediated during this phase by reperfusion of the kidney. Initiation is followed by an extension phase which is characterized by continued ischemic injury and inflammation and may involve endothelial damage and vascular congestion. During the maintenance phase, lasting from 1 to 2 weeks, renal cell injury occurs, and glomerular filtration and urine output reaches a minimum. A recovery phase can follow in which the renal epithelium is repaired and GFR gradually recovers. Despite this, the survival rate of subjects with ARF may be as low as about 60%.

Acute kidney injury caused by radiocontrast agents (also called contrast media) and other nephrotoxins such as cyclosporine, antibiotics including aminoglycosides and anticancer drugs such as cisplatin manifests over a period of days to about a week. Contrast induced nephropathy (CIN, which is AKI caused by radiocontrast agents) is thought to be caused by intrarenal vasoconstriction (leading to ischemic injury) and from the generation of reactive oxygen species that are directly toxic to renal tubular epithelial cells. CIN classically presents as an acute (onset within 24-48h) but reversible (peak 3-5 days, resolution within 1 week) rise in blood urea nitrogen and serum creatinine.

A commonly reported criteria for defining and detecting AKI is an abrupt (typically within about 2-7 days or within a period of hospitalization) elevation of serum creatinine. Although the use of serum creatinine elevation to define and detect AKI is well established, the magnitude of the serum creatinine elevation and the time over which it is measured to define AKI varies considerably among publications. Traditionally, relatively large increases in serum creatinine such as 100%, 200%, an increase of at least 100% to a value over 2 mg/dL and other definitions were used to define AKI. However, the recent trend has been towards using smaller serum creatinine rises to define AKI. The relationship between serum creatinine rise, AKI and the associated health risks are reviewed in Praught and Shlipak, Curr Opin Nephrol Hypertens 14:265-270, 2005 and Chertow et al, J Am Soc Nephrol 16: 3365-3370, 2005. As described in these publications, acute worsening renal function (AKI) and increased risk of death and other detrimental outcomes are now known to be associated with very small increases in serum creatinine. These increases may be determined as a relative (percent) value or a nominal value. Relative increases in serum creatinine as small as 20% from the pre-injury value have been reported to indicate acutely worsening renal function (AKI) and increased health risk, but the more commonly reported value to define AKI and increased health risk is a relative increase of at least 25%. Nominal increases as small as 0.3 mg/dL, 0.2 mg/dL or even 0.1 mg/dL have been reported to indicate worsening renal function and increased risk of death. Various time periods for the serum creatinine to rise to these threshold values have been used to define AKI, for example, ranging from 2 days, 3 days, 7 days, or a variable period defined as the time the patient is in the hospital or intensive care unit. These studies indicate there is not a particular threshold serum creatinine rise (or time period for the rise) for worsening renal function or AKI, but rather a continuous increase in risk with increasing magnitude of serum creatinine rise.

One study (Lassnigg et all, J Am Soc Nephrol 15:1597-1605, 2004investigated both increases and decreases in serum creatinine. Patients with a mild fall in serum creatinine of -0.1 to -0.3 mg/dL following heart surgery had the lowest mortality rate. Patients with a larger fall in serum creatinine (more than or equal to -0.4 mg/dL) or any increase in serum creatinine had a larger mortality rate. These findings caused the authors to conclude that even very subtle changes in renal function (as detected by small creatinine changes within 48 hours of surgery) seriously effect patient's outcomes. In an effort to reach consensus on a unified classification system for using serum creatinine to define AKI in clinical trials and in clinical practice, Bellomo et al., Crit Care. 8(4):R204-12, 2004, the following classifications for stratifying AKI patients:
"Risk": serum creatinine increased 1.5 fold from baseline OR urine production of <0.5 ml/kg body weight/hr for 6 hours;
"Injury": serum creatinine increased 2.0 fold from baseline OR urine production <0.5 ml/kg/hr for 12 h;
"Failure": serum creatinine increased 3.0 fold from baseline OR creatinine >355 µmol/l (with a rise of >44) or urine output below 0.3 ml/kg/hr for 24 h or anuria for at least 12 hours;
And included two clinical outcomes:
   "Loss": persistent need for renal replacement therapy for more than four weeks.
   "ESRD": end stage renal disease-the need for dialysis for more than 3 months.

These criteria are called the RIFLE criteria, which provide a useful clinical tool to classify renal status. As discussed in Kellum, Crit. Care Med. 36: S141-45, 2008 and Ricci et al., Kidney Int. 73, 538-546, 2008, the RIFLE criteria provide a uniform definition of AKI which has been validated in numerous studies.

More recently, Mehta et al., Crit. Care 1 1:R31 (doi:10.1186.cc5713), 2007, proposes the following similar classifications for stratifying AKI patients, which have been modified from RIFLE:
"Stage I": increase in serum creatinine of more than or equal to 0.3 mg/dL (≥ 26.4 µmol/L) or increase to more than or equal to 150% (1.5-fold) from baseline OR urine output less than 0.5 mL/kg per hour for more than 6 hours;
"Stage II": increase in serum creatinine to more than 200% (> 2-fold) from baseline OR urine output less than 0.5 mL/kg per hour for more than 12 hours;
"Stage III": increase in serum creatinine to more than 300% (> 3-fold) from baseline OR serum creatinine ≥ 354 µmol/L accompanied by an acute increase of at least 44 µmol/L OR urine output less than 0.3 mL/kg per hour for 24 hours or anuria for 12 hours.

The CIN Consensus Working Panel (McCollough et al, Rev Cardiovasc Med. 2006;7(4):177-197*)* uses a serum creatinine rise of 25% to define Contrast induced nephropathy (which is a type of AKI).Although various groups propose slightly different criteria for using serum creatinine to detect AKI, the consensus is that small changes in serum creatinine, such as 0.3 mg/dL or 25%, are sufficient to detect AKI (worsening renal function) and that the magnitude of the serum creatinine change is an indicator of the severity of the AKI and mortality risk.

Although serial measurement of serum creatinine over a period of days is an accepted method of detecting and diagnosing AKI and is considered one of the most important tools to evaluate AKI patients, serum creatinine is generally regarded to have several limitations in the diagnosis, assessment and monitoring of AKI patients. The time period for serum creatinine to rise to values (e.g., a 0.3 mg/dL or 25% rise) considered diagnostic for AKI can be 48 hours or longer depending on the definition used. Since cellular injury in AKI can occur over a period of hours, serum creatinine elevations detected at 48 hours or longer can be a late indicator of injury, and relying on serum creatinine can thus delay diagnosis of AKI. Furthermore, serum creatinine is not a good indicator of the exact kidney status and treatment needs during the most acute phases of AKI when kidney function is changing rapidly. Some patients with AKI will recover fully, some will need dialysis (either short term or long term) and some will have other detrimental outcomes including death, major adverse cardiac events and chronic kidney disease. Because serum creatinine is a marker of filtration rate, it does not differentiate between the causes of AKI (pre-renal, intrinsic renal, post-renal obstruction, atheroembolic, etc) or the category or location of injury in intrinsic renal disease (for example, tubular, glomerular or interstitial in origin). Urine output is similarly limited, Knowing these things can be of vital importance in managing and treating patients with AKI.

These limitations underscore the need for better methods to detect and assess AKI, particularly in the early and subclinical stages, but also in later stages when recovery and repair of the kidney can occur. Furthermore, there is a need to better identify patients who are at risk of having an AKI.

### BRIEF SUMMARY OF THE INVENTION

It is an object of the disclosure to provide methods and compositions for evaluating renal function in a subject. As described herein, measurement of one or more biomarkers selected from the group consisting of Cancer antigen CA 15-3, C-C Motif chemokine 18, C-C Motif chemokine 24, Cathepsin D, C-X-C Motif chemokine 13, C-C motif chemokine 8, Interleukin-2 receptor alpha chain, Insulin-like growth factor-binding protein 3, Interleukin-11, Matrix Metalloproteinase-8, Transforming growth factor alpha, IgG1, and IgG2 (each referred to herein as a "kidney injury marker") can be used for diagnosis, prognosis, risk stratification, staging, monitoring, categorizing and determination of further diagnosis and treatment regimens in subjects suffering or at risk of suffering from an injury to renal function, reduced renal function, and/or acute renal failure (also called acute kidney injury).

The kidney injury markers as described herein may be used, individually or in panels comprising a plurality of kidney injury markers, for risk stratification (that is, to identify subjects at risk for a future injury to renal function, for future progression to reduced renal function, for future progression to ARF, for future improvement in renal function, *etc*.); for diagnosis of existing disease (that is, to identify subjects who have suffered an injury to renal function, who have progressed to reduced renal function, who have progressed to ARF, *etc*.); for monitoring for deterioration or improvement of renal function; and for predicting a future medical outcome, such as improved or worsening renal function, a decreased or increased mortality risk, a decreased or increased risk that a subject will require renal replacement therapy (*i.e.,* hemodialysis, peritoneal dialysis, hemofiltration, and/or renal transplantation, a decreased or increased risk that a subject will recover from an injury to renal function, a decreased or increased risk that a subject will recover from ARF, a decreased or increased risk that a subject will progress to end stage renal disease, a decreased or increased risk that a subject will progress to chronic renal failure, a decreased or increased risk that a subject will suffer rejection of a transplanted kidney, *etc.*

In a first aspect, the present invention relates to a method for evaluating renal status in a subject, comprising:
performing one or more assays that generate(s) a detectable signal indicative of the presence or amount of a physiologically relevant concentration of C-C Motif chemokine 24, and optionally one or more biomarkers selected from the group consisting of Cancer antigen CA 15-3, C-C Motif chemokine 18, Cathepsin D, C-X-C Motif chemokine 13, C-C motif chemokine 8, Interleukin-2 receptor alpha chain, Insulin-like growth factor-binding protein 3, Interleukin-11, Matrix Metalloproteinase-8, Transforming growth factor alpha, IgG1, and IgG2 on a body fluid sample obtained from the subject to provide an assay result; and correlating the assay result(s) to the renal status of the subject,
and wherein the correlating step comprises comparing an assay result in the form of the C-C Motif chemokine 24 concentration to a predetermined threshold selected to be indicative of the occurrence or nonoccurrence of a change in renal status or the likelihood of one or more future changes in renal status.

In a second aspect, the present invention relates to a use of C-C Motif chemokine 24 and optionally one or more biomarkers selected from the group consisting of Cancer antigen CA 15-3, C-C Motif chemokine 18, Cathepsin D, C-X-C Motif chemokine 13, C-C motif chemokine 8, Interleukin-2 receptor alpha chain, Insulin-like growth factor-binding protein 3, Interleukin-11, Matrix Metalloproteinase-8, Transforming growth factor alpha, IgG 1, and IgG2 for the evaluation of renal injury, preferably for the evaluation of acute renal injury, said use comprising
comparing a measured concentration of C-C Motif chemokine 24 to a predetermined threshold selected to be indicative of the occurrence or nonoccurrence of a change in renal status or the likelihood of one or more future changes in renal status.

Also disclosed are methods for evaluating renal status in a subject. These methods comprise performing an assay method that is configured to detect one or more biomarkers selected from the group consisting of Cancer antigen CA 15-3, C-C Motif chemokine 18, C-C Motif chemokine 24, Cathepsin D, C-X-C Motif chemokine 13, C-C motif chemokine 8, Interleukin-2 receptor alpha chain, Insulin-like growth factor-binding protein 3, Interleukin-11, Matrix Metalloproteinase-8, Transforming growth factor alpha, IgG1, and IgG2 is/are then correlated to the renal status of the subject. This correlation to renal status may include correlating the assay result(s) to one or more of risk stratification, diagnosis, prognosis, staging, classifying and monitoring of the subject as described herein. Thus, the methods utilize one or more kidney injury markers as disclosed herein for the evaluation of renal injury.

In certain embodiments, the methods for evaluating renal status described herein are methods for risk stratification of the subject; that is, assigning a likelihood of one or more future changes in renal status to the subject. In these embodiments, the assay result(s) is/are correlated to one or more such future changes. The following are preferred risk stratification embodiments.

In preferred risk stratification embodiments, these methods comprise determining a subject's risk for a future injury to renal function, and the assay result(s) is/are correlated to a likelihood of such a future injury to renal function. For example, the measured concentration(s) may each be compared to a threshold value. For a "positive going" kidney injury marker, an increased likelihood of suffering a future injury to renal function is assigned to the subject when the measured concentration is above the threshold, relative to a likelihood assigned when the measured concentration is below the threshold. For a "negative going" kidney injury marker, an increased likelihood of suffering a future injury to renal function is assigned to the subject when the measured concentration is below the threshold, relative to a likelihood assigned when the measured concentration is above the threshold.

In other preferred risk stratification embodiments, these methods comprise determining a subject's risk for future reduced renal function, and the assay result(s) is/are correlated to a likelihood of such reduced renal function. For example, the measured concentrations may each be compared to a threshold value. For a "positive going" kidney injury marker, an increased likelihood of suffering a future reduced renal function is assigned to the subject when the measured concentration is above the threshold, relative to a likelihood assigned when the measured concentration is below the threshold. For a "negative going" kidney injury marker, an increased likelihood of future reduced renal function is assigned to the subject when the measured concentration is below the threshold, relative to a likelihood assigned when the measured concentration is above the threshold.

In still other preferred risk stratification embodiments, these methods comprise determining a subject's likelihood for a future improvement in renal function, and the assay result(s) is/are correlated to a likelihood of such a future improvement in renal function. For example, the measured concentration(s) may each be compared to a threshold value. For a "positive going" kidney injury marker, an increased likelihood of a future improvement in renal function is assigned to the subject when the measured concentration is below the threshold, relative to a likelihood assigned when the measured concentration is above the threshold. For a "negative going" kidney injury marker, an increased likelihood of a future improvement in renal function is assigned to the subject when the measured concentration is above the threshold, relative to a likelihood assigned when the measured concentration is below the threshold.

In yet other preferred risk stratification embodiments, these methods comprise determining a subject's risk for progression to ARF, and the result(s) is/are correlated to a likelihood of such progression to ARF. For example, the measured concentration(s) may each be compared to a threshold value. For a "positive going" kidney injury marker, an increased likelihood of progression to ARF is assigned to the subject when the measured concentration is above the threshold, relative to a likelihood assigned when the measured concentration is below the threshold. For a "negative going" kidney injury marker, an increased likelihood of progression to ARF is assigned to the subject when the measured concentration is below the threshold, relative to a likelihood assigned when the measured concentration is above the threshold.

And in other preferred risk stratification embodiments, these methods comprise determining a subject's outcome risk, and the assay result(s) is/are correlated to a likelihood of the occurrence of a clinical outcome related to a renal injury suffered by the subject. For example, the measured concentration(s) may each be compared to a threshold value. For a "positive going" kidney injury marker, an increased likelihood of one or more of: acute kidney injury, progression to a worsening stage of AKI, mortality, a requirement for renal replacement therapy, a requirement for withdrawal of renal toxins, end stage renal disease, heart failure, stroke, myocardial infarction, progression to chronic kidney disease, etc., is assigned to the subject when the measured concentration is above the threshold, relative to a likelihood assigned when the measured concentration is below the threshold. For a "negative going" kidney injury marker, an increased likelihood of one or more of: acute kidney injury, progression to a worsening stage of AKI, mortality, a requirement for renal replacement therapy, a requirement for withdrawal of renal toxins, end stage renal disease, heart failure, stroke, myocardial infarction, progression to chronic kidney disease, etc., is assigned to the subject when the measured concentration is below the threshold, relative to a likelihood assigned when the measured concentration is above the threshold.

In such risk stratification embodiments, preferably the likelihood or risk assigned is that an event of interest is more or less likely to occur within 180 days of the time at which the body fluid sample is obtained from the subject. In particularly preferred embodiments, the likelihood or risk assigned relates to an event of interest occurring within a shorter time period such as 18 months, 120 days, 90 days, 60 days, 45 days, 30 days, 21 days, 14 days, 7 days, 5 days, 96 hours, 72 hours, 48 hours, 36 hours, 24 hours, 12 hours, or less. A risk at 0 hours of the time at which the body fluid sample is obtained from the subject is equivalent to diagnosis of a current condition.

In preferred risk stratification embodiments, the subject is selected for risk stratification based on the pre-existence in the subject of one or more known risk factors for prerenal, intrinsic renal, or postrenal ARF. For example, a subject undergoing or having undergone major vascular surgery, coronary artery bypass, or other cardiac surgery; a subject having pre-existing congestive heart failure, preeclampsia, eclampsia, diabetes mellitus, hypertension, coronary artery disease, proteinuria, renal insufficiency, glomerular filtration below the normal range, cirrhosis, serum creatinine above the normal range, or sepsis; or a subject exposed to NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin are all preferred subjects for monitoring risks according to the methods described herein. This list is not meant to be limiting. By "pre-existence" in this context is meant that the risk factor exists at the time the body fluid sample is obtained from the subject. In particularly preferred embodiments, a subject is chosen for risk stratification based on an existing diagnosis of injury to renal function, reduced renal function, or ARF.

In other embodiments, the methods for evaluating renal status described herein are methods for diagnosing a renal injury in the subject; that is, assessing whether or not a subject has suffered from an injury to renal function, reduced renal function, or ARF. In these embodiments, the assay result(s), for example measured concentration(s) of C-C Motif chemokine 24, and optionally further of one or more biomarkers selected from the group consisting of Cancer antigen CA 15-3, C-C Motif chemokine 18, Cathepsin D, C-X-C Motif chemokine 13, C-C motif chemokine 8, Interleukin-2 receptor alpha chain, Insulin-like growth factor-binding protein 3, Interleukin-11, Matrix Metalloproteinase-8, Transforming growth factor alpha, IgG1, and IgG2 is/are correlated to the occurrence or nonoccurrence of a change in renal status. The following are preferred diagnostic embodiments.

In preferred diagnostic embodiments, these methods comprise diagnosing the occurrence or nonoccurrence of an injury to renal function, and the assay result(s) is/are correlated to the occurrence or nonoccurrence of such an injury. For example, each of the measured concentration(s) may be compared to a threshold value. For a positive going marker, an increased likelihood of the occurrence of an injury to renal function is assigned to the subject when the measured concentration is above the threshold (relative to the likelihood assigned when the measured concentration is below the threshold); alternatively, when the measured concentration is below the threshold, an increased likelihood of the nonoccurrence of an injury to renal function may be assigned to the subject (relative to the likelihood assigned when the measured concentration is above the threshold). For a negative going marker, an increased likelihood of the occurrence of an injury to renal function is assigned to the subject when the measured concentration is below the threshold (relative to the likelihood assigned when the measured concentration is above the threshold); alternatively, when the measured concentration is above the threshold, an increased likelihood of the nonoccurrence of an injury to renal function may be assigned to the subject (relative to the likelihood assigned when the measured concentration is below the threshold).

In other preferred diagnostic embodiments, these methods comprise diagnosing the occurrence or nonoccurrence of reduced renal function, and the assay result(s) is/are correlated to the occurrence or nonoccurrence of an injury causing reduced renal function. For example, each of the measured concentration(s) may be compared to a threshold value. For a positive going marker, an increased likelihood of the occurrence of an injury causing reduced renal function is assigned to the subject when the measured concentration is above the threshold (relative to the likelihood assigned when the measured concentration is below the threshold); alternatively, when the measured concentration is below the threshold, an increased likelihood of the nonoccurrence of an injury causing reduced renal function may be assigned to the subject (relative to the likelihood assigned when the measured concentration is above the threshold). For a negative going marker, an increased likelihood of the occurrence of an injury causing reduced renal function is assigned to the subject when the measured concentration is below the threshold (relative to the likelihood assigned when the measured concentration is above the threshold); alternatively, when the measured concentration is above the threshold, an increased likelihood of the nonoccurrence of an injury causing reduced renal function may be assigned to the subject (relative to the likelihood assigned when the measured concentration is below the threshold).

In yet other preferred diagnostic embodiments, these methods comprise diagnosing the occurrence or nonoccurrence of ARF, and the assay result(s) is/are correlated to the occurrence or nonoccurrence of an injury causing ARF. For example, each of the measured concentration(s) may be compared to a threshold value. For a positive going marker, an increased likelihood of the occurrence of ARF is assigned to the subject when the measured concentration is above the threshold (relative to the likelihood assigned when the measured concentration is below the threshold); alternatively, when the measured concentration is below the threshold, an increased likelihood of the nonoccurrence of ARF may be assigned to the subject (relative to the likelihood assigned when the measured concentration is above the threshold). For a negative going marker, an increased likelihood of the occurrence of ARF is assigned to the subject when the measured concentration is below the threshold (relative to the likelihood assigned when the measured concentration is above the threshold); alternatively, when the measured concentration is above the threshold, an increased likelihood of the nonoccurrence of ARF may be assigned to the subject (relative to the likelihood assigned when the measured concentration is below the threshold).

In still other preferred diagnostic embodiments, these methods comprise diagnosing a subject as being in need of renal replacement therapy, and the assay result(s) is/are correlated to a need for renal replacement therapy. For example, each of the measured concentration(s) may be compared to a threshold value. For a positive going marker, an increased likelihood of the occurrence of an injury creating a need for renal replacement therapy is assigned to the subject when the measured concentration is above the threshold (relative to the likelihood assigned when the measured concentration is below the threshold); alternatively, when the measured concentration is below the threshold, an increased likelihood of the nonoccurrence of an injury creating a need for renal replacement therapy may be assigned to the subject (relative to the likelihood assigned when the measured concentration is above the threshold). For a negative going marker, an increased likelihood of the occurrence of an injury creating a need for renal replacement therapy is assigned to the subject when the measured concentration is below the threshold (relative to the likelihood assigned when the measured concentration is above the threshold); alternatively, when the measured concentration is above the threshold, an increased likelihood of the nonoccurrence of an injury creating a need for renal replacement therapy may be assigned to the subject (relative to the likelihood assigned when the measured concentration is below the threshold).

In still other preferred diagnostic embodiments, these methods comprise diagnosing a subject as being in need of renal transplantation, and the assay result(s0 is/are correlated to a need for renal transplantation. For example, each of the measured concentration(s) may be compared to a threshold value. For a positive going marker, an increased likelihood of the occurrence of an injury creating a need for renal transplantation is assigned to the subject when the measured concentration is above the threshold (relative to the likelihood assigned when the measured concentration is below the threshold); alternatively, when the measured concentration is below the threshold, an increased likelihood of the nonoccurrence of an injury creating a need for renal transplantation may be assigned to the subject (relative to the likelihood assigned when the measured concentration is above the threshold). For a negative going marker, an increased likelihood of the occurrence of an injury creating a need for renal transplantation is assigned to the subject when the measured concentration is below the threshold (relative to the likelihood assigned when the measured concentration is above the threshold); alternatively, when the measured concentration is above the threshold, an increased likelihood of the nonoccurrence of an injury creating a need for renal transplantation may be assigned to the subject (relative to the likelihood assigned when the measured concentration is below the threshold).

In still other embodiments, the methods for evaluating renal status described herein are methods for monitoring a renal injury in the subject; that is, assessing whether or not renal function is improving or worsening in a subject who has suffered from an injury to renal function, reduced renal function, or ARF. In these embodiments, the assay result(s), for example measured concentration(s) of C-C Motif chemokine 24, and optionally further of one or more biomarkers selected from the group consisting of Cancer antigen CA 15-3, C-C Motif chemokine 18, Cathepsin D, C-X-C Motif chemokine 13, C-C motif chemokine 8, Interleukin-2 receptor alpha chain, Insulin-like growth factor-binding protein 3, Interleukin-11, Matrix Metalloproteinase-8, Transforming growth factor alpha, IgG1, and IgG2 is/are correlated to the occurrence or nonoccurrence of a change in renal status. The following are preferred monitoring embodiments.

In preferred monitoring embodiments, these methods comprise monitoring renal status in a subject suffering from an injury to renal function, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In other preferred monitoring embodiments, these methods comprise monitoring renal status in a subject suffering from reduced renal function, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In yet other preferred monitoring embodiments, these methods comprise monitoring renal status in a subject suffering from acute renal failure, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In other additional preferred monitoring embodiments, these methods comprise monitoring renal status in a subject at risk of an injury to renal function due to the pre-existence of one or more known risk factors for prerenal, intrinsic renal, or postrenal ARF, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In still other embodiments, the methods for evaluating renal status described herein are methods for classifying a renal injury in the subject; that is, determining whether a renal injury in a subject is prerenal, intrinsic renal, or postrenal; and/or further subdividing these classes into subclasses such as acute tubular injury, acute glomerulonephritis acute tubulointerstitial nephritis, acute vascular nephropathy, or infiltrative disease; and/or assigning a likelihood that a subject will progress to a particular RIFLE stage. In these embodiments, the assay result(s), for example measured concentration(s) of C-C Motif chemokine 24, and optionally further of one or more biomarkers selected from the group consisting of Cancer antigen CA 15-3, C-C Motif chemokine 18, Cathepsin D, C-X-C Motif chemokine 13, C-C motif chemokine 8, Interleukin-2 receptor alpha chain, Insulin-like growth factor-binding protein 3, Interleukin-11, Matrix Metalloproteinase-8, Transforming growth factor alpha, IgG1, and IgG2 is/are correlated to a particular class and/or subclass. The following are preferred classification embodiments.

In preferred classification embodiments, these methods comprise determining whether a renal injury in a subject is prerenal, intrinsic renal, or postrenal; and/or further subdividing these classes into subclasses such as acute tubular injury, acute glomerulonephritis acute tubulointerstitial nephritis, acute vascular nephropathy, or infiltrative disease; and/or assigning a likelihood that a subject will progress to a particular RIFLE stage, and the assay result(s) is/are correlated to the injury classification for the subject. For example, the measured concentration may be compared to a threshold value, and when the measured concentration is above the threshold, a particular classification is assigned; alternatively, when the measured concentration is below the threshold, a different classification may be assigned to the subject.

A variety of methods may be used by the skilled artisan to arrive at a desired threshold value for use in these methods. For example, the threshold value may be determined from a population of normal subjects by selecting a concentration representing the 75th, 85th, 90th, 95th, or 99th percentile of a kidney injury marker measured in such normal subjects. Alternatively, the threshold value may be determined from a "diseased" population of subjects, e.g., those suffering from an injury or having a predisposition for an injury (e.g., progression to ARF or some other clinical outcome such as death, dialysis, renal transplantation, etc.), by selecting a concentration representing the 75th, 85th, 90th, 95th, or 99th percentile of a kidney injury marker measured in such subjects. In another alternative, the threshold value may be determined from a prior measurement of a kidney injury marker in the same subject; that is, a temporal change in the level of a kidney injury marker in the subject may be used to assign risk to the subject.

The foregoing discussion is not meant to imply, however, that the kidney injury markers must be compared to corresponding individual thresholds. Methods for combining assay results can comprise the use of multivariate logistical regression, loglinear modeling, neural network analysis, n-of-m analysis, decision tree analysis, calculating ratios of markers, etc. This list is not meant to be limiting. In these methods, a composite result which is determined by combining individual markers may be treated as if it is itself a marker; that is, a threshold may be determined for the composite result as described herein for individual markers, and the composite result for an individual patient compared to this threshold.

The ability of a particular test to distinguish two populations can be established using ROC analysis. For example, ROC curves established from a "first" subpopulation which is predisposed to one or more future changes in renal status, and a "second" subpopulation which is not so predisposed can be used to calculate a ROC curve, and the area under the curve provides a measure of the quality of the test. Preferably, the tests described herein provide a ROC curve area greater than 0.5, preferably at least 0.6, more preferably 0.7, still more preferably at least 0.8, even more preferably at least 0.9, and most preferably at least 0.95.

In certain aspects, the measured concentration of one or more kidney injury markers, or a composite of such markers, may be treated as continuous variables. For example, any particular concentration can be converted into a corresponding probability of a future reduction in renal function for the subject, the occurrence of an injury, a classification, etc. In yet another alternative, a threshold that can provide an acceptable level of specificity and sensitivity in separating a population of subjects into "bins" such as a "first" subpopulation (e.g., which is predisposed to one or more future changes in renal status, the occurrence of an injury, a classification, etc.) and a "second" subpopulation which is not so predisposed. A threshold value is selected to separate this first and second population by one or more of the following measures of test accuracy:
an odds ratio greater than 1, preferably at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less;
a specificity of greater than 0.5, preferably at least about 0.6, more preferably at least about 0.7, still more preferably at least about 0.8, even more preferably at least about 0.9 and most preferably at least about 0.95, with a corresponding sensitivity greater than 0.2, preferably greater than about 0.3, more preferably greater than about 0.4, still more preferably at least about 0.5, even more preferably about 0.6, yet more preferably greater than about 0.7, still more preferably greater than about 0.8, more preferably greater than about 0.9, and most preferably greater than about 0.95;
a sensitivity of greater than 0.5, preferably at least about 0.6, more preferably at least about 0.7, still more preferably at least about 0.8, even more preferably at least about 0.9 and most preferably at least about 0.95, with a corresponding specificity greater than 0.2, preferably greater than about 0.3, more preferably greater than about 0.4, still more preferably at least about 0.5, even more preferably about 0.6, yet more preferably greater than about 0.7, still more preferably greater than about 0.8, more preferably greater than about 0.9, and most preferably greater than about 0.95;
at least about 75% sensitivity, combined with at least about 75% specificity;
a positive likelihood ratio (calculated as sensitivity/(1-specificity)) of greater than 1, at least about 2, more preferably at least about 3, still more preferably at least about 5, and most preferably at least about 10; or
a negative likelihood ratio (calculated as (1-sensitivity)/specificity) of less than 1, less than or equal to about 0.5, more preferably less than or equal to about 0.3, and most preferably less than or equal to about 0.1.
The term "about" in the context of any of the above measurements refers to +/- 5% of a given measurement.

Multiple thresholds may also be used to assess renal status in a subject. For example, a "first" subpopulation which is predisposed to one or more future changes in renal status, the occurrence of an injury, a classification, etc., and a "second" subpopulation which is not so predisposed can be combined into a single group. This group is then subdivided into three or more equal parts (known as tertiles, quartiles, quintiles, etc., depending on the number of subdivisions). An odds ratio is assigned to subjects based on which subdivision they fall into. If one considers a tertile, the lowest or highest tertile can be used as a reference for comparison of the other subdivisions. This reference subdivision is assigned an odds ratio of 1. The second tertile is assigned an odds ratio that is relative to that first tertile. That is, someone in the second tertile might be 3 times more likely to suffer one or more future changes in renal status in comparison to someone in the first tertile. The third tertile is also assigned an odds ratio that is relative to that first tertile.

In certain embodiments, the assay method is an immunoassay. Antibodies for use in such assays will specifically bind a full length kidney injury marker of interest, and may also bind one or more polypeptides that are "related" thereto, as that term is defined hereinafter. Numerous immunoassay formats are known to those of skill in the art. Preferred body fluid samples are selected from the group consisting of urine, blood, serum, saliva, tears, and plasma. In the case of those kidney injury markers which are membrane proteins as described hereinafter, preferred assays detect soluble forms thereof.

The foregoing method steps should not be interpreted to mean that the kidney injury marker assay result(s) is/are used in isolation in the methods described herein. Rather, additional variables or other clinical indicia may be included in the methods described herein. For example, a risk stratification, diagnostic, classification, monitoring, etc. method may combine the assay result(s) with one or more variables measured for the subject selected from the group consisting of demographic information (e.g., weight, sex, age, race), medical history (e.g., family history, type of surgery, pre-existing disease such as aneurism, congestive heart failure, preeclampsia, eclampsia, diabetes mellitus, hypertension, coronary artery disease, proteinuria, renal insufficiency, or sepsis, type of toxin exposure such as NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin), clinical variables (e.g., blood pressure, temperature, respiration rate), risk scores (APACHE score, PREDICT score, TIMI Risk Score for UA/NSTEMI, Framingham Risk Score, risk scores of Thakar et al. (J. Am. Soc. Nephrol. 16: 162-68, 2005), Mehran et al. (J. Am. Coll. Cardiol. 44: 1393-99, 2004), Wijeysundera et al. (JAMA 297: 1801-9, 2007), Goldstein and Chawla (Clin. J. Am. Soc. Nephrol. 5: 943-49, 2010), or Chawla et al. (Kidney Intl. 68: 2274-80, 2005)), a glomerular filtration rate, an estimated glomerular filtration rate, a urine production rate, a serum or plasma creatinine concentration, a urine creatinine concentration, a fractional excretion of sodium, a urine sodium concentration, a urine creatinine to serum or plasma creatinine ratio, a urine specific gravity, a urine osmolality, a urine urea nitrogen to plasma urea nitrogen ratio, a plasma BUN to creatnine ratio, a renal failure index calculated as urine sodium / (urine creatinine / plasma creatinine), a serum or plasma neutrophil gelatinase (NGAL) concentration, a urine NGAL concentration, a serum or plasma cystatin C concentration, a serum or plasma cardiac troponin concentration, a serum or plasma BNP concentration, a serum or plasma NTproBNP concentration, and a serum or plasma proBNP concentration. Other measures of renal function which may be combined with one or more kidney injury marker assay result(s) are described hereinafter and in Harrison's Principles of Internal Medicine, 17th Ed., McGraw Hill, New York, pages 1741-1830, and Current Medical Diagnosis & Treatment 2008, 47th Ed, McGraw Hill, New York, pages 785-815.

When more than one marker is measured, the individual markers may be measured in samples obtained at the same time, or may be determined from samples obtained at different (e.g., an earlier or later) times. The individual markers may also be measured on the same or different body fluid samples. For example, one kidney injury marker may be measured in a serum or plasma sample and another kidney injury marker may be measured in a urine sample. In addition, assignment of a likelihood may combine an individual kidney injury marker assay result with temporal changes in one or more additional variables.

In various related aspectsdisclosed are also devices and kits for performing the methods described herein. Suitable kits comprise reagents sufficient for performing an assay for at least one of the described kidney injury markers, together with instructions for performing the described threshold comparisons.

In certain embodiments, reagents for performing such assays are provided in an assay device, and such assay devices may be included in such a kit. Preferred reagents can comprise one or more solid phase antibodies, the solid phase antibody comprising antibody that detects the intended biomarker target(s) bound to a solid support. In the case of sandwich immunoassays, such reagents can also include one or more detectably labeled antibodies, the detectably labeled antibody comprising antibody that detects the intended biomarker target(s) bound to a detectable label. Additional optional elements that may be provided as part of an assay device are described hereinafter.

Detectable labels may include molecules that are themselves detectable (e.g., fluorescent moieties, electrochemical labels, ecl (electrochemical luminescence) labels, metal chelates, colloidal metal particles, etc.) as well as molecules that may be indirectly detected by production of a detectable reaction product (e.g., enzymes such as horseradish peroxidase, alkaline phosphatase, etc.) or through the use of a specific binding molecule which itself may be detectable (e.g., a labeled antibody that binds to the second antibody, biotin, digoxigenin, maltose, oligohistidine, 2,4-dintrobenzene, phenylarsenate, ssDNA, dsDNA, etc.).

Generation of a signal from the signal development element can be performed using various optical, acoustical, and electrochemical methods well known in the art. Examples of detection modes include fluorescence, radiochemical detection, reflectance, absorbance, amperometry, conductance, impedance, interferometry, ellipsometry, etc. In certain of these methods, the solid phase antibody is coupled to a transducer (e.g., a diffraction grating, electrochemical sensor, etc) for generation of a signal, while in others, a signal is generated by a transducer that is spatially separate from the solid phase antibody (e.g., a fluorometer that employs an excitation light source and an optical detector). This list is not meant to be limiting. Antibody-based biosensors may also be employed to determine the presence or amount of analytes that optionally eliminate the need for a labeled molecule.

### DETAILED DESCRIPTION OF THE INVENTION

The present disclosure relates to methods and compositions for diagnosis, differential diagnosis, risk stratification, monitoring, classifying and determination of treatment regimens in subjects suffering or at risk of suffering from injury to renal function, reduced renal function and/or acute renal failure through measurement of one or more kidney injury markers. In various embodiments, a measured concentration of C-C Motif chemokine 24, and optionally further of one or more biomarkers selected from the group consisting of Cancer antigen CA 15-3, C-C Motif chemokine 18, Cathepsin D, C-X-C Motif chemokine 13, C-C motif chemokine 8, Interleukin-2 receptor alpha chain, Insulin-like growth factor-binding protein 3, Interleukin-11, Matrix Metalloproteinase-8, Transforming growth factor alpha, IgG1, and IgG2 or one or more markers related thereto, are correlated to the renal status of the subject.

For purposes of this document, the following definitions apply:
As used herein, an "injury to renal function" is an abrupt (within 14 days, preferably within 7 days, more preferably within 72 hours, and still more preferably within 48 hours) measurable reduction in a measure of renal function. Such an injury may be identified, for example, by a decrease in glomerular filtration rate or estimated GFR, a reduction in urine output, an increase in serum creatinine, an increase in serum cystatin C, a requirement for renal replacement therapy, *etc.* "Improvement in Renal Function" is an abrupt (within 14 days, preferably within 7 days, more preferably within 72 hours, and still more preferably within 48 hours) measurable increase in a measure of renal function. Preferred methods for measuring and/or estimating GFR are described hereinafter.

As used herein, "reduced renal function" is an abrupt (within 14 days, preferably within 7 days, more preferably within 72 hours, and still more preferably within 48 hours) reduction in kidney function identified by an absolute increase in serum creatinine of greater than or equal to 0.1 mg/dL (≥ 8.8 µmol/L), a percentage increase in serum creatinine of greater than or equal to 20% (1.2-fold from baseline), or a reduction in urine output (documented oliguria of less than 0.5 ml/kg per hour).

As used herein, "acute renal failure" or "ARF" is an abrupt (within 14 days, preferably within 7 days, more preferably within 72 hours, and still more preferably within 48 hours) reduction in kidney function identified by an absolute increase in serum creatinine of greater than or equal to 0.3 mg/dl (≥ 26.4 µmol/l), a percentage increase in serum creatinine of greater than or equal to 50% (1. 5-fold from baseline), or a reduction in urine output (documented oliguria of less than 0.5 ml/kg per hour for at least 6 hours). This term is synonymous with "acute kidney injury" or "AKI."

As used herein, the term "C-C motif chemokine 8" refers to one or more polypeptides present in a biological sample that are derived from the C-C motif chemokine 8 precursor (Swiss-Prot P80075 (SEQ ID NO: 1)).

The following domains have been identified in C-C motif chemokine 8:

| Residues | Length | Domain ID |
|---|---|---|
| 1-23 | 23 | Signal peptide |
| 24-99 | 76 | C-C motif chemokine 8 |
| 29-99 | 76 | MCP-2(6-76) |

As used herein, the term "Interleukin-2 receptor subunit alpha" refers to one or more polypeptides present in a biological sample that are derived from the Interleukin-2 receptor subunit alpha precursor (Swiss-Prot P01589 (SEQ ID NO: 2)):

Interleukin-2 receptor subunit alpha is a single-pass type I membrane protein having a large extracellular domain, some or all of which is present in soluble forms of Interleukin-2 receptor subunit alpha generated either through alternative splicing event which deletes all or a portion of the transmembrane domain, or by proteolysis of the membrane-bound form. In the case of an immunoassay, one or more antibodies that bind to epitopes within this extracellular domain may be used to detect these soluble form(s). The following domains have been identified in Interleukin-2 receptor subunit alpha:

| Residues | Length | Domain ID |
|---|---|---|
| 1-21 | 21 | Signal peptide |
| 22-272 | 251 | Interleukin-2 receptor subunit alpha |
| 22-240 | 219 | Extracellular domain |
| 241-259 | 19 | Transmembrane domain |
| 260-272 | 13 | Cytoplasmic domain |

As used herein, the term "Insulin-like growth factor-binding protein 3" refers to one or more polypeptides present in a biological sample that are derived from the Insulin-like growth factor-binding protein 3 precursor (Swiss-Prot P17936 (SEQ ID NO: 3)).

The following domains have been identified in Insulin-like growth factor-binding protein 3:

| Residues | Length | Domain ID |
|---|---|---|
| 1-27 | 27 | Signal peptide |
| 28-291 | 264 | Insulin-like growth factor-binding protein 3 |

As used herein, the term "Interleukin-11" refers to one or more polypeptides present in a biological sample that are derived from the Interleukin-11 precursor (Swiss-Prot P20809 (SEQ ID NO: 4)).

The following domains have been identified in Interleukin-11:

| Residues | Length | Domain ID |
|---|---|---|
| 1-21 | 21 | Signal peptide |
| 22-199 | 178 | Interleukin-11 |

As used herein, the term "Neutrophil collagenase" (also known as MMP-8 and matrix metalloproteinase 8) refers to one or more polypeptides present in a biological sample that are derived from the Neutrophil collagenase precursor (Swiss-Prot P22894 (SEQ ID NO: 5)).

The following domains have been identified in Neutrophil collagenase:

| Residues | Length | Domain ID |
|---|---|---|
| 1-20 | 20 | Signal peptide |
| 21-100 | 80 | Activation peptide |
| 101-467 | 367 | Neutrophil collagenase |

As used herein, the term "Transforming growth factor alpha" refers to one or more polypeptides present in a biological sample that are derived from the Transforming growth factor alpha precursor (Swiss-Prot P01135 (SEQ ID NO: 6)).

The following domains have been identified in Transforming growth factor alpha:

| Residues | Length | Domain ID |
|---|---|---|
| 1-23 | 23 | Signal peptide |
| 24-160 | 131 | pro-Transforming growth factor alpha |
| 24-39 | 16 | propeptide |
| 40-89 | 50 | Transforming growth factor alpha |
| 90-160 | 71 | propeptide |
| 32 | | Missing in isoform 2 |
| 32 | | Missing in isoform 3 |
| 159-160 | | VV → ATLG in isoform 3 |
| 159-160 | | VV → GCRLY in isoform 4 |
| 32 | | Missing in isoform 5 |
| 159-160 | | VV → GCRLY in isoform 5 |

As used herein, the term "Cancer Antigen CA 15-3" refers to one or more polypeptides present in a biological sample that are derived from the Mucin-16 precursor (Swiss-Prot Q8WXI7 (SEQ ID NO: 7)):

Cancer antigen CA 15-3 is a single-pass type I membrane protein having a large extracellular domain, some or all of which is present in soluble forms Cancer antigen CA 15-3 generated either through alternative splicing event which deletes all or a portion of the transmembrane domain, or by proteolysis of the membrane-bound form. In the case of an immunoassay, one or more antibodies that bind to epitopes within this extracellular domain may be used to detect these soluble form(s). The following domains have been identified in Cancer antigen CA 15-3:

| Residues | Length | Domain ID |
|---|---|---|
| 1-22152 | 22152 | Cancer antigen CA 15-3 |
| 1-22096 | 22096 | Extracellular domain |
| 22097-22117 | 21 | Transmembrane domain |
| 22118-22152 | 35 | Cytoplasmic domain |

As used herein, the term "C-C motif chemokine 18" refers to one or more polypeptides present in a biological sample that are derived from the C-C motif chemokine 18 precursor (Swiss-Prot P55774 (SEQ ID NO: 8)).

The following domains have been identified in C-C motif chemokine 18:

| Residues | Length | Domain ID |
|---|---|---|
| 1-20 | 20 | Signal peptide |
| 21-89 | 69 | C-C motif chemokine 18 |
| 21-88 | 68 | CCL 18 (1-68) |
| 23-89 | 67 | CCL 18 (3-69) |
| 24-89 | 66 | CCL 18 (4-69) |

As used herein, the term "C-C motif chemokine 24" refers to one or more polypeptides present in a biological sample that are derived from the C-C motif chemokine 24 precursor (Swiss-Prot 000175 (SEQ ID NO: 9)), and that are indicative of the presence or amount of a physiologically relevant concentration of C-C motif chemokine 24,

The following domains have been identified in C-C motif chemokine 24:

| Residues | Length | Domain ID |
|---|---|---|
| 1-26 | 26 | Signal peptide |
| 27-119 | 93 | C-C motif chemokine 24 |

As used herein, the term "Cathepsin D" refers to one or more polypeptides present in a biological sample that are derived from the Cathepsin D precursor (Swiss-Prot P07339 (SEQ ID NO: 10)).

The following domains have been identified in Capthesin D:

| Residues | Length | Domain ID |
|---|---|---|
| 1-18 | 18 | Signal peptide |
| 19-64 | 46 | Activation peptide |
| 65-412 | 348 | Cathepsin D |
| 65-161 | 348 | Cathepsin D light chain |
| 169-412 | 348 | Cathepsin D heavy chain |

As used herein, the term "C-X-C Motif chemokine 13" refers to one or more polypeptides present in a biological sample that are derived from the C-X-C Motif chemokine 13 precursor (Swiss-Prot 043927 (SEQ ID NO: 11)).

The following domains have been identified in C-X-C Motif chemokine 13:

| Residues | Length | Domain ID |
|---|---|---|
| 1-22 | 22 | Signal peptide |
| 23-109 | 87 | C-X-C Motif chemokine 13 |

As used herein, the term "IgG1" refers to subclass 1 of the glycoprotein immunoglobulin G (IgG), a major effector molecule of the humoral immune response in man. Antibodies of the IgG class express their predominant activity during a secondary antibody response. The basic immunoglobulin G molecule has a four-chain structure, comprising two identical heavy (H) chains and two identical light (L) chains, linked together by inter-chain disulfide bonds. Each heavy chain is encoded by 4 distinct types of gene segments, designated V_{H} (variable), D (diversity), J_{H} (joining) and C_{H}(constant). The variable region of the heavy chain is encoded by the V_{H}, D and J_{H} segments. The light chains are encoded by the 3 gene segments, V_{L}, J_{L} and C_{L}. The variable region of the light chains is encoded by the V_{L} and J_{L} segments.

As used herein, the term "IgG2" refers to subclass 2 of the glycoprotein immunoglobulin G (IgG), a major effector molecule of the humoral immune response in man. Antibodies of the IgG class express their predominant activity during a secondary antibody response. The basic immunoglobulin G molecule has a four-chain structure, comprising two identical heavy (H) chains and two identical light (L) chains, linked together by inter-chain disulfide bonds. Each heavy chain is encoded by 4 distinct types of gene segments, designated V_{H} (variable), D (diversity), J_{H} (joining) and C_{H}(constant). The variable region of the heavy chain is encoded by the V_{H}, D and J_{H} segments. The light chains are encoded by the 3 gene segments, V_{L}, J_{L} and C_{L}. The variable region of the light chains is encoded by the V_{L} and J_{L} segments.

The length and flexibility of the hinge region varies among the IgG subclasses. The hinge region of IgG1 encompasses amino acids 216-231 and since it is freely flexible, the Fab fragments can rotate about their axes of symmetry and move within a sphere centered at the first of two inter-heavy chain disulfide bridges (23). IgG2 has a shorter hinge than IgG1, with 12 amino acid residues and four disulfide bridges. The hinge region of IgG2 lacks a glycine residue, it is relatively short and contains a rigid poly-proline double helix, stabilised by extra inter-heavy chain disulfide bridges. These properties restrict the flexibility of the IgG2 molecule (24). IgG3 differs from the other subclasses by its unique extended hinge region (about four times as long as the IgG1 hinge), containing 62 amino acids (including 21 prolines and 11 cysteines), forming an inflexible poly-proline double helix (25,26). In IgG3 the Fab fragments are relatively far away from the Fc fragment, giving the molecule a greater flexibility. The elongated hinge in IgG3 is also responsible for its higher molecular weight compared to the other subclasses. The hinge region of IgG4 is shorter than that of IgG1 and its flexibility is intermediate between that of IgG1 and IgG2.

The four IgG subclasses also differ with respect to the number of inter-heavy chain disulfide bonds in the hinge region (26). The structural differences between the IgG subclasses are also reflected in their susceptibility to proteolytic enzymes. IgG3 is very susceptible to cleavage by these enzymes, whereas IgG2 is relatively resistant. IgG1 and IgG4 exhibit an intermediary sensitivity, depending upon the enzyme used. Since these proteolytic enzymes all cleave IgG molecules near or within the hinge region, it is likely that the high sensitivity of IgG3 to enzyme digestion is related to its accessible hinge. Another structural difference between the human IgG subclasses is the linkage of the heavy and light chain by a disulfide bond. This bond links the carboxy-terminal of the light chain with the cysteine residue at position 220 (in IgG) or at position 131 (in IgG2, IgG3 and IgG4) of the CH1 sequence of the heavy chain.

As a consequence of the structural differences, the four IgG subclasses may be distinguished from one another, for example using antibodies that are specific for differences between the isoforms. In the present application, a level of IgG1 is determined using an assay which distinguishes this subclass, relative to the other subclasses.

As used herein, the term "relating a signal to the presence or amount" of an analyte reflects the following understanding. Assay signals are typically related to the presence or amount of an analyte through the use of a standard curve calculated using known concentrations of the analyte of interest. As the term is used herein, an assay is "configured to detect" an analyte if an assay can generate a detectable signal indicative of the presence or amount of a physiologically relevant concentration of the analyte. Because an antibody epitope is on the order of 8 amino acids, an immunoassay configured to detect a marker of interest will also detect polypeptides related to the marker sequence, so long as those polypeptides contain the epitope(s) necessary to bind to the antibody or antibodies used in the assay. The term "related marker" as used herein with regard to a biomarker such as one of the kidney injury markers described herein refers to one or more fragments, variants, etc., of a particular marker or its biosynthetic parent that may be detected as a surrogate for the marker itself or as independent biomarkers. The term also refers to one or more polypeptides present in a biological sample that are derived from the biomarker precursor complexed to additional species, such as binding proteins, receptors, heparin, lipids, sugars, etc.

In this regard, the skilled artisan will understand that the signals obtained from an immunoassay are a direct result of complexes formed between one or more antibodies and the target biomolecule (*i.e.,* the analyte) and polypeptides containing the necessary epitope(s) to which the antibodies bind. While such assays may detect the full length biomarker and the assay result be expressed as a concentration of a biomarker of interest, the signal from the assay is actually a result of all such "immunoreactive" polypeptides present in the sample. Expression of biomarkers may also be determined by means other than immunoassays, including protein measurements (such as dot blots, western blots, chromatographic methods, mass spectrometry, *etc*.) and nucleic acid measurements (mRNA quatitation). This list is not meant to be limiting.

The term "positive going" marker as that term is used herein refer to a marker that is determined to be elevated in subjects suffering from a disease or condition, relative to subjects not suffering from that disease or condition. The term "negative going" marker as that term is used herein refer to a marker that is determined to be reduced in subjects suffering from a disease or condition, relative to subjects not suffering from that disease or condition.

The term "subject" as used herein refers to a human or non-human organism. Thus, the methods and compositions described herein are applicable to both human and veterinary disease. Further, while a subject is preferably a living organism, the invention described herein may be used in post-mortem analysis as well. Preferred subjects are humans, and most preferably "patients," which as used herein refers to living humans that are receiving medical care for a disease or condition. This includes persons with no defined illness who are being investigated for signs of pathology.

Preferably, an analyte is measured in a sample. Such a sample may be obtained from a subject, or may be obtained from biological materials intended to be provided to the subject. For example, a sample may be obtained from a kidney being evaluated for possible transplantation into a subject, and an analyte measurement used to evaluate the kidney for preexisting damage. Preferred samples are body fluid samples.

The term "body fluid sample" as used herein refers to a sample of bodily fluid obtained for the purpose of diagnosis, prognosis, classification or evaluation of a subject of interest, such as a patient or transplant donor. In certain embodiments, such a sample may be obtained for the purpose of determining the outcome of an ongoing condition or the effect of a treatment regimen on a condition. Preferred body fluid samples include blood, serum, plasma, cerebrospinal fluid, urine, saliva, sputum, and pleural effusions. In addition, one of skill in the art would realize that certain body fluid samples would be more readily analyzed following a fractionation or purification procedure, for example, separation of whole blood into serum or plasma components.

The term "diagnosis" as used herein refers to methods by which the skilled artisan can estimate and/or determine the probability ("a likelihood") of whether or not a patient is suffering from a given disease or condition. As used herein "diagnosis" includes using the results of an assay, most preferably an immunoassay, for a kidney injury marker disclosed herein, optionally together with other clinical characteristics, to arrive at a diagnosis (that is, the occurrence or nonoccurrence) of an acute renal injury or ARF for the subject from which a sample was obtained and assayed. That such a diagnosis is "determined" is not meant to imply that the diagnosis is 100% accurate. Many biomarkers are indicative of multiple conditions. The skilled clinician does not use biomarker results in an informational vacuum, but rather test results are used together with other clinical indicia to arrive at a diagnosis. Thus, a measured biomarker level on one side of a predetermined diagnostic threshold indicates a greater likelihood of the occurrence of disease in the subject relative to a measured level on the other side of the predetermined diagnostic threshold.

Similarly, a prognostic risk signals a probability ("a likelihood") that a given course or outcome will occur. A level or a change in level of a prognostic indicator, which in turn is associated with an increased probability of morbidity (e.g., worsening renal function, future ARF, or death) is referred to as being "indicative of an increased likelihood" of an adverse outcome in a patient.

### Marker Assays

In general, immunoassays involve contacting a sample containing or suspected of containing a biomarker of interest with at least one antibody that specifically binds to the biomarker. A signal is then generated indicative of the presence or amount of complexes formed by the binding of polypeptides in the sample to the antibody. The signal is then related to the presence or amount of the biomarker in the sample. Numerous methods and devices are well known to the skilled artisan for the detection and analysis of biomarkers. *See, e.g.,* U.S. Patents 6,143,576; 6,113,855; 6,019,944; 5,985,579; 5,947,124; 5,939,272; 5,922,615; 5,885,527; 5,851,776; 5,824,799; 5,679,526; 5,525,524; and 5,480,792, and The Immunoassay Handbook, David Wild, ed. Stockton Press, New York, 1994.

The assay devices and methods known in the art can utilize labeled molecules in various sandwich, competitive, or non-competitive assay formats, to generate a signal that is related to the presence or amount of the biomarker of interest. Suitable assay formats also include chromatographic, mass spectrographic, and protein "blotting" methods. Additionally, certain methods and devices, such as biosensors and optical immunoassays, may be employed to determine the presence or amount of analytes without the need for a labeled molecule. *See, e.g.,* U.S. Patents 5,631,171; and 5,955,377. One skilled in the art also recognizes that robotic instrumentation including but not limited to Beckman ACCESS®, Abbott AXSYM®, Roche ELECSYS®, Dade Behring STRATUS® systems are among the immunoassay analyzers that are capable of performing immunoassays. But any suitable immunoassay may be utilized, for example, enzyme-linked immunoassays (ELISA), radioimmunoassays (RIAs), competitive binding assays, and the like.

Antibodies or other polypeptides may be immobilized onto a variety of solid supports for use in assays. Solid phases that may be used to immobilize specific binding members include include those developed and/or used as solid phases in solid phase binding assays. Examples of suitable solid phases include membrane filters, cellulose-based papers, beads (including polymeric, latex and paramagnetic particles), glass, silicon wafers, microparticles, nanoparticles, TentaGels, AgroGels, PEGA gels, SPOCC gels, and multiple-well plates. An assay strip could be prepared by coating the antibody or a plurality of antibodies in an array on solid support. This strip could then be dipped into the test sample and then processed quickly through washes and detection steps to generate a measurable signal, such as a colored spot. Antibodies or other polypeptides may be bound to specific zones of assay devices either by conjugating directly to an assay device surface, or by indirect binding. In an example of the later case, antibodies or other polypeptides may be immobilized on particles or other solid supports, and that solid support immobilized to the device surface.

Biological assays require methods for detection, and one of the most common methods for quantitation of results is to conjugate a detectable label to a protein or nucleic acid that has affinity for one of the components in the biological system being studied. Detectable labels may include molecules that are themselves detectable (*e.g.,* fluorescent moieties, electrochemical labels, metal chelates, *etc*.) as well as molecules that may be indirectly detected by production of a detectable reaction product (*e.g.,* enzymes such as horseradish peroxidase, alkaline phosphatase, *etc*.) or by a specific binding molecule which itself may be detectable (*e.g.,* biotin, digoxigenin, maltose, oligohistidine, 2,4-dintrobenzene, phenylarsenate, ssDNA, dsDNA, etc.).

Preparation of solid phases and detectable label conjugates often comprise the use of chemical cross-linkers. Cross-linking reagents contain at least two reactive groups, and are divided generally into homo functional cross-linkers (containing identical reactive groups) and heterofunctional cross-linkers (containing non-identical reactive groups). Homobifunctional cross-linkers that couple through amines, sulfhydryls or react non-specifically are available from many commercial sources. Maleimides, alkyl and aryl halides, alpha-haloacyls and pyridyl disulfides are thiol reactive groups. Maleimides, alkyl and aryl halides, and alpha-haloacyls react with sulfhydryls to form thiol ether bonds, while pyridyl disulfides react with sulfhydryls to produce mixed disulfides. The pyridyl disulfide product is cleavable. Imidoesters are also very useful for protein-protein cross-links. A variety of heterobifunctional cross-linkers, each combining different attributes for successful conjugation, are commercially available.

In certain aspects, kits for the analysis of the described kidney injury markers are disclosed. The kit comprises reagents for the analysis of at least one test sample which comprise at least one antibody that a kidney injury marker. The kit can also include devices and instructions for performing one or more of the diagnostic and/or prognostic correlations described herein. Preferred kits will comprise an antibody pair for performing a sandwich assay, or a labeled species for performing a competitive assay, for the analyte. Preferably, an antibody pair comprises a first antibody conjugated to a solid phase and a second antibody conjugated to a detectable label, wherein each of the first and second antibodies that bind a kidney injury marker. Most preferably each of the antibodies are monoclonal antibodies. The instructions for use of the kit and performing the correlations can be in the form of labeling, which refers to any written or recorded material that is attached to, or otherwise accompanies a kit at any time during its manufacture, transport, sale or use. For example, the term labeling encompasses advertising leaflets and brochures, packaging materials, instructions, audio or video cassettes, computer discs, as well as writing imprinted directly on kits.

### Antibodies

The term "antibody" as used herein refers to a peptide or polypeptide derived from, modeled after or substantially encoded by an immunoglobulin gene or immunoglobulin genes, or fragments thereof, capable of specifically binding an antigen or epitope. *See, e.g.* Fundamental Immunology, 3rd Edition, W.E. Paul, ed., Raven Press, N.Y. (1993); Wilson (1994; J. Immunol. Methods 175:267-273; Yarmush (1992) J. Biochem. Biophys. Methods 25:85-97. The term antibody includes antigen-binding portions, i.e., "antigen binding sites," (e.g., fragments, subsequences, complementarity determining regions (CDRs)) that retain capacity to bind antigen, including (i) a Fab fragment, a monovalent fragment consisting of the VL, VH, CL and CHl domains; (ii) a F(ab')2 fragment, a bivalent fragment comprising two Fab fragments linked by a disulfide bridge at the hinge region; (iii) a Fd fragment consisting of the VH and CHl domains; (iv) a Fv fragment consisting of the VL and VH domains of a single arm of an antibody, (v) a dAb fragment (Ward et al., (1989) Nature 341:544-546), which consists of a VH domain; and (vi) an isolated complementarity determining region (CDR). Single chain antibodies are also included by reference in the term "antibody."

Antibodies used in the immunoassays described herein preferably specifically bind to a kidney injury marker disclosed herein. The term "specifically binds" is not intended to indicate that an antibody binds exclusively to its intended target since, as noted above, an antibody binds to any polypeptide displaying the epitope(s) to which the antibody binds. Rather, an antibody "specifically binds" if its affinity for its intended target is about 5-fold greater when compared to its affinity for a non-target molecule which does not display the appropriate epitope(s). Preferably the affinity of the antibody will be at least about 5 fold, preferably 10 fold, more preferably 25-fold, even more preferably 50-fold, and most preferably 100-fold or more, greater for a target molecule than its affinity for a non-target molecule. In preferred embodiments, Preferred antibodies bind with affinities of at least about 10⁷ M⁻¹, and preferably between about 10⁸ M⁻¹ to about 10⁹ M⁻¹, about 10⁹ M⁻¹ to about 10¹⁰ M⁻¹, or about 10¹⁰ M⁻¹ to about 10¹² M⁻¹.

Affinity is calculated as K_{d} = k_{off}/kₒₙ (k_{off} is the dissociation rate constant, Kₒₙ is the association rate constant and K_{d} is the equilibrium constant). Affinity can be determined at equilibrium by measuring the fraction bound (r) of labeled ligand at various concentrations (c). The data are graphed using the Scatchard equation: r/c = K(n-r): where r = moles of bound ligand/mole of receptor at equilibrium; c = free ligand concentration at equilibrium; K = equilibrium association constant; and n = number of ligand binding sites per receptor molecule. By graphical analysis, r/c is plotted on the Y-axis versus r on the X-axis, thus producing a Scatchard plot. Antibody affinity measurement by Scatchard analysis is well known in the art. *See, e.g.,* van Erp et al., J. Immunoassay 12: 425-43, 1991; Nelson and Griswold, Comput. Methods Programs Biomed. 27: 65-8, 1988.

The term "epitope" refers to an antigenic determinant capable of specific binding to an antibody. Epitopes usually consist of chemically active surface groupings of molecules such as amino acids or sugar side chains and usually have specific three dimensional structural characteristics, as well as specific charge characteristics. Conformational and nonconformational epitopes are distinguished in that the binding to the former but not the latter is lost in the presence of denaturing solvents.

Numerous publications discuss the use of phage display technology to produce and screen libraries of polypeptides for binding to a selected analyte. *See, e.g,* Cwirla et al., Proc. Natl. Acad. Sci. USA 87, 6378-82, 1990; Devlin et al., Science 249, 404-6, 1990, Scott and Smith, Science 249, 386-88, 1990; and Ladner et al., U.S. Pat. No. 5,571,698. A basic concept of phage display methods is the establishment of a physical association between DNA encoding a polypeptide to be screened and the polypeptide. This physical association is provided by the phage particle, which displays a polypeptide as part of a capsid enclosing the phage genome which encodes the polypeptide. The establishment of a physical association between polypeptides and their genetic material allows simultaneous mass screening of very large numbers of phage bearing different polypeptides. Phage displaying a polypeptide with affinity to a target bind to the target and these phage are enriched by affinity screening to the target. The identity of polypeptides displayed from these phage can be determined from their respective genomes. Using these methods a polypeptide identified as having a binding affinity for a desired target can then be synthesized in bulk by conventional means. *See, e.g.,* U.S. Patent No. 6,057,098.

The antibodies that are generated by these methods may then be selected by first screening for affinity and specificity with the purified polypeptide of interest and, if required, comparing the results to the affinity and specificity of the antibodies with polypeptides that are desired to be excluded from binding. The screening procedure can involve immobilization of the purified polypeptides in separate wells of microtiter plates. The solution containing a potential antibody or groups of antibodies is then placed into the respective microtiter wells and incubated for about 30 min to 2 h. The microtiter wells are then washed and a labeled secondary antibody (for example, an anti-mouse antibody conjugated to alkaline phosphatase if the raised antibodies are mouse antibodies) is added to the wells and incubated for about 30 min and then washed. Substrate is added to the wells and a color reaction will appear where antibody to the immobilized polypeptide(s) are present.

The antibodies so identified may then be further analyzed for affinity and specificity in the assay design selected. In the development of immunoassays for a target protein, the purified target protein acts as a standard with which to judge the sensitivity and specificity of the immunoassay using the antibodies that have been selected. Because the binding affinity of various antibodies may differ; certain antibody pairs (e.g., in sandwich assays) may interfere with one another sterically, etc., assay performance of an antibody may be a more important measure than absolute affinity and specificity of an antibody.

While the present application describes antibody-based binding assays in detail, alternatives to antibodies as binding species in assays are well known in the art. These include receptors for a particular target, aptamers, etc. Aptamers are oligonucleic acid or peptide molecules that bind to a specific target molecule. Aptamers are usually created by selecting them from a large random sequence pool, but natural aptamers also exist. High-affinity aptamers containing modified nucleotides conferring improved characteristics on the ligand, such as improved in vivo stability or improved delivery characteristics. Examples of such modifications include chemical substitutions at the ribose and/or phosphate and/or base positions, and may include amino acid side chain functionalities.

### Assay Correlations

The term "correlating" as used herein in reference to the use of biomarkers refers to comparing the presence or amount of the biomarker(s) in a patient to its presence or amount in persons known to suffer from, or known to be at risk of, a given condition; or in persons known to be free of a given condition. Often, this takes the form of comparing an assay result in the form of a biomarker concentration to a predetermined threshold selected to be indicative of the occurrence or nonoccurrence of a disease or the likelihood of some future outcome.

Selecting a diagnostic threshold involves, among other things, consideration of the probability of disease, distribution of true and false diagnoses at different test thresholds, and estimates of the consequences of treatment (or a failure to treat) based on the diagnosis. For example, when considering administering a specific therapy which is highly efficacious and has a low level of risk, few tests are needed because clinicians can accept substantial diagnostic uncertainty. On the other hand, in situations where treatment options are less effective and more risky, clinicians often need a higher degree of diagnostic certainty. Thus, cost/benefit analysis is involved in selecting a diagnostic threshold.

Suitable thresholds may be determined in a variety of ways. For example, one recommended diagnostic threshold for the diagnosis of acute myocardial infarction using cardiac troponin is the 97.5th percentile of the concentration seen in a normal population. Another method may be to look at serial samples from the same patient, where a prior "baseline" result is used to monitor for temporal changes in a biomarker level.

Population studies may also be used to select a decision threshold. Reciever Operating Characteristic ("ROC") arose from the field of signal dectection therory developed during World War II for the analysis of radar images, and ROC analysis is often used to select a threshold able to best distinguish a "diseased" subpopulation from a "nondiseased" subpopulation. A false positive in this case occurs when the person tests positive, but actually does not have the disease. A false negative, on the other hand, occurs when the person tests negative, suggesting they are healthy, when they actually do have the disease. To draw a ROC curve, the true positive rate (TPR) and false positive rate (FPR) are determined as the decision threshold is varied continuously. Since TPR is equivalent with sensitivity and FPR is equal to 1 - specificity, the ROC graph is sometimes called the sensitivity vs (1 - specificity) plot. A perfect test will have an area under the ROC curve of 1.0; a random test will have an area of 0.5. A threshold is selected to provide an acceptable level of specificity and sensitivity.

In this context, "diseased" is meant to refer to a population having one characteristic (the presence of a disease or condition or the occurrence of some outcome) and "nondiseased" is meant to refer to a population lacking the characteristic. While a single decision threshold is the simplest application of such a method, multiple decision thresholds may be used. For example, below a first threshold, the absence of disease may be assigned with relatively high confidence, and above a second threshold the presence of disease may also be assigned with relatively high confidence. Between the two thresholds may be considered indeterminate. This is meant to be exemplary in nature only.

In addition to threshold comparisons, other methods for correlating assay results to a patient classification (occurrence or nonoccurrence of disease, likelihood of an outcome, etc.) include decision trees, rule sets, Bayesian methods, and neural network methods. These methods can produce probability values representing the degree to which a subject belongs to one classification out of a plurality of classifications.

Measures of test accuracy may be obtained as described in Fischer et al., Intensive Care Med. 29: 1043-51, 2003, and used to determine the effectiveness of a given biomarker. These measures include sensitivity and specificity, predictive values, likelihood ratios, diagnostic odds ratios, and ROC curve areas. The area under the curve ("AUC") of a ROC plot is equal to the probability that a classifier will rank a randomly chosen positive instance higher than a randomly chosen negative one. The area under the ROC curve may be thought of as equivalent to the Mann-Whitney U test, which tests for the median difference between scores obtained in the two groups considered if the groups are of continuous data, or to the Wilcoxon test of ranks.

As discussed above, suitable tests may exhibit one or more of the following results on these various measures: a specificity of greater than 0.5, preferably at least 0.6, more preferably at least 0.7, still more preferably at least 0.8, even more preferably at least 0.9 and most preferably at least 0.95, with a corresponding sensitivity greater than 0.2, preferably greater than 0.3, more preferably greater than 0.4, still more preferably at least 0.5, even more preferably 0.6, yet more preferably greater than 0.7, still more preferably greater than 0.8, more preferably greater than 0.9, and most preferably greater than 0.95; a sensitivity of greater than 0.5, preferably at least 0.6, more preferably at least 0.7, still more preferably at least 0.8, even more preferably at least 0.9 and most preferably at least 0.95, with a corresponding specificity greater than 0.2, preferably greater than 0.3, more preferably greater than 0.4, still more preferably at least 0.5, even more preferably 0.6, yet more preferably greater than 0.7, still more preferably greater than 0.8, more preferably greater than 0.9, and most preferably greater than 0.95; at least 75% sensitivity, combined with at least 75% specificity; a ROC curve area of greater than 0.5, preferably at least 0.6, more preferably 0.7, still more preferably at least 0.8, even more preferably at least 0.9, and most preferably at least 0.95; an odds ratio different from 1, preferably at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less; a positive likelihood ratio (calculated as sensitivity/(1-specificity)) of greater than 1, at least 2, more preferably at least 3, still more preferably at least 5, and most preferably at least 10; and or a negative likelihood ratio (calculated as (1-sensitivity)/specificity) of less than 1, less than or equal to 0.5, more preferably less than or equal to 0.3, and most preferably less than or equal to 0.1

Additional clinical indicia may be combined with the kidney injury marker assay result(s). These include other biomarkers related to renal status. Examples include the following, which recite the common biomarker name, followed by the Swiss-Prot entry number for that biomarker or its parent: Actin (P68133); Adenosine deaminase binding protein (DPP4, P27487); Alpha-1-acid glycoprotein 1 (P02763); Alpha-1-microglobulin (P02760); Albumin (P02768); Angiotensinogenase (Renin, P00797); Annexin A2 (P07355); Beta-glucuronidase (P08236); B-2-microglobulin (P61679); Beta-galactosidase (P16278); BMP-7 (P18075); Brain natriuretic peptide (proBNP, BNP-32, NTproBNP; P16860); Calcium-binding protein Beta (S100-beta, P04271); Carbonic anhydrase (Q16790); Casein Kinase 2 (P68400); Ceruloplasmin (P00450); Clusterin (P10909); Complement C3 (P01024); Cysteine-rich protein (CYR61, 000622); Cytochrome C (P99999); Epidermal growth factor (EGF, P01133); Endothelin-1 (P05305); Exosomal Fetuin-A (P02765); Fatty acid-binding protein, heart (FABP3, P05413); Fatty acid-binding protein, liver (P07148); Ferritin (light chain, P02793; heavy chain P02794); Fructose-1,6-biphosphatase (P09467); GRO-alpha (CXCL1, (P09341); Growth Hormone (P01241); Hepatocyte growth factor (P14210); Insulin-like growth factor I (P01343); Immunoglobulin G; Immunoglobulin Light Chains (Kappa and Lambda); Interferon gamma (P01308); Lysozyme (P61626); Interleukin-1 alpha (P01583); Interleukin-2 (P60568); Interleukin-4 (P60568); Interleukin-9 (P15248); Interleukin-12p40 (P29460); Interleukin-13 (P35225); Interleukin-16 (Q14005); L1 cell adhesion molecule (P32004); Lactate dehydrogenase (P00338); Leucine Aminopeptidase (P28838); Meprin A-alpha subunit (Q16819); Meprin A-beta subunit (Q16820); Midkine (P21741); MIP2-alpha (CXCL2, P19875); MMP-2 (P08253); MMP-9 (P14780); Netrin-1 (095631); Neutral endopeptidase (P08473); Osteopontin (P10451); Renal papillary antigen 1 (RPA1); Renal papillary antigen 2 (RPA2); Retinol binding protein (P09455); Ribonuclease; S100 calcium-binding protein A6 (P06703); Serum Amyloid P Component (P02743); Sodium/Hydrogen exchanger isoform (NHE3, P48764); Spermidine/spermine N1-acetyltransferase (P21673); TGF-Beta1 (P01137); Transferrin (P02787); Trefoil factor 3 (TFF3, Q07654); Toll-Like protein 4 (000206); Total protein; Tubulointerstitial nephritis antigen (Q9UJW2); Uromodulin (Tamm-Horsfall protein, P07911).

For purposes of risk stratification, Adiponectin (Q15848); Alkaline phosphatase (P05186); Aminopeptidase N (P15144); CalbindinD28k (P05937); Cystatin C (P01034); 8 subunit of FIFO ATPase (P03928); Gamma-glutamyltransferase (P19440); GSTa (alpha-glutathione-S-transferase, P08263); GSTpi (Glutathione-S-transferase P; GST class-pi; P09211); IGFBP-1 (P08833); IGFBP-2 (P18065); IGFBP-6 (P24592); Integral membrane protein 1 (Itm1, P46977); Interleukin-6 (P05231); Interleukin-8 (P10145); Interleukin-18 (Q14116); IP-10 (10 kDa interferon-gamma-induced protein, P02778); IRPR (IFRD1, 000458); Isovaleryl-CoA dehydrogenase (IVD, P26440); I-TAC/CXCL11 (014625); Keratin 19 (P08727); Kim-1 (Hepatitis A virus cellular receptor 1, O43656); L-arginine:glycine amidinotransferase (P50440); Leptin (P41159); Lipocalin2 (NGAL, P80188); MCP-1 (P13500); MIG (Gamma-interferon-induced monokine Q07325); MIP-1a (P10147); MIP-3a (P78556); MIP-1beta (P13236); MIP-1d (Q16663); NAG (N-acetyl-beta-D-glucosaminidase, P54802); Organic ion transporter (OCT2, 015244); Osteoprotegerin (014788); P8 protein (060356); Plasminogen activator inhibitor 1 (PAI-1, P05121); ProANP(1-98) (P01160); Protein phosphatase 1-beta (PPI-beta, P62140); Rab GDI-beta (P50395); Renal kallikrein (Q86U61); RT1.B-1 (alpha) chain of the integral membrane protein (Q5Y7A8); Soluble tumor necrosis factor receptor superfamily member 1A (sTNFR-I, P19438); Soluble tumor necrosis factor receptor superfamily member 1B (sTNFR-II, P20333); Tissue inhibitor of metalloproteinases 3 (TIMP-3, P35625); uPAR (Q03405) may be combined with the kidney injury marker assay result(s).

Other clinical indicia which may be combined with the kidney injury marker assay result(s) includes demographic information (e.g., weight, sex, age, race), medical history (e.g., family history, type of surgery, pre-existing disease such as aneurism, congestive heart failure, preeclampsia, eclampsia, diabetes mellitus, hypertension, coronary artery disease, proteinuria, renal insufficiency, or sepsis, type of toxin exposure such as NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin), clinical variables (e.g., blood pressure, temperature, respiration rate), risk scores (APACHE score, PREDICT score, TIMI Risk Score for UA/NSTEMI, Framingham Risk Score), a urine total protein measurement, a glomerular filtration rate, an estimated glomerular filtration rate, a urine production rate, a serum or plasma creatinine concentration, a renal papillary antigen 1 (RPA1) measurement; a renal papillary antigen 2 (RPA2) measurement; a urine creatinine concentration, a fractional excretion of sodium, a urine sodium concentration, a urine creatinine to serum or plasma creatinine ratio, a urine specific gravity, a urine osmolality, a urine urea nitrogen to plasma urea nitrogen ratio, a plasma BUN to creatnine ratio, and/or a renal failure index calculated as urine sodium / (urine creatinine / plasma creatinine). Other measures of renal function which may be combined with the kidney injury marker assay result(s) are described hereinafter and in Harrison's Principles of Internal Medicine, 17th Ed., McGraw Hill, New York, pages 1741-1830, and Current Medical Diagnosis & Treatment 2008, 47th Ed, McGraw Hill, New York, pages 785-815.

Combining assay results/clinical indicia in this manner can comprise the use of multivariate logistical regression, loglinear modeling, neural network analysis, n-of-m analysis, decision tree analysis, etc. This list is not meant to be limiting.

### Diagnosis of Acute Renal Failure

As noted above, the terms "acute renal (or kidney) injury" and "acute renal (or kidney) failure" as used herein are defined in part in terms of changes in serum creatinine from a baseline value. Most definitions of ARF have common elements, including the use of serum creatinine and, often, urine output. Patients may present with renal dysfunction without an available baseline measure of renal function for use in this comparison. In such an event, one may estimate a baseline serum creatinine value by assuming the patient initially had a normal GFR. Glomerular filtration rate (GFR) is the volume of fluid filtered from the renal (kidney) glomerular capillaries into the Bowman's capsule per unit time. Glomerular filtration rate (GFR) can be calculated by measuring any chemical that has a steady level in the blood, and is freely filtered but neither reabsorbed nor secreted by the kidneys. GFR is typically expressed in units of ml/min: $G F R = \frac{Urine Concentration \times Urine Flow}{Plasma Concentration}$

By normalizing the GFR to the body surface area, a GFR of approximately 75-100 ml/min per 1.73 m² can be assumed. The rate therefore measured is the quantity of the substance in the urine that originated from a calculable volume of blood.

There are several different techniques used to calculate or estimate the glomerular filtration rate (GFR or eGFR). In clinical practice, however, creatinine clearance is used to measure GFR. Creatinine is produced naturally by the body (creatinine is a metabolite of creatine, which is found in muscle). It is freely filtered by the glomerulus, but also actively secreted by the renal tubules in very small amounts such that creatinine clearance overestimates actual GFR by 10-20%. This margin of error is acceptable considering the ease with which creatinine clearance is measured.

Creatinine clearance (CCr) can be calculated if values for creatinine's urine concentration (U_{Cr}), urine flow rate (V), and creatinine's plasma concentration (P_{Cr}) are known. Since the product of urine concentration and urine flow rate yields creatinine's excretion rate, creatinine clearance is also said to be its excretion rate (U_{Cr}×V) divided by its plasma concentration. This is commonly represented mathematically as: ${C}_{C r} = \frac{{U}_{C r} \times V}{{P}_{C r}}$ Commonly a 24 hour urine collection is undertaken, from empty-bladder one morning to the contents of the bladder the following morning, with a comparative blood test then taken: ${C}_{C r} = \frac{{U}_{C r} \times 24 - hour volume}{{P}_{C r} \times 24 \times 60 m i n s}$ To allow comparison of results between people of different sizes, the CCr is often corrected for the body surface area (BSA) and expressed compared to the average sized man as ml/min/1.73 m2. While most adults have a BSA that approaches 1.7 (1.6-1.9), extremely obese or slim patients should have their CCr corrected for their actual BSA: ${C}_{C r - c o r r e c t e d} = \frac{{C}_{C r} \times 1.73}{B S A}$

The accuracy of a creatinine clearance measurement (even when collection is complete) is limited because as glomerular filtration rate (GFR) falls creatinine secretion is increased, and thus the rise in serum creatinine is less. Thus, creatinine excretion is much greater than the filtered load, resulting in a potentially large overestimation of the GFR (as much as a twofold difference). However, for clinical purposes it is important to determine whether renal function is stable or getting worse or better. This is often determined by monitoring serum creatinine alone. Like creatinine clearance, the serum creatinine will not be an accurate reflection of GFR in the non-steady-state condition of ARF. Nonetheless, the degree to which serum creatinine changes from baseline will reflect the change in GFR. Serum creatinine is readily and easily measured and it is specific for renal function.

For purposes of determining urine output on a Urine output on a mL/kg/hr basis, hourly urine collection and measurement is adequate. In the case where, for example, only a cumulative 24-h output was available and no patient weights are provided, minor modifications of the RIFLE urine output criteria have been described. For example, Bagshaw et al., Nephrol. Dial. Transplant. 23: 1203-1210, 2008, assumes an average patient weight of 70 kg, and patients are assigned a RIFLE classification based on the following: <35 mL/h (Risk), <21 mL/h (Injury) or <4 mL/h (Failure).

### Selecting a Treatment Regimen

Once a diagnosis is obtained, the clinician can readily select a treatment regimen that is compatible with the diagnosis, such as initiating renal replacement therapy, withdrawing delivery of compounds that are known to be damaging to the kidney, kidney transplantation, delaying or avoiding procedures that are known to be damaging to the kidney, modifying diuretic administration, initiating goal directed therapy, etc. The skilled artisan is aware of appropriate treatments for numerous diseases discussed in relation to the methods of diagnosis described herein. See, e.g., Merck Manual of Diagnosis and Therapy, 17th Ed. Merck Research Laboratories, Whitehouse Station, NJ, 1999. In addition, since the methods and compositions described herein provide prognostic information, the markers described herein may be used to monitor a course of treatment. For example, improved or worsened prognostic state may indicate that a particular treatment is or is not efficacious.

One skilled in the art readily appreciates that the present invention is well adapted to carry out the objects and obtain the ends and advantages mentioned, as well as those inherent therein. The examples provided herein are representative of preferred embodiments and are exemplary.

### Example 1: Contrast-induced nephropathy sample collection

The objective of this sample collection study is to collect samples of plasma and urine and clinical data from patients before and after receiving intravascular contrast media. Approximately 250 adults undergoing radiographic/angiographic procedures involving intravascular administration of iodinated contrast media are enrolled. To be enrolled in the study, each patient must meet all of the following inclusion criteria and none of the following exclusion criteria:
Inclusion Criteria
   males and females 18 years of age or older;
   undergoing a radiographic / angiographic procedure (such as a CT scan or coronary intervention) involving the intravascular administration of contrast media;
   expected to be hospitalized for at least 48 hours after contrast administration.
   able and willing to provide written informed consent for study participation and to comply with all study procedures.
Exclusion Criteria
   renal transplant recipients;
   acutely worsening renal function prior to the contrast procedure;
   already receiving dialysis (either acute or chronic) or in imminent need of dialysis at enrollment;
   expected to undergo a major surgical procedure (such as involving cardiopulmonary bypass) or an additional imaging procedure with contrast media with significant risk for further renal insult within the 48 hrs following contrast administration;
   participation in an interventional clinical study with an experimental therapy within the previous 30 days;
   known infection with human immunodeficiency virus (HIV) or a hepatitis virus.

Immediately prior to the first contrast administration (and after any pre-procedure hydration), an EDTA anti-coagulated blood sample (10 mL) and a urine sample (10 mL) are collected from each patient. Blood and urine samples are then collected at 4 (±0.5), 8 (±1), 24 (±2) 48 (±2), and 72 (±2) hrs following the last administration of contrast media during the index contrast procedure. Blood is collected via direct venipuncture or via other available venous access, such as an existing femoral sheath, central venous line, peripheral intravenous line or hep-lock. These study blood samples are processed to plasma at the clinical site, frozen and shipped to Astute Medical, Inc., San Diego, CA. The study urine samples are frozen and shipped to Astute Medical, Inc.

Serum creatinine is assessed at the site immediately prior to the first contrast administration (after any pre-procedure hydration) and at 4 (±0.5), 8 (±1), 24 (±2) and 48 (±2)), and 72 (±2) hours following the last administration of contrast (ideally at the same time as the study samples are obtained). In addition, each patient's status is evaluated through day 30 with regard to additional serum and urine creatinine measurements, a need for dialysis, hospitalization status, and adverse clinical outcomes (including mortality).

Prior to contrast administration, each patient is assigned a risk based on the following assessment: systolic blood pressure <80 mm Hg = 5 points; intra-arterial balloon pump = 5 points; congestive heart failure (Class III-IV or history of pulmonary edema) = 5 points; age >75 yrs = 4 points; hematocrit level <39% for men, <35% for women = 3 points; diabetes = 3 points; contrast media volume = 1 point for each 100 mL; serum creatinine level >1.5 g/dL = 4 points OR estimated GFR 40-60 mL/min/1.73 m² = 2 points, 20-40 mL/min/1.73 m² = 4 points, < 20 mL/min/1.73 m² = 6 points. The risks assigned are as follows: risk for CIN and dialysis: 5 or less total points = risk of CIN - 7.5%, risk of dialysis - 0.04%; 6-10 total points = risk of CIN - 14%, risk of dialysis - 0.12%; 11-16 total points = risk of CIN - 26.1%, risk of dialysis - 1.09%; >16 total points = risk of CIN - 57.3%, risk of dialysis - 12.8%.

### Example 2: Cardiac surgery sample collection

The objective of this sample collection study is to collect samples of plasma and urine and clinical data from patients before and after undergoing cardiovascular surgery, a procedure known to be potentially damaging to kidney function. Approximately 900 adults undergoing such surgery are enrolled. To be enrolled in the study, each patient must meet all of the following inclusion criteria and none of the following exclusion criteria:
Inclusion Criteria
   males and females 18 years of age or older;
   undergoing cardiovascular surgery;
   Toronto/Ottawa Predictive Risk Index for Renal Replacement risk score of at least 2 (Wijeysundera et al., JAMA 297: 1801-9, 2007); and
   able and willing to provide written informed consent for study participation and to comply with all study procedures.
Exclusion Criteria
   known pregnancy;
   previous renal transplantation;
   acutely worsening renal function prior to enrollment (e.g., any category of RIFLE criteria);
   already receiving dialysis (either acute or chronic) or in imminent need of dialysis at enrollment;
   currently enrolled in another clinical study or expected to be enrolled in another clinical study within 7 days of cardiac surgery that involves drug infusion or a therapeutic intervention for AKI;
   known infection with human immunodeficiency virus (HIV) or a hepatitis virus.

Within 3 hours prior to the first incision (and after any pre-procedure hydration), an EDTA anti-coagulated blood sample (10 mL), whole blood (3 mL), and a urine sample (35 mL) are collected from each patient. Blood and urine samples are then collected at 3 (±0.5), 6 (±0.5), 12 (±1), 24 (±2) and 48 (±2) hrs following the procedure and then daily on days 3 through 7 if the subject remains in the hospital. Blood is collected via direct venipuncture or via other available venous access, such as an existing femoral sheath, central venous line, peripheral intravenous line or hep-lock. These study blood samples are frozen and shipped to Astute Medical, Inc., San Diego, CA. The study urine samples are frozen and shipped to Astute Medical, Inc.

### Example 3: Acutely ill subject sample collection

The objective of this study is to collect samples from acutely ill patients. Approximately 1900 adults expected to be in the ICU for at least 48 hours will be enrolled. To be enrolled in the study, each patient must meet all of the following inclusion criteria and none of the following exclusion criteria:
Inclusion Criteria
males and females 18 years of age or older;
   Study population 1: approximately 300 patients that have at least one of:
      shock (SBP < 90 mmHg and/or need for vasopressor support to maintain MAP > 60 mmHg and/or documented drop in SBP of at least 40 mmHg); and
      sepsis;
   Study population 2: approximately 300 patients that have at least one of:
      IV antibiotics ordered in computerized physician order entry (CPOE) within 24 hours of enrollment;
      contrast media exposure within 24 hours of enrollment;
      increased Intra-Abdominal Pressure with acute decompensated heart failure; and
      severe trauma as the primary reason for ICU admission and likely to be hospitalized in the ICU for 48 hours after enrollment;
   Study population 3: approximately 300 patients expected to be hospitalized through acute care setting (ICU or ED) with a known risk factor for acute renal injury (e.g. sepsis, hypotension/shock (Shock = systolic BP < 90 mmHg and/or the need for vasopressor support to maintain a MAP > 60 mmHg and/or a documented drop in SBP > 40 mmHg), major trauma, hemorrhage, or major surgery); and/or expected to be hospitalized to the ICU for at least 24 hours after enrollment;
   Study population 4: approximately 1000 patients that are 21 years of age or older, within 24 hours of being admitted into the ICU, expected to have an indwelling urinary catheter for at least 48 hours after enrollment, and have at least one of the following acute conditions within 24 hours prior to enrollment:
      (i) respiratory SOFA score of ≥ 2 (PaO2/FiO2 <300), (ii) cardiovascular SOFA score of ≥ 1 (MAP < 70 mm Hg and/or any vasopressor required).
         Exclusion Criteria
         known pregnancy;
         institutionalized individuals;
         previous renal transplantation;
         known acutely worsening renal function prior to enrollment (e.g., any category of RIFLE criteria);
         received dialysis (either acute or chronic) within 5 days prior to enrollment or in imminent need of dialysis at the time of enrollment;
         known infection with human immunodeficiency virus (HIV) or a hepatitis virus;
         meets any of the following:
            (i) active bleeding with an anticipated need for > 4 units PRBC in a day;
            (ii) hemoglobin < 7 g/dL;
            (iii) any other condition that in the physician's opinion would contraindicate drawing serial blood samples for clinical study purposes;
   meets only the SBP < 90 mmHg inclusion criterion set forth above, and does not have shock in the attending physician's or principal investigator's opinion;

After obtaining informed consent, an EDTA anti-coagulated blood sample (10 mL) and a urine sample (25-50 mL) are collected from each patient. Blood and urine samples are then collected at 4 (± 0.5) and 8 (± 1) hours after contrast administration (if applicable); at 12 (± 1), 24 (± 2), 36 (± 2), 48 (± 2), 60 (± 2), 72 (± 2), and 84 (± 2) hours after enrollment, and thereafter daily up to day 7 to day 14 while the subject is hospitalized. Blood is collected via direct venipuncture or via other available venous access, such as an existing femoral sheath, central venous line, peripheral intravenous line or hep-lock. These study blood samples are processed to plasma at the clinical site, frozen and shipped to Astute Medical, Inc., San Diego, CA. The study urine samples are frozen and shipped to Astute Medical, Inc.

### Example 4. Immunoassay format

Analytes are measured using standard sandwich enzyme immunoassay techniques. A first antibody which binds the analyte is immobilized in wells of a 96 well polystyrene microplate. Analyte standards and test samples are pipetted into the appropriate wells and any analyte present is bound by the immobilized antibody. After washing away any unbound substances, a horseradish peroxidase-conjugated second antibody which binds the analyte is added to the wells, thereby forming sandwich complexes with the analyte (if present) and the first antibody. Following a wash to remove any unbound antibody-enzyme reagent, a substrate solution comprising tetramethylbenzidine and hydrogen peroxide is added to the wells. Color develops in proportion to the amount of analyte present in the sample. The color development is stopped and the intensity of the color is measured at 540 nm or 570 nm. An analyte concentration is assigned to the test sample by comparison to a standard curve determined from the analyte standards.

Units for the concentrations reported in the following data tables are as follows: Cancer antigen CA 15-3 - U/mL, C-C Motif chemokine 18 - ng/mL, C-C Motif chemokine 24 - pg/mL, Cathepsin D - pg/mL, C-X-C Motif chemokine 13 - pg/mL, C-C motif chemokine 8 - pg/mL, Interleukin-2 receptor alpha chain - pg/mL, Insulin-like growth factor-binding protein 3 - ng/mL, Interleukin-11 - pg/mL, Matrix Metalloproteinase-8 - pg/mL, Transforming growth factor alpha - pg/mL, IgG1 - ng/mL, and IgG2 - ng/mL. In the case of those kidney injury markers which are membrane proteins as described herein, the assays used in these examples detect soluble forms thereof.

Commercially-available reagents were sourced from the following vendors:

| Analyte | Assay Source | Catalog number |
|---|---|---|
| CA 15-3 | EMD Chemicals | Cat. # BPHCP001-6 |
| Cathepsin D | Millipore | Cat. #HNDG3-36K |
| C-C motif chemokine 18 | Millipore | Cat. #HNDG2-36K |
| C-C motif chemokine 24 | Millipore | Cat. # MPXHCYP2-62K |
| C-C motif chemokine 8 | Millipore | Cat. # MPXHCYP2-62K |
| C-X-C motif chemokine 13 | Millipore | Cat. # MPXHCYP2-62K |
| Immunoglobulin G, subclass 1 | Millipore | Cat. # HGAM-301 |
| Immunoglobulin G, subclass 2 | Millipore | Cat. # HGAM-301 |
| Insulin-like growth factor-binding protein 3 | Millipore | Cat. # HIGFBP-53K |
| Interleukin-11 | Millipore | Cat. # MPXHCYP3-63K |
| Interleukin-2 receptor alpha chain | Millipore | Cat. # MPXHCYTO-60K |
| Matrix Metalloproteinase-8 | R&D Systems | Cat. # LMP000, Cat. # LMP908 |
| Transforming growth factor alpha | Millipore | Cat. # MPXHCYTO-60K |

### Example 5. Apparently Healthy Donor and Chronic Disease Patient Samples

Human urine samples from donors with no known chronic or acute disease ("Apparently Healthy Donors") were purchased from two vendors (Golden West Biologicals, Inc., 27625 Commerce Center Dr., Temecula, CA 92590 and Virginia Medical Research, Inc., 915 First Colonial Rd., Virginia Beach, VA 23454). The urine samples were shipped and stored frozen at less than -20° C. The vendors supplied demographic information for the individual donors including gender, race (Black /White), smoking status and age.

Human urine samples from donors with various chronic diseases ("Chronic Disease Patients") including congestive heart failure, coronary artery disease, chronic kidney disease, chronic obstructive pulmonary disease, diabetes mellitus and hypertension were purchased from Virginia Medical Research, Inc., 915 First Colonial Rd., Virginia Beach, VA 23454. The urine samples were shipped and stored frozen at less than -20 degrees centigrade. The vendor provided a case report form for each individual donor with age, gender, race (Black/White), smoking status and alcohol use, height, weight, chronic disease(s) diagnosis, current medications and previous surgeries.

### Example 6. Use of Kidney Injury Markers for evaluating renal status in patients

Patients from the intensive care unit (ICU) were enrolled in the following study. Each patient was classified by kidney status as non-injury (0), risk of injury (R), injury (I), and failure (F) according to the maximum stage reached within 7 days of enrollment as determined by the RIFLE criteria. EDTA anti-coagulated blood samples (10 mL) and a urine samples (25-30 mL) were collected from each patient at enrollment, 4 (± 0.5) and 8 (± 1) hours after contrast administration (if applicable); at 12 (± 1), 24 (± 2), and 48 (± 2) hours after enrollment, and thereafter daily up to day 7 to day 14 while the subject is hospitalized. Markers were each measured by standard immunoassay methods using commercially available assay reagents in the urine samples and the plasma component of the blood samples collected.

Two cohorts were defined to represent a "diseased" and a "normal" population. While these terms are used for convenience, "diseased" and "normal" simply represent two cohorts for comparison (say RIFLE 0 vs RIFLE R, I and F; RIFLE 0 vs RIFLE R; RIFLE 0 and R vs RIFLE I and F; etc.). The time "prior max stage" represents the time at which a sample is collected, relative to the time a particular patient reaches the lowest disease stage as defined for that cohort, binned into three groups which are +/- 12 hours. For example, "24 hr prior" which uses 0 vs R, I, F as the two cohorts would mean 24 hr (+/- 12 hours) prior to reaching stage R (or I if no sample at R, or F if no sample at R or I).

A receiver operating characteristic (ROC) curve was generated for each biomarker measured and the area under each ROC curve (AUC) is determined. Patients in Cohort 2 were also separated according to the reason for adjudication to cohort 2 as being based on serum creatinine measurements (sCr), being based on urine output (UO), or being based on either serum creatinine measurements or urine output. Using the same example discussed above (0 vs R, I, F), for those patients adjudicated to stage R, I, or F on the basis of serum creatinine measurements alone, the stage 0 cohort may include patients adjudicated to stage R, I, or F on the basis of urine output; for those patients adjudicated to stage R, I, or F on the basis of urine output alone, the stage 0 cohort may include patients adjudicated to stage R, I, or F on the basis of serum creatinine measurements; and for those patients adjudicated to stage R, I, or F on the basis of serum creatinine measurements or urine output, the stage 0 cohort contains only patients in stage 0 for both serum creatinine measurements and urine output. Also, in the data for patients adjudicated on the basis of serum creatinine measurements or urine output, the adjudication method which yielded the most severe RIFLE stage is used.

The ability to distinguish cohort 1 from Cohort 2 was determined using ROC analysis. SE is the standard error of the AUC, n is the number of sample or individual patients ("pts," as indicated). Standard errors are calculated as described in Hanley, J. A., and McNeil, B.J., The meaning and use of the area under a receiver operating characteristic (ROC) curve. Radiology (1982) 143: 29-36; p values are calculated with a two-tailed Z-test. An AUC < 0.5 is indicative of a negative going marker for the comparison, and an AUC > 0.5 is indicative of a positive going marker for the comparison.

Various threshold (or "cutoff") concentrations were selected, and the associated sensitivity and specificity for distinguishing cohort 1 from cohort 2 are determined. OR is the odds ratio calculated for the particular cutoff concentration, and 95% CI is the confidence interval for the odds ratio.

**Table 1: Comparison of marker levels in urine samples collected from Cohort 1 (patients that did not progress beyond RIFLE stage 0) and in urine samples collected from subjects at 0,24 hours, and 48 hours prior to reaching stage R, I or F in Cohort 2.**

| **c-C motif chemokine 18** | | | | | | | | | |
|---|---|---|---|---|---|---|---|---|---|
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 0.336 | | 0.757 | 0.336 | | 0.545 | 0.336 | | 0.551 |
| Average | 2.29 | | 3.44 | 2.29 | | 2.98 | 2.29 | | 2.60 |
| Stdev | 7.31 | | 7.76 | 7.31 | | 7.93 | 7.31 | | 8.13 |
| p(t-test) | | | 0.13 | | | 0.35 | | | 0.78 |
| Min | 3.13E-5 | | 0.000952 | 3.13E-5 | | 0.00194 | 3.13E-5 | | 0.0160 |
| Max | 40.0 | | 40.0 | 40.0 | | 40.0 | 40.0 | | 40.0 |
| n (Samp) | 463 | | 119 | 463 | | 128 | 463 | | 47 |
| n (Patient) | 223 | | 119 | 223 | | 128 | 223 | | 47 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 0.399 | | 1.05 | 0.399 | | 0.966 | 0.399 | | 0.486 |
| Average | 2.26 | | 5.09 | 2.26 | | 2.77 | 2.26 | | 3.91 |
| Stdev | 6.86 | | 9.91 | 6.86 | | 5.93 | 6.86 | | 10.7 |
| p(t-test) | | | 0.012 | | | 0.62 | | | 0.24 |
| Min | 3.13E-5 | | 0.000952 | 3.13E-5 | | 0.00497 | 3.13E-5 | | 0.0172 |
| Max | 40.0 | | 40.0 | 40.0 | | 37.8 | 40.0 | | 40.0 |
| n (Samp) | 1015 | | 40 | 1015 | | 46 | 1015 | | 26 |
| n (Patient) | 374 | | 40 | 374 | | 46 | 374 | | 26 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 0.377 | | 0.996 | 0.377 | | 0.708 | 0.377 | | 0.701 |
| Average | 2.54 | | 4.28 | 2.54 | | 4.16 | 2.54 | | 3.83 |
| Stdev | 7.63 | | 9.11 | 7.63 | | 9.73 | 7.63 | | 9.66 |
| p(t-test) | | | 0.042 | | | 0.054 | | | 0.30 |
| Min | 3.13E-5 | | 0.0153 | 3.13E-5 | | 0.00194 | 3.13E-5 | | 0.0160 |
| Max | 40.0 | | 40.0 | 40.0 | | 40.0 | 40.0 | | 40.0 |
| n(Samp) | 436 | | 107 | 436 | | 117 | 436 | | 44 |
| n (Patient) | 173 | | 107 | 173 | | 117 | 173 | | 44 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.64 | 0.66 | 0.65 | 0.59 | 0.60 | 0.60 | 0.55 | 0.53 | 0.57 |
| SE | 0.030 | 0.048 | 0.031 | 0.029 | 0.045 | 0.030 | 0.045 | 0.058 | 0.047 |
| p | 1.1E-6 | 6.4E-4 | 1.9E-6 | 0.0024 | 0.024 | 0.0012 | 0.28 | 0.66 | 0.12 |
| nCohort 1 | 463 | 1015 | 436 | 463 | 1015 | 436 | 463 | 1015 | 436 |
| nCohort 2 | 119 | 40 | 107 | 128 | 46 | 117 | 47 | 26 | 44 |
| Cutoff 1 | 0.369 | 0.674 | 0.403 | 0.228 | 0.249 | 0.369 | 0.244 | 0.225 | 0.315 |
| Sens 1 | 71% | 70% | 70% | 70% | 72% | 70% | 70% | 73% | 70% |
| Spec 1 | 52% | 63% | 52% | 36% | 35% | 49% | 38% | 32% | 45% |
| Cutoff 2 | 0.223 | 0.315 | 0.258 | 0.142 | 0.150 | 0.177 | 0.134 | 0.129 | 0.162 |
| Sens 2 | 81% | 80% | 80% | 80% | 80% | 80% | 81% | 81% | 82% |
| Spec 2 | 35% | 44% | 36% | 26% | 24% | 27% | 25% | 20% | 25% |
| Cutoff 3 | 0.111 | 0.138 | 0.138 | 0.0629 | 0.0568 | 0.0751 | 0.0403 | 0.0403 | 0.124 |
| Sens 3 | 91% | 90% | 91% | 91% | 91% | 91% | 91% | 92% | 91% |
| Spec 3 | 21% | 21% | 21% | 12% | 8% | 13% | 9% | 7% | 20% |
| Cutoff 4 | 0.751 | 0.893 | 0.906 | 0.751 | 0.893 | 0.906 | 0.751 | 0.893 | 0.906 |
| Sens 4 | 50% | 62% | 50% | 45% | 52% | 46% | 40% | 35% | 36% |
| Spec 4 | 70% | 70% | 70% | 70% | 70% | 70% | 70% | 70% | 70% |
| Cutoff 5 | 1.12 | 1.51 | 1.63 | 1.12 | 1.51 | 1.63 | 1.12 | 1.51 | 1.63 |
| Sens 5 | 40% | 45% | 37% | 37% | 39% | 32% | 21% | 27% | 20% |
| Spec 5 | 80% | 80% | 80% | 80% | 80% | 80% | 80% | 80% | 80% |
| Cutoff 6 | 3.17 | 3.53 | 3.59 | 3.17 | 3.53 | 3.59 | 3.17 | 3.53 | 3.59 |
| Sens 6 | 24% | 22% | 26% | 16% | 20% | 16% | 9% | 12% | 11% |
| Spec 6 | 90% | 90% | 90% | 90% | 90% | 90% | 90% | 90% | 90% |
| OR Quart 2 | 1.3 | 1.2 | 1.6 | 0.73 | 0.39 | 0.75 | 0.78 | 0.83 | 0.65 |
| p Value | 0.40 | 0.77 | 0.22 | 0.32 | 0.12 | 0.40 | 0.61 | 0.76 | 0.43 |
| 95% CI of | 0.68 | 0.36 | 0.77 | 0.39 | 0.12 | 0.38 | 0.30 | 0.25 | 0.22 |
| OR Quart2 | 2.6 | 4.0 | 3.3 | 1.4 | 1.3 | 1.5 | 2.0 | 2.8 | 1.9 |
| OR Quart 3 | 2.0 | 2.0 | 2.2 | 1.3 | 1.2 | 1.6 | 1.7 | 1.3 | 2.0 |
| p Value | 0.029 | 0.20 | 0.022 | 0.32 | 0.66 | 0.14 | 0.22 | 0.59 | 0.10 |
| 95% CI of | 1.1 | 0.68 | 1.1 | 0.75 | 0.51 | 0.87 | 0.73 | 0.46 | 0.87 |
| OR Quart3 | 3.9 | 6.0 | 4.5 | 2.4 | 2.8 | 2.8 | 3.9 | 3.9 | 4.8 |
| OR Quart 4 | 3.8 | 4.0 | 4.1 | 2.3 | 2.1 | 2.2 | 1.3 | 1.2 | 1.4 |
| p Value | 1.9E-5 | 0.0066 | 2.5E-5 | 0.0027 | 0.067 | 0.0059 | 0.53 | 0.78 | 0.49 |
| 95% CI of | 2.1 | 1.5 | 2.1 | 1.3 | 0.95 | 1.3 | 0.56 | 0.39 | 0.55 |
| OR Quart4 | 7.0 | 11 | 8.0 | 4.0 | 4.5 | 4.0 | 3.1 | 3.5 | 3.4 |

| | | | | | | | | | |
|---|---|---|---|---|---|---|---|---|---|
| **C-C motif chemokine 24** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 8.31 | | 20.6 | 8.31 | | 15.9 | 8.31 | | 9.14 |
| Average | 19.2 | | 46.8 | 19.2 | | 57.8 | 19.2 | | 18.4 |
| Stdev | 49.6 | | 108 | 49.6 | | 237 | 49.6 | | 34.6 |
| p(t-test) | | | 4.9E-5 | | | 0.0012 | | | 0.91 |
| Min | 0.0120 | | 0.0160 | 0.0120 | | 0.0120 | 0.0120 | | 0.0193 |
| Max | 730 | | 1010 | 730 | | 2380 | 730 | | 216 |
| n (Samp) | 463 | | 120 | 463 | | 130 | 463 | | 47 |
| n (Patient) | 223 | | 120 | 223 | | 130 | 223 | | 47 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 11.9 | | 28.7 | 11.9 | | 20.3 | 11.9 | | 13.7 |
| Average | 31.8 | | 46.7 | 31.8 | | 39.1 | 31.8 | | 31.2 |
| Stdev | 113 | | 62.4 | 113 | | 63.5 | 113 | | 54.6 |
| p(t-test) | | | 0.41 | | | 0.66 | | | 0.98 |
| Min | 0.0120 | | 0.0160 | 0.0120 | | 0.0196 | 0.0120 | | 0.0215 |
| Max | 2380 | | 290 | 2380 | | 347 | 2380 | | 216 |
| n (Samp) | 1019 | | 40 | 1019 | | 46 | 1019 | | 26 |
| n (Patient) | 375 | | 40 | 375 | | 46 | 375 | | 26 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 8.53 | | 25.8 | 8.53 | | 18.2 | 8.53 | | 13.1 |
| Average | 21.8 | | 85.0 | 21.8 | | 86.7 | 21.8 | | 49.4 |
| Stdev | 53.7 | | 298 | 53.7 | | 309 | 53.7 | | 182 |
| p(t-test) | | | 3.6E-5 | | | 3.6E-5 | | | 0.020 |
| Min | 0.0120 | | 0.0160 | 0.0120 | | 0.0120 | 0.0120 | | 0.0160 |
| Max | 730 | | 2790 | 730 | | 2380 | 730 | | 1170 |
| n (Samp) | 435 | | 108 | 435 | | 119 | 435 | | 44 |
| n (Patient) | 173 | | 108 | 173 | | 119 | 173 | | 44 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.66 | 0.62 | 0.69 | 0.63 | 0.59 | 0.65 | 0.52 | 0.50 | 0.56 |
| SE | 0.029 | 0.048 | 0.030 | 0.029 | 0.045 | 0.030 | 0.045 | 0.058 | 0.047 |
| p | 2.7E-8 | 0.0095 | 1.5E-10 | 7.5E-6 | 0.035 | 1.1E-6 | 0.66 | 0.94 | 0.22 |
| nCohort 1 | 463 | 1019 | 435 | 463 | 1019 | 435 | 463 | 1019 | 435 |
| nCohort 2 | 120 | 40 | 108 | 130 | 46 | 119 | 47 | 26 | 44 |
| Cutoff 1 | 8.53 | 10.4 | 11.5 | 7.45 | 9.88 | 8.20 | 3.27 | 1.48 | 5.35 |
| Sens 1 | 70% | 70% | 70% | 70% | 72% | 71% | 70% | 73% | 70% |
| Spec 1 | 51% | 47% | 59% | 48% | 46% | 49% | 35% | 22% | 42% |
| Cutoff 2 | 4.36 | 0.405 | 6.04 | 3.71 | 3.71 | 4.18 | 0.405 | 0.0635 | 1.03 |
| Sens 2 | 80% | 80% | 81% | 80% | 80% | 81% | 81% | 81% | 82% |
| Spec 2 | 38% | 20% | 43% | 36% | 28% | 38% | 28% | 18% | 28% |
| Cutoff 3 | 0.0469 | 0.0344 | 0.0562 | 0.0552 | 0.0469 | 0.0562 | 0.0235 | 0.0277 | 0.0263 |
| Sens 3 | 91% | 90% | 91% | 90% | 91% | 91% | 94% | 92% | 91% |
| Spec 3 | 19% | 11% | 23% | 22% | 13% | 23% | 8% | 7% | 7% |
| Cutoff 4 | 16.0 | 23.8 | 16.7 | 16.0 | 23.8 | 16.7 | 16.0 | 23.8 | 16.7 |
| Sens 4 | 57% | 57% | 61% | 50% | 46% | 50% | 36% | 38% | 41% |
| Spec 4 | 70% | 70% | 70% | 70% | 70% | 70% | 70% | 70% | 70% |
| Cutoff 5 | 25.6 | 36.4 | 25.7 | 25.6 | 36.4 | 25.7 | 25.6 | 36.4 | 25.7 |
| Sens 5 | 46% | 40% | 50% | 35% | 28% | 39% | 15% | 23% | 20% |
| Spec 5 | 80% | 80% | 80% | 80% | 80% | 80% | 80% | 80% | 80% |
| Cutoff 6 | 40.7 | 62.3 | 48.2 | 40.7 | 62.3 | 48.2 | 40.7 | 62.3 | 48.2 |
| Sens 6 | 30% | 20% | 28% | 22% | 15% | 22% | 9% | 12% | 9% |
| Spec 6 | 90% | 90% | 90% | 90% | 90% | 90% | 90% | 90% | 90% |
| OR Quart 2 | 1.2 | 0.54 | 1.8 | 2.0 | 0.87 | 2.2 | 2.0 | 0.55 | 1.6 |
| p Value | 0.62 | 0.28 | 0.14 | 0.041 | 0.79 | 0.023 | 0.14 | 0.29 | 0.34 |
| 95% CI of | 0.60 | 0.18 | 0.83 | 1.0 | 0.31 | 1.1 | 0.81 | 0.18 | 0.60 |
| OR Quart2 | 2.3 | 1.6 | 3.8 | 3.7 | 2.4 | 4.5 | 4.8 | 1.7 | 4.3 |
| OR Quart 3 | 1.5 | 0.77 | 2.2 | 2.2 | 1.8 | 2.6 | 1.4 | 0.33 | 2.1 |
| p Value | 0.20 | 0.61 | 0.037 | 0.015 | 0.20 | 0.0068 | 0.48 | 0.096 | 0.12 |
| 95% CI of | 0.80 | 0.28 | 1.0 | 1.2 | 0.74 | 1.3 | 0.55 | 0.087 | 0.82 |
| OR Quart3 | 2.9 | 2.1 | 4.6 | 4.2 | 4.3 | 5.1 | 3.6 | 1.2 | 5.4 |
| OR Quart 4 | 4.3 | 2.2 | 6.3 | 3.9 | 2.2 | 4.4 | 1.7 | 1.0 | 1.8 |
| p Value | 1.9E-6 | 0.059 | 1.3E-7 | 1.2E-5 | 0.073 | 1.0E-5 | 0.27 | 0.99 | 0.24 |
| 95% CI of | 2.3 | 0.97 | 3.2 | 2.1 | 0.93 | 2.3 | 0.67 | 0.39 | 0.67 |
| OR Quart4 | 7.7 | 4.9 | 13 | 7.2 | 5.2 | 8.4 | 4.2 | 2.6 | 4.7 |

| | | | | | | | | | |
|---|---|---|---|---|---|---|---|---|---|
| **C-C motif chemokine 8** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 1.21 | | 1.72 | 1.21 | | 2.97 | 1.21 | | 2.77 |
| Average | 10.1 | | 9.75 | 10.1 | | 13.6 | 10.1 | | 10.8 |
| Stdev | 24.9 | | 21.6 | 24.9 | | 33.2 | 24.9 | | 34.3 |
| p(t-test) | | | 0.88 | | | 0.19 | | | 0.86 |
| Min | 0.0250 | | 0.0635 | 0.0250 | | 0.0250 | 0.0250 | | 0.0250 |
| Max | 250 | | 158 | 250 | | 294 | 250 | | 213 |
| n (Samp) | 463 | | 120 | 463 | | 130 | 463 | | 47 |
| n (Patient) | 223 | | 120 | 223 | | 130 | 223 | | 47 |
| | | | | | | | | | |
| sCr only | 0hr prior to AKI | | stage | 24hr prior to AKI | | stage | 48hr prior to AKI | | stage |
| | Cohort 1 | | Cohort 2 | Cohort 1 | | Cohort 2 | Cohort 1 | | Cohort 2 |
| Median | 1.36 | | 2.23 | 1.36 | | 6.03 | 1.36 | | 2.14 |
| Average | 10.6 | | 16.4 | 10.6 | | 20.7 | 10.6 | | 18.6 |
| Stdev | 32.3 | | 36.8 | 32.3 | | 46.5 | 32.3 | | 45.7 |

| sCr only | 0hr prior to AKI stage | | | 24hr prior to AKI stage | | | 48hr prior to AKI stage | | |
|---|---|---|---|---|---|---|---|---|---|
| | Cohort 1 | Cohort 2 | | Cohort 1 | Cohort 2 | | Cohort 1 | Cohort 2 | |
| p(t-test) | | 0.27 | | | 0.042 | | | 0.22 | |
| Min | 0.0250 | 0.0635 | | 0.0250 | 0.0250 | | 0.0250 | 0.0250 | |
| Max | 492 | 185 | | 492 | 279 | | 492 | 213 | |
| n (Samp) | 1019 | 40 | | 1019 | 46 | | 1019 | 26 | |
| n (Patient) | 375 | 40 | | 375 | 46 | | 375 | 26 | |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | Cohort 2 | | Cohort 1 | Cohort 2 | | Cohort 1 | Cohort 2 | |
| Median | 1.21 | 2.59 | | 1.21 | 2.77 | | 1.21 | 2.16 | |
| Average | 10.6 | 12.3 | | 10.6 | 16.1 | | 10.6 | 12.5 | |
| Stdev | 26.0 | 27.1 | | 26.0 | 36.2 | | 26.0 | 37.4 | |
| p(t-test) | | 0.54 | | | 0.066 | | | 0.67 | |
| Min | 0.0250 | 0.0701 | | 0.0250 | 0.0250 | | 0.0250 | 0.0472 | |
| Max | 250 | 176 | | 250 | 294 | | 250 | 214 | |
| n(Samp) | 435 | 108 | | 435 | 119 | | 435 | 44 | |
| n (Patient) | 173 | 108 | | 173 | 119 | | 173 | 44 | |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.57 | 0.58 | 0.59 | 0.60 | 0.62 | 0.60 | 0.56 | 0.55 | 0.53 |
| SE | 0.030 | 0.048 | 0.032 | 0.029 | 0.045 | 0.030 | 0.045 | 0.059 | 0.046 |
| p | 0.017 | 0.092 | 0.0041 | 9.0E-4 | 0.0090 | 8.1E-4 | 0.21 | 0.42 | 0.47 |
| nCohort 1 | 463 | 1019 | 435 | 463 | 1019 | 435 | 463 | 1019 | 435 |
| nCohort 2 | 120 | 40 | 108 | 130 | 46 | 119 | 47 | 26 | 44 |
| Cutoff 1 | 0.453 | 0.802 | 0.802 | 0.491 | 0.802 | 0.567 | 0.802 | 0.802 | 0.491 |
| Sens 1 | 72% | 72% | 73% | 73% | 74% | 71% | 74% | 73% | 70% |
| Spec 1 | 43% | 44% | 46% | 43% | 44% | 45% | 47% | 44% | 42% |
| Cutoff 2 | 0.154 | 0.240 | 0.239 | 0.239 | 0.239 | 0.240 | 0.239 | 0.136 | 0.154 |
| Sens 2 | 82% | 85% | 81% | 80% | 80% | 81% | 81% | 81% | 82% |
| Spec 2 | 33% | 35% | 33% | 36% | 32% | 36% | 36% | 25% | 29% |
| Cutoff 3 | 0.0945 | 0.127 | 0.127 | 0.0775 | 0.0775 | 0.127 | 0.0569 | 0.0705 | 0.0701 |
| Sens 3 | 90% | 90% | 91% | 92% | 93% | 91% | 94% | 92% | 91% |
| Spec 3 | 22% | 22% | 23% | 18% | 14% | 23% | 10% | 12% | 12% |
| Cutoff 4 | 5.70 | 6.40 | 5.77 | 5.70 | 6.40 | 5.77 | 5.70 | 6.40 | 5.77 |
| Sens 4 | 33% | 38% | 39% | 41% | 50% | 41% | 23% | 35% | 20% |
| Spec 4 | 70% | 70% | 70% | 70% | 70% | 70% | 70% | 70% | 70% |
| Cutoff 5 | 11.1 | 11.3 | 11.7 | 11.1 | 11.3 | 11.7 | 11.1 | 11.3 | 11.7 |
| Sens 5 | 19% | 22% | 24% | 25% | 35% | 24% | 13% | 19% | 14% |
| Spec 5 | 80% | 80% | 80% | 80% | 80% | 80% | 80% | 80% | 80% |
| Cutoff 6 | 28.3 | 24.5 | 25.3 | 28.3 | 24.5 | 25.3 | 28.3 | 24.5 | 25.3 |
| Sens 6 | 9% | 12% | 15% | 12% | 20% | 16% | 4% | 12% | 7% |
| Spec 6 | 90% | 90% | 90% | 90% | 90% | 90% | 90% | 90% | 90% |
| OR Quart 2 | 2.4 | 2.5 | 1.8 | 1.8 | 0.87 | 1.8 | 1.1 | 1.0 | 1.3 |
| p Value | 0.0066 | 0.096 | 0.074 | 0.061 | 0.79 | 0.075 | 0.80 | 1.0 | 0.62 |
| 95% CI of | 1.3 | 0.85 | 0.94 | 0.97 | 0.31 | 0.94 | 0.40 | 0.32 | 0.47 |
| OR Quart2 | 4.7 | 7.1 | 3.6 | 3.5 | 2.4 | 3.6 | 3.3 | 3.1 | 3.6 |
| OR Quart 3 | 3.4 | 2.5 | 2.7 | 3.0 | 1.5 | 3.1 | 4.0 | 1.3 | 3.2 |
| p Value | 1.6E-4 | 0.096 | 0.0028 | 4.7E-4 | 0.37 | 4.9E-4 | 0.0021 | 0.59 | 0.011 |
| 95% CI of | 1.8 | 0.85 | 1.4 | 1.6 | 0.61 | 1.6 | 1.7 | 0.46 | 1.3 |
| OR Quart3 | 6.3 | 7.1 | 5.1 | 5.4 | 3.8 | 5.9 | 9.7 | 3.9 | 7.9 |
| OR Quart 4 | 1.8 | 2.2 | 2.0 | 2.6 | 2.5 | 2.7 | 1.1 | 1.00 | 1.1 |
| p Value | 0.076 | 0.14 | 0.038 | 0.0027 | 0.036 | 0.0029 | 0.80 | 0.99 | 0.80 |
| 95% CI of | 0.94 | 0.77 | 1.0 | 1.4 | 1.1 | 1.4 | 0.40 | 0.32 | 0.40 |
| OR Quart4 | 3.6 | 6.5 | 3.9 | 4.7 | 5.7 | 5.1 | 3.3 | 3.1 | 3.3 |

| | | | | | | | | | |
|---|---|---|---|---|---|---|---|---|---|
| **Cathepsin D** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 65300 | | 94300 | 65300 | | 87400 | 65300 | | 69000 |
| Average | 69400 | | 98700 | 69400 | | 88000 | 69400 | | 77300 |
| Stdev | 35600 | | 44500 | 35600 | | 43900 | 35600 | | 42200 |
| p(t-test) | | | 1.3E-13 | | | 9.3E-7 | | | 0.16 |
| Min | 656 | | 2920 | 656 | | 3320 | 656 | | 4810 |
| Max | 200000 | | 200000 | 200000 | | 200000 | 200000 | | 200000 |
| n (Samp) | 463 | | 119 | 463 | | 128 | 463 | | 47 |
| n (Patient) | 223 | | 119 | 223 | | 128 | 223 | | 47 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 74500 | | 106000 | 74500 | | 85500 | 74500 | | 75700 |
| Average | 79700 | | 104000 | 79700 | | 94500 | 79700 | | 79600 |
| Stdev | 40600 | | 50300 | 40600 | | 50700 | 40600 | | 47400 |
| p(t-test) | | | 2.7E-4 | | | 0.017 | | | 0.99 |
| Min | 656 | | 2920 | 656 | | 4880 | 656 | | 5800 |
| Max | 200000 | | 200000 | 200000 | | 200000 | 200000 | | 200000 |
| n (Samp) | 1015 | | 40 | 1015 | | 46 | 1015 | | 26 |
| n (Patient) | 374 | | 40 | 374 | | 46 | 374 | | 26 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 70200 | | 99000 | 70200 | | 88700 | 70200 | | 80600 |
| Average | 75500 | | 103000 | 75500 | | 93900 | 75500 | | 83900 |
| Stdev | 38400 | | 43300 | 38400 | | 42600 | 38400 | | 41000 |
| p(t-test) | | | 1.6E-10 | | | 8.5E-6 | | | 0.17 |
| Min | 2520 | | 12600 | 2520 | | 3320 | 2520 | | 4810 |
| Max | 200000 | | 200000 | 200000 | | 200000 | 200000 | | 200000 |
| n (Samp) | 436 | | 107 | 436 | | 117 | 436 | | 44 |
| n (Patient) | 173 | | 107 | 173 | | 117 | 173 | | 44 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.70 | 0.65 | 0.69 | 0.63 | 0.59 | 0.63 | 0.55 | 0.48 | 0.57 |
| SE | 0.029 | 0.048 | 0.031 | 0.029 | 0.045 | 0.030 | 0.045 | 0.058 | 0.047 |
| p | 1.1E-11 | 0.0015 | 6.3E-10 | 6.6E-6 | 0.037 | 8.8E-6 | 0.23 | 0.78 | 0.14 |
| nCohort 1 | 463 | 1015 | 436 | 463 | 1015 | 436 | 463 | 1015 | 436 |
| nCohort 2 | 119 | 40 | 107 | 128 | 46 | 117 | 47 | 26 | 44 |
| Cutoff 1 | 72900 | 81200 | 74300 | 65000 | 70400 | 70900 | 56000 | 46800 | 61900 |
| Sens 1 | 71% | 70% | 70% | 70% | 72% | 70% | 70% | 73% | 70% |
| Spec 1 | 58% | 57% | 55% | 50% | 46% | 52% | 38% | 21% | 41% |
| Cutoff 2 | 62500 | 63200 | 69000 | 52000 | 58400 | 58800 | 45500 | 42000 | 54100 |
| Sens 2 | 81% | 80% | 80% | 80% | 80% | 80% | 81% | 81% | 82% |
| Spec 2 | 46% | 37% | 48% | 32% | 32% | 38% | 25% | 18% | 31% |
| Cutoff 3 | 43600 | 43100 | 54100 | 26100 | 19700 | 41500 | 22000 | 22000 | 39600 |
| Sens 3 | 91% | 90% | 91% | 91% | 91% | 91% | 91% | 92% | 91% |
| Spec 3 | 24% | 18% | 31% | 11% | 5% | 18% | 8% | 6% | 16% |
| Cutoff 4 | 82600 | 98000 | 90300 | 82600 | 98000 | 90300 | 82600 | 98000 | 90300 |
| Sens 4 | 60% | 52% | 55% | 55% | 41% | 48% | 40% | 31% | 41% |
| Spec 4 | 70% | 70% | 70% | 70% | 70% | 70% | 70% | 70% | 70% |
| Cutoff 5 | 99400 | 112000 | 108000 | 99400 | 112000 | 108000 | 99400 | 112000 | 108000 |
| Sens 5 | 48% | 40% | 43% | 36% | 37% | 37% | 23% | 15% | 20% |
| Spec 5 | 80% | 80% | 80% | 80% | 80% | 80% | 80% | 80% | 80% |
| Cutoff 6 | 121000 | 135000 | 126000 | 121000 | 135000 | 126000 | 121000 | 135000 | 126000 |
| Sens 6 | 29% | 25% | 27% | 21% | 20% | 22% | 13% | 8% | 18% |
| Spec 6 | 90% | 90% | 90% | 90% | 90% | 90% | 90% | 90% | 90% |
| OR Quart 2 | 1.1 | 0.56 | 2.3 | 1.0 | 1.0 | 1.2 | 0.99 | 0.62 | 2.0 |
| p Value | 0.72 | 0.36 | 0.042 | 0.89 | 1.0 | 0.60 | 0.99 | 0.41 | 0.17 |
| 95% CI of | 0.55 | 0.16 | 1.0 | 0.55 | 0.37 | 0.61 | 0.40 | 0.20 | 0.75 |
| OR Quart2 | 2.4 | 1.9 | 5.1 | 2.0 | 2.7 | 2.4 | 2.5 | 1.9 | 5.1 |
| OR Quart 3 | 2.4 | 1.3 | 3.4 | 1.9 | 1.7 | 2.0 | 1.2 | 0.62 | 1.6 |
| p Value | 0.012 | 0.62 | 0.0017 | 0.042 | 0.27 | 0.031 | 0.66 | 0.41 | 0.33 |
| 95% CI of | 1.2 | 0.47 | 1.6 | 1.0 | 0.68 | 1.1 | 0.51 | 0.20 | 0.61 |
| OR Quart3 | 4.6 | 3.5 | 7.3 | 3.4 | 4.1 | 3.8 | 2.9 | 1.9 | 4.4 |
| OR Quart 4 | 5.4 | 3.0 | 6.6 | 3.1 | 2.2 | 3.3 | 1.6 | 1.0 | 2.0 |
| p Value | 1.6E-7 | 0.014 | 4.8E-7 | 1.3E-4 | 0.073 | 1.2E-4 | 0.30 | 0.99 | 0.17 |
| 95% CI of | 2.9 | 1.2 | 3.2 | 1.7 | 0.93 | 1.8 | 0.67 | 0.37 | 0.75 |
| OR Quart4 | 10 | 7.2 | 14 | 5.4 | 5.2 | 6.1 | 3.6 | 2.7 | 5.1 |

| | | | | | | | | | |
|---|---|---|---|---|---|---|---|---|---|
| **C-X-C motif chemokine 13** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 0.0163 | | 1.10 | 0.0163 | | 0.590 | 0.0163 | | 0.448 |
| Average | 8.44 | | 7.15 | 8.44 | | 7.71 | 8.44 | | 6.34 |
| Stdev | 53.2 | | 23.6 | 53.2 | | 21.5 | 53.2 | | 16.8 |
| p(t-test) | | | 0.80 | | | 0.88 | | | 0.79 |
| Min | 0.00269 | | 0.00269 | 0.00269 | | 0.00269 | 0.00269 | | 0.00269 |
| Max | 832 | | 210 | 832 | | 131 | 832 | | 75.7 |
| n (Samp) | 463 | | 120 | 463 | | 130 | 463 | | 47 |
| n (Patient) | 223 | | 120 | 223 | | 130 | 223 | | 47 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 0.0222 | | 1.41 | 0.0222 | | 0.880 | 0.0222 | | 0.520 |
| Average | 7.21 | | 16.4 | 7.21 | | 8.42 | 7.21 | | 6.73 |
| Stdev | 40.5 | | 51.0 | 40.5 | | 20.0 | 40.5 | | 17.0 |
| p(t-test) | | | 0.16 | | | 0.84 | | | 0.95 |
| Min | 0.00269 | | 0.00471 | 0.00269 | | 0.00269 | 0.00269 | | 0.00269 |
| Max | 832 | | 252 | 832 | | 79.1 | 832 | | 77.1 |
| n (Samp) | 1019 | | 40 | 1019 | | 46 | 1019 | | 26 |
| n (Patient) | 375 | | 40 | 375 | | 46 | 375 | | 26 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 0.0163 | | 1.68 | 0.0163 | | 0.772 | 0.0163 | | 0.644 |
| Average | 8.43 | | 27.2 | 8.43 | | 26.4 | 8.43 | | 16.1 |
| Stdev | 54.5 | | 187 | 54.5 | | 171 | 54.5 | | 67.5 |
| p(t-test) | | | 0.072 | | | 0.062 | | | 0.39 |
| Min | 0.00269 | | 0.00269 | 0.00269 | | 0.00269 | 0.00269 | | 0.00474 |
| Max | 832 | | 1930 | 832 | | 1850 | 832 | | 440 |
| n (Samp) | 435 | | 108 | 435 | | 119 | 435 | | 44 |
| n (Patient) | 173 | | 108 | 173 | | 119 | 173 | | 44 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.64 | 0.63 | 0.68 | 0.58 | 0.58 | 0.61 | 0.57 | 0.54 | 0.61 |
| SE | 0.030 | 0.048 | 0.031 | 0.029 | 0.045 | 0.030 | 0.045 | 0.059 | 0.047 |
| p | 2.9E-6 | 0.0070 | 6.2E-9 | 0.0096 | 0.075 | 2.8E-4 | 0.14 | 0.50 | 0.020 |
| nCohort 1 | 463 | 1019 | 435 | 463 | 1019 | 435 | 463 | 1019 | 435 |
| nCohort 2 | 120 | 40 | 108 | 130 | 46 | 119 | 47 | 26 | 44 |
| Cutoff 1 | 0.0222 | 0.0193 | 0.392 | 0.0126 | 0.0126 | 0.0155 | 0.0151 | 0.0130 | 0.0174 |
| Sens 1 | 70% | 72% | 70% | 72% | 72% | 72% | 70% | 73% | 70% |
| Spec 1 | 55% | 47% | 62% | 37% | 32% | 48% | 44% | 34% | 50% |
| Cutoff 2 | 0.0126 | 0.0126 | 0.0193 | 0.00821 | 0.00821 | 0.00930 | 0.00821 | 0.0104 | 0.00930 |
| Sens 2 | 82% | 80% | 81% | 83% | 87% | 83% | 81% | 81% | 82% |
| Spec 2 | 37% | 32% | 51% | 26% | 21% | 27% | 26% | 27% | 27% |
| Cutoff 3 | 0.00821 | 0.0104 | 0.00821 | 0.00564 | 0.00789 | 0.00564 | 0.00471 | 0.00471 | 0.00789 |
| Sens 3 | 90% | 90% | 92% | 91% | 91% | 92% | 96% | 92% | 91% |
| Spec 3 | 26% | 27% | 22% | 17% | 18% | 14% | 10% | 8% | 20% |
| Cutoff 4 | 1.14 | 1.32 | 1.05 | 1.14 | 1.32 | 1.05 | 1.14 | 1.32 | 1.05 |
| Sens 4 | 49% | 50% | 58% | 36% | 43% | 44% | 34% | 38% | 41% |
| Spec 4 | 70% | 70% | 70% | 70% | 70% | 70% | 70% | 70% | 70% |
| Cutoff 5 | 3.19 | 3.33 | 2.67 | 3.19 | 3.33 | 2.67 | 3.19 | 3.33 | 2.67 |
| Sens 5 | 28% | 35% | 38% | 22% | 28% | 29% | 19% | 19% | 27% |
| Spec 5 | 80% | 80% | 80% | 80% | 80% | 80% | 80% | 80% | 80% |
| Cutoff 6 | 10.2 | 10.2 | 8.59 | 10.2 | 10.2 | 8.59 | 10.2 | 10.2 | 8.59 |
| Sens 6 | 13% | 20% | 18% | 14% | 15% | 22% | 13% | 15% | 16% |
| Spec 6 | 90% | 90% | 90% | 90% | 90% | 90% | 90% | 90% | 90% |
| OR Quart 2 | 1.7 | 2.3 | 1.1 | 1.3 | 0.63 | 1.0 | 1.3 | 1.2 | 0.99 |
| p Value | 0.16 | 0.17 | 0.86 | 0.44 | 0.34 | 0.89 | 0.64 | 0.76 | 0.99 |
| 95% CI of | 0.81 | 0.69 | 0.49 | 0.69 | 0.24 | 0.54 | 0.48 | 0.36 | 0.34 |
| OR Quart2 | 3.4 | 7.5 | 2.4 | 2.3 | 1.6 | 2.0 | 3.3 | 4.0 | 2.9 |
| OR Quart 3 | 3.8 | 2.5 | 3.5 | 2.2 | 1.2 | 2.3 | 2.1 | 1.8 | 2.1 |
| p Value | 7.4E-5 | 0.12 | 3.3E-4 | 0.0084 | 0.68 | 0.0060 | 0.092 | 0.29 | 0.12 |
| 95% CI of | 2.0 | 0.79 | 1.8 | 1.2 | 0.52 | 1.3 | 0.88 | 0.60 | 0.82 |
| OR Quart3 | 7.3 | 8.2 | 7.0 | 3.8 | 2.7 | 4.2 | 5.2 | 5.5 | 5.4 |
| OR Quart 4 | 3.8 | 4.5 | 4.5 | 1.8 | 1.4 | 2.3 | 1.7 | 1.2 | 2.5 |
| p Value | 7.4E-5 | 0.0079 | 1.3E-5 | 0.048 | 0.43 | 0.0066 | 0.27 | 0.77 | 0.057 |
| 95% CI of | 2.0 | 1.5 | 2.3 | 1.0 | 0.62 | 1.3 | 0.67 | 0.36 | 0.97 |
| OR Quart4 | 7.3 | 13 | 8.8 | 3.2 | 3.1 | 4.2 | 4.2 | 4.0 | 6.2 |

| | | | | | | | | | |
|---|---|---|---|---|---|---|---|---|---|
| **Insulin-like growth factor-binding protein 3** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 298 | | 508 | 298 | | 491 | 298 | | 620 |
| Average | 559 | | 748 | 559 | | 1030 | 559 | | 733 |
| Stdev | 935 | | 758 | 935 | | 1970 | 935 | | 684 |
| p(t-test) | | | 0.027 | | | 6.9E-5 | | | 0.21 |
| Min | 3.84 | | 5.67 | 3.84 | | 8.21 | 3.84 | | 3.83 |
| Max | 12500 | | 4890 | 12500 | | 12500 | 12500 | | 3220 |
| n (Samp) | 499 | | 142 | 499 | | 141 | 499 | | 48 |
| n (Patient) | 235 | | 142 | 235 | | 141 | 235 | | 48 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 388 | | 432 | 388 | | 376 | 388 | | 423 |
| Average | 665 | | 827 | 665 | | 917 | 665 | | 635 |
| Stdev | 1060 | | 1290 | 1060 | | 1400 | 1060 | | 583 |
| p(t-test) | | | 0.32 | | | 0.10 | | | 0.88 |
| Min | 0.0478 | | 5.67 | 0.0478 | | 14.5 | 0.0478 | | 3.83 |
| Max | 12500 | | 7720 | 12500 | | 8160 | 12500 | | 1890 |
| n (Samp) | 1097 | | 46 | 1097 | | 51 | 1097 | | 28 |
| n (Patient) | 397 | | 46 | 397 | | 51 | 397 | | 28 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 311 | | 599 | 311 | | 590 | 311 | | 699 |
| Average | 617 | | 1010 | 617 | | 1130 | 617 | | 771 |
| Stdev | 1150 | | 1400 | 1150 | | 2020 | 1150 | | 659 |
| p(t-test) | | | 0.0012 | | | 1.9E-4 | | | 0.37 |
| Min | 3.84 | | 21.2 | 3.84 | | 8.21 | 3.84 | | 10.2 |
| Max | 12500 | | 12500 | 12500 | | 12500 | 12500 | | 3220 |
| n (Samp) | 487 | | 129 | 487 | | 131 | 487 | | 47 |
| n (Patient) | 190 | | 129 | 190 | | 131 | 190 | | 47 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.63 | 0.52 | 0.67 | 0.60 | 0.53 | 0.63 | 0.62 | 0.52 | 0.64 |
| SE | 0.028 | 0.044 | 0.028 | 0.028 | 0.042 | 0.029 | 0.045 | 0.056 | 0.045 |
| p | 5.3E-6 | 0.58 | 1.7E-9 | 3.0E-4 | 0.43 | 3.4E-6 | 0.0095 | 0.68 | 0.0020 |
| nCohort 1 | 499 | 1097 | 487 | 499 | 1097 | 487 | 499 | 1097 | 487 |
| nCohort 2 | 142 | 46 | 129 | 141 | 51 | 131 | 48 | 28 | 47 |
| Cutoff 1 | 275 | 205 | 349 | 225 | 202 | 314 | 219 | 174 | 369 |
| Sens 1 | 70% | 72% | 71% | 70% | 71% | 70% | 71% | 71% | 70% |
| Spec 1 | 48% | 32% | 54% | 42% | 32% | 51% | 41% | 28% | 56% |
| Cutoff 2 | 191 | 136 | 257 | 116 | 163 | 176 | 145 | 110 | 191 |
| Sens 2 | 80% | 80% | 81% | 80% | 80% | 80% | 81% | 82% | 81% |
| Spec 2 | 39% | 23% | 45% | 28% | 26% | 34% | 32% | 20% | 36% |
| Cutoff 3 | 105 | 27.5 | 141 | 46.2 | 43.4 | 80.9 | 51.9 | 43.4 | 77.9 |
| Sens 3 | 90% | 91% | 91% | 90% | 90% | 90% | 92% | 93% | 91% |
| Spec 3 | 27% | 5% | 30% | 11% | 7% | 19% | 14% | 7% | 19% |
| Cutoff 4 | 585 | 710 | 587 | 585 | 710 | 587 | 585 | 710 | 587 |
| Sens 4 | 43% | 35% | 51% | 44% | 41% | 50% | 52% | 36% | 55% |
| Spec 4 | 70% | 70% | 70% | 70% | 70% | 70% | 70% | 70% | 70% |
| Cutoff 5 | 829 | 997 | 859 | 829 | 997 | 859 | 829 | 997 | 859 |
| Sens 5 | 34% | 26% | 40% | 36% | 27% | 40% | 35% | 29% | 32% |
| Spec 5 | 80% | 80% | 80% | 80% | 80% | 80% | 80% | 80% | 80% |
| Cutoff 6 | 1260 | 1420 | 1320 | 1260 | 1420 | 1320 | 1260 | 1420 | 1320 |
| Sens 6 | 20% | 17% | 22% | 21% | 18% | 21% | 19% | 14% | 15% |
| Spec 6 | 90% | 90% | 90% | 90% | 90% | 90% | 90% | 90% | 90% |
| OR Quart 2 | 1.8 | 1.00 | 2.6 | 1.1 | 1.6 | 0.89 | 1.5 | 1.0 | 1.6 |
| p Value | 0.067 | 0.99 | 0.0083 | 0.77 | 0.23 | 0.73 | 0.46 | 1.0 | 0.41 |
| 95% CI of | 0.96 | 0.42 | 1.3 | 0.61 | 0.73 | 0.47 | 0.54 | 0.35 | 0.52 |
| OR Quart2 | 3.3 | 2.3 | 5.4 | 2.0 | 3.7 | 1.7 | 3.9 | 2.9 | 5.1 |
| OR Quart 3 | 2.5 | 0.90 | 3.6 | 1.4 | 0.69 | 1.6 | 1.5 | 0.85 | 2.5 |
| p Value | 0.0030 | 0.82 | 3.1E-4 | 0.20 | 0.46 | 0.14 | 0.46 | 0.78 | 0.089 |
| 95% CI of | 1.4 | 0.38 | 1.8 | 0.82 | 0.26 | 0.86 | 0.54 | 0.28 | 0.87 |
| OR Quart3 | 4.5 | 2.2 | 7.3 | 2.5 | 1.8 | 2.8 | 3.9 | 2.6 | 7.4 |
| OR Quart 4 | 3.5 | 1.3 | 6.2 | 2.5 | 1.9 | 3.0 | 3.3 | 1.1 | 5.0 |
| p Value | 2.1E-5 | 0.55 | 1.2E-7 | 8.6E-4 | 0.13 | 7.9E-5 | 0.0081 | 0.80 | 0.0016 |
| 95% CI of | 2.0 | 0.57 | 3.2 | 1.5 | 0.84 | 1.8 | 1.4 | 0.41 | 1.8 |
| OR Quart4 | 6.3 | 2.9 | 12 | 4.2 | 4.1 | 5.3 | 8.1 | 3.2 | 14 |

| | | | | | | | | | |
|---|---|---|---|---|---|---|---|---|---|
| **Immunoglogulin G1** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 2600 | | 4230 | 2600 | | 4600 | 2600 | | 3720 |
| Average | 5310 | | 6310 | 5310 | | 7320 | 5310 | | 5200 |
| Stdev | 7650 | | 5510 | 7650 | | 9110 | 7650 | | 4150 |
| p(t-test) | | | 0.18 | | | 0.013 | | | 0.92 |
| Min | 59.7 | | 342 | 59.7 | | 138 | 59.7 | | 177 |
| Max | 80000 | | 28400 | 80000 | | 80000 | 80000 | | 16300 |
| n (Samp) | 461 | | 119 | 461 | | 126 | 461 | | 47 |
| n (Patient) | 222 | | 119 | 222 | | 126 | 222 | | 47 |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 3180 | | 5010 | 3180 | | 6800 | 3180 | | 4830 |
| Average | 5750 | | 7340 | 5750 | | 8240 | 5750 | | 6160 |
| Stdev | 7310 | | 6940 | 7310 | | 7030 | 7310 | | 4600 |
| p(t-test) | | | 0.18 | | | 0.024 | | | 0.78 |
| Min | 3.36 | | 342 | 3.36 | | 388 | 3.36 | | 177 |
| Max | 80000 | | 28400 | 80000 | | 25600 | 80000 | | 14900 |
| n (Samp) | 1011 | | 40 | 1011 | | 46 | 1011 | | 26 |
| n (Patient) | 373 | | 40 | 373 | | 46 | 373 | | 26 |
| | | | | | | | | | |

| UO only | 0hr prior to AKI | | stage | 24hr prior to AKI | | stage | 48hr prior to AKI | | stage |
|---|---|---|---|---|---|---|---|---|---|
| | Cohort 1 | | Cohort 2 | Cohort 1 | | Cohort 2 | Cohort 1 | | Cohort 2 |
| Median | 2690 | | 5000 | 2690 | | 5650 | 2690 | | 3900 |
| Average | 5520 | | 7480 | 5520 | | 8340 | 5520 | | 6110 |
| Stdev | 7740 | | 6370 | 7740 | | 9920 | 7740 | | 5690 |
| p(t-test) | | | 0.016 | | | 0.0012 | | | 0.62 |
| Min | 59.7 | | 663 | 59.7 | | 138 | 59.7 | | 550 |
| Max | 80000 | | 35700 | 80000 | | 80000 | 80000 | | 25300 |
| n (Samp) | 433 | | 107 | 433 | | 115 | 433 | | 44 |
| n (Patient) | 171 | | 107 | 171 | | 115 | 171 | | 44 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.63 | 0.60 | 0.66 | 0.62 | 0.61 | 0.64 | 0.59 | 0.57 | 0.62 |
| SE | 0.030 | 0.048 | 0.031 | 0.029 | 0.045 | 0.030 | 0.045 | 0.059 | 0.047 |
| p | 1.2E-5 | 0.033 | 2.2E-7 | 7.0E-5 | 0.017 | 2.8E-6 | 0.053 | 0.23 | 0.014 |
| nCohort 1 | 461 | 1011 | 433 | 461 | 1011 | 433 | 461 | 1011 | 433 |
| nCohort 2 | 119 | 40 | 107 | 126 | 46 | 115 | 47 | 26 | 44 |
| Cutoff 1 | 2770 | 3070 | 3090 | 2420 | 2620 | 3070 | 2500 | 2440 | 2880 |
| Sens 1 | 71% | 70% | 70% | 71% | 72% | 70% | 70% | 73% | 70% |
| Spec 1 | 52% | 49% | 55% | 46% | 42% | 55% | 48% | 39% | 52% |
| Cutoff 2 | 2010 | 2840 | 2510 | 1690 | 1550 | 1990 | 1690 | 1690 | 2370 |
| Sens 2 | 81% | 80% | 80% | 80% | 80% | 80% | 81% | 81% | 82% |
| Spec 2 | 39% | 45% | 46% | 31% | 23% | 38% | 31% | 26% | 44% |
| Cutoff 3 | 1390 | 1090 | 1600 | 924 | 911 | 1280 | 1080 | 831 | 1670 |
| Sens 3 | 91% | 90% | 91% | 90% | 91% | 90% | 91% | 92% | 91% |
| Spec 3 | 25% | 14% | 29% | 16% | 12% | 21% | 18% | 10% | 30% |
| Cutoff 4 | 4740 | 5870 | 4990 | 4740 | 5870 | 4990 | 4740 | 5870 | 4990 |
| Sens 4 | 45% | 32% | 50% | 49% | 52% | 53% | 36% | 46% | 36% |
| Spec 4 | 70% | 70% | 70% | 70% | 70% | 70% | 70% | 70% | 70% |
| Cutoff 5 | 7460 | 8580 | 7650 | 7460 | 8580 | 7650 | 7460 | 8580 | 7650 |
| Sens 5 | 29% | 30% | 38% | 33% | 41% | 38% | 28% | 35% | 27% |
| Spec 5 | 80% | 80% | 80% | 80% | 80% | 80% | 80% | 80% | 80% |
| Cutoff 6 | 13400 | 13900 | 14200 | 13400 | 13900 | 14200 | 13400 | 13900 | 14200 |
| Sens 6 | 12% | 18% | 12% | 16% | 22% | 17% | 6% | 4% | 11% |
| Spec 6 | 90% | 90% | 90% | 90% | 90% | 90% | 90% | 90% | 90% |
| OR Quart 2 | 1.9 | 1.3 | 2.0 | 0.94 | 0.69 | 1.4 | 2.5 | 0.80 | 6.0 |
| p Value | 0.085 | 0.59 | 0.090 | 0.85 | 0.46 | 0.37 | 0.088 | 0.74 | 0.022 |
| 95% CI of | 0.92 | 0.46 | 0.90 | 0.48 | 0.26 | 0.68 | 0.87 | 0.21 | 1.3 |
| OR Quart2 | 3.8 | 3.9 | 4.3 | 1.8 | 1.8 | 2.8 | 7.5 | 3.0 | 27 |
| OR Quart 3 | 3.6 | 2.4 | 3.8 | 2.2 | 0.69 | 2.9 | 3.0 | 1.0 | 10 |
| p Value | 1.6E-4 | 0.078 | 3.4E-4 | 0.0077 | 0.46 | 0.0015 | 0.039 | 1.0 | 0.0020 |
| 95% CI of | 1.8 | 0.91 | 1.8 | 1.2 | 0.26 | 1.5 | 1.1 | 0.29 | 2.4 |
| OR Quart3 | 6.9 | 6.3 | 7.9 | 4.0 | 1.8 | 5.6 | 8.7 | 3.5 | 46 |
| OR Quart 4 | 3.7 | 2.0 | 5.1 | 2.6 | 2.3 | 3.8 | 3.5 | 2.5 | 7.1 |
| p Value | 1.0E-4 | 0.16 | 8.4E-6 | 0.0011 | 0.034 | 4.2E-5 | 0.017 | 0.096 | 0.011 |
| 95% CI of | 1.9 | 0.75 | 2.5 | 1.5 | 1.1 | 2.0 | 1.2 | 0.85 | 1.6 |
| OR Quart4 | 7.1 | 5.5 | 10 | 4.7 | 5.0 | 7.3 | 9.9 | 7.1 | 32 |

| | | | | | | | | | |
|---|---|---|---|---|---|---|---|---|---|
| **Immunoglogulin G2** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 7990 | | 11000 | 7990 | | 12100 | 7990 | | 9560 |
| Average | 16000 | | 20900 | 16000 | | 26500 | 16000 | | 16600 |
| Stdev | 26200 | | 31200 | 26200 | | 45400 | 26200 | | 26800 |
| p(t-test) | | | 0.077 | | | 8.8E-4 | | | 0.87 |
| Min | 119 | | 819 | 119 | | 640 | 119 | | 1050 |
| Max | 240000 | | 240000 | 240000 | | 240000 | 240000 | | 172000 |
| n (Samp) | 461 | | 119 | 461 | | 126 | 461 | | 47 |
| n (Patient) | 222 | | 119 | 222 | | 126 | 222 | | 47 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 9050 | | 11500 | 9050 | | 13400 | 9050 | | 9970 |
| Average | 17700 | | 24000 | 17700 | | 35800 | 17700 | | 22000 |
| Stdev | 29600 | | 43100 | 29600 | | 55000 | 29600 | | 34000 |
| p(t-test) | | | 0.19 | | | 1.1E-4 | | | 0.46 |
| Min | 119 | | 819 | 119 | | 1070 | 119 | | 1310 |
| Max | 240000 | | 240000 | 240000 | | 240000 | 240000 | | 172000 |
| n (Samp) | 1011 | | 40 | 1011 | | 46 | 1011 | | 26 |
| n (Patient) | 373 | | 40 | 373 | | 46 | 373 | | 26 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 8540 | | 13200 | 8540 | | 13500 | 8540 | | 10800 |
| Average | 17400 | | 26300 | 17400 | | 29800 | 17400 | | 21300 |
| Stdev | 29900 | | 38600 | 29900 | | 50500 | 29900 | | 38200 |
| p(t-test) | | | 0.010 | | | 8.8E-4 | | | 0.43 |
| Min | 334 | | 2930 | 334 | | 640 | 334 | | 1050 |
| Max | 240000 | | 240000 | 240000 | | 240000 | 240000 | | 240000 |
| n (Samp) | 433 | | 107 | 433 | | 115 | 433 | | 44 |
| n (Patient) | 171 | | 107 | 171 | | 115 | 171 | | 44 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.60 | 0.55 | 0.63 | 0.59 | 0.58 | 0.61 | 0.54 | 0.56 | 0.56 |
| SE | 0.030 | 0.048 | 0.031 | 0.029 | 0.045 | 0.031 | 0.045 | 0.059 | 0.047 |
| p | 0.0016 | 0.26 | 2.4E-5 | 0.0022 | 0.064 | 4.8E-4 | 0.40 | 0.30 | 0.21 |
| nCohort 1 | 461 | 1011 | 433 | 461 | 1011 | 433 | 461 | 1011 | 433 |
| nCohort 2 | 119 | 40 | 107 | 126 | 46 | 115 | 47 | 26 | 44 |
| Cutoff 1 | 7640 | 7760 | 8630 | 6690 | 5500 | 7670 | 6460 | 6690 | 7030 |
| Sens 1 | 71% | 70% | 70% | 71% | 72% | 70% | 70% | 73% | 70% |
| Spec 1 | 48% | 43% | 51% | 42% | 29% | 45% | 41% | 37% | 42% |
| Cutoff 2 | 5300 | 6310 | 6460 | 4860 | 4630 | 5930 | 4240 | 6520 | 4680 |
| Sens 2 | 81% | 80% | 80% | 80% | 80% | 80% | 81% | 81% | 82% |
| Spec 2 | 33% | 34% | 38% | 29% | 23% | 33% | 25% | 36% | 24% |
| Cutoff 3 | 3890 | 2220 | 4750 | 2560 | 3170 | 3280 | 2800 | 3420 | 3060 |
| Sens 3 | 91% | 90% | 91% | 90% | 91% | 91% | 91% | 92% | 91% |
| Spec 3 | 21% | 9% | 24% | 12% | 13% | 14% | 14% | 15% | 13% |
| Cutoff 4 | 14400 | 15000 | 14500 | 14400 | 15000 | 14500 | 14400 | 15000 | 14500 |
| Sens 4 | 36% | 30% | 44% | 43% | 43% | 45% | 34% | 42% | 36% |
| Spec 4 | 70% | 70% | 70% | 70% | 70% | 70% | 70% | 70% | 70% |
| Cutoff 5 | 19800 | 21300 | 19800 | 19800 | 21300 | 19800 | 19800 | 21300 | 19800 |
| Sens 5 | 25% | 25% | 33% | 31% | 37% | 33% | 21% | 27% | 23% |
| Spec 5 | 80% | 80% | 80% | 80% | 80% | 80% | 80% | 80% | 80% |
| Cutoff 6 | 32800 | 36600 | 35300 | 32800 | 36600 | 35300 | 32800 | 36600 | 35300 |
| Sens 6 | 16% | 15% | 20% | 18% | 26% | 17% | 9% | 12% | 11% |
| Spec 6 | 90% | 90% | 90% | 90% | 90% | 90% | 90% | 90% | 90% |
| OR Quart 2 | 1.4 | 0.87 | 1.7 | 1.1 | 0.63 | 1.3 | 0.89 | 1.4 | 0.88 |
| p Value | 0.32 | 0.79 | 0.16 | 0.77 | 0.34 | 0.41 | 0.81 | 0.56 | 0.80 |
| 95% CI of | 0.72 | 0.31 | 0.82 | 0.59 | 0.24 | 0.69 | 0.35 | 0.44 | 0.33 |
| OR Quart2 | 2.7 | 2.4 | 3.4 | 2.0 | 1.6 | 2.5 | 2.3 | 4.5 | 2.4 |
| OR Quart 3 | 3.0 | 1.8 | 2.9 | 1.7 | 1.0 | 1.9 | 1.7 | 1.0 | 1.6 |
| p Value | 4.5E-4 | 0.20 | 0.0020 | 0.064 | 1.0 | 0.046 | 0.22 | 1.0 | 0.28 |
| 95% CI of | 1.6 | 0.74 | 1.5 | 0.97 | 0.43 | 1.0 | 0.73 | 0.29 | 0.68 |
| OR Quart3 | 5.5 | 4.3 | 5.7 | 3.1 | 2.3 | 3.5 | 3.9 | 3.5 | 3.9 |
| OR Quart 4 | 2.2 | 1.4 | 3.3 | 2.1 | 1.6 | 2.6 | 1.2 | 1.8 | 1.5 |
| p Value | 0.016 | 0.49 | 5.4E-4 | 0.0091 | 0.25 | 0.0025 | 0.66 | 0.29 | 0.38 |
| 95% CI of | 1.2 | 0.55 | 1.7 | 1.2 | 0.72 | 1.4 | 0.51 | 0.60 | 0.61 |
| OR Quart4 | 4.0 | 3.5 | 6.4 | 3.8 | 3.4 | 4.7 | 2.9 | 5.5 | 3.6 |

| | | | | | | | | | |
|---|---|---|---|---|---|---|---|---|---|
| **Interleukin-11** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 109 | | 223 | 109 | | 165 | 109 | | 120 |
| Average | 186 | | 304 | 186 | | 233 | 186 | | 193 |
| Stdev | 225 | | 268 | 225 | | 205 | 225 | | 328 |
| p(t-test) | | | 1.2E-6 | | | 0.033 | | | 0.83 |
| Min | 0.154 | | 13.2 | 0.154 | | 7.35 | 0.154 | | 0.0822 |
| Max | 1980 | | 1780 | 1980 | | 1050 | 1980 | | 2260 |
| n (Samp) | 461 | | 119 | 461 | | 129 | 461 | | 47 |
| n (Patient) | 223 | | 119 | 223 | | 129 | 223 | | 47 |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 131 | | 226 | 131 | | 202 | 131 | | 124 |
| Average | 216 | | 253 | 216 | | 240 | 216 | | 167 |
| Stdev | 249 | | 173 | 249 | | 191 | 249 | | 139 |
| p(t-test) | | | 0.35 | | | 0.52 | | | 0.32 |
| Min | 0.0822 | | 13.2 | 0.0822 | | 7.35 | 0.0822 | | 0.0822 |
| Max | 2260 | | 659 | 2260 | | 996 | 2260 | | 558 |
| n (Samp) | 1017 | | 40 | 1017 | | 46 | 1017 | | 26 |
| n (Patient) | 375 | | 40 | 375 | | 46 | 375 | | 26 |
| | | | | | | | | | |

| UO only | 0hr prior to AKI | | stage | 24hr prior to AKI | | stage | 48hr prior to AKI | | stage |
|---|---|---|---|---|---|---|---|---|---|
| | Cohort 1 | | Cohort 2 | Cohort 1 | | Cohort 2 | Cohort 1 | | Cohort 2 |
| Median | 131 | | 232 | 131 | | 169 | 131 | | 138 |
| Average | 206 | | 349 | 206 | | 264 | 206 | | 244 |
| Stdev | 234 | | 381 | 234 | | 315 | 234 | | 448 |
| p(t-test) | | | 1.1E-6 | | | 0.026 | | | 0.35 |
| Min | 2.83 | | 41.8 | 2.83 | | 8.36 | 2.83 | | 14.5 |
| Max | 1980 | | 2900 | 1980 | | 2590 | 1980 | | 2260 |
| n (Samp) | 434 | | 107 | 434 | | 118 | 434 | | 44 |
| n (Patient) | 173 | | 107 | 173 | | 118 | 173 | | 44 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.70 | 0.61 | 0.69 | 0.62 | 0.58 | 0.59 | 0.52 | 0.47 | 0.48 |
| SE | 0.029 | 0.048 | 0.031 | 0.029 | 0.045 | 0.030 | 0.045 | 0.058 | 0.046 |
| p | 6.9E-12 | 0.029 | 1.2E-9 | 7.4E-5 | 0.072 | 0.0033 | 0.69 | 0.62 | 0.68 |
| nCohort 1 | 461 | 1017 | 434 | 461 | 1017 | 434 | 461 | 1017 | 434 |
| nCohort 2 | 119 | 40 | 107 | 129 | 46 | 118 | 47 | 26 | 44 |
| Cutoff 1 | 144 | 111 | 152 | 106 | 105 | 111 | 79.8 | 66.2 | 79.8 |
| Sens 1 | 71% | 70% | 70% | 71% | 72% | 70% | 70% | 73% | 70% |
| Spec 1 | 61% | 43% | 56% | 48% | 40% | 44% | 36% | 23% | 31% |
| Cutoff 2 | 107 | 78.7 | 121 | 76.0 | 76.5 | 85.7 | 66.1 | 57.1 | 66.1 |
| Sens 2 | 81% | 80% | 80% | 81% | 80% | 81% | 81% | 81% | 82% |
| Spec 2 | 49% | 29% | 47% | 34% | 28% | 32% | 30% | 19% | 24% |
| Cutoff 3 | 73.6 | 41.6 | 87.1 | 55.8 | 40.7 | 66.2 | 53.9 | 41.4 | 31.8 |
| Sens 3 | 91% | 90% | 91% | 91% | 91% | 91% | 91% | 92% | 91% |
| Spec 3 | 33% | 10% | 33% | 25% | 10% | 24% | 23% | 10% | 6% |
| Cutoff 4 | 189 | 218 | 217 | 189 | 218 | 217 | 189 | 218 | 217 |
| Sens 4 | 58% | 55% | 52% | 43% | 46% | 37% | 21% | 27% | 23% |
| Spec 4 | 70% | 70% | 70% | 70% | 70% | 70% | 70% | 70% | 70% |
| Cutoff 5 | 254 | 308 | 289 | 254 | 308 | 289 | 254 | 308 | 289 |
| Sens 5 | 46% | 35% | 42% | 31% | 30% | 28% | 17% | 15% | 18% |
| Spec 5 | 80% | 80% | 80% | 80% | 80% | 80% | 80% | 80% | 80% |
| Cutoff 6 | 399 | 503 | 414 | 399 | 503 | 414 | 399 | 503 | 414 |
| Sens 6 | 27% | 8% | 28% | 15% | 7% | 15% | 6% | 4% | 7% |
| Spec 6 | 90% | 90% | 90% | 90% | 90% | 90% | 90% | 90% | 90% |
| OR Quart 2 | 2.7 | 1.6 | 2.6 | 2.3 | 1.6 | 1.8 | 2.5 | 1.6 | 1.6 |
| p Value | 0.017 | 0.40 | 0.024 | 0.013 | 0.35 | 0.081 | 0.055 | 0.40 | 0.27 |
| 95% CI of | 1.2 | 0.52 | 1.1 | 1.2 | 0.61 | 0.93 | 0.98 | 0.52 | 0.68 |
| OR Quart2 | 6.1 | 5.0 | 5.9 | 4.5 | 4.2 | 3.4 | 6.2 | 5.0 | 4.0 |
| OR Quart 3 | 4.6 | 1.8 | 4.2 | 3.3 | 1.3 | 2.3 | 2.1 | 1.0 | 1.4 |
| p Value | 1.1E-4 | 0.29 | 3.7E-4 | 3.0E-4 | 0.62 | 0.011 | 0.12 | 1.0 | 0.49 |
| 95% CI of | 2.1 | 0.60 | 1.9 | 1.7 | 0.47 | 1.2 | 0.83 | 0.29 | 0.55 |
| OR Quart3 | 10 | 5.5 | 9.2 | 6.3 | 3.5 | 4.2 | 5.5 | 3.5 | 3.4 |
| OR Quart 4 | 9.0 | 3.8 | 7.2 | 3.6 | 2.8 | 2.4 | 1.5 | 1.6 | 1.0 |
| p Value | 1.2E-8 | 0.0097 | 4.4E-7 | 8.9E-5 | 0.021 | 0.0072 | 0.45 | 0.40 | 0.99 |
| 95% CI of | 4.2 | 1.4 | 3.3 | 1.9 | 1.2 | 1.3 | 0.54 | 0.52 | 0.39 |
| OR Quart4 | 19 | 10 | 15 | 6.8 | 6.9 | 4.4 | 4.0 | 5.0 | 2.6 |

| | | | | | | | | | |
|---|---|---|---|---|---|---|---|---|---|
| **Interleukin-2 receptor alpha chain** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 434 | | 788 | 434 | | 572 | 434 | | 526 |
| Average | 833 | | 1150 | 833 | | 1190 | 833 | | 986 |
| Stdev | 1060 | | 1220 | 1060 | | 1640 | 1060 | | 1440 |
| p(t-test) | | | 0.0055 | | | 0.0028 | | | 0.37 |
| Min | 0.0317 | | 0.0339 | 0.0317 | | 0.0339 | 0.0317 | | 0.120 |
| Max | 10400 | | 6080 | 10400 | | 9090 | 10400 | | 7740 |
| n (Samp) | 462 | | 120 | 462 | | 130 | 462 | | 47 |
| n (Patient) | 223 | | 120 | 223 | | 130 | 223 | | 47 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 622 | | 431 | 622 | | 570 | 622 | | 515 |
| Average | 1060 | | 1110 | 1060 | | 1190 | 1060 | | 1020 |
| Stdev | 1380 | | 1720 | 1380 | | 1550 | 1380 | | 1450 |
| p(t-test) | | | 0.82 | | | 0.56 | | | 0.87 |
| Min | 0.0317 | | 0.0339 | 0.0317 | | 0.0365 | 0.0317 | | 0.141 |
| Max | 10400 | | 7330 | 10400 | | 7190 | 10400 | | 5640 |
| n (Samp) | 1019 | | 40 | 1019 | | 46 | 1019 | | 26 |
| n (Patient) | 375 | | 40 | 375 | | 46 | 375 | | 26 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 454 | | 942 | 454 | | 734 | 454 | | 533 |
| Average | 840 | | 1310 | 840 | | 1330 | 840 | | 981 |
| Stdev | 1070 | | 1260 | 1070 | | 1690 | 1070 | | 1430 |
| p(t-test) | | | 8.8E-5 | | | 1.3E-4 | | | 0.42 |
| Min | 0.0317 | | 0.0339 | 0.0317 | | 0.0339 | 0.0317 | | 0.120 |
| Max | 10400 | | 6080 | 10400 | | 9090 | 10400 | | 7740 |
| n (Samp) | 436 | | 108 | 436 | | 119 | 436 | | 44 |
| n (Patient) | 173 | | 108 | 173 | | 119 | 173 | | 44 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.60 | 0.44 | 0.65 | 0.54 | 0.50 | 0.58 | 0.52 | 0.47 | 0.52 |
| SE | 0.030 | 0.048 | 0.031 | 0.029 | 0.044 | 0.030 | 0.045 | 0.058 | 0.046 |
| p | 9.8E-4 | 0.19 | 1.9E-6 | 0.14 | 0.93 | 0.0089 | 0.64 | 0.58 | 0.66 |
| nCohort 1 | 462 | 1019 | 436 | 462 | 1019 | 436 | 462 | 1019 | 436 |
| nCohort 2 | 120 | 40 | 108 | 130 | 46 | 119 | 47 | 26 | 44 |
| Cutoff 1 | 394 | 73.3 | 548 | 216 | 226 | 280 | 213 | 195 | 205 |
| Sens 1 | 70% | 70% | 70% | 70% | 72% | 71% | 70% | 73% | 70% |
| Spec 1 | 48% | 13% | 55% | 34% | 27% | 37% | 34% | 24% | 31% |
| Cutoff 2 | 228 | 38.4 | 342 | 139 | 148 | 152 | 139 | 158 | 135 |
| Sens 2 | 80% | 80% | 81% | 80% | 80% | 81% | 81% | 81% | 82% |
| Spec 2 | 35% | 9% | 42% | 25% | 20% | 26% | 25% | 21% | 23% |
| Cutoff 3 | 55.0 | 23.9 | 128 | 33.7 | 35.6 | 49.3 | 45.1 | 126 | 40.9 |
| Sens 3 | 90% | 90% | 91% | 90% | 91% | 91% | 91% | 92% | 91% |
| Spec 3 | 16% | 8% | 23% | 13% | 9% | 14% | 14% | 17% | 12% |
| Cutoff 4 | 932 | 1150 | 928 | 932 | 1150 | 928 | 932 | 1150 | 928 |
| Sens 4 | 46% | 30% | 51% | 37% | 35% | 42% | 34% | 19% | 34% |
| Spec 4 | 70% | 70% | 70% | 70% | 70% | 70% | 70% | 70% | 70% |
| Cutoff 5 | 1410 | 1690 | 1330 | 1410 | 1690 | 1330 | 1410 | 1690 | 1330 |
| Sens 5 | 24% | 18% | 31% | 25% | 24% | 31% | 19% | 15% | 23% |
| Spec 5 | 80% | 80% | 80% | 80% | 80% | 80% | 80% | 80% | 80% |
| Cutoff 6 | 2100 | 2510 | 2210 | 2100 | 2510 | 2210 | 2100 | 2510 | 2210 |
| Sens 6 | 15% | 15% | 18% | 16% | 15% | 18% | 11% | 12% | 11% |
| Spec 6 | 90% | 90% | 90% | 90% | 90% | 90% | 90% | 90% | 90% |
| OR Quart 2 | 0.89 | 0.49 | 1.7 | 0.88 | 0.74 | 0.94 | 1.1 | 1.2 | 1.1 |
| p Value | 0.72 | 0.20 | 0.19 | 0.66 | 0.51 | 0.85 | 0.82 | 0.76 | 0.81 |
| 95% CI of | 0.46 | 0.17 | 0.78 | 0.50 | 0.31 | 0.51 | 0.45 | 0.36 | 0.44 |
| OR Quart2 | 1.7 | 1.5 | 3.6 | 1.6 | 1.8 | 1.7 | 2.7 | 4.0 | 2.9 |
| OR Quart 3 | 2.1 | 0.90 | 4.0 | 1.1 | 1.0 | 1.2 | 1.3 | 1.4 | 1.5 |
| p Value | 0.011 | 0.82 | 1.0E-4 | 0.78 | 0.99 | 0.47 | 0.51 | 0.56 | 0.37 |
| 95% CI of | 1.2 | 0.36 | 2.0 | 0.62 | 0.44 | 0.69 | 0.56 | 0.44 | 0.62 |
| OR Quart3 | 3.8 | 2.2 | 8.1 | 1.9 | 2.3 | 2.2 | 3.2 | 4.5 | 3.7 |
| OR Quart 4 | 2.0 | 1.6 | 4.2 | 1.3 | 1.1 | 1.8 | 1.3 | 1.6 | 1.4 |
| p Value | 0.023 | 0.23 | 6.6E-5 | 0.34 | 0.83 | 0.038 | 0.53 | 0.40 | 0.49 |
| 95% CI of | 1.1 | 0.73 | 2.1 | 0.76 | 0.49 | 1.0 | 0.56 | 0.52 | 0.55 |
| OR Quart4 | 3.5 | 3.7 | 8.4 | 2.2 | 2.4 | 3.2 | 3.1 | 5.0 | 3.4 |

| | | | | | | | | | |
|---|---|---|---|---|---|---|---|---|---|
| **Neutrophil collagenase** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 2540 | | 4730 | 2540 | | 5520 | 2540 | | 3780 |
| Average | 11100 | | 18100 | 11100 | | 19900 | 11100 | | 16200 |
| Stdev | 40300 | | 56700 | 40300 | | 45400 | 40300 | | 36300 |
| p(t-test) | | | 0.099 | | | 0.028 | | | 0.41 |
| Min | 0.114 | | 150 | 0.114 | | 22.5 | 0.114 | | 50.0 |
| Max | 670000 | | 625000 | 670000 | | 312000 | 670000 | | 236000 |
| n (Samp) | 498 | | 142 | 498 | | 140 | 498 | | 48 |
| n (Patient) | 235 | | 142 | 235 | | 140 | 235 | | 48 |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 3460 | | 3530 | 3460 | | 7850 | 3460 | | 4980 |
| Average | 15100 | | 14000 | 15100 | | 36300 | 15100 | | 16200 |
| Stdev | 44100 | | 25900 | 44100 | | 106000 | 44100 | | 44400 |
| p(t-test) | | | 0.86 | | | 0.0024 | | | 0.90 |
| Min | 0.114 | | 150 | 0.114 | | 40.5 | 0.114 | | 111 |
| Max | 670000 | | 128000 | 670000 | | 625000 | 670000 | | 236000 |
| n (Samp) | 1095 | | 46 | 1095 | | 51 | 1095 | | 28 |
| n (Patient) | 398 | | 46 | 398 | | 51 | 398 | | 28 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 2550 | | 5870 | 2550 | | 6090 | 2550 | | 4960 |
| Average | 8970 | | 26600 | 8970 | | 24800 | 8970 | | 11700 |
| Stdev | 22700 | | 78300 | 22700 | | 53900 | 22700 | | 16600 |
| p(t-test) | | | 1.7E-5 | | | 6.8E-7 | | | 0.42 |
| Min | 0.114 | | 0.260 | 0.114 | | 22.5 | 0.114 | | 50.0 |
| Max | 300000 | | 625000 | 300000 | | 327000 | 300000 | | 66900 |
| n (Samp) | 485 | | 129 | 485 | | 130 | 485 | | 47 |
| n (Patient) | 190 | | 129 | 190 | | 130 | 190 | | 47 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.62 | 0.54 | 0.65 | 0.61 | 0.57 | 0.64 | 0.58 | 0.54 | 0.59 |
| SE | 0.028 | 0.044 | 0.029 | 0.028 | 0.043 | 0.029 | 0.045 | 0.056 | 0.045 |
| p | 2.5E-5 | 0.33 | 1.5E-7 | 6.4E-5 | 0.091 | 9.6E-7 | 0.075 | 0.51 | 0.055 |
| nCohort 1 | 498 | 1095 | 485 | 498 | 1095 | 485 | 498 | 1095 | 485 |
| nCohort 2 | 142 | 46 | 129 | 140 | 51 | 130 | 48 | 28 | 47 |
| Cutoff 1 | 2030 | 1700 | 2620 | 2250 | 1970 | 2450 | 1430 | 1820 | 1550 |
| Sens 1 | 70% | 72% | 71% | 70% | 71% | 70% | 71% | 71% | 70% |
| Spec 1 | 44% | 34% | 51% | 47% | 37% | 49% | 37% | 36% | 38% |
| Cutoff 2 | 1110 | 977 | 1710 | 1060 | 935 | 1460 | 870 | 1410 | 898 |
| Sens 2 | 80% | 80% | 81% | 80% | 80% | 80% | 81% | 82% | 81% |
| Spec 2 | 31% | 22% | 40% | 30% | 21% | 36% | 27% | 29% | 26% |
| Cutoff 3 | 765 | 418 | 844 | 536 | 546 | 804 | 191 | 365 | 387 |
| Sens 3 | 90% | 91% | 91% | 90% | 90% | 90% | 92% | 93% | 91% |
| Spec 3 | 25% | 11% | 25% | 19% | 14% | 25% | 7% | 10% | 13% |
| Cutoff 4 | 6260 | 8700 | 6150 | 6260 | 8700 | 6150 | 6260 | 8700 | 6150 |
| Sens 4 | 45% | 41% | 49% | 46% | 49% | 50% | 44% | 32% | 47% |
| Spec 4 | 70% | 70% | 70% | 70% | 70% | 70% | 70% | 70% | 70% |
| Cutoff 5 | 10000 | 14900 | 9570 | 10000 | 14900 | 9570 | 10000 | 14900 | 9570 |
| Sens 5 | 35% | 28% | 39% | 35% | 27% | 38% | 35% | 18% | 34% |
| Spec 5 | 80% | 80% | 80% | 80% | 80% | 80% | 80% | 80% | 80% |
| Cutoff 6 | 20800 | 32400 | 21900 | 20800 | 32400 | 21900 | 20800 | 32400 | 21900 |
| Sens 6 | 16% | 7% | 20% | 19% | 14% | 20% | 21% | 7% | 15% |
| Spec 6 | 90% | 90% | 90% | 90% | 90% | 90% | 90% | 90% | 90% |
| OR Quart 2 | 1.4 | 0.74 | 2.1 | 1.3 | 0.52 | 1.8 | 1.1 | 2.0 | 1.1 |
| p Value | 0.29 | 0.51 | 0.026 | 0.46 | 0.18 | 0.083 | 0.82 | 0.25 | 0.81 |
| 95% CI of | 0.76 | 0.31 | 1.1 | 0.69 | 0.21 | 0.93 | 0.44 | 0.60 | 0.44 |
| OR Quart2 | 2.5 | 1.8 | 4.2 | 2.3 | 1.3 | 3.4 | 2.8 | 6.8 | 2.9 |
| OR Quart 3 | 2.1 | 0.66 | 2.6 | 2.0 | 1.0 | 2.4 | 1.2 | 2.5 | 1.2 |
| p Value | 0.012 | 0.37 | 0.0038 | 0.018 | 1.0 | 0.0053 | 0.64 | 0.12 | 0.64 |
| 95% CI of | 1.2 | 0.26 | 1.4 | 1.1 | 0.46 | 1.3 | 0.50 | 0.79 | 0.50 |
| OR Quart3 | 3.7 | 1.6 | 5.1 | 3.5 | 2.2 | 4.6 | 3.1 | 8.2 | 3.1 |
| OR Quart 4 | 2.9 | 1.4 | 4.6 | 2.6 | 1.4 | 3.7 | 2.1 | 1.5 | 2.0 |
| p Value | 1.6E-4 | 0.35 | 2.4E-6 | 7.1E-4 | 0.36 | 2.1E-5 | 0.076 | 0.53 | 0.10 |
| 95% CI of | 1.7 | 0.67 | 2.4 | 1.5 | 0.68 | 2.0 | 0.92 | 0.42 | 0.87 |
| OR Quart4 | 5.1 | 3.1 | 8.6 | 4.5 | 2.9 | 6.9 | 4.9 | 5.4 | 4.7 |

| | | | | | | | | | |
|---|---|---|---|---|---|---|---|---|---|
| **Protransforming growth factor alpha** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 5.17 | | 7.34 | 5.17 | | 8.58 | 5.17 | | 5.71 |
| Average | 10.2 | | 12.2 | 10.2 | | 11.9 | 10.2 | | 7.65 |
| Stdev | 23.4 | | 16.8 | 23.4 | | 11.0 | 23.4 | | 7.10 |
| p(t-test) | | | 0.38 | | | 0.43 | | | 0.46 |
| Min | 0.00184 | | 0.00574 | 0.00184 | | 0.00603 | 0.00184 | | 0.00440 |
| Max | 361 | | 135 | 361 | | 54.1 | 361 | | 32.4 |
| n (Samp) | 462 | | 116 | 462 | | 130 | 462 | | 47 |
| n (Patient) | 223 | | 116 | 223 | | 130 | 223 | | 47 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 6.18 | | 7.25 | 6.18 | | 7.37 | 6.18 | | 4.95 |
| Average | 10.8 | | 9.21 | 10.8 | | 10.0 | 10.8 | | 6.94 |
| Stdev | 19.7 | | 7.72 | 19.7 | | 10.1 | 19.7 | | 6.70 |
| p(t-test) | | | 0.61 | | | 0.79 | | | 0.32 |
| Min | 0.00184 | | 0.00574 | 0.00184 | | 0.00574 | 0.00184 | | 0.00574 |
| Max | 361 | | 30.2 | 361 | | 50.4 | 361 | | 27.7 |
| n (Samp) | 1014 | | 39 | 1014 | | 46 | 1014 | | 26 |
| n (Patient) | 375 | | 39 | 375 | | 46 | 375 | | 26 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 5.14 | | 8.56 | 5.14 | | 10.2 | 5.14 | | 6.78 |
| Average | 10.3 | | 14.4 | 10.3 | | 13.6 | 10.3 | | 8.83 |
| Stdev | 24.0 | | 18.7 | 24.0 | | 12.8 | 24.0 | | 7.87 |
| p(t-test) | | | 0.10 | | | 0.15 | | | 0.68 |
| Min | 0.00184 | | 0.00603 | 0.00184 | | 0.00603 | 0.00184 | | 0.00440 |
| Max | 361 | | 135 | 361 | | 77.6 | 361 | | 36.0 |
| n (Samp) | 436 | | 104 | 436 | | 119 | 436 | | 44 |
| n (Patient) | 173 | | 104 | 173 | | 119 | 173 | | 44 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.59 | 0.52 | 0.64 | 0.63 | 0.52 | 0.67 | 0.52 | 0.43 | 0.55 |
| SE | 0.030 | 0.048 | 0.032 | 0.029 | 0.044 | 0.030 | 0.045 | 0.059 | 0.047 |
| p | 0.0022 | 0.67 | 1.7E-5 | 5.1E-6 | 0.61 | 2.5E-8 | 0.73 | 0.25 | 0.24 |
| nCohort 1 | 462 | 1014 | 436 | 462 | 1014 | 436 | 462 | 1014 | 436 |
| nCohort 2 | 116 | 39 | 104 | 130 | 46 | 119 | 47 | 26 | 44 |
| Cutoff 1 | 4.08 | 3.95 | 5.24 | 5.35 | 3.53 | 6.12 | 2.73 | 2.73 | 3.27 |
| Sens 1 | 71% | 72% | 70% | 70% | 72% | 71% | 70% | 73% | 70% |
| Spec 1 | 41% | 32% | 51% | 52% | 29% | 56% | 29% | 22% | 32% |
| Cutoff 2 | 3.05 | 2.30 | 3.71 | 3.25 | 3.07 | 3.61 | 2.55 | 2.17 | 2.52 |
| Sens 2 | 80% | 82% | 81% | 80% | 80% | 81% | 81% | 81% | 82% |
| Spec 2 | 32% | 18% | 36% | 34% | 25% | 35% | 27% | 17% | 23% |
| Cutoff 3 | 2.14 | 0.804 | 2.37 | 2.15 | 0.00713 | 2.17 | 1.65 | 1.60 | 1.79 |
| Sens 3 | 91% | 92% | 90% | 90% | 91% | 92% | 91% | 92% | 91% |
| Spec 3 | 23% | 6% | 22% | 23% | 2% | 21% | 18% | 12% | 18% |
| Cutoff 4 | 8.54 | 9.97 | 8.15 | 8.54 | 9.97 | 8.15 | 8.54 | 9.97 | 8.15 |
| Sens 4 | 43% | 41% | 51% | 51% | 33% | 61% | 32% | 27% | 41% |
| Spec 4 | 70% | 70% | 70% | 70% | 70% | 70% | 70% | 70% | 70% |
| Cutoff 5 | 11.8 | 13.4 | 11.2 | 11.8 | 13.4 | 11.2 | 11.8 | 13.4 | 11.2 |
| Sens 5 | 30% | 23% | 38% | 36% | 28% | 45% | 21% | 12% | 30% |
| Spec 5 | 80% | 80% | 80% | 80% | 80% | 80% | 80% | 80% | 80% |
| Cutoff 6 | 19.3 | 22.6 | 19.8 | 19.3 | 22.6 | 19.8 | 19.3 | 22.6 | 19.8 |
| Sens 6 | 17% | 8% | 20% | 16% | 9% | 20% | 6% | 4% | 7% |
| Spec 6 | 90% | 90% | 90% | 90% | 90% | 90% | 90% | 90% | 90% |
| OR Quart 2 | 1.8 | 0.69 | 1.9 | 1.2 | 0.81 | 1.1 | 1.6 | 1.0 | 0.56 |
| p Value | 0.064 | 0.46 | 0.077 | 0.51 | 0.65 | 0.87 | 0.28 | 1.0 | 0.24 |
| 95% CI of | 0.97 | 0.26 | 0.93 | 0.65 | 0.33 | 0.52 | 0.68 | 0.29 | 0.21 |
| OR Quart2 | 3.5 | 1.8 | 4.0 | 2.4 | 2.0 | 2.2 | 3.9 | 3.5 | 1.5 |
| OR Quart 3 | 1.7 | 1.0 | 2.5 | 2.4 | 1.0 | 2.1 | 1.2 | 1.6 | 0.73 |
| p Value | 0.11 | 1.0 | 0.013 | 0.0040 | 1.0 | 0.021 | 0.64 | 0.40 | 0.49 |
| 95% CI of | 0.89 | 0.41 | 1.2 | 1.3 | 0.43 | 1.1 | 0.50 | 0.52 | 0.30 |
| OR Quart3 | 3.2 | 2.4 | 5.0 | 4.4 | 2.3 | 4.0 | 3.1 | 5.0 | 1.8 |
| OR Quart 4 | t 2.7 | 1.2 | 4.0 | 3.4 | 1.4 | 4.3 | 1.5 | 1.6 | 1.4 |
| p Value | 0.0017 | 0.67 | 7.6E-5 | 5.8E-5 | 0.42 | 3.7E-6 | 0.39 | 0.40 | 0.42 |
| 95% CI of | 1.4 | 0.51 | 2.0 | 1.9 | 0.62 | 2.3 | 0.61 | 0.52 | 0.62 |
| OR Quart4 | 4.9 | 2.8 | 7.8 | 6.1 | 3.1 | 7.9 | 3.6 | 5.0 | 3.1 |

| | | | | | | | | | |
|---|---|---|---|---|---|---|---|---|---|
| **CA 15-3** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 423 | | 434 | 423 | | 434 | 423 | | 434 |
| Average | 280 | | 376 | 280 | | 378 | 280 | | 361 |
| Stdev | 244 | | 215 | 244 | | 212 | 244 | | 141 |
| p(t-test) | | | 0.0018 | | | 0.0022 | | | 0.098 |
| Min | 3.03 | | 11.6 | 3.03 | | 6.39 | 3.03 | | 21.6 |
| Max | 784 | | 784 | 784 | | 784 | 784 | | 434 |
| n (Samp) | 194 | | 86 | 194 | | 76 | 194 | | 26 |
| n (Patient) | 123 | | 86 | 123 | | 76 | 123 | | 26 |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 434 | | 434 | 434 | | 434 | 434 | | 434 |
| Average | 331 | | 306 | 331 | | 360 | 331 | | 296 |
| Stdev | 227 | | 228 | 227 | | 202 | 227 | | 186 |
| p(t-test) | | | 0.59 | | | 0.50 | | | 0.55 |
| Min | 1.17 | | 9.20 | 1.17 | | 20.6 | 1.17 | | 11.7 |
| Max | 784 | | 784 | 784 | | 784 | 784 | | 434 |
| n (Samp) | 428 | | 26 | 428 | | 28 | 428 | | 16 |
| n (Patient) | 210 | | 26 | 210 | | 28 | 210 | | 16 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 434 | | 434 | 434 | | 434 | 434 | | 434 |
| Average | 286 | | 395 | 286 | | 377 | 286 | | 352 |
| Stdev | 233 | | 193 | 233 | | 216 | 233 | | 147 |
| p(t-test) | | | 2.9E-4 | | | 0.0041 | | | 0.16 |
| Min | 3.03 | | 12.2 | 3.03 | | 6.39 | 3.03 | | 28.4 |
| Max | 784 | | 784 | 784 | | 784 | 784 | | 434 |
| n (Samp) | 210 | | 78 | 210 | | 72 | 210 | | 27 |
| n (Patient) | 119 | | 78 | 119 | | 72 | 119 | | 27 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.62 | 0.47 | 0.63 | 0.63 | 0.55 | 0.62 | 0.62 | 0.48 | 0.60 |
| SE | 0.037 | 0.059 | 0.038 | 0.039 | 0.058 | 0.040 | 0.062 | 0.074 | 0.061 |
| p | 0.0010 | 0.64 | 5.3E-4 | 0.0013 | 0.42 | 0.0027 | 0.056 | 0.81 | 0.099 |
| nCohort 1 | 194 | 428 | 210 | 194 | 428 | 210 | 194 | 428 | 210 |
| nCohort 2 | 86 | 26 | 78 | 76 | 28 | 72 | 26 | 16 | 27 |
| Cutoff 1 | 320 | 66.6 | 427 | 413 | 413 | 427 | 413 | 113 | 427 |
| Sens 1 | 71% | 73% | 74% | 72% | 71% | 71% | 73% | 75% | 70% |
| Spec 1 | 47% | 25% | 47% | 50% | 39% | 47% | 50% | 30% | 47% |
| Cutoff 2 | 123 | 52.6 | 253 | 129 | 88.9 | 64.1 | 248 | 62.4 | 239 |
| Sens 2 | 80% | 81% | 81% | 80% | 82% | 81% | 81% | 81% | 81% |
| Spec 2 | 44% | 23% | 45% | 45% | 28% | 34% | 47% | 24% | 44% |
| Cutoff 3 | 48.4 | 25.1 | 52.2 | 33.2 | 24.5 | 42.9 | 68.6 | 21.3 | 68.6 |
| Sens 3 | 91% | 92% | 91% | 91% | 93% | 90% | 92% | 94% | 93% |
| Spec 3 | 33% | 13% | 32% | 25% | 12% | 28% | 39% | 11% | 35% |
| Cutoff 4 | 434 | 434 | 434 | 434 | 434 | 434 | 434 | 434 | 434 |
| Sens 4 | 12% | 8% | 10% | 12% | 7% | 14% | 0% | 0% | 0% |
| Spec 4 | 91% | 90% | 92% | 91% | 90% | 92% | 91% | 90% | 92% |
| Cutoff 5 | 434 | 434 | 434 | 434 | 434 | 434 | 434 | 434 | 434 |
| Sens 5 | 12% | 8% | 10% | 12% | 7% | 14% | 0% | 0% | 0% |
| Spec 5 | 91% | 90% | 92% | 91% | 90% | 92% | 91% | 90% | 92% |
| Cutoff 6 | 434 | 434 | 434 | 434 | 434 | 434 | 434 | 434 | 434 |
| Sens 6 | 12% | 8% | 10% | 12% | 7% | 14% | 0% | 0% | 0% |
| Spec 6 | 91% | 90% | 92% | 91% | 90% | 92% | 91% | 90% | 92% |
| OR Quart 2 | 5.8 | 0.50 | 5.9 | 3.3 | 4.5 | 4.2 | 6.6 | >6.3 | 10 |
| p Value | 8.1E-5 | 0.57 | 1.4E-4 | 0.0094 | 0.0091 | 0.0014 | 0.085 | <0.090 | 0.029 |
| 95% CI of | 2.4 | 0.045 | 2.4 | 1.3 | 1.5 | 1.7 | 0.77 | >0.75 | 1.3 |
| OR Quart2 | 14 | 5.6 | 15 | 8.1 | 14 | 10 | 57 | na | 85 |
| OR Quart 3 | 9.2 | 9.1 | 8.8 | 9.7 | 1.5 | 5.5 | 28 | >6.3 | 23 |
| p Value | 6.4E-7 | 0.0037 | 2.6E-6 | 4.7E-7 | 0.52 | 1.4E-4 | 0.0014 | <0.090 | 0.0026 |
| 95% CI of | 3.8 | 2.1 | 3.5 | 4.0 | 0.42 | 2.3 | 3.7 | >0.75 | 3.0 |
| OR Quart3 | 22 | 41 | 22 | 23 | 5.6 | 13 | 220 | na | 180 |
| OR Quart 4 | 1.3 | 3.7 | 1.2 | 1.1 | 0.49 | 1.3 | 0 | >4.1 | 0 |
| p Value | 0.61 | 0.11 | 0.79 | 0.82 | 0.42 | 0.64 | na | <0.21 | na |
| 95% CI of | 0.48 | 0.75 | 0.40 | 0.41 | 0.088 | 0.47 | na | >0.46 | na |
| OR Quart4 | 3.5 | 18 | 3.4 | 3.1 | 2.7 | 3.4 | na | na | na |

**Table 2: Comparison of marker levels in urine samples collected from Cohort 1 (patients that did not progress beyond RIFLE stage 0 or R) and in urine samples collected from subjects at 0, 24 hours, and 48 hours prior to reaching stage I or F in Cohort 2.**

| **C-C motif chemokine 18** | | | | | | | | | |
|---|---|---|---|---|---|---|---|---|---|
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 0.418 | | 0.977 | 0.418 | | 1.37 | 0.418 | | 0.524 |
| Average | 2.19 | | 3.40 | 2.19 | | 5.34 | 2.19 | | 6.72 |
| Stdev | 6.72 | | 6.53 | 6.72 | | 10.3 | 6.72 | | 13.8 |
| p(t-test) | | | 0.17 | | | 3.2E-4 | | | 1.1E-4 |
| Min | 3.13E-5 | | 0.00308 | 3.13E-5 | | 0.0139 | 3.13E-5 | | 0.00215 |
| Max | 40.0 | | 40.0 | 40.0 | | 40.0 | 40.0 | | 40.0 |
| n (Samp) | 927 | | 62 | 927 | | 70 | 927 | | 39 |
| n (Patient) | 360 | | 62 | 360 | | 70 | 360 | | 39 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 0.455 | | 1.90 | 0.455 | | 1.34 | 0.455 | | 1.26 |
| Average | 2.54 | | 8.19 | 2.54 | | 6.52 | 2.54 | | 5.50 |
| Stdev | 7.37 | | 12.4 | 7.37 | | 10.7 | 7.37 | | 11.1 |
| p(t-test) | | | 0.0035 | | | 0.024 | | | 0.10 |
| Min | 3.13E-5 | | 0.192 | 3.13E-5 | | 0.150 | 3.13E-5 | | 0.0404 |
| Max | 40.0 | | 40.0 | 40.0 | | 40.0 | 40.0 | | 37.8 |
| n (Samp) | 1230 | | 15 | 1230 | | 18 | 1230 | | 17 |
| n (Patient) | 440 | | 15 | 440 | | 18 | 440 | | 17 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 0.456 | | 0.951 | 0.456 | | 1.71 | 0.456 | | 0.692 |
| Average | 2.32 | | 4.12 | 2.32 | | 6.37 | 2.32 | | 8.23 |
| Stdev | 6.87 | | 8.22 | 6.87 | | 11.6 | 6.87 | | 14.6 |
| p(t-test) | | | 0.060 | | | 2.5E-5 | | | 4.6E-6 |
| Min | 3.13E-5 | | 0.00308 | 3.13E-5 | | 0.0139 | 3.13E-5 | | 0.00215 |
| Max | 40.0 | | 40.0 | 40.0 | | 40.0 | 40.0 | | 40.0 |
| n (Samp) | 815 | | 57 | 815 | | 63 | 815 | | 34 |
| n (Patient) | 282 | | 57 | 282 | | 63 | 282 | | 34 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.65 | 0.79 | 0.63 | 0.67 | 0.70 | 0.68 | 0.58 | 0.66 | 0.60 |
| SE | 0.039 | 0.070 | 0.041 | 0.036 | 0.070 | 0.038 | 0.049 | 0.073 | 0.052 |
| p | 1.9E-4 | 2.6E-5 | 0.0015 | 2.2E-6 | 0.0046 | 4.5E-6 | 0.094 | 0.025 | 0.047 |
| nCohort 1 | 927 | 1230 | 815 | 927 | 1230 | 815 | 927 | 1230 | 815 |
| nCohort 2 | 62 | 15 | 57 | 70 | 18 | 63 | 39 | 17 | 34 |
| Cutoff 1 | 0.351 | 1.29 | 0.351 | 0.408 | 0.533 | 0.408 | 0.372 | 0.415 | 0.373 |
| Sens 1 | 71% | 73% | 70% | 70% | 72% | 71% | 72% | 71% | 71% |
| Spec 1 | 46% | 75% | 44% | 50% | 54% | 48% | 47% | 48% | 45% |
| Cutoff 2 | 0.231 | 0.985 | 0.231 | 0.282 | 0.340 | 0.282 | 0.131 | 0.388 | 0.131 |
| Sens 2 | 81% | 80% | 81% | 80% | 83% | 81% | 82% | 82% | 82% |
| Spec 2 | 33% | 69% | 31% | 41% | 43% | 38% | 21% | 47% | 19% |
| Cutoff 3 | 0.156 | 0.767 | 0.147 | 0.150 | 0.181 | 0.142 | 0.0423 | 0.225 | 0.0950 |
| Sens 3 | 90% | 93% | 91% | 90% | 94% | 90% | 92% | 94% | 91% |
| Spec 3 | 25% | 63% | 22% | 25% | 26% | 21% | 7% | 30% | 13% |
| Cutoff 4 | 0.900 | 1.02 | 0.982 | 0.900 | 1.02 | 0.982 | 0.900 | 1.02 | 0.982 |
| Sens 4 | 53% | 73% | 49% | 57% | 61% | 59% | 38% | 59% | 44% |
| Spec 4 | 70% | 70% | 70% | 70% | 70% | 70% | 70% | 70% | 70% |
| Cutoff 5 | 1.48 | 1.77 | 1.61 | 1.48 | 1.77 | 1.61 | 1.48 | 1.77 | 1.61 |
| Sens 5 | 42% | 53% | 42% | 49% | 44% | 51% | 33% | 35% | 38% |
| Spec 5 | 80% | 80% | 80% | 80% | 80% | 80% | 80% | 80% | 80% |
| Cutoff 6 | 3.30 | 3.86 | 3.53 | 3.30 | 3.86 | 3.53 | 3.30 | 3.86 | 3.53 |
| Sens 6 | 26% | 33% | 28% | 29% | 28% | 33% | 23% | 24% | 24% |
| Spec 6 | 90% | 90% | 90% | 90% | 90% | 90% | 90% | 90% | 90% |
| OR Quart 2 | 2.2 | >1.0 | 1.8 | 1.9 | 3.0 | 1.5 | 1.00 | 5.0 | 0.71 |
| p Value | 0.088 | <1.00 | 0.19 | 0.14 | 0.34 | 0.37 | 0.99 | 0.14 | 0.56 |
| 95% CI of | 0.89 | >0.062 | 0.74 | 0.80 | 0.31 | 0.61 | 0.37 | 0.59 | 0.22 |
| OR Quart2 | 5.5 | na | 4.4 | 4.6 | 29 | 3.8 | 2.7 | 43 | 2.3 |
| OR Quart 3 | 1.6 | >5.1 | 1.0 | 1.4 | 5.1 | 1.0 | 1.3 | 3.0 | 1.3 |
| p Value | 0.34 | <0.14 | 1.0 | 0.48 | 0.14 | 1.0 | 0.63 | 0.34 | 0.61 |
| 95% CI of | 0.61 | >0.59 | 0.37 | 0.55 | 0.59 | 0.37 | 0.49 | 0.31 | 0.47 |
| OR Quart3 | 4.2 | na | 2.7 | 3.5 | 44 | 2.7 | 3.3 | 29 | 3.6 |
| OR Quart 4 | 4.5 | >9.2 | 3.7 | 5.1 | 9.2 | 5.0 | 1.7 | 8.2 | 1.9 |
| p Value | 4.5E-4 | <0.035 | 0.0016 | 5.4E-5 | 0.035 | 7.1E-5 | 0.27 | 0.048 | 0.18 |
| 95% CI of | 1.9 | >1.2 | 1.6 | 2.3 | 1.2 | 2.3 | 0.67 | 1.0 | 0.74 |
| OR Quart4 | 11 | na | 8.4 | 11 | 73 | 11 | 4.1 | 66 | 4.9 |

| | | | | | | | | | |
|---|---|---|---|---|---|---|---|---|---|
| **C-C motif chemokine 24** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 11.1 | | 26.1 | 11.1 | | 27.2 | 11.1 | | 13.3 |

| sCr or UO | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Average | 25.6 | | 78.6 | 25.6 | | 100 | 25.6 | | 65.6 |
| Stdev | 60.0 | | 180 | 60.0 | | 331 | 60.0 | | 197 |
| p(t-test) | | | 4.5E-8 | | | 1.1E-8 | | | 5.4E-4 |
| Min | 0.0120 | | 0.0123 | 0.0120 | | 0.0347 | 0.0120 | | 0.0123 |
| Max | 1090 | | 1010 | 1090 | | 2380 | 1090 | | 1170 |
| n (Samp) | 928 | | 62 | 928 | | 70 | 928 | | 39 |
| n (Patient) | 361 | | 62 | 361 | | 70 | 361 | | 39 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 13.4 | | 60.3 | 13.4 | | 28.9 | 13.4 | | 25.4 |
| Average | 37.8 | | 104 | 37.8 | | 60.4 | 37.8 | | 46.1 |
| Stdev | 125 | | 123 | 125 | | 68.1 | 125 | | 67.5 |
| p(t-test) | | | 0.041 | | | 0.44 | | | 0.78 |
| Min | 0.0120 | | 0.444 | 0.0120 | | 2.39 | 0.0120 | | 0.0196 |
| Max | 2380 | | 373 | 2380 | | 214 | 2380 | | 249 |
| n (Samp) | 1232 | | 15 | 1232 | | 18 | 1232 | | 17 |
| n (Patient) | 441 | | 15 | 441 | | 18 | 441 | | 17 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 11.3 | | 26.8 | 11.3 | | 27.4 | 11.3 | | 15.7 |
| Average | 26.4 | | 125 | 26.4 | | 139 | 26.4 | | 79.3 |
| Stdev | 63.0 | | 403 | 63.0 | | 416 | 63.0 | | 210 |
| p(t-test) | | | 1.9E-9 | | | 1.9E-11 | | | 5.4E-5 |
| Min | 0.0120 | | 0.0123 | 0.0120 | | 0.0347 | 0.0120 | | 0.0123 |
| Max | 1090 | | 2790 | 1090 | | 2380 | 1090 | | 1170 |
| n (Samp) | 817 | | 57 | 817 | | 63 | 817 | | 34 |
| n (Patient) | 283 | | 57 | 283 | | 63 | 283 | | 34 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.68 | 0.75 | 0.68 | 0.68 | 0.69 | 0.69 | 0.52 | 0.59 | 0.56 |
| SE | 0.039 | 0.073 | 0.040 | 0.036 | 0.070 | 0.038 | 0.048 | 0.073 | 0.052 |
| p | 5.1E-6 | 5.2E-4 | 5.7E-6 | 5.9E-7 | 0.0067 | 3.0E-7 | 0.61 | 0.20 | 0.22 |
| nCohort 1 | 928 | 1232 | 817 | 928 | 1232 | 817 | 928 | 1232 | 817 |
| nCohort 2 | 62 | 15 | 57 | 70 | 18 | 63 | 39 | 17 | 34 |
| Cutoff 1 | 10.4 | 25.9 | 11.3 | 14.5 | 15.6 | 14.5 | 4.24 | 14.9 | 4.59 |
| Sens 1 | 71% | 73% | 70% | 70% | 72% | 71% | 72% | 71% | 71% |
| Spec 1 | 48% | 69% | 50% | 57% | 55% | 58% | 32% | 54% | 33% |
| Cutoff 2 | 6.47 | 21.9 | 6.55 | 9.55 | 8.15 | 9.78 | 0.303 | 2.37 | 2.59 |
| Sens 2 | 81% | 80% | 81% | 80% | 83% | 81% | 82% | 82% | 82% |
| Spec 2 | 37% | 65% | 38% | 46% | 39% | 46% | 22% | 24% | 27% |
| Cutoff 3 | 2.85 | 2.85 | 1.36 | 4.66 | 3.22 | 5.39 | 0.0277 | 0.0277 | 0.0562 |
| Sens 3 | 90% | 93% | 91% | 90% | 94% | 90% | 92% | 94% | 91% |
| Spec 3 | 28% | 25% | 24% | 33% | 26% | 35% | 8% | 7% | 19% |
| Cutoff 4 | 22.3 | 26.6 | 21.8 | 22.3 | 26.6 | 21.8 | 22.3 | 26.6 | 21.8 |
| Sens 4 | 58% | 67% | 60% | 57% | 61% | 59% | 36% | 41% | 44% |
| Spec 4 | 70% | 70% | 70% | 70% | 70% | 70% | 70% | 70% | 70% |
| Cutoff 5 | 34.1 | 40.7 | 33.4 | 34.1 | 40.7 | 33.4 | 34.1 | 40.7 | 33.4 |
| Sens 5 | 42% | 67% | 44% | 39% | 39% | 41% | 21% | 24% | 26% |
| Spec 5 | 80% | 80% | 80% | 80% | 80% | 80% | 80% | 80% | 80% |
| Cutoff 6 | 57.3 | 69.9 | 62.1 | 57.3 | 69.9 | 62.1 | 57.3 | 69.9 | 62.1 |
| Sens 6 | 27% | 33% | 28% | 20% | 28% | 21% | 15% | 18% | 21% |
| Spec 6 | 90% | 90% | 90% | 90% | 90% | 90% | 90% | 90% | 90% |
| OR Quart 2 | 2.4 | 0.50 | 2.0 | 5.2 | 3.0 | 4.2 | 1.3 | 0.25 | 1.5 |
| p Value | 0.077 | 0.57 | 0.16 | 0.0096 | 0.34 | 0.029 | 0.64 | 0.21 | 0.44 |
| 95% CI of | 0.91 | 0.045 | 0.75 | 1.5 | 0.31 | 1.2 | 0.49 | 0.028 | 0.53 |
| OR Quart2 | 6.4 | 5.5 | 5.6 | 18 | 29 | 15 | 3.2 | 2.2 | 4.3 |
| OR Quart 3 | 2.4 | 1.00 | 2.1 | 6.8 | 6.1 | 6.1 | 1.5 | 2.0 | 1.5 |
| p Value | 0.076 | 1.00 | 0.16 | 0.0023 | 0.095 | 0.0045 | 0.37 | 0.25 | 0.44 |
| 95% CI of | 0.91 | 0.14 | 0.76 | 2.0 | 0.73 | 1.7 | 0.61 | 0.60 | 0.53 |
| OR Quart3 | 6.4 | 7.1 | 5.6 | 23 | 51 | 21 | 3.8 | 6.8 | 4.3 |
| OR Quart 4 | 5.1 | 5.1 | 5.0 | 12 | 8.2 | 12 | 1.1 | 1.00 | 1.7 |
| p Value | 3.8E-4 | 0.036 | 5.2E-4 | 3.5E-5 | 0.048 | 5.4E-5 | 0.81 | 1.00 | 0.32 |
| 95% CI of | 2.1 | 1.1 | 2.0 | 3.8 | 1.0 | 3.6 | 0.43 | 0.25 | 0.60 |
| OR Quart4 | 13 | 24 | 12 | 41 | 66 | 39 | 3.0 | 4.0 | 4.7 |

| | | | | | | | | | |
|---|---|---|---|---|---|---|---|---|---|
| **C-C motif chemokine 8** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 1.21 | | 5.60 | 1.21 | | 6.10 | 1.21 | | 1.51 |
| Average | 9.02 | | 18.6 | 9.02 | | 19.2 | 9.02 | | 18.6 |
| Stdev | 25.8 | | 33.5 | 25.8 | | 42.6 | 25.8 | | 41.8 |
| p(t-test) | | | 0.0056 | | | 0.0027 | | | 0.028 |
| Min | 0.0250 | | 0.0775 | 0.0250 | | 0.0250 | 0.0250 | | 0.0250 |
| Max | 473 | | 162 | 473 | | 294 | 473 | | 214 |
| n (Samp) | 928 | | 62 | 928 | | 70 | 928 | | 39 |
| n (Patient) | 361 | | 62 | 361 | | 70 | 361 | | 39 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 1.36 | | 7.59 | 1.36 | | 8.78 | 1.36 | | 2.77 |
| Average | 11.2 | | 23.8 | 11.2 | | 40.3 | 11.2 | | 11.6 |
| Stdev | 32.4 | | 36.8 | 32.4 | | 75.9 | 32.4 | | 16.3 |
| p(t-test) | | | 0.14 | | | 2.5E-4 | | | 0.96 |
| Min | 0.0250 | | 0.127 | 0.0250 | | 0.0569 | 0.0250 | | 0.0250 |
| Max | 492 | | 116 | 492 | | 279 | 492 | | 46.5 |
| n (Samp) | 1232 | | 15 | 1232 | | 18 | 1232 | | 17 |
| n (Patient) | 441 | | 15 | 441 | | 18 | 441 | | 17 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 1.21 | | 5.77 | 1.21 | | 6.84 | 1.21 | | 2.14 |
| Average | 9.52 | | 22.6 | 9.52 | | 22.9 | 9.52 | | 20.3 |
| Stdev | 27.3 | | 39.9 | 27.3 | | 45.9 | 27.3 | | 44.5 |
| p(t-test) | | | 7.8E-4 | | | 4.5E-4 | | | 0.029 |
| Min | 0.0250 | | 0.0775 | 0.0250 | | 0.0250 | 0.0250 | | 0.0250 |
| Max | 473 | | 176 | 473 | | 294 | 473 | | 214 |
| n (Samp) | 817 | | 57 | 817 | | 63 | 817 | | 34 |
| n (Patient) | 283 | | 57 | 283 | | 63 | 283 | | 34 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.67 | 0.63 | 0.69 | 0.65 | 0.67 | 0.66 | 0.56 | 0.55 | 0.57 |
| SE | 0.039 | 0.078 | 0.040 | 0.037 | 0.071 | 0.039 | 0.049 | 0.072 | 0.052 |
| p | 8.0E-6 | 0.093 | 2.4E-6 | 4.7E-5 | 0.016 | 2.2E-5 | 0.18 | 0.49 | 0.16 |
| nCohort 1 | 928 | 1232 | 817 | 928 | 1232 | 817 | 928 | 1232 | 817 |
| nCohort 2 | 62 | 15 | 57 | 70 | 18 | 63 | 39 | 17 | 34 |
| Cutoff 1 | 1.55 | 0.240 | 2.74 | 1.21 | 3.16 | 1.55 | 0.438 | 0.802 | 0.453 |
| Sens 1 | 71% | 73% | 70% | 71% | 72% | 71% | 72% | 71% | 71% |
| Spec 1 | 54% | 33% | 59% | 51% | 59% | 54% | 41% | 43% | 41% |
| Cutoff 2 | 0.802 | 0.239 | 0.802 | 0.240 | 0.239 | 0.453 | 0.154 | 0.127 | 0.240 |
| Sens 2 | 82% | 80% | 86% | 81% | 89% | 81% | 85% | 82% | 85% |
| Spec 2 | 45% | 31% | 46% | 36% | 31% | 41% | 29% | 21% | 35% |
| Cutoff 3 | 0.154 | 0.127 | 0.240 | 0.154 | 0.154 | 0.127 | 0.0775 | 0.0705 | 0.127 |
| Sens 3 | 92% | 93% | 91% | 90% | 94% | 90% | 92% | 94% | 91% |
| Spec 3 | 29% | 21% | 35% | 29% | 27% | 21% | 16% | 11% | 21% |
| Cutoff 4 | 5.57 | 6.87 | 5.57 | 5.57 | 6.87 | 5.57 | 5.57 | 6.87 | 5.57 |
| Sens 4 | 50% | 53% | 51% | 53% | 61% | 56% | 33% | 35% | 35% |
| Spec 4 | 70% | 70% | 70% | 70% | 70% | 70% | 70% | 70% | 70% |
| Cutoff 5 | 10.6 | 12.1 | 11.1 | 10.6 | 12.1 | 11.1 | 10.6 | 12.1 | 11.1 |
| Sens 5 | 39% | 33% | 40% | 30% | 44% | 33% | 31% | 35% | 24% |
| Spec 5 | 80% | 80% | 80% | 80% | 80% | 80% | 80% | 80% | 80% |
| Cutoff 6 | 21.9 | 26.4 | 22.0 | 21.9 | 26.4 | 22.0 | 21.9 | 26.4 | 22.0 |
| Sens 6 | 24% | 27% | 28% | 20% | 22% | 25% | 18% | 18% | 18% |
| Spec 6 | 90% | 90% | 90% | 90% | 90% | 90% | 90% | 90% | 90% |
| OR Quart 2 | 5.2 | 1.5 | 2.8 | 2.4 | 4.0 | 1.4 | 2.7 | 1.0 | 3.1 |
| p Value | 0.0096 | 0.66 | 0.079 | 0.077 | 0.21 | 0.46 | 0.065 | 1.0 | 0.053 |
| 95% CI of | 1.5 | 0.25 | 0.89 | 0.91 | 0.45 | 0.54 | 0.94 | 0.25 | 0.98 |
| OR Quart2 | 18 | 9.0 | 9.0 | 6.4 | 36 | 3.9 | 7.6 | 4.0 | 9.8 |
| OR Quart 3 | 6.4 | 1.5 | 4.5 | 4.1 | 4.0 | 3.0 | 1.8 | 0.75 | 2.0 |
| p Value | 0.0032 | 0.66 | 0.0075 | 0.0025 | 0.21 | 0.013 | 0.29 | 0.70 | 0.25 |
| 95% CI of | 1.9 | 0.25 | 1.5 | 1.6 | 0.45 | 1.3 | 0.60 | 0.17 | 0.60 |
| OR Quart3 | 22 | 9.0 | 14 | 10 | 36 | 7.4 | 5.5 | 3.4 | 6.8 |
| OR Quart 4 | 9.5 | 3.5 | 6.9 | 4.9 | 9.2 | 4.1 | 2.5 | 1.5 | 2.6 |
| p Value | 2.6E-4 | 0.12 | 4.2E-4 | 5.6E-4 | 0.036 | 0.0013 | 0.095 | 0.53 | 0.12 |
| 95% CI of | 2.8 | 0.73 | 2.4 | 2.0 | 1.2 | 1.7 | 0.85 | 0.42 | 0.79 |
| OR Quart4 | 32 | 17 | 20 | 12 | 73 | 9.6 | 7.1 | 5.4 | 8.3 |

| **Cathepsin D** | | | | | | | | | |
|---|---|---|---|---|---|---|---|---|---|
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 72900 | | 109000 | 72900 | | 93800 | 72900 | | 87500 |
| Average | 77100 | | 113000 | 77100 | | 103000 | 77100 | | 90300 |
| Stdev | 39300 | | 46500 | 39300 | | 43200 | 39300 | | 45100 |
| p(t-test) | | | 8.8E-12 | | | 1.9E-7 | | | 0.042 |
| Min | 656 | | 3710 | 656 | | 22600 | 656 | | 4320 |
| Max | 200000 | | 200000 | 200000 | | 200000 | 200000 | | 200000 |
| n (Samp) | 927 | | 62 | 927 | | 70 | 927 | | 39 |
| n (Patient) | 360 | | 62 | 360 | | 70 | 360 | | 39 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 76100 | | 141000 | 76100 | | 98000 | 76100 | | 111000 |
| Average | 82300 | | 128000 | 82300 | | 112000 | 82300 | | 97600 |
| Stdev | 42300 | | 39000 | 42300 | | 43000 | 42300 | | 43200 |
| p(t-test) | | | 3.2E-5 | | | 0.0034 | | | 0.14 |
| Min | 656 | | 70300 | 656 | | 52100 | 656 | | 22100 |
| Max | 200000 | | 193000 | 200000 | | 200000 | 200000 | | 200000 |
| n (Samp) | 1230 | | 15 | 1230 | | 18 | 1230 | | 17 |
| n (Patient) | 440 | | 15 | 440 | | 18 | 440 | | 17 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 74300 | | 111000 | 74300 | | 97500 | 74300 | | 94700 |
| Average | 79900 | | 114000 | 79900 | | 105000 | 79900 | | 97200 |
| Stdev | 38900 | | 47800 | 38900 | | 44600 | 38900 | | 44600 |
| p(t-test) | | | 4.5E-10 | | | 9.0E-7 | | | 0.012 |
| Min | 2520 | | 3710 | 2520 | | 22600 | 2520 | | 4320 |
| Max | 200000 | | 200000 | 200000 | | 200000 | 200000 | | 200000 |
| n (Samp) | 815 | | 57 | 815 | | 63 | 815 | | 34 |
| n (Patient) | 282 | | 57 | 282 | | 63 | 282 | | 34 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.73 | 0.79 | 0.72 | 0.67 | 0.69 | 0.67 | 0.59 | 0.61 | 0.63 |
| SE | 0.037 | 0.070 | 0.039 | 0.036 | 0.070 | 0.038 | 0.049 | 0.073 | 0.052 |
| p | 1.1E-9 | 3.2E-5 | 4.4E-8 | 2.2E-6 | 0.0060 | 1.4E-5 | 0.067 | 0.13 | 0.015 |
| nCohort 1 | 927 | 1230 | 815 | 927 | 1230 | 815 | 927 | 1230 | 815 |
| nCohort 2 | 62 | 15 | 57 | 70 | 18 | 63 | 39 | 17 | 34 |
| Cutoff 1 | 83000 | 100000 | 84700 | 78100 | 84700 | 77500 | 65500 | 70400 | 73300 |
| Sens 1 | 71% | 73% | 70% | 70% | 72% | 71% | 72% | 71% | 71% |
| Spec 1 | 62% | 70% | 60% | 57% | 58% | 53% | 43% | 44% | 49% |
| Cutoff 2 | 73800 | 94300 | 76300 | 68200 | 79000 | 68200 | 55600 | 55600 | 65500 |
| Sens 2 | 81% | 80% | 81% | 80% | 83% | 81% | 82% | 82% | 82% |
| Spec 2 | 52% | 66% | 53% | 45% | 53% | 43% | 31% | 27% | 40% |
| Cutoff 3 | 61400 | 73800 | 59600 | 54900 | 53700 | 55200 | 38800 | 40900 | 54900 |
| Sens 3 | 90% | 93% | 91% | 90% | 94% | 90% | 92% | 94% | 91% |
| Spec 3 | 37% | 47% | 33% | 30% | 25% | 28% | 15% | 16% | 27% |
| Cutoff 4 | 92900 | 101000 | 96900 | 92900 | 101000 | 96900 | 92900 | 101000 | 96900 |
| Sens 4 | 61% | 67% | 61% | 51% | 50% | 51% | 46% | 59% | 47% |
| Spec 4 | 70% | 70% | 70% | 70% | 70% | 70% | 70% | 70% | 70% |
| Cutoff 5 | 111000 | 117000 | 112000 | 111000 | 117000 | 112000 | 111000 | 117000 | 112000 |
| Sens 5 | 48% | 53% | 49% | 39% | 44% | 40% | 26% | 35% | 26% |
| Spec 5 | 80% | 80% | 80% | 80% | 80% | 80% | 80% | 80% | 80% |
| Cutoff 6 | 130000 | 140000 | 133000 | 130000 | 140000 | 133000 | 130000 | 140000 | 133000 |
| Sens 6 | 37% | 53% | 32% | 23% | 28% | 24% | 18% | 6% | 21% |
| Spec 6 | 90% | 90% | 90% | 90% | 90% | 90% | 90% | 90% | 90% |
| OR Quart 2 | 2.3 | >2.0 | 1.2 | 2.9 | 2.0 | 2.7 | 2.0 | 2.0 | 2.7 |
| p Value | 0.17 | <0.57 | 0.76 | 0.044 | 0.57 | 0.065 | 0.20 | 0.42 | 0.14 |
| 95% CI of | 0.70 | >0.18 | 0.36 | 1.0 | 0.18 | 0.94 | 0.68 | 0.36 | 0.71 |
| OR Quart2 | 7.6 | na | 4.0 | 8.2 | 22 | 7.7 | 6.0 | 11 | 10 |
| OR Quart 3 | 4.2 | >4.1 | 3.1 | 4.7 | 7.1 | 3.8 | 2.0 | 1.00 | 3.1 |
| p Value | 0.011 | <0.21 | 0.029 | 0.0020 | 0.067 | 0.0091 | 0.20 | 1.00 | 0.094 |
| 95% CI of | 1.4 | >0.45 | 1.1 | 1.8 | 0.87 | 1.4 | 0.69 | 0.14 | 0.82 |
| OR Quart3 | 13 | na | 8.8 | 13 | 58 | 11 | 6.1 | 7.1 | 12 |
| OR Quart 4 | 9.3 | >9.2 | 7.1 | 6.4 | 8.2 | 6.0 | 2.9 | 4.6 | 4.9 |
| p Value | 3.3E-5 | <0.035 | 7.2E-5 | 1.7E-4 | 0.048 | 3.2E-4 | 0.044 | 0.053 | 0.014 |
| 95% CI of | 3.3 | >1.2 | 2.7 | 2.4 | 1.0 | 2.3 | 1.0 | 0.98 | 1.4 |
| OR Quart4 | 27 | na | 19 | 17 | 66 | 16 | 8.2 | 21 | 17 |

| | | | | | | | | | |
|---|---|---|---|---|---|---|---|---|---|
| **C-X-C motif chemokine 13** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 0.0222 | | 1.42 | 0.0222 | | 1.46 | 0.0222 | | 0.232 |
| Average | 5.79 | | 13.4 | 5.79 | | 16.4 | 5.79 | | 17.2 |
| Stdev | 38.4 | | 34.8 | 38.4 | | 33.5 | 38.4 | | 71.5 |
| p(t-test) | | | 0.13 | | | 0.025 | | | 0.083 |
| Min | 0.00269 | | 0.00471 | 0.00269 | | 0.00269 | 0.00269 | | 0.00269 |
| Max | 832 | | 210 | 832 | | 131 | 832 | | 440 |
| n (Samp) | 928 | | 62 | 928 | | 70 | 928 | | 39 |
| n (Patient) | 361 | | 62 | 361 | | 70 | 361 | | 39 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 0.115 | | 3.89 | 0.115 | | 3.31 | 0.115 | | 1.39 |
| Average | 8.27 | | 22.4 | 8.27 | | 20.2 | 8.27 | | 9.64 |
| Stdev | 41.2 | | 45.9 | 41.2 | | 50.6 | 41.2 | | 20.1 |
| p(t-test) | | | 0.19 | | | 0.22 | | | 0.89 |
| Min | 0.00269 | | 0.00627 | 0.00269 | | 0.00822 | 0.00269 | | 0.00269 |
| Max | 832 | | 171 | 832 | | 210 | 832 | | 79.0 |
| n (Samp) | 1232 | | 15 | 1232 | | 18 | 1232 | | 17 |
| n (Patient) | 441 | | 15 | 441 | | 18 | 441 | | 17 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 0.0222 | | 1.79 | 0.0222 | | 1.86 | 0.0222 | | 1.09 |
| Average | 6.06 | | 48.6 | 6.06 | | 48.7 | 6.06 | | 20.3 |
| Stdev | 40.6 | | 257 | 40.6 | | 234 | 40.6 | | 76.3 |
| p(t-test) | | | 4.8E-5 | | | 1.0E-5 | | | 0.057 |
| Min | 0.00269 | | 0.00471 | 0.00269 | | 0.00269 | 0.00269 | | 0.00269 |
| Max | 832 | | 1930 | 832 | | 1850 | 832 | | 440 |
| n (Samp) | 817 | | 57 | 817 | | 63 | 817 | | 34 |
| n (Patient) | 283 | | 57 | 283 | | 63 | 283 | | 34 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.67 | 0.68 | 0.69 | 0.67 | 0.68 | 0.69 | 0.56 | 0.56 | 0.62 |
| SE | 0.039 | 0.077 | 0.040 | 0.036 | 0.070 | 0.038 | 0.048 | 0.073 | 0.052 |
| p | 8.2E-6 | 0.020 | 1.1E-6 | 2.1E-6 | 0.012 | 3.2E-7 | 0.23 | 0.43 | 0.020 |
| nCohort 1 | 928 | 1232 | 817 | 928 | 1232 | 817 | 928 | 1232 | 817 |
| nCohort 2 | 62 | 15 | 57 | 70 | 18 | 63 | 39 | 17 | 34 |
| Cutoff 1 | 0.352 | 1.02 | 0.362 | 0.335 | 0.605 | 0.392 | 0.0109 | 0.0130 | 0.0174 |
| Sens 1 | 71% | 73% | 72% | 70% | 72% | 71% | 74% | 71% | 71% |
| Spec 1 | 58% | 64% | 58% | 58% | 58% | 59% | 29% | 33% | 47% |
| Cutoff 2 | 0.0126 | 0.0109 | 0.0193 | 0.0130 | 0.0109 | 0.0140 | 0.00930 | 0.00564 | 0.0109 |
| Sens 2 | 81% | 80% | 81% | 80% | 83% | 81% | 82% | 82% | 82% |
| Spec 2 | 34% | 25% | 48% | 36% | 25% | 37% | 28% | 13% | 27% |
| Cutoff 3 | 0.00930 | 0.00756 | 0.00930 | 0.00834 | 0.00821 | 0.0109 | 0.00564 | 0.00269 | 0.00930 |
| Sens 3 | 90% | 93% | 95% | 90% | 100% | 90% | 92% | 94% | 91% |
| Spec 3 | 28% | 15% | 26% | 25% | 19% | 27% | 15% | 2% | 26% |
| Cutoff 4 | 1.13 | 1.62 | 1.13 | 1.13 | 1.62 | 1.13 | 1.13 | 1.62 | 1.13 |
| Sens 4 | 56% | 67% | 60% | 56% | 67% | 59% | 44% | 47% | 50% |
| Spec 4 | 70% | 70% | 70% | 70% | 70% | 70% | 70% | 70% | 70% |
| Cutoff 5 | 2.67 | 3.52 | 2.66 | 2.67 | 3.52 | 2.66 | 2.67 | 3.52 | 2.66 |
| Sens 5 | 39% | 53% | 44% | 40% | 50% | 43% | 28% | 35% | 32% |
| Spec 5 | 80% | 80% | 80% | 80% | 80% | 80% | 80% | 80% | 80% |
| Cutoff 6 | 8.75 | 11.3 | 8.56 | 8.75 | 11.3 | 8.56 | 8.75 | 11.3 | 8.56 |
| Sens 6 | 26% | 33% | 28% | 26% | 22% | 30% | 15% | 18% | 21% |
| Spec 6 | 90% | 90% | 90% | 90% | 90% | 90% | 90% | 90% | 90% |
| OR Quart 2 | 2.7 | 0.33 | 2.0 | 1.9 | 0.66 | 1.5 | 1.4 | 0.20 | 3.1 |
| p Value | 0.065 | 0.34 | 0.20 | 0.18 | 0.65 | 0.43 | 0.47 | 0.14 | 0.096 |
| 95% CI of | 0.94 | 0.034 | 0.69 | 0.74 | 0.11 | 0.53 | 0.54 | 0.023 | 0.82 |
| OR Quart2 | 7.6 | 3.2 | 6.1 | 4.8 | 4.0 | 4.3 | 3.9 | 1.7 | 12 |
| OR Quart 3 | 3.4 | 0.66 | 3.1 | 2.9 | 0.66 | 3.6 | 1.1 | 0.60 | 2.7 |
| p Value | 0.020 | 0.65 | 0.029 | 0.020 | 0.66 | 0.0075 | 0.80 | 0.48 | 0.14 |
| 95% CI of | 1.2 | 0.11 | 1.1 | 1.2 | 0.11 | 1.4 | 0.41 | 0.14 | 0.71 |
| OR Quart3 | 9.3 | 4.0 | 8.8 | 6.9 | 4.0 | 9.1 | 3.2 | 2.5 | 10 |
| OR Quart 4 | 6.2 | 3.0 | 6.0 | 4.9 | 3.8 | 5.2 | 2.1 | 1.6 | 4.9 |
| p Value | 2.4E-4 | 0.097 | 3.2E-4 | 2.1E-4 | 0.044 | 3.4E-4 | 0.13 | 0.41 | 0.014 |
| 95% CI of | 2.3 | 0.82 | 2.3 | 2.1 | 1.0 | 2.1 | 0.81 | 0.52 | 1.4 |
| OR Quart4 | 16 | 11 | 16 | 11 | 14 | 13 | 5.2 | 5.0 | 17 |

| **Insulin-like growth factor-binding protein 3** | | | | | | | | | |
|---|---|---|---|---|---|---|---|---|---|
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 355 | | 654 | 355 | | 566 | 355 | | 559 |
| Average | 611 | | 1050 | 611 | | 1210 | 611 | | 688 |
| Stdev | 946 | | 1310 | 946 | | 2170 | 946 | | 574 |
| p(t-test) | | | 3.3E-4 | | | 2.2E-6 | | | 0.59 |
| Min | 0.0478 | | 17.3 | 0.0478 | | 32.5 | 0.0478 | | 0.311 |
| Max | 12500 | | 7720 | 12500 | | 12500 | 12500 | | 2100 |
| n (Samp) | 1005 | | 70 | 1005 | | 80 | 1005 | | 46 |
| n (Patient) | 386 | | 70 | 386 | | 80 | 386 | | 46 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 408 | | 542 | 408 | | 618 | 408 | | 540 |
| Average | 715 | | 1100 | 715 | | 1280 | 715 | | 797 |
| Stdev | 1130 | | 1190 | 1130 | | 1880 | 1130 | | 745 |
| p(t-test) | | | 0.17 | | | 0.024 | | | 0.74 |
| Min | 0.0478 | | 38.8 | 0.0478 | | 44.3 | 0.0478 | | 44.1 |
| Max | 12500 | | 4040 | 12500 | | 8160 | 12500 | | 2990 |
| n (Samp) | 1341 | | 16 | 1341 | | 21 | 1341 | | 20 |
| n (Patient) | 472 | | 16 | 472 | | 21 | 472 | | 20 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 367 | | 713 | 367 | | 646 | 367 | | 701 |
| Average | 630 | | 1320 | 630 | | 1340 | 630 | | 812 |
| Stdev | 1030 | | 1980 | 1030 | | 2260 | 1030 | | 675 |
| p(t-test) | | | 1.7E-6 | | | 6.5E-7 | | | 0.27 |
| Min | 0.0478 | | 17.3 | 0.0478 | | 32.5 | 0.0478 | | 0.311 |
| Max | 12500 | | 12500 | 12500 | | 12500 | 12500 | | 2870 |
| n (Samp) | 897 | | 65 | 897 | | 73 | 897 | | 40 |
| n (Patient) | 310 | | 65 | 310 | | 73 | 310 | | 40 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.65 | 0.61 | 0.67 | 0.62 | 0.62 | 0.64 | 0.57 | 0.58 | 0.61 |
| SE | 0.037 | 0.075 | 0.038 | 0.035 | 0.066 | 0.036 | 0.045 | 0.067 | 0.048 |
| p | 3.7E-5 | 0.13 | 4.9E-6 | 3.5E-4 | 0.080 | 6.4E-5 | 0.097 | 0.22 | 0.023 |
| nCohort 1 | 1005 | 1341 | 897 | 1005 | 1341 | 897 | 1005 | 1341 | 897 |
| nCohort 2 | 70 | 16 | 65 | 80 | 21 | 73 | 46 | 20 | 40 |
| Cutoff 1 | 408 | 212 | 411 | 313 | 287 | 319 | 201 | 294 | 271 |
| Sens 1 | 70% | 75% | 71% | 70% | 71% | 71% | 72% | 70% | 70% |
| Spec 1 | 54% | 33% | 53% | 46% | 40% | 45% | 35% | 41% | 40% |
| Cutoff 2 | 259 | 210 | 262 | 205 | 259 | 245 | 138 | 249 | 191 |
| Sens 2 | 80% | 81% | 80% | 80% | 81% | 81% | 80% | 80% | 80% |
| Spec 2 | 42% | 33% | 40% | 35% | 38% | 38% | 27% | 37% | 31% |
| Cutoff 3 | 118 | 163 | 118 | 114 | 163 | 118 | 43.4 | 139 | 52.6 |
| Sens 3 | 90% | 94% | 91% | 90% | 90% | 90% | 91% | 90% | 90% |
| Spec 3 | 24% | 26% | 21% | 23% | 26% | 21% | 8% | 24% | 9% |
| Cutoff 4 | 651 | 746 | 645 | 651 | 746 | 645 | 651 | 746 | 645 |
| Sens 4 | 51% | 44% | 57% | 45% | 43% | 51% | 46% | 45% | 50% |
| Spec 4 | 70% | 70% | 70% | 70% | 70% | 70% | 70% | 70% | 70% |
| Cutoff 5 | 922 | 1040 | 926 | 922 | 1040 | 926 | 922 | 1040 | 926 |
| Sens 5 | 33% | 38% | 37% | 35% | 43% | 38% | 33% | 30% | 40% |
| Spec 5 | 80% | 80% | 80% | 80% | 80% | 80% | 80% | 80% | 80% |
| Cutoff 6 | 1350 | 1540 | 1330 | 1350 | 1540 | 1330 | 1350 | 1540 | 1330 |
| Sens 6 | 23% | 19% | 26% | 22% | 19% | 27% | 15% | 10% | 22% |
| Spec 6 | 90% | 90% | 90% | 90% | 90% | 90% | 90% | 90% | 90% |
| OR Quart 2 | 1.4 | 4.0 | 1.00 | 1.8 | 2.5 | 1.6 | 1.00 | 2.0 | 1.1 |
| p Value | 0.49 | 0.21 | 0.99 | 0.14 | 0.27 | 0.23 | 0.99 | 0.32 | 0.79 |
| 95% CI of | 0.55 | 0.45 | 0.37 | 0.83 | 0.48 | 0.73 | 0.39 | 0.50 | 0.41 |
| OR Quart2 | 3.5 | 36 | 2.7 | 3.8 | 13 | 3.7 | 2.6 | 8.1 | 3.2 |
| OR Quart 3 | 2.9 | 4.0 | 2.5 | 1.8 | 2.5 | 1.8 | 1.1 | 1.3 | 0.85 |
| p Value | 0.012 | 0.21 | 0.034 | 0.14 | 0.27 | 0.17 | 0.82 | 0.70 | 0.78 |
| 95% CI of | 1.3 | 0.45 | 1.1 | 0.83 | 0.49 | 0.79 | 0.44 | 0.30 | 0.28 |
| OR Quart3 | 6.6 | 36 | 5.8 | 3.8 | 13 | 3.9 | 2.8 | 6.0 | 2.6 |
| OR Quart 4 | 3.9 | 7.1 | 4.1 | 3.0 | 4.6 | 3.3 | 2.1 | 2.4 | 2.9 |
| p Value | 8.3E-4 | 0.067 | 5.3E-4 | 0.0021 | 0.053 | 0.0017 | 0.083 | 0.22 | 0.020 |
| 95% CI of | 1.8 | 0.87 | 1.8 | 1.5 | 0.98 | 1.6 | 0.91 | 0.60 | 1.2 |
| OR Quart4 | 8.8 | 58 | 9.2 | 6.2 | 21 | 6.8 | 4.7 | 9.2 | 6.9 |

| | | | | | | | | | |
|---|---|---|---|---|---|---|---|---|---|
| **Immunoglogulin G1** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 3060 | | 5910 | 3060 | | 7970 | 3060 | | 4550 |
| Average | 5430 | | 8350 | 5430 | | 10300 | 5430 | | 6440 |
| Stdev | 6740 | | 7390 | 6740 | | 11400 | 6740 | | 6380 |
| p(t-test) | | | 0.0011 | | | 6.3E-8 | | | 0.36 |
| Min | 59.7 | | 447 | 59.7 | | 634 | 59.7 | | 55.9 |
| Max | 80000 | | 35700 | 80000 | | 80000 | 80000 | | 25300 |
| n (Samp) | 922 | | 62 | 922 | | 70 | 922 | | 39 |
| n (Patient) | 358 | | 62 | 358 | | 70 | 358 | | 39 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 3410 | | 5220 | 3410 | | 10900 | 3410 | | 9840 |
| Average | 6040 | | 9080 | 6040 | | 10000 | 6040 | | 9430 |
| Stdev | 7380 | | 7850 | 7380 | | 8360 | 7380 | | 7740 |
| p(t-test) | | | 0.11 | | | 0.023 | | | 0.060 |
| Min | 3.36 | | 504 | 3.36 | | 658 | 3.36 | | 177 |
| Max | 80000 | | 24700 | 80000 | | 26100 | 80000 | | 23800 |
| n (Samp) | 1225 | | 15 | 1225 | | 18 | 1225 | | 17 |
| n (Patient) | 438 | | 15 | 438 | | 18 | 438 | | 17 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 3060 | | 6850 | 3060 | | 8300 | 3060 | | 5080 |
| Average | 5550 | | 9370 | 5550 | | 11400 | 5550 | | 7220 |
| Stdev | 6860 | | 7890 | 6860 | | 12000 | 6860 | | 6330 |
| p(t-test) | | | 6.2E-5 | | | 2.1E-9 | | | 0.16 |
| Min | 33.0 | | 447 | 33.0 | | 634 | 33.0 | | 55.9 |
| Max | 80000 | | 35700 | 80000 | | 80000 | 80000 | | 25300 |
| n (Samp) | 810 | | 57 | 810 | | 63 | 810 | | 34 |
| n (Patient) | 280 | | 57 | 280 | | 63 | 280 | | 34 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.66 | 0.64 | 0.69 | 0.67 | 0.61 | 0.71 | 0.55 | 0.60 | 0.61 |
| SE | 0.039 | 0.078 | 0.040 | 0.036 | 0.071 | 0.038 | 0.048 | 0.073 | 0.052 |
| p | 3.4E-5 | 0.068 | 3.2E-6 | 2.5E-6 | 0.11 | 2.0E-8 | 0.35 | 0.17 | 0.031 |
| nCohort 1 | 922 | 1225 | 810 | 922 | 1225 | 810 | 922 | 1225 | 810 |
| nCohort 2 | 62 | 15 | 57 | 70 | 18 | 63 | 39 | 17 | 34 |
| Cutoff 1 | 3550 | 3550 | 4050 | 4350 | 2320 | 4540 | 1740 | 1740 | 2930 |
| Sens 1 | 71% | 73% | 70% | 70% | 72% | 71% | 72% | 71% | 71% |
| Spec 1 | 57% | 52% | 61% | 64% | 34% | 65% | 27% | 25% | 48% |
| Cutoff 2 | 2720 | 2920 | 2720 | 2010 | 1090 | 2630 | 1390 | 831 | 1740 |
| Sens 2 | 81% | 80% | 81% | 80% | 83% | 81% | 82% | 82% | 82% |
| Spec 2 | 45% | 43% | 46% | 33% | 13% | 44% | 20% | 9% | 28% |
| Cutoff 3 | 1130 | 933 | 1880 | 1190 | 673 | 1600 | 541 | 541 | 1390 |
| Sens 3 | 90% | 93% | 91% | 90% | 94% | 90% | 92% | 94% | 91% |
| Spec 3 | 15% | 11% | 30% | 16% | 6% | 24% | 5% | 5% | 21% |
| Cutoff 4 | 5430 | 6270 | 5590 | 5430 | 6270 | 5590 | 5430 | 6270 | 5590 |
| Sens 4 | 52% | 47% | 58% | 60% | 56% | 62% | 44% | 59% | 47% |
| Spec 4 | 70% | 70% | 70% | 70% | 70% | 70% | 70% | 70% | 70% |
| Cutoff 5 | 8100 | 8950 | 8470 | 8100 | 8950 | 8470 | 8100 | 8950 | 8470 |
| Sens 5 | 42% | 33% | 44% | 49% | 56% | 49% | 31% | 53% | 32% |
| Spec 5 | 80% | 80% | 80% | 80% | 80% | 80% | 80% | 80% | 80% |
| Cutoff 6 | 13100 | 14200 | 13400 | 13100 | 14200 | 13400 | 13100 | 14200 | 13400 |
| Sens 6 | 18% | 27% | 23% | 31% | 44% | 32% | 18% | 29% | 21% |
| Spec 6 | 90% | 90% | 90% | 90% | 90% | 90% | 90% | 90% | 90% |
| OR Quart 2 | 1.4 | 1.0 | 1.8 | 0.57 | 1.00 | 0.62 | 0.69 | 0.20 | 0.83 |
| p Value | 0.46 | 1.0 | 0.29 | 0.25 | 1.00 | 0.40 | 0.46 | 0.14 | 0.76 |
| 95% CI of | 0.54 | 0.14 | 0.60 | 0.22 | 0.25 | 0.20 | 0.26 | 0.023 | 0.25 |
| OR Quart2 | 3.9 | 7.1 | 5.5 | 1.5 | 4.0 | 1.9 | 1.8 | 1.7 | 2.8 |
| OR Quart 3 | 2.7 | 2.5 | 3.1 | 1.2 | 0 | 1.9 | 0.90 | 0.20 | 1.9 |
| p Value | 0.029 | 0.27 | 0.030 | 0.69 | na | 0.14 | 0.81 | 0.14 | 0.22 |
| 95% CI of | 1.1 | 0.49 | 1.1 | 0.53 | na | 0.80 | 0.36 | 0.023 | 0.68 |
| OR Quart3 | 6.6 | 13 | 8.8 | 2.6 | na | 4.7 | 2.2 | 1.7 | 5.2 |
| OR Quart 4 | 4.2 | 3.0 | 6.3 | 3.4 | 2.5 | 5.0 | 1.3 | 2.0 | 2.1 |
| p Value | 9.4E-4 | 0.18 | 2.2E-4 | 3.4E-4 | 0.12 | 7.1E-5 | 0.53 | 0.20 | 0.16 |
| 95% CI of | 1.8 | 0.61 | 2.4 | 1.8 | 0.79 | 2.3 | 0.56 | 0.68 | 0.76 |
| OR Quart4 | 9.9 | 15 | 17 | 6.8 | 8.2 | 11 | 3.1 | 6.0 | 5.6 |

| **Immunoglogulin G2** | | | | | | | | | |
|---|---|---|---|---|---|---|---|---|---|
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 8730 | | 13800 | 8730 | | 16800 | 8730 | | 8880 |
| Average | 16700 | | 27200 | 16700 | | 38600 | 16700 | | 23800 |
| Stdev | 28100 | | 43200 | 28100 | | 60500 | 28100 | | 43900 |
| p(t-test) | | | 0.0063 | | | 2.5E-8 | | | 0.13 |
| Min | 119 | | 810 | 119 | | 1870 | 119 | | 25.4 |
| Max | 240000 | | 240000 | 240000 | | 240000 | 240000 | | 240000 |
| n (Samp) | 922 | | 62 | 922 | | 70 | 922 | | 39 |
| n (Patient) | 358 | | 62 | 358 | | 70 | 358 | | 39 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 9310 | | 11800 | 9310 | | 16700 | 9310 | | 40000 |
| Average | 18900 | | 26000 | 18900 | | 39600 | 18900 | | 50100 |
| Stdev | 33500 | | 44100 | 33500 | | 59100 | 33500 | | 64700 |
| p(t-test) | | | 0.41 | | | 0.010 | | | 1.8E-4 |
| Min | 119 | | 3150 | 119 | | 3190 | 119 | | 1310 |
| Max | 240000 | | 182000 | 240000 | | 240000 | 240000 | | 240000 |
| n (Samp) | 1225 | | 15 | 1225 | | 18 | 1225 | | 17 |
| n (Patient) | 438 | | 15 | 438 | | 18 | 438 | | 17 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 9320 | | 14200 | 9320 | | 18200 | 9320 | | 9690 |
| Average | 17600 | | 34200 | 17600 | | 42800 | 17600 | | 23600 |
| Stdev | 28600 | | 53700 | 28600 | | 63300 | 28600 | | 43000 |
| p(t-test) | | | 9.3E-5 | | | 3.9E-9 | | | 0.24 |
| Min | 334 | | 810 | 334 | | 1870 | 334 | | 25.4 |
| Max | 240000 | | 240000 | 240000 | | 240000 | 240000 | | 240000 |
| n (Samp) | 810 | | 57 | 810 | | 63 | 810 | | 34 |
| n (Patient) | 280 | | 57 | 280 | | 63 | 280 | | 34 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.63 | 0.61 | 0.63 | 0.65 | 0.62 | 0.67 | 0.51 | 0.63 | 0.54 |
| SE | 0.039 | 0.078 | 0.041 | 0.037 | 0.071 | 0.038 | 0.047 | 0.073 | 0.052 |
| p | 8.5E-4 | 0.15 | 9.9E-4 | 3.0E-5 | 0.090 | 1.2E-5 | 0.90 | 0.075 | 0.44 |
| nCohort 1 | 922 | 1225 | 810 | 922 | 1225 | 810 | 922 | 1225 | 810 |
| nCohort 2 | 62 | 15 | 57 | 70 | 18 | 63 | 39 | 17 | 34 |
| Cutoff 1 | 8220 | 8900 | 8240 | 8630 | 6340 | 9450 | 5240 | 6690 | 8010 |
| Sens 1 | 71% | 73% | 70% | 70% | 72% | 71% | 72% | 71% | 71% |
| Spec 1 | 47% | 48% | 45% | 50% | 33% | 51% | 28% | 36% | 44% |
| Cutoff 2 | 6900 | 8400 | 6900 | 5620 | 4630 | 6460 | 4240 | 4240 | 4620 |
| Sens 2 | 81% | 80% | 81% | 80% | 83% | 81% | 82% | 82% | 82% |
| Spec 2 | 39% | 45% | 38% | 31% | 22% | 35% | 21% | 20% | 21% |
| Cutoff 3 | 4910 | 5870 | 4400 | 3990 | 3980 | 4130 | 1800 | 1800 | 2270 |
| Sens 3 | 90% | 93% | 91% | 90% | 94% | 90% | 92% | 94% | 91% |
| Spec 3 | 25% | 30% | 20% | 20% | 18% | 19% | 6% | 5% | 8% |
| Cutoff 4 | 14400 | 15700 | 14900 | 14400 | 15700 | 14900 | 14400 | 15700 | 14900 |
| Sens 4 | 45% | 40% | 47% | 53% | 50% | 54% | 28% | 59% | 32% |
| Spec 4 | 70% | 70% | 70% | 70% | 70% | 70% | 70% | 70% | 70% |
| Cutoff 5 | 19700 | 21200 | 20100 | 19700 | 21200 | 20100 | 19700 | 21200 | 20100 |
| Sens 5 | 44% | 33% | 40% | 43% | 44% | 43% | 23% | 59% | 26% |
| Spec 5 | 80% | 80% | 80% | 80% | 80% | 80% | 80% | 80% | 80% |
| Cutoff 6 | 32100 | 37400 | 35300 | 32100 | 37400 | 35300 | 32100 | 37400 | 35300 |
| Sens 6 | 19% | 7% | 25% | 31% | 28% | 30% | 18% | 53% | 15% |
| Spec 6 | 90% | 90% | 90% | 90% | 90% | 90% | 90% | 90% | 90% |
| OR Quart 2 | 2.5 | 5.1 | 1.3 | 0.91 | 0.75 | 1.1 | 0.63 | 0.75 | 1.0 |
| p Value | 0.043 | 0.14 | 0.51 | 0.83 | 0.70 | 0.81 | 0.34 | 0.70 | 1.0 |
| 95% CI of | 1.0 | 0.59 | 0.56 | 0.39 | 0.17 | 0.44 | 0.24 | 0.17 | 0.37 |
| OR Quart2 | 6.2 | 44 | 3.3 | 2.1 | 3.4 | 2.8 | 1.6 | 3.4 | 2.7 |
| OR Quart 3 | 1.6 | 3.0 | 1.1 | 1.1 | 0.50 | 1.5 | 1.1 | 0 | 1.1 |
| p Value | 0.34 | 0.34 | 0.82 | 0.84 | 0.42 | 0.38 | 0.83 | na | 0.80 |
| 95% CI of | 0.61 | 0.31 | 0.44 | 0.49 | 0.090 | 0.62 | 0.47 | na | 0.43 |
| OR Quart3 | 4.2 | 29 | 2.8 | 2.4 | 2.7 | 3.5 | 2.5 | na | 3.0 |
| OR Quart 4 | 4.2 | 6.1 | 3.1 | 3.1 | 2.3 | 3.8 | 0.81 | 2.5 | 1.1 |
| p Value | 9.4E-4 | 0.095 | 0.0043 | 0.0011 | 0.17 | 6.1E-4 | 0.64 | 0.12 | 0.80 |
| 95% CI of | 1.8 | 0.73 | 1.4 | 1.6 | 0.69 | 1.8 | 0.33 | 0.79 | 0.43 |
| OR Quart4 | 9.9 | 51 | 6.9 | 6.2 | 7.5 | 8.3 | 2.0 | 8.2 | 3.0 |

| | | | | | | | | | |
|---|---|---|---|---|---|---|---|---|---|
| **Interleukin-11** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 129 | | 241 | 129 | | 212 | 129 | | 120 |
| Average | 209 | | 341 | 209 | | 288 | 209 | | 207 |
| Stdev | 243 | | 387 | 243 | | 334 | 243 | | 338 |
| p(t-test) | | | 8.2E-5 | | | 0.012 | | | 0.97 |
| Min | 0.0822 | | 21.2 | 0.0822 | | 28.3 | 0.0822 | | 14.5 |
| Max | 2260 | | 2900 | 2260 | | 2590 | 2260 | | 2140 |
| n (Samp) | 929 | | 62 | 929 | | 69 | 929 | | 39 |
| n (Patient) | 361 | | 62 | 361 | | 69 | 361 | | 39 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 135 | | 158 | 135 | | 224 | 135 | | 174 |
| Average | 231 | | 256 | 231 | | 258 | 231 | | 192 |
| Stdev | 303 | | 226 | 303 | | 144 | 303 | | 127 |
| p(t-test) | | | 0.75 | | | 0.71 | | | 0.59 |
| Min | 0.0822 | | 21.2 | 0.0822 | | 32.3 | 0.0822 | | 35.3 |
| Max | 3780 | | 830 | 3780 | | 547 | 3780 | | 549 |
| n (Samp) | 1232 | | 15 | 1232 | | 18 | 1232 | | 17 |
| n (Patient) | 441 | | 15 | 441 | | 18 | 441 | | 17 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 139 | | 285 | 139 | | 210 | 139 | | 121 |
| Average | 217 | | 374 | 217 | | 307 | 217 | | 221 |
| Stdev | 246 | | 416 | 246 | | 374 | 246 | | 360 |
| p(t-test) | | | 1.2E-5 | | | 0.0083 | | | 0.94 |
| Min | 0.0822 | | 27.2 | 0.0822 | | 28.3 | 0.0822 | | 14.5 |
| Max | 2260 | | 2900 | 2260 | | 2590 | 2260 | | 2140 |
| n (Samp) | 817 | | 57 | 817 | | 62 | 817 | | 34 |
| n (Patient) | 283 | | 57 | 283 | | 62 | 283 | | 34 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.68 | 0.58 | 0.69 | 0.64 | 0.65 | 0.62 | 0.50 | 0.54 | 0.49 |
| SE | 0.038 | 0.078 | 0.040 | 0.037 | 0.071 | 0.039 | 0.047 | 0.072 | 0.051 |
| p | 2.4E-6 | 0.33 | 2.1E-6 | 1.6E-4 | 0.033 | 0.0015 | 0.95 | 0.55 | 0.79 |
| nCohort 1 | 929 | 1232 | 817 | 929 | 1232 | 817 | 929 | 1232 | 817 |
| nCohort 2 | 62 | 15 | 57 | 69 | 18 | 62 | 39 | 17 | 34 |
| Cutoff 1 | 149 | 136 | 158 | 123 | 182 | 123 | 88.1 | 135 | 88.1 |
| Sens 1 | 71% | 73% | 70% | 71% | 72% | 71% | 72% | 71% | 71% |
| Spec 1 | 57% | 50% | 56% | 49% | 62% | 45% | 35% | 50% | 31% |
| Cutoff 2 | 119 | 87.1 | 128 | 101 | 105 | 101 | 71.2 | 93.8 | 74.7 |
| Sens 2 | 81% | 80% | 81% | 81% | 83% | 81% | 82% | 82% | 82% |
| Spec 2 | 47% | 32% | 46% | 40% | 39% | 37% | 26% | 35% | 24% |
| Cutoff 3 | 79.8 | 63.1 | 85.3 | 76.3 | 89.4 | 79.6 | 54.9 | 41.4 | 62.9 |
| Sens 3 | 90% | 93% | 91% | 91% | 94% | 90% | 92% | 94% | 91% |
| Spec 3 | 31% | 20% | 29% | 28% | 33% | 28% | 18% | 10% | 19% |
| Cutoff 4 | 212 | 230 | 230 | 212 | 230 | 230 | 212 | 230 | 230 |
| Sens 4 | 55% | 33% | 54% | 51% | 44% | 45% | 26% | 29% | 26% |
| Spec 4 | 70% | 70% | 70% | 70% | 70% | 70% | 70% | 70% | 70% |
| Cutoff 5 | 301 | 328 | 307 | 301 | 328 | 307 | 301 | 328 | 307 |
| Sens 5 | 42% | 33% | 44% | 33% | 33% | 34% | 13% | 12% | 15% |
| Spec 5 | 80% | 80% | 80% | 80% | 80% | 80% | 80% | 80% | 80% |
| Cutoff 6 | 461 | 509 | 460 | 461 | 509 | 460 | 461 | 509 | 460 |
| Sens 6 | 26% | 13% | 28% | 13% | 6% | 15% | 5% | 6% | 6% |
| Spec 6 | 90% | 90% | 90% | 90% | 90% | 90% | 90% | 90% | 90% |
| OR Quart 2 | 2.8 | 1.00 | 4.2 | 2.6 | 3.0 | 2.8 | 1.6 | 1.0 | 1.3 |
| p Value | 0.079 | 1.00 | 0.029 | 0.053 | 0.34 | 0.036 | 0.34 | 1.0 | 0.59 |
| 95% CI of | 0.89 | 0.14 | 1.2 | 0.99 | 0.31 | 1.1 | 0.61 | 0.20 | 0.46 |
| OR Quart2 | 9.0 | 7.1 | 15 | 6.8 | 29 | 7.3 | 4.2 | 5.0 | 3.9 |
| OR Quart 3 | 4.8 | 3.0 | 4.5 | 3.7 | 8.2 | 3.0 | 2.1 | 2.4 | 2.2 |
| p Value | 0.0054 | 0.18 | 0.019 | 0.0053 | 0.048 | 0.025 | 0.13 | 0.22 | 0.11 |
| 95% CI of | 1.6 | 0.61 | 1.3 | 1.5 | 1.0 | 1.1 | 0.82 | 0.61 | 0.84 |
| OR Quart3 | 14 | 15 | 16 | 9.4 | 66 | 7.7 | 5.2 | 9.2 | 6.0 |
| OR Quart 4 | 8.0 | 2.5 | 11 | 4.9 | 6.1 | 4.1 | 1.0 | 1.3 | 1.2 |
| p Value | 1.2E-4 | 0.27 | 9.9E-5 | 5.6E-4 | 0.096 | 0.0024 | 1.0 | 0.71 | 0.77 |
| 95% CI of | 2.8 | 0.48 | 3.3 | 2.0 | 0.73 | 1.7 | 0.35 | 0.30 | 0.39 |
| OR Quart4 | 23 | 13 | 36 | 12 | 51 | 10 | 2.9 | 6.0 | 3.6 |

| **Interleukin-2 receptor alpha chain** | | | | | | | | | |
|---|---|---|---|---|---|---|---|---|---|
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 554 | | 923 | 554 | | 868 | 554 | | 524 |
| Average | 909 | | 1440 | 909 | | 1430 | 909 | | 1190 |
| Stdev | 1090 | | 1520 | 1090 | | 1780 | 1090 | | 1700 |
| p(t-test) | | | 3.1E-4 | | | 3.0E-4 | | | 0.12 |
| Min | 0.0317 | | 0.0482 | 0.0317 | | 0.0482 | 0.0317 | | 0.0383 |
| Max | 10400 | | 6080 | 10400 | | 9090 | 10400 | | 7740 |
| n (Samp) | 930 | | 62 | 930 | | 70 | 930 | | 39 |
| n (Patient) | 361 | | 62 | 361 | | 70 | 361 | | 39 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 621 | | 499 | 621 | | 957 | 621 | | 421 |
| Average | 1100 | | 1150 | 1100 | | 1680 | 1100 | | 881 |
| Stdev | 1420 | | 1520 | 1420 | | 1930 | 1420 | | 923 |
| p(t-test) | | | 0.89 | | | 0.086 | | | 0.54 |
| Min | 0.0317 | | 0.0482 | 0.0317 | | 0.0482 | 0.0317 | | 51.4 |
| Max | 10400 | | 6080 | 10400 | | 7190 | 10400 | | 2590 |
| n (Samp) | 1234 | | 15 | 1234 | | 18 | 1234 | | 17 |
| n (Patient) | 441 | | 15 | 441 | | 18 | 441 | | 17 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 563 | | 1080 | 563 | | 911 | 563 | | 728 |
| Average | 927 | | 1640 | 927 | | 1570 | 927 | | 1380 |
| Stdev | 1110 | | 1540 | 1110 | | 1840 | 1110 | | 1780 |
| p(t-test) | | | 5.2E-6 | | | 2.7E-5 | | | 0.022 |
| Min | 0.0317 | | 0.249 | 0.0317 | | 0.0632 | 0.0317 | | 0.0383 |
| Max | 10400 | | 6080 | 10400 | | 9090 | 10400 | | 7740 |
| n (Samp) | 819 | | 57 | 819 | | 63 | 819 | | 34 |
| n (Patient) | 283 | | 57 | 283 | | 63 | 283 | | 34 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.61 | 0.51 | 0.66 | 0.60 | 0.58 | 0.62 | 0.51 | 0.48 | 0.55 |
| SE | 0.039 | 0.076 | 0.040 | 0.037 | 0.071 | 0.039 | 0.048 | 0.071 | 0.052 |
| p | 0.0042 | 0.85 | 1.0E-4 | 0.0093 | 0.24 | 0.0014 | 0.78 | 0.75 | 0.31 |
| nCohort 1 | 930 | 1234 | 819 | 930 | 1234 | 819 | 930 | 1234 | 819 |
| nCohort 2 | 62 | 15 | 57 | 70 | 18 | 63 | 39 | 17 | 34 |
| Cutoff 1 | 446 | 411 | 528 | 417 | 373 | 466 | 226 | 226 | 333 |
| Sens 1 | 71% | 73% | 70% | 70% | 72% | 71% | 72% | 71% | 71% |
| Spec 1 | 45% | 40% | 48% | 44% | 37% | 45% | 31% | 27% | 36% |
| Cutoff 2 | 278 | 277 | 368 | 272 | 235 | 318 | 135 | 135 | 189 |
| Sens 2 | 81% | 80% | 81% | 80% | 83% | 81% | 82% | 82% | 82% |
| Spec 2 | 35% | 31% | 39% | 34% | 28% | 35% | 21% | 18% | 26% |
| Cutoff 3 | 112 | 21.0 | 167 | 109 | 55.0 | 152 | 35.7 | 66.8 | 35.7 |
| Sens 3 | 90% | 93% | 91% | 90% | 94% | 90% | 92% | 94% | 91% |
| Spec 3 | 18% | 8% | 24% | 17% | 11% | 23% | 11% | 12% | 10% |
| Cutoff 4 | 1050 | 1150 | 1060 | 1050 | 1150 | 1060 | 1050 | 1150 | 1060 |
| Sens 4 | 44% | 40% | 51% | 39% | 50% | 41% | 28% | 24% | 35% |
| Spec 4 | 70% | 70% | 70% | 70% | 70% | 70% | 70% | 70% | 70% |
| Cutoff 5 | 1490 | 1740 | 1500 | 1490 | 1740 | 1500 | 1490 | 1740 | 1500 |
| Sens 5 | 34% | 13% | 40% | 31% | 33% | 35% | 21% | 24% | 29% |
| Spec 5 | 80% | 80% | 80% | 80% | 80% | 80% | 80% | 80% | 80% |
| Cutoff 6 | 2250 | 2620 | 2290 | 2250 | 2620 | 2290 | 2250 | 2620 | 2290 |
| Sens 6 | 23% | 7% | 28% | 17% | 17% | 21% | 18% | 0% | 21% |
| Spec 6 | 90% | 90% | 90% | 90% | 90% | 90% | 90% | 90% | 90% |
| OR Quart 2 | 2.3 | 1.7 | 2.2 | 1.9 | 1.3 | 2.4 | 1.2 | 0.75 | 1.7 |
| p Value | 0.050 | 0.48 | 0.11 | 0.12 | 0.70 | 0.062 | 0.65 | 0.70 | 0.31 |
| 95% CI of | 1.0 | 0.40 | 0.84 | 0.84 | 0.30 | 0.96 | 0.50 | 0.17 | 0.61 |
| OR Quart2 | 5.5 | 7.1 | 6.0 | 4.1 | 6.0 | 5.9 | 3.0 | 3.4 | 4.8 |
| OR Quart 3 | 1.7 | 0.66 | 2.2 | 2.0 | 1.0 | 2.7 | 1.0 | 1.5 | 1.0 |
| p Value | 0.27 | 0.66 | 0.11 | 0.090 | 1.0 | 0.029 | 1.0 | 0.53 | 1.0 |
| 95% CI of | 0.68 | 0.11 | 0.84 | 0.90 | 0.20 | 1.1 | 0.39 | 0.42 | 0.32 |
| OR Quart3 | 4.1 | 4.0 | 6.0 | 4.3 | 5.0 | 6.6 | 2.6 | 5.4 | 3.2 |
| OR Quart 4 | 3.1 | 1.7 | 4.6 | 2.4 | 2.7 | 3.4 | 1.1 | 1.0 | 2.0 |
| p Value | 0.0077 | 0.48 | 0.0011 | 0.023 | 0.14 | 0.0064 | 0.82 | 1.00 | 0.16 |
| 95% CI of | 1.3 | 0.40 | 1.8 | 1.1 | 0.71 | 1.4 | 0.44 | 0.25 | 0.75 |
| OR Quart4 | 7.0 | 7.1 | 11 | 5.2 | 10 | 8.0 | 2.8 | 4.0 | 5.6 |

| | | | | | | | | | |
|---|---|---|---|---|---|---|---|---|---|
| **Neutrophil collagenase** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 3070 | | 6050 | 3070 | | 8280 | 3070 | | 3260 |
| Average | 13400 | | 18500 | 13400 | | 34000 | 13400 | | 9350 |
| Stdev | 38800 | | 30300 | 38800 | | 83200 | 38800 | | 15200 |
| p(t-test) | | | 0.29 | | | 5.1E-5 | | | 0.48 |
| Min | 0.114 | | 0.159 | 0.114 | | 25.8 | 0.114 | | 111 |
| Max | 670000 | | 136000 | 670000 | | 670000 | 670000 | | 66900 |
| n (Samp) | 1005 | | 69 | 1005 | | 80 | 1005 | | 45 |
| n (Patient) | 387 | | 69 | 387 | | 80 | 387 | | 45 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 3570 | | 4670 | 3570 | | 6260 | 3570 | | 5710 |
| Average | 14700 | | 20300 | 14700 | | 69300 | 14700 | | 17200 |
| Stdev | 41000 | | 40500 | 41000 | | 157000 | 41000 | | 27500 |
| p(t-test) | | | 0.59 | | | 4.2E-8 | | | 0.79 |
| Min | 0.114 | | 0.260 | 0.114 | | 25.8 | 0.114 | | 111 |
| Max | 670000 | | 128000 | 670000 | | 625000 | 670000 | | 115000 |
| n (Samp) | 1339 | | 16 | 1339 | | 21 | 1339 | | 19 |
| n (Patient) | 473 | | 16 | 473 | | 21 | 473 | | 19 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 3120 | | 7870 | 3120 | | 10400 | 3120 | | 3470 |
| Average | 13700 | | 31600 | 13700 | | 42800 | 13700 | | 10000 |
| Stdev | 35000 | | 78300 | 35000 | | 94000 | 35000 | | 15600 |
| p(t-test) | | | 4.5E-4 | | | 2.0E-8 | | | 0.51 |
| Min | 0.114 | | 0.159 | 0.114 | | 0.260 | 0.114 | | 25.8 |
| Max | 365000 | | 561000 | 365000 | | 670000 | 365000 | | 66900 |
| n (Samp) | 896 | | 64 | 896 | | 73 | 896 | | 40 |
| n (Patient) | 311 | | 64 | 311 | | 73 | 311 | | 40 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.64 | 0.52 | 0.67 | 0.66 | 0.57 | 0.70 | 0.50 | 0.57 | 0.52 |
| SE | 0.037 | 0.074 | 0.038 | 0.034 | 0.065 | 0.035 | 0.044 | 0.069 | 0.047 |
| p | 1.9E-4 | 0.75 | 1.2E-5 | 2.1E-6 | 0.32 | 2.4E-8 | 0.92 | 0.34 | 0.70 |
| nCohort 1 | 1005 | 1339 | 896 | 1005 | 1339 | 896 | 1005 | 1339 | 896 |
| nCohort 2 | 69 | 16 | 64 | 80 | 21 | 73 | 45 | 19 | 40 |
| Cutoff 1 | 3000 | 1060 | 3260 | 3320 | 1020 | 4640 | 1270 | 2660 | 1680 |
| Sens 1 | 71% | 75% | 70% | 70% | 71% | 71% | 71% | 74% | 70% |
| Spec 1 | 50% | 23% | 51% | 52% | 22% | 59% | 30% | 43% | 36% |
| Cutoff 2 | 2400 | 1050 | 2830 | 2190 | 978 | 3080 | 742 | 751 | 813 |
| Sens 2 | 81% | 81% | 81% | 80% | 81% | 81% | 80% | 84% | 80% |
| Spec 2 | 45% | 23% | 48% | 43% | 22% | 50% | 20% | 18% | 21% |
| Cutoff 3 | 1050 | 358 | 1720 | 962 | 570 | 1550 | 387 | 191 | 571 |
| Sens 3 | 91% | 94% | 91% | 90% | 90% | 90% | 91% | 95% | 90% |
| Spec 3 | 25% | 9% | 36% | 24% | 14% | 34% | 12% | 4% | 16% |
| Cutoff 4 | 7950 | 9120 | 7860 | 7950 | 9120 | 7860 | 7950 | 9120 | 7860 |
| Sens 4 | 45% | 38% | 50% | 52% | 43% | 58% | 27% | 47% | 30% |
| Spec 4 | 70% | 70% | 70% | 70% | 70% | 70% | 70% | 70% | 70% |
| Cutoff 5 | 13800 | 15700 | 13900 | 13800 | 15700 | 13900 | 13800 | 15700 | 13900 |
| Sens 5 | 33% | 19% | 36% | 39% | 33% | 41% | 20% | 32% | 22% |
| Spec 5 | 80% | 80% | 80% | 80% | 80% | 80% | 80% | 80% | 80% |
| Cutoff 6 | 29000 | 32400 | 30900 | 29000 | 32400 | 30900 | 29000 | 32400 | 30900 |
| Sens 6 | 17% | 12% | 19% | 26% | 24% | 29% | 9% | 16% | 10% |
| Spec 6 | 90% | 90% | 90% | 90% | 90% | 90% | 90% | 90% | 90% |
| OR Quart 2 | 2.4 | 0.40 | 4.2 | 1.2 | 0.28 | 2.2 | 0.92 | 0.59 | 1.0 |
| p Value | 0.063 | 0.27 | 0.011 | 0.68 | 0.12 | 0.11 | 0.84 | 0.48 | 1.0 |
| 95% CI of | 0.95 | 0.076 | 1.4 | 0.52 | 0.058 | 0.83 | 0.40 | 0.14 | 0.39 |
| OR Quart2 | 5.8 | 2.1 | 13 | 2.7 | 1.4 | 6.0 | 2.1 | 2.5 | 2.6 |
| OR Quart 3 | 2.7 | 0.80 | 4.2 | 1.9 | 0.42 | 3.4 | 0.83 | 0.80 | 1.1 |
| p Value | 0.030 | 0.73 | 0.011 | 0.10 | 0.22 | 0.011 | 0.66 | 0.74 | 0.81 |
| 95% CI of | 1.1 | 0.21 | 1.4 | 0.88 | 0.11 | 1.3 | 0.35 | 0.21 | 0.45 |
| OR Quart3 | 6.5 | 3.0 | 13 | 4.0 | 1.7 | 8.5 | 1.9 | 3.0 | 2.8 |
| OR Quart 4 | 4.3 | 1.00 | 7.8 | 3.6 | 1.3 | 6.6 | 1.0 | 1.4 | 1.4 |
| p Value | 6.9E-4 | 1.00 | 1.6E-4 | 3.2E-4 | 0.61 | 2.9E-5 | 0.99 | 0.57 | 0.50 |
| 95% CI of | 1.9 | 0.29 | 2.7 | 1.8 | 0.48 | 2.7 | 0.44 | 0.44 | 0.56 |
| OR Quart4 | 10 | 3.5 | 23 | 7.2 | 3.5 | 16 | 2.3 | 4.5 | 3.3 |

| **Protransforming growth factor alpha** | | | | | | | | | |
|---|---|---|---|---|---|---|---|---|---|
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 5.81 | | 8.51 | 5.81 | | 10.3 | 5.81 | | 6.15 |
| Average | 10.7 | | 12.5 | 10.7 | | 16.0 | 10.7 | | 9.77 |
| Stdev | 22.4 | | 12.1 | 22.4 | | 19.8 | 22.4 | | 10.6 |
| p(t-test) | | | 0.54 | | | 0.057 | | | 0.80 |
| Min | 0.00184 | | 0.00603 | 0.00184 | | 0.00603 | 0.00184 | | 0.00454 |
| Max | 361 | | 58.8 | 361 | | 135 | 361 | | 52.3 |
| n (Samp) | 925 | | 62 | 925 | | 69 | 925 | | 39 |
| n (Patient) | 361 | | 62 | 361 | | 69 | 361 | | 39 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 6.55 | | 12.3 | 6.55 | | 13.2 | 6.55 | | 6.54 |
| Average | 11.4 | | 11.8 | 11.4 | | 16.9 | 11.4 | | 10.6 |
| Stdev | 21.7 | | 6.57 | 21.7 | | 16.6 | 21.7 | | 11.4 |
| p(t-test) | | | 0.93 | | | 0.28 | | | 0.89 |
| Min | 0.00184 | | 0.00603 | 0.00184 | | 0.00603 | 0.00184 | | 0.00574 |
| Max | 361 | | 27.7 | 361 | | 59.6 | 361 | | 45.2 |
| n (Samp) | 1228 | | 15 | 1228 | | 18 | 1228 | | 17 |
| n (Patient) | 441 | | 15 | 441 | | 18 | 441 | | 17 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 5.87 | | 8.48 | 5.87 | | 10.9 | 5.87 | | 6.31 |
| Average | 11.1 | | 13.7 | 11.1 | | 16.6 | 11.1 | | 10.8 |
| Stdev | 23.7 | | 14.0 | 23.7 | | 19.7 | 23.7 | | 11.4 |
| p(t-test) | | | 0.42 | | | 0.075 | | | 0.94 |
| Min | 0.00184 | | 0.0114 | 0.00184 | | 0.781 | 0.00184 | | 0.00454 |
| Max | 361 | | 63.1 | 361 | | 135 | 361 | | 52.3 |
| n (Samp) | 814 | | 57 | 814 | | 62 | 814 | | 34 |
| n (Patient) | 283 | | 57 | 283 | | 62 | 283 | | 34 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.62 | 0.66 | 0.63 | 0.65 | 0.63 | 0.67 | 0.53 | 0.53 | 0.54 |
| SE | 0.039 | 0.077 | 0.041 | 0.037 | 0.071 | 0.039 | 0.048 | 0.072 | 0.052 |
| p | 0.0018 | 0.035 | 0.0012 | 8.3E-5 | 0.071 | 6.1E-6 | 0.57 | 0.71 | 0.40 |
| nCohort 1 | 925 | 1228 | 814 | 925 | 1228 | 814 | 925 | 1228 | 814 |
| nCohort 2 | 62 | 15 | 57 | 69 | 18 | 62 | 39 | 17 | 34 |
| Cutoff 1 | 6.53 | 9.29 | 6.56 | 6.12 | 4.91 | 7.51 | 3.64 | 5.14 | 3.71 |
| Sens 1 | 71% | 73% | 70% | 71% | 72% | 71% | 72% | 71% | 71% |
| Spec 1 | 54% | 66% | 54% | 51% | 39% | 61% | 31% | 41% | 31% |
| Cutoff 2 | 5.18 | 7.12 | 5.42 | 4.16 | 3.53 | 5.57 | 2.57 | 2.47 | 3.00 |
| Sens 2 | 81% | 80% | 81% | 81% | 83% | 81% | 82% | 82% | 82% |
| Spec 2 | 45% | 54% | 47% | 36% | 28% | 48% | 21% | 18% | 25% |
| Cutoff 3 | 2.06 | 3.69 | 2.04 | 2.10 | 2.84 | 2.37 | 1.71 | 1.85 | 0.530 |
| Sens 3 | 90% | 93% | 91% | 91% | 94% | 90% | 92% | 94% | 91% |
| Spec 3 | 17% | 29% | 16% | 17% | 22% | 19% | 14% | 14% | 5% |
| Cutoff 4 | 9.53 | 10.3 | 9.60 | 9.53 | 10.3 | 9.60 | 9.53 | 10.3 | 9.60 |
| Sens 4 | 42% | 60% | 44% | 55% | 56% | 58% | 41% | 29% | 44% |
| Spec 4 | 70% | 70% | 70% | 70% | 70% | 70% | 70% | 70% | 70% |
| Cutoff 5 | 12.4 | 14.0 | 12.4 | 12.4 | 14.0 | 12.4 | 12.4 | 14.0 | 12.4 |
| Sens 5 | 34% | 40% | 37% | 39% | 44% | 42% | 28% | 24% | 32% |
| Spec 5 | 80% | 80% | 80% | 80% | 80% | 80% | 80% | 80% | 80% |
| Cutoff 6 | 20.3 | 23.5 | 21.6 | 20.3 | 23.5 | 21.6 | 20.3 | 23.5 | 21.6 |
| Sens 6 | 16% | 7% | 16% | 20% | 28% | 21% | 10% | 12% | 12% |
| Spec 6 | 90% | 90% | 90% | 90% | 90% | 90% | 90% | 90% | 90% |
| OR Quart 2 | 1.00 | 2.0 | 1.00 | 1.3 | 1.7 | 1.4 | 1.3 | 1.3 | 1.3 |
| p Value | 0.99 | 0.57 | 0.99 | 0.51 | 0.48 | 0.46 | 0.63 | 0.74 | 0.61 |
| 95% CI of | 0.37 | 0.18 | 0.34 | 0.56 | 0.40 | 0.54 | 0.49 | 0.33 | 0.47 |
| OR Quart2 | 2.7 | 22 | 2.9 | 3.3 | 7.1 | 3.9 | 3.3 | 4.7 | 3.6 |
| OR Quart 3 | 3.1 | 4.0 | 3.2 | 1.8 | 0 | 2.1 | 1.1 | 0.75 | 0.71 |
| p Value | 0.0080 | 0.21 | 0.0094 | 0.16 | na | 0.12 | 0.81 | 0.70 | 0.56 |
| 95% CI of | 1.3 | 0.45 | 1.3 | 0.79 | na | 0.82 | 0.43 | 0.17 | 0.22 |
| OR Quart3 | 7.0 | 36 | 7.7 | 4.2 | na | 5.2 | 3.0 | 3.4 | 2.3 |
| OR Quart 4 | 3.1 | 8.2 | 3.4 | 3.9 | 3.4 | 5.0 | 1.5 | 1.2 | 1.9 |
| p Value | 0.0080 | 0.048 | 0.0064 | 4.5E-4 | 0.065 | 1.9E-4 | 0.36 | 0.74 | 0.18 |
| 95% CI of | 1.3 | 1.0 | 1.4 | 1.8 | 0.93 | 2.1 | 0.61 | 0.33 | 0.75 |
| OR Quart4 | 7.0 | 66 | 8.1 | 8.4 | 12 | 12 | 3.8 | 4.7 | 4.9 |

| | | | | | | | | | |
|---|---|---|---|---|---|---|---|---|---|
| **CA 15-3** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 434 | | 434 | 434 | | 434 | 434 | | 434 |
| Average | 314 | | 393 | 314 | | 352 | 314 | | 329 |
| Stdev | 236 | | 116 | 236 | | 212 | 236 | | 171 |
| p(t-test) | | | 0.041 | | | 0.28 | | | 0.75 |
| Min | 2.21 | | 1.17 | 2.21 | | 21.3 | 2.21 | | 4.72 |
| Max | 784 | | 434 | 784 | | 784 | 784 | | 434 |
| n (Samp) | 407 | | 39 | 407 | | 50 | 407 | | 27 |
| n (Patient) | 214 | | 39 | 214 | | 50 | 214 | | 27 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | nd | | nd | 434 | | 434 | 434 | | 434 |
| Average | nd | | nd | 329 | | 350 | 329 | | 351 |
| Stdev | nd | | nd | 223 | | 223 | 223 | | 152 |
| p(t-test) | nd | | nd | | | 0.78 | | | 0.75 |
| Min | nd | | nd | 1.17 | | 39.8 | 1.17 | | 43.5 |
| Max | nd | | nd | 784 | | 784 | 784 | | 434 |
| n (Samp) | nd | | nd | 535 | | 10 | 535 | | 11 |
| n (Patient) | nd | | nd | 256 | | 10 | 256 | | 11 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 434 | | 434 | 434 | | 434 | 434 | | 434 |
| Average | 316 | | 394 | 316 | | 375 | 316 | | 327 |
| Stdev | 232 | | 116 | 232 | | 215 | 232 | | 177 |
| p(t-test) | | | 0.039 | | | 0.095 | | | 0.81 |
| Min | 2.21 | | 1.17 | 2.21 | | 21.3 | 2.21 | | 4.72 |
| Max | 784 | | 434 | 784 | | 784 | 784 | | 434 |
| n (Samp) | 380 | | 39 | 380 | | 47 | 380 | | 25 |
| n (Patient) | 189 | | 39 | 189 | | 47 | 189 | | 25 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.60 | nd | 0.60 | 0.57 | 0.52 | 0.59 | 0.54 | 0.54 | 0.53 |
| SE | 0.050 | nd | 0.050 | 0.044 | 0.094 | 0.046 | 0.058 | 0.090 | 0.061 |
| p | 0.055 | nd | 0.048 | 0.11 | 0.80 | 0.056 | 0.54 | 0.62 | 0.60 |
| nCohort 1 | 407 | nd | 380 | 407 | 535 | 380 | 407 | 535 | 380 |
| nCohort 2 | 39 | nd | 39 | 50 | 10 | 47 | 27 | 11 | 25 |
| Cutoff 1 | 427 | nd | 427 | 239 | 265 | 427 | 427 | 427 | 427 |
| Sens 1 | 82% | nd | 85% | 70% | 70% | 70% | 70% | 73% | 72% |
| Spec 1 | 43% | nd | 42% | 39% | 35% | 42% | 43% | 39% | 42% |
| Cutoff 2 | 427 | nd | 427 | 80.0 | 167 | 87.5 | 75.8 | 265 | 75.8 |
| Sens 2 | 82% | nd | 85% | 80% | 80% | 81% | 81% | 82% | 80% |
| Spec 2 | 43% | nd | 42% | 31% | 32% | 30% | 30% | 35% | 29% |
| Cutoff 3 | 265 | nd | 265 | 43.7 | 45.0 | 42.6 | 18.9 | 75.8 | 18.8 |
| Sens 3 | 92% | nd | 92% | 90% | 90% | 91% | 93% | 91% | 92% |
| Spec 3 | 40% | nd | 39% | 22% | 20% | 22% | 12% | 26% | 11% |
| Cutoff 4 | 434 | nd | 434 | 434 | 434 | 434 | 434 | 434 | 434 |
| Sens 4 | 0% | nd | 0% | 12% | 10% | 15% | 0% | 0% | 0% |
| Spec 4 | 90% | nd | 91% | 90% | 91% | 91% | 90% | 91% | 91% |
| Cutoff 5 | 434 | nd | 434 | 434 | 434 | 434 | 434 | 434 | 434 |
| Sens 5 | 0% | nd | 0% | 12% | 10% | 15% | 0% | 0% | 0% |
| Spec 5 | 90% | nd | 91% | 90% | 91% | 91% | 90% | 91% | 91% |
| Cutoff 6 | 434 | nd | 434 | 434 | 434 | 434 | 434 | 434 | 434 |
| Sens 6 | 0% | nd | 0% | 12% | 10% | 15% | 0% | 0% | 0% |
| Spec 6 | 90% | nd | 91% | 90% | 91% | 91% | 90% | 91% | 91% |
| OR Quart 2 | 8.3 | nd | 11 | 1.0 | 3.6 | 1.3 | 0.99 | 11 | 1.0 |
| p Value | 8.3E-4 | nd | 1.3E-4 | 1.0 | 0.11 | 0.59 | 0.99 | 0.025 | 1.0 |
| 95% CI of | 2.4 | nd | 3.2 | 0.36 | 0.74 | 0.45 | 0.24 | 1.3 | 0.24 |
| OR Quart2 | 29 | nd | 38 | 2.8 | 18 | 4.0 | 4.1 | 84 | 4.1 |
| OR Quart 3 | 5.6 | nd | 3.5 | 4.3 | 0 | 5.3 | 5.6 | 0 | 4.9 |
| p Value | 0.0077 | nd | 0.060 | 6.1E-4 | na | 4.4E-4 | 0.0026 | na | 0.0057 |
| 95% CI of | 1.6 | nd | 0.95 | 1.9 | na | 2.1 | 1.8 | na | 1.6 |
| OR Quart3 | 20 | nd | 13 | 9.9 | na | 14 | 17 | na | 15 |
| OR Quart 4 | 0 | nd | 0 | 0.73 | 0.49 | 1.2 | 0 | 0 | 0 |
| p Value | na | nd | na | 0.57 | 0.57 | 0.79 | na | na | na |
| 95% CI of | na | nd | na | 0.24 | 0.044 | 0.38 | na | na | na |
| OR Quart4 | na | nd | na | 2.2 | 5.5 | 3.6 | na | na | na |

**Table 3: Comparison of marker levels in urine samples collected within 12 hours of reaching stage R from Cohort 1 (patients that reached, but did not progress beyond, RIFLE stage R) and from Cohort 2 (patients that reached RIFLE stage I or F).**

| **C-C motif chemokine 8** | | | | | | |
|---|---|---|---|---|---|---|
| | sCr or UO | | sCr only | | UO only | |
| | Cohort 1 | Cohort 2 | Cohort 1 | Cohort 2 | Cohort 1 | Cohort 2 |
| Median | 1.72 | 4.28 | 1.72 | 3.74 | 1.72 | 4.29 |
| Average | 11.4 | 14.2 | 20.6 | 8.92 | 10.2 | 10.3 |
| Stdev | 46.1 | 35.5 | 47.4 | 11.7 | 48.3 | 20.5 |
| p(t-test) | | 0.71 | | 0.37 | | 1.00 |
| Min | 0.0635 | 0.0250 | 0.0635 | 0.206 | 0.0701 | 0.0250 |
| Max | 473 | 214 | 214 | 40.5 | 473 | 108 |
| n (Samp) | 124 | 45 | 49 | 14 | 97 | 31 |
| n (Patient) | 124 | 45 | 49 | 14 | 97 | 31 |
| | | | | | | |

| | At Enrollment | | | | | |
|---|---|---|---|---|---|---|
| | sCr or UO | | sCr only | | UO only | |
| AUC | 0.58 | | 0.56 | | 0.57 | |
| SE | 0.051 | | 0.089 | | 0.060 | |
| p | 0.097 | | 0.48 | | 0.22 | |
| nCohort 1 | 124 | | 49 | | 97 | |
| nCohort 2 | 45 | | 14 | | 31 | |
| Cutoff 1 | 0.829 | | 1.21 | | 0.746 | |
| Sens 1 | 71% | | 79% | | 74% | |
| Spec 1 | 38% | | 45% | | 34% | |
| Cutoff 2 | 0.627 | | 0.756 | | 0.555 | |
| Sens 2 | 80% | | 86% | | 81% | |
| Spec 2 | 35% | | 33% | | 34% | |
| Cutoff 3 | 0.136 | | 0.306 | | 0.0775 | |
| Sens 3 | 91% | | 93% | | 90% | |
| Spec 3 | 19% | | 22% | | 6% | |
| Cutoff 4 | 4.86 | | 9.22 | | 4.72 | |
| Sens 4 | 44% | | 29% | | 48% | |
| Spec 4 | 70% | | 71% | | 70% | |
| Cutoff 5 | 8.21 | | 19.7 | | 7.85 | |
| Sens 5 | 31% | | 21% | | 35% | |
| Spec 5 | 81% | | 82% | | 80% | |
| Cutoff 6 | 21.9 | | 57.1 | | 19.7 | |
| Sens 6 | 11% | | 0% | | 13% | |
| Spec 6 | 90% | | 92% | | 91% | |
| OR Quart 2 | 2.7 | | 2.2 | | 1.0 | |
| p Value | 0.074 | | 0.42 | | 1.0 | |
| 95% CI of | 0.91 | | 0.33 | | 0.28 | |
| OR Quart2 | 8.0 | | 14 | | 3.5 | |
| OR Quart 3 | 1.9 | | 3.0 | | 1.2 | |
| p Value | 0.27 | | 0.24 | | 0.76 | |
| 95% CI of | 0.61 | | 0.48 | | 0.36 | |
| OR Quart3 | 5.7 | | 18 | | 4.1 | |
| OR Quart 4 | 3.6 | | 1.5 | | 2.6 | |
| p Value | 0.019 | | 0.68 | | 0.10 | |
| 95% CI of | 1.2 | | 0.21 | | 0.83 | |
| OR Quart4 | 10 | | 11 | | 8.1 | |

| | | | | | | |
|---|---|---|---|---|---|---|
| **Immunoglogulin G1** | | | | | | |
| | sCr or UO | | sCr only | | UO only | |
| | Cohort 1 | Cohort 2 | Cohort 1 | Cohort 2 | Cohort 1 | Cohort 2 |
| Median | 4050 | 6810 | 4770 | 9980 | 3950 | 7590 |
| Average | 6150 | 8820 | 8120 | 10500 | 6060 | 7790 |
| Stdev | 6120 | 7880 | 8760 | 8330 | 5490 | 5330 |
| p(t-test) | | 0.023 | | 0.37 | | 0.13 |
| Min | 342 | 572 | 342 | 699 | 424 | 634 |
| Max | 35500 | 30900 | 35500 | 28400 | 27500 | 22200 |
| n (Samp) | 122 | 44 | 48 | 14 | 96 | 30 |
| n (Patient) | 122 | 44 | 48 | 14 | 96 | 30 |
| | | | | | | |

| | At Enrollment | | | | | |
|---|---|---|---|---|---|---|
| | sCr or UO | | sCr only | | UO only | |
| AUC | 0.60 | | 0.59 | | 0.61 | |
| SE | 0.051 | | 0.089 | | 0.061 | |
| p | 0.054 | | 0.32 | | 0.060 | |
| nCohort 1 | 122 | | 48 | | 96 | |
| nCohort 2 | 44 | | 14 | | 30 | |
| Cutoff 1 | 3600 | | 3630 | | 4450 | |
| Sens 1 | 70% | | 71% | | 70% | |
| Spec 1 | 45% | | 42% | | 57% | |
| Cutoff 2 | 1790 | | 1480 | | 2710 | |
| Sens 2 | 82% | | 86% | | 80% | |
| Spec 2 | 20% | | 17% | | 33% | |
| Cutoff 3 | 1470 | | 1420 | | 1600 | |
| Sens 3 | 91% | | 93% | | 90% | |
| Spec 3 | 13% | | 17% | | 14% | |
| Cutoff 4 | 6730 | | 6730 | | 7270 | |
| Sens 4 | 50% | | 64% | | 53% | |
| Spec 4 | 70% | | 71% | | 71% | |
| Cutoff 5 | 9090 | | 16800 | | 10500 | |
| Sens 5 | 36% | | 21% | | 27% | |
| Spec 5 | 80% | | 81% | | 80% | |
| Cutoff 6 | 13500 | | 22800 | | 13100 | |
| Sens 6 | 23% | | 7% | | 17% | |
| Spec 6 | 90% | | 92% | | 91% | |
| OR Quart 2 | 0.62 | | 0.18 | | 0.43 | |
| p Value | 0.39 | | 0.15 | | 0.27 | |
| 95% CI of | 0.21 | | 0.018 | | 0.098 | |
| OR Quart2 | 1.8 | | 1.9 | | 1.9 | |
| OR Quart 3 | 1.1 | | 1.0 | | 2.3 | |
| p Value | 0.80 | | 1.0 | | 0.16 | |
| 95% CI of | 0.42 | | 0.20 | | 0.72 | |
| OR Quart3 | 3.1 | | 5.0 | | 7.3 | |
| OR Quart 4 | 1.9 | | 1.2 | | 1.9 | |
| p Value | 0.18 | | 0.78 | | 0.28 | |
| 95% CI of | 0.74 | | 0.26 | | 0.59 | |
| OR Quart4 | 4.9 | | 5.9 | | 6.1 | |

| | | | | | | |
|---|---|---|---|---|---|---|
| **Interleukin-11** | | | | | | |
| | sCr or UO | | sCr only | | UO only | |
| | Cohort 1 | Cohort 2 | Cohort 1 | Cohort 2 | Cohort 1 | Cohort 2 |
| Median | 215 | 179 | 223 | 200 | 217 | 157 |
| Average | 309 | 246 | 315 | 224 | 310 | 194 |
| Stdev | 297 | 329 | 363 | 163 | 298 | 153 |
| p(t-test) | | 0.24 | | 0.37 | | 0.044 |
| Min | 13.2 | 14.5 | 13.2 | 31.9 | 41.4 | 14.5 |
| Max | 1780 | 2140 | 2140 | 547 | 1780 | 753 |
| n (Samp) | 124 | 44 | 49 | 14 | 97 | 30 |
| n (Patient) | 124 | 44 | 49 | 14 | 97 | 30 |
| | | | | | | |

| | At Enrollment | | | | | |
|---|---|---|---|---|---|---|
| | sCr or UO | | sCr only | | UO only | |
| AUC | 0.41 | | 0.45 | | 0.37 | |
| SE | 0.051 | | 0.089 | | 0.061 | |
| p | 0.087 | | 0.61 | | 0.037 | |
| nCohort 1 | 124 | | 49 | | 97 | |
| nCohort 2 | 44 | | 14 | | 30 | |
| Cutoff 1 | 113 | | 113 | | 101 | |
| Sens 1 | 70% | | 71% | | 70% | |
| Spec 1 | 23% | | 33% | | 16% | |
| Cutoff 2 | 85.8 | | 50.4 | | 87.1 | |
| Sens 2 | 82% | | 86% | | 80% | |
| Spec 2 | 15% | | 12% | | 13% | |
| Cutoff 3 | 42.5 | | 41.4 | | 42.5 | |
| Sens 3 | 91% | | 93% | | 90% | |
| Spec 3 | 5% | | 10% | | 3% | |
| Cutoff 4 | 347 | | 390 | | 341 | |
| Sens 4 | 16% | | 21% | | 10% | |
| Spec 4 | 70% | | 71% | | 70% | |
| Cutoff 5 | 458 | | 426 | | 469 | |
| Sens 5 | 9% | | 14% | | 3% | |
| Spec 5 | 81% | | 82% | | 80% | |
| Cutoff 6 | 613 | | 659 | | 601 | |
| Sens 6 | 5% | | 0% | | 3% | |
| Spec 6 | 90% | | 92% | | 91% | |
| OR Quart 2 | 1.6 | | 3.2 | | 3.2 | |
| p Value | 0.42 | | 0.21 | | 0.11 | |
| 95% CI of | 0.53 | | 0.52 | | 0.77 | |
| OR Quart2 | 4.6 | | 20 | | 14 | |
| OR Quart 3 | 2.5 | | 1.6 | | 3.2 | |
| p Value | 0.083 | | 0.63 | | 0.11 | |
| 95% CI of | 0.89 | | 0.23 | | 0.77 | |
| OR Quart3 | 7.0 | | 11 | | 14 | |
| OR Quart 4 | 2.2 | | 2.5 | | 5.3 | |
| p Value | 0.13 | | 0.33 | | 0.019 | |
| 95% CI of | 0.79 | | 0.39 | | 1.3 | |
| OR Quart4 | 6.4 | | 17 | | 22 | |

| | | | | | | |
|---|---|---|---|---|---|---|
| **Neutrophil collagenase** | | | | | | |
| | sCr or UO | | sCr only | | UO only | |
| | Cohort 1 | Cohort 2 | Cohort 1 | Cohort 2 | Cohort 1 | Cohort 2 |
| Median | 4380 | 7320 | 3490 | 12000 | 5330 | 5620 |
| Average | 13500 | 36800 | 8510 | 15400 | 17400 | 43100 |
| Stdev | 23700 | 118000 | 13200 | 19200 | 27900 | 138000 |
| p(t-test) | | 0.027 | | 0.10 | | 0.066 |
| Min | 150 | 23.8 | 23.8 | 25.8 | 124 | 0.260 |
| Max | 128000 | 670000 | 63000 | 62500 | 128000 | 670000 |
| n (Samp) | 138 | 58 | 56 | 16 | 108 | 42 |
| n (Patient) | 138 | 58 | 56 | 16 | 108 | 42 |
| | | | | | | |

| | At Enrollment | | | | | |
|---|---|---|---|---|---|---|
| | sCr or UO | | sCr only | | UO only | |
| AUC | 0.58 | | 0.61 | | 0.54 | |
| SE | 0.046 | | 0.083 | | 0.053 | |
| p | 0.096 | | 0.19 | | 0.47 | |
| nCohort 1 | 138 | | 56 | | 108 | |
| nCohort 2 | 58 | | 16 | | 42 | |
| Cutoff 1 | 2620 | | 1580 | | 2940 | |
| Sens 1 | 71% | | 75% | | 71% | |
| Spec 1 | 41% | | 36% | | 39% | |
| Cutoff 2 | 1580 | | 987 | | 1630 | |
| Sens 2 | 81% | | 81% | | 81% | |
| Spec 2 | 32% | | 25% | | 27% | |
| Cutoff 3 | 566 | | 365 | | 1110 | |
| Sens 3 | 91% | | 94% | | 90% | |
| Spec 3 | 10% | | 11% | | 21% | |
| Cutoff 4 | 9550 | | 7860 | | 12900 | |
| Sens 4 | 41% | | 56% | | 38% | |
| Spec 4 | 70% | | 71% | | 70% | |
| Cutoff 5 | 15700 | | 10700 | | 22200 | |
| Sens 5 | 33% | | 56% | | 24% | |
| Spec 5 | 80% | | 80% | | 81% | |
| Cutoff 6 | 48400 | | 28300 | | 60700 | |
| Sens 6 | 12% | | 12% | | 5% | |
| Spec 6 | 91% | | 91% | | 91% | |
| OR Quart 2 | 1.9 | | 0.70 | | 1.9 | |
| p Value | 0.17 | | 0.67 | | 0.23 | |
| 95% CI of | 0.76 | | 0.13 | | 0.67 | |
| OR Quart2 | 4.7 | | 3.7 | | 5.3 | |
| OR Quart 3 | 1.4 | | 0.44 | | 0.85 | |
| p Value | 0.48 | | 0.38 | | 0.77 | |
| 95% CI of | 0.55 | | 0.069 | | 0.27 | |
| OR Quart3 | 3.6 | | 2.8 | | 2.6 | |
| OR Quart 4 | 2.5 | | 2.2 | | 2.1 | |
| p Value | 0.049 | | 0.28 | | 0.15 | |
| 95% CI of | 1.0 | | 0.52 | | 0.76 | |
| OR Quart4 | 6.1 | | 9.6 | | 5.9 | |

**Table 4: Comparison of the maximum marker levels in urine samples collected from Cohort 1 (patients that did not progress beyond RIFLE stage 0) and the maximum values in urine samples collected from subjects between enrollment and 0, 24 hours, and 48 hours prior to reaching stage F in Cohort 2.**

| **C-C motif chemokine 18** | | | | | | | | | |
|---|---|---|---|---|---|---|---|---|---|
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 0.531 | | 2.90 | 0.531 | | 2.72 | 0.531 | | 2.38 |
| Average | 2.66 | | 12.9 | 2.66 | | 10.2 | 2.66 | | 9.02 |
| Stdev | 7.47 | | 16.2 | 7.47 | | 14.4 | 7.47 | | 15.4 |
| p(t-test) | | | 1.0E-8 | | | 8.6E-6 | | | 0.0030 |
| Min | 3.13E-5 | | 0.226 | 3.13E-5 | | 0.204 | 3.13E-5 | | 0.126 |
| Max | 40.0 | | 40.0 | 40.0 | | 40.0 | 40.0 | | 40.0 |
| n (Samp) | 223 | | 30 | 223 | | 30 | 223 | | 16 |
| n (Patient) | 223 | | 30 | 223 | | 30 | 223 | | 16 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 0.649 | | 2.95 | 0.649 | | 2.72 | 0.649 | | 2.95 |
| Average | 3.73 | | 15.1 | 3.73 | | 8.44 | 3.73 | | 8.52 |
| Stdev | 9.09 | | 18.0 | 9.09 | | 11.7 | 9.09 | | 13.3 |
| p(t-test) | | | 3.0E-5 | | | 0.070 | | | 0.17 |
| Min | 3.13E-5 | | 0.460 | 3.13E-5 | | 0.204 | 3.13E-5 | | 0.460 |
| Max | 40.0 | | 40.0 | 40.0 | | 40.0 | 40.0 | | 37.8 |
| n (Samp) | 374 | | 13 | 374 | | 13 | 374 | | 7 |
| n (Patient) | 374 | | 13 | 374 | | 13 | 374 | | 7 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 0.620 | | 2.95 | 0.620 | | 2.85 | 0.620 | | 2.53 |
| Average | 3.05 | | 13.9 | 3.05 | | 13.2 | 3.05 | | 9.95 |
| Stdev | 7.82 | | 16.8 | 7.82 | | 16.7 | 7.82 | | 16.3 |
| p(t-test) | | | 3.6E-7 | | | 1.8E-6 | | | 0.0048 |
| Min | 3.13E-5 | | 0.226 | 3.13E-5 | | 0.226 | 3.13E-5 | | 0.126 |
| Max | 40.0 | | 40.0 | 40.0 | | 40.0 | 40.0 | | 40.0 |
| n (Samp) | 173 | | 23 | 173 | | 23 | 173 | | 14 |
| n (Patient) | 173 | | 23 | 173 | | 23 | 173 | | 14 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.82 | 0.79 | 0.80 | 0.80 | 0.75 | 0.79 | 0.72 | 0.78 | 0.68 |
| SE | 0.048 | 0.076 | 0.057 | 0.050 | 0.079 | 0.058 | 0.074 | 0.10 | 0.081 |
| p | 2.2E-11 | 1.2E-4 | 1.0E-7 | 2.6E-9 | 0.0014 | 6.2E-7 | 0.0028 | 0.0079 | 0.027 |
| nCohort 1 | 223 | 374 | 173 | 223 | 374 | 173 | 223 | 374 | 173 |
| nCohort 2 | 30 | 13 | 23 | 30 | 13 | 23 | 16 | 7 | 14 |
| Cutoff 1 | 1.98 | 1.97 | 1.98 | 1.98 | 1.97 | 1.98 | 0.714 | 1.99 | 1.34 |
| Sens 1 | 70% | 77% | 74% | 70% | 77% | 74% | 75% | 71% | 71% |
| Spec 1 | 83% | 76% | 76% | 83% | 76% | 76% | 61% | 76% | 71% |
| Cutoff 2 | 1.48 | 1.04 | 1.51 | 1.04 | 1.04 | 1.34 | 0.451 | 1.97 | 0.228 |
| Sens 2 | 80% | 85% | 83% | 80% | 85% | 83% | 81% | 86% | 86% |
| Spec 2 | 78% | 63% | 71% | 72% | 63% | 71% | 45% | 76% | 18% |
| Cutoff 3 | 0.531 | 0.531 | 0.862 | 0.451 | 0.451 | 0.767 | 0.225 | 0.451 | 0.205 |
| Sens 3 | 90% | 92% | 91% | 90% | 92% | 91% | 94% | 100% | 93% |
| Spec 3 | 50% | 43% | 60% | 45% | 39% | 57% | 28% | 39% | 18% |
| Cutoff 4 | 1.03 | 1.41 | 1.34 | 1.03 | 1.41 | 1.34 | 1.03 | 1.41 | 1.34 |
| Sens 4 | 83% | 77% | 87% | 80% | 77% | 83% | 69% | 86% | 71% |
| Spec 4 | 70% | 70% | 71% | 70% | 70% | 71% | 70% | 70% | 71% |
| Cutoff 5 | 1.71 | 2.82 | 2.68 | 1.71 | 2.82 | 2.68 | 1.71 | 2.82 | 2.68 |
| Sens 5 | 73% | 54% | 61% | 73% | 46% | 57% | 62% | 57% | 50% |
| Spec 5 | 80% | 80% | 80% | 80% | 80% | 80% | 80% | 80% | 80% |
| Cutoff 6 | 3.97 | 8.25 | 4.19 | 3.97 | 8.25 | 4.19 | 3.97 | 8.25 | 4.19 |
| Sens 6 | 43% | 38% | 43% | 37% | 31% | 39% | 25% | 29% | 21% |
| Spec 6 | 90% | 90% | 90% | 90% | 90% | 90% | 90% | 90% | 90% |
| OR Quart 2 | 3.1 | >2.0 | 0 | 3.1 | 0.99 | 0 | 3.1 | >1.0 | 0 |
| p Value | 0.33 | <0.57 | na | 0.33 | 0.99 | na | 0.34 | <0.99 | na |
| 95% CI of | 0.31 | >0.18 | na | 0.31 | 0.061 | na | 0.31 | >0.062 | na |
| OR Quart2 | 31 | na | na | 31 | 16 | na | 30 | na | na |
| OR Quart 3 | 4.2 | >3.1 | 3.9 | 4.2 | 3.0 | 3.9 | 0.98 | >0 | 0.98 |
| p Value | 0.20 | <0.34 | 0.100 | 0.20 | 0.34 | 0.100 | 0.99 | <na | 0.98 |
| 95% CI of | 0.46 | >0.31 | 0.77 | 0.46 | 0.31 | 0.77 | 0.060 | >na | 0.19 |
| OR Quart3 | 39 | na | 20 | 39 | 30 | 20 | 16 | na | 5.1 |
| OR Quart 4 | 32 | >8.6 | 9.4 | 32 | 8.5 | 9.4 | 13 | >6.3 | 2.9 |
| p Value | 8.4E-4 | <0.044 | 0.0045 | 8.4E-4 | 0.045 | 0.0045 | 0.016 | <0.090 | 0.13 |
| 95% CI of | 4.2 | >1.1 | 2.0 | 4.2 | 1.0 | 2.0 | 1.6 | >0.75 | 0.73 |
| OR Quart4 | 250 | na | 44 | 250 | 70 | 44 | 100 | na | 12 |

| **C-C motif chemokine 24** | | | | | | | | | |
|---|---|---|---|---|---|---|---|---|---|
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 13.0 | | 51.0 | 13.0 | | 30.9 | 13.0 | | 26.4 |
| Average | 26.0 | | 308 | 26.0 | | 269 | 26.0 | | 95.6 |
| Stdev | 58.4 | | 690 | 58.4 | | 598 | 58.4 | | 288 |
| p(t-test) | | | 5.8E-9 | | | 1.0E-8 | | | 0.0037 |
| Min | 0.0120 | | 2.55 | 0.0120 | | 2.55 | 0.0120 | | 2.55 |
| Max | 730 | | 2790 | 730 | | 2380 | 730 | | 1170 |
| n (Samp) | 223 | | 30 | 223 | | 30 | 223 | | 16 |
| n (Patient) | 223 | | 30 | 223 | | 30 | 223 | | 16 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 17.8 | | 30.9 | 17.8 | | 28.7 | 17.8 | | 28.0 |
| Average | 48.4 | | 93.4 | 48.4 | | 63.7 | 48.4 | | 53.5 |
| Stdev | 160 | | 110 | 160 | | 74.6 | 160 | | 86.7 |
| p(t-test) | | | 0.32 | | | 0.73 | | | 0.93 |
| Min | 0.0120 | | 2.55 | 0.0120 | | 2.55 | 0.0120 | | 2.55 |
| Max | 2380 | | 373 | 2380 | | 249 | 2380 | | 249 |
| n (Samp) | 375 | | 13 | 375 | | 13 | 375 | | 7 |
| n (Patient) | 375 | | 13 | 375 | | 13 | 375 | | 7 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 13.9 | | 61.9 | 13.9 | | 41.6 | 13.9 | | 28.3 |
| Average | 30.6 | | 390 | 30.6 | | 342 | 30.6 | | 112 |
| Stdev | 66.3 | | 773 | 66.3 | | 669 | 66.3 | | 306 |
| p(t-test) | | | 7.2E-9 | | | 9.0E-9 | | | 0.0051 |
| Min | 0.0120 | | 3.62 | 0.0120 | | 3.62 | 0.0120 | | 3.62 |
| Max | 730 | | 2790 | 730 | | 2380 | 730 | | 1170 |
| n (Samp) | 173 | | 23 | 173 | | 23 | 173 | | 14 |
| n (Patient) | 173 | | 23 | 173 | | 23 | 173 | | 14 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.81 | 0.72 | 0.83 | 0.78 | 0.66 | 0.81 | 0.66 | 0.58 | 0.70 |
| SE | 0.049 | 0.082 | 0.054 | 0.051 | 0.084 | 0.056 | 0.076 | 0.11 | 0.081 |
| p | 2.2E-10 | 0.0078 | 6.4E-10 | 4.2E-8 | 0.051 | 2.2E-8 | 0.034 | 0.49 | 0.015 |
| nCohort 1 | 223 | 375 | 173 | 223 | 375 | 173 | 223 | 375 | 173 |
| nCohort 2 | 30 | 13 | 23 | 30 | 13 | 23 | 16 | 7 | 14 |
| Cutoff 1 | 28.4 | 27.3 | 30.4 | 27.2 | 25.9 | 27.8 | 16.6 | 26.9 | 20.4 |
| Sens 1 | 70% | 77% | 74% | 70% | 77% | 74% | 75% | 71% | 71% |
| Spec 1 | 78% | 64% | 77% | 76% | 62% | 75% | 61% | 63% | 66% |
| Cutoff 2 | 25.4 | 25.4 | 27.8 | 20.4 | 10.9 | 26.6 | 8.65 | 8.65 | 16.6 |
| Sens 2 | 80% | 85% | 83% | 80% | 85% | 83% | 81% | 86% | 86% |
| Spec 2 | 73% | 61% | 75% | 68% | 35% | 73% | 40% | 31% | 58% |
| Cutoff 3 | 16.6 | 8.65 | 19.3 | 10.9 | 8.65 | 19.3 | 3.21 | 2.51 | 3.71 |
| Sens 3 | 90% | 92% | 91% | 90% | 92% | 91% | 94% | 100% | 93% |
| Spec 3 | 61% | 31% | 63% | 48% | 31% | 63% | 28% | 18% | 25% |
| Cutoff 4 | 23.3 | 36.9 | 24.6 | 23.3 | 36.9 | 24.6 | 23.3 | 36.9 | 24.6 |
| Sens 4 | 80% | 46% | 83% | 77% | 38% | 83% | 56% | 14% | 64% |
| Spec 4 | 71% | 70% | 71% | 71% | 70% | 71% | 71% | 70% | 71% |
| Cutoff 5 | 35.1 | 51.0 | 40.3 | 35.1 | 51.0 | 40.3 | 35.1 | 51.0 | 40.3 |
| Sens 5 | 53% | 46% | 61% | 43% | 38% | 52% | 19% | 14% | 29% |
| Spec 5 | 80% | 80% | 80% | 80% | 80% | 80% | 80% | 80% | 80% |
| Cutoff 6 | 62.8 | 87.2 | 74.4 | 62.8 | 87.2 | 74.4 | 62.8 | 87.2 | 74.4 |
| Sens 6 | 43% | 31% | 48% | 37% | 15% | 39% | 6% | 14% | 14% |
| Spec 6 | 90% | 90% | 90% | 90% | 90% | 90% | 90% | 90% | 90% |
| OR Quart 2 | 2.0 | 1.0 | 0 | 3.1 | 2.0 | 0 | 3.1 | 0.99 | 0 |
| p Value | 0.57 | 1.0 | na | 0.33 | 0.57 | na | 0.34 | 0.99 | na |
| 95% CI of | 0.18 | 0.062 | na | 0.31 | 0.18 | na | 0.31 | 0.061 | na |
| OR Quart2 | 23 | 16 | na | 31 | 23 | na | 30 | 16 | na |
| OR Quart 3 | 12 | 5.2 | 9.4 | 12 | 5.2 | 12 | 4.1 | 4.1 | 3.2 |
| p Value | 0.021 | 0.13 | 0.039 | 0.021 | 0.13 | 0.019 | 0.21 | 0.21 | 0.17 |
| 95% CI of | 1.4 | 0.60 | 1.1 | 1.4 | 0.60 | 1.5 | 0.45 | 0.45 | 0.61 |
| OR Quart3 | 94 | 46 | 78 | 94 | 46 | 100 | 38 | 38 | 17 |
| OR Quart 4 | 22 | 6.3 | 19 | 21 | 5.2 | 16 | 8.9 | 0.99 | 3.2 |
| p Value | 0.0030 | 0.090 | 0.0053 | 0.0039 | 0.13 | 0.0098 | 0.042 | 0.99 | 0.17 |
| 95% CI of | 2.9 | 0.75 | 2.4 | 2.6 | 0.60 | 1.9 | 1.1 | 0.061 | 0.61 |
| OR Quart4 | 170 | 54 | 150 | 160 | 46 | 130 | 74 | 16 | 17 |

| | | | | | | | | | |
|---|---|---|---|---|---|---|---|---|---|
| **C-C motif chemokine 8** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 2.74 | | 24.0 | 2.74 | | 22.9 | 2.74 | | 13.6 |
| Average | 11.9 | | 175 | 11.9 | | 169 | 11.9 | | 250 |
| Stdev | 27.9 | | 664 | 27.9 | | 665 | 27.9 | | 912 |
| p(t-test) | | | 2.8E-4 | | | 4.6E-4 | | | 9.2E-5 |
| Min | 0.0250 | | 0.206 | 0.0250 | | 0.136 | 0.0250 | | 0.136 |
| Max | 250 | | 3670 | 250 | | 3670 | 250 | | 3670 |
| n (Samp) | 223 | | 30 | 223 | | 30 | 223 | | 16 |
| n (Patient) | 223 | | 30 | 223 | | 30 | 223 | | 16 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 4.72 | | 22.7 | 4.72 | | 22.7 | 4.72 | | 22.7 |
| Average | 17.7 | | 49.4 | 17.7 | | 45.4 | 17.7 | | 36.6 |
| Stdev | 47.6 | | 82.2 | 47.6 | | 79.2 | 47.6 | | 47.0 |
| p(t-test) | | | 0.022 | | | 0.045 | | | 0.30 |
| Min | 0.0250 | | 0.206 | 0.0250 | | 0.206 | 0.0250 | | 4.53 |
| Max | 492 | | 279 | 492 | | 279 | 492 | | 140 |
| n (Samp) | 375 | | 13 | 375 | | 13 | 375 | | 7 |
| n (Patient) | 375 | | 13 | 375 | | 13 | 375 | | 7 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 4.72 | | 30.8 | 4.72 | | 30.8 | 4.72 | | 13.6 |
| Average | 14.8 | | 220 | 14.8 | | 209 | 14.8 | | 284 |
| Stdev | 32.0 | | 755 | 32.0 | | 757 | 32.0 | | 975 |
| p(t-test) | | | 3.9E-4 | | | 7.8E-4 | | | 2.7E-4 |
| Min | 0.0250 | | 0.829 | 0.0250 | | 0.136 | 0.0250 | | 0.136 |
| Max | 250 | | 3670 | 250 | | 3670 | 250 | | 3670 |
| n(Samp) | 173 | | 23 | 173 | | 23 | 173 | | 14 |
| n (Patient) | 173 | | 23 | 173 | | 23 | 173 | | 14 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.79 | 0.70 | 0.82 | 0.77 | 0.69 | 0.78 | 0.70 | 0.77 | 0.65 |
| SE | 0.051 | 0.083 | 0.056 | 0.052 | 0.083 | 0.059 | 0.075 | 0.11 | 0.082 |
| p | 6.8E-9 | 0.017 | 1.3E-8 | 3.2E-7 | 0.022 | 2.1E-6 | 0.0080 | 0.010 | 0.078 |
| nCohort 1 | 223 | 375 | 173 | 223 | 375 | 173 | 223 | 375 | 173 |
| nCohort 2 | 30 | 13 | 23 | 30 | 13 | 23 | 16 | 7 | 14 |
| Cutoff 1 | 10.4 | 4.68 | 12.5 | 9.39 | 4.35 | 10.4 | 4.35 | 21.7 | 4.35 |
| Sens 1 | 70% | 77% | 74% | 70% | 77% | 74% | 75% | 71% | 71% |
| Spec 1 | 74% | 48% | 73% | 71% | 48% | 69% | 56% | 81% | 47% |
| Cutoff 2 | 7.37 | 4.35 | 9.05 | 4.35 | 3.16 | 8.05 | 1.21 | 7.37 | 0.756 |
| Sens 2 | 80% | 85% | 83% | 80% | 85% | 83% | 81% | 86% | 86% |
| Spec 2 | 67% | 48% | 66% | 56% | 46% | 65% | 39% | 59% | 26% |
| Cutoff 3 | 1.21 | 0.438 | 4.35 | 0.650 | 0.438 | 1.21 | 0.438 | 4.35 | 0.438 |
| Sens 3 | 90% | 92% | 91% | 90% | 92% | 91% | 94% | 100% | 93% |
| Spec 3 | 39% | 24% | 47% | 32% | 24% | 31% | 30% | 48% | 21% |
| Cutoff 4 | 9.38 | 12.0 | 11.1 | 9.38 | 12.0 | 11.1 | 9.38 | 12.0 | 11.1 |
| Sens 4 | 73% | 62% | 74% | 70% | 62% | 70% | 56% | 71% | 57% |
| Spec 4 | 70% | 70% | 71% | 70% | 70% | 71% | 70% | 70% | 71% |
| Cutoff 5 | 12.8 | 20.3 | 18.0 | 12.8 | 20.3 | 18.0 | 12.8 | 20.3 | 18.0 |
| Sens 5 | 63% | 62% | 70% | 63% | 62% | 65% | 50% | 71% | 43% |
| Spec 5 | 80% | 80% | 80% | 80% | 80% | 80% | 80% | 80% | 80% |
| Cutoff 6 | 31.5 | 36.6 | 34.7 | 31.5 | 36.6 | 34.7 | 31.5 | 36.6 | 34.7 |
| Sens 6 | 37% | 23% | 48% | 37% | 15% | 48% | 19% | 14% | 21% |
| Spec 6 | 90% | 90% | 90% | 90% | 90% | 90% | 90% | 90% | 90% |
| OR Quart 2 | 3.1 | 0.49 | 2.0 | 2.1 | 1.0 | 1.0 | 3.1 | >1.0 | 1.5 |
| p Value | 0.33 | 0.57 | 0.57 | 0.41 | 1.0 | 1.0 | 0.34 | <1.0 | 0.67 |
| 95% CI of | 0.31 | 0.044 | 0.18 | 0.36 | 0.14 | 0.14 | 0.31 | >0.062 | 0.24 |
| OR Quart2 | 31 | 5.5 | 23 | 12 | 7.2 | 7.4 | 30 | na | 9.4 |
| OR Quart 3 | 7.8 | 1.0 | 4.3 | 2.6 | 0.49 | 2.1 | 4.1 | >1.0 | 1.5 |
| p Value | 0.059 | 1.0 | 0.20 | 0.26 | 0.57 | 0.41 | 0.21 | <0.99 | 0.67 |
| 95% CI of | 0.92 | 0.14 | 0.46 | 0.49 | 0.044 | 0.36 | 0.45 | >0.062 | 0.24 |
| OR Quart3 | 65 | 7.2 | 40 | 14 | 5.5 | 12 | 38 | na | 9.4 |
| OR Quart 4 | 26 | 4.3 | 23 | 13 | 4.3 | 10 | 8.9 | >5.2 | 3.2 |
| p Value | 0.0018 | 0.071 | 0.0029 | 8.9E-4 | 0.071 | 0.0029 | 0.042 | <0.13 | 0.17 |
| 95% CI of | 3.4 | 0.88 | 2.9 | 2.9 | 0.88 | 2.2 | 1.1 | >0.60 | 0.61 |
| OR Quart4 | 200 | 21 | 180 | 58 | 21 | 48 | 74 | na | 17 |

| **Cathepsin D** | | | | | | | | | |
|---|---|---|---|---|---|---|---|---|---|
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 71300 | | 128000 | 71300 | | 123000 | 71300 | | 121000 |
| Average | 74100 | | 136000 | 74100 | | 131000 | 74100 | | 130000 |
| Stdev | 38900 | | 41600 | 38900 | | 39100 | 38900 | | 42500 |
| p(t-test) | | | 1.8E-14 | | | 7.3E-13 | | | 7.9E-8 |
| Min | 656 | | 65600 | 656 | | 65600 | 656 | | 65600 |
| Max | 200000 | | 200000 | 200000 | | 200000 | 200000 | | 200000 |
| n (Samp) | 223 | | 30 | 223 | | 30 | 223 | | 16 |
| n (Patient) | 223 | | 30 | 223 | | 30 | 223 | | 16 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 84600 | | 130000 | 84600 | | 120000 | 84600 | | 117000 |
| Average | 90900 | | 127000 | 90900 | | 121000 | 90900 | | 127000 |
| Stdev | 44700 | | 34200 | 44700 | | 29100 | 44700 | | 24800 |
| p(t-test) | | | 0.0040 | | | 0.018 | | | 0.035 |
| Min | 656 | | 71400 | 656 | | 71400 | 656 | | 110000 |
| Max | 200000 | | 188000 | 200000 | | 181000 | 200000 | | 181000 |
| n (Samp) | 374 | | 13 | 374 | | 13 | 374 | | 7 |
| n (Patient) | 374 | | 13 | 374 | | 13 | 374 | | 7 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 78900 | | 138000 | 78900 | | 130000 | 78900 | | 125000 |
| Average | 84300 | | 143000 | 84300 | | 138000 | 84300 | | 129000 |
| Stdev | 40600 | | 41200 | 40600 | | 40000 | 40600 | | 43300 |
| p(t-test) | | | 7.6E-10 | | | 9.9E-9 | | | 1.0E-4 |
| Min | 2520 | | 65600 | 2520 | | 65600 | 2520 | | 65600 |
| Max | 200000 | | 200000 | 200000 | | 200000 | 200000 | | 200000 |
| n(Samp) | 173 | | 23 | 173 | | 23 | 173 | | 14 |
| n (Patient) | 173 | | 23 | 173 | | 23 | 173 | | 14 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.86 | 0.74 | 0.84 | 0.85 | 0.72 | 0.83 | 0.84 | 0.77 | 0.78 |
| SE | 0.044 | 0.080 | 0.053 | 0.045 | 0.081 | 0.054 | 0.063 | 0.11 | 0.075 |
| p | 4.4E-16 | 0.0021 | 9.5E-11 | 1.2E-14 | 0.0069 | 1.7E-9 | 9.2E-8 | 0.011 | 1.9E-4 |
| nCohort 1 | 223 | 374 | 173 | 223 | 374 | 173 | 223 | 374 | 173 |
| nCohort 2 | 30 | 13 | 23 | 30 | 13 | 23 | 16 | 7 | 14 |
| Cutoff 1 | 117000 | 109000 | 119000 | 112000 | 109000 | 112000 | 109000 | 115000 | 109000 |
| Sens 1 | 70% | 77% | 74% | 70% | 77% | 74% | 75% | 71% | 71% |
| Spec 1 | 86% | 70% | 81% | 84% | 70% | 77% | 83% | 72% | 74% |
| Cutoff 2 | 109000 | 92900 | 111000 | 109000 | 89500 | 110000 | 99400 | 112000 | 96100 |
| Sens 2 | 80% | 85% | 83% | 80% | 85% | 83% | 81% | 86% | 86% |
| Spec 2 | 83% | 56% | 75% | 83% | 53% | 75% | 76% | 72% | 65% |
| Cutoff 3 | 84400 | 84400 | 96100 | 84400 | 84400 | 96100 | 66900 | 109000 | 66900 |
| Sens 3 | 90% | 92% | 91% | 90% | 92% | 91% | 94% | 100% | 93% |
| Spec 3 | 67% | 50% | 65% | 67% | 50% | 65% | 48% | 70% | 39% |
| Cutoff 4 | 92600 | 110000 | 103000 | 92600 | 110000 | 103000 | 92600 | 110000 | 103000 |
| Sens 4 | 87% | 69% | 87% | 83% | 69% | 87% | 88% | 86% | 71% |
| Spec 4 | 70% | 70% | 71% | 70% | 70% | 71% | 70% | 70% | 71% |
| Cutoff 5 | 104000 | 133000 | 117000 | 104000 | 133000 | 117000 | 104000 | 133000 | 117000 |
| Sens 5 | 80% | 38% | 78% | 80% | 23% | 70% | 75% | 14% | 57% |
| Spec 5 | 80% | 80% | 80% | 80% | 80% | 80% | 80% | 80% | 80% |
| Cutoff 6 | 133000 | 151000 | 143000 | 133000 | 151000 | 143000 | 133000 | 151000 | 143000 |
| Sens 6 | 43% | 15% | 43% | 40% | 8% | 39% | 38% | 14% | 36% |
| Spec 6 | 90% | 90% | 90% | 90% | 90% | 90% | 90% | 90% | 90% |
| OR Quart 2 | >3.1 | >2.0 | >2.1 | >3.1 | >2.0 | >2.1 | >2.0 | >0 | >2.0 |
| p Value | <0.33 | <0.57 | <0.55 | <0.33 | <0.57 | <0.55 | <0.57 | <na | <0.56 |
| 95% CI of | >0.32 | >0.18 | >0.18 | >0.32 | >0.18 | >0.18 | >0.18 | >na | >0.18 |
| OR Quart2 | na | na | na | na | na | na | na | na | na |
| OR Quart 3 | >3.1 | >4.1 | >5.6 | >3.1 | >5.2 | >6.8 | >2.0 | >5.3 | >4.3 |
| p Value | <0.33 | <0.21 | <0.12 | <0.33 | <0.13 | <0.081 | <0.57 | <0.13 | <0.20 |
| 95% CI of | >0.32 | >0.45 | >0.63 | >0.32 | >0.60 | >0.79 | >0.18 | >0.60 | >0.46 |
| OR Quart3 | na | na | na | na | na | na | na | na | na |
| OR Quart 4 | >38 | >7.5 | >24 | >38 | >6.3 | >22 | >15 | >2.0 | >9.4 |
| p Value | <4.8E-4 | <0.062 | <0.0027 | <4.8E-4 | <0.090 | <0.0036 | <0.011 | <0.57 | <0.038 |
| 95% CI of | >4.9 | >0.90 | >3.0 | >4.9 | >0.75 | >2.7 | >1.9 | >0.18 | >1.1 |
| OR Quart4 | na | na | na | na | na | na | na | na | na |

| | | | | | | | | | |
|---|---|---|---|---|---|---|---|---|---|
| **C-X-C motif chemokine 13** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 0.224 | | 9.29 | 0.224 | | 8.44 | 0.224 | | 3.11 |
| Average | 10.4 | | 107 | 10.4 | | 104 | 10.4 | | 39.9 |
| Stdev | 61.2 | | 355 | 61.2 | | 341 | 61.2 | | 109 |
| p(t-test) | | | 2.4E-4 | | | 2.5E-4 | | | 0.082 |
| Min | 0.00269 | | 0.0163 | 0.00269 | | 0.0163 | 0.00269 | | 0.0163 |
| Max | 832 | | 1930 | 832 | | 1850 | 832 | | 440 |
| n (Samp) | 223 | | 30 | 223 | | 30 | 223 | | 16 |
| n (Patient) | 223 | | 30 | 223 | | 30 | 223 | | 16 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 0.772 | | 8.40 | 0.772 | | 5.14 | 0.772 | | 5.14 |
| Average | 11.8 | | 18.6 | 11.8 | | 16.7 | 11.8 | | 15.2 |
| Stdev | 53.0 | | 27.8 | 53.0 | | 27.8 | 53.0 | | 28.4 |
| p(t-test) | | | 0.65 | | | 0.74 | | | 0.87 |
| Min | 0.00269 | | 0.0174 | 0.00269 | | 0.0174 | 0.00269 | | 0.0222 |
| Max | 832 | | 80.5 | 832 | | 79.0 | 832 | | 79.0 |
| n (Samp) | 375 | | 13 | 375 | | 13 | 375 | | 7 |
| n (Patient) | 375 | | 13 | 375 | | 13 | 375 | | 7 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 0.392 | | 23.5 | 0.392 | | 20.4 | 0.392 | | 2.87 |
| Average | 12.0 | | 139 | 12.0 | | 135 | 12.0 | | 45.2 |
| Stdev | 68.6 | | 402 | 68.6 | | 387 | 68.6 | | 117 |
| p(t-test) | | | 1.9E-4 | | | 1.9E-4 | | | 0.10 |
| Min | 0.00269 | | 0.0163 | 0.00269 | | 0.0163 | 0.00269 | | 0.0163 |
| Max | 832 | | 1930 | 832 | | 1850 | 832 | | 440 |
| n (Samp) | 173 | | 23 | 173 | | 23 | 173 | | 14 |
| n (Patient) | 173 | | 23 | 173 | | 23 | 173 | | 14 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.82 | 0.73 | 0.83 | 0.81 | 0.71 | 0.82 | 0.73 | 0.70 | 0.72 |
| SE | 0.048 | 0.081 | 0.054 | 0.049 | 0.082 | 0.055 | 0.073 | 0.11 | 0.079 |
| p | 5.8E-11 | 0.0040 | 1.5E-9 | 4.8E-10 | 0.011 | 3.4E-9 | 0.0014 | 0.078 | 0.0048 |
| nCohort 1 | 223 | 375 | 173 | 223 | 375 | 173 | 223 | 375 | 173 |
| nCohort 2 | 30 | 13 | 23 | 30 | 13 | 23 | 16 | 7 | 14 |
| Cutoff 1 | 4.27 | 2.65 | 2.84 | 2.84 | 2.57 | 2.84 | 1.18 | 3.20 | 2.32 |
| Sens 1 | 70% | 77% | 74% | 70% | 77% | 74% | 75% | 71% | 71% |
| Spec 1 | 80% | 67% | 71% | 74% | 66% | 71% | 65% | 70% | 70% |
| Cutoff 2 | 2.65 | 0.449 | 2.65 | 2.49 | 0.449 | 2.65 | 0.448 | 0.449 | 0.448 |
| Sens 2 | 80% | 85% | 83% | 80% | 85% | 83% | 81% | 86% | 86% |
| Spec 2 | 74% | 45% | 71% | 73% | 45% | 71% | 56% | 45% | 51% |
| Cutoff 3 | 0.0193 | 0.0193 | 0.448 | 0.0193 | 0.0193 | 0.448 | 0.0155 | 0.0193 | 0.0155 |
| Sens 3 | 90% | 92% | 91% | 90% | 92% | 91% | 100% | 100% | 100% |
| Spec 3 | 42% | 32% | 51% | 42% | 32% | 51% | 39% | 32% | 31% |
| Cutoff 4 | 1.82 | 3.20 | 2.49 | 1.82 | 3.20 | 2.49 | 1.82 | 3.20 | 2.49 |
| Sens 4 | 80% | 69% | 83% | 80% | 62% | 83% | 69% | 71% | 64% |
| Spec 4 | 70% | 70% | 71% | 70% | 70% | 71% | 70% | 70% | 71% |
| Cutoff 5 | 4.64 | 7.41 | 5.36 | 4.64 | 7.41 | 5.36 | 4.64 | 7.41 | 5.36 |
| Sens 5 | 67% | 54% | 65% | 60% | 46% | 65% | 38% | 43% | 36% |
| Spec 5 | 80% | 80% | 80% | 80% | 80% | 80% | 80% | 80% | 80% |
| Cutoff 6 | 12.3 | 18.7 | 14.4 | 12.3 | 18.7 | 14.4 | 12.3 | 18.7 | 14.4 |
| Sens 6 | 47% | 31% | 57% | 43% | 23% | 52% | 25% | 14% | 29% |
| Spec 6 | 90% | 90% | 90% | 90% | 90% | 90% | 90% | 90% | 90% |
| OR Quart 2 | >4.3 | >3.1 | >3.2 | >4.3 | >3.1 | >3.2 | >3.1 | >2.0 | >3.1 |
| p Value | <0.20 | <0.33 | <0.32 | <0.20 | <0.33 | <0.32 | <0.33 | <0.57 | <0.33 |
| 95% CI of | >0.46 | >0.32 | >0.32 | >0.46 | >0.32 | >0.32 | >0.31 | >0.18 | >0.31 |
| OR Quart2 | na | na | na | na | na | na | na | na | na |
| OR Quart 3 | >6.6 | >1.0 | >5.6 | >7.9 | >3.1 | >5.6 | >6.6 | >1.0 | >5.5 |
| p Value | <0.084 | <0.99 | <0.12 | <0.057 | <0.33 | <0.12 | <0.086 | <0.99 | <0.13 |
| 95% CI of | >0.77 | >0.062 | >0.63 | >0.94 | >0.32 | >0.63 | >0.76 | >0.062 | >0.61 |
| OR Quart3 | na | na | na | na | na | na | na | na | na |
| OR Quart 4 | >29 | >9.9 | >22 | >27 | >7.5 | >22 | >7.8 | >4.1 | >6.7 |
| p Value | <0.0013 | <0.031 | <0.0036 | <0.0017 | <0.061 | <0.0036 | <0.059 | <0.21 | <0.083 |
| 95% CI of | >3.7 | >1.2 | >2.7 | >3.4 | >0.91 | >2.7 | >0.93 | >0.45 | >0.78 |
| OR Quart4 | na | na | na | na | na | na | na | na | na |

| **Insulin-like growth factor-binding protein 3** | | | | | | | | | |
|---|---|---|---|---|---|---|---|---|---|
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 375 | | 686 | 375 | | 686 | 375 | | 609 |
| Average | 692 | | 2080 | 692 | | 1580 | 692 | | 840 |
| Stdev | 1130 | | 3120 | 1130 | | 2400 | 1130 | | 578 |
| p(t-test) | | | 1.1E-6 | | | 3.9E-4 | | | 0.55 |
| Min | 3.84 | | 108 | 3.84 | | 88.0 | 3.84 | | 186 |
| Max | 12500 | | 12500 | 12500 | | 12500 | 12500 | | 2060 |
| n (Samp) | 235 | | 35 | 235 | | 35 | 235 | | 21 |
| n (Patient) | 235 | | 35 | 235 | | 35 | 235 | | 21 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 574 | | 862 | 574 | | 862 | 574 | | 1000 |
| Average | 949 | | 1440 | 949 | | 1370 | 949 | | 1640 |
| Stdev | 1480 | | 1900 | 1480 | | 1860 | 1480 | | 2210 |
| p(t-test) | | | 0.19 | | | 0.26 | | | 0.13 |
| Min | 3.84 | | 108 | 3.84 | | 88.0 | 3.84 | | 411 |
| Max | 12500 | | 8160 | 12500 | | 8160 | 12500 | | 8160 |
| n (Samp) | 397 | | 17 | 397 | | 17 | 397 | | 11 |
| n (Patient) | 397 | | 17 | 397 | | 17 | 397 | | 11 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 434 | | 993 | 434 | | 993 | 434 | | 533 |
| Average | 846 | | 2640 | 846 | | 1930 | 846 | | 792 |
| Stdev | 1490 | | 3590 | 1490 | | 2790 | 1490 | | 631 |
| p(t-test) | | | 9.6E-6 | | | 0.0028 | | | 0.89 |
| Min | 3.84 | | 186 | 3.84 | | 186 | 3.84 | | 186 |
| Max | 12500 | | 12500 | 12500 | | 12500 | 12500 | | 2060 |
| n (Samp) | 190 | | 25 | 190 | | 25 | 190 | | 16 |
| n (Patient) | 190 | | 25 | 190 | | 25 | 190 | | 16 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.70 | 0.62 | 0.70 | 0.69 | 0.61 | 0.69 | 0.66 | 0.68 | 0.58 |
| SE | 0.052 | 0.074 | 0.061 | 0.052 | 0.074 | 0.062 | 0.067 | 0.090 | 0.077 |
| p | 7.3E-5 | 0.11 | 0.0011 | 2.0E-4 | 0.14 | 0.0025 | 0.019 | 0.049 | 0.33 |
| nCohort 1 | 235 | 397 | 190 | 235 | 397 | 190 | 235 | 397 | 190 |
| nCohort 2 | 35 | 17 | 25 | 35 | 17 | 25 | 21 | 11 | 16 |
| Cutoff 1 | 478 | 543 | 478 | 452 | 543 | 452 | 452 | 662 | 293 |
| Sens 1 | 71% | 71% | 72% | 71% | 71% | 72% | 71% | 73% | 75% |
| Spec 1 | 58% | 49% | 54% | 57% | 49% | 53% | 57% | 55% | 36% |
| Cutoff 2 | 388 | 411 | 388 | 323 | 411 | 323 | 323 | 543 | 267 |
| Sens 2 | 80% | 82% | 80% | 80% | 82% | 80% | 81% | 82% | 81% |
| Spec 2 | 51% | 40% | 46% | 44% | 40% | 38% | 44% | 49% | 33% |
| Cutoff 3 | 258 | 108 | 267 | 238 | 108 | 257 | 258 | 456 | 193 |
| Sens 3 | 91% | 94% | 92% | 91% | 94% | 92% | 90% | 91% | 94% |
| Spec 3 | 37% | 14% | 33% | 34% | 14% | 31% | 37% | 44% | 24% |
| Cutoff 4 | 736 | 1060 | 836 | 736 | 1060 | 836 | 736 | 1060 | 836 |
| Sens 4 | 49% | 41% | 52% | 49% | 41% | 52% | 43% | 45% | 38% |
| Spec 4 | 70% | 70% | 70% | 70% | 70% | 70% | 70% | 70% | 70% |
| Cutoff 5 | 1100 | 1380 | 1130 | 1100 | 1380 | 1130 | 1100 | 1380 | 1130 |
| Sens 5 | 40% | 35% | 44% | 40% | 29% | 44% | 29% | 27% | 25% |
| Spec 5 | 80% | 80% | 80% | 80% | 80% | 80% | 80% | 80% | 80% |
| Cutoff 6 | 1500 | 1870 | 1560 | 1500 | 1870 | 1560 | 1500 | 1870 | 1560 |
| Sens 6 | 34% | 24% | 40% | 34% | 18% | 40% | 24% | 18% | 12% |
| Spec 6 | 90% | 90% | 90% | 90% | 90% | 90% | 90% | 90% | 90% |
| OR Quart 2 | 3.7 | 2.0 | 5.3 | 3.7 | 2.0 | 2.6 | >5.4 | >3.1 | 2.0 |
| p Value | 0.11 | 0.42 | 0.13 | 0.11 | 0.42 | 0.27 | <0.13 | <0.33 | 0.42 |
| 95% CI of | 0.75 | 0.36 | 0.60 | 0.75 | 0.36 | 0.48 | >0.62 | >0.32 | 0.36 |
| OR Quart2 | 19 | 11 | 47 | 19 | 11 | 14 | na | na | 12 |
| OR Quart 3 | 7.1 | 2.0 | 9.0 | 7.1 | 2.0 | 3.8 | >9.1 | >4.2 | 3.3 |
| p Value | 0.013 | 0.42 | 0.041 | 0.013 | 0.42 | 0.11 | <0.040 | <0.21 | 0.16 |
| 95% CI of | 1.5 | 0.37 | 1.1 | 1.5 | 0.37 | 0.75 | >1.1 | >0.46 | 0.63 |
| OR Quart3 | 33 | 11 | 75 | 33 | 11 | 19 | na | na | 17 |
| OR Quart 4 | 8.4 | 3.6 | 13 | 8.4 | 3.6 | 6.5 | >9.1 | >4.2 | 2.0 |
| p Value | 0.0062 | 0.11 | 0.015 | 0.0062 | 0.11 | 0.018 | <0.040 | <0.21 | 0.42 |
| 95% CI of | 1.8 | 0.74 | 1.7 | 1.8 | 0.74 | 1.4 | >1.1 | >0.46 | 0.36 |
| OR Quart4 | 39 | 18 | 110 | 39 | 18 | 31 | na | na | 12 |

| | | | | | | | | | |
|---|---|---|---|---|---|---|---|---|---|
| **Immunoglogulin G1** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 3510 | | 14800 | 3510 | | 14600 | 3510 | | 9360 |
| Average | 6360 | | 16700 | 6360 | | 15900 | 6360 | | 11200 |
| Stdev | 8760 | | 14300 | 8760 | | 14100 | 8760 | | 6710 |
| p(t-test) | | | 6.8E-8 | | | 5.0E-7 | | | 0.032 |
| Min | 59.7 | | 980 | 59.7 | | 980 | 59.7 | | 2380 |
| Max | 80000 | | 80000 | 80000 | | 80000 | 80000 | | 25300 |
| n (Samp) | 222 | | 30 | 222 | | 30 | 222 | | 16 |
| n (Patient) | 222 | | 30 | 222 | | 30 | 222 | | 16 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 4660 | | 11200 | 4660 | | 11200 | 4660 | | 9840 |
| Average | 7920 | | 13300 | 7920 | | 12300 | 7920 | | 10200 |
| Stdev | 9560 | | 8270 | 9560 | | 7830 | 9560 | | 4610 |
| p(t-test) | | | 0.046 | | | 0.099 | | | 0.53 |
| Min | 59.7 | | 980 | 59.7 | | 980 | 59.7 | | 2880 |
| Max | 80000 | | 28300 | 80000 | | 28300 | 80000 | | 15300 |
| n (Samp) | 373 | | 13 | 373 | | 13 | 373 | | 7 |
| n (Patient) | 373 | | 13 | 373 | | 13 | 373 | | 7 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 3840 | | 14900 | 3840 | | 14800 | 3840 | | 10900 |
| Average | 6750 | | 18700 | 6750 | | 18000 | 6750 | | 11800 |
| Stdev | 9200 | | 15600 | 9200 | | 15300 | 9200 | | 7010 |
| p(t-test) | | | 2.8E-7 | | | 1.2E-6 | | | 0.046 |
| Min | 59.7 | | 2880 | 59.7 | | 2880 | 59.7 | | 2380 |
| Max | 80000 | | 80000 | 80000 | | 80000 | 80000 | | 25300 |
| n(Samp) | 171 | | 23 | 171 | | 23 | 171 | | 14 |
| n (Patient) | 171 | | 23 | 171 | | 23 | 171 | | 14 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.84 | 0.73 | 0.86 | 0.83 | 0.71 | 0.86 | 0.77 | 0.70 | 0.76 |
| SE | 0.047 | 0.081 | 0.050 | 0.048 | 0.082 | 0.051 | 0.070 | 0.11 | 0.076 |
| p | 5.5E-13 | 0.0051 | 4.7E-13 | 7.0E-12 | 0.012 | 1.8E-12 | 1.1E-4 | 0.077 | 5.1E-4 |
| nCohort 1 | 222 | 373 | 171 | 222 | 373 | 171 | 222 | 373 | 171 |
| nCohort 2 | 30 | 13 | 23 | 30 | 13 | 23 | 16 | 7 | 14 |
| Cutoff 1 | 9450 | 7480 | 11000 | 9450 | 6410 | 11000 | 6410 | 8560 | 8690 |
| Sens 1 | 70% | 77% | 74% | 70% | 77% | 74% | 75% | 71% | 71% |
| Spec 1 | 80% | 66% | 85% | 80% | 61% | 85% | 72% | 70% | 80% |
| Cutoff 2 | 8150 | 6410 | 8710 | 8150 | 5190 | 8710 | 6150 | 6410 | 3840 |
| Sens 2 | 80% | 85% | 83% | 80% | 85% | 83% | 81% | 86% | 86% |
| Spec 2 | 77% | 61% | 80% | 77% | 53% | 80% | 72% | 61% | 50% |
| Cutoff 3 | 6150 | 2840 | 6150 | 5190 | 2840 | 6150 | 2840 | 2840 | 2840 |
| Sens 3 | 90% | 92% | 91% | 90% | 92% | 91% | 94% | 100% | 93% |
| Spec 3 | 72% | 32% | 71% | 64% | 32% | 71% | 43% | 32% | 38% |
| Cutoff 4 | 5990 | 8650 | 6090 | 5990 | 8650 | 6090 | 5990 | 8650 | 6090 |
| Sens 4 | 90% | 62% | 91% | 87% | 62% | 91% | 81% | 57% | 79% |
| Spec 4 | 70% | 70% | 70% | 70% | 70% | 70% | 70% | 70% | 70% |
| Cutoff 5 | 9450 | 12900 | 8710 | 9450 | 12900 | 8710 | 9450 | 12900 | 8710 |
| Sens 5 | 70% | 46% | 83% | 70% | 46% | 83% | 50% | 43% | 64% |
| Spec 5 | 80% | 80% | 80% | 80% | 80% | 80% | 80% | 80% | 80% |
| Cutoff 6 | 15800 | 17100 | 15800 | 15800 | 17100 | 15800 | 15800 | 17100 | 15800 |
| Sens 6 | 40% | 38% | 43% | 30% | 23% | 35% | 19% | 0% | 21% |
| Spec 6 | 90% | 90% | 90% | 90% | 90% | 90% | 90% | 90% | 90% |
| OR Quart 2 | 1.0 | 0.99 | >1.0 | 1.0 | 0.99 | >1.0 | >2.0 | >1.0 | >3.2 |
| p Value | 1.0 | 0.99 | <1.0 | 1.0 | 0.99 | <1.0 | <0.57 | <0.99 | <0.32 |
| 95% CI of | 0.061 | 0.061 | >0.061 | 0.061 | 0.061 | >0.061 | >0.18 | >0.062 | >0.32 |
| OR Quart2 | 16 | 16 | na | 16 | 16 | na | na | na | na |
| OR Quart 3 | 9.0 | 4.1 | >5.6 | 9.0 | 4.1 | >5.6 | >3.2 | >3.1 | >1.0 |
| p Value | 0.041 | 0.21 | <0.12 | 0.041 | 0.21 | <0.12 | <0.33 | <0.33 | <0.99 |
| 95% CI of | 1.1 | 0.45 | >0.63 | 1.1 | 0.45 | >0.63 | >0.32 | >0.32 | >0.062 |
| OR Quart3 | 74 | 38 | na | 74 | 38 | na | na | na | na |
| OR Quart 4 | 29 | 7.4 | >26 | 29 | 7.4 | >26 | >13 | >3.1 | >12 |
| p Value | 0.0013 | 0.064 | <0.0021 | 0.0013 | 0.064 | <0.0021 | <0.015 | <0.33 | <0.019 |
| 95% CI of | 3.7 | 0.89 | >3.2 | 3.7 | 0.89 | >3.2 | >1.7 | >0.32 | >1.5 |
| OR Quart4 | 220 | 61 | na | 220 | 61 | na | na | na | na |

| **Immunoglogulin G2** | | | | | | | | | |
|---|---|---|---|---|---|---|---|---|---|
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 9710 | | 38600 | 9710 | | 36400 | 9710 | | 27800 |
| Average | 20300 | | 71300 | 20300 | | 65400 | 20300 | | 46400 |
| Stdev | 34400 | | 73800 | 34400 | | 72200 | 34400 | | 58500 |
| p(t-test) | | | 7.4E-10 | | | 3.2E-8 | | | 0.0059 |
| Min | 119 | | 2380 | 119 | | 2380 | 119 | | 4940 |
| Max | 240000 | | 240000 | 240000 | | 240000 | 240000 | | 240000 |
| n (Samp) | 222 | | 30 | 222 | | 30 | 222 | | 16 |
| n (Patient) | 222 | | 30 | 222 | | 30 | 222 | | 16 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 13000 | | 52100 | 13000 | | 36800 | 13000 | | 20100 |
| Average | 26100 | | 73800 | 26100 | | 62100 | 26100 | | 62000 |
| Stdev | 40800 | | 71300 | 40800 | | 67800 | 40800 | | 84800 |
| p(t-test) | | | 7.0E-5 | | | 0.0025 | | | 0.025 |
| Min | 119 | | 2380 | 119 | | 2380 | 119 | | 4940 |
| Max | 240000 | | 240000 | 240000 | | 240000 | 240000 | | 240000 |
| n (Samp) | 373 | | 13 | 373 | | 13 | 373 | | 7 |
| n (Patient) | 373 | | 13 | 373 | | 13 | 373 | | 7 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 10700 | | 40500 | 10700 | | 40500 | 10700 | | 35700 |
| Average | 22500 | | 81500 | 22500 | | 77900 | 22500 | | 54600 |
| Stdev | 39100 | | 84000 | 39100 | | 81100 | 39100 | | 62500 |
| p(t-test) | | | 4.1E-8 | | | 1.7E-7 | | | 0.0056 |
| Min | 334 | | 8300 | 334 | | 8300 | 334 | | 8300 |
| Max | 240000 | | 240000 | 240000 | | 240000 | 240000 | | 240000 |
| n(Samp) | 171 | | 23 | 171 | | 23 | 171 | | 14 |
| n (Patient) | 171 | | 23 | 171 | | 23 | 171 | | 14 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.80 | 0.73 | 0.81 | 0.79 | 0.70 | 0.81 | 0.74 | 0.65 | 0.76 |
| SE | 0.050 | 0.081 | 0.056 | 0.051 | 0.083 | 0.056 | 0.073 | 0.11 | 0.077 |
| p | 3.6E-9 | 0.0041 | 1.7E-8 | 1.2E-8 | 0.018 | 2.2E-8 | 9.4E-4 | 0.18 | 8.5E-4 |
| nCohort 1 | 222 | 373 | 171 | 222 | 373 | 171 | 222 | 373 | 171 |
| nCohort 2 | 30 | 13 | 23 | 30 | 13 | 23 | 16 | 7 | 14 |
| Cutoff 1 | 19300 | 20100 | 14800 | 19300 | 13300 | 14800 | 13700 | 13300 | 14800 |
| Sens 1 | 70% | 77% | 74% | 70% | 77% | 74% | 75% | 71% | 71% |
| Spec 1 | 73% | 67% | 65% | 73% | 52% | 65% | 64% | 52% | 65% |
| Cutoff 2 | 14500 | 8240 | 14500 | 14500 | 8240 | 14500 | 9820 | 8240 | 9820 |
| Sens 2 | 80% | 85% | 83% | 80% | 85% | 83% | 81% | 86% | 86% |
| Spec 2 | 66% | 33% | 64% | 66% | 33% | 64% | 50% | 33% | 46% |
| Cutoff 3 | 8830 | 4910 | 12300 | 8830 | 4910 | 12300 | 8240 | 4910 | 8540 |
| Sens 3 | 90% | 92% | 91% | 90% | 92% | 91% | 94% | 100% | 93% |
| Spec 3 | 48% | 19% | 54% | 48% | 19% | 54% | 45% | 19% | 42% |
| Cutoff 4 | 17500 | 21800 | 18100 | 17500 | 21800 | 18100 | 17500 | 21800 | 18100 |
| Sens 4 | 70% | 69% | 70% | 70% | 62% | 70% | 56% | 43% | 57% |
| Spec 4 | 70% | 70% | 70% | 70% | 70% | 70% | 70% | 70% | 70% |
| Cutoff 5 | 23700 | 31900 | 22900 | 23700 | 31900 | 22900 | 23700 | 31900 | 22900 |
| Sens 5 | 63% | 62% | 65% | 60% | 54% | 65% | 50% | 43% | 57% |
| Spec 5 | 80% | 80% | 80% | 80% | 80% | 80% | 80% | 80% | 80% |
| Cutoff 6 | 40900 | 52000 | 44400 | 40900 | 52000 | 44400 | 40900 | 52000 | 44400 |
| Sens 6 | 47% | 54% | 48% | 43% | 46% | 48% | 31% | 43% | 36% |
| Spec 6 | 90% | 90% | 90% | 90% | 90% | 90% | 90% | 90% | 90% |
| OR Quart 2 | 3.1 | 0.49 | >3.1 | 3.1 | 0.49 | >3.1 | >4.2 | 1.0 | >3.2 |
| p Value | 0.33 | 0.56 | <0.33 | 0.33 | 0.56 | <0.33 | <0.20 | 1.0 | <0.32 |
| 95% CI of | 0.31 | 0.044 | >0.31 | 0.31 | 0.044 | >0.31 | >0.46 | 0.062 | >0.32 |
| OR Quart2 | 31 | 5.5 | na | 31 | 5.5 | na | na | 16 | na |
| OR Quart 3 | 7.8 | 1.0 | >5.6 | 9.0 | 1.5 | >5.6 | >4.3 | 2.0 | >3.2 |
| p Value | 0.059 | 1.0 | <0.12 | 0.041 | 0.65 | <0.12 | <0.20 | 0.57 | <0.32 |
| 95% CI of | 0.92 | 0.14 | >0.63 | 1.1 | 0.25 | >0.63 | >0.47 | 0.18 | >0.32 |
| OR Quart3 | 65 | 7.2 | na | 74 | 9.3 | na | na | 23 | na |
| OR Quart 4 | 27 | 4.2 | >21 | 25 | 3.7 | >21 | >9.1 | 3.1 | >9.4 |
| p Value | 0.0017 | 0.073 | <0.0039 | 0.0021 | 0.11 | <0.0039 | <0.041 | 0.34 | <0.038 |
| 95% CI of | 3.5 | 0.87 | >2.7 | 3.2 | 0.74 | >2.7 | >1.1 | 0.31 | >1.1 |
| OR Quart4 | 210 | 20 | na | 190 | 18 | na | na | 30 | na |

| | | | | | | | | | |
|---|---|---|---|---|---|---|---|---|---|
| **Interleukin-11** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 129 | | 344 | 129 | | 270 | 129 | | 266 |
| Average | 208 | | 537 | 208 | | 472 | 208 | | 492 |
| Stdev | 256 | | 608 | 256 | | 560 | 256 | | 637 |
| p(t-test) | | | 2.2E-7 | | | 1.5E-5 | | | 2.6E-4 |
| Min | 0.154 | | 48.0 | 0.154 | | 48.0 | 0.154 | | 85.8 |
| Max | 1980 | | 2900 | 1980 | | 2590 | 1980 | | 2140 |
| n (Samp) | 223 | | 30 | 223 | | 30 | 223 | | 16 |
| n (Patient) | 223 | | 30 | 223 | | 30 | 223 | | 16 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 174 | | 295 | 174 | | 219 | 174 | | 212 |
| Average | 285 | | 322 | 285 | | 313 | 285 | | 237 |
| Stdev | 315 | | 259 | 315 | | 262 | 315 | | 112 |
| p(t-test) | | | 0.67 | | | 0.75 | | | 0.69 |
| Min | 0.154 | | 48.0 | 0.154 | | 48.0 | 0.154 | | 115 |
| Max | 2260 | | 1040 | 2260 | | 1040 | 2260 | | 436 |
| n (Samp) | 375 | | 13 | 375 | | 13 | 375 | | 7 |
| n (Patient) | 375 | | 13 | 375 | | 13 | 375 | | 7 |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 179 | | 396 | 179 | | 295 | 179 | | 321 |
| Average | 263 | | 664 | 263 | | 585 | 263 | | 582 |
| Stdev | 283 | | 683 | 283 | | 640 | 283 | | 682 |
| p(t-test) | | | 7.1E-7 | | | 3.7E-5 | | | 5.7E-4 |
| Min | 2.83 | | 90.9 | 2.83 | | 90.9 | 2.83 | | 85.8 |
| Max | 1980 | | 2900 | 1980 | | 2590 | 1980 | | 2140 |
| n(Samp) | 173 | | 23 | 173 | | 23 | 173 | | 14 |
| n (Patient) | 173 | | 23 | 173 | | 23 | 173 | | 14 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.77 | 0.60 | 0.76 | 0.74 | 0.59 | 0.71 | 0.73 | 0.57 | 0.68 |
| SE | 0.052 | 0.084 | 0.061 | 0.054 | 0.084 | 0.063 | 0.074 | 0.11 | 0.081 |
| p | 1.7E-7 | 0.23 | 2.4E-5 | 1.0E-5 | 0.29 | 0.0010 | 0.0019 | 0.54 | 0.026 |
| nCohort 1 | 223 | 375 | 173 | 223 | 375 | 173 | 223 | 375 | 173 |
| nCohort 2 | 30 | 13 | 23 | 30 | 13 | 23 | 16 | 7 | 14 |
| Cutoff 1 | 230 | 135 | 250 | 180 | 135 | 212 | 143 | 179 | 235 |
| Sens 1 | 70% | 77% | 74% | 70% | 77% | 74% | 75% | 71% | 71% |
| Spec 1 | 73% | 40% | 64% | 64% | 40% | 58% | 57% | 52% | 62% |
| Cutoff 2 | 143 | 114 | 212 | 135 | 114 | 143 | 135 | 135 | 114 |
| Sens 2 | 80% | 85% | 83% | 80% | 85% | 83% | 81% | 86% | 86% |
| Spec 2 | 57% | 33% | 58% | 55% | 33% | 44% | 55% | 40% | 34% |
| Cutoff 3 | 114 | 110 | 128 | 110 | 110 | 114 | 85.8 | 114 | 89.1 |
| Sens 3 | 90% | 92% | 91% | 90% | 92% | 91% | 94% | 100% | 93% |
| Spec 3 | 47% | 32% | 36% | 46% | 32% | 34% | 37% | 33% | 24% |
| Cutoff 4 | 212 | 301 | 299 | 212 | 301 | 299 | 212 | 301 | 299 |
| Sens 4 | 77% | 38% | 61% | 67% | 38% | 48% | 62% | 14% | 50% |
| Spec 4 | 70% | 70% | 71% | 70% | 70% | 71% | 70% | 70% | 71% |
| Cutoff 5 | 301 | 461 | 383 | 301 | 461 | 383 | 301 | 461 | 383 |
| Sens 5 | 53% | 15% | 52% | 43% | 15% | 43% | 44% | 0% | 43% |
| Spec 5 | 80% | 80% | 80% | 80% | 80% | 80% | 80% | 80% | 80% |
| Cutoff 6 | 487 | 694 | 569 | 487 | 694 | 569 | 487 | 694 | 569 |
| Sens 6 | 33% | 8% | 39% | 30% | 8% | 35% | 19% | 0% | 29% |
| Spec 6 | 90% | 90% | 90% | 90% | 90% | 90% | 90% | 90% | 90% |
| OR Quart 2 | 4.2 | 3.1 | 3.1 | 4.2 | 3.1 | 2.1 | >3.1 | >2.0 | 0.98 |
| p Value | 0.20 | 0.34 | 0.33 | 0.20 | 0.34 | 0.41 | <0.33 | <0.57 | 0.98 |
| 95% CI of | 0.46 | 0.31 | 0.31 | 0.46 | 0.31 | 0.36 | >0.31 | >0.18 | 0.13 |
| OR Quart2 | 39 | 30 | 31 | 39 | 30 | 12 | na | na | 7.3 |
| OR Quart 3 | 7.8 | 5.2 | 8.0 | 12 | 5.2 | 3.3 | >5.4 | >4.2 | 1.5 |
| p Value | 0.059 | 0.13 | 0.056 | 0.021 | 0.13 | 0.16 | <0.13 | <0.20 | 0.67 |
| 95% CI of | 0.92 | 0.60 | 0.95 | 1.4 | 0.60 | 0.63 | >0.61 | >0.46 | 0.24 |
| OR Quart3 | 65 | 46 | 68 | 94 | 46 | 17 | na | na | 9.4 |
| OR Quart 4 | 24 | 4.1 | 16 | 19 | 4.1 | 6.8 | >9.1 | >1.0 | 3.8 |
| p Value | 0.0023 | 0.21 | 0.0098 | 0.0051 | 0.21 | 0.016 | <0.041 | <1.0 | 0.10 |
| 95% CI of | 3.1 | 0.45 | 1.9 | 2.4 | 0.45 | 1.4 | >1.1 | >0.062 | 0.76 |
| OR Quart4 | 190 | 38 | 130 | 150 | 38 | 33 | na | na | 20 |

| **Interleukin-2 receptor alpha chain** | | | | | | | | | |
|---|---|---|---|---|---|---|---|---|---|
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 634 | | 1730 | 634 | | 1720 | 634 | | 980 |
| Average | 1020 | | 2350 | 1020 | | 2180 | 1020 | | 2070 |
| Stdev | 1210 | | 2340 | 1210 | | 2280 | 1210 | | 2470 |
| p(t-test) | | | 1.6E-6 | | | 2.4E-5 | | | 0.0026 |
| Min | 0.0708 | | 0.118 | 0.0708 | | 0.118 | 0.0708 | | 437 |
| Max | 10400 | | 10400 | 10400 | | 10400 | 10400 | | 10400 |
| n (Samp) | 223 | | 30 | 223 | | 30 | 223 | | 16 |
| n (Patient) | 223 | | 30 | 223 | | 30 | 223 | | 16 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 880 | | 1300 | 880 | | 1300 | 880 | | 2450 |
| Average | 1390 | | 2050 | 1390 | | 2040 | 1390 | | 2570 |
| Stdev | 1620 | | 2100 | 1620 | | 2100 | 1620 | | 2370 |
| p(t-test) | | | 0.16 | | | 0.16 | | | 0.059 |
| Min | 0.0708 | | 0.118 | 0.0708 | | 0.118 | 0.0708 | | 437 |
| Max | 10400 | | 7190 | 10400 | | 7190 | 10400 | | 7190 |
| n (Samp) | 375 | | 13 | 375 | | 13 | 375 | | 7 |
| n (Patient) | 375 | | 13 | 375 | | 13 | 375 | | 7 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 767 | | 2490 | 767 | | 2200 | 767 | | 1140 |
| Average | 1130 | | 2710 | 1130 | | 2480 | 1130 | | 2170 |
| Stdev | 1300 | | 2450 | 1300 | | 2410 | 1300 | | 2610 |
| p(t-test) | | | 3.0E-6 | | | 5.1E-5 | | | 0.0095 |
| Min | 0.116 | | 50.9 | 0.116 | | 50.9 | 0.116 | | 437 |
| Max | 10400 | | 10400 | 10400 | | 10400 | 10400 | | 10400 |
| n(Samp) | 173 | | 23 | 173 | | 23 | 173 | | 14 |
| n (Patient) | 173 | | 23 | 173 | | 23 | 173 | | 14 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.70 | 0.59 | 0.74 | 0.69 | 0.59 | 0.73 | 0.70 | 0.69 | 0.68 |
| SE | 0.056 | 0.084 | 0.061 | 0.056 | 0.084 | 0.063 | 0.075 | 0.11 | 0.082 |
| p | 2.8E-4 | 0.31 | 7.2E-5 | 6.9E-4 | 0.31 | 3.1E-4 | 0.0094 | 0.098 | 0.030 |
| nCohort 1 | 223 | 375 | 173 | 223 | 375 | 173 | 223 | 375 | 173 |
| nCohort 2 | 30 | 13 | 23 | 30 | 13 | 23 | 16 | 7 | 14 |
| Cutoff 1 | 750 | 493 | 1070 | 750 | 493 | 954 | 712 | 960 | 750 |
| Sens 1 | 70% | 77% | 74% | 70% | 77% | 74% | 75% | 71% | 71% |
| Spec 1 | 53% | 32% | 63% | 53% | 32% | 59% | 52% | 53% | 49% |
| Cutoff 2 | 491 | 420 | 491 | 491 | 420 | 491 | 594 | 594 | 491 |
| Sens 2 | 80% | 85% | 83% | 80% | 85% | 83% | 81% | 86% | 86% |
| Spec 2 | 44% | 29% | 41% | 44% | 29% | 41% | 48% | 38% | 41% |
| Cutoff 3 | 384 | 55.0 | 411 | 384 | 55.0 | 411 | 484 | 420 | 484 |
| Sens 3 | 90% | 92% | 91% | 90% | 92% | 91% | 94% | 100% | 93% |
| Spec 3 | 40% | 9% | 38% | 40% | 9% | 38% | 44% | 29% | 41% |
| Cutoff 4 | 1250 | 1600 | 1270 | 1250 | 1600 | 1270 | 1250 | 1600 | 1270 |
| Sens 4 | 60% | 46% | 65% | 57% | 46% | 61% | 44% | 57% | 50% |
| Spec 4 | 70% | 70% | 71% | 70% | 70% | 71% | 70% | 70% | 71% |
| Cutoff 5 | 1690 | 2070 | 1890 | 1690 | 2070 | 1890 | 1690 | 2070 | 1890 |
| Sens 5 | 50% | 46% | 57% | 50% | 46% | 57% | 44% | 57% | 43% |
| Spec 5 | 80% | 80% | 80% | 80% | 80% | 80% | 80% | 80% | 80% |
| Cutoff 6 | 2530 | 3270 | 2620 | 2530 | 3270 | 2620 | 2530 | 3270 | 2620 |
| Sens 6 | 37% | 23% | 43% | 27% | 23% | 30% | 19% | 29% | 21% |
| Spec 6 | 90% | 90% | 90% | 90% | 90% | 90% | 90% | 90% | 90% |
| OR Quart 2 | 2.1 | 1.5 | 5.5 | 2.1 | 1.5 | 5.5 | >4.2 | >2.0 | >5.5 |
| p Value | 0.31 | 0.65 | 0.13 | 0.31 | 0.65 | 0.13 | <0.20 | <0.57 | <0.13 |
| 95% CI of | 0.50 | 0.25 | 0.61 | 0.50 | 0.25 | 0.61 | >0.46 | >0.18 | >0.61 |
| OR Quart2 | 8.8 | 9.3 | 49 | 8.8 | 9.3 | 49 | na | na | na |
| OR Quart 3 | 2.1 | 1.0 | 4.3 | 2.1 | 1.0 | 4.3 | >5.4 | >1.0 | >3.1 |
| p Value | 0.31 | 1.0 | 0.20 | 0.31 | 1.0 | 0.20 | <0.13 | <0.99 | <0.33 |
| 95% CI of | 0.50 | 0.14 | 0.46 | 0.50 | 0.14 | 0.46 | >0.61 | >0.062 | >0.31 |
| OR Quart3 | 8.8 | 7.2 | 40 | 8.8 | 7.2 | 40 | na | na | na |
| OR Quart 4 | 6.1 | 3.1 | 17 | 6.1 | 3.1 | 17 | >7.8 | >4.1 | >6.7 |
| p Value | 0.0061 | 0.17 | 0.0072 | 0.0061 | 0.17 | 0.0072 | <0.059 | <0.21 | <0.083 |
| 95% CI of | 1.7 | 0.62 | 2.2 | 1.7 | 0.62 | 2.2 | >0.93 | >0.45 | >0.78 |
| OR Quart4 | 22 | 16 | 140 | 22 | 16 | 140 | na | na | na |

| | | | | | | | | | |
|---|---|---|---|---|---|---|---|---|---|
| **Neutrophil collagenase** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 3750 | | 21100 | 3750 | | 20800 | 3750 | | 20800 |
| Average | 17900 | | 92500 | 17900 | | 89700 | 17900 | | 60700 |
| Stdev | 56800 | | 164000 | 56800 | | 165000 | 56800 | | 137000 |
| p(t-test) | | | 4.4E-7 | | | 1.1E-6 | | | 0.0059 |
| Min | 0.114 | | 25.8 | 0.114 | | 25.8 | 0.114 | | 580 |
| Max | 670000 | | 649000 | 670000 | | 649000 | 670000 | | 625000 |
| n (Samp) | 235 | | 34 | 235 | | 34 | 235 | | 20 |
| n (Patient) | 235 | | 34 | 235 | | 34 | 235 | | 20 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 6390 | | 10800 | 6390 | | 10800 | 6390 | | 21100 |
| Average | 25600 | | 113000 | 25600 | | 86800 | 25600 | | 37900 |
| Stdev | 65500 | | 215000 | 65500 | | 175000 | 65500 | | 47300 |
| p(t-test) | | | 5.6E-6 | | | 7.7E-4 | | | 0.54 |
| Min | 0.114 | | 25.8 | 0.114 | | 25.8 | 0.114 | | 2660 |
| Max | 670000 | | 649000 | 670000 | | 649000 | 670000 | | 151000 |
| n (Samp) | 398 | | 17 | 398 | | 17 | 398 | | 11 |
| n (Patient) | 398 | | 17 | 398 | | 17 | 398 | | 11 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 4250 | | 48900 | 4250 | | 41500 | 4250 | | 21000 |
| Average | 14300 | | 131000 | 14300 | | 110000 | 14300 | | 114000 |
| Stdev | 32500 | | 200000 | 32500 | | 178000 | 32500 | | 215000 |
| p(t-test) | | | 2.8E-12 | | | 1.9E-10 | | | 3.3E-8 |
| Min | 0.114 | | 580 | 0.114 | | 580 | 0.114 | | 580 |
| Max | 300000 | | 649000 | 300000 | | 649000 | 300000 | | 649000 |
| n (Samp) | 190 | | 24 | 190 | | 24 | 190 | | 15 |
| n (Patient) | 190 | | 24 | 190 | | 24 | 190 | | 15 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.75 | 0.61 | 0.82 | 0.74 | 0.60 | 0.81 | 0.75 | 0.67 | 0.76 |
| SE | 0.051 | 0.074 | 0.054 | 0.051 | 0.074 | 0.055 | 0.065 | 0.091 | 0.074 |
| p | 1.2E-6 | 0.13 | 1.8E-9 | 3.1E-6 | 0.18 | 1.4E-8 | 1.0E-4 | 0.057 | 3.2E-4 |
| nCohort 1 | 235 | 398 | 190 | 235 | 398 | 190 | 235 | 398 | 190 |
| nCohort 2 | 34 | 17 | 24 | 34 | 17 | 24 | 20 | 11 | 15 |
| Cutoff 1 | 10800 | 5530 | 20000 | 7010 | 5530 | 14600 | 7010 | 6050 | 7160 |
| Sens 1 | 71% | 71% | 71% | 71% | 71% | 71% | 70% | 73% | 73% |
| Spec 1 | 72% | 46% | 82% | 64% | 46% | 76% | 64% | 48% | 63% |
| Cutoff 2 | 3260 | 3170 | 11900 | 3260 | 3000 | 7160 | 5930 | 5930 | 5930 |
| Sens 2 | 82% | 82% | 83% | 82% | 82% | 83% | 80% | 82% | 80% |
| Spec 2 | 46% | 33% | 74% | 46% | 32% | 63% | 60% | 48% | 58% |
| Cutoff 3 | 2710 | 112 | 3260 | 2630 | 112 | 3260 | 3260 | 3170 | 3260 |
| Sens 3 | 91% | 94% | 92% | 91% | 94% | 92% | 90% | 91% | 93% |
| Spec 3 | 42% | 1% | 42% | 41% | 1% | 42% | 46% | 33% | 42% |
| Cutoff 4 | 9820 | 16300 | 9300 | 9820 | 16300 | 9300 | 9820 | 16300 | 9300 |
| Sens 4 | 71% | 47% | 83% | 68% | 47% | 79% | 65% | 55% | 67% |
| Spec 4 | 70% | 70% | 70% | 70% | 70% | 70% | 70% | 70% | 70% |
| Cutoff 5 | 18200 | 27300 | 19400 | 18200 | 27300 | 19400 | 18200 | 27300 | 19400 |
| Sens 5 | 59% | 35% | 71% | 56% | 35% | 67% | 55% | 36% | 60% |
| Spec 5 | 80% | 80% | 80% | 80% | 80% | 80% | 80% | 80% | 80% |
| Cutoff 6 | 33500 | 55700 | 32200 | 33500 | 55700 | 32200 | 33500 | 55700 | 32200 |
| Sens 6 | 41% | 29% | 54% | 41% | 29% | 54% | 35% | 27% | 47% |
| Spec 6 | 90% | 90% | 90% | 90% | 90% | 90% | 90% | 90% | 90% |
| OR Quart 2 | 1.4 | 3.1 | 2.0 | 1.4 | 3.1 | 2.0 | 2.0 | >4.2 | 1.0 |
| p Value | 0.70 | 0.17 | 0.58 | 0.70 | 0.17 | 0.58 | 0.58 | <0.21 | 1.0 |
| 95% CI of | 0.29 | 0.61 | 0.18 | 0.29 | 0.61 | 0.18 | 0.18 | >0.46 | 0.061 |
| OR Quart2 | 6.3 | 16 | 23 | 6.3 | 16 | 23 | 23 | na | 16 |
| OR Quart 3 | 2.5 | 0.99 | 4.2 | 2.9 | 0.99 | 5.4 | 6.4 | >2.0 | 4.3 |
| p Value | 0.20 | 0.99 | 0.20 | 0.13 | 0.99 | 0.13 | 0.090 | <0.56 | 0.20 |
| 95% CI of | 0.62 | 0.14 | 0.46 | 0.73 | 0.14 | 0.61 | 0.75 | >0.18 | 0.46 |
| OR Quart3 | 10 | 7.2 | 39 | 11 | 7.2 | 48 | 55 | na | 39 |
| OR Quart 4 | 8.9 | 3.6 | 24 | 8.3 | 3.6 | 22 | 13 | >5.2 | 10 |
| p Value | 7.5E-4 | 0.11 | 0.0025 | 0.0011 | 0.11 | 0.0034 | 0.016 | <0.14 | 0.029 |
| 95% CI of | 2.5 | 0.74 | 3.0 | 2.3 | 0.74 | 2.8 | 1.6 | >0.60 | 1.3 |
| OR Quart4 | 32 | 18 | 190 | 30 | 18 | 170 | 100 | na | 86 |

| **Protransforming growth factor alpha** | | | | | | | | | |
|---|---|---|---|---|---|---|---|---|---|
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 6.37 | | 16.8 | 6.37 | | 15.7 | 6.37 | | 14.5 |
| Average | 13.8 | | 25.7 | 13.8 | | 24.3 | 13.8 | | 25.1 |
| Stdev | 31.8 | | 23.8 | 31.8 | | 23.3 | 31.8 | | 28.7 |
| p(t-test) | | | 0.049 | | | 0.081 | | | 0.17 |
| Min | 0.00574 | | 2.98 | 0.00574 | | 1.41 | 0.00574 | | 5.53 |
| Max | 361 | | 122 | 361 | | 122 | 361 | | 122 |
| n (Samp) | 223 | | 30 | 223 | | 30 | 223 | | 16 |
| n (Patient) | 223 | | 30 | 223 | | 30 | 223 | | 16 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 9.29 | | 15.5 | 9.29 | | 15.5 | 9.29 | | 15.9 |
| Average | 16.5 | | 19.3 | 16.5 | | 18.4 | 16.5 | | 22.4 |
| Stdev | 29.1 | | 13.5 | 29.1 | | 13.8 | 29.1 | | 17.1 |
| p(t-test) | | | 0.73 | | | 0.81 | | | 0.59 |
| Min | 0.00574 | | 2.98 | 0.00574 | | 1.41 | 0.00574 | | 5.53 |
| Max | 361 | | 50.4 | 361 | | 50.4 | 361 | | 50.4 |
| n (Samp) | 375 | | 13 | 375 | | 13 | 375 | | 7 |
| n (Patient) | 375 | | 13 | 375 | | 13 | 375 | | 7 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 7.74 | | 18.0 | 7.74 | | 17.8 | 7.74 | | 14.5 |
| Average | 16.2 | | 30.0 | 16.2 | | 26.9 | 16.2 | | 25.0 |
| Stdev | 35.5 | | 26.9 | 35.5 | | 25.4 | 35.5 | | 30.2 |
| p(t-test) | | | 0.073 | | | 0.16 | | | 0.37 |
| Min | 0.00662 | | 5.53 | 0.00662 | | 5.53 | 0.00662 | | 5.53 |
| Max | 361 | | 122 | 361 | | 122 | 361 | | 122 |
| n(Samp) | 173 | | 23 | 173 | | 23 | 173 | | 14 |
| n (Patient) | 173 | | 23 | 173 | | 23 | 173 | | 14 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.80 | 0.67 | 0.79 | 0.78 | 0.65 | 0.78 | 0.77 | 0.69 | 0.73 |
| SE | 0.050 | 0.083 | 0.058 | 0.051 | 0.084 | 0.059 | 0.070 | 0.11 | 0.079 |
| p | 2.3E-9 | 0.038 | 6.7E-7 | 3.5E-8 | 0.079 | 3.0E-6 | 8.9E-5 | 0.086 | 0.0037 |
| nCohort 1 | 223 | 375 | 173 | 223 | 375 | 173 | 223 | 375 | 173 |
| nCohort 2 | 30 | 13 | 23 | 30 | 13 | 23 | 16 | 7 | 14 |
| Cutoff 1 | 13.1 | 12.2 | 13.6 | 12.7 | 11.3 | 12.7 | 11.4 | 12.6 | 12.4 |
| Sens 1 | 70% | 77% | 74% | 70% | 77% | 74% | 75% | 71% | 71% |
| Spec 1 | 78% | 64% | 73% | 77% | 59% | 72% | 72% | 65% | 72% |
| Cutoff 2 | 12.1 | 11.3 | 12.1 | 11.1 | 9.43 | 11.4 | 9.43 | 9.43 | 8.10 |
| Sens 2 | 80% | 85% | 83% | 80% | 85% | 83% | 81% | 86% | 86% |
| Spec 2 | 76% | 59% | 71% | 72% | 51% | 69% | 65% | 51% | 52% |
| Cutoff 3 | 8.13 | 5.52 | 8.10 | 8.13 | 5.52 | 8.10 | 6.18 | 5.52 | 6.18 |
| Sens 3 | 90% | 92% | 91% | 90% | 92% | 91% | 94% | 100% | 93% |
| Spec 3 | 59% | 31% | 52% | 59% | 31% | 52% | 48% | 31% | 40% |
| Cutoff 4 | 10.8 | 15.5 | 11.8 | 10.8 | 15.5 | 11.8 | 10.8 | 15.5 | 11.8 |
| Sens 4 | 83% | 54% | 83% | 80% | 54% | 78% | 75% | 57% | 71% |
| Spec 4 | 70% | 70% | 71% | 70% | 70% | 71% | 70% | 70% | 71% |
| Cutoff 5 | 14.8 | 20.2 | 17.9 | 14.8 | 20.2 | 17.9 | 14.8 | 20.2 | 17.9 |
| Sens 5 | 57% | 31% | 52% | 53% | 31% | 48% | 44% | 43% | 36% |
| Spec 5 | 80% | 80% | 80% | 80% | 80% | 80% | 80% | 80% | 80% |
| Cutoff 6 | 25.0 | 33.1 | 28.7 | 25.0 | 33.1 | 28.7 | 25.0 | 33.1 | 28.7 |
| Sens 6 | 30% | 15% | 35% | 27% | 15% | 30% | 25% | 29% | 21% |
| Spec 6 | 90% | 90% | 90% | 90% | 90% | 90% | 90% | 90% | 90% |
| OR Quart 2 | 2.0 | 1.0 | >3.2 | 2.0 | 1.0 | >3.2 | >2.0 | >1.0 | >3.1 |
| p Value | 0.57 | 1.0 | <0.32 | 0.57 | 1.0 | <0.32 | <0.57 | <1.0 | <0.33 |
| 95% CI of | 0.18 | 0.062 | >0.32 | 0.18 | 0.062 | >0.32 | >0.18 | >0.062 | >0.31 |
| OR Quart2 | 23 | 16 | na | 23 | 16 | na | na | na | na |
| OR Quart 3 | 10 | 6.3 | >8.2 | 10 | 7.5 | >9.6 | >4.2 | >3.1 | >6.7 |
| p Value | 0.029 | 0.090 | <0.054 | 0.029 | 0.062 | <0.037 | <0.20 | <0.33 | <0.083 |
| 95% CI of | 1.3 | 0.75 | >0.97 | 1.3 | 0.90 | >1.1 | >0.46 | >0.32 | >0.78 |
| OR Quart3 | 84 | 54 | na | 84 | 62 | na | na | na | na |
| OR Quart 4 | 24 | 5.2 | >18 | 24 | 4.1 | >16 | >12 | >3.1 | >5.5 |
| p Value | 0.0023 | 0.13 | <0.0068 | 0.0023 | 0.21 | <0.0093 | <0.021 | <0.34 | <0.13 |
| 95% CI of | 3.1 | 0.60 | >2.2 | 3.1 | 0.45 | >2.0 | >1.5 | >0.31 | >0.61 |
| OR Quart4 | 190 | 46 | na | 190 | 38 | na | na | na | na |

| | | | | | | | | | |
|---|---|---|---|---|---|---|---|---|---|
| **CA 15-3** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 434 | | 434 | 434 | | 434 | 434 | | 434 |
| Average | 327 | | 448 | 327 | | 426 | 327 | | 393 |
| Stdev | 253 | | 156 | 253 | | 184 | 253 | | 109 |
| p(t-test) | | | 0.050 | | | 0.11 | | | 0.36 |
| Min | 3.03 | | 63.1 | 3.03 | | 39.8 | 3.03 | | 63.1 |
| Max | 784 | | 784 | 784 | | 784 | 784 | | 434 |
| n (Samp) | 123 | | 18 | 123 | | 18 | 123 | | 13 |
| n (Patient) | 123 | | 18 | 123 | | 18 | 123 | | 13 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 434 | | 434 | 434 | | 434 | 434 | | 434 |
| Average | 379 | | 451 | 379 | | 415 | 379 | | 413 |
| Stdev | 241 | | 121 | 241 | | 174 | 241 | | 59.2 |
| p(t-test) | | | 0.33 | | | 0.63 | | | 0.69 |
| Min | 3.03 | | 266 | 3.03 | | 39.8 | 3.03 | | 266 |
| Max | 784 | | 784 | 784 | | 784 | 784 | | 434 |
| n (Samp) | 210 | | 11 | 210 | | 11 | 210 | | 8 |
| n (Patient) | 210 | | 11 | 210 | | 11 | 210 | | 8 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 434 | | 434 | 434 | | 434 | 434 | | 434 |
| Average | 338 | | 441 | 338 | | 441 | 338 | | 388 |
| Stdev | 243 | | 172 | 243 | | 172 | 243 | | 131 |
| p(t-test) | | | 0.19 | | | 0.19 | | | 0.57 |
| Min | 3.03 | | 63.1 | 3.03 | | 63.1 | 3.03 | | 63.1 |
| Max | 784 | | 784 | 784 | | 784 | 784 | | 434 |
| n (Samp) | 119 | | 10 | 119 | | 10 | 119 | | 8 |
| n (Patient) | 119 | | 10 | 119 | | 10 | 119 | | 8 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.65 | 0.58 | 0.64 | 0.63 | 0.54 | 0.64 | 0.60 | 0.54 | 0.58 |
| SE | 0.074 | 0.092 | 0.098 | 0.075 | 0.091 | 0.098 | 0.087 | 0.11 | 0.11 |
| p | 0.037 | 0.38 | 0.14 | 0.083 | 0.65 | 0.14 | 0.27 | 0.70 | 0.49 |
| nCohort 1 | 123 | 210 | 119 | 123 | 210 | 119 | 123 | 210 | 119 |
| nCohort 2 | 18 | 11 | 10 | 18 | 11 | 10 | 13 | 8 | 8 |
| Cutoff 1 | 413 | 413 | 363 | 413 | 413 | 363 | 413 | 413 | 363 |
| Sens 1 | 89% | 91% | 90% | 83% | 82% | 90% | 85% | 88% | 88% |
| Spec 1 | 42% | 32% | 37% | 42% | 32% | 37% | 42% | 32% | 37% |
| Cutoff 2 | 413 | 413 | 363 | 413 | 413 | 363 | 413 | 413 | 363 |
| Sens 2 | 89% | 91% | 90% | 83% | 82% | 90% | 85% | 88% | 88% |
| Spec 2 | 42% | 32% | 37% | 42% | 32% | 37% | 42% | 32% | 37% |
| Cutoff 3 | 248 | 413 | 363 | 61.3 | 253 | 363 | 248 | 253 | 61.3 |
| Sens 3 | 94% | 91% | 90% | 94% | 91% | 90% | 92% | 100% | 100% |
| Spec 3 | 40% | 32% | 37% | 28% | 29% | 37% | 40% | 29% | 25% |
| Cutoff 4 | 434 | 434 | 434 | 434 | 434 | 434 | 434 | 434 | 434 |
| Sens 4 | 17% | 9% | 20% | 17% | 9% | 20% | 0% | 0% | 0% |
| Spec 4 | 86% | 83% | 87% | 86% | 83% | 87% | 86% | 83% | 87% |
| Cutoff 5 | 434 | 434 | 434 | 434 | 434 | 434 | 434 | 434 | 434 |
| Sens 5 | 17% | 9% | 20% | 17% | 9% | 20% | 0% | 0% | 0% |
| Spec 5 | 86% | 83% | 87% | 86% | 83% | 87% | 86% | 83% | 87% |
| Cutoff 6 | 784 | 784 | 784 | 784 | 784 | 784 | 784 | 784 | 784 |
| Sens 6 | 0% | 0% | 0% | 0% | 0% | 0% | 0% | 0% | 0% |
| Spec 6 | 100% | 100% | 100% | 100% | 100% | 100% | 100% | 100% | 100% |
| OR Quart 2 | >12 | >8.0 | 7.2 | 12 | 6.6 | 7.2 | >10 | >5.4 | 0 |
| p Value | <0.022 | <0.055 | 0.077 | 0.023 | 0.085 | 0.077 | <0.032 | <0.13 | na |
| 95% CI of | >1.4 | >0.95 | 0.81 | 1.4 | 0.77 | 0.81 | >1.2 | >0.61 | na |
| OR Quart2 | na | na | 63 | 99 | 57 | 63 | na | na | na |
| OR Quart 3 | >7.2 | >3.2 | 1.0 | 5.7 | 3.1 | 1.0 | >4.5 | >3.2 | 8.4 |
| p Value | <0.074 | <0.32 | 1.0 | 0.12 | 0.33 | 1.0 | <0.19 | <0.32 | 0.054 |
| 95% CI of | >0.82 | >0.32 | 0.060 | 0.63 | 0.31 | 0.060 | >0.48 | >0.32 | 0.97 |
| OR Quart3 | na | na | 17 | 51 | 31 | 17 | na | na | 73 |
| OR Quart 4 | >3.2 | >1.0 | 2.0 | 3.1 | 0.98 | 2.0 | >1.0 | >0 | 0 |
| p Value | <0.33 | <1.0 | 0.58 | 0.34 | 0.99 | 0.58 | <0.98 | <na | na |
| 95% CI of | >0.31 | >0.061 | 0.17 | 0.31 | 0.060 | 0.17 | >0.062 | >na | na |
| OR Quart4 | na | na | 23 | 31 | 16 | 23 | na | na | na |

**Table 5: Comparison of marker levels in EDTA samples collected from Cohort 1 (patients that did not progress beyond RIFLE stage 0) and in EDTA samples collected from subjects at 0, 24 hours, and 48 hours prior to reaching stage R, I or F in Cohort 2.**

| **C-C motif chemokine 18** | | | | | | | | | |
|---|---|---|---|---|---|---|---|---|---|
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| +Median | 161 | | 203 | 161 | | 207 | 161 | | 190 |
| Average | 217 | | 302 | 217 | | 307 | 217 | | 310 |
| Stdev | 171 | | 316 | 171 | | 299 | 171 | | 318 |
| p(t-test) | | | 0.024 | | | 0.013 | | | 0.038 |
| Min | 37.8 | | 40.9 | 37.8 | | 41.6 | 37.8 | | 82.0 |
| Max | 1020 | | 2110 | 1020 | | 1650 | 1020 | | 1550 |
| n (Samp) | 121 | | 53 | 121 | | 53 | 121 | | 26 |
| n (Patient) | 87 | | 53 | 87 | | 53 | 87 | | 26 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 180 | | 221 | 180 | | 279 | 180 | | 220 |
| Average | 257 | | 264 | 257 | | 296 | 257 | | 262 |
| Stdev | 251 | | 185 | 251 | | 166 | 251 | | 160 |
| p(t-test) | | | 0.92 | | | 0.56 | | | 0.96 |
| Min | 34.0 | | 40.9 | 34.0 | | 41.6 | 34.0 | | 66.4 |
| Max | 2110 | | 848 | 2110 | | 679 | 2110 | | 548 |
| n (Samp) | 288 | | 16 | 288 | | 15 | 288 | | 10 |
| n (Patient) | 161 | | 16 | 161 | | 15 | 161 | | 10 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 167 | | 192 | 167 | | 201 | 167 | | 182 |
| Average | 229 | | 303 | 229 | | 298 | 229 | | 314 |
| Stdev | 174 | | 336 | 174 | | 297 | 174 | | 336 |
| p(t-test) | | | 0.063 | | | 0.053 | | | 0.070 |
| Min | 37.8 | | 46.6 | 37.8 | | 59.1 | 37.8 | | 66.1 |
| Max | 848 | | 2110 | 848 | | 1650 | 848 | | 1550 |
| n (Samp) | 125 | | 43 | 125 | | 54 | 125 | | 23 |
| n (Patient) | 80 | | 43 | 80 | | 54 | 80 | | 23 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.61 | 0.57 | 0.58 | 0.60 | 0.63 | 0.57 | 0.60 | 0.57 | 0.56 |
| SE | 0.048 | 0.076 | 0.052 | 0.048 | 0.079 | 0.047 | 0.064 | 0.096 | 0.067 |
| p | 0.024 | 0.33 | 0.11 | 0.039 | 0.10 | 0.14 | 0.10 | 0.48 | 0.33 |
| nCohort 1 | 121 | 288 | 125 | 121 | 288 | 125 | 121 | 288 | 125 |
| nCohort 2 | 53 | 16 | 43 | 53 | 15 | 54 | 26 | 10 | 23 |
| Cutoff 1 | 162 | 167 | 161 | 147 | 169 | 154 | 138 | 186 | 118 |
| Sens 1 | 72% | 75% | 72% | 72% | 73% | 70% | 73% | 70% | 74% |
| Spec 1 | 50% | 45% | 48% | 45% | 46% | 45% | 44% | 53% | 33% |
| Cutoff 2 | 132 | 162 | 132 | 107 | 162 | 107 | 118 | 152 | 105 |
| Sens 2 | 81% | 81% | 81% | 81% | 80% | 81% | 81% | 80% | 83% |
| Spec 2 | 40% | 43% | 38% | 28% | 43% | 25% | 36% | 40% | 24% |
| Cutoff 3 | 99.3 | 114 | 99.3 | 73.3 | 126 | 73.3 | 102 | 102 | 82.0 |
| Sens 3 | 91% | 94% | 91% | 91% | 93% | 91% | 92% | 90% | 91% |
| Spec 3 | 24% | 26% | 20% | 12% | 32% | 10% | 26% | 20% | 14% |
| Cutoff 4 | 228 | 283 | 274 | 228 | 283 | 274 | 228 | 283 | 274 |
| Sens 4 | 47% | 31% | 35% | 43% | 47% | 33% | 46% | 30% | 30% |
| Spec 4 | 70% | 70% | 70% | 70% | 70% | 70% | 70% | 70% | 70% |
| Cutoff 5 | 302 | 361 | 345 | 302 | 361 | 345 | 302 | 361 | 345 |
| Sens 5 | 32% | 19% | 30% | 32% | 33% | 26% | 23% | 20% | 26% |
| Spec 5 | 80% | 80% | 80% | 80% | 80% | 80% | 80% | 80% | 80% |
| Cutoff 6 | 464 | 516 | 484 | 464 | 516 | 484 | 464 | 516 | 484 |
| Sens 6 | 15% | 6% | 14% | 19% | 7% | 17% | 23% | 10% | 22% |
| Spec 6 | 90% | 90% | 90% | 90% | 90% | 90% | 90% | 90% | 90% |
| OR Quart 2 | 1.3 | 1.5 | 1.0 | 1.1 | 4.1 | 0.58 | 2.6 | 0.49 | 0.78 |
| p Value | 0.65 | 0.65 | 1.0 | 0.85 | 0.21 | 0.29 | 0.20 | 0.56 | 0.72 |
| 95% CI of | 0.46 | 0.25 | 0.34 | 0.41 | 0.45 | 0.21 | 0.61 | 0.043 | 0.19 |
| OR Quart2 | 3.4 | 9.4 | 3.0 | 2.9 | 38 | 1.6 | 11 | 5.5 | 3.2 |
| OR Quart 3 | 2.0 | 3.8 | 2.1 | 1.6 | 4.1 | 1.9 | 3.5 | 2.1 | 1.8 |
| p Value | 0.15 | 0.11 | 0.14 | 0.34 | 0.21 | 0.14 | 0.077 | 0.41 | 0.36 |
| 95% CI of | 0.77 | 0.75 | 0.78 | 0.61 | 0.45 | 0.80 | 0.87 | 0.37 | 0.52 |
| OR Quart3 | 5.3 | 19 | 5.8 | 4.1 | 38 | 4.7 | 14 | 12 | 6.0 |
| OR Quart 4 | 2.6 | 2.1 | 1.9 | 2.3 | 6.3 | 1.3 | 2.6 | 1.5 | 1.2 |
| p Value | 0.047 | 0.41 | 0.21 | 0.081 | 0.091 | 0.53 | 0.20 | 0.66 | 0.74 |
| 95% CI of | 1.0 | 0.37 | 0.69 | 0.90 | 0.74 | 0.54 | 0.61 | 0.24 | 0.34 |
| OR Quart4 | 6.8 | 12 | 5.2 | 5.8 | 54 | 3.3 | 11 | 9.2 | 4.5 |

| | | | | | | | | | |
|---|---|---|---|---|---|---|---|---|---|
| **C-C motif chemokine 24** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 225 | | 409 | 225 | | 258 | 225 | | 177 |
| Average | 418 | | 443 | 418 | | 391 | 418 | | 265 |
| Stdev | 512 | | 312 | 512 | | 316 | 512 | | 209 |
| p(t-test) | | | 0.77 | | | 0.75 | | | 0.22 |
| Min | 0.0260 | | 47.3 | 0.0260 | | 54.7 | 0.0260 | | 2.15 |
| Max | 2920 | | 1470 | 2920 | | 1380 | 2920 | | 579 |
| n (Samp) | 93 | | 45 | 93 | | 42 | 93 | | 18 |
| n (Patient) | 64 | | 45 | 64 | | 42 | 64 | | 18 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 225 | | 533 | 225 | | 459 | 225 | | 554 |
| Average | 357 | | 535 | 357 | | 456 | 357 | | 615 |
| Stdev | 403 | | 315 | 403 | | 282 | 403 | | 353 |
| p(t-test) | | | 0.10 | | | 0.31 | | | 0.060 |
| Min | 0.0260 | | 105 | 0.0260 | | 94.6 | 0.0260 | | 141 |
| Max | 2920 | | 1260 | 2920 | | 931 | 2920 | | 1100 |
| n (Samp) | 224 | | 14 | 224 | | 18 | 224 | | 9 |
| n (Patient) | 131 | | 14 | 131 | | 18 | 131 | | 9 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 270 | | 409 | 270 | | 259 | 270 | | 126 |
| Average | 453 | | 490 | 453 | | 392 | 453 | | 232 |
| Stdev | 493 | | 461 | 493 | | 344 | 493 | | 212 |
| p(t-test) | | | 0.69 | | | 0.47 | | | 0.091 |
| Min | 24.7 | | 47.3 | 24.7 | | 38.1 | 24.7 | | 2.15 |
| Max | 2920 | | 2430 | 2920 | | 1380 | 2920 | | 637 |
| n (Samp) | 102 | | 37 | 102 | | 41 | 102 | | 15 |
| n (Patient) | 63 | | 37 | 63 | | 41 | 63 | | 15 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.61 | 0.72 | 0.55 | 0.55 | 0.65 | 0.49 | 0.43 | 0.75 | 0.34 |
| SE | 0.052 | 0.079 | 0.056 | 0.054 | 0.073 | 0.054 | 0.076 | 0.096 | 0.081 |
| p | 0.042 | 0.0051 | 0.39 | 0.36 | 0.041 | 0.83 | 0.39 | 0.0091 | 0.042 |
| nCohort 1 | 93 | 224 | 102 | 93 | 224 | 102 | 93 | 224 | 102 |
| nCohort 2 | 45 | 14 | 37 | 42 | 18 | 41 | 18 | 9 | 15 |
| Cutoff 1 | 221 | 350 | 188 | 171 | 207 | 155 | 96.1 | 400 | 85.2 |
| Sens 1 | 71% | 71% | 70% | 71% | 72% | 71% | 72% | 78% | 73% |
| Spec 1 | 49% | 69% | 30% | 37% | 47% | 25% | 24% | 73% | 13% |
| Cutoff 2 | 171 | 221 | 154 | 108 | 196 | 92.4 | 85.2 | 212 | 70.8 |
| Sens 2 | 80% | 86% | 81% | 81% | 83% | 83% | 83% | 89% | 80% |
| Spec 2 | 37% | 50% | 25% | 25% | 46% | 15% | 18% | 47% | 11% |
| Cutoff 3 | 122 | 207 | 92.4 | 90.5 | 108 | 80.9 | 2.15 | 140 | 2.15 |
| Sens 3 | 91% | 93% | 92% | 90% | 94% | 90% | 94% | 100% | 93% |
| Spec 3 | 26% | 47% | 15% | 19% | 27% | 12% | 1% | 31% | 0% |
| Cutoff 4 | 393 | 365 | 541 | 393 | 365 | 541 | 393 | 365 | 541 |
| Sens 4 | 51% | 64% | 38% | 36% | 56% | 24% | 33% | 78% | 13% |
| Spec 4 | 71% | 70% | 71% | 71% | 70% | 71% | 71% | 70% | 71% |
| Cutoff 5 | 664 | 548 | 705 | 664 | 548 | 705 | 664 | 548 | 705 |
| Sens 5 | 18% | 50% | 22% | 21% | 39% | 17% | 0% | 56% | 0% |
| Spec 5 | 81% | 80% | 80% | 81% | 80% | 80% | 81% | 80% | 80% |
| Cutoff 6 | 940 | 855 | 993 | 940 | 855 | 993 | 940 | 855 | 993 |
| Sens 6 | 7% | 14% | 8% | 7% | 6% | 10% | 0% | 33% | 0% |
| Spec 6 | 90% | 90% | 90% | 90% | 90% | 90% | 90% | 90% | 90% |
| OR Quart 2 | 2.1 | >3.1 | 0.96 | 0.96 | 5.3 | 1.3 | 1.8 | >2.1 | 6.0 |
| p Value | 0.19 | <0.33 | 0.95 | 0.94 | 0.13 | 0.60 | 0.45 | <0.56 | 0.11 |
| 95% CI of | 0.69 | >0.31 | 0.31 | 0.33 | 0.60 | 0.47 | 0.39 | >0.18 | 0.66 |
| OR Quart2 | 6.7 | na | 2.9 | 2.8 | 47 | 3.7 | 8.4 | na | 55 |
| OR Quart 3 | 3.7 | >3.2 | 1.5 | 1.7 | 3.1 | 1.3 | 1.8 | >1.0 | 0 |
| p Value | 0.021 | <0.33 | 0.46 | 0.34 | 0.33 | 0.60 | 0.45 | <0.99 | na |
| 95% CI of | 1.2 | >0.32 | 0.51 | 0.59 | 0.31 | 0.47 | 0.39 | >0.062 | na |
| OR Quart3 | 11 | na | 4.3 | 4.6 | 31 | 3.7 | 8.4 | na | na |
| OR Quart 4 | 2.8 | >9.1 | 1.3 | 1.3 | 10 | 1.2 | 1.9 | >6.6 | 13 |
| p Value | 0.075 | <0.041 | 0.63 | 0.65 | 0.030 | 0.73 | 0.42 | <0.086 | 0.019 |
| 95% CI of | 0.90 | >1.1 | 0.44 | 0.45 | 1.3 | 0.42 | 0.41 | >0.77 | 1.5 |
| OR Quart4 | 8.4 | na | 3.8 | 3.6 | 83 | 3.4 | 8.9 | na | 110 |

| | | | | | | | | | |
|---|---|---|---|---|---|---|---|---|---|
| **C-C motif chemokine 8** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 12.8 | | 12.4 | 12.8 | | 13.6 | 12.8 | | 16.9 |
| Average | 17.4 | | 13.3 | 17.4 | | 18.7 | 17.4 | | 20.9 |
| Stdev | 23.1 | | 9.56 | 23.1 | | 26.9 | 23.1 | | 17.7 |
| p(t-test) | | | 0.24 | | | 0.78 | | | 0.55 |
| Min | 0.162 | | 0.162 | 0.162 | | 0.170 | 0.162 | | 1.32 |
| Max | 148 | | 50.3 | 148 | | 180 | 148 | | 72.1 |
| n (Samp) | 93 | | 45 | 93 | | 42 | 93 | | 18 |
| n (Patient) | 64 | | 45 | 64 | | 42 | 64 | | 18 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 13.2 | | 11.3 | 13.2 | | 12.7 | 13.2 | | 18.1 |
| Average | 16.7 | | 12.7 | 16.7 | | 22.4 | 16.7 | | 23.8 |
| Stdev | 20.5 | | 10.7 | 20.5 | | 37.7 | 20.5 | | 15.3 |
| p(t-test) | | | 0.47 | | | 0.30 | | | 0.30 |
| Min | 0.162 | | 0.162 | 0.162 | | 0.170 | 0.162 | | 11.5 |
| Max | 180 | | 38.1 | 180 | | 169 | 180 | | 59.4 |
| n (Samp) | 224 | | 14 | 224 | | 18 | 224 | | 9 |
| n (Patient) | 131 | | 14 | 131 | | 18 | 131 | | 9 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 12.3 | | 12.4 | 12.3 | | 13.8 | 12.3 | | 16.3 |
| Average | 17.6 | | 12.7 | 17.6 | | 24.9 | 17.6 | | 16.9 |
| Stdev | 22.8 | | 9.01 | 22.8 | | 40.3 | 22.8 | | 16.3 |
| p(t-test) | | | 0.20 | | | 0.17 | | | 0.90 |
| Min | 0.162 | | 0.170 | 0.162 | | 0.162 | 0.162 | | 0.162 |
| Max | 148 | | 50.3 | 148 | | 180 | 148 | | 72.1 |
| n (Samp) | 102 | | 37 | 102 | | 41 | 102 | | 15 |
| n (Patient) | 63 | | 37 | 63 | | 41 | 63 | | 15 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.46 | 0.43 | 0.47 | 0.54 | 0.50 | 0.55 | 0.61 | 0.71 | 0.55 |
| SE | 0.053 | 0.082 | 0.056 | 0.054 | 0.071 | 0.054 | 0.076 | 0.098 | 0.082 |
| p | 0.51 | 0.41 | 0.57 | 0.52 | 0.98 | 0.37 | 0.14 | 0.029 | 0.55 |
| nCohort 1 | 93 | 224 | 102 | 93 | 224 | 102 | 93 | 224 | 102 |
| nCohort 2 | 45 | 14 | 37 | 42 | 18 | 41 | 18 | 9 | 15 |
| Cutoff 1 | 7.62 | 6.24 | 8.03 | 10.1 | 10.6 | 9.32 | 12.9 | 14.4 | 11.3 |
| Sens 1 | 71% | 71% | 70% | 71% | 72% | 71% | 72% | 78% | 73% |
| Spec 1 | 25% | 19% | 28% | 37% | 36% | 30% | 53% | 57% | 44% |
| Cutoff 2 | 6.24 | 1.20 | 6.24 | 7.60 | 6.24 | 6.24 | 11.3 | 11.5 | 9.54 |
| Sens 2 | 82% | 86% | 81% | 81% | 83% | 83% | 83% | 89% | 80% |
| Spec 2 | 19% | 7% | 20% | 25% | 19% | 20% | 39% | 38% | 33% |
| Cutoff 3 | 1.16 | 1.16 | 1.16 | 4.81 | 1.67 | 4.81 | 9.47 | 11.3 | 1.20 |
| Sens 3 | 93% | 93% | 95% | 90% | 94% | 90% | 94% | 100% | 93% |
| Spec 3 | 6% | 5% | 8% | 18% | 9% | 19% | 29% | 38% | 10% |
| Cutoff 4 | 17.2 | 16.9 | 17.2 | 17.2 | 16.9 | 17.2 | 17.2 | 16.9 | 17.2 |
| Sens 4 | 18% | 29% | 19% | 36% | 33% | 34% | 39% | 67% | 33% |
| Spec 4 | 71% | 70% | 71% | 71% | 70% | 71% | 71% | 70% | 71% |
| Cutoff 5 | 20.1 | 19.7 | 22.1 | 20.1 | 19.7 | 22.1 | 20.1 | 19.7 | 22.1 |
| Sens 5 | 11% | 14% | 8% | 24% | 28% | 22% | 17% | 44% | 7% |
| Spec 5 | 81% | 80% | 80% | 81% | 80% | 80% | 81% | 80% | 80% |
| Cutoff 6 | 28.4 | 26.8 | 31.0 | 28.4 | 26.8 | 31.0 | 28.4 | 26.8 | 31.0 |
| Sens 6 | 4% | 14% | 3% | 10% | 17% | 12% | 17% | 22% | 7% |
| Spec 6 | 90% | 90% | 90% | 90% | 90% | 90% | 90% | 90% | 90% |
| OR Quart 2 | 2.4 | 1.0 | 2.9 | 0.96 | 0.98 | 0.60 | 4.3 | >2.1 | 1.6 |
| p Value | 0.11 | 0.98 | 0.065 | 0.94 | 0.98 | 0.37 | 0.20 | <0.56 | 0.64 |
| 95% CI of | 0.83 | 0.20 | 0.94 | 0.34 | 0.27 | 0.20 | 0.45 | >0.18 | 0.24 |
| OR Quart2 | 6.7 | 5.3 | 8.7 | 2.7 | 3.6 | 1.8 | 42 | na | 10 |
| OR Quart 3 | 1.5 | 1.0 | 1.7 | 0.96 | 0.58 | 1.4 | 14 | >3.2 | 6.1 |
| p Value | 0.42 | 1.0 | 0.38 | 0.94 | 0.47 | 0.50 | 0.015 | <0.32 | 0.031 |
| 95% CI of | 0.53 | 0.19 | 0.52 | 0.34 | 0.13 | 0.52 | 1.7 | >0.32 | 1.2 |
| OR Quart3 | 4.5 | 5.2 | 5.3 | 2.7 | 2.5 | 3.8 | 120 | na | 31 |
| OR Quart 4 | t 1.8 | 1.8 | 1.7 | 1.3 | 0.98 | 1.1 | 3.1 | >4.2 | 0.47 |
| p Value | 0.26 | 0.45 | 0.35 | 0.66 | 0.98 | 0.85 | 0.34 | <0.20 | 0.54 |
| 95% CI of | 0.64 | 0.40 | 0.54 | 0.45 | 0.27 | 0.40 | 0.30 | >0.46 | 0.040 |
| OR Quart4 | 5.3 | 7.7 | 5.6 | 3.5 | 3.6 | 3.1 | 32 | na | 5.4 |

| | | | | | | | | | |
|---|---|---|---|---|---|---|---|---|---|
| **Cathepsin D** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 234000 | | 264000 | 234000 | | 249000 | 234000 | | 239000 |
| Average | 255000 | | 257000 | 255000 | | 278000 | 255000 | | 272000 |
| Stdev | 136000 | | 138000 | 136000 | | 149000 | 136000 | | 179000 |
| p(t-test) | | | 0.93 | | | 0.32 | | | 0.59 |
| Min | 34100 | | 55100 | 34100 | | 55100 | 34100 | | 54000 |
| Max | 1020000 | | 653000 | 1020000 | | 648000 | 1020000 | | 853000 |
| n (Samp) | 120 | | 53 | 120 | | 53 | 120 | | 26 |
| n (Patient) | 86 | | 53 | 86 | | 53 | 86 | | 26 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 231000 | | 210000 | 231000 | | 325000 | 231000 | | 175000 |
| Average | 266000 | | 222000 | 266000 | | 302000 | 266000 | | 221000 |
| Stdev | 175000 | | 110000 | 175000 | | 145000 | 175000 | | 99200 |
| p(t-test) | | | 0.32 | | | 0.44 | | | 0.41 |
| Min | 34100 | | 55100 | 34100 | | 78800 | 34100 | | 129000 |
| Max | 1870000 | | 413000 | 1870000 | | 507000 | 1870000 | | 437000 |
| n (Samp) | 287 | | 16 | 287 | | 15 | 287 | | 10 |
| n (Patient) | 160 | | 16 | 160 | | 15 | 160 | | 10 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 239000 | | 280000 | 239000 | | 252000 | 239000 | | 248000 |
| Average | 265000 | | 296000 | 265000 | | 295000 | 265000 | | 291000 |
| Stdev | 136000 | | 216000 | 136000 | | 164000 | 136000 | | 186000 |
| p(t-test) | | | 0.27 | | | 0.21 | | | 0.42 |
| Min | 69400 | | 62900 | 69400 | | 55100 | 69400 | | 54000 |
| Max | 1020000 | | 1350000 | 1020000 | | 655000 | 1020000 | | 853000 |
| n (Samp) | 124 | | 43 | 124 | | 54 | 124 | | 23 |
| n (Patient) | 79 | | 43 | 79 | | 54 | 79 | | 23 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.50 | 0.44 | 0.52 | 0.53 | 0.60 | 0.54 | 0.50 | 0.44 | 0.52 |
| SE | 0.048 | 0.076 | 0.052 | 0.048 | 0.079 | 0.047 | 0.063 | 0.096 | 0.066 |
| p | 0.94 | 0.41 | 0.64 | 0.47 | 0.23 | 0.42 | 0.98 | 0.53 | 0.77 |
| nCohort 1 | 120 | 287 | 124 | 120 | 287 | 124 | 120 | 287 | 124 |
| nCohort 2 | 53 | 16 | 43 | 53 | 15 | 54 | 26 | 10 | 23 |
| Cutoff 1 | 143000 | 143000 | 167000 | 167000 | 185000 | 169000 | 161000 | 157000 | 169000 |
| Sens 1 | 72% | 75% | 72% | 72% | 73% | 70% | 73% | 70% | 74% |
| Spec 1 | 18% | 23% | 28% | 29% | 38% | 28% | 25% | 28% | 29% |
| Cutoff 2 | 125000 | 126000 | 126000 | 136000 | 167000 | 136000 | 154000 | 154000 | 161000 |
| Sens 2 | 81% | 81% | 81% | 81% | 80% | 81% | 81% | 80% | 83% |
| Spec 2 | 11% | 15% | 7% | 14% | 33% | 10% | 21% | 27% | 23% |
| Cutoff 3 | 93800 | 87200 | 105000 | 116000 | 130000 | 114000 | 107000 | 130000 | 107000 |
| Sens 3 | 91% | 94% | 91% | 91% | 93% | 91% | 92% | 90% | 91% |
| Spec 3 | 6% | 4% | 6% | 8% | 17% | 6% | 7% | 17% | 6% |
| Cutoff 4 | 300000 | 307000 | 307000 | 300000 | 307000 | 307000 | 300000 | 307000 | 307000 |
| Sens 4 | 36% | 25% | 40% | 42% | 53% | 41% | 27% | 20% | 30% |
| Spec 4 | 70% | 70% | 70% | 70% | 70% | 70% | 70% | 70% | 70% |
| Cutoff 5 | 330000 | 362000 | 342000 | 330000 | 362000 | 342000 | 330000 | 362000 | 342000 |
| Sens 5 | 32% | 12% | 26% | 36% | 33% | 39% | 19% | 10% | 26% |
| Spec 5 | 80% | 80% | 81% | 80% | 80% | 81% | 80% | 80% | 81% |
| Cutoff 6 | 407000 | 462000 | 426000 | 407000 | 462000 | 426000 | 407000 | 462000 | 426000 |
| Sens 6 | 15% | 0% | 14% | 25% | 20% | 22% | 15% | 0% | 17% |
| Spec 6 | 90% | 90% | 90% | 90% | 90% | 90% | 90% | 90% | 90% |
| OR Quart 2 | 0.35 | 0.74 | 0.40 | 0.57 | 0.99 | 0.42 | 1.5 | 3.1 | 0.97 |
| p Value | 0.036 | 0.70 | 0.10 | 0.24 | 0.99 | 0.083 | 0.52 | 0.33 | 0.96 |
| 95% CI of | 0.13 | 0.16 | 0.13 | 0.22 | 0.19 | 0.16 | 0.46 | 0.32 | 0.26 |
| OR Quart2 | 0.93 | 3.4 | 1.2 | 1.5 | 5.0 | 1.1 | 4.8 | 31 | 3.7 |
| OR Quart 3 | 0.46 | 1.0 | 0.86 | 0.49 | 1.0 | 0.57 | 0.81 | 4.2 | 1.4 |
| p Value | 0.11 | 1.0 | 0.75 | 0.15 | 1.0 | 0.24 | 0.74 | 0.20 | 0.56 |
| 95% CI of | 0.18 | 0.24 | 0.33 | 0.19 | 0.20 | 0.22 | 0.22 | 0.46 | 0.41 |
| OR Quart3 | 1.2 | 4.2 | 2.2 | 1.3 | 5.1 | 1.5 | 2.9 | 39 | 5.1 |
| OR Quart 4 | 1.1 | 1.3 | 1.2 | 1.4 | 2.1 | 1.7 | 1.2 | 2.1 | 1.2 |
| p Value | 0.90 | 0.72 | 0.69 | 0.43 | 0.32 | 0.23 | 0.72 | 0.56 | 0.78 |
| 95% CI of | 0.45 | 0.33 | 0.48 | 0.60 | 0.50 | 0.72 | 0.37 | 0.18 | 0.33 |
| OR Quart4 | 2.5 | 5.0 | 3.1 | 3.4 | 8.5 | 4.0 | 4.2 | 23 | 4.3 |

| | | | | | | | | | |
|---|---|---|---|---|---|---|---|---|---|
| **C-X-C motif chemokine 13** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 45.8 | | 52.8 | 45.8 | | 69.6 | 45.8 | | 80.6 |
| Average | 138 | | 116 | 138 | | 118 | 138 | | 224 |
| Stdev | 297 | | 292 | 297 | | 216 | 297 | | 275 |
| p(t-test) | | | 0.68 | | | 0.69 | | | 0.26 |
| Min | 9.90 | | 17.0 | 9.90 | | 11.3 | 9.90 | | 15.9 |
| Max | 1790 | | 2000 | 1790 | | 1340 | 1790 | | 918 |
| n (Samp) | 93 | | 45 | 93 | | 42 | 93 | | 18 |
| n (Patient) | 64 | | 45 | 64 | | 42 | 64 | | 18 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 48.7 | | 62.3 | 48.7 | | 69.5 | 48.7 | | 43.9 |
| Average | 125 | | 207 | 125 | | 187 | 125 | | 128 |
| Stdev | 242 | | 517 | 242 | | 455 | 242 | | 238 |
| p(t-test) | | | 0.26 | | | 0.33 | | | 0.96 |
| Min | 9.90 | | 25.4 | 9.90 | | 18.6 | 9.90 | | 17.3 |
| Max | 1790 | | 2000 | 1790 | | 2000 | 1790 | | 758 |
| n (Samp) | 224 | | 14 | 224 | | 18 | 224 | | 9 |
| n (Patient) | 131 | | 14 | 131 | | 18 | 131 | | 9 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 47.3 | | 56.6 | 47.3 | | 69.3 | 47.3 | | 81.6 |
| Average | 123 | | 76.0 | 123 | | 170 | 123 | | 229 |
| Stdev | 242 | | 57.1 | 242 | | 285 | 242 | | 257 |
| p(t-test) | | | 0.25 | | | 0.32 | | | 0.12 |
| Min | 9.90 | | 17.0 | 9.90 | | 11.3 | 9.90 | | 15.9 |
| Max | 1790 | | 287 | 1790 | | 1340 | 1790 | | 918 |
| n (Samp) | 102 | | 37 | 102 | | 41 | 102 | | 15 |
| n (Patient) | 63 | | 37 | 63 | | 41 | 63 | | 15 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.54 | 0.57 | 0.52 | 0.55 | 0.58 | 0.58 | 0.63 | 0.50 | 0.64 |
| SE | 0.053 | 0.082 | 0.056 | 0.054 | 0.073 | 0.054 | 0.076 | 0.099 | 0.081 |
| p | 0.47 | 0.42 | 0.66 | 0.38 | 0.25 | 0.15 | 0.096 | 0.97 | 0.082 |
| nCohort 1 | 93 | 224 | 102 | 93 | 224 | 102 | 93 | 224 | 102 |
| nCohort 2 | 45 | 14 | 37 | 42 | 18 | 41 | 18 | 9 | 15 |
| Cutoff 1 | 37.0 | 46.6 | 37.3 | 33.7 | 52.7 | 39.4 | 41.8 | 34.7 | 47.3 |
| Sens 1 | 71% | 71% | 70% | 71% | 72% | 71% | 72% | 78% | 73% |
| Spec 1 | 44% | 48% | 41% | 42% | 53% | 41% | 46% | 37% | 52% |
| Cutoff 2 | 34.0 | 28.0 | 34.5 | 28.3 | 32.8 | 33.7 | 29.5 | 34.0 | 29.5 |
| Sens 2 | 80% | 86% | 81% | 81% | 83% | 80% | 83% | 89% | 80% |
| Spec 2 | 42% | 27% | 35% | 35% | 35% | 34% | 41% | 36% | 33% |
| Cutoff 3 | 23.0 | 25.8 | 18.7 | 20.5 | 24.4 | 22.7 | 16.8 | 17.2 | 16.8 |
| Sens 3 | 91% | 93% | 92% | 90% | 94% | 90% | 94% | 100% | 93% |
| Spec 3 | 26% | 24% | 12% | 17% | 21% | 20% | 12% | 12% | 11% |
| Cutoff 4 | 106 | 98.9 | 102 | 106 | 98.9 | 102 | 106 | 98.9 | 102 |
| Sens 4 | 24% | 29% | 24% | 33% | 33% | 39% | 39% | 22% | 47% |
| Spec 4 | 71% | 70% | 71% | 71% | 70% | 71% | 71% | 70% | 71% |
| Cutoff 5 | 170 | 136 | 140 | 170 | 136 | 140 | 170 | 136 | 140 |
| Sens 5 | 9% | 14% | 11% | 7% | 22% | 22% | 39% | 11% | 47% |
| Spec 5 | 81% | 80% | 80% | 81% | 80% | 80% | 81% | 80% | 80% |
| Cutoff 6 | 251 | 254 | 232 | 251 | 254 | 232 | 251 | 254 | 232 |
| Sens 6 | 4% | 7% | 3% | 7% | 6% | 15% | 39% | 11% | 47% |
| Spec 6 | 90% | 90% | 90% | 90% | 90% | 90% | 90% | 90% | 90% |
| OR Quart 2 | 2.3 | 1.5 | 2.7 | 1.5 | 1.5 | 1.3 | 0.96 | 2.1 | 0.64 |
| p Value | 0.13 | 0.66 | 0.11 | 0.44 | 0.66 | 0.61 | 0.96 | 0.56 | 0.64 |
| 95% CI of | 0.78 | 0.24 | 0.81 | 0.51 | 0.24 | 0.43 | 0.18 | 0.18 | 0.099 |
| OR Quart2 | 6.7 | 9.3 | 8.7 | 4.7 | 9.3 | 4.1 | 5.2 | 23 | 4.2 |
| OR Quart 3 | 3.4 | 3.2 | 3.9 | 2.9 | 4.5 | 2.0 | 1.7 | 5.5 | 1.0 |
| p Value | 0.024 | 0.16 | 0.023 | 0.050 | 0.066 | 0.21 | 0.48 | 0.13 | 1.0 |
| 95% CI of | 1.2 | 0.62 | 1.2 | 1.0 | 0.91 | 0.68 | 0.37 | 0.62 | 0.18 |
| OR Quart3 | 10.0 | 17 | 12 | 8.6 | 22 | 5.9 | 8.1 | 48 | 5.4 |
| OR Quart 4 | 1.3 | 1.5 | 1.4 | 1.5 | 2.6 | 2.3 | 2.7 | 1.0 | 2.6 |
| p Value | 0.61 | 0.66 | 0.56 | 0.44 | 0.27 | 0.14 | 0.19 | 0.99 | 0.19 |
| 95% CI of | 0.43 | 0.24 | 0.41 | 0.51 | 0.48 | 0.77 | 0.61 | 0.062 | 0.61 |
| OR Quart4 | 4.1 | 9.3 | 5.1 | 4.7 | 14 | 6.6 | 12 | 17 | 11 |

| | | | | | | | | | |
|---|---|---|---|---|---|---|---|---|---|
| **Insulin-like growth factor-binding protein 3** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 3010 | | 3320 | 3010 | | 3210 | 3010 | | 3990 |
| Average | 3360 | | 3500 | 3360 | | 3620 | 3360 | | 4050 |
| Stdev | 1520 | | 1470 | 1520 | | 1710 | 1520 | | 1940 |
| p(t-test) | | | 0.58 | | | 0.33 | | | 0.050 |
| Min | 823 | | 651 | 823 | | 730 | 823 | | 804 |
| Max | 8260 | | 7620 | 8260 | | 8600 | 8260 | | 7140 |
| n (Samp) | 121 | | 53 | 121 | | 53 | 121 | | 26 |
| n (Patient) | 87 | | 53 | 87 | | 53 | 87 | | 26 |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 3050 | | 3580 | 3050 | | 4790 | 3050 | | 5000 |
| Average | 3390 | | 4110 | 3390 | | 4440 | 3390 | | 4510 |
| Stdev | 1500 | | 1990 | 1500 | | 1990 | 1500 | | 1960 |
| p(t-test) | | | 0.065 | | | 0.0096 | | | 0.022 |
| Min | 651 | | 1820 | 651 | | 1210 | 651 | | 1660 |
| Max | 8260 | | 7620 | 8260 | | 8600 | 8260 | | 7140 |
| n (Samp) | 288 | | 16 | 288 | | 15 | 288 | | 10 |
| n (Patient) | 161 | | 16 | 161 | | 15 | 161 | | 10 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 2960 | | 3320 | 2960 | | 3220 | 2960 | | 3300 |
| Average | 3290 | | 3420 | 3290 | | 3520 | 3290 | | 3630 |
| Stdev | 1510 | | 1370 | 1510 | | 1580 | 1510 | | 1780 |
| p(t-test) | | | 0.61 | | | 0.36 | | | 0.34 |
| Min | 823 | | 651 | 823 | | 730 | 823 | | 804 |
| Max | 8260 | | 7300 | 8260 | | 7520 | 8260 | | 7100 |
| n (Samp) | 125 | | 43 | 125 | | 54 | 125 | | 23 |
| n (Patient) | 80 | | 43 | 80 | | 54 | 80 | | 23 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.54 | 0.59 | 0.55 | 0.54 | 0.66 | 0.55 | 0.61 | 0.67 | 0.56 |
| SE | 0.048 | 0.077 | 0.052 | 0.048 | 0.078 | 0.047 | 0.064 | 0.095 | 0.067 |
| p | 0.42 | 0.23 | 0.32 | 0.41 | 0.039 | 0.33 | 0.090 | 0.068 | 0.41 |
| nCohort 1 | 121 | 288 | 125 | 121 | 288 | 125 | 121 | 288 | 125 |
| nCohort 2 | 53 | 16 | 43 | 53 | 15 | 54 | 26 | 10 | 23 |
| Cutoff 1 | 2620 | 2560 | 2670 | 2370 | 3020 | 2430 | 2700 | 3050 | 2430 |
| Sens 1 | 72% | 75% | 72% | 72% | 73% | 70% | 73% | 70% | 74% |
| Spec 1 | 40% | 36% | 44% | 30% | 50% | 34% | 44% | 50% | 34% |
| Cutoff 2 | 2160 | 2070 | 2160 | 2290 | 2800 | 2290 | 2040 | 2430 | 2000 |
| Sens 2 | 81% | 81% | 81% | 81% | 80% | 81% | 81% | 80% | 83% |
| Spec 2 | 26% | 19% | 28% | 27% | 43% | 30% | 23% | 31% | 22% |
| Cutoff 3 | 1960 | 1940 | 1740 | 1780 | 2130 | 1740 | 1490 | 2430 | 1490 |
| Sens 3 | 91% | 94% | 91% | 91% | 93% | 91% | 92% | 90% | 91% |
| Spec 3 | 20% | 15% | 11% | 12% | 20% | 11% | 7% | 31% | 7% |
| Cutoff 4 | 4130 | 4130 | 4040 | 4130 | 4130 | 4040 | 4130 | 4130 | 4040 |
| Sens 4 | 28% | 44% | 33% | 34% | 53% | 37% | 50% | 60% | 39% |
| Spec 4 | 70% | 70% | 70% | 70% | 70% | 70% | 70% | 70% | 70% |
| Cutoff 5 | 4680 | 4650 | 4560 | 4680 | 4650 | 4560 | 4680 | 4650 | 4560 |
| Sens 5 | 17% | 38% | 16% | 26% | 53% | 26% | 38% | 60% | 30% |
| Spec 5 | 80% | 80% | 80% | 80% | 80% | 80% | 80% | 80% | 80% |
| Cutoff 6 | 5410 | 5490 | 5410 | 5410 | 5490 | 5410 | 5410 | 5490 | 5410 |
| Sens 6 | 8% | 31% | 5% | 19% | 40% | 15% | 31% | 40% | 22% |
| Spec 6 | 90% | 90% | 90% | 90% | 90% | 90% | 90% | 90% | 90% |
| OR Quart 2 | 0.86 | 0.74 | 1.6 | 1.5 | 0.65 | 2.1 | 0.44 | 3.0 | 0.63 |
| p Value | 0.76 | 0.70 | 0.42 | 0.37 | 0.64 | 0.12 | 0.28 | 0.34 | 0.50 |
| 95% CI of | 0.33 | 0.16 | 0.53 | 0.60 | 0.11 | 0.83 | 0.10 | 0.31 | 0.16 |
| OR Quart2 | 2.2 | 3.4 | 4.6 | 4.0 | 4.0 | 5.6 | 1.9 | 30 | 2.4 |
| OR Quart 3 | 1.9 | 0.74 | 2.8 | 1.4 | 0.65 | 1.6 | 0.97 | 0 | 0.81 |
| p Value | 0.18 | 0.70 | 0.051 | 0.47 | 0.64 | 0.36 | 0.96 | na | 0.74 |
| 95% CI of | 0.76 | 0.16 | 0.99 | 0.55 | 0.11 | 0.60 | 0.28 | na | 0.22 |
| OR Quart3 | 4.6 | 3.4 | 7.8 | 3.7 | 4.0 | 4.2 | 3.3 | na | 2.9 |
| OR Quart 4 | 0.97 | 1.5 | 1.8 | 1.9 | 2.8 | 2.1 | 2.1 | 6.3 | 1.4 |
| p Value | 0.95 | 0.52 | 0.29 | 0.18 | 0.14 | 0.12 | 0.19 | 0.091 | 0.55 |
| 95% CI of | 0.38 | 0.42 | 0.61 | 0.74 | 0.72 | 0.83 | 0.69 | 0.75 | 0.44 |
| OR Quart4 | 2.5 | 5.7 | 5.1 | 4.8 | 11 | 5.6 | 6.5 | 54 | 4.6 |

| | | | | | | | | | |
|---|---|---|---|---|---|---|---|---|---|
| **Immunoglogulin G1** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 8750000 | | 8400000 | 8750000 | | 9530000 | 8750000 | | 7580000 |
| Average | 1.06E7 | | 9010000 | 1.06E7 | | 1.24E7 | 1.06E7 | | 1.30E7 |
| Stdev | 6040000 | | 4090000 | 6040000 | | 8340000 | 6040000 | | 9920000 |
| p(t-test) | | | 0.14 | | | 0.19 | | | 0.28 |
| Min | 3800000 | | 2060000 | 3800000 | | 3530000 | 3800000 | | 4390000 |
| Max | 3.48E7 | | 2.33E7 | 3.48E7 | | 4.18E7 | 3.48E7 | | 3.57E7 |
| n (Samp) | 94 | | 35 | 94 | | 37 | 94 | | 10 |
| n (Patient) | 67 | | 35 | 67 | | 37 | 67 | | 10 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 8820000 | | 7580000 | 8820000 | | 1.02E7 | nd | | nd |
| Average | 1.02E7 | | 9650000 | 1.02E7 | | 1.51E7 | nd | | nd |
| Stdev | 5310000 | | 6060000 | 5310000 | | 1.34E7 | nd | | nd |
| p(t-test) | | | 0.73 | | | 0.021 | nd | | nd |
| Min | 2060000 | | 3270000 | 2060000 | | 4390000 | nd | | nd |
| Max | 3.48E7 | | 2.33E7 | 3.48E7 | | 4.18E7 | nd | | nd |
| n (Samp) | 205 | | 10 | 205 | | 8 | nd | | nd |
| n (Patient) | 126 | | 10 | 126 | | 8 | nd | | nd |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 7990000 | | 8420000 | 7990000 | | 9200000 | 7990000 | | 7690000 |
| Average | 9560000 | | 8580000 | 9560000 | | 1.21E7 | 9560000 | | 1.34E7 |
| Stdev | 5620000 | | 3160000 | 5620000 | | 7850000 | 5620000 | | 9630000 |
| p(t-test) | | | 0.38 | | | 0.039 | | | 0.043 |
| Min | 3270000 | | 2060000 | 3270000 | | 3530000 | 3270000 | | 4390000 |
| Max | 3.48E7 | | 1.63E7 | 3.48E7 | | 4.18E7 | 3.48E7 | | 3.57E7 |
| n (Samp) | 101 | | 28 | 101 | | 36 | 101 | | 12 |
| n (Patient) | 65 | | 28 | 65 | | 36 | 65 | | 12 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.45 | 0.42 | 0.51 | 0.55 | 0.56 | 0.61 | 0.54 | nd | 0.62 |
| SE | 0.058 | 0.097 | 0.062 | 0.057 | 0.11 | 0.056 | 0.098 | nd | 0.091 |
| p | 0.38 | 0.42 | 0.82 | 0.41 | 0.55 | 0.041 | 0.69 | nd | 0.18 |
| nCohort 1 | 94 | 205 | 101 | 94 | 205 | 101 | 94 | nd | 101 |
| nCohort 2 | 35 | 10 | 28 | 37 | 8 | 36 | 10 | nd | 12 |
| Cutoff 1 | 6710000 | 6260000 | 7160000 | 7600000 | 7160000 | 7600000 | 7140000 | nd | 7140000 |
| Sens 1 | 71% | 70% | 71% | 70% | 75% | 72% | 90% | nd | 92% |
| Spec 1 | 27% | 17% | 44% | 40% | 33% | 50% | 31% | nd | 41% |
| Cutoff 2 | 6260000 | 5810000 | 6300000 | 6740000 | 5340000 | 6940000 | 7140000 | nd | 7140000 |
| Sens 2 | 80% | 80% | 82% | 81% | 88% | 81% | 90% | nd | 92% |
| Spec 2 | 20% | 15% | 34% | 29% | 12% | 38% | 31% | nd | 41% |
| Cutoff 3 | 5320000 | 5320000 | 5320000 | 5340000 | 4360000 | 5370000 | 7140000 | nd | 7140000 |
| Sens 3 | 94% | 90% | 93% | 92% | 100% | 92% | 90% | nd | 92% |
| Spec 3 | 10% | 8% | 13% | 14% | 4% | 21% | 31% | nd | 41% |
| Cutoff 4 | 1.13E7 | 1.11E7 | 1.05E7 | 1.13E7 | 1.11E7 | 1.05E7 | 1.13E7 | nd | 1.05E7 |
| Sens 4 | 23% | 30% | 25% | 32% | 38% | 36% | 40% | nd | 42% |
| Spec 4 | 70% | 70% | 70% | 70% | 70% | 70% | 70% | nd | 70% |
| Cutoff 5 | 1.43E7 | 1.34E7 | 1.17E7 | 1.43E7 | 1.34E7 | 1.17E7 | 1.43E7 | nd | 1.17E7 |
| Sens 5 | 9% | 20% | 14% | 30% | 25% | 36% | 30% | nd | 42% |
| Spec 5 | 81% | 80% | 80% | 81% | 80% | 80% | 81% | nd | 80% |
| Cutoff 6 | 1.98E7 | 1.71E7 | 1.59E7 | 1.98E7 | 1.71E7 | 1.59E7 | 1.98E7 | nd | 1.59E7 |
| Sens 6 | 3% | 10% | 4% | 16% | 25% | 19% | 20% | nd | 25% |
| Spec 6 | 90% | 90% | 90% | 90% | 90% | 90% | 90% | nd | 90% |
| OR Quart 2 | 1.2 | 0 | 1.5 | 2.2 | 0.49 | 2.7 | 6.0 | nd | 5.9 |
| p Value | 0.72 | na | 0.52 | 0.19 | 0.57 | 0.13 | 0.12 | nd | 0.12 |
| 95% CI of | 0.39 | na | 0.42 | 0.69 | 0.043 | 0.74 | 0.64 | nd | 0.64 |
| OR Quart2 | 3.9 | na | 5.4 | 6.8 | 5.6 | 9.8 | 55 | nd | 54 |
| OR Quart 3 | 1.9 | 1.0 | 2.5 | 1.6 | 1.5 | 3.1 | 0 | nd | 1.0 |
| p Value | 0.24 | 1.0 | 0.15 | 0.42 | 0.65 | 0.080 | na | nd | 1.0 |
| 95% CI of | 0.64 | 0.19 | 0.73 | 0.50 | 0.25 | 0.87 | na | nd | 0.059 |
| OR Quart3 | 5.9 | 5.2 | 8.2 | 5.2 | 9.5 | 11 | na | nd | 17 |
| OR Quart 4 | 1.5 | 1.4 | 1.2 | 2.2 | 0.98 | 4.4 | 4.5 | nd | 5.6 |
| p Value | 0.52 | 0.68 | 0.78 | 0.19 | 0.98 | 0.019 | 0.19 | nd | 0.13 |
| 95% CI of | 0.47 | 0.30 | 0.33 | 0.69 | 0.13 | 1.3 | 0.47 | nd | 0.61 |
| OR Quart4 | 4.5 | 6.5 | 4.4 | 6.8 | 7.2 | 15 | 44 | nd | 52 |

| | | | | | | | | | |
|---|---|---|---|---|---|---|---|---|---|
| **Immunoglogulin G2** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 1.41E7 | | 1.03E7 | 1.41E7 | | 1.08E7 | 1.41E7 | | 1.08E7 |
| Average | 1.56E7 | | 1.09E7 | 1.56E7 | | 1.31E7 | 1.56E7 | | 1.03E7 |
| Stdev | 1.10E7 | | 8880000 | 1.10E7 | | 6520000 | 1.10E7 | | 6120000 |
| p(t-test) | | | 0.027 | | | 0.19 | | | 0.14 |
| Min | 219000 | | 190000 | 219000 | | 1750000 | 219000 | | 1750000 |
| Max | 5.80E7 | | 4.70E7 | 5.80E7 | | 3.11E7 | 5.80E7 | | 1.77E7 |
| n (Samp) | 94 | | 35 | 94 | | 37 | 94 | | 10 |
| n (Patient) | 67 | | 35 | 67 | | 37 | 67 | | 10 |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 1.03E7 | | 1.21E7 | 1.03E7 | | 1.34E7 | nd | | nd |
| Average | 1.30E7 | | 1.47E7 | 1.30E7 | | 1.41E7 | nd | | nd |
| Stdev | 9120000 | | 1.27E7 | 9120000 | | 9110000 | nd | | nd |
| p(t-test) | | | 0.57 | | | 0.74 | nd | | nd |
| Min | 190000 | | 1750000 | 190000 | | 1750000 | nd | | nd |
| Max | 5.80E7 | | 4.70E7 | 5.80E7 | | 2.70E7 | nd | | nd |
| n (Samp) | 205 | | 10 | 205 | | 8 | nd | | nd |
| n (Patient) | 126 | | 10 | 126 | | 8 | nd | | nd |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 1.03E7 | | 1.03E7 | 1.03E7 | | 1.08E7 | 1.03E7 | | 1.08E7 |
| Average | 1.41E7 | | 1.02E7 | 1.41E7 | | 1.38E7 | 1.41E7 | | 1.34E7 |
| Stdev | 1.02E7 | | 7510000 | 1.02E7 | | 7620000 | 1.02E7 | | 1.20E7 |
| p(t-test) | | | 0.060 | | | 0.87 | | | 0.82 |
| Min | 219000 | | 190000 | 219000 | | 1750000 | 219000 | | 1750000 |
| Max | 5.80E7 | | 2.91E7 | 5.80E7 | | 3.69E7 | 5.80E7 | | 4.70E7 |
| n (Samp) | 101 | | 28 | 101 | | 36 | 101 | | 12 |
| n (Patient) | 65 | | 28 | 65 | | 36 | 65 | | 12 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.38 | 0.52 | 0.41 | 0.47 | 0.56 | 0.53 | 0.39 | nd | 0.48 |
| SE | 0.057 | 0.095 | 0.063 | 0.057 | 0.11 | 0.057 | 0.099 | nd | 0.089 |
| p | 0.036 | 0.79 | 0.14 | 0.59 | 0.58 | 0.58 | 0.27 | nd | 0.79 |
| nCohort 1 | 94 | 205 | 101 | 94 | 205 | 101 | 94 | nd | 101 |
| nCohort 2 | 35 | 10 | 28 | 37 | 8 | 36 | 10 | nd | 12 |
| Cutoff 1 | 6810000 | 9110000 | 2450000 | 1.02E7 | 1.02E7 | 9110000 | 6700000 | nd | 6700000 |
| Sens 1 | 71% | 80% | 71% | 70% | 75% | 75% | 70% | nd | 75% |
| Spec 1 | 19% | 31% | 15% | 38% | 41% | 31% | 16% | nd | 20% |
| Cutoff 2 | 1750000 | 9110000 | 219000 | 7340000 | 4550000 | 7850000 | 4550000 | nd | 4550000 |
| Sens 2 | 80% | 80% | 96% | 84% | 88% | 83% | 80% | nd | 83% |
| Spec 2 | 9% | 31% | 1% | 21% | 18% | 26% | 14% | nd | 17% |
| Cutoff 3 | 563000 | 1930000 | 219000 | 4550000 | 563000 | 5450000 | 1750000 | nd | 1750000 |
| Sens 3 | 97% | 90% | 96% | 92% | 100% | 92% | 90% | nd | 92% |
| Spec 3 | 2% | 11% | 1% | 14% | 1% | 18% | 9% | nd | 8% |
| Cutoff 4 | 1.77E7 | 1.61E7 | 1.64E7 | 1.77E7 | 1.61E7 | 1.64E7 | 1.77E7 | nd | 1.64E7 |
| Sens 4 | 14% | 30% | 18% | 16% | 50% | 31% | 0% | nd | 33% |
| Spec 4 | 71% | 70% | 72% | 71% | 70% | 72% | 71% | nd | 72% |
| Cutoff 5 | 2.34E7 | 1.77E7 | 2.09E7 | 2.34E7 | 1.77E7 | 2.09E7 | 2.34E7 | nd | 2.09E7 |
| Sens 5 | 6% | 20% | 7% | 8% | 25% | 11% | 0% | nd | 8% |
| Spec 5 | 81% | 81% | 80% | 81% | 81% | 80% | 81% | nd | 80% |
| Cutoff 6 | 3.13E7 | 2.52E7 | 2.66E7 | 3.13E7 | 2.52E7 | 2.66E7 | 3.13E7 | nd | 2.66E7 |
| Sens 6 | 3% | 10% | 4% | 0% | 25% | 8% | 0% | nd | 8% |
| Spec 6 | 91% | 91% | 90% | 91% | 91% | 90% | 91% | nd | 90% |
| OR Quart 2 | 1.5 | 1.5 | 1.6 | 2.2 | 1.0 | 1.2 | >4.7 | nd | 2.9 |
| p Value | 0.51 | 0.66 | 0.49 | 0.16 | 1.0 | 0.77 | <0.18 | nd | 0.22 |
| 95% CI of | 0.46 | 0.24 | 0.44 | 0.72 | 0.14 | 0.38 | >0.49 | nd | 0.52 |
| OR Quart2 | 4.9 | 9.4 | 5.6 | 7.1 | 7.4 | 3.7 | na | nd | 17 |
| OR Quart 3 | 2.0 | 0.98 | 1.6 | 2.6 | 0.49 | 2.1 | >2.2 | nd | 0.50 |
| p Value | 0.23 | 0.98 | 0.49 | 0.10 | 0.57 | 0.18 | <0.54 | nd | 0.58 |
| 95% CI of | 0.64 | 0.13 | 0.44 | 0.83 | 0.043 | 0.71 | >0.18 | nd | 0.043 |
| OR Quart3 | 6.5 | 7.2 | 5.6 | 8.0 | 5.6 | 6.3 | na | nd | 5.8 |
| OR Quart 4 | 2.4 | 1.5 | 2.2 | 1.5 | 1.5 | 1.3 | >4.7 | nd | 2.2 |
| p Value | 0.14 | 0.66 | 0.21 | 0.51 | 0.66 | 0.61 | <0.18 | nd | 0.37 |
| 95% CI of | 0.75 | 0.24 | 0.64 | 0.46 | 0.24 | 0.43 | >0.49 | nd | 0.38 |
| OR Quart4 | 7.4 | 9.4 | 7.5 | 4.9 | 9.4 | 4.1 | na | nd | 13 |

| | | | | | | | | | |
|---|---|---|---|---|---|---|---|---|---|
| **Interleukin-11** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 60.0 | | 90.7 | 60.0 | | 74.1 | 60.0 | | 44.3 |
| Average | 245 | | 134 | 245 | | 115 | 245 | | 90.2 |
| Stdev | 951 | | 286 | 951 | | 141 | 951 | | 135 |
| p(t-test) | | | 0.45 | | | 0.38 | | | 0.49 |
| Min | 0.368 | | 0.359 | 0.368 | | 0.359 | 0.368 | | 0.359 |
| Max | 6920 | | 1940 | 6920 | | 802 | 6920 | | 569 |
| n (Samp) | 94 | | 45 | 94 | | 42 | 94 | | 18 |
| n (Patient) | 65 | | 45 | 65 | | 42 | 65 | | 18 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 59.4 | | 91.0 | 59.4 | | 89.3 | 59.4 | | 67.2 |
| Average | 148 | | 236 | 148 | | 465 | 148 | | 118 |
| Stdev | 621 | | 502 | 621 | | 1250 | 621 | | 171 |
| p(t-test) | | | 0.60 | | | 0.060 | | | 0.89 |
| Min | 0.359 | | 0.480 | 0.359 | | 0.442 | 0.359 | | 20.4 |
| Max | 6920 | | 1940 | 6920 | | 5420 | 6920 | | 569 |
| n (Samp) | 225 | | 14 | 225 | | 18 | 225 | | 9 |
| n (Patient) | 132 | | 14 | 132 | | 18 | 132 | | 9 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 47.4 | | 71.6 | 47.4 | | 83.4 | 47.4 | | 38.4 |
| Average | 218 | | 83.1 | 218 | | 107 | 218 | | 60.7 |
| Stdev | 909 | | 62.4 | 909 | | 125 | 909 | | 69.0 |
| p(t-test) | | | 0.37 | | | 0.44 | | | 0.51 |
| Min | 0.368 | | 0.359 | 0.368 | | 0.359 | 0.368 | | 0.359 |
| Max | 6920 | | 234 | 6920 | | 741 | 6920 | | 233 |
| n (Samp) | 103 | | 37 | 103 | | 41 | 103 | | 15 |
| n (Patient) | 64 | | 37 | 64 | | 41 | 64 | | 15 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.53 | 0.57 | 0.53 | 0.53 | 0.64 | 0.57 | 0.47 | 0.56 | 0.44 |
| SE | 0.053 | 0.082 | 0.056 | 0.054 | 0.073 | 0.054 | 0.075 | 0.10 | 0.082 |
| p | 0.56 | 0.39 | 0.57 | 0.53 | 0.048 | 0.22 | 0.73 | 0.56 | 0.50 |
| nCohort 1 | 94 | 225 | 103 | 94 | 225 | 103 | 94 | 225 | 103 |
| nCohort 2 | 45 | 14 | 37 | 42 | 18 | 41 | 18 | 9 | 15 |
| Cutoff 1 | 46.9 | 38.4 | 42.3 | 41.7 | 55.3 | 47.4 | 33.9 | 43.7 | 20.2 |
| Sens 1 | 71% | 71% | 70% | 71% | 72% | 71% | 72% | 78% | 73% |
| Spec 1 | 46% | 39% | 47% | 41% | 49% | 50% | 40% | 44% | 33% |
| Cutoff 2 | 29.7 | 6.00 | 15.1 | 15.1 | 29.7 | 20.2 | 20.2 | 38.4 | 7.90 |
| Sens 2 | 82% | 86% | 81% | 81% | 83% | 83% | 83% | 89% | 87% |
| Spec 2 | 34% | 16% | 26% | 23% | 31% | 33% | 31% | 39% | 24% |
| Cutoff 3 | 0.608 | 0.442 | 0.442 | 6.00 | 10.7 | 6.00 | 7.90 | 20.2 | 0.442 |
| Sens 3 | 91% | 100% | 92% | 90% | 94% | 90% | 94% | 100% | 93% |
| Spec 3 | 9% | 8% | 6% | 15% | 23% | 16% | 22% | 28% | 6% |
| Cutoff 4 | 139 | 113 | 118 | 139 | 113 | 118 | 139 | 113 | 118 |
| Sens 4 | 24% | 29% | 24% | 29% | 44% | 29% | 17% | 11% | 13% |
| Spec 4 | 70% | 71% | 71% | 70% | 71% | 71% | 70% | 71% | 71% |
| Cutoff 5 | 190 | 155 | 159 | 190 | 155 | 159 | 190 | 155 | 159 |
| Sens 5 | 9% | 29% | 14% | 19% | 44% | 17% | 17% | 11% | 13% |
| Spec 5 | 81% | 80% | 81% | 81% | 80% | 81% | 81% | 80% | 81% |
| Cutoff 6 | 270 | 209 | 221 | 270 | 209 | 221 | 270 | 209 | 221 |
| Sens 6 | 4% | 21% | 3% | 7% | 33% | 7% | 6% | 11% | 7% |
| Spec 6 | 90% | 91% | 90% | 90% | 91% | 90% | 90% | 91% | 90% |
| OR Quart 2 | 1.5 | 0.64 | 1.2 | 1.4 | 0.98 | 2.2 | 1.4 | >3.1 | 1.6 |
| p Value | 0.46 | 0.64 | 0.77 | 0.58 | 0.98 | 0.17 | 0.69 | <0.33 | 0.61 |
| 95% CI of | 0.51 | 0.10 | 0.38 | 0.46 | 0.19 | 0.71 | 0.28 | >0.31 | 0.25 |
| OR Quart2 | 4.3 | 4.0 | 3.7 | 4.0 | 5.1 | 6.8 | 6.9 | na | 10 |
| OR Quart 3 | 3.1 | 1.7 | 2.7 | 2.3 | 1.3 | 3.6 | 3.3 | >5.5 | 3.5 |
| p Value | 0.033 | 0.48 | 0.072 | 0.12 | 0.71 | 0.023 | 0.10 | <0.13 | 0.15 |
| 95% CI of | 1.1 | 0.39 | 0.92 | 0.80 | 0.29 | 1.2 | 0.78 | >0.62 | 0.65 |
| OR Quart3 | 8.6 | 7.4 | 7.8 | 6.5 | 6.2 | 11 | 14 | na | 19 |
| OR Quart 4 | 1.1 | 1.3 | 1.2 | 1.4 | 2.9 | 1.7 | 1.0 | >1.0 | 2.2 |
| p Value | 0.83 | 0.71 | 0.77 | 0.58 | 0.13 | 0.39 | 1.0 | <1.0 | 0.37 |
| 95% CI of | 0.38 | 0.29 | 0.38 | 0.46 | 0.72 | 0.52 | 0.18 | >0.061 | 0.38 |
| OR Quart4 | 3.4 | 6.2 | 3.7 | 4.0 | 11 | 5.3 | 5.4 | na | 13 |

| | | | | | | | | | |
|---|---|---|---|---|---|---|---|---|---|
| **Interleukin-2 receptor alpha chain** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 18.1 | | 22.6 | 18.1 | | 18.3 | 18.1 | | 20.6 |
| Average | 38.7 | | 84.1 | 38.7 | | 115 | 38.7 | | 90.3 |
| Stdev | 51.1 | | 146 | 51.1 | | 221 | 51.1 | | 180 |
| p(t-test) | | | 0.0079 | | | 0.0018 | | | 0.020 |
| Min | 0.0290 | | 0.0290 | 0.0290 | | 0.0290 | 0.0290 | | 0.0515 |
| Max | 232 | | 715 | 232 | | 1040 | 232 | | 714 |
| n (Samp) | 94 | | 45 | 94 | | 42 | 94 | | 18 |
| n (Patient) | 65 | | 45 | 65 | | 42 | 65 | | 18 |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 18.3 | | 47.9 | 18.3 | | 42.6 | 18.3 | | 22.6 |
| Average | 61.1 | | 86.0 | 61.1 | | 95.8 | 61.1 | | 148 |
| Stdev | 121 | | 135 | 121 | | 174 | 121 | | 282 |
| p(t-test) | | | 0.46 | | | 0.26 | | | 0.049 |
| Min | 0.0290 | | 0.0461 | 0.0290 | | 0.0290 | 0.0290 | | 0.0290 |
| Max | 1040 | | 518 | 1040 | | 715 | 1040 | | 862 |
| n (Samp) | 225 | | 14 | 225 | | 18 | 225 | | 9 |
| n (Patient) | 132 | | 14 | 132 | | 18 | 132 | | 9 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 29.5 | | 13.6 | 29.5 | | 39.6 | 29.5 | | 24.1 |
| Average | 46.4 | | 89.1 | 46.4 | | 152 | 46.4 | | 98.0 |
| Stdev | 53.6 | | 159 | 53.6 | | 261 | 53.6 | | 192 |
| p(t-test) | | | 0.018 | | | 1.3E-4 | | | 0.027 |
| Min | 0.0290 | | 0.0290 | 0.0290 | | 0.0290 | 0.0290 | | 0.0515 |
| Max | 232 | | 715 | 232 | | 1040 | 232 | | 714 |
| n (Samp) | 103 | | 37 | 103 | | 41 | 103 | | 15 |
| n (Patient) | 64 | | 37 | 64 | | 41 | 64 | | 15 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.56 | 0.60 | 0.51 | 0.56 | 0.58 | 0.57 | 0.56 | 0.53 | 0.54 |
| SE | 0.053 | 0.082 | 0.056 | 0.054 | 0.073 | 0.054 | 0.076 | 0.100 | 0.081 |
| p | 0.25 | 0.22 | 0.80 | 0.31 | 0.29 | 0.21 | 0.46 | 0.79 | 0.60 |
| nCohort 1 | 94 | 225 | 103 | 94 | 225 | 103 | 94 | 225 | 103 |
| nCohort 2 | 45 | 14 | 37 | 42 | 18 | 41 | 18 | 9 | 15 |
| Cutoff 1 | 2.97 | 8.15 | 2.33 | 0.0569 | 11.0 | 4.31 | 5.94 | 0.0569 | 14.2 |
| Sens 1 | 71% | 71% | 70% | 71% | 72% | 71% | 72% | 78% | 73% |
| Spec 1 | 32% | 40% | 27% | 24% | 43% | 30% | 37% | 21% | 40% |
| Cutoff 2 | 0.0515 | 2.19 | 0.0515 | 0.0515 | 0.0515 | 0.0515 | 0.0612 | 0.0515 | 5.94 |
| Sens 2 | 84% | 86% | 84% | 81% | 89% | 83% | 83% | 89% | 80% |
| Spec 2 | 17% | 30% | 17% | 17% | 16% | 17% | 29% | 16% | 31% |
| Cutoff 3 | 0.0359 | 0.0359 | 0.0290 | 0.0359 | 0.0290 | 0.0359 | 0.0515 | 0 | 0.0612 |
| Sens 3 | 91% | 100% | 95% | 90% | 94% | 95% | 94% | 100% | 93% |
| Spec 3 | 11% | 9% | 5% | 11% | 4% | 10% | 17% | 0% | 23% |
| Cutoff 4 | 41.0 | 45.8 | 56.7 | 41.0 | 45.8 | 56.7 | 41.0 | 45.8 | 56.7 |
| Sens 4 | 44% | 50% | 35% | 45% | 50% | 41% | 28% | 33% | 33% |
| Spec 4 | 70% | 70% | 71% | 70% | 70% | 71% | 70% | 70% | 71% |
| Cutoff 5 | 68.3 | 84.9 | 89.4 | 68.3 | 84.9 | 89.4 | 68.3 | 84.9 | 89.4 |
| Sens 5 | 36% | 36% | 27% | 31% | 22% | 34% | 28% | 33% | 27% |
| Spec 5 | 81% | 80% | 81% | 81% | 80% | 81% | 81% | 80% | 81% |
| Cutoff 6 | 117 | 153 | 132 | 117 | 153 | 132 | 117 | 153 | 132 |
| Sens 6 | 22% | 14% | 19% | 24% | 11% | 29% | 17% | 33% | 13% |
| Spec 6 | 90% | 90% | 90% | 90% | 90% | 90% | 90% | 90% | 90% |
| OR Quart 2 | 1.3 | 1.5 | 0.87 | 0.66 | 0.72 | 0.74 | 1.8 | 0.32 | 3.4 |
| p Value | 0.66 | 0.66 | 0.79 | 0.43 | 0.68 | 0.59 | 0.45 | 0.32 | 0.16 |
| 95% CI of | 0.45 | 0.24 | 0.30 | 0.23 | 0.16 | 0.25 | 0.39 | 0.032 | 0.62 |
| OR Quart2 | 3.5 | 9.3 | 2.5 | 1.9 | 3.4 | 2.2 | 8.4 | 3.1 | 18 |
| OR Quart 3 | 0.60 | 1.5 | 0.62 | 0.39 | 0.98 | 0.87 | 1.8 | 0.65 | 1.6 |
| p Value | 0.37 | 0.66 | 0.41 | 0.10 | 0.98 | 0.79 | 0.45 | 0.65 | 0.64 |
| 95% CI of | 0.20 | 0.24 | 0.21 | 0.13 | 0.23 | 0.30 | 0.39 | 0.11 | 0.24 |
| OR Quart3 | 1.8 | 9.3 | 1.9 | 1.2 | 4.1 | 2.5 | 8.4 | 4.1 | 10 |
| OR Quart 4 | 2.0 | 3.2 | 1.1 | 1.4 | 1.8 | 1.7 | 1.8 | 0.98 | 2.1 |
| p Value | 0.16 | 0.17 | 0.79 | 0.46 | 0.36 | 0.32 | 0.45 | 0.98 | 0.42 |
| 95% CI of | 0.75 | 0.61 | 0.41 | 0.55 | 0.50 | 0.61 | 0.39 | 0.19 | 0.35 |
| OR Quart4 | 5.5 | 16 | 3.2 | 3.8 | 6.6 | 4.5 | 8.4 | 5.1 | 12 |

| | | | | | | | | | |
|---|---|---|---|---|---|---|---|---|---|
| **Protransforming growth factor alpha** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 1.51 | | 1.45 | 1.51 | | 2.44 | 1.51 | | 4.16 |
| Average | 6.28 | | 6.62 | 6.28 | | 13.7 | 6.28 | | 7.24 |
| Stdev | 30.0 | | 24.2 | 30.0 | | 43.6 | 30.0 | | 9.10 |
| p(t-test) | | | 0.95 | | | 0.25 | | | 0.89 |
| Min | 0.00228 | | 0.00228 | 0.00228 | | 0.00305 | 0.00228 | | 0.00305 |
| Max | 287 | | 162 | 287 | | 245 | 287 | | 30.3 |
| n (Samp) | 94 | | 45 | 94 | | 42 | 94 | | 18 |
| n (Patient) | 65 | | 45 | 65 | | 42 | 65 | | 18 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 1.47 | | 2.13 | 1.47 | | 3.26 | 1.47 | | 0.885 |
| Average | 7.40 | | 5.15 | 7.40 | | 31.0 | 7.40 | | 4.52 |
| Stdev | 27.1 | | 8.71 | 27.1 | | 67.7 | 27.1 | | 9.72 |
| p(t-test) | | | 0.76 | | | 0.0027 | | | 0.75 |
| Min | 0.00228 | | 0.00228 | 0.00228 | | 0.00305 | 0.00228 | | 0.0105 |
| Max | 287 | | 30.8 | 287 | | 245 | 287 | | 30.3 |
| n (Samp) | 225 | | 14 | 225 | | 18 | 225 | | 9 |
| n (Patient) | 132 | | 14 | 132 | | 18 | 132 | | 9 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 1.22 | | 1.26 | 1.22 | | 2.41 | 1.22 | | 4.80 |
| Average | 5.94 | | 6.29 | 5.94 | | 9.09 | 5.94 | | 6.62 |
| Stdev | 28.7 | | 26.3 | 28.7 | | 25.6 | 28.7 | | 7.52 |
| p(t-test) | | | 0.95 | | | 0.54 | | | 0.93 |
| Min | 0.00228 | | 0.00305 | 0.00228 | | 0.00228 | 0.00228 | | 0.00305 |
| Max | 287 | | 162 | 287 | | 153 | 287 | | 28.3 |
| n (Samp) | 103 | | 37 | 103 | | 41 | 103 | | 15 |
| n (Patient) | 64 | | 37 | 64 | | 41 | 64 | | 15 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.51 | 0.50 | 0.51 | 0.58 | 0.63 | 0.59 | 0.71 | 0.49 | 0.73 |
| SE | 0.053 | 0.080 | 0.056 | 0.054 | 0.073 | 0.054 | 0.073 | 0.099 | 0.078 |
| p | 0.86 | 0.99 | 0.81 | 0.12 | 0.067 | 0.084 | 0.0047 | 0.90 | 0.0034 |
| nCohort 1 | 94 | 225 | 103 | 94 | 225 | 103 | 94 | 225 | 103 |
| nCohort 2 | 45 | 14 | 37 | 42 | 18 | 41 | 18 | 9 | 15 |
| Cutoff 1 | 0.597 | 0.490 | 0.597 | 0.666 | 0.875 | 0.875 | 1.35 | 0.674 | 3.84 |
| Sens 1 | 71% | 71% | 70% | 71% | 72% | 71% | 72% | 78% | 73% |
| Spec 1 | 37% | 29% | 41% | 38% | 38% | 46% | 48% | 34% | 81% |
| Cutoff 2 | 0.0907 | 0.00228 | 0.0907 | 0.490 | 0.637 | 0.141 | 0.875 | 0.666 | 1.35 |
| Sens 2 | 80% | 86% | 81% | 81% | 83% | 80% | 83% | 89% | 80% |
| Spec 2 | 21% | 4% | 22% | 32% | 34% | 27% | 41% | 34% | 52% |
| Cutoff 3 | 0.00305 | 0 | 0.00305 | 0.00305 | 0.0223 | 0.00305 | 0.340 | 0.00584 | 0.0907 |
| Sens 3 | 91% | 100% | 95% | 93% | 94% | 93% | 94% | 100% | 93% |
| Spec 3 | 15% | 0% | 17% | 15% | 20% | 17% | 29% | 17% | 22% |
| Cutoff 4 | 3.05 | 3.27 | 2.83 | 3.05 | 3.27 | 2.83 | 3.05 | 3.27 | 2.83 |
| Sens 4 | 24% | 29% | 19% | 38% | 50% | 41% | 67% | 22% | 73% |
| Spec 4 | 70% | 70% | 71% | 70% | 70% | 71% | 70% | 70% | 71% |
| Cutoff 5 | 3.96 | 4.80 | 3.84 | 3.96 | 4.80 | 3.84 | 3.96 | 4.80 | 3.84 |
| Sens 5 | 24% | 29% | 19% | 33% | 39% | 32% | 56% | 11% | 73% |
| Spec 5 | 81% | 80% | 81% | 81% | 80% | 81% | 81% | 80% | 81% |
| Cutoff 6 | 6.50 | 9.07 | 7.79 | 6.50 | 9.07 | 7.79 | 6.50 | 9.07 | 7.79 |
| Sens 6 | 13% | 14% | 8% | 24% | 33% | 22% | 28% | 11% | 20% |
| Spec 6 | 90% | 90% | 90% | 90% | 90% | 90% | 90% | 90% | 90% |
| OR Quart 2 | 1.4 | 0.47 | 1.4 | 1.6 | 2.6 | 1.3 | 5.9 | 2.1 | 0.96 |
| p Value | 0.50 | 0.40 | 0.59 | 0.42 | 0.27 | 0.59 | 0.12 | 0.56 | 0.97 |
| 95% CI of | 0.52 | 0.083 | 0.46 | 0.53 | 0.48 | 0.46 | 0.64 | 0.18 | 0.13 |
| OR Quart2 | 3.9 | 2.7 | 4.0 | 4.5 | 14 | 3.9 | 54 | 23 | 7.3 |
| OR Quart 3 | 1.1 | 0.98 | 1.8 | 1.2 | 1.5 | 1.3 | 2.1 | 5.4 | 0.48 |
| p Value | 0.86 | 0.98 | 0.29 | 0.78 | 0.66 | 0.59 | 0.56 | 0.13 | 0.56 |
| 95% CI of | 0.39 | 0.23 | 0.61 | 0.39 | 0.24 | 0.46 | 0.18 | 0.61 | 0.041 |
| OR Quart3 | 3.1 | 4.1 | 5.0 | 3.5 | 9.3 | 3.9 | 24 | 47 | 5.6 |
| OR Quart 4 | 1.1 | 0.98 | 0.84 | 2.3 | 4.4 | 2.0 | 15 | 1.0 | 6.8 |
| p Value | 0.86 | 0.98 | 0.77 | 0.12 | 0.069 | 0.20 | 0.013 | 0.99 | 0.021 |
| 95% CI of | 0.39 | 0.23 | 0.27 | 0.80 | 0.89 | 0.70 | 1.8 | 0.062 | 1.3 |
| OR Quart4 | 3.1 | 4.1 | 2.6 | 6.5 | 22 | 5.6 | 130 | 17 | 34 |

| | | | | | | | | | |
|---|---|---|---|---|---|---|---|---|---|
| **CA 15-3** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 0.248 | | 0.246 | 0.248 | | 0.380 | 0.248 | | 0.210 |
| Average | 0.476 | | 0.678 | 0.476 | | 1.88 | 0.476 | | 3.33 |
| Stdev | 0.801 | | 1.63 | 0.801 | | 6.00 | 0.801 | | 12.1 |
| p(t-test) | | | 0.28 | | | 0.012 | | | 0.010 |
| Min | 0.00165 | | 0.00165 | 0.00165 | | 0.0177 | 0.00165 | | 0.0355 |
| Max | 6.54 | | 11.0 | 6.54 | | 36.7 | 6.54 | | 60.0 |
| n (Samp) | 121 | | 53 | 121 | | 53 | 121 | | 26 |
| n (Patient) | 87 | | 53 | 87 | | 53 | 87 | | 26 |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 0.276 | | 0.254 | 0.276 | | 0.528 | 0.276 | | 0.360 |
| Average | 1.11 | | 0.413 | 1.11 | | 1.07 | 1.11 | | 0.876 |
| Stdev | 4.63 | | 0.570 | 4.63 | | 2.34 | 4.63 | | 1.70 |
| p(t-test) | | | 0.55 | | | 0.98 | | | 0.87 |
| Min | 0.00165 | | 0.0294 | 0.00165 | | 0.0463 | 0.00165 | | 0.0355 |
| Max | 60.0 | | 2.27 | 60.0 | | 9.48 | 60.0 | | 5.66 |
| n (Samp) | 288 | | 16 | 288 | | 15 | 288 | | 10 |
| n (Patient) | 161 | | 16 | 161 | | 15 | 161 | | 10 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 0.292 | | 0.246 | 0.292 | | 0.395 | 0.292 | | 0.399 |
| Average | 0.541 | | 0.734 | 0.541 | | 1.88 | 0.541 | | 3.76 |
| Stdev | 0.887 | | 1.78 | 0.887 | | 5.95 | 0.887 | | 12.8 |
| p(t-test) | | | 0.36 | | | 0.015 | | | 0.0055 |
| Min | 0.00165 | | 0.00165 | 0.00165 | | 0.0177 | 0.00165 | | 0.0642 |
| Max | 6.54 | | 11.0 | 6.54 | | 36.7 | 6.54 | | 60.0 |
| n (Samp) | 125 | | 43 | 125 | | 54 | 125 | | 23 |
| n (Patient) | 80 | | 43 | 80 | | 54 | 80 | | 23 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.51 | 0.43 | 0.49 | 0.62 | 0.62 | 0.60 | 0.52 | 0.49 | 0.54 |
| SE | 0.048 | 0.076 | 0.051 | 0.047 | 0.079 | 0.047 | 0.063 | 0.093 | 0.067 |
| p | 0.78 | 0.35 | 0.85 | 0.012 | 0.14 | 0.036 | 0.76 | 0.95 | 0.51 |
| nCohort 1 | 121 | 288 | 125 | 121 | 288 | 125 | 121 | 288 | 125 |
| nCohort 2 | 53 | 16 | 43 | 53 | 15 | 54 | 26 | 10 | 23 |
| Cutoff 1 | 0.171 | 0.0734 | 0.172 | 0.203 | 0.345 | 0.235 | 0.126 | 0.147 | 0.148 |
| Sens 1 | 72% | 75% | 72% | 72% | 73% | 70% | 73% | 70% | 74% |
| Spec 1 | 32% | 6% | 28% | 40% | 60% | 41% | 25% | 24% | 26% |
| Cutoff 2 | 0.126 | 0.0598 | 0.136 | 0.143 | 0.187 | 0.186 | 0.107 | 0.100 | 0.126 |
| Sens 2 | 81% | 81% | 81% | 83% | 80% | 81% | 81% | 80% | 83% |
| Spec 2 | 25% | 5% | 22% | 26% | 32% | 30% | 22% | 15% | 22% |
| Cutoff 3 | 0.0598 | 0.0294 | 0.0734 | 0.117 | 0.117 | 0.127 | 0.0762 | 0.0762 | 0.107 |
| Sens 3 | 91% | 94% | 91% | 91% | 93% | 91% | 92% | 90% | 91% |
| Spec 3 | 7% | 2% | 8% | 23% | 18% | 22% | 8% | 7% | 20% |
| Cutoff 4 | 0.373 | 0.447 | 0.411 | 0.373 | 0.447 | 0.411 | 0.373 | 0.447 | 0.411 |
| Sens 4 | 34% | 19% | 33% | 51% | 60% | 48% | 46% | 50% | 48% |
| Spec 4 | 70% | 70% | 70% | 70% | 70% | 70% | 70% | 70% | 70% |
| Cutoff 5 | 0.536 | 0.685 | 0.720 | 0.536 | 0.685 | 0.720 | 0.536 | 0.685 | 0.720 |
| Sens 5 | 25% | 12% | 16% | 34% | 27% | 26% | 27% | 30% | 22% |
| Spec 5 | 80% | 80% | 80% | 80% | 80% | 80% | 80% | 80% | 80% |
| Cutoff 6 | 1.08 | 1.41 | 1.14 | 1.08 | 1.41 | 1.14 | 1.08 | 1.41 | 1.14 |
| Sens 6 | 9% | 6% | 9% | 17% | 7% | 19% | 15% | 10% | 17% |
| Spec 6 | 90% | 90% | 90% | 90% | 90% | 90% | 90% | 90% | 90% |
| OR Quart 2 | 1.3 | 1.4 | 0.77 | 0.75 | 0.32 | 1.2 | 0.97 | 0.24 | 0.81 |
| p Value | 0.53 | 0.70 | 0.61 | 0.57 | 0.33 | 0.67 | 0.95 | 0.21 | 0.74 |
| 95% CI of | 0.54 | 0.29 | 0.28 | 0.27 | 0.033 | 0.47 | 0.30 | 0.027 | 0.22 |
| OR Quart2 | 3.3 | 6.3 | 2.1 | 2.0 | 3.1 | 3.2 | 3.1 | 2.2 | 2.9 |
| OR Quart 3 | 0.89 | 1.4 | 1.3 | 1.4 | 1.3 | 1.7 | 0.24 | 0.24 | 0.63 |
| p Value | 0.81 | 0.70 | 0.63 | 0.48 | 0.71 | 0.27 | 0.086 | 0.21 | 0.50 |
| 95% CI of | 0.34 | 0.29 | 0.49 | 0.55 | 0.29 | 0.66 | 0.046 | 0.026 | 0.16 |
| OR Quart3 | 2.3 | 6.3 | 3.3 | 3.6 | 6.2 | 4.3 | 1.2 | 2.2 | 2.4 |
| OR Quart 4 | 1.3 | 1.7 | 0.88 | 2.2 | 2.4 | 2.1 | 1.5 | 1.0 | 1.4 |
| p Value | 0.53 | 0.47 | 0.80 | 0.087 | 0.21 | 0.13 | 0.45 | 0.98 | 0.55 |
| 95% CI of | 0.54 | 0.39 | 0.33 | 0.89 | 0.61 | 0.82 | 0.51 | 0.24 | 0.44 |
| OR Quart4 | 3.3 | 7.4 | 2.4 | 5.5 | 9.8 | 5.2 | 4.6 | 4.2 | 4.6 |

**Table 6: Comparison of marker levels in EDTA samples collected from Cohort 1 (patients that did not progress beyond RIFLE stage 0 or R) and in EDTA samples collected from subjects at 0, 24 hours, and 48 hours prior to reaching stage I or F in Cohort 2.**

| **C-C motif chemokine 18** | | | | | | | | | |
|---|---|---|---|---|---|---|---|---|---|
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 172 | | 215 | 172 | | 248 | 172 | | 220 |
| Average | 240 | | 229 | 240 | | 325 | 240 | | 302 |
| Stdev | 220 | | 100 | 220 | | 300 | 220 | | 325 |
| p(t-test) | | | 0.85 | | | 0.062 | | | 0.24 |
| Min | 31.7 | | 51.5 | 31.7 | | 92.4 | 31.7 | | 103 |
| Max | 2110 | | 378 | 2110 | | 1650 | 2110 | | 1550 |
| n (Samp) | 282 | | 16 | 282 | | 28 | 282 | | 20 |
| n (Patient) | 160 | | 16 | 160 | | 28 | 160 | | 20 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | nd | | nd | nd | | nd | 180 | | 266 |
| Average | nd | | nd | nd | | nd | 252 | | 280 |
| Stdev | nd | | nd | nd | | nd | 237 | | 101 |
| p(t-test) | nd | | nd | nd | | nd | | | 0.77 |
| Min | nd | | nd | nd | | nd | 31.7 | | 154 |
| Max | nd | | nd | nd | | nd | 2110 | | 422 |
| n (Samp) | nd | | nd | nd | | nd | 353 | | 6 |
| n (Patient) | nd | | nd | nd | | nd | 193 | | 6 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 176 | | 238 | 176 | | 254 | 176 | | 198 |
| Average | 251 | | 231 | 251 | | 328 | 251 | | 310 |
| Stdev | 226 | | 108 | 226 | | 307 | 226 | | 354 |
| p(t-test) | | | 0.75 | | | 0.11 | | | 0.32 |
| Min | 37.8 | | 51.5 | 37.8 | | 92.4 | 37.8 | | 103 |
| Max | 2110 | | 378 | 2110 | | 1650 | 2110 | | 1550 |
| n (Samp) | 258 | | 13 | 258 | | 26 | 258 | | 17 |
| n (Patient) | 140 | | 13 | 140 | | 26 | 140 | | 17 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.57 | nd | 0.55 | 0.64 | nd | 0.63 | 0.59 | 0.67 | 0.55 |
| SE | 0.076 | nd | 0.084 | 0.059 | nd | 0.061 | 0.069 | 0.12 | 0.074 |
| p | 0.33 | nd | 0.56 | 0.020 | nd | 0.040 | 0.18 | 0.17 | 0.46 |
| nCohort 1 | 282 | nd | 258 | 282 | nd | 258 | 282 | 353 | 258 |
| nCohort 2 | 16 | nd | 13 | 28 | nd | 26 | 20 | 6 | 17 |
| Cutoff 1 | 161 | nd | 159 | 169 | nd | 189 | 152 | 207 | 120 |
| Sens 1 | 75% | nd | 77% | 71% | nd | 73% | 70% | 83% | 71% |
| Spec 1 | 45% | nd | 42% | 48% | nd | 55% | 41% | 59% | 30% |
| Cutoff 2 | 159 | nd | 144 | 132 | nd | 150 | 118 | 207 | 117 |
| Sens 2 | 81% | nd | 85% | 82% | nd | 81% | 80% | 83% | 82% |
| Spec 2 | 44% | nd | 38% | 36% | nd | 40% | 31% | 59% | 29% |
| Cutoff 3 | 87.9 | nd | 87.9 | 107 | nd | 107 | 105 | 152 | 103 |
| Sens 3 | 94% | nd | 92% | 93% | nd | 92% | 90% | 100% | 94% |
| Spec 3 | 19% | nd | 18% | 27% | nd | 26% | 26% | 39% | 24% |
| Cutoff 4 | 268 | nd | 278 | 268 | nd | 278 | 268 | 276 | 278 |
| Sens 4 | 38% | nd | 38% | 46% | nd | 46% | 40% | 50% | 35% |
| Spec 4 | 70% | nd | 70% | 70% | nd | 70% | 70% | 70% | 70% |
| Cutoff 5 | 345 | nd | 384 | 345 | nd | 384 | 345 | 347 | 384 |
| Sens 5 | 19% | nd | 0% | 32% | nd | 19% | 15% | 33% | 12% |
| Spec 5 | 80% | nd | 80% | 80% | nd | 80% | 80% | 80% | 80% |
| Cutoff 6 | 497 | nd | 537 | 497 | nd | 537 | 497 | 497 | 537 |
| Sens 6 | 0% | nd | 0% | 11% | nd | 12% | 10% | 0% | 12% |
| Spec 6 | 90% | nd | 90% | 90% | nd | 90% | 90% | 90% | 90% |
| OR Quart 2 | 2.0 | nd | 1.5 | 3.7 | nd | 1.4 | 1.3 | >1.0 | 1.3 |
| p Value | 0.42 | nd | 0.66 | 0.11 | nd | 0.70 | 0.71 | <1.0 | 0.71 |
| 95% CI of | 0.36 | nd | 0.24 | 0.74 | nd | 0.29 | 0.29 | >0.062 | 0.29 |
| OR Quart2 | 11 | nd | 9.3 | 18 | nd | 6.3 | 6.2 | na | 6.2 |
| OR Quart 3 | 2.6 | nd | 2.0 | 4.3 | nd | 3.3 | 2.1 | >2.0 | 1.7 |
| p Value | 0.26 | nd | 0.42 | 0.069 | nd | 0.084 | 0.31 | <0.57 | 0.48 |
| 95% CI of | 0.49 | nd | 0.36 | 0.89 | nd | 0.85 | 0.50 | >0.18 | 0.39 |
| OR Quart3 | 14 | nd | 11 | 21 | nd | 13 | 8.7 | na | 7.4 |
| OR Quart 4 | 2.6 | nd | 2.0 | 6.2 | nd | 3.7 | 2.4 | >3.1 | 1.7 |
| p Value | 0.27 | nd | 0.42 | 0.021 | nd | 0.054 | 0.21 | <0.34 | 0.48 |
| 95% CI of | 0.48 | nd | 0.36 | 1.3 | nd | 0.98 | 0.61 | >0.31 | 0.39 |
| OR Quart4 | 14 | nd | 11 | 29 | nd | 14 | 9.8 | na | 7.4 |

| | | | | | | | | | |
|---|---|---|---|---|---|---|---|---|---|
| **C-C motif chemokine 24** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 259 | | 392 | 259 | | 198 | 259 | | 323 |

| sCr or UO | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Average | 397 | | 544 | 397 | | 325 | 397 | | 429 |
| Stdev | 402 | | 578 | 402 | | 311 | 402 | | 404 |
| p(t-test) | | | 0.17 | | | 0.36 | | | 0.75 |
| Min | 0.0260 | | 40.3 | 0.0260 | | 13.1 | 0.0260 | | 12.4 |
| Max | 2920 | | 2430 | 2920 | | 1100 | 2920 | | 1380 |
| n (Samp) | 216 | | 16 | 216 | | 28 | 216 | | 17 |
| n (Patient) | 132 | | 16 | 132 | | 28 | 132 | | 17 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 270 | | 238 | 270 | | 173 | 270 | | 323 |
| Average | 409 | | 394 | 409 | | 345 | 409 | | 471 |
| Stdev | 412 | | 311 | 412 | | 478 | 412 | | 461 |
| p(t-test) | | | 0.90 | | | 0.45 | | | 0.58 |
| Min | 2.15 | | 40.3 | 2.15 | | 13.1 | 2.15 | | 12.4 |
| Max | 2920 | | 980 | 2920 | | 2430 | 2920 | | 1380 |
| n (Samp) | 198 | | 13 | 198 | | 29 | 198 | | 15 |
| n (Patient) | 117 | | 13 | 117 | | 29 | 117 | | 15 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.58 | nd | 0.51 | 0.43 | nd | 0.39 | 0.51 | nd | 0.50 |
| SE | 0.077 | nd | 0.083 | 0.059 | nd | 0.059 | 0.073 | nd | 0.078 |
| p | 0.29 | nd | 0.93 | 0.23 | nd | 0.064 | 0.89 | nd | 0.99 |
| nCohort 1 | 216 | nd | 198 | 216 | nd | 198 | 216 | nd | 198 |
| nCohort 2 | 16 | nd | 13 | 28 | nd | 29 | 17 | nd | 15 |
| Cutoff 1 | 201 | nd | 173 | 85.2 | nd | 80.9 | 181 | nd | 96.1 |
| Sens 1 | 75% | nd | 77% | 71% | nd | 72% | 71% | nd | 73% |
| Spec 1 | 38% | nd | 29% | 12% | nd | 11% | 33% | nd | 15% |
| Cutoff 2 | 171 | nd | 154 | 69.9 | nd | 67.2 | 85.2 | nd | 85.2 |
| Sens 2 | 81% | nd | 85% | 82% | nd | 83% | 82% | nd | 80% |
| Spec 2 | 31% | nd | 26% | 10% | nd | 9% | 12% | nd | 12% |
| Cutoff 3 | 67.2 | nd | 67.2 | 33.2 | nd | 25.7 | 25.7 | nd | 25.7 |
| Sens 3 | 94% | nd | 92% | 93% | nd | 93% | 94% | nd | 93% |
| Spec 3 | 10% | nd | 9% | 2% | nd | 2% | 2% | nd | 2% |
| Cutoff 4 | 438 | nd | 440 | 438 | nd | 440 | 438 | nd | 440 |
| Sens 4 | 50% | nd | 38% | 32% | nd | 28% | 41% | nd | 40% |
| Spec 4 | 70% | nd | 70% | 70% | nd | 70% | 70% | nd | 70% |
| Cutoff 5 | 580 | nd | 625 | 580 | nd | 625 | 580 | nd | 625 |
| Sens 5 | 44% | nd | 31% | 21% | nd | 14% | 24% | nd | 33% |
| Spec 5 | 80% | nd | 80% | 80% | nd | 80% | 80% | nd | 80% |
| Cutoff 6 | 881 | nd | 940 | 881 | nd | 940 | 881 | nd | 940 |
| Sens 6 | 12% | nd | 8% | 11% | nd | 3% | 18% | nd | 27% |
| Spec 6 | 90% | nd | 90% | 90% | nd | 90% | 90% | nd | 90% |
| OR Quart 2 | 2.6 | nd | 2.6 | 1.0 | nd | 1.2 | 0.38 | nd | 0.31 |
| p Value | 0.26 | nd | 0.27 | 1.0 | nd | 0.75 | 0.26 | nd | 0.17 |
| 95% CI of | 0.49 | nd | 0.48 | 0.30 | nd | 0.35 | 0.070 | nd | 0.060 |
| OR Quart2 | 14 | nd | 14 | 3.3 | nd | 4.3 | 2.0 | nd | 1.6 |
| OR Quart 3 | 1.0 | nd | 0.48 | 0.82 | nd | 1.2 | 0.79 | nd | 0.31 |
| p Value | 1.0 | nd | 0.55 | 0.75 | nd | 0.75 | 0.73 | nd | 0.17 |
| 95% CI of | 0.14 | nd | 0.042 | 0.24 | nd | 0.35 | 0.20 | nd | 0.060 |
| OR Quart3 | 7.3 | nd | 5.5 | 2.8 | nd | 4.3 | 3.1 | nd | 1.6 |
| OR Quart 4 | 3.8 | nd | 2.6 | 2.0 | nd | 2.8 | 1.2 | nd | 0.83 |
| p Value | 0.10 | nd | 0.27 | 0.20 | nd | 0.068 | 0.77 | nd | 0.78 |
| 95% CI of | 0.76 | nd | 0.48 | 0.69 | nd | 0.93 | 0.35 | nd | 0.24 |
| OR Quart4 | 19 | nd | 14 | 5.9 | nd | 8.7 | 4.2 | nd | 2.9 |

| | | | | | | | | | |
|---|---|---|---|---|---|---|---|---|---|
| **C-C motif chemokine 8** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 12.7 | | 14.8 | 12.7 | | 13.7 | 12.7 | | 17.5 |
| Average | 18.3 | | 18.6 | 18.3 | | 17.2 | 18.3 | | 24.4 |
| Stdev | 26.5 | | 10.7 | 26.5 | | 18.2 | 26.5 | | 17.8 |
| p(t-test) | | | 0.97 | | | 0.83 | | | 0.36 |
| Min | 0.162 | | 6.68 | 0.162 | | 1.20 | 0.162 | | 9.54 |
| Max | 180 | | 41.0 | 180 | | 101 | 180 | | 72.1 |
| n (Samp) | 216 | | 16 | 216 | | 28 | 216 | | 17 |
| n (Patient) | 132 | | 16 | 132 | | 28 | 132 | | 17 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 12.3 | | 14.5 | 12.3 | | 13.8 | 12.3 | | 16.3 |
| Average | 19.1 | | 16.6 | 19.1 | | 16.6 | 19.1 | | 23.8 |
| Stdev | 28.1 | | 9.85 | 28.1 | | 17.6 | 28.1 | | 18.9 |
| p(t-test) | | | 0.74 | | | 0.63 | | | 0.53 |
| Min | 0.162 | | 6.68 | 0.162 | | 1.20 | 0.162 | | 9.54 |
| Max | 180 | | 41.0 | 180 | | 101 | 180 | | 72.1 |
| n (Samp) | 198 | | 13 | 198 | | 29 | 198 | | 15 |
| n (Patient) | 117 | | 13 | 117 | | 29 | 117 | | 15 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.61 | nd | 0.58 | 0.53 | nd | 0.53 | 0.70 | nd | 0.68 |
| SE | 0.077 | nd | 0.085 | 0.059 | nd | 0.058 | 0.073 | nd | 0.078 |
| p | 0.14 | nd | 0.37 | 0.57 | nd | 0.56 | 0.0050 | nd | 0.021 |
| nCohort 1 | 216 | nd | 198 | 216 | nd | 198 | 216 | nd | 198 |
| nCohort 2 | 16 | nd | 13 | 28 | nd | 29 | 17 | nd | 15 |
| Cutoff 1 | 12.8 | nd | 9.69 | 10.7 | nd | 10.7 | 15.4 | nd | 13.7 |
| Sens 1 | 75% | nd | 85% | 71% | nd | 72% | 71% | nd | 73% |
| Spec 1 | 50% | nd | 35% | 39% | nd | 42% | 62% | nd | 56% |
| Cutoff 2 | 9.69 | nd | 9.69 | 7.48 | nd | 7.48 | 13.0 | nd | 13.0 |
| Sens 2 | 88% | nd | 85% | 82% | nd | 83% | 82% | nd | 80% |
| Spec 2 | 33% | nd | 35% | 24% | nd | 25% | 52% | nd | 54% |
| Cutoff 3 | 8.22 | nd | 8.22 | 4.81 | nd | 4.81 | 11.3 | nd | 11.3 |
| Sens 3 | 94% | nd | 92% | 93% | nd | 93% | 94% | nd | 93% |
| Spec 3 | 28% | nd | 29% | 18% | nd | 19% | 41% | nd | 44% |
| Cutoff 4 | 17.1 | nd | 17.0 | 17.1 | nd | 17.0 | 17.1 | nd | 17.0 |
| Sens 4 | 31% | nd | 23% | 25% | nd | 21% | 53% | nd | 40% |
| Spec 4 | 70% | nd | 70% | 70% | nd | 70% | 70% | nd | 70% |
| Cutoff 5 | 20.1 | nd | 20.3 | 20.1 | nd | 20.3 | 20.1 | nd | 20.3 |
| Sens 5 | 25% | nd | 15% | 21% | nd | 17% | 29% | nd | 27% |
| Spec 5 | 80% | nd | 80% | 80% | nd | 80% | 80% | nd | 80% |
| Cutoff 6 | 29.8 | nd | 33.4 | 29.8 | nd | 33.4 | 29.8 | nd | 33.4 |
| Sens 6 | 19% | nd | 8% | 11% | nd | 3% | 24% | nd | 20% |
| Spec 6 | 90% | nd | 91% | 90% | nd | 91% | 90% | nd | 91% |
| OR Quart 2 | 4.2 | nd | 3.1 | 0.82 | nd | 0.80 | >3.2 | nd | >2.1 |
| p Value | 0.20 | nd | 0.34 | 0.75 | nd | 0.73 | <0.32 | nd | <0.56 |
| 95% CI of | 0.46 | nd | 0.31 | 0.24 | nd | 0.23 | >0.32 | nd | >0.18 |
| OR Quart2 | 39 | nd | 30 | 2.8 | nd | 2.8 | na | nd | na |
| OR Quart 3 | 7.8 | nd | 7.8 | 1.8 | nd | 2.2 | >9.3 | nd | >9.4 |
| p Value | 0.058 | nd | 0.060 | 0.29 | nd | 0.14 | <0.039 | nd | <0.038 |
| 95% CI of | 0.93 | nd | 0.92 | 0.61 | nd | 0.77 | >1.1 | nd | >1.1 |
| OR Quart3 | 66 | nd | 65 | 5.3 | nd | 6.4 | na | nd | na |
| OR Quart 4 | 4.2 | nd | 2.0 | 1.2 | nd | 0.98 | >6.6 | nd | >5.4 |
| p Value | 0.20 | nd | 0.58 | 0.77 | nd | 0.97 | <0.086 | nd | <0.13 |
| 95% CI of | 0.46 | nd | 0.18 | 0.38 | nd | 0.30 | >0.77 | nd | >0.61 |
| OR Quart4 | 39 | nd | 23 | 3.8 | nd | 3.2 | na | nd | na |

| | | | | | | | | | |
|---|---|---|---|---|---|---|---|---|---|
| **Cathepsin D** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 229000 | | 205000 | 229000 | | 234000 | 229000 | | 249000 |
| Average | 257000 | | 294000 | 257000 | | 292000 | 257000 | | 281000 |
| Stdev | 147000 | | 295000 | 147000 | | 170000 | 147000 | | 135000 |
| p(t-test) | | | 0.36 | | | 0.24 | | | 0.47 |
| Min | 34100 | | 127000 | 34100 | | 90500 | 34100 | | 83000 |
| Max | 1020000 | | 1350000 | 1020000 | | 655000 | 1020000 | | 561000 |
| n (Samp) | 281 | | 16 | 281 | | 28 | 281 | | 20 |
| n (Patient) | 159 | | 16 | 159 | | 28 | 159 | | 20 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | nd | | nd | nd | | nd | 227000 | | 309000 |
| Average | nd | | nd | nd | | nd | 260000 | | 329000 |
| Stdev | nd | | nd | nd | | nd | 166000 | | 197000 |
| p(t-test) | nd | | nd | nd | | nd | | | 0.32 |
| Min | nd | | nd | nd | | nd | 34100 | | 131000 |
| Max | nd | | nd | nd | | nd | 1870000 | | 561000 |
| n (Samp) | nd | | nd | nd | | nd | 352 | | 6 |
| n (Patient) | nd | | nd | nd | | nd | 192 | | 6 |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 236000 | | 218000 | 236000 | | 267000 | 236000 | | 262000 |
| Average | 266000 | | 237000 | 266000 | | 332000 | 266000 | | 310000 |
| Stdev | 148000 | | 92200 | 148000 | | 261000 | 148000 | | 146000 |
| p(t-test) | | | 0.49 | | | 0.046 | | | 0.24 |
| Min | 54000 | | 130000 | 54000 | | 90500 | 54000 | | 83000 |
| Max | 1020000 | | 411000 | 1020000 | | 1350000 | 1020000 | | 655000 |
| n (Samp) | 257 | | 13 | 257 | | 26 | 257 | | 17 |
| n (Patient) | 139 | | 13 | 139 | | 26 | 139 | | 17 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.49 | nd | 0.48 | 0.55 | nd | 0.56 | 0.57 | 0.60 | 0.61 |
| SE | 0.075 | nd | 0.083 | 0.058 | nd | 0.061 | 0.069 | 0.12 | 0.075 |
| p | 0.92 | nd | 0.76 | 0.42 | nd | 0.36 | 0.29 | 0.42 | 0.15 |
| nCohort 1 | 281 | nd | 257 | 281 | nd | 257 | 281 | 352 | 257 |
| nCohort 2 | 16 | nd | 13 | 28 | nd | 26 | 20 | 6 | 17 |
| Cutoff 1 | 159000 | nd | 167000 | 148000 | nd | 154000 | 212000 | 157000 | 244000 |
| Sens 1 | 75% | nd | 77% | 71% | nd | 73% | 70% | 83% | 71% |
| Spec 1 | 30% | nd | 32% | 26% | nd | 25% | 44% | 29% | 54% |
| Cutoff 2 | 143000 | nd | 159000 | 141000 | nd | 142000 | 169000 | 157000 | 212000 |
| Sens 2 | 81% | nd | 85% | 82% | nd | 81% | 80% | 83% | 82% |
| Spec 2 | 24% | nd | 28% | 22% | nd | 20% | 34% | 29% | 42% |
| Cutoff 3 | 129000 | nd | 137000 | 126000 | nd | 126000 | 157000 | 130000 | 166000 |
| Sens 3 | 94% | nd | 92% | 93% | nd | 92% | 90% | 100% | 94% |
| Spec 3 | 18% | nd | 19% | 16% | nd | 13% | 30% | 17% | 31% |
| Cutoff 4 | 305000 | nd | 310000 | 305000 | nd | 310000 | 305000 | 306000 | 310000 |
| Sens 4 | 31% | nd | 23% | 43% | nd | 38% | 35% | 50% | 35% |
| Spec 4 | 70% | nd | 70% | 70% | nd | 70% | 70% | 70% | 70% |
| Cutoff 5 | 357000 | nd | 374000 | 357000 | nd | 374000 | 357000 | 352000 | 374000 |
| Sens 5 | 19% | nd | 8% | 32% | nd | 35% | 25% | 50% | 29% |
| Spec 5 | 80% | nd | 80% | 80% | nd | 80% | 80% | 80% | 80% |
| Cutoff 6 | 450000 | nd | 469000 | 450000 | nd | 469000 | 450000 | 450000 | 469000 |
| Sens 6 | 6% | nd | 0% | 21% | nd | 19% | 15% | 33% | 18% |
| Spec 6 | 90% | nd | 90% | 90% | nd | 90% | 90% | 90% | 90% |
| OR Quart 2 | 0.49 | nd | 0.67 | 0.60 | nd | 0.68 | 3.2 | 2.0 | 4.1 |
| p Value | 0.42 | nd | 0.66 | 0.39 | nd | 0.53 | 0.17 | 0.57 | 0.21 |
| 95% CI of | 0.088 | nd | 0.11 | 0.19 | nd | 0.21 | 0.62 | 0.18 | 0.45 |
| OR Quart2 | 2.8 | nd | 4.1 | 1.9 | nd | 2.3 | 16 | 22 | 38 |
| OR Quart 3 | 1.6 | nd | 2.1 | 0.73 | nd | 0.68 | 3.2 | 0 | 7.7 |
| p Value | 0.50 | nd | 0.31 | 0.58 | nd | 0.53 | 0.17 | na | 0.060 |
| 95% CI of | 0.42 | nd | 0.50 | 0.24 | nd | 0.21 | 0.62 | na | 0.92 |
| OR Quart3 | 5.8 | nd | 8.8 | 2.2 | nd | 2.3 | 16 | na | 64 |
| OR Quart 4 | 1.0 | nd | 0.67 | 1.1 | nd | 1.3 | 3.1 | 3.0 | 5.2 |
| p Value | 0.98 | nd | 0.66 | 0.82 | nd | 0.62 | 0.17 | 0.34 | 0.14 |
| 95% CI of | 0.24 | nd | 0.11 | 0.41 | nd | 0.46 | 0.61 | 0.31 | 0.60 |
| OR Quart4 | 4.2 | nd | 4.1 | 3.1 | nd | 3.7 | 16 | 30 | 46 |

| **C-X-C motif chemokine 13** | | | | | | | | | |
|---|---|---|---|---|---|---|---|---|---|
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 47.3 | | 51.3 | 47.3 | | 50.8 | 47.3 | | 47.6 |
| Average | 124 | | 177 | 124 | | 105 | 124 | | 180 |
| Stdev | 237 | | 487 | 237 | | 245 | 237 | | 278 |
| p(t-test) | | | 0.44 | | | 0.69 | | | 0.36 |
| Min | 9.90 | | 15.3 | 9.90 | | 12.9 | 9.90 | | 12.5 |
| Max | 1790 | | 2000 | 1790 | | 1340 | 1790 | | 918 |
| n (Samp) | 216 | | 16 | 216 | | 28 | 216 | | 17 |
| n (Patient) | 132 | | 16 | 132 | | 28 | 132 | | 17 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 52.2 | | 49.8 | 52.2 | | 53.6 | 52.2 | | 50.0 |
| Average | 123 | | 54.6 | 123 | | 103 | 123 | | 159 |
| Stdev | 218 | | 26.9 | 218 | | 241 | 218 | | 247 |
| p(t-test) | | | 0.26 | | | 0.64 | | | 0.55 |
| Min | 9.90 | | 15.3 | 9.90 | | 12.9 | 9.90 | | 12.5 |
| Max | 1790 | | 109 | 1790 | | 1340 | 1790 | | 918 |
| n (Samp) | 198 | | 13 | 198 | | 29 | 198 | | 15 |
| n (Patient) | 117 | | 13 | 117 | | 29 | 117 | | 15 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.49 | nd | 0.44 | 0.50 | nd | 0.48 | 0.51 | nd | 0.51 |
| SE | 0.075 | nd | 0.085 | 0.058 | nd | 0.058 | 0.073 | nd | 0.078 |
| p | 0.94 | nd | 0.51 | 0.94 | nd | 0.69 | 0.89 | nd | 0.85 |
| nCohort 1 | 216 | nd | 198 | 216 | nd | 198 | 216 | nd | 198 |
| nCohort 2 | 16 | nd | 13 | 28 | nd | 29 | 17 | nd | 15 |
| Cutoff 1 | 35.2 | nd | 35.2 | 39.1 | nd | 39.1 | 34.2 | nd | 34.2 |
| Sens 1 | 75% | nd | 77% | 71% | nd | 72% | 71% | nd | 73% |
| Spec 1 | 37% | nd | 34% | 42% | nd | 40% | 36% | nd | 33% |
| Cutoff 2 | 34.2 | nd | 34.2 | 29.3 | nd | 29.3 | 27.8 | nd | 27.8 |
| Sens 2 | 81% | nd | 85% | 82% | nd | 83% | 82% | nd | 80% |
| Spec 2 | 36% | nd | 33% | 30% | nd | 27% | 25% | nd | 23% |
| Cutoff 3 | 25.2 | nd | 24.9 | 23.5 | nd | 23.5 | 15.4 | nd | 15.4 |
| Sens 3 | 94% | nd | 92% | 93% | nd | 93% | 94% | nd | 93% |
| Spec 3 | 22% | nd | 19% | 20% | nd | 18% | 6% | nd | 6% |
| Cutoff 4 | 102 | nd | 106 | 102 | nd | 106 | 102 | nd | 106 |
| Sens 4 | 12% | nd | 8% | 14% | nd | 10% | 29% | nd | 33% |
| Spec 4 | 70% | nd | 70% | 70% | nd | 70% | 70% | nd | 70% |
| Cutoff 5 | 140 | nd | 141 | 140 | nd | 141 | 140 | nd | 141 |
| Sens 5 | 6% | nd | 0% | 7% | nd | 3% | 29% | nd | 33% |
| Spec 5 | 80% | nd | 80% | 80% | nd | 80% | 80% | nd | 80% |
| Cutoff 6 | 251 | nd | 254 | 251 | nd | 254 | 251 | nd | 254 |
| Sens 6 | 6% | nd | 0% | 4% | nd | 3% | 24% | nd | 20% |
| Spec 6 | 90% | nd | 90% | 90% | nd | 90% | 90% | nd | 90% |
| OR Quart 2 | 9.1 | nd | >6.8 | 3.1 | nd | 4.8 | 1.7 | nd | 1.0 |
| p Value | 0.040 | nd | <0.082 | 0.063 | nd | 0.020 | 0.47 | nd | 1.0 |
| 95% CI of | 1.1 | nd | >0.79 | 0.94 | nd | 1.3 | 0.39 | nd | 0.24 |
| OR Quart2 | 75 | nd | na | 10 | nd | 18 | 7.6 | nd | 4.2 |
| OR Quart 3 | 5.4 | nd | >5.5 | 2.5 | nd | 3.4 | 1.4 | nd | 0.48 |
| p Value | 0.13 | nd | <0.12 | 0.15 | nd | 0.080 | 0.70 | nd | 0.41 |
| 95% CI of | 0.61 | nd | >0.62 | 0.72 | nd | 0.86 | 0.29 | nd | 0.084 |
| OR Quart3 | 48 | nd | na | 8.5 | nd | 13 | 6.4 | nd | 2.7 |
| OR Quart 4 | 2.0 | nd | >2.1 | 1.0 | nd | 1.8 | 1.7 | nd | 1.2 |
| p Value | 0.57 | nd | <0.54 | 1.0 | nd | 0.45 | 0.48 | nd | 0.75 |
| 95% CI of | 0.18 | nd | >0.19 | 0.24 | nd | 0.40 | 0.39 | nd | 0.32 |
| OR Quart4 | 23 | nd | na | 4.2 | nd | 7.8 | 7.5 | nd | 4.9 |

| | | | | | | | | | |
|---|---|---|---|---|---|---|---|---|---|
| **Insulin-like growth factor-binding protein 3** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 3220 | | 2570 | 3220 | | 3020 | 3220 | | 2890 |
| Average | 3530 | | 3330 | 3530 | | 3480 | 3530 | | 3450 |
| Stdev | 1570 | | 1940 | 1570 | | 1530 | 1570 | | 2040 |
| p(t-test) | | | 0.63 | | | 0.87 | | | 0.83 |
| Min | 823 | | 698 | 823 | | 651 | 823 | | 730 |
| Max | 8600 | | 6560 | 8600 | | 7360 | 8600 | | 7520 |
| n (Samp) | 282 | | 16 | 282 | | 28 | 282 | | 20 |
| n (Patient) | 160 | | 16 | 160 | | 28 | 160 | | 20 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | nd | | nd | nd | | nd | 3090 | | 3030 |
| Average | nd | | nd | nd | | nd | 3480 | | 3870 |
| Stdev | nd | | nd | nd | | nd | 1600 | | 1920 |
| p(t-test) | nd | | nd | nd | | nd | | | 0.55 |
| Min | nd | | nd | nd | | nd | 651 | | 2160 |
| Max | nd | | nd | nd | | nd | 8600 | | 6560 |
| n (Samp) | nd | | nd | nd | | nd | 353 | | 6 |
| n (Patient) | nd | | nd | nd | | nd | 193 | | 6 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 3060 | | 2280 | 3060 | | 3020 | 3060 | | 3020 |
| Average | 3410 | | 2650 | 3410 | | 3540 | 3410 | | 3240 |
| Stdev | 1460 | | 1430 | 1460 | | 1610 | 1460 | | 1950 |
| p(t-test) | | | 0.071 | | | 0.66 | | | 0.66 |
| Min | 823 | | 698 | 823 | | 651 | 823 | | 730 |
| Max | 8260 | | 6250 | 8260 | | 7360 | 8260 | | 7520 |
| n (Samp) | 258 | | 13 | 258 | | 26 | 258 | | 17 |
| n (Patient) | 140 | | 13 | 140 | | 26 | 140 | | 17 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.44 | nd | 0.34 | 0.50 | nd | 0.53 | 0.46 | 0.56 | 0.44 |
| SE | 0.076 | nd | 0.084 | 0.057 | nd | 0.060 | 0.068 | 0.12 | 0.074 |
| p | 0.43 | nd | 0.052 | 0.99 | nd | 0.67 | 0.57 | 0.62 | 0.45 |
| nCohort 1 | 282 | nd | 258 | 282 | nd | 258 | 282 | 353 | 258 |
| nCohort 2 | 16 | nd | 13 | 28 | nd | 26 | 20 | 6 | 17 |
| Cutoff 1 | 2130 | nd | 1950 | 2410 | nd | 2410 | 2370 | 2430 | 2040 |
| Sens 1 | 75% | nd | 77% | 71% | nd | 73% | 70% | 83% | 71% |
| Spec 1 | 21% | nd | 15% | 28% | nd | 29% | 27% | 31% | 19% |
| Cutoff 2 | 1950 | nd | 1610 | 2220 | nd | 2260 | 1880 | 2430 | 1590 |
| Sens 2 | 81% | nd | 85% | 82% | nd | 81% | 80% | 83% | 82% |
| Spec 2 | 15% | nd | 7% | 23% | nd | 24% | 12% | 31% | 7% |
| Cutoff 3 | 1210 | nd | 1210 | 2130 | nd | 2130 | 1490 | 2150 | 730 |
| Sens 3 | 94% | nd | 92% | 93% | nd | 92% | 90% | 100% | 94% |
| Spec 3 | 2% | nd | 3% | 21% | nd | 22% | 6% | 22% | 0% |
| Cutoff 4 | 4250 | nd | 4130 | 4250 | nd | 4130 | 4250 | 4230 | 4130 |
| Sens 4 | 25% | nd | 8% | 36% | nd | 38% | 30% | 33% | 29% |
| Spec 4 | 70% | nd | 70% | 70% | nd | 70% | 70% | 70% | 70% |
| Cutoff 5 | 4870 | nd | 4680 | 4870 | nd | 4680 | 4870 | 4800 | 4680 |
| Sens 5 | 25% | nd | 8% | 14% | nd | 27% | 20% | 33% | 18% |
| Spec 5 | 80% | nd | 80% | 80% | nd | 80% | 80% | 80% | 80% |
| Cutoff 6 | 5740 | nd | 5510 | 5740 | nd | 5510 | 5740 | 5740 | 5510 |
| Sens 6 | 25% | nd | 8% | 7% | nd | 8% | 20% | 33% | 12% |
| Spec 6 | 90% | nd | 90% | 90% | nd | 90% | 90% | 90% | 90% |
| OR Quart 2 | 0.75 | nd | 3.1 | 0.86 | nd | 1.2 | 0.59 | 2.0 | 1.7 |
| p Value | 0.71 | nd | 0.33 | 0.79 | nd | 0.77 | 0.48 | 0.57 | 0.47 |
| 95% CI of | 0.16 | nd | 0.31 | 0.27 | nd | 0.38 | 0.14 | 0.18 | 0.39 |
| OR Quart2 | 3.5 | nd | 30 | 2.7 | nd | 3.7 | 2.6 | 22 | 7.5 |
| OR Quart 3 | 0.49 | nd | 3.1 | 1.2 | nd | 0.65 | 1.2 | 0.99 | 1.0 |
| p Value | 0.41 | nd | 0.33 | 0.79 | nd | 0.51 | 0.75 | 0.99 | 1.0 |
| 95% CI of | 0.086 | nd | 0.31 | 0.40 | nd | 0.17 | 0.36 | 0.061 | 0.19 |
| OR Quart3 | 2.7 | nd | 30 | 3.4 | nd | 2.4 | 4.2 | 16 | 5.1 |
| OR Quart 4 | 1.9 | nd | 6.6 | 1.0 | nd | 1.6 | 1.2 | 2.0 | 2.1 |
| p Value | 0.34 | nd | 0.085 | 0.98 | nd | 0.42 | 0.74 | 0.57 | 0.30 |
| 95% CI of | 0.52 | nd | 0.77 | 0.34 | nd | 0.53 | 0.36 | 0.18 | 0.51 |
| OR Quart4 | 6.6 | nd | 56 | 3.0 | nd | 4.7 | 4.2 | 22 | 8.9 |

| | | | | | | | | | |
|---|---|---|---|---|---|---|---|---|---|
| **Immunoglogulin G1** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 8800000 | | 7170000 | 8800000 | | 1.04E7 | 8800000 | | 9000000 |
| Average | 1.04E7 | | 7280000 | 1.04E7 | | 1.29E7 | 1.04E7 | | 9890000 |
| Stdev | 5920000 | | 1700000 | 5920000 | | 7430000 | 5920000 | | 4770000 |
| p(t-test) | | | 0.12 | | | 0.076 | | | 0.82 |
| Min | 2060000 | | 4390000 | 2060000 | | 4620000 | 2060000 | | 4390000 |
| Max | 4.18E7 | | 9590000 | 4.18E7 | | 2.98E7 | 4.18E7 | | 2.05E7 |
| n (Samp) | 205 | | 9 | 205 | | 20 | 205 | | 8 |
| n (Patient) | 127 | | 9 | 127 | | 20 | 127 | | 8 |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 8690000 | | 7170000 | 8690000 | | 1.01E7 | 8690000 | | 8470000 |
| Average | 1.01E7 | | 7280000 | 1.01E7 | | 1.21E7 | 1.01E7 | | 9950000 |
| Stdev | 5930000 | | 1700000 | 5930000 | | 6630000 | 5930000 | | 5150000 |
| p(t-test) | | | 0.15 | | | 0.19 | | | 0.94 |
| Min | 2060000 | | 4390000 | 2060000 | | 4620000 | 2060000 | | 4390000 |
| Max | 4.18E7 | | 9590000 | 4.18E7 | | 2.63E7 | 4.18E7 | | 2.05E7 |
| n (Samp) | 191 | | 9 | 191 | | 18 | 191 | | 7 |
| n (Patient) | 113 | | 9 | 113 | | 18 | 113 | | 7 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.34 | nd | 0.36 | 0.61 | nd | 0.60 | 0.51 | nd | 0.52 |
| SE | 0.10 | nd | 0.10 | 0.070 | nd | 0.073 | 0.10 | nd | 0.11 |
| p | 0.11 | nd | 0.17 | 0.11 | nd | 0.15 | 0.94 | nd | 0.89 |
| nCohort 1 | 205 | nd | 191 | 205 | nd | 191 | 205 | nd | 191 |
| nCohort 2 | 9 | nd | 9 | 20 | nd | 18 | 8 | nd | 7 |
| Cutoff 1 | 6940000 | nd | 6940000 | 7810000 | nd | 7810000 | 8010000 | nd | 8010000 |
| Sens 1 | 78% | nd | 78% | 75% | nd | 72% | 75% | nd | 71% |
| Spec 1 | 28% | nd | 31% | 40% | nd | 43% | 44% | nd | 47% |
| Cutoff 2 | 5340000 | nd | 5340000 | 7160000 | nd | 7140000 | 6940000 | nd | 6940000 |
| Sens 2 | 89% | nd | 89% | 80% | nd | 83% | 88% | nd | 86% |
| Spec 2 | 13% | nd | 14% | 34% | nd | 34% | 28% | nd | 31% |
| Cutoff 3 | 4360000 | nd | 4360000 | 6940000 | nd | 6940000 | 4360000 | nd | 4360000 |
| Sens 3 | 100% | nd | 100% | 95% | nd | 94% | 100% | nd | 100% |
| Spec 3 | 5% | nd | 6% | 28% | nd | 31% | 5% | nd | 6% |
| Cutoff 4 | 1.13E7 | nd | 1.11E7 | 1.13E7 | nd | 1.11E7 | 1.13E7 | nd | 1.11E7 |
| Sens 4 | 0% | nd | 0% | 40% | nd | 39% | 25% | nd | 29% |
| Spec 4 | 71% | nd | 70% | 71% | nd | 70% | 71% | nd | 70% |
| Cutoff 5 | 1.38E7 | nd | 1.33E7 | 1.38E7 | nd | 1.33E7 | 1.38E7 | nd | 1.33E7 |
| Sens 5 | 0% | nd | 0% | 25% | nd | 22% | 12% | nd | 14% |
| Spec 5 | 80% | nd | 80% | 80% | nd | 80% | 80% | nd | 80% |
| Cutoff 6 | 1.71E7 | nd | 1.63E7 | 1.71E7 | nd | 1.63E7 | 1.71E7 | nd | 1.63E7 |
| Sens 6 | 0% | nd | 0% | 20% | nd | 22% | 12% | nd | 14% |
| Spec 6 | 90% | nd | 91% | 90% | nd | 91% | 90% | nd | 91% |
| OR Quart 2 | >2.1 | nd | >2.1 | 6.6 | nd | 6.7 | 3.1 | nd | 3.1 |
| p Value | <0.55 | nd | <0.55 | 0.086 | nd | 0.085 | 0.33 | nd | 0.34 |
| 95% CI of | >0.19 | nd | >0.18 | 0.77 | nd | 0.77 | 0.31 | nd | 0.31 |
| OR Quart2 | na | nd | na | 57 | nd | 57 | 31 | nd | 31 |
| OR Quart 3 | >5.5 | nd | >5.6 | 9.2 | nd | 6.7 | 3.1 | nd | 2.0 |
| p Value | <0.13 | nd | <0.12 | 0.040 | nd | 0.085 | 0.33 | nd | 0.57 |
| 95% CI of | >0.62 | nd | >0.63 | 1.1 | nd | 0.77 | 0.31 | nd | 0.18 |
| OR Quart3 | na | nd | na | 76 | nd | 57 | 31 | nd | 23 |
| OR Quart 4 | >2.1 | nd | >2.1 | 5.3 | nd | 5.3 | 0.98 | nd | 0.98 |
| p Value | <0.55 | nd | <0.55 | 0.13 | nd | 0.13 | 0.99 | nd | 0.99 |
| 95% CI of | >0.19 | nd | >0.18 | 0.60 | nd | 0.60 | 0.060 | nd | 0.060 |
| OR Quart4 | na | nd | na | 47 | nd | 47 | 16 | nd | 16 |

| **Immunoglogulin G2** | | | | | | | | | |
|---|---|---|---|---|---|---|---|---|---|
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 1.08E7 | | 1.02E7 | 1.08E7 | | 1.02E7 | 1.08E7 | | 1.05E7 |
| Average | 1.37E7 | | 8150000 | 1.37E7 | | 1.05E7 | 1.37E7 | | 1.01E7 |
| Stdev | 9640000 | | 6300000 | 9640000 | | 5640000 | 9640000 | | 5710000 |
| p(t-test) | | | 0.090 | | | 0.15 | | | 0.30 |
| Min | 190000 | | 1750000 | 190000 | | 1750000 | 190000 | | 1750000 |
| Max | 5.80E7 | | 1.70E7 | 5.80E7 | | 2.70E7 | 5.80E7 | | 1.70E7 |
| n (Samp) | 205 | | 9 | 205 | | 20 | 205 | | 8 |
| n (Patient) | 127 | | 9 | 127 | | 20 | 127 | | 8 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 1.03E7 | | 1.02E7 | 1.03E7 | | 1.02E7 | 1.03E7 | | 1.02E7 |
| Average | 1.33E7 | | 8150000 | 1.33E7 | | 1.05E7 | 1.33E7 | | 1.00E7 |
| Stdev | 9390000 | | 6300000 | 9390000 | | 5580000 | 9390000 | | 6160000 |
| p(t-test) | | | 0.11 | | | 0.22 | | | 0.37 |
| Min | 190000 | | 1750000 | 190000 | | 1750000 | 190000 | | 1750000 |
| Max | 5.80E7 | | 1.70E7 | 5.80E7 | | 2.70E7 | 5.80E7 | | 1.70E7 |
| n (Samp) | 191 | | 9 | 191 | | 18 | 191 | | 7 |
| n (Patient) | 113 | | 9 | 113 | | 18 | 113 | | 7 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.34 | nd | 0.35 | 0.39 | nd | 0.40 | 0.41 | nd | 0.41 |
| SE | 0.10 | nd | 0.10 | 0.070 | nd | 0.073 | 0.11 | nd | 0.12 |
| p | 0.10 | nd | 0.13 | 0.11 | nd | 0.16 | 0.38 | nd | 0.42 |
| nCohort 1 | 205 | nd | 191 | 205 | nd | 191 | 205 | nd | 191 |
| nCohort 2 | 9 | nd | 9 | 20 | nd | 18 | 8 | nd | 7 |
| Cutoff 1 | 1930000 | nd | 1930000 | 8850000 | nd | 8850000 | 7850000 | nd | 7850000 |
| Sens 1 | 78% | nd | 78% | 70% | nd | 72% | 75% | nd | 71% |
| Spec 1 | 9% | nd | 9% | 27% | nd | 28% | 25% | nd | 27% |
| Cutoff 2 | 563000 | nd | 219000 | 6700000 | nd | 6700000 | 1930000 | nd | 1930000 |
| Sens 2 | 100% | nd | 100% | 80% | nd | 83% | 88% | nd | 86% |
| Spec 2 | 1% | nd | 1% | 20% | nd | 21% | 9% | nd | 9% |
| Cutoff 3 | 563000 | nd | 219000 | 4550000 | nd | 219000 | 563000 | nd | 219000 |
| Sens 3 | 100% | nd | 100% | 90% | nd | 100% | 100% | nd | 100% |
| Spec 3 | 1% | nd | 1% | 18% | nd | 1% | 1% | nd | 1% |
| Cutoff 4 | 1.64E7 | nd | 1.64E7 | 1.64E7 | nd | 1.64E7 | 1.64E7 | nd | 1.64E7 |
| Sens 4 | 22% | nd | 22% | 10% | nd | 6% | 12% | nd | 14% |
| Spec 4 | 71% | nd | 72% | 71% | nd | 72% | 71% | nd | 72% |
| Cutoff 5 | 1.85E7 | nd | 1.84E7 | 1.85E7 | nd | 1.84E7 | 1.85E7 | nd | 1.84E7 |
| Sens 5 | 0% | nd | 0% | 5% | nd | 6% | 0% | nd | 0% |
| Spec 5 | 80% | nd | 80% | 80% | nd | 80% | 80% | nd | 80% |
| Cutoff 6 | 2.55E7 | nd | 2.52E7 | 2.55E7 | nd | 2.52E7 | 2.55E7 | nd | 2.52E7 |
| Sens 6 | 0% | nd | 0% | 5% | nd | 6% | 0% | nd | 0% |
| Spec 6 | 90% | nd | 90% | 90% | nd | 90% | 90% | nd | 90% |
| OR Quart 2 | 1.0 | nd | 0 | 6.7 | nd | 6.8 | 3.2 | nd | 2.1 |
| p Value | 0.99 | nd | na | 0.083 | nd | 0.082 | 0.32 | nd | 0.55 |
| 95% CI of | 0.062 | nd | na | 0.78 | nd | 0.79 | 0.32 | nd | 0.18 |
| OR Quart2 | 17 | nd | na | 58 | nd | 58 | 32 | nd | 24 |
| OR Quart 3 | 3.1 | nd | 1.5 | 9.3 | nd | 8.1 | 2.1 | nd | 2.0 |
| p Value | 0.33 | nd | 0.65 | 0.038 | nd | 0.055 | 0.56 | nd | 0.57 |
| 95% CI of | 0.31 | nd | 0.24 | 1.1 | nd | 0.96 | 0.18 | nd | 0.18 |
| OR Quart3 | 31 | nd | 9.6 | 77 | nd | 68 | 24 | nd | 23 |
| OR Quart 4 | 4.3 | nd | 2.1 | 5.5 | nd | 4.3 | 2.1 | nd | 2.1 |
| p Value | 0.20 | nd | 0.41 | 0.13 | nd | 0.20 | 0.56 | nd | 0.55 |
| 95% CI of | 0.47 | nd | 0.36 | 0.62 | nd | 0.47 | 0.18 | nd | 0.18 |
| OR Quart4 | 40 | nd | 12 | 49 | nd | 40 | 24 | nd | 24 |

| | | | | | | | | | |
|---|---|---|---|---|---|---|---|---|---|
| **Interleukin-11** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 67.2 | | 37.8 | 67.2 | | 89.1 | 67.2 | | 51.1 |
| Average | 162 | | 185 | 162 | | 98.0 | 162 | | 109 |
| Stdev | 635 | | 477 | 635 | | 75.8 | 635 | | 142 |
| p(t-test) | | | 0.89 | | | 0.60 | | | 0.73 |
| Min | 0.359 | | 0.442 | 0.359 | | 0.480 | 0.359 | | 0.480 |
| Max | 6920 | | 1940 | 6920 | | 392 | 6920 | | 569 |
| n (Samp) | 217 | | 16 | 217 | | 28 | 217 | | 17 |
| n (Patient) | 133 | | 16 | 133 | | 28 | 133 | | 17 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 62.2 | | 33.9 | 62.2 | | 90.7 | 62.2 | | 55.3 |
| Average | 156 | | 47.3 | 156 | | 86.9 | 156 | | 88.3 |
| Stdev | 660 | | 52.1 | 660 | | 50.4 | 660 | | 80.0 |
| p(t-test) | | | 0.55 | | | 0.57 | | | 0.69 |
| Min | 0.359 | | 0.442 | 0.359 | | 0.480 | 0.359 | | 0.480 |
| Max | 6920 | | 176 | 6920 | | 198 | 6920 | | 233 |
| n (Samp) | 199 | | 13 | 199 | | 29 | 199 | | 15 |
| n (Patient) | 118 | | 13 | 118 | | 29 | 118 | | 15 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.43 | nd | 0.37 | 0.57 | nd | 0.57 | 0.51 | nd | 0.53 |
| SE | 0.077 | nd | 0.085 | 0.059 | nd | 0.059 | 0.073 | nd | 0.079 |
| p | 0.36 | nd | 0.14 | 0.24 | nd | 0.21 | 0.91 | nd | 0.70 |
| nCohort 1 | 217 | nd | 199 | 217 | nd | 199 | 217 | nd | 199 |
| nCohort 2 | 16 | nd | 13 | 28 | nd | 29 | 17 | nd | 15 |
| Cutoff 1 | 7.90 | nd | 6.00 | 55.3 | nd | 55.3 | 33.9 | nd | 33.9 |
| Sens 1 | 75% | nd | 77% | 71% | nd | 72% | 71% | nd | 80% |
| Spec 1 | 21% | nd | 18% | 46% | nd | 47% | 35% | nd | 36% |
| Cutoff 2 | 6.00 | nd | 0.608 | 47.0 | nd | 31.8 | 20.2 | nd | 33.9 |
| Sens 2 | 81% | nd | 85% | 82% | nd | 83% | 82% | nd | 80% |
| Spec 2 | 17% | nd | 12% | 44% | nd | 32% | 27% | nd | 36% |
| Cutoff 3 | 0.442 | nd | 0.442 | 15.1 | nd | 7.90 | 0.608 | nd | 6.07 |
| Sens 3 | 94% | nd | 92% | 93% | nd | 93% | 94% | nd | 93% |
| Spec 3 | 8% | nd | 8% | 23% | nd | 22% | 12% | nd | 21% |
| Cutoff 4 | 118 | nd | 113 | 118 | nd | 113 | 118 | nd | 113 |
| Sens 4 | 19% | nd | 15% | 25% | nd | 21% | 29% | nd | 27% |
| Spec 4 | 70% | nd | 70% | 70% | nd | 70% | 70% | nd | 70% |
| Cutoff 5 | 163 | nd | 158 | 163 | nd | 158 | 163 | nd | 158 |
| Sens 5 | 19% | nd | 8% | 14% | nd | 10% | 29% | nd | 27% |
| Spec 5 | 80% | nd | 82% | 80% | nd | 82% | 80% | nd | 82% |
| Cutoff 6 | 246 | nd | 209 | 246 | nd | 209 | 246 | nd | 209 |
| Sens 6 | 12% | nd | 0% | 4% | nd | 0% | 6% | nd | 13% |
| Spec 6 | 90% | nd | 90% | 90% | nd | 90% | 90% | nd | 90% |
| OR Quart 2 | 0.67 | nd | 2.0 | 2.1 | nd | 1.3 | 2.5 | nd | 3.2 |
| p Value | 0.66 | nd | 0.57 | 0.31 | nd | 0.73 | 0.21 | nd | 0.17 |
| 95% CI of | 0.11 | nd | 0.18 | 0.50 | nd | 0.32 | 0.61 | nd | 0.61 |
| OR Quart2 | 4.1 | nd | 23 | 8.9 | nd | 5.0 | 10 | nd | 17 |
| OR Quart 3 | 1.8 | nd | 6.6 | 5.2 | nd | 4.7 | 0.65 | nd | 1.5 |
| p Value | 0.45 | nd | 0.085 | 0.013 | nd | 0.0095 | 0.65 | nd | 0.65 |
| 95% CI of | 0.40 | nd | 0.77 | 1.4 | nd | 1.5 | 0.11 | nd | 0.25 |
| OR Quart3 | 7.7 | nd | 57 | 19 | nd | 15 | 4.1 | nd | 9.5 |
| OR Quart 4 | 2.2 | nd | 4.2 | 2.1 | nd | 1.3 | 1.7 | nd | 2.0 |
| p Value | 0.30 | nd | 0.20 | 0.32 | nd | 0.73 | 0.48 | nd | 0.42 |
| 95% CI of | 0.51 | nd | 0.46 | 0.49 | nd | 0.32 | 0.39 | nd | 0.36 |
| OR Quart4 | 9.1 | nd | 39 | 8.7 | nd | 5.0 | 7.5 | nd | 12 |

| | | | | | | | | | |
|---|---|---|---|---|---|---|---|---|---|
| **Interleukin-2 receptor alpha chain** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 18.3 | | 18.7 | 18.3 | | 25.1 | 18.3 | | 22.6 |
| Average | 59.5 | | 84.0 | 59.5 | | 65.2 | 59.5 | | 121 |
| Stdev | 126 | | 139 | 126 | | 140 | 126 | | 224 |
| p(t-test) | | | 0.46 | | | 0.82 | | | 0.072 |
| Min | 0.0290 | | 0.0290 | 0.0290 | | 0.0290 | 0.0290 | | 0.0461 |
| Max | 1040 | | 518 | 1040 | | 715 | 1040 | | 862 |
| n (Samp) | 217 | | 16 | 217 | | 28 | 217 | | 17 |
| n (Patient) | 133 | | 16 | 133 | | 28 | 133 | | 17 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 24.1 | | 23.9 | 24.1 | | 28.8 | 24.1 | | 30.0 |
| Average | 67.8 | | 95.2 | 67.8 | | 84.5 | 67.8 | | 135 |
| Stdev | 136 | | 152 | 136 | | 162 | 136 | | 236 |
| p(t-test) | | | 0.49 | | | 0.55 | | | 0.084 |
| Min | 0.0290 | | 0.0290 | 0.0290 | | 0.0290 | 0.0290 | | 0.0461 |
| Max | 1040 | | 518 | 1040 | | 715 | 1040 | | 862 |
| n (Samp) | 199 | | 13 | 199 | | 29 | 199 | | 15 |
| n (Patient) | 118 | | 13 | 118 | | 29 | 118 | | 15 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.57 | nd | 0.55 | 0.50 | nd | 0.52 | 0.56 | nd | 0.56 |
| SE | 0.077 | nd | 0.085 | 0.058 | nd | 0.058 | 0.075 | nd | 0.079 |
| p | 0.38 | nd | 0.52 | 0.99 | nd | 0.78 | 0.43 | nd | 0.47 |
| nCohort 1 | 217 | nd | 199 | 217 | nd | 199 | 217 | nd | 199 |
| nCohort 2 | 16 | nd | 13 | 28 | nd | 29 | 17 | nd | 15 |
| Cutoff 1 | 10.6 | nd | 11.0 | 0.0515 | nd | 6.51 | 3.62 | nd | 3.62 |
| Sens 1 | 75% | nd | 77% | 75% | nd | 72% | 71% | nd | 73% |
| Spec 1 | 42% | nd | 38% | 17% | nd | 34% | 32% | nd | 29% |
| Cutoff 2 | 6.86 | nd | 10.6 | 0.0429 | nd | 0.0359 | 0.0569 | nd | 0.0612 |
| Sens 2 | 81% | nd | 85% | 86% | nd | 86% | 88% | nd | 80% |
| Spec 2 | 39% | nd | 38% | 9% | nd | 9% | 23% | nd | 24% |
| Cutoff 3 | 1.56 | nd | 1.56 | 0.0290 | nd | 0.0290 | 0.0515 | nd | 0.0569 |
| Sens 3 | 94% | nd | 92% | 96% | nd | 97% | 94% | nd | 93% |
| Spec 3 | 30% | nd | 26% | 5% | nd | 6% | 17% | nd | 20% |
| Cutoff 4 | 48.3 | nd | 54.6 | 48.3 | nd | 54.6 | 48.3 | nd | 54.6 |
| Sens 4 | 31% | nd | 31% | 32% | nd | 38% | 35% | nd | 40% |
| Spec 4 | 70% | nd | 70% | 70% | nd | 70% | 70% | nd | 70% |
| Cutoff 5 | 88.9 | nd | 102 | 88.9 | nd | 102 | 88.9 | nd | 102 |
| Sens 5 | 31% | nd | 31% | 11% | nd | 14% | 29% | nd | 27% |
| Spec 5 | 80% | nd | 80% | 80% | nd | 80% | 80% | nd | 80% |
| Cutoff 6 | 148 | nd | 153 | 148 | nd | 153 | 148 | nd | 153 |
| Sens 6 | 19% | nd | 23% | 11% | nd | 14% | 24% | nd | 27% |
| Spec 6 | 90% | nd | 90% | 90% | nd | 90% | 90% | nd | 90% |
| OR Quart 2 | 7.8 | nd | 6.6 | 0.20 | nd | 0.59 | 0.98 | nd | 0.72 |
| p Value | 0.058 | nd | 0.085 | 0.043 | nd | 0.38 | 0.98 | nd | 0.68 |
| 95% CI of | 0.93 | nd | 0.77 | 0.040 | nd | 0.18 | 0.23 | nd | 0.15 |
| OR Quart2 | 66 | nd | 57 | 0.95 | nd | 1.9 | 4.1 | nd | 3.4 |
| OR Quart 3 | 3.1 | nd | 2.0 | 0.87 | nd | 1.1 | 1.0 | nd | 0.74 |
| p Value | 0.33 | nd | 0.57 | 0.79 | nd | 0.79 | 1.0 | nd | 0.70 |
| 95% CI of | 0.31 | nd | 0.18 | 0.31 | nd | 0.41 | 0.24 | nd | 0.16 |
| OR Quart3 | 31 | nd | 23 | 2.4 | nd | 3.2 | 4.2 | nd | 3.5 |
| OR Quart 4 | 5.3 | nd | 4.2 | 0.98 | nd | 0.86 | 1.2 | nd | 1.2 |
| p Value | 0.13 | nd | 0.20 | 0.97 | nd | 0.78 | 0.75 | nd | 0.75 |
| 95% CI of | 0.60 | nd | 0.46 | 0.36 | nd | 0.29 | 0.32 | nd | 0.32 |
| OR Quart4 | 47 | nd | 39 | 2.7 | nd | 2.5 | 4.9 | nd | 4.9 |

| | | | | | | | | | |
|---|---|---|---|---|---|---|---|---|---|
| **Neutrophil collagenase** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 1090 | | 1320 | 1090 | | 1800 | 1090 | | 1160 |
| Average | 2990 | | 1400 | 2990 | | 5630 | 2990 | | 4630 |
| Stdev | 6890 | | 1170 | 6890 | | 9430 | 6890 | | 7310 |
| p(t-test) | | | 0.36 | | | 0.064 | | | 0.31 |
| Min | 57.2 | | 21.8 | 57.2 | | 1.14 | 57.2 | | 131 |
| Max | 55000 | | 3920 | 55000 | | 38700 | 55000 | | 28000 |
| n (Samp) | 281 | | 16 | 281 | | 28 | 281 | | 20 |

| sCr or UO | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| n (Patient) | 160 | | 16 | 160 | | 28 | 160 | | 20 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | nd | | nd | nd | | nd | 1130 | | 885 |
| Average | nd | | nd | nd | | nd | 3250 | | 9310 |
| Stdev | nd | | nd | nd | | nd | 6960 | | 15400 |
| p(t-test) | nd | | nd | nd | | nd | | | 0.040 |
| Min | nd | | nd | nd | | nd | 57.2 | | 264 |
| Max | nd | | nd | nd | | nd | 55000 | | 38700 |
| n (Samp) | nd | | nd | nd | | nd | 352 | | 6 |
| n (Patient) | nd | | nd | nd | | nd | 193 | | 6 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 1100 | | 1420 | 1100 | | 2360 | 1100 | | 1160 |
| Average | 3390 | | 1590 | 3390 | | 5940 | 3390 | | 4490 |
| Stdev | 7790 | | 1190 | 7790 | | 9700 | 7790 | | 7460 |
| p(t-test) | | | 0.41 | | | 0.12 | | | 0.57 |
| Min | 40.0 | | 196 | 40.0 | | 21.8 | 40.0 | | 1.14 |
| Max | 55000 | | 3920 | 55000 | | 38700 | 55000 | | 28000 |
| n (Samp) | 257 | | 13 | 257 | | 26 | 257 | | 17 |
| n (Patient) | 140 | | 13 | 140 | | 26 | 140 | | 17 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.47 | nd | 0.52 | 0.61 | nd | 0.62 | 0.55 | 0.51 | 0.54 |
| SE | 0.075 | nd | 0.083 | 0.059 | nd | 0.061 | 0.068 | 0.12 | 0.074 |
| p | 0.72 | nd | 0.83 | 0.065 | nd | 0.045 | 0.44 | 0.96 | 0.57 |
| nCohort 1 | 281 | nd | 257 | 281 | nd | 257 | 281 | 352 | 257 |
| nCohort 2 | 16 | nd | 13 | 28 | nd | 26 | 20 | 6 | 17 |
| Cutoff 1 | 520 | nd | 669 | 799 | nd | 906 | 792 | 618 | 792 |
| Sens 1 | 75% | nd | 77% | 71% | nd | 73% | 70% | 83% | 71% |
| Spec 1 | 21% | nd | 30% | 38% | nd | 42% | 37% | 26% | 37% |
| Cutoff 2 | 318 | nd | 520 | 554 | nd | 743 | 724 | 618 | 724 |
| Sens 2 | 81% | nd | 85% | 82% | nd | 81% | 80% | 83% | 82% |
| Spec 2 | 8% | nd | 21% | 23% | nd | 35% | 35% | 26% | 34% |
| Cutoff 3 | 193 | nd | 200 | 414 | nd | 414 | 451 | 261 | 131 |
| Sens 3 | 94% | nd | 92% | 93% | nd | 92% | 90% | 100% | 94% |
| Spec 3 | 2% | nd | 3% | 15% | nd | 14% | 17% | 5% | 2% |
| Cutoff 4 | 1700 | nd | 1830 | 1700 | nd | 1830 | 1700 | 1880 | 1830 |
| Sens 4 | 31% | nd | 31% | 50% | nd | 54% | 35% | 33% | 35% |
| Spec 4 | 70% | nd | 70% | 70% | nd | 70% | 70% | 70% | 70% |
| Cutoff 5 | 2830 | nd | 3110 | 2830 | nd | 3110 | 2830 | 3180 | 3110 |
| Sens 5 | 12% | nd | 15% | 39% | nd | 38% | 25% | 33% | 24% |
| Spec 5 | 80% | nd | 80% | 80% | nd | 80% | 80% | 80% | 80% |
| Cutoff 6 | 4950 | nd | 5930 | 4950 | nd | 5930 | 4950 | 7160 | 5930 |
| Sens 6 | 0% | nd | 0% | 29% | nd | 31% | 25% | 33% | 24% |
| Spec 6 | 90% | nd | 90% | 90% | nd | 90% | 90% | 90% | 90% |
| OR Quart 2 | 1.3 | nd | 0.65 | 0.82 | nd | 0.78 | 2.1 | 3.0 | 1.3 |
| p Value | 0.72 | nd | 0.64 | 0.75 | nd | 0.71 | 0.31 | 0.34 | 0.71 |
| 95% CI of | 0.33 | nd | 0.10 | 0.24 | nd | 0.20 | 0.50 | 0.31 | 0.29 |
| OR Quart2 | 5.0 | nd | 4.0 | 2.8 | nd | 3.0 | 8.7 | 30 | 6.2 |
| OR Quart 3 | 0.49 | nd | 1.7 | 0.65 | nd | 0.78 | 1.4 | 0 | 1.4 |
| p Value | 0.42 | nd | 0.47 | 0.52 | nd | 0.71 | 0.70 | na | 0.70 |
| 95% CI of | 0.088 | nd | 0.39 | 0.18 | nd | 0.20 | 0.29 | na | 0.29 |
| OR Quart3 | 2.8 | nd | 7.5 | 2.4 | nd | 3.0 | 6.3 | na | 6.3 |
| OR Quart 4 | 1.3 | nd | 0.98 | 2.4 | nd | 2.9 | 2.4 | 2.0 | 2.1 |
| p Value | 0.72 | nd | 0.99 | 0.099 | nd | 0.055 | 0.21 | 0.57 | 0.32 |
| 95% CI of | 0.33 | nd | 0.19 | 0.85 | nd | 0.98 | 0.61 | 0.18 | 0.49 |
| OR Quart4 | 5.0 | nd | 5.1 | 6.6 | nd | 8.7 | 9.8 | 22 | 8.6 |

| | | | | | | | | | |
|---|---|---|---|---|---|---|---|---|---|
| **Protransforming growth factor alpha** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 1.45 | | 1.67 | 1.45 | | 2.22 | 1.45 | | 2.57 |
| Average | 5.56 | | 16.8 | 5.56 | | 13.3 | 5.56 | | 21.2 |
| Stdev | 26.1 | | 35.1 | 26.1 | | 32.4 | 26.1 | | 41.5 |
| p(t-test) | | | 0.11 | | | 0.15 | | | 0.025 |
| Min | 0.00228 | | 0.00228 | 0.00228 | | 0.00228 | 0.00228 | | 0.00228 |
| Max | 287 | | 137 | 287 | | 162 | 287 | | 153 |
| n (Samp) | 217 | | 16 | 217 | | 28 | 217 | | 17 |
| n (Patient) | 133 | | 16 | 133 | | 28 | 133 | | 17 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 1.26 | | 1.22 | 1.26 | | 1.87 | 1.26 | | 2.57 |
| Average | 5.63 | | 17.9 | 5.63 | | 12.3 | 5.63 | | 21.8 |
| Stdev | 27.2 | | 38.5 | 27.2 | | 31.7 | 27.2 | | 44.0 |
| p(t-test) | | | 0.13 | | | 0.23 | | | 0.036 |
| Min | 0.00228 | | 0.00228 | 0.00228 | | 0.00228 | 0.00228 | | 0.00228 |
| Max | 287 | | 137 | 287 | | 162 | 287 | | 153 |
| n (Samp) | 199 | | 13 | 199 | | 29 | 199 | | 15 |
| n (Patient) | 118 | | 13 | 118 | | 29 | 118 | | 15 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.60 | nd | 0.58 | 0.61 | nd | 0.62 | 0.66 | nd | 0.64 |
| SE | 0.077 | nd | 0.085 | 0.060 | nd | 0.059 | 0.074 | nd | 0.080 |
| p | 0.21 | nd | 0.32 | 0.075 | nd | 0.033 | 0.036 | nd | 0.084 |
| nCohort 1 | 217 | nd | 199 | 217 | nd | 199 | 217 | nd | 199 |
| nCohort 2 | 16 | nd | 13 | 28 | nd | 29 | 17 | nd | 15 |
| Cutoff 1 | 0.597 | nd | 0.597 | 0.885 | nd | 0.885 | 1.70 | nd | 0.666 |
| Sens 1 | 75% | nd | 77% | 71% | nd | 72% | 71% | nd | 73% |
| Spec 1 | 35% | nd | 38% | 41% | nd | 44% | 54% | nd | 39% |
| Cutoff 2 | 0.540 | nd | 0.537 | 0.704 | nd | 0.704 | 0.674 | nd | 0.190 |
| Sens 2 | 81% | nd | 85% | 82% | nd | 83% | 82% | nd | 80% |
| Spec 2 | 35% | nd | 36% | 37% | nd | 40% | 37% | nd | 28% |
| Cutoff 3 | 0.0223 | nd | 0.0223 | 0.190 | nd | 0.309 | 0.00228 | nd | 0.00228 |
| Sens 3 | 94% | nd | 92% | 93% | nd | 93% | 94% | nd | 93% |
| Spec 3 | 19% | nd | 21% | 25% | nd | 31% | 5% | nd | 5% |
| Cutoff 4 | 3.05 | nd | 2.70 | 3.05 | nd | 2.70 | 3.05 | nd | 2.70 |
| Sens 4 | 44% | nd | 38% | 39% | nd | 38% | 47% | nd | 47% |
| Spec 4 | 71% | nd | 70% | 71% | nd | 70% | 71% | nd | 70% |
| Cutoff 5 | 4.18 | nd | 4.00 | 4.18 | nd | 4.00 | 4.18 | nd | 4.00 |
| Sens 5 | 38% | nd | 31% | 32% | nd | 31% | 47% | nd | 47% |
| Spec 5 | 80% | nd | 80% | 80% | nd | 80% | 80% | nd | 80% |
| Cutoff 6 | 6.50 | nd | 6.43 | 6.50 | nd | 6.43 | 6.50 | nd | 6.43 |
| Sens 6 | 31% | nd | 31% | 25% | nd | 24% | 41% | nd | 40% |
| Spec 6 | 90% | nd | 90% | 90% | nd | 90% | 90% | nd | 90% |
| OR Quart 2 | 3.2 | nd | 2.7 | 3.8 | nd | 5.9 | 0.64 | nd | 1.5 |
| p Value | 0.16 | nd | 0.26 | 0.052 | nd | 0.027 | 0.64 | nd | 0.66 |
| 95% CI of | 0.62 | nd | 0.49 | 0.99 | nd | 1.2 | 0.10 | nd | 0.24 |
| OR Quart2 | 17 | nd | 14 | 15 | nd | 28 | 4.0 | nd | 9.4 |
| OR Quart 3 | 1.0 | nd | 1.0 | 1.7 | nd | 3.8 | 1.4 | nd | 1.5 |
| p Value | 1.0 | nd | 1.0 | 0.47 | nd | 0.10 | 0.70 | nd | 0.65 |
| 95% CI of | 0.14 | nd | 0.14 | 0.39 | nd | 0.76 | 0.29 | nd | 0.25 |
| OR Quart3 | 7.3 | nd | 7.4 | 7.6 | nd | 19 | 6.4 | nd | 9.5 |
| OR Quart 4 | 3.2 | nd | 2.1 | 3.7 | nd | 5.9 | 2.9 | nd | 3.8 |
| p Value | 0.17 | nd | 0.41 | 0.055 | nd | 0.027 | 0.13 | nd | 0.11 |
| 95% CI of | 0.61 | nd | 0.36 | 0.97 | nd | 1.2 | 0.72 | nd | 0.75 |
| OR Quart4 | 16 | nd | 12 | 14 | nd | 28 | 11 | nd | 19 |

| | | | | | | | | | |
|---|---|---|---|---|---|---|---|---|---|
| **CA 15-3** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 0.253 | | 0.375 | 0.253 | | 0.552 | 0.253 | | 0.502 |
| Average | 0.895 | | 1.25 | 0.895 | | 1.93 | 0.895 | | 1.79 |
| Stdev | 4.27 | | 3.04 | 4.27 | | 4.80 | 4.27 | | 4.30 |
| p(t-test) | | | 0.75 | | | 0.23 | | | 0.37 |
| Min | 0.00165 | | 0.00154 | 0.00165 | | 0.0463 | 0.00165 | | 0.0355 |
| Max | 60.0 | | 12.5 | 60.0 | | 24.0 | 60.0 | | 17.8 |
| n (Samp) | 282 | | 16 | 282 | | 28 | 282 | | 20 |
| n (Patient) | 160 | | 16 | 160 | | 28 | 160 | | 20 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | nd | | nd | nd | | nd | 0.292 | | 0.334 |
| Average | nd | | nd | nd | | nd | 1.05 | | 0.350 |
| Stdev | nd | | nd | nd | | nd | 4.23 | | 0.248 |
| p(t-test) | nd | | nd | nd | | nd | | | 0.69 |
| Min | nd | | nd | nd | | nd | 0.00154 | | 0.0355 |
| Max | nd | | nd | nd | | nd | 60.0 | | 0.682 |
| n (Samp) | nd | | nd | nd | | nd | 353 | | 6 |
| n (Patient) | nd | | nd | nd | | nd | 193 | | 6 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 0.284 | | 0.366 | 0.284 | | 0.588 | 0.284 | | 0.530 |
| Average | 0.965 | | 1.40 | 0.965 | | 2.08 | 0.965 | | 2.07 |
| Stdev | 4.46 | | 3.37 | 4.46 | | 4.96 | 4.46 | | 4.63 |
| p(t-test) | | | 0.73 | | | 0.23 | | | 0.32 |
| Min | 0.00165 | | 0.00154 | 0.00165 | | 0.109 | 0.00165 | | 0.141 |
| Max | 60.0 | | 12.5 | 60.0 | | 24.0 | 60.0 | | 17.8 |
| n (Samp) | 258 | | 13 | 258 | | 26 | 258 | | 17 |
| n (Patient) | 140 | | 13 | 140 | | 26 | 140 | | 17 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.59 | nd | 0.58 | 0.66 | nd | 0.66 | 0.64 | 0.48 | 0.67 |
| SE | 0.077 | nd | 0.085 | 0.058 | nd | 0.061 | 0.069 | 0.12 | 0.074 |
| p | 0.25 | nd | 0.35 | 0.0064 | nd | 0.0078 | 0.035 | 0.87 | 0.019 |
| nCohort 1 | 282 | nd | 258 | 282 | nd | 258 | 282 | 353 | 258 |
| nCohort 2 | 16 | nd | 13 | 28 | nd | 26 | 20 | 6 | 17 |
| Cutoff 1 | 0.266 | nd | 0.261 | 0.249 | nd | 0.249 | 0.392 | 0.187 | 0.409 |
| Sens 1 | 75% | nd | 77% | 71% | nd | 73% | 70% | 83% | 71% |
| Spec 1 | 51% | nd | 47% | 50% | nd | 46% | 69% | 31% | 67% |
| Cutoff 2 | 0.191 | nd | 0.191 | 0.187 | nd | 0.191 | 0.176 | 0.187 | 0.176 |
| Sens 2 | 81% | nd | 85% | 82% | nd | 81% | 80% | 83% | 82% |
| Spec 2 | 34% | nd | 30% | 33% | nd | 30% | 32% | 31% | 28% |
| Cutoff 3 | 0.0177 | nd | 0.0177 | 0.133 | nd | 0.136 | 0.147 | 0.0338 | 0.147 |
| Sens 3 | 94% | nd | 92% | 93% | nd | 92% | 90% | 100% | 94% |
| Spec 3 | 2% | nd | 2% | 23% | nd | 21% | 25% | 3% | 22% |
| Cutoff 4 | 0.409 | nd | 0.456 | 0.409 | nd | 0.456 | 0.409 | 0.476 | 0.456 |
| Sens 4 | 38% | nd | 38% | 57% | nd | 58% | 65% | 50% | 59% |
| Spec 4 | 70% | nd | 70% | 70% | nd | 70% | 70% | 70% | 70% |
| Cutoff 5 | 0.614 | nd | 0.708 | 0.614 | nd | 0.708 | 0.614 | 0.701 | 0.708 |
| Sens 5 | 31% | nd | 23% | 46% | nd | 38% | 30% | 0% | 29% |
| Spec 5 | 80% | nd | 80% | 80% | nd | 80% | 80% | 80% | 80% |
| Cutoff 6 | 1.14 | nd | 1.20 | 1.14 | nd | 1.20 | 1.14 | 1.40 | 1.20 |
| Sens 6 | 19% | nd | 15% | 25% | nd | 31% | 15% | 0% | 18% |
| Spec 6 | 90% | nd | 90% | 90% | nd | 90% | 90% | 90% | 90% |
| OR Quart 2 | 0.32 | nd | 1.5 | 1.2 | nd | 1.7 | 0.99 | 2.0 | 0.99 |
| p Value | 0.33 | nd | 0.66 | 0.75 | nd | 0.47 | 0.99 | 0.57 | 0.99 |
| 95% CI of | 0.033 | nd | 0.24 | 0.32 | nd | 0.39 | 0.19 | 0.18 | 0.13 |
| OR Quart2 | 3.1 | nd | 9.3 | 4.8 | nd | 7.5 | 5.0 | 23 | 7.2 |
| OR Quart 3 | 2.1 | nd | 1.5 | 1.3 | nd | 1.7 | 1.4 | 2.0 | 2.6 |
| p Value | 0.31 | nd | 0.66 | 0.73 | nd | 0.47 | 0.70 | 0.57 | 0.27 |
| 95% CI of | 0.50 | nd | 0.24 | 0.33 | nd | 0.39 | 0.29 | 0.18 | 0.48 |
| OR Quart3 | 8.7 | nd | 9.3 | 4.9 | nd | 7.5 | 6.3 | 23 | 14 |
| OR Quart 4 | 2.1 | nd | 2.6 | 4.0 | nd | 5.1 | 3.6 | 1.0 | 4.3 |
| p Value | 0.32 | nd | 0.27 | 0.019 | nd | 0.015 | 0.058 | 0.99 | 0.071 |
| 95% CI of | 0.49 | nd | 0.48 | 1.3 | nd | 1.4 | 0.96 | 0.062 | 0.88 |
| OR Quart4 | 8.6 | nd | 14 | 13 | nd | 19 | 14 | 16 | 21 |

**Table 7: Comparison of marker levels in EDTA samples collected within 12 hours of reaching stage R from Cohort 1 (patients that reached, but did not progress beyond, RIFLE stage R) and from Cohort 2 (patients that reached RIFLE stage I or F).**

| **C-C motif chemokine 8** | | | | | | |
|---|---|---|---|---|---|---|
| | sCr or UO | | sCr only | | UO only | |
| | Cohort 1 | Cohort 2 | Cohort 1 | Cohort 2 | Cohort 1 | Cohort 2 |
| Median | 13.7 | 13.8 | nd | nd | 13.8 | 13.8 |
| Average | 19.7 | 14.2 | nd | nd | 13.8 | 13.4 |
| Stdev | 31.0 | 7.93 | nd | nd | 10.1 | 8.09 |
| p(t-test) | | 0.45 | nd | nd | | 0.88 |
| Min | 0.162 | 1.20 | nd | nd | 0.170 | 1.20 |
| Max | 166 | 34.9 | nd | nd | 50.3 | 34.9 |
| n (Samp) | 41 | 19 | nd | nd | 31 | 15 |
| n (Patient) | 41 | 19 | nd | nd | 31 | 15 |
| | | | | | | |

| | At Enrollment | | | | | |
|---|---|---|---|---|---|---|
| | sCr or UO | | sCr only | | UO only | |
| AUC | 0.52 | | nd | | 0.50 | |
| SE | 0.081 | | nd | | 0.092 | |
| p | 0.77 | | nd | | 0.97 | |
| nCohort 1 | 41 | | nd | | 31 | |
| nCohort 2 | 19 | | nd | | 15 | |
| Cutoff 1 | 10.7 | | nd | | 10.6 | |
| Sens 1 | 74% | | nd | | 73% | |
| Spec 1 | 41% | | nd | | 42% | |
| Cutoff 2 | 7.48 | | nd | | 8.22 | |
| Sens 2 | 84% | | nd | | 80% | |
| Spec 2 | 22% | | nd | | 32% | |
| Cutoff 3 | 1.16 | | nd | | 1.16 | |
| Sens 3 | 100% | | nd | | 100% | |
| Spec 3 | 7% | | nd | | 6% | |
| Cutoff 4 | 16.9 | | nd | | 16.9 | |
| Sens 4 | 26% | | nd | | 13% | |
| Spec 4 | 71% | | nd | | 71% | |
| Cutoff 5 | 18.9 | | nd | | 18.9 | |
| Sens 5 | 21% | | nd | | 13% | |
| Spec 5 | 80% | | nd | | 81% | |
| Cutoff 6 | 26.8 | | nd | | 25.2 | |
| Sens 6 | 5% | | nd | | 7% | |
| Spec 6 | 90% | | nd | | 90% | |
| OR Quart 2 | 1.4 | | nd | | 6.0 | |
| p Value | 0.69 | | nd | | 0.068 | |
| 95% CI of | 0.29 | | nd | | 0.87 | |
| OR Quart2 | 6.6 | | nd | | 41 | |
| OR Quart 3 | 1.4 | | nd | | 2.5 | |
| p Value | 0.69 | | nd | | 0.35 | |
| 95% CI of | 0.29 | | nd | | 0.36 | |
| OR Quart3 | 6.6 | | nd | | 17 | |
| OR Quart 4 | 1.4 | | nd | | 1.9 | |
| p Value | 0.69 | | nd | | 0.54 | |
| 95% CI of | 0.29 | | nd | | 0.25 | |
| OR Quart4 | 6.6 | | nd | | 14 | |

| | | | | | | |
|---|---|---|---|---|---|---|
| **Immunoglogulin G1** | | | | | | |
| | sCr or UO | | sCr only | | UO only | |
| | Cohort 1 | Cohort 2 | Cohort 1 | Cohort 2 | Cohort 1 | Cohort 2 |
| Median | 8820000 | 8470000 | nd | nd | 8840000 | 8470000 |
| Average | 9870000 | 1.04E7 | nd | nd | 9700000 | 8840000 |
| Stdev | 4230000 | 6820000 | nd | nd | 3800000 | 2660000 |
| p(t-test) | | 0.74 | nd | nd | | 0.58 |
| Min | 2060000 | 4620000 | nd | nd | 2060000 | 4620000 |
| Max | 2.33E7 | 2.72E7 | nd | nd | 1.75E7 | 1.20E7 |
| n (Samp) | 41 | 9 | nd | nd | 30 | 7 |
| n (Patient) | 41 | 9 | nd | nd | 30 | 7 |
| | | | | | | |

| | At Enrollment | | | | | |
|---|---|---|---|---|---|---|
| | sCr or UO | | sCr only | | UO only | |
| AUC | 0.46 | | nd | | 0.45 | |
| SE | 0.11 | | nd | | 0.12 | |
| p | 0.73 | | nd | | 0.66 | |
| nCohort 1 | 41 | | nd | | 30 | |
| nCohort 2 | 9 | | nd | | 7 | |
| Cutoff 1 | 7140000 | | nd | | 7600000 | |
| Sens 1 | 78% | | nd | | 71% | |
| Spec 1 | 29% | | nd | | 33% | |
| Cutoff 2 | 4620000 | | nd | | 6710000 | |
| Sens 2 | 89% | | nd | | 86% | |
| Spec 2 | 2% | | nd | | 27% | |
| Cutoff 3 | 2060000 | | nd | | 2060000 | |
| Sens 3 | 100% | | nd | | 100% | |
| Spec 3 | 2% | | nd | | 3% | |
| Cutoff 4 | 1.17E7 | | nd | | 1.15E7 | |
| Sens 4 | 33% | | nd | | 29% | |
| Spec 4 | 71% | | nd | | 70% | |
| Cutoff 5 | 1.34E7 | | nd | | 1.34E7 | |
| Sens 5 | 11% | | nd | | 0% | |
| Spec 5 | 80% | | nd | | 80% | |
| Cutoff 6 | 1.59E7 | | nd | | 1.39E7 | |
| Sens 6 | 11% | | nd | | 0% | |
| Spec 6 | 90% | | nd | | 90% | |
| OR Quart 2 | 4.0 | | nd | | 0.50 | |
| p Value | 0.26 | | nd | | 0.60 | |
| 95% CI of | 0.35 | | nd | | 0.037 | |
| OR Quart2 | 45 | | nd | | 6.7 | |
| OR Quart 3 | 3.6 | | nd | | 2.0 | |
| p Value | 0.30 | | nd | | 0.51 | |
| 95% CI of | 0.32 | | nd | | 0.25 | |
| OR Quart3 | 40 | | nd | | 16 | |
| OR Quart 4 | 2.4 | | nd | | 0.50 | |
| p Value | 0.50 | | nd | | 0.60 | |
| 95% CI of | 0.19 | | nd | | 0.037 | |
| OR Quart4 | 31 | | nd | | 6.7 | |

| | | | | | | |
|---|---|---|---|---|---|---|
| **Interleukin-11** | | | | | | |
| | sCr or UO | | sCr only | | UO only | |
| | Cohort 1 | Cohort 2 | Cohort 1 | Cohort 2 | Cohort 1 | Cohort 2 |
| Median | 91.3 | 87.4 | nd | nd | 91.9 | 76.6 |
| Average | 112 | 86.0 | nd | nd | 98.1 | 77.8 |
| Stdev | 130 | 49.3 | nd | nd | 86.1 | 52.9 |
| p(t-test) | | 0.40 | nd | nd | | 0.41 |
| Min | 0.359 | 0.480 | nd | nd | 0.359 | 0.480 |
| Max | 741 | 191 | nd | nd | 371 | 191 |
| n (Samp) | 41 | 19 | nd | nd | 31 | 15 |
| n (Patient) | 41 | 19 | nd | nd | 31 | 15 |
| | | | | | | |

| | At Enrollment | | | | | |
|---|---|---|---|---|---|---|
| | sCr or UO | | sCr only | | UO only | |
| AUC | 0.47 | | nd | | 0.45 | |
| SE | 0.081 | | nd | | 0.092 | |
| p | 0.76 | | nd | | 0.61 | |
| nCohort 1 | 41 | | nd | | 31 | |
| nCohort 2 | 19 | | nd | | 15 | |
| Cutoff 1 | 55.3 | | nd | | 47.0 | |
| Sens 1 | 79% | | nd | | 73% | |
| Spec 1 | 34% | | nd | | 35% | |
| Cutoff 2 | 47.3 | | nd | | 10.7 | |
| Sens 2 | 84% | | nd | | 80% | |
| Spec 2 | 34% | | nd | | 26% | |
| Cutoff 3 | 0.736 | | nd | | 0.736 | |
| Sens 3 | 95% | | nd | | 93% | |
| Spec 3 | 20% | | nd | | 26% | |
| Cutoff 4 | 139 | | nd | | 146 | |
| Sens 4 | | 11% | | nd | | 7% |
| Spec 4 | | 71% | | nd | | 71% |
| Cutoff 5 | | 163 | | nd | | 163 |
| Sens 5 | | 11% | | nd | | 7% |
| Spec 5 | | 80% | | nd | | 81% |
| Cutoff 6 | | 190 | | nd | | 184 |
| Sens 6 | | 5% | | nd | | 7% |
| Spec 6 | | 90% | | nd | | 90% |
| OR Quart 2 | | 4.3 | | nd | | 13 |
| p Value | | 0.11 | | nd | | 0.033 |
| 95% CI of | | 0.71 | | nd | | 1.2 |
| OR Quart2 | | 27 | | nd | | 140 |
| OR Quart 3 | | 7.4 | | nd | | 7.9 |
| p Value | | 0.029 | | nd | | 0.085 |
| 95% CI of | | 1.2 | | nd | | 0.75 |
| OR Quart3 | | 45 | | nd | | 82 |
| OR Quart 4 | | 1.6 | | nd | | 4.1 |
| p Value | | 0.63 | | nd | | 0.25 |
| 95% CI of | | 0.23 | | nd | | 0.36 |
| OR Quart4 | | 11 | | nd | | 47 |

**Table 8: Comparison of the maximum marker levels in EDTA samples collected from Cohort 1 (patients that did not progress beyond RIFLE stage 0) and the maximum values in EDTA samples collected from subjects between enrollment and 0,24 hours, and 48 hours prior to reaching stage F in Cohort 2.**

| **C-C motif chemokine 18** | | | | | | | | | |
|---|---|---|---|---|---|---|---|---|---|
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 170 | | 303 | 170 | | 303 | nd | | nd |
| Average | 230 | | 472 | 230 | | 463 | nd | | nd |
| Stdev | 178 | | 512 | 178 | | 518 | nd | | nd |
| p(t-test) | | | 0.0041 | | | 0.0058 | nd | | nd |
| Min | 38.6 | | 103 | 38.6 | | 103 | nd | | nd |
| Max | 1020 | | 1650 | 1020 | | 1650 | nd | | nd |
| n (Samp) | 87 | | 8 | 87 | | 8 | nd | | nd |
| n (Patient) | 87 | | 8 | 87 | | 8 | nd | | nd |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 173 | | 380 | 173 | | 380 | nd | | nd |
| Average | 247 | | 580 | 247 | | 580 | nd | | nd |
| Stdev | 181 | | 557 | 181 | | 557 | nd | | nd |
| p(t-test) | | | 6.5E-4 | | | 6.5E-4 | nd | | nd |
| Min | 38.6 | | 118 | 38.6 | | 118 | nd | | nd |
| Max | 848 | | 1650 | 848 | | 1650 | nd | | nd |
| n (Samp) | 80 | | 6 | 80 | | 6 | nd | | nd |
| n (Patient) | 80 | | 6 | 80 | | 6 | nd | | nd |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.68 | nd | 0.75 | 0.65 | nd | 0.75 | nd | nd | nd |
| SE | 0.11 | nd | 0.12 | 0.11 | nd | 0.12 | nd | nd | nd |
| p | 0.10 | nd | 0.037 | 0.18 | nd | 0.037 | nd | nd | nd |
| nCohort 1 | 87 | nd | 80 | 87 | nd | 80 | nd | nd | nd |
| nCohort 2 | 8 | nd | 6 | 8 | nd | 6 | nd | nd | nd |
| Cutoff 1 | 186 | nd | 264 | 120 | nd | 264 | nd | nd | nd |
| Sens 1 | 75% | nd | 83% | 75% | nd | 83% | nd | nd | nd |
| Spec 1 | 56% | nd | 65% | 31% | nd | 65% | nd | nd | nd |
| Cutoff 2 | 118 | nd | 264 | 118 | nd | 264 | nd | nd | nd |
| Sens 2 | 88% | nd | 83% | 88% | nd | 83% | nd | nd | nd |
| Spec 2 | 30% | nd | 65% | 30% | nd | 65% | nd | nd | nd |
| Cutoff 3 | 103 | nd | 117 | 103 | nd | 117 | nd | nd | nd |
| Sens 3 | 100% | nd | 100% | 100% | nd | 100% | nd | nd | nd |
| Spec 3 | 21% | nd | 25% | 21% | nd | 25% | nd | nd | nd |
| Cutoff 4 | 246 | nd | 283 | 246 | nd | 283 | nd | nd | nd |
| Sens 4 | 62% | nd | 67% | 62% | nd | 67% | nd | nd | nd |
| Spec 4 | 70% | nd | 70% | 70% | nd | 70% | nd | nd | nd |
| Cutoff 5 | 309 | nd | 379 | 309 | nd | 379 | nd | nd | nd |
| Sens 5 | 50% | nd | 50% | 50% | nd | 50% | nd | nd | nd |
| Spec 5 | 80% | nd | 80% | 80% | nd | 80% | nd | nd | nd |
| Cutoff 6 | 484 | nd | 507 | 484 | nd | 507 | nd | nd | nd |
| Sens 6 | 25% | nd | 33% | 25% | nd | 33% | nd | nd | nd |
| Spec 6 | 91% | nd | 90% | 91% | nd | 90% | nd | nd | nd |
| OR Quart 2 | 0.96 | nd | 0 | 2.0 | nd | 0 | nd | nd | nd |
| p Value | 0.98 | nd | na | 0.58 | nd | na | nd | nd | nd |
| 95% CI of | 0.056 | nd | na | 0.17 | nd | na | nd | nd | nd |
| OR Quart2 | 16 | nd | na | 24 | nd | na | nd | nd | nd |
| OR Quart 3 | 2.0 | nd | 1.0 | 0.96 | nd | 1.0 | nd | nd | nd |
| p Value | 0.58 | nd | 1.0 | 0.98 | nd | 1.0 | nd | nd | nd |
| 95% CI of | 0.17 | nd | 0.058 | 0.056 | nd | 0.058 | nd | nd | nd |
| OR Quart3 | 24 | nd | 17 | 16 | nd | 17 | nd | nd | nd |
| OR Quart 4 | 4.4 | nd | 4.4 | 4.4 | nd | 4.4 | nd | nd | nd |
| p Value | 0.20 | nd | 0.20 | 0.20 | nd | 0.20 | nd | nd | nd |
| 95% CI of | 0.45 | nd | 0.45 | 0.45 | nd | 0.45 | nd | nd | nd |
| OR Quart4 | 43 | nd | 44 | 43 | nd | 44 | nd | nd | nd |

| | | | | | | | | | |
|---|---|---|---|---|---|---|---|---|---|
| **C-C motif chemokine 24** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 222 | | 259 | 222 | | 259 | 222 | | 155 |
| Average | 400 | | 476 | 400 | | 366 | 400 | | 299 |
| Stdev | 493 | | 643 | 493 | | 299 | 493 | | 235 |

| sCr or UO | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| p(t-test) | | | 0.64 | | | 0.82 | | | 0.60 |
| Min | 0.0260 | | 86.4 | 0.0260 | | 86.4 | 0.0260 | | 86.4 |
| Max | 2920 | | 2430 | 2920 | | 1100 | 2920 | | 614 |
| n (Samp) | 64 | | 12 | 64 | | 12 | 64 | | 7 |
| n (Patient) | 64 | | 12 | 64 | | 12 | 64 | | 7 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 245 | | 554 | 245 | | 554 | nd | | nd |
| Average | 386 | | 755 | 386 | | 534 | nd | | nd |
| Stdev | 423 | | 843 | 423 | | 341 | nd | | nd |
| p(t-test) | | | 0.049 | | | 0.40 | nd | | nd |
| Min | 0.0260 | | 95.2 | 0.0260 | | 95.2 | nd | | nd |
| Max | 2920 | | 2430 | 2920 | | 1100 | nd | | nd |
| n (Samp) | 131 | | 6 | 131 | | 6 | nd | | nd |
| n (Patient) | 131 | | 6 | 131 | | 6 | nd | | nd |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 282 | | 163 | 282 | | 163 | 282 | | 148 |
| Average | 467 | | 237 | 467 | | 237 | 467 | | 249 |
| Stdev | 499 | | 182 | 499 | | 182 | 499 | | 213 |
| p(t-test) | | | 0.20 | | | 0.20 | | | 0.30 |
| Min | 24.7 | | 86.4 | 24.7 | | 86.4 | 24.7 | | 86.4 |
| Max | 2920 | | 614 | 2920 | | 614 | 2920 | | 614 |
| n (Samp) | 63 | | 8 | 63 | | 8 | 63 | | 6 |
| n (Patient) | 63 | | 8 | 63 | | 8 | 63 | | 6 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.54 | 0.68 | 0.35 | 0.54 | 0.67 | 0.35 | 0.48 | nd | 0.35 |
| SE | 0.093 | 0.12 | 0.11 | 0.093 | 0.12 | 0.11 | 0.12 | nd | 0.13 |
| p | 0.63 | 0.14 | 0.17 | 0.66 | 0.16 | 0.17 | 0.85 | nd | 0.23 |
| nCohort 1 | 64 | 131 | 63 | 64 | 131 | 63 | 64 | nd | 63 |
| nCohort 2 | 12 | 6 | 8 | 12 | 6 | 8 | 7 | nd | 6 |
| Cutoff 1 | 154 | 282 | 138 | 154 | 282 | 138 | 134 | nd | 89.8 |
| Sens 1 | 75% | 83% | 75% | 75% | 83% | 75% | 71% | nd | 83% |
| Spec 1 | 36% | 56% | 22% | 36% | 56% | 22% | 30% | nd | 13% |
| Cutoff 2 | 134 | 282 | 89.8 | 134 | 282 | 89.8 | 94.1 | nd | 89.8 |
| Sens 2 | 83% | 83% | 88% | 83% | 83% | 88% | 86% | nd | 83% |
| Spec 2 | 30% | 56% | 13% | 30% | 56% | 13% | 22% | nd | 13% |
| Cutoff 3 | 94.1 | 95.2 | 85.2 | 94.1 | 95.2 | 85.2 | 85.2 | nd | 85.2 |
| Sens 3 | 92% | 100% | 100% | 92% | 100% | 100% | 100% | nd | 100% |
| Spec 3 | 22% | 22% | 11% | 22% | 22% | 11% | 17% | nd | 11% |
| Cutoff 4 | 400 | 463 | 567 | 400 | 463 | 567 | 400 | nd | 567 |
| Sens 4 | 42% | 67% | 12% | 42% | 67% | 12% | 43% | nd | 17% |
| Spec 4 | 70% | 70% | 71% | 70% | 70% | 71% | 70% | nd | 71% |
| Cutoff 5 | 657 | 625 | 715 | 657 | 625 | 715 | 657 | nd | 715 |
| Sens 5 | 8% | 17% | 0% | 8% | 17% | 0% | 0% | nd | 0% |
| Spec 5 | 81% | 80% | 81% | 81% | 80% | 81% | 81% | nd | 81% |
| Cutoff 6 | 855 | 859 | 991 | 855 | 859 | 991 | 855 | nd | 991 |
| Sens 6 | 8% | 17% | 0% | 8% | 17% | 0% | 0% | nd | 0% |
| Spec 6 | 91% | 90% | 90% | 91% | 90% | 90% | 91% | nd | 90% |
| OR Quart 2 | 1.6 | 0 | 1.0 | 1.6 | 0 | 1.0 | 0.47 | nd | 1.1 |
| p Value | 0.63 | na | 1.0 | 0.63 | na | 1.0 | 0.55 | nd | 0.97 |
| 95% CI of | 0.23 | na | 0.058 | 0.23 | na | 0.058 | 0.039 | nd | 0.061 |
| OR Quart2 | 11 | na | 17 | 11 | na | 17 | 5.7 | nd | 18 |
| OR Quart 3 | 2.3 | 2.1 | 3.4 | 2.3 | 2.1 | 3.4 | 1.0 | nd | 1.1 |
| p Value | 0.38 | 0.56 | 0.31 | 0.38 | 0.56 | 0.31 | 1.0 | nd | 0.97 |
| 95% CI of | 0.36 | 0.18 | 0.32 | 0.36 | 0.18 | 0.32 | 0.13 | nd | 0.061 |
| OR Quart3 | 14 | 24 | 36 | 14 | 24 | 36 | 8.0 | nd | 18 |
| OR Quart 4 | 1.6 | 3.1 | 3.6 | 1.6 | 3.1 | 3.6 | 1.1 | nd | 3.6 |
| p Value | 0.63 | 0.34 | 0.29 | 0.63 | 0.34 | 0.29 | 0.95 | nd | 0.29 |
| 95% CI of | 0.23 | 0.31 | 0.34 | 0.23 | 0.31 | 0.34 | 0.13 | nd | 0.34 |
| OR Quart4 | 11 | 31 | 39 | 11 | 31 | 39 | 8.6 | nd | 39 |

| | | | | | | | | | |
|---|---|---|---|---|---|---|---|---|---|
| **C-C motif chemokine 8** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 13.9 | | 21.7 | 13.9 | | 21.7 | 13.9 | | 23.8 |
| Average | 20.1 | | 42.4 | 20.1 | | 42.3 | 20.1 | | 37.2 |
| Stdev | 26.8 | | 48.6 | 26.8 | | 48.7 | 26.8 | | 35.5 |
| p(t-test) | | | 0.025 | | | 0.026 | | | 0.13 |
| Min | 1.20 | | 6.24 | 1.20 | | 6.24 | 1.20 | | 6.24 |
| Max | 148 | | 169 | 148 | | 169 | 148 | | 101 |
| n (Samp) | 64 | | 12 | 64 | | 12 | 64 | | 7 |
| n (Patient) | 64 | | 12 | 64 | | 12 | 64 | | 7 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 15.1 | | 21.8 | 15.1 | | 21.1 | nd | | nd |
| Average | 20.2 | | 55.5 | 20.2 | | 55.3 | nd | | nd |
| Stdev | 25.1 | | 65.4 | 25.1 | | 65.6 | nd | | nd |
| p(t-test) | | | 0.0027 | | | 0.0028 | nd | | nd |
| Min | 1.20 | | 6.24 | 1.20 | | 6.24 | nd | | nd |
| Max | 180 | | 169 | 180 | | 169 | nd | | nd |
| n (Samp) | 131 6 | | | 131 | | 6 | nd | | nd |
| n (Patient) | 131 6 | | | 131 | | 6 | nd | | nd |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 13.7 | | 21.7 | 13.7 | | 21.7 | 13.7 | | 20.4 |
| Average | 21.2 | | 26.3 | 21.2 | | 26.3 | 21.2 | | 26.5 |
| Stdev | 27.6 | | 20.6 | 27.6 | | 20.6 | 27.6 | | 23.7 |
| p(t-test) | | | 0.61 | | | 0.61 | | | 0.65 |
| Min | 1.20 | | 6.24 | 1.20 | | 6.24 | 1.20 | | 6.24 |
| Max | 148 | | 72.1 | 148 | | 72.1 | 148 | | 72.1 |
| n (Samp) | 63 | | 8 | 63 | | 8 | 63 | | 6 |
| n (Patient) | 63 | | 8 | 63 | | 8 | 63 | | 6 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.70 | 0.66 | 0.66 | 0.70 | 0.65 | 0.66 | 0.69 | nd | 0.63 |
| SE | 0.090 | 0.12 | 0.11 | 0.090 | 0.12 | 0.11 | 0.12 | nd | 0.13 |
| p | 0.025 | 0.21 | 0.15 | 0.030 | 0.24 | 0.15 | 0.097 | nd | 0.29 |
| nCohort 1 | 64 | 131 | 63 | 64 | 131 | 63 | 64 | nd | 63 |
| nCohort 2 | 12 | 6 | 8 | 12 | 6 | 8 | 7 | nd | 6 |
| Cutoff 1 | 14.2 | 13.4 | 14.2 | 13.7 | 13.4 | 14.2 | 16.9 | nd | 11.3 |
| Sens 1 | 75% | 83% | 75% | 75% | 83% | 75% | 71% | nd | 83% |
| Spec 1 | 52% | 44% | 54% | 50% | 44% | 54% | 64% | nd | 41% |
| Cutoff 2 | 13.0 | 13.4 | 11.3 | 13.0 | 13.4 | 11.3 | 11.3 | nd | 11.3 |
| Sens 2 | 83% | 83% | 88% | 83% | 83% | 88% | 86% | nd | 83% |
| Spec 2 | 48% | 44% | 41% | 48% | 44% | 41% | 36% | nd | 41% |
| Cutoff 3 | 11.3 | 5.84 | 4.81 | 11.3 | 5.84 | 4.81 | 4.81 | nd | 4.81 |
| Sens 3 | 92% | 100% | 100% | 92% | 100% | 100% | 100% | nd | 100% |
| Spec 3 | 36% | 13% | 14% | 36% | 13% | 14% | 16% | nd | 14% |
| Cutoff 4 | 18.9 | 18.9 | 20.1 | 18.9 | 18.9 | 20.1 | 18.9 | nd | 20.1 |
| Sens 4 | 58% | 50% | 50% | 58% | 50% | 50% | 57% | nd | 50% |
| Spec 4 | 70% | 70% | 73% | 70% | 70% | 73% | 70% | nd | 73% |
| Cutoff 5 | 22.4 | 22.1 | 24.3 | 22.4 | 22.1 | 24.3 | 22.4 | nd | 24.3 |
| Sens 5 | 50% | 50% | 38% | 50% | 50% | 38% | 57% | nd | 33% |
| Spec 5 | 81% | 80% | 81% | 81% | 80% | 81% | 81% | nd | 81% |
| Cutoff 6 | 30.2 | 30.5 | 39.5 | 30.2 | 30.5 | 39.5 | 30.2 | nd | 39.5 |
| Sens 6 | 33% | 33% | 12% | 33% | 33% | 12% | 29% | nd | 17% |
| Spec 6 | 91% | 90% | 92% | 91% | 90% | 92% | 91% | nd | 92% |
| OR Quart 2 | 3.4 | 2.1 | 0.94 | 3.4 | 2.1 | 0.94 | 0.94 | nd | 1.0 |
| p Value | 0.31 | 0.56 | 0.97 | 0.31 | 0.56 | 0.97 | 0.97 | nd | 1.0 |
| 95% CI of | 0.32 | 0.18 | 0.054 | 0.32 | 0.18 | 0.054 | 0.054 | nd | 0.057 |
| OR Quart2 | 36 | 24 | 16 | 36 | 24 | 16 | 16 | nd | 17 |
| OR Quart 3 | 2.1 | 0 | 2.0 | 2.1 | 0 | 2.0 | 0.94 | nd | 1.0 |
| p Value | 0.55 | na | 0.59 | 0.55 | na | 0.59 | 0.97 | nd | 1.0 |
| 95% CI of | 0.18 | na | 0.16 | 0.18 | na | 0.16 | 0.054 | nd | 0.057 |
| OR Quart3 | 26 | na | 24 | 26 | na | 24 | 16 | nd | 17 |
| OR Quart 4 | 8.3 | 3.1 | 4.6 | 8.3 | 3.1 | 4.6 | 4.6 | nd | 3.2 |
| p Value | 0.063 | 0.34 | 0.20 | 0.063 | 0.34 | 0.20 | 0.20 | nd | 0.34 |
| 95% CI of | 0.89 | 0.31 | 0.46 | 0.89 | 0.31 | 0.46 | 0.46 | nd | 0.30 |
| OR Quart4 | 78 | 31 | 46 | 78 | 31 | 46 | 46 | nd | 34 |

| **Cathepsin D** | | | | | | | | | |
|---|---|---|---|---|---|---|---|---|---|
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 233000 | | 479000 | 233000 | | 479000 | nd | | nd |
| Average | 256000 | | 538000 | 256000 | | 452000 | nd | | nd |
| Stdev | 149000 | | 348000 | 149000 | | 144000 | nd | | nd |
| p(t-test) | | | 2.6E-5 | | | 5.9E-4 | nd | | nd |
| Min | 34100 | | 266000 | 34100 | | 266000 | nd | | nd |
| Max | 1020000 | | 1350000 | 1020000 | | 655000 | nd | | nd |
| n (Samp) | 86 | | 8 | 86 | | 8 | nd | | nd |
| n (Patient) | 86 | | 8 | 86 | | 8 | nd | | nd |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 247000 | | 479000 | 247000 | | 479000 | nd | | nd |
| Average | 278000 | | 442000 | 278000 | | 442000 | nd | | nd |
| Stdev | 153000 | | 128000 | 153000 | | 128000 | nd | | nd |
| p(t-test) | | | 0.012 | | | 0.012 | nd | | nd |
| Min | 69400 | | 266000 | 69400 | | 266000 | nd | | nd |
| Max | 1020000 | | 561000 | 1020000 | | 561000 | nd | | nd |
| n (Samp) | 79 | | 6 | 79 | | 6 | nd | | nd |
| n (Patient) | 79 | | 6 | 79 | | 6 | nd | | nd |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.86 | nd | 0.83 | 0.86 | nd | 0.83 | nd | nd | nd |
| SE | 0.084 | nd | 0.10 | 0.084 | nd | 0.10 | nd | nd | nd |
| p | 1.3E-5 | nd | 0.0016 | 1.8E-5 | nd | 0.0016 | nd | nd | nd |
| nCohort 1 | 86 | nd | 79 | 86 | nd | 79 | nd | nd | nd |
| nCohort 2 | 8 | nd | 6 | 8 | nd | 6 | nd | nd | nd |
| Cutoff 1 | 307000 | nd | 307000 | 307000 | nd | 307000 | nd | nd | nd |
| Sens 1 | 75% | nd | 83% | 75% | nd | 83% | nd | nd | nd |
| Spec 1 | 74% | nd | 67% | 74% | nd | 67% | nd | nd | nd |
| Cutoff 2 | 307000 | nd | 307000 | 307000 | nd | 307000 | nd | nd | nd |
| Sens 2 | 88% | nd | 83% | 88% | nd | 83% | nd | nd | nd |
| Spec 2 | 73% | nd | 67% | 73% | nd | 67% | nd | nd | nd |
| Cutoff 3 | 263000 | nd | 263000 | 263000 | nd | 263000 | nd | nd | nd |
| Sens 3 | 100% | nd | 100% | 100% | nd | 100% | nd | nd | nd |
| Spec 3 | 66% | nd | 58% | 66% | nd | 58% | nd | nd | nd |
| Cutoff 4 | 302000 | nd | 310000 | 302000 | nd | 310000 | nd | nd | nd |
| Sens 4 | 88% | nd | 67% | 88% | nd | 67% | nd | nd | nd |
| Spec 4 | 71% | nd | 71% | 71% | nd | 71% | nd | nd | nd |
| Cutoff 5 | 324000 | nd | 374000 | 324000 | nd | 374000 | nd | nd | nd |
| Sens 5 | 62% | nd | 67% | 62% | nd | 67% | nd | nd | nd |
| Spec 5 | 80% | nd | 81% | 80% | nd | 81% | nd | nd | nd |
| Cutoff 6 | 442000 | nd | 471000 | 442000 | nd | 471000 | nd | nd | nd |
| Sens 6 | 62% | nd | 50% | 62% | nd | 50% | nd | nd | nd |
| Spec 6 | 91% | nd | 91% | 91% | nd | 91% | nd | nd | nd |
| OR Quart 2 | >0 | nd | >0 | >0 | nd | >0 | nd | nd | nd |
| p Value | <na | nd | <na | <na | nd | <na | nd | nd | nd |
| 95% CI of | >na | nd | >na | >na | nd | >na | nd | nd | nd |
| OR Quart2 | na | nd | na | na | nd | na | nd | nd | nd |
| OR Quart 3 | >3.4 | nd | >2.2 | >3.4 | nd | >2.2 | nd | nd | nd |
| p Value | <0.30 | nd | <0.53 | <0.30 | nd | <0.53 | nd | nd | nd |
| 95% CI of | >0.33 | nd | >0.19 | >0.33 | nd | >0.19 | nd | nd | nd |
| OR Quart3 | na | nd | na | na | nd | na | nd | nd | nd |
| OR Quart 4 | >6.1 | nd | >4.7 | >6.1 | nd | >4.7 | nd | nd | nd |
| p Value | <0.11 | nd | <0.19 | <0.11 | nd | <0.19 | nd | nd | nd |
| 95% CI of | >0.65 | nd | >0.48 | >0.65 | nd | >0.48 | nd | nd | nd |
| OR Quart4 | na | nd | na | na | nd | na | nd | nd | nd |

| | | | | | | | | | |
|---|---|---|---|---|---|---|---|---|---|
| **C-X-C motif chemokine 13** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 47.3 | | 83.0 | 47.3 | | 83.0 | 47.3 | | 82.8 |
| Average | 162 | | 306 | 162 | | 306 | 162 | | 190 |
| Stdev | 351 | | 564 | 351 | | 564 | 351 | | 234 |
| p(t-test) | | | 0.24 | | | 0.24 | | | 0.84 |
| Min | 9.90 | | 15.1 | 9.90 | | 15.1 | 9.90 | | 45.0 |
| Max | 1790 | | 2000 | 1790 | | 2000 | 1790 | | 685 |
| n (Samp) | 64 | | 12 | 64 | | 12 | 64 | | 7 |
| n (Patient) | 64 | | 12 | 64 | | 12 | 64 | | 7 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 53.6 | | 83.0 | 53.6 | | 83.0 | nd | | nd |
| Average | 150 | | 399 | 150 | | 399 | nd | | nd |
| Stdev | 294 | | 785 | 294 | | 785 | nd | | nd |
| p(t-test) | | | 0.070 | | | 0.070 | nd | | nd |
| Min | 9.90 | | 15.1 | 9.90 | | 15.1 | nd | | nd |
| Max | 1790 | | 2000 | 1790 | | 2000 | nd | | nd |
| n (Samp) | 131 | | 6 | 131 | | 6 | nd | | nd |
| n (Patient) | 131 | | 6 | 131 | | 6 | nd | | nd |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 51.9 | | 77.2 | 51.9 | | 77.2 | 51.9 | | 77.2 |
| Average | 149 | | 180 | 149 | | 180 | 149 | | 208 |
| Stdev | 298 | | 221 | 298 | | 221 | 298 | | 251 |
| p(t-test) | | | 0.78 | | | 0.78 | | | 0.64 |
| Min | 9.90 | | 35.6 | 9.90 | | 35.6 | 9.90 | | 45.0 |
| Max | 1790 | | 685 | 1790 | | 685 | 1790 | | 685 |
| n (Samp) | 63 | | 8 | 63 | | 8 | 63 | | 6 |
| n (Patient) | 63 | | 8 | 63 | | 8 | 63 | | 6 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.65 | 0.60 | 0.64 | 0.65 | 0.60 | 0.64 | 0.67 | nd | 0.66 |
| SE | 0.092 | 0.12 | 0.11 | 0.092 | 0.12 | 0.11 | 0.12 | nd | 0.13 |
| p | 0.11 | 0.45 | 0.22 | 0.11 | 0.45 | 0.22 | 0.15 | nd | 0.20 |
| nCohort 1 | 64 | 131 | 63 | 64 | 131 | 63 | 64 | nd | 63 |
| nCohort 2 | 12 | 6 | 8 | 12 | 6 | 8 | 7 | nd | 6 |
| Cutoff 1 | 66.4 | 70.0 | 66.4 | 66.4 | 70.0 | 66.4 | 68.9 | nd | 66.4 |
| Sens 1 | 75% | 83% | 75% | 75% | 83% | 75% | 71% | nd | 83% |
| Spec 1 | 58% | 56% | 54% | 58% | 56% | 54% | 58% | nd | 54% |
| Cutoff 2 | 44.3 | 70.0 | 44.3 | 44.3 | 70.0 | 44.3 | 66.4 | nd | 66.4 |
| Sens 2 | 83% | 83% | 88% | 83% | 83% | 88% | 86% | nd | 83% |
| Spec 2 | 45% | 56% | 41% | 45% | 56% | 41% | 58% | nd | 54% |
| Cutoff 3 | 35.5 | 15.0 | 35.5 | 35.5 | 15.0 | 35.5 | 44.3 | nd | 44.3 |
| Sens 3 | 92% | 100% | 100% | 92% | 100% | 100% | 100% | nd | 100% |
| Spec 3 | 39% | 5% | 32% | 39% | 5% | 32% | 45% | nd | 41% |
| Cutoff 4 | 106 | 114 | 111 | 106 | 114 | 111 | 106 | nd | 111 |
| Sens 4 | 42% | 33% | 38% | 42% | 33% | 38% | 29% | nd | 33% |
| Spec 4 | 70% | 70% | 71% | 70% | 70% | 71% | 70% | nd | 71% |
| Cutoff 5 | 170 | 155 | 140 | 170 | 155 | 140 | 170 | nd | 140 |
| Sens 5 | 25% | 17% | 38% | 25% | 17% | 38% | 29% | nd | 33% |
| Spec 5 | 81% | 80% | 81% | 81% | 80% | 81% | 81% | nd | 81% |
| Cutoff 6 | 254 | 260 | 254 | 254 | 260 | 254 | 254 | nd | 254 |
| Sens 6 | 25% | 17% | 25% | 25% | 17% | 25% | 29% | nd | 33% |
| Spec 6 | 91% | 90% | 90% | 91% | 90% | 90% | 91% | nd | 90% |
| OR Quart 2 | 2.1 | 0 | >2.1 | 2.1 | 0 | >2.1 | >1.0 | nd | >1.1 |
| p Value | 0.55 | na | <0.55 | 0.55 | na | <0.55 | <1.0 | nd | <0.97 |
| 95% CI of | 0.18 | na | >0.18 | 0.18 | na | >0.18 | >0.058 | nd | >0.061 |
| OR Quart2 | 26 | na | na | 26 | na | na | na | nd | na |
| OR Quart 3 | 4.8 | 3.2 | >3.4 | 4.8 | 3.2 | >3.4 | >4.9 | nd | >3.6 |
| p Value | 0.18 | 0.33 | <0.31 | 0.18 | 0.33 | <0.31 | <0.18 | nd | <0.29 |
| 95% CI of | 0.48 | 0.32 | >0.32 | 0.48 | 0.32 | >0.32 | >0.49 | nd | >0.34 |
| OR Quart3 | 48 | 32 | na | 48 | 32 | na | na | nd | na |
| OR Quart 4 | 6.4 | 2.0 | >3.4 | 6.4 | 2.0 | >3.4 | >2.1 | nd | >2.1 |
| p Value | 0.11 | 0.58 | <0.31 | 0.11 | 0.58 | <0.31 | <0.55 | nd | <0.55 |
| 95% CI of | 0.67 | 0.17 | >0.32 | 0.67 | 0.17 | >0.32 | >0.18 | nd | >0.18 |
| OR Quart4 | 61 | 23 | na | 61 | 23 | na | na | nd | na |

| | | | | | | | | | |
|---|---|---|---|---|---|---|---|---|---|
| **Insulin-like growth factor-binding protein 3** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 3530 | | 4230 | 3530 | | 3620 | nd | | nd |
| Average | 3640 | | 4460 | 3640 | | 4160 | nd | | nd |
| Stdev | 1520 | | 1990 | 1520 | | 1840 | nd | | nd |
| p(t-test) | | | 0.16 | | | 0.36 | nd | | nd |
| Min | 823 | | 2070 | 823 | | 2070 | nd | | nd |
| Max | 8260 | | 7360 | 8260 | | 7360 | nd | | nd |
| n (Samp) | 87 | | 8 | 87 | | 8 | nd | | nd |
| n (Patient) | 87 | | 8 | 87 | | 8 | nd | | nd |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 3370 | | 4230 | 3370 | | 4230 | nd | | nd |
| Average | 3670 | | 4490 | 3670 | | 4490 | nd | | nd |
| Stdev | 1550 | | 2000 | 1550 | | 2000 | nd | | nd |
| p(t-test) | | | 0.22 | | | 0.22 | nd | | nd |
| Min | 823 | | 2070 | 823 | | 2070 | nd | | nd |
| Max | 8260 | | 7360 | 8260 | | 7360 | nd | | nd |
| n (Samp) | 80 | | 6 | 80 | | 6 | nd | | nd |
| n (Patient) | 80 | | 6 | 80 | | 6 | nd | | nd |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.63 | nd | 0.64 | 0.59 | nd | 0.64 | nd | nd | nd |
| SE | 0.11 | nd | 0.13 | 0.11 | nd | 0.13 | nd | nd | nd |
| p | 0.23 | nd | 0.25 | 0.43 | nd | 0.25 | nd | nd | nd |
| nCohort 1 | 87 | nd | 80 | 87 | nd | 80 | nd | nd | nd |
| nCohort 2 | 8 | nd | 6 | 8 | nd | 6 | nd | nd | nd |
| Cutoff 1 | 3050 | nd | 3090 | 3050 | nd | 3090 | nd | nd | nd |
| Sens 1 | 75% | nd | 83% | 75% | nd | 83% | nd | nd | nd |
| Spec 1 | 46% | nd | 48% | 46% | nd | 48% | nd | nd | nd |
| Cutoff 2 | 2400 | nd | 3090 | 2400 | nd | 3090 | nd | nd | nd |
| Sens 2 | 88% | nd | 83% | 88% | nd | 83% | nd | nd | nd |
| Spec 2 | 22% | nd | 48% | 22% | nd | 48% | nd | nd | nd |
| Cutoff 3 | 2040 | nd | 2040 | 2040 | nd | 2040 | nd | nd | nd |
| Sens 3 | 100% | nd | 100% | 100% | nd | 100% | nd | nd | nd |
| Spec 3 | 16% | nd | 15% | 16% | nd | 15% | nd | nd | nd |
| Cutoff 4 | 4330 | nd | 4220 | 4330 | nd | 4220 | nd | nd | nd |
| Sens 4 | 50% | nd | 50% | 38% | nd | 50% | nd | nd | nd |
| Spec 4 | 70% | nd | 70% | 70% | nd | 70% | nd | nd | nd |
| Cutoff 5 | 4990 | nd | 4990 | 4990 | nd | 4990 | nd | nd | nd |
| Sens 5 | 50% | nd | 50% | 38% | nd | 50% | nd | nd | nd |
| Spec 5 | 80% | nd | 80% | 80% | nd | 80% | nd | nd | nd |
| Cutoff 6 | 5650 | nd | 5590 | 5650 | nd | 5590 | nd | nd | nd |
| Sens 6 | 38% | nd | 33% | 25% | nd | 33% | nd | nd | nd |
| Spec 6 | 91% | nd | 90% | 91% | nd | 90% | nd | nd | nd |
| OR Quart 2 | 0.95 | nd | 2.0 | 0.95 | nd | 2.0 | nd | nd | nd |
| p Value | 0.96 | nd | 0.58 | 0.96 | nd | 0.58 | nd | nd | nd |
| 95% CI of | 0.12 | nd | 0.17 | 0.12 | nd | 0.17 | nd | nd | nd |
| OR Quart2 | 7.4 | nd | 24 | 7.4 | nd | 24 | nd | nd | nd |
| OR Quart 3 | 0 | nd | 0 | 0.46 | nd | 0 | nd | nd | nd |
| p Value | na | nd | na | 0.53 | nd | na | nd | nd | nd |
| 95% CI of | na | nd | na | 0.039 | nd | na | nd | nd | nd |
| OR Quart3 | na | nd | na | 5.4 | nd | na | nd | nd | nd |
| OR Quart 4 | 2.1 | nd | 3.2 | 1.5 | nd | 3.2 | nd | nd | nd |
| p Value | 0.42 | nd | 0.34 | 0.67 | nd | 0.34 | nd | nd | nd |
| 95% CI of | 0.35 | nd | 0.30 | 0.23 | nd | 0.30 | nd | nd | nd |
| OR Quart4 | 13 | nd | 33 | 9.9 | nd | 33 | nd | nd | nd |

| **Interleukin-11** | | | | | | | | | |
|---|---|---|---|---|---|---|---|---|---|
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 98.9 | | 101 | 98.9 | | 101 | 98.9 | | 90.7 |
| Average | 333 | | 576 | 333 | | 576 | 333 | | 129 |
| Stdev | 1130 | | 1530 | 1130 | | 1530 | 1130 | | 76.7 |
| p(t-test) | | | 0.52 | | | 0.52 | | | 0.64 |
| Min | 0.368 | | 31.8 | 0.368 | | 31.8 | 0.368 | | 38.4 |
| Max | 6920 | | 5420 | 6920 | | 5420 | 6920 | | 233 |
| n (Samp) | 65 | | 12 | 65 | | 12 | 65 | | 7 |
| n (Patient) | 65 | | 12 | 65 | | 12 | 65 | | 7 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 91.9 | | 83.6 | 91.9 | | 83.6 | nd | | nd |
| Average | 218 | | 143 | 218 | | 143 | nd | | nd |
| Stdev | 804 | | 136 | 804 | | 136 | nd | | nd |
| p(t-test) | | | 0.82 | | | 0.82 | nd | | nd |
| Min | 0.359 | | 31.8 | 0.359 | | 31.8 | nd | | nd |
| Max | 6920 | | 389 | 6920 | | 389 | nd | | nd |
| n (Samp) | 132 | | 6 | 132 | | 6 | nd | | nd |
| n (Patient) | 132 | | 6 | 132 | | 6 | nd | | nd |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 90.7 | | 101 | 90.7 | | 101 | 90.7 | | 83.7 |
| Average | 319 | | 778 | 319 | | 778 | 319 | | 114 |
| Stdev | 1140 | | 1880 | 1140 | | 1880 | 1140 | | 72.9 |
| p(t-test) | | | 0.33 | | | 0.33 | | | 0.67 |
| Min | 0.368 | | 51.1 | 0.368 | | 51.1 | 0.368 | | 38.4 |
| Max | 6920 | | 5420 | 6920 | | 5420 | 6920 | | 233 |
| n (Samp) | 64 | | 8 | 64 | | 8 | 64 | | 6 |
| n (Patient) | 64 | | 8 | 64 | | 8 | 64 | | 6 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.59 | 0.56 | 0.63 | 0.59 | 0.56 | 0.63 | 0.57 | nd | 0.57 |
| SE | 0.093 | 0.12 | 0.11 | 0.093 | 0.12 | 0.11 | 0.12 | nd | 0.13 |
| p | 0.31 | 0.63 | 0.24 | 0.31 | 0.63 | 0.24 | 0.55 | nd | 0.59 |
| nCohort 1 | 65 | 132 | 64 | 65 | 132 | 64 | 65 | nd | 64 |
| nCohort 2 | 12 | 6 | 8 | 12 | 6 | 8 | 7 | nd | 6 |
| Cutoff 1 | 67.2 | 51.1 | 76.5 | 67.2 | 51.1 | 76.5 | 76.5 | nd | 71.6 |
| Sens 1 | 75% | 83% | 75% | 75% | 83% | 75% | 71% | nd | 83% |
| Spec 1 | 43% | 39% | 47% | 43% | 39% | 47% | 43% | nd | 47% |
| Cutoff 2 | 51.1 | 51.1 | 71.6 | 51.1 | 51.1 | 71.6 | 67.2 | nd | 71.6 |
| Sens 2 | 83% | 83% | 88% | 83% | 83% | 88% | 86% | nd | 83% |
| Spec 2 | 38% | 39% | 47% | 38% | 39% | 47% | 43% | nd | 47% |
| Cutoff 3 | 47.4 | 29.7 | 47.4 | 47.4 | 29.7 | 47.4 | 33.9 | nd | 33.9 |
| Sens 3 | 92% | 100% | 100% | 92% | 100% | 100% | 100% | nd | 100% |
| Spec 3 | 38% | 23% | 41% | 38% | 23% | 41% | 32% | nd | 33% |
| Cutoff 4 | 159 | 158 | 155 | 159 | 158 | 155 | 159 | nd | 155 |
| Sens 4 | 42% | 33% | 38% | 42% | 33% | 38% | 43% | nd | 33% |
| Spec 4 | 71% | 73% | 70% | 71% | 73% | 70% | 71% | nd | 70% |
| Cutoff 5 | 209 | 196 | 190 | 209 | 196 | 190 | 209 | nd | 190 |
| Sens 5 | 33% | 33% | 25% | 33% | 33% | 25% | 29% | nd | 17% |
| Spec 5 | 80% | 81% | 81% | 80% | 81% | 81% | 80% | nd | 81% |
| Cutoff 6 | 370 | 261 | 306 | 370 | 261 | 306 | 370 | nd | 306 |
| Sens 6 | 17% | 17% | 12% | 17% | 17% | 12% | 0% | nd | 0% |
| Spec 6 | 91% | 90% | 91% | 91% | 90% | 91% | 91% | nd | 91% |
| OR Quart 2 | >8.8 | >4.4 | >5.1 | >8.8 | >4.4 | >5.1 | >5.1 | nd | >3.4 |
| p Value | <0.057 | <0.20 | <0.16 | <0.057 | <0.20 | <0.16 | <0.16 | nd | <0.31 |
| 95% CI of | >0.94 | >0.46 | >0.52 | >0.94 | >0.46 | >0.52 | >0.52 | nd | >0.32 |
| OR Quart2 | na | na | na | na | na | na | na | nd | na |
| OR Quart 3 | >2.2 | >0 | >2.2 | >2.2 | >0 | >2.2 | >1.1 | nd | >1.1 |
| p Value | <0.53 | <na | <0.52 | <0.53 | <na | <0.52 | <0.97 | nd | <0.97 |
| 95% CI of | >0.19 | >na | >0.19 | >0.19 | >na | >0.19 | >0.061 | nd | >0.061 |
| OR Quart3 | na | na | na | na | na | na | na | nd | na |
| OR Quart 4 | >4.8 | >2.1 | >2.2 | >4.8 | >2.1 | >2.2 | >2.2 | nd | >2.1 |
| p Value | <0.18 | <0.56 | <0.52 | <0.18 | <0.56 | <0.52 | <0.52 | nd | <0.55 |
| 95% CI of | >0.48 | >0.18 | >0.19 | >0.48 | >0.18 | >0.19 | >0.19 | nd | >0.18 |
| OR Quart4 | na | na | na | na | na | na | na | nd | na |

| | | | | | | | | | |
|---|---|---|---|---|---|---|---|---|---|
| **Interleukin-2 receptor alpha chain** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 22.9 | | 93.0 | 22.9 | | 85.8 | 22.9 | | 89.7 |
| Average | 45.2 | | 158 | 45.2 | | 152 | 45.2 | | 121 |
| Stdev | 56.9 | | 163 | 56.9 | | 166 | 56.9 | | 114 |
| p(t-test) | | | 3.7E-5 | | | 9.1E-5 | | | 0.0041 |
| Min | 0.0359 | | 5.03 | 0.0359 | | 5.03 | 0.0359 | | 12.5 |
| Max | 232 | | 533 | 232 | | 533 | 232 | | 352 |
| n (Samp) | 65 | | 12 | 65 | | 12 | 65 | | 7 |
| n (Patient) | 65 | | 12 | 65 | | 12 | 65 | | 7 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 28.1 | | 72.0 | 28.1 | | 47.9 | nd | | nd |
| Average | 80.9 | | 66.1 | 80.9 | | 55.7 | nd | | nd |
| Stdev | 146 | | 51.1 | 146 | | 50.1 | nd | | nd |
| p(t-test) | | | 0.81 | | | 0.67 | nd | | nd |
| Min | 0.0359 | | 5.03 | 0.0359 | | 5.03 | nd | | nd |
| Max | 1040 | | 139 | 1040 | | 139 | nd | | nd |
| n (Samp) | 132 | | 6 | 132 | | 6 | nd | | nd |
| n (Patient) | 132 | | 6 | 132 | | 6 | nd | | nd |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 38.4 | | 122 | 38.4 | | 122 | 38.4 | | 75.9 |
| Average | 55.1 | | 196 | 55.1 | | 196 | 55.1 | | 117 |
| Stdev | 59.7 | | 188 | 59.7 | | 188 | 59.7 | | 125 |
| p(t-test) | | | 1.9E-5 | | | 1.9E-5 | | | 0.032 |
| Min | 0.0359 | | 12.5 | 0.0359 | | 12.5 | 0.0359 | | 12.5 |
| Max | 232 | | 533 | 232 | | 533 | 232 | | 352 |
| n (Samp) | 64 | | 8 | 64 | | 8 | 64 | | 6 |
| n (Patient) | 64 | | 8 | 64 | | 8 | 64 | | 6 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.78 | 0.60 | 0.77 | 0.76 | 0.57 | 0.77 | 0.77 | nd | 0.69 |
| SE | 0.083 | 0.13 | 0.10 | 0.085 | 0.12 | 0.10 | 0.11 | nd | 0.12 |
| p | 8.5E-4 | 0.40 | 0.0085 | 0.0026 | 0.60 | 0.0085 | 0.013 | nd | 0.13 |
| nCohort 1 | 65 | 132 | 64 | 65 | 132 | 64 | 65 | nd | 64 |
| nCohort 2 | 12 | 6 | 8 | 12 | 6 | 8 | 7 | nd | 6 |
| Cutoff 1 | 61.5 | 10.6 | 61.5 | 33.6 | 10.6 | 61.5 | 61.5 | nd | 29.5 |
| Sens 1 | 75% | 83% | 75% | 75% | 83% | 75% | 71% | nd | 83% |
| Spec 1 | 74% | 36% | 66% | 57% | 36% | 66% | 74% | nd | 47% |
| Cutoff 2 | 29.5 | 10.6 | 29.5 | 29.5 | 10.6 | 29.5 | 29.5 | nd | 29.5 |
| Sens 2 | 83% | 83% | 88% | 83% | 83% | 88% | 86% | nd | 83% |
| Spec 2 | 57% | 36% | 47% | 57% | 36% | 47% | 57% | nd | 47% |
| Cutoff 3 | 10.6 | 4.75 | 10.6 | 10.6 | 4.75 | 10.6 | 10.6 | nd | 10.6 |
| Sens 3 | 92% | 100% | 100% | 92% | 100% | 100% | 100% | nd | 100% |
| Spec 3 | 42% | 28% | 33% | 42% | 28% | 33% | 42% | nd | 33% |
| Cutoff 4 | 51.4 | 68.3 | 74.5 | 51.4 | 68.3 | 74.5 | 51.4 | nd | 74.5 |
| Sens 4 | 75% | 50% | 62% | 67% | 33% | 62% | 71% | nd | 50% |
| Spec 4 | 71% | 70% | 70% | 71% | 70% | 70% | 71% | nd | 70% |
| Cutoff 5 | 89.4 | 132 | 105 | 89.4 | 132 | 105 | 89.4 | nd | 105 |
| Sens 5 | 58% | 17% | 50% | 50% | 17% | 50% | 57% | nd | 33% |
| Spec 5 | 80% | 80% | 81% | 80% | 80% | 81% | 80% | nd | 81% |
| Cutoff 6 | 133 | 203 | 148 | 133 | 203 | 148 | 133 | nd | 148 |
| Sens 6 | 42% | 0% | 50% | 42% | 0% | 50% | 43% | nd | 33% |
| Spec 6 | 91% | 90% | 91% | 91% | 90% | 91% | 91% | nd | 91% |
| OR Quart 2 | >2.2 | >2.1 | >2.2 | >2.2 | >2.1 | >2.2 | >1.1 | nd | >2.1 |
| p Value | <0.53 | <0.56 | <0.52 | <0.53 | <0.56 | <0.52 | <0.97 | nd | <0.55 |
| 95% CI of | >0.19 | >0.18 | >0.19 | >0.19 | >0.18 | >0.19 | >0.061 | nd | >0.18 |
| OR Quart2 | na | na | na | na | na | na | na | nd | na |
| OR Quart 3 | >3.6 | >2.1 | >2.2 | >5.1 | >3.3 | >2.2 | >2.2 | nd | >1.1 |
| p Value | <0.29 | <0.55 | <0.52 | <0.17 | <0.31 | <0.52 | <0.52 | nd | <0.97 |
| 95% CI of | >0.34 | >0.18 | >0.19 | >0.51 | >0.32 | >0.19 | >0.19 | nd | >0.061 |
| OR Quart3 | na | na | na | na | na | na | na | nd | na |
| OR Quart 4 | >10 | >2.1 | >5.1 | >8.1 | >1.0 | >5.1 | >5.1 | nd | >3.4 |
| p Value | <0.039 | <0.56 | <0.16 | <0.065 | <1.0 | <0.16 | <0.16 | nd | <0.31 |
| 95% CI of | >1.1 | >0.18 | >0.52 | >0.88 | >0.060 | >0.52 | >0.52 | nd | >0.32 |
| OR Quart4 | na | na | na | na | na | na | na | nd | na |

| **Neutrophil collagenase** | | | | | | | | | |
|---|---|---|---|---|---|---|---|---|---|
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 1220 | | 4220 | 1220 | | 4220 | nd | | nd |
| Average | 2280 | | 11600 | 2280 | | 11600 | nd | | nd |
| Stdev | 2900 | | 15500 | 2900 | | 15500 | nd | | nd |
| p(t-test) | | | 3.1E-6 | | | 3.2E-6 | nd | | nd |
| Min | 131 | | 21.8 | 131 | | 1.14 | nd | | nd |
| Max | 16000 | | 38700 | 16000 | | 38700 | nd | | nd |
| n (Samp) | 87 | | 8 | 87 | | 8 | nd | | nd |
| n (Patient) | 87 | | 8 | 87 | | 8 | nd | | nd |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 1190 | | 5900 | 1190 | | 5900 | nd | | nd |
| Average | 3030 | | 14800 | 3030 | | 14800 | nd | | nd |
| Stdev | 6790 | | 16900 | 6790 | | 16900 | nd | | nd |
| p(t-test) | | | 5.5E-4 | | | 5.5E-4 | nd | | nd |
| Min | 131 | | 1500 | 131 | | 1500 | nd | | nd |
| Max | 55000 | | 38700 | 55000 | | 38700 | nd | | nd |
| n (Samp) | 80 | | 6 | 80 | | 6 | nd | | nd |
| n (Patient) | 80 | | 6 | 80 | | 6 | nd | | nd |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.76 | nd | 0.86 | 0.76 | nd | 0.86 | nd | nd | nd |
| SE | 0.10 | nd | 0.098 | 0.10 | nd | 0.098 | nd | nd | nd |
| p | 0.012 | nd | 2.3E-4 | 0.012 | nd | 2.3E-4 | nd | nd | nd |
| nCohort 1 | 87 | nd | 80 | 87 | nd | 80 | nd | nd | nd |
| nCohort 2 | 8 | nd | 6 | 8 | nd | 6 | nd | nd | nd |
| Cutoff 1 | 2830 | nd | 2830 | 2830 | nd | 2830 | nd | nd | nd |
| Sens 1 | 75% | nd | 83% | 75% | nd | 83% | nd | nd | nd |
| Spec 1 | 77% | nd | 76% | 77% | nd | 76% | nd | nd | nd |
| Cutoff 2 | 1490 | nd | 2830 | 1490 | nd | 2830 | nd | nd | nd |
| Sens 2 | 88% | nd | 83% | 88% | nd | 83% | nd | nd | nd |
| Spec 2 | 62% | nd | 76% | 62% | nd | 76% | nd | nd | nd |
| Cutoff 3 | 0 | nd | 1490 | 0 | nd | 1490 | nd | nd | nd |
| Sens 3 | 100% | nd | 100% | 100% | nd | 100% | nd | nd | nd |
| Spec 3 | 0% | nd | 62% | 0% | nd | 62% | nd | nd | nd |
| Cutoff 4 | 2000 | nd | 2060 | 2000 | nd | 2060 | nd | nd | nd |
| Sens 4 | 75% | nd | 83% | 75% | nd | 83% | nd | nd | nd |
| Spec 4 | 70% | nd | 70% | 70% | nd | 70% | nd | nd | nd |
| Cutoff 5 | 3180 | nd | 3180 | 3180 | nd | 3180 | nd | nd | nd |
| Sens 5 | 62% | nd | 67% | 62% | nd | 67% | nd | nd | nd |
| Spec 5 | 80% | nd | 80% | 80% | nd | 80% | nd | nd | nd |
| Cutoff 6 | 5900 | nd | 5900 | 5900 | nd | 5900 | nd | nd | nd |
| Sens 6 | 38% | nd | 50% | 38% | nd | 50% | nd | nd | nd |
| Spec 6 | 91% | nd | 90% | 91% | nd | 90% | nd | nd | nd |
| OR Quart 2 | 0 | nd | >0 | 0 | nd | >0 | nd | nd | nd |
| p Value | na | nd | <na | na | nd | <na | nd | nd | nd |
| 95% CI of | na | nd | >na | na | nd | >na | nd | nd | nd |
| OR Quart2 | na | nd | na | na | nd | na | nd | nd | nd |
| OR Quart 3 | 2.0 | nd | >2.2 | 2.0 | nd | >2.2 | nd | nd | nd |
| p Value | 0.58 | nd | <0.53 | 0.58 | nd | <0.53 | nd | nd | nd |
| 95% CI of | 0.17 | nd | >0.19 | 0.17 | nd | >0.19 | nd | nd | nd |
| OR Quart3 | 24 | nd | na | 24 | nd | na | nd | nd | nd |
| OR Quart 4 | 5.8 | nd | >4.7 | 5.8 | nd | >4.7 | nd | nd | nd |
| p Value | 0.12 | nd | <0.19 | 0.12 | nd | <0.19 | nd | nd | nd |
| 95% CI of | 0.62 | nd | >0.48 | 0.62 | nd | >0.48 | nd | nd | nd |
| OR Quart4 | 54 | nd | na | 54 | nd | na | nd | nd | nd |

| | | | | | | | | | |
|---|---|---|---|---|---|---|---|---|---|
| **Protransforming growth factor alpha** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 2.51 | | 10.2 | 2.51 | | 10.2 | 2.51 | | 8.34 |
| Average | 8.67 | | 30.9 | 8.67 | | 30.9 | 8.67 | | 16.5 |
| Stdev | 35.8 | | 50.3 | 35.8 | | 50.4 | 35.8 | | 23.6 |
| p(t-test) | | | 0.068 | | | 0.069 | | | 0.57 |
| Min | 0.00305 | | 0.566 | 0.00305 | | 0.227 | 0.00305 | | 0.783 |
| Max | 287 | | 173 | 287 | | 173 | 287 | | 68.3 |
| n (Samp) | 65 | | 12 | 65 | | 12 | 65 | | 7 |
| n (Patient) | 65 | | 12 | 65 | | 12 | 65 | | 7 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 2.57 | | 4.38 | 2.57 | | 4.38 | nd | | nd |
| Average | 9.04 | | 14.3 | 9.04 | | 14.2 | nd | | nd |
| Stdev | 30.5 | | 24.0 | 30.5 | | 24.1 | nd | | nd |
| p(t-test) | | | 0.68 | | | 0.68 | nd | | nd |
| Min | 0.00305 | | 0.566 | 0.00305 | | 0.227 | nd | | nd |
| Max | 287 | | 62.6 | 287 | | 62.6 | nd | | nd |
| n (Samp) | 132 | | 6 | 132 | | 6 | nd | | nd |
| n (Patient) | 132 | | 6 | 132 | | 6 | nd | | nd |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 2.34 | | 12.3 | 2.34 | | 12.3 | 2.34 | | 10.2 |
| Average | 8.46 | | 38.0 | 8.46 | | 38.0 | 8.46 | | 19.0 |
| Stdev | 36.1 | | 58.4 | 36.1 | | 58.4 | 36.1 | | 24.9 |
| p(t-test) | | | 0.047 | | | 0.047 | | | 0.49 |
| Min | 0.00305 | | 0.783 | 0.00305 | | 0.783 | 0.00305 | | 0.783 |
| Max | 287 | | 173 | 287 | | 173 | 287 | | 68.3 |
| n (Samp) | 64 | | 8 | 64 | | 8 | 64 | | 6 |
| n (Patient) | 64 | | 8 | 64 | | 8 | 64 | | 6 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.74 | 0.63 | 0.85 | 0.73 | 0.62 | 0.85 | 0.75 | nd | 0.81 |
| SE | 0.087 | 0.12 | 0.088 | 0.088 | 0.12 | 0.088 | 0.11 | nd | 0.11 |
| p | 0.0060 | 0.28 | 6.8E-5 | 0.0088 | 0.34 | 6.8E-5 | 0.026 | nd | 0.0042 |
| nCohort 1 | 65 | 132 | 64 | 65 | 132 | 64 | 65 | nd | 64 |
| nCohort 2 | 12 | 6 | 8 | 12 | 6 | 8 | 7 | nd | 6 |
| Cutoff 1 | 2.57 | 1.80 | 7.01 | 2.57 | 1.80 | 7.01 | 5.39 | nd | 5.39 |
| Sens 1 | 75% | 83% | 75% | 75% | 83% | 75% | 71% | nd | 83% |
| Spec 1 | 52% | 42% | 89% | 52% | 42% | 89% | 86% | nd | 88% |
| Cutoff 2 | 1.80 | 1.80 | 5.39 | 1.80 | 1.80 | 5.39 | 1.80 | nd | 5.39 |
| Sens 2 | 83% | 83% | 88% | 83% | 83% | 88% | 86% | nd | 83% |
| Spec 2 | 40% | 42% | 88% | 40% | 42% | 88% | 40% | nd | 88% |
| Cutoff 3 | 0.775 | 0.540 | 0.775 | 0.775 | 0.137 | 0.775 | 0.775 | nd | 0.775 |
| Sens 3 | 92% | 100% | 100% | 92% | 100% | 100% | 100% | nd | 100% |
| Spec 3 | 25% | 20% | 30% | 25% | 11% | 30% | 25% | nd | 30% |
| Cutoff 4 | 3.59 | 4.18 | 3.36 | 3.59 | 4.18 | 3.36 | 3.59 | nd | 3.36 |
| Sens 4 | 67% | 50% | 88% | 67% | 50% | 88% | 71% | nd | 83% |
| Spec 4 | 71% | 70% | 70% | 71% | 70% | 70% | 71% | nd | 70% |
| Cutoff 5 | 4.80 | 5.28 | 4.36 | 4.80 | 5.28 | 4.36 | 4.80 | nd | 4.36 |
| Sens 5 | 67% | 50% | 88% | 67% | 50% | 88% | 71% | nd | 83% |
| Spec 5 | 80% | 80% | 81% | 80% | 80% | 81% | 80% | nd | 81% |
| Cutoff 6 | 7.33 | 13.7 | 9.25 | 7.33 | 13.7 | 9.25 | 7.33 | nd | 9.25 |
| Sens 6 | 58% | 17% | 62% | 58% | 17% | 62% | 57% | nd | 50% |
| Spec 6 | 91% | 90% | 91% | 91% | 90% | 91% | 91% | nd | 91% |
| OR Quart 2 | 1.0 | 2.0 | >1.1 | 1.0 | 2.0 | >1.1 | 1.0 | nd | >1.0 |
| p Value | 1.0 | 0.58 | <0.97 | 1.0 | 0.58 | <0.97 | 1.0 | nd | <1.0 |
| 95% CI of | 0.13 | 0.17 | >0.061 | 0.13 | 0.17 | >0.061 | 0.058 | nd | >0.058 |
| OR Quart2 | 7.9 | 23 | na | 7.9 | 23 | na | 17 | nd | na |
| OR Quart 3 | 0 | 0 | >0 | 0 | 0 | >0 | 0 | nd | >0 |
| p Value | na | na | <na | na | na | <na | na | nd | <na |
| 95% CI of | na | na | >na | na | na | >na | na | nd | >na |
| OR Quart3 | na | na | na | na | na | na | na | nd | na |
| OR Quart 4 | 5.7 | 3.1 | >11 | 5.7 | 3.1 | >11 | 6.5 | nd | >6.5 |
| p Value | 0.048 | 0.34 | <0.032 | 0.048 | 0.34 | <0.032 | 0.10 | nd | <0.10 |
| 95% CI of | 1.0 | 0.31 | >1.2 | 1.0 | 0.31 | >1.2 | 0.68 | nd | >0.68 |
| OR Quart4 | 32 | 31 | na | 32 | 31 | na | 63 | nd | na |

| | | | | | | | | | |
|---|---|---|---|---|---|---|---|---|---|
| **CA 15-3** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 0.245 | | 0.833 | 0.245 | | 0.644 | nd | | nd |
| Average | 0.500 | | 1.04 | 0.500 | | 0.953 | nd | | nd |
| Stdev | 0.916 | | 0.811 | 0.916 | | 0.817 | nd | | nd |
| p(t-test) | | | 0.11 | | | 0.18 | nd | | nd |
| Min | 0.0328 | | 0.249 | 0.0328 | | 0.249 | nd | | nd |
| Max | 6.54 | | 2.69 | 6.54 | | 2.69 | nd | | nd |
| n (Samp) | 87 | | 8 | 87 | | 8 | nd | | nd |
| n (Patient) | 87 | | 8 | 87 | | 8 | nd | | nd |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 0.289 | | 0.588 | 0.289 | | 0.588 | nd | | nd |
| Average | 0.616 | | 1.01 | 0.616 | | 1.01 | nd | | nd |
| Stdev | 1.07 | | 0.952 | 1.07 | | 0.952 | nd | | nd |
| p(t-test) | | | 0.39 | | | 0.39 | nd | | nd |
| Min | 0.0328 | | 0.249 | 0.0328 | | 0.249 | nd | | nd |
| Max | 6.54 | | 2.69 | 6.54 | | 2.69 | nd | | nd |
| n (Samp) | 80 | | 6 | 80 | | 6 | nd | | nd |
| n (Patient) | 80 | | 6 | 80 | | 6 | nd | | nd |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.82 | nd | 0.75 | 0.81 | nd | 0.75 | nd | nd | nd |
| SE | 0.093 | nd | 0.12 | 0.094 | nd | 0.12 | nd | nd | nd |
| p | 5.7E-4 | nd | 0.039 | 8.9E-4 | nd | 0.039 | nd | nd | nd |
| nCohort 1 | 87 | nd | 80 | 87 | nd | 80 | nd | nd | nd |
| nCohort 2 | 8 | nd | 6 | 8 | nd | 6 | nd | nd | nd |
| Cutoff 1 | 0.476 | nd | 0.346 | 0.476 | nd | 0.346 | nd | nd | nd |
| Sens 1 | 75% | nd | 83% | 75% | nd | 83% | nd | nd | nd |
| Spec 1 | 83% | nd | 59% | 83% | nd | 59% | nd | nd | nd |
| Cutoff 2 | 0.326 | nd | 0.346 | 0.326 | nd | 0.346 | nd | nd | nd |
| Sens 2 | 88% | nd | 83% | 88% | nd | 83% | nd | nd | nd |
| Spec 2 | 68% | nd | 59% | 68% | nd | 59% | nd | nd | nd |
| Cutoff 3 | 0.248 | nd | 0.248 | 0.248 | nd | 0.248 | nd | nd | nd |
| Sens 3 | 100% | nd | 100% | 100% | nd | 100% | nd | nd | nd |
| Spec 3 | 54% | nd | 46% | 54% | nd | 46% | nd | nd | nd |
| Cutoff 4 | 0.353 | nd | 0.402 | 0.353 | nd | 0.402 | nd | nd | nd |
| Sens 4 | 75% | nd | 67% | 75% | nd | 67% | nd | nd | nd |
| Spec 4 | 70% | nd | 70% | 70% | nd | 70% | nd | nd | nd |
| Cutoff 5 | 0.466 | nd | 0.708 | 0.466 | nd | 0.708 | nd | nd | nd |
| Sens 5 | 75% | nd | 33% | 75% | nd | 33% | nd | nd | nd |
| Spec 5 | 80% | nd | 80% | 80% | nd | 80% | nd | nd | nd |
| Cutoff 6 | 1.28 | nd | 1.57 | 1.28 | nd | 1.57 | nd | nd | nd |
| Sens 6 | 25% | nd | 33% | 25% | nd | 33% | nd | nd | nd |
| Spec 6 | 91% | nd | 90% | 91% | nd | 90% | nd | nd | nd |
| OR Quart 2 | >0 | nd | >1.0 | >0 | nd | >1.0 | nd | nd | nd |
| p Value | <na | nd | <1.0 | <na | nd | <1.0 | nd | nd | nd |
| 95% CI of | >na | nd | >0.059 | >na | nd | >0.059 | nd | nd | nd |
| OR Quart2 | na | nd | na | na | nd | na | nd | nd | nd |
| OR Quart 3 | >2.1 | nd | >1.0 | >2.1 | nd | >1.0 | nd | nd | nd |
| p Value | <0.56 | nd | <0.97 | <0.56 | nd | <0.97 | nd | nd | nd |
| 95% CI of | >0.18 | nd | >0.061 | >0.18 | nd | >0.061 | nd | nd | nd |
| OR Quart3 | na | nd | na | na | nd | na | nd | nd | nd |
| OR Quart 4 | >77 | nd | >4.7 | >7.7 | nd | >4.7 | nd | nd | nd |
| p Value | <0.070 | nd | <0.19 | <0.070 | nd | <0.19 | nd | nd | nd |
| 95% CI of | >0.85 | nd | >0.48 | >0.85 | nd | >0.48 | nd | nd | nd |
| OR Quart4 | na | nd | na | na | nd | na | nd | nd | nd |

**Table 9: Comparison of marker levels in urine samples collected from Cohort 1 (patients that did not progress beyond RIFLE stage 0, R, or I) and in urine samples collected from Cohort 2 (subjects who progress to RIFLE stage F) at 0, 24 hours, and 48 hours prior to the subject reaching RIFLE stage I.**

| **C-C motif chemokine 18** | | | | | | | | | |
|---|---|---|---|---|---|---|---|---|---|
| sCr or UO | 0hr prior to AKI stage 24hr prior to AKI stage 48hr prior to AKI stage | | | | | | | | |
| | Cohort 1 | | Cohort 2 | Cohort 1 | | Cohort 2 | Cohort 1 | | Cohort 2 |
| Median | 0.474 | | 2.53 | 0.474 | | 3.21 | 0.474 | | 0.628 |
| Average | 2.27 | | 6.02 | 2.27 | | 10.9 | 2.27 | | 12.6 |
| Stdev | 6.63 | | 9.81 | 6.63 | | 14.5 | 6.63 | | 18.9 |
| p(t-test) | | | 0.0092 | | | 2.1E-8 | | | 1.7E-6 |
| Min | 3.13E-5 | | 0.0680 | 3.13E-5 | | 0.138 | 3.13E-5 | | 0.00268 |
| Max | 40.0 | | 40.0 | 40.0 | | 40.0 | 40.0 | | 40.0 |
| n (Samp) | 1273 | | 22 | 1273 | | 20 | 1273 | | 10 |
| n (Patient) | 451 | | 22 | 451 | | 20 | 451 | | 10 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 0.490 | | 4.14 | nd | | nd | nd | | nd |
| Average | 2.63 | | 9.65 | nd | | nd | nd | | nd |
| Stdev | 7.46 | | 10.8 | nd | | nd | nd | | nd |
| p(t-test) | | | 0.0082 | nd | | nd | nd | | nd |
| Min | 3.13E-5 | | 0.204 | nd | | nd | nd | | nd |
| Max | 40.0 | | 31.3 | nd | | nd | nd | | nd |
| n (Samp) | 1337 | | 8 | nd | | nd | nd | | nd |
| n (Patient) | 466 | | 8 | nd | | nd | nd | | nd |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 0.490 | | 2.16 | 0.490 | | 3.58 | 0.490 | | 0.724 |
| Average | 2.46 | | 8.94 | 2.46 | | 13.1 | 2.46 | | 17.3 |
| Stdev | 6.94 | | 14.1 | 6.94 | | 16.2 | 6.94 | | 21.2 |
| p(t-test) | | | 6.6E-4 | | | 2.3E-10 | | | 4.2E-8 |
| Min | 3.13E-5 | | 0.0680 | 3.13E-5 | | 0.138 | 3.13E-5 | | 0.119 |
| Max | 40.0 | | 40.0 | 40.0 | | 40.0 | 40.0 | | 40.0 |
| n (Samp) | 1119 | | 14 | 1119 | | 19 | 1119 | | 7 |
| n (Patient) | 361 | | 14 | 361 | | 19 | 361 | | 7 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.72 | 0.83 | 0.71 | 0.78 | nd | 0.79 | 0.56 | nd | 0.58 |
| SE | 0.062 | 0.090 | 0.079 | 0.062 | nd | 0.062 | 0.094 | nd | 0.11 |
| p | 4.1E-4 | 2.4E-4 | 0.0090 | 7.2E-6 | nd | 2.8E-6 | 0.53 | nd | 0.48 |
| nCohort 1 | 1273 | 1337 | 1119 | 1273 | nd | 1119 | 1273 | nd | 1119 |
| nCohort 2 | 22 | 8 | 14 | 20 | nd | 19 | 10 | nd | 7 |
| Cutoff 1 | 0.862 | 2.72 | 0.862 | 1.98 | nd | 1.98 | 0.131 | nd | 0.131 |
| Sens 1 | 73% | 75% | 71% | 70% | nd | 74% | 70% | nd | 71% |
| Spec 1 | 65% | 85% | 63% | 83% | nd | 81% | 19% | nd | 17% |
| Cutoff 2 | 0.351 | 2.33 | 0.232 | 0.788 | nd | 0.788 | 0.125 | nd | 0.125 |
| Sens 2 | 82% | 88% | 86% | 80% | nd | 84% | 80% | nd | 86% |
| Spec 2 | 43% | 83% | 29% | 63% | nd | 62% | 18% | nd | 17% |
| Cutoff 3 | 0.204 | 0.204 | 0.164 | 0.270 | nd | 0.142 | 0.118 | nd | 0.118 |
| Sens 3 | 91% | 100% | 93% | 90% | nd | 95% | 90% | nd | 100% |
| Spec 3 | 27% | 27% | 22% | 36% | nd | 19% | 18% | nd | 16% |
| Cutoff 4 | 1.04 | 1.10 | 1.12 | 1.04 | nd | 1.12 | 1.04 | nd | 1.12 |
| Sens 4 | 68% | 88% | 64% | 75% | nd | 79% | 40% | nd | 43% |
| Spec 4 | 70% | 70% | 70% | 70% | nd | 70% | 70% | nd | 70% |
| Cutoff 5 | 1.76 | 1.90 | 1.90 | 1.76 | nd | 1.90 | 1.76 | nd | 1.90 |
| Sens 5 | 55% | 88% | 50% | 70% | nd | 74% | 40% | nd | 43% |
| Spec 5 | 80% | 80% | 80% | 80% | nd | 80% | 80% | nd | 80% |
| Cutoff 6 | 3.72 | 4.03 | 3.96 | 3.72 | nd | 3.96 | 3.72 | nd | 3.96 |
| Sens 6 | 36% | 50% | 43% | 45% | nd | 47% | 40% | nd | 43% |
| Spec 6 | 90% | 90% | 90% | 90% | nd | 90% | 90% | nd | 90% |
| OR Quart 2 | 1.5 | >1.0 | 0.50 | 1.0 | nd | 0.50 | 0 | nd | 0 |
| p Value | 0.66 | <1.00 | 0.57 | 1.0 | nd | 0.57 | na | nd | na |
| 95% CI of | 0.25 | >0.062 | 0.045 | 0.14 | nd | 0.045 | na | nd | na |
| OR Quart2 | 9.0 | na | 5.5 | 7.1 | nd | 5.5 | na | nd | na |
| OR Quart 3 | 2.0 | >0 | 1.5 | 0.50 | nd | 0.50 | 0.50 | nd | 0.33 |
| p Value | 0.42 | <na | 0.66 | 0.57 | nd | 0.57 | 0.42 | nd | 0.34 |
| 95% CI of | 0.36 | >na | 0.25 | 0.045 | nd | 0.045 | 0.090 | nd | 0.034 |
| OR Quart3 | 11 | na | 9.1 | 5.5 | nd | 5.5 | 2.7 | nd | 3.2 |
| OR Quart 4 | 6.7 | >7.1 | 4.1 | 7.8 | nd | 7.8 | 1.00 | nd | 1.00 |
| p Value | 0.013 | <0.067 | 0.077 | 0.0067 | nd | 0.0066 | 1.00 | nd | 1.00 |
| 95% CI of | 1.5 | >0.87 | 0.86 | 1.8 | nd | 1.8 | 0.25 | nd | 0.20 |
| OR Quart4 | 30 | na | 19 | 34 | nd | 35 | 4.0 | nd | 5.0 |

| | | | | | | | | | |
|---|---|---|---|---|---|---|---|---|---|
| **C-C motif chemokine 24** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 12.9 | | 39.0 | 12.9 | | 34.2 | 12.9 | | 14.0 |
| Average | 28.6 | | 216 | 28.6 | | 269 | 28.6 | | 127 |
| Stdev | 58.5 | | 474 | 58.5 | | 594 | 58.5 | | 368 |
| p(t-test) | | | 1.8E-24 | | | 2.4E-29 | | | 2.6E-6 |
| Min | 0.0120 | | 5.58 | 0.0120 | | 0.0347 | 0.0120 | | 0.0635 |
| Max | 1090 | | 1930 | 1090 | | 2380 | 1090 | | 1170 |
| n (Samp) | 1275 | | 22 | 1275 | | 20 | 1275 | | 10 |
| n (Patient) | 452 | | 22 | 452 | | 20 | 452 | | 10 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 13.7 | | 57.3 | nd | | nd | nd | | nd |
| Average | 42.1 | | 88.9 | nd | | nd | nd | | nd |
| Stdev | 152 | | 118 | nd | | nd | nd | | nd |
| p(t-test) | | | 0.38 | nd | | nd | nd | | nd |
| Min | 0.0120 | | 11.1 | nd | | nd | nd | | nd |
| Max | 2790 | | 373 | nd | | nd | nd | | nd |
| n (Samp) | 1339 | | 8 | nd | | nd | nd | | nd |
| n (Patient) | 467 | | 8 | nd | | nd | nd | | nd |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 13.2 | | 56.4 | 13.2 | | 37.2 | 13.2 | | 14.8 |
| Average | 29.6 | | 391 | 29.6 | | 384 | 29.6 | | 178 |
| Stdev | 60.6 | | 769 | 60.6 | | 710 | 60.6 | | 439 |
| p(t-test) | | | 6.3E-37 | | | 1.8E-42 | | | 1.3E-8 |
| Min | 0.0120 | | 5.58 | 0.0120 | | 0.0347 | 0.0120 | | 4.32 |
| Max | 1090 | | 2790 | 1090 | | 2380 | 1090 | | 1170 |
| n (Samp) | 1122 | | 14 | 1122 | | 19 | 1122 | | 7 |
| n (Patient) | 362 | | 14 | 362 | | 19 | 362 | | 7 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.73 | 0.79 | 0.76 | 0.71 | nd | 0.76 | 0.50 | nd | 0.53 |
| SE | 0.062 | 0.096 | 0.075 | 0.066 | nd | 0.065 | 0.092 | nd | 0.11 |
| p | 1.7E-4 | 0.0031 | 6.2E-4 | 0.0012 | nd | 6.7E-5 | 1.00 | nd | 0.77 |
| nCohort 1 | 1275 | 1339 | 1122 | 1275 | nd | 1122 | 1275 | nd | 1122 |
| nCohort 2 | 22 | 8 | 14 | 20 | nd | 19 | 10 | nd | 7 |
| Cutoff 1 | 21.9 | 25.9 | 22.5 | 27.0 | nd | 27.2 | 6.94 | nd | 6.94 |
| Sens 1 | 73% | 75% | 71% | 70% | nd | 74% | 70% | nd | 71% |
| Spec 1 | 65% | 68% | 66% | 71% | nd | 71% | 37% | nd | 36% |
| Cutoff 2 | 9.98 | 21.9 | 8.63 | 14.2 | nd | 16.6 | 4.59 | nd | 4.59 |
| Sens 2 | 82% | 88% | 86% | 80% | nd | 84% | 80% | nd | 86% |
| Spec 2 | 44% | 63% | 40% | 53% | nd | 57% | 30% | nd | 29% |
| Cutoff 3 | 8.65 | 11.1 | 5.84 | 3.78 | nd | 3.78 | 4.24 | nd | 4.24 |
| Sens 3 | 91% | 100% | 93% | 90% | nd | 95% | 90% | nd | 100% |
| Spec 3 | 42% | 45% | 32% | 29% | nd | 28% | 30% | nd | 28% |
| Cutoff 4 | 26.4 | 28.1 | 26.7 | 26.4 | nd | 26.7 | 26.4 | nd | 26.7 |
| Sens 4 | 59% | 62% | 64% | 70% | nd | 79% | 10% | nd | 14% |
| Spec 4 | 70% | 70% | 70% | 70% | nd | 70% | 70% | nd | 70% |
| Cutoff 5 | 40.3 | 42.2 | 40.9 | 40.3 | nd | 40.9 | 40.3 | nd | 40.9 |
| Sens 5 | 50% | 62% | 64% | 40% | nd | 47% | 10% | nd | 14% |
| Spec 5 | 80% | 80% | 80% | 80% | nd | 80% | 80% | nd | 80% |
| Cutoff 6 | 69.6 | 78.2 | 72.3 | 69.6 | nd | 72.3 | 69.6 | nd | 72.3 |
| Sens 6 | 32% | 38% | 43% | 35% | nd | 37% | 10% | nd | 14% |
| Spec 6 | 90% | 90% | 90% | 90% | nd | 90% | 90% | nd | 90% |
| OR Quart 2 | >6.1 | >1.0 | >4.1 | 1.00 | nd | 2.0 | 3.0 | nd | >3.0 |
| p Value | <0.095 | <1.0 | <0.21 | 1.00 | nd | 0.57 | 0.34 | nd | <0.34 |
| 95% CI of | >0.73 | >0.062 | >0.45 | 0.14 | nd | 0.18 | 0.31 | nd | >0.31 |
| OR Quart2 | na | na | na | 7.1 | nd | 22 | 29 | nd | na |
| OR Quart 3 | >5.1 | >2.0 | >1.0 | 3.0 | nd | 5.1 | 5.1 | nd | >3.0 |
| p Value | <0.14 | <0.57 | <1.00 | 0.18 | nd | 0.14 | 0.14 | nd | <0.34 |
| 95% CI of | >0.59 | >0.18 | >0.062 | 0.61 | nd | 0.59 | 0.59 | nd | >0.31 |
| OR Quart3 | na | na | na | 15 | nd | 44 | 44 | nd | na |
| OR Quart 4 | >11 | >5.1 | >9.3 | 5.1 | nd | 11 | 1.00 | nd | >1.0 |
| p Value | <0.020 | <0.14 | <0.035 | 0.036 | nd | 0.020 | 1.00 | nd | <1.0 |
| 95% CI of | >1.5 | >0.59 | >1.2 | 1.1 | nd | 1.5 | 0.062 | nd | >0.062 |
| OR Quart4 | na | na | na | 24 | nd | 89 | 16 | nd | na |

| | | | | | | | | | |
|---|---|---|---|---|---|---|---|---|---|
| **C-C motif chemokine 8** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 1.36 | | 23.8 | 1.36 | | 10.0 | 1.36 | | 1.61 |
| Average | 9.75 | | 211 | 9.75 | | 42.0 | 9.75 | | 16.5 |
| Stdev | 26.6 | | 775 | 26.6 | | 70.5 | 26.6 | | 34.1 |
| p(t-test) | | | 1.7E-19 | | | 2.9E-7 | | | 0.43 |
| Min | 0.0250 | | 0.206 | 0.0250 | | 0.0945 | 0.0250 | | 0.0472 |
| Max | 473 | | 3670 | 473 | | 294 | 473 | | 111 |
| n (Samp) | 1275 | | 22 | 1275 | | 20 | 1275 | | 10 |
| n (Patient) | 452 | | 22 | 452 | | 20 | 452 | | 10 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 1.42 | | 16.3 | nd | | nd | nd | | nd |
| Average | 14.1 | | 29.6 | nd | | nd | nd | | nd |
| Stdev | 105 | | 40.1 | nd | | nd | nd | | nd |
| p(t-test) | | | 0.68 | nd | | nd | nd | | nd |
| Min | 0.0250 | | 0.206 | nd | | nd | nd | | nd |
| Max | 3670 | | 116 | nd | | nd | nd | | nd |
| n (Samp) | 1339 | | 8 | nd | | nd | nd | | nd |
| n (Patient) | 467 | | 8 | nd | | nd | nd | | nd |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 1.36 | | 31.3 | 1.36 | | 30.8 | 1.36 | | 2.77 |
| Average | 10.1 | | 59.3 | 10.1 | | 51.5 | 10.1 | | 20.2 |
| Stdev | 27.7 | | 66.2 | 27.7 | | 71.6 | 27.7 | | 40.5 |
| p(t-test) | | | 2.0E-10 | | | 9.3E-10 | | | 0.34 |
| Min | 0.0250 | | 0.829 | 0.0250 | | 0.136 | 0.0250 | | 0.0472 |
| Max | 473 | | 176 | 473 | | 294 | 473 | | 111 |
| n (Samp) | 1122 | | 14 | 1122 | | 19 | 1122 | | 7 |
| n (Patient) | 362 | | 14 | 362 | | 19 | 362 | | 7 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.77 | 0.71 | 0.81 | 0.73 | nd | 0.79 | 0.52 | nd | 0.55 |
| SE | 0.059 | 0.10 | 0.071 | 0.065 | nd | 0.062 | 0.093 | nd | 0.11 |
| p | 3.7E-6 | 0.046 | 1.4E-5 | 4.2E-4 | nd | 3.7E-6 | 0.79 | nd | 0.68 |
| nCohort 1 | 1275 | 1339 | 1122 | 1275 | nd | 1122 | 1275 | nd | 1122 |
| nCohort 2 | 22 | 8 | 14 | 20 | nd | 19 | 10 | nd | 7 |
| Cutoff 1 | 2.74 | 2.74 | 12.6 | 3.20 | nd | 8.37 | 0.240 | nd | 0.438 |
| Sens 1 | 77% | 75% | 71% | 70% | nd | 74% | 70% | nd | 71% |
| Spec 1 | 56% | 55% | 82% | 60% | nd | 74% | 34% | nd | 39% |
| Cutoff 2 | 0.802 | 0.491 | 0.802 | 1.21 | nd | 1.21 | 0.127 | nd | 0.127 |
| Sens 2 | 91% | 88% | 100% | 80% | nd | 84% | 80% | nd | 86% |
| Spec 2 | 44% | 38% | 44% | 49% | nd | 49% | 21% | nd | 20% |
| Cutoff 3 | 0.802 | 0.154 | 0.802 | 0.491 | nd | 0.491 | 0.0705 | nd | 0.0250 |
| Sens 3 | 91% | 100% | 100% | 90% | nd | 95% | 90% | nd | 100% |
| Spec 3 | 44% | 26% | 44% | 40% | nd | 39% | 11% | nd | 2% |
| Cutoff 4 | 6.78 | 7.38 | 6.78 | 6.78 | nd | 6.78 | 6.78 | nd | 6.78 |
| Sens 4 | 68% | 62% | 71% | 65% | nd | 74% | 40% | nd | 43% |
| Spec 4 | 70% | 70% | 70% | 70% | nd | 70% | 70% | nd | 70% |
| Cutoff 5 | 11.5 | 12.5 | 11.8 | 11.5 | nd | 11.8 | 11.5 | nd | 11.8 |
| Sens 5 | 64% | 50% | 71% | 45% | nd | 58% | 40% | nd | 29% |
| Spec 5 | 80% | 80% | 80% | 80% | nd | 80% | 80% | nd | 80% |
| Cutoff 6 | 22.8 | 27.7 | 23.0 | 22.8 | nd | 23.0 | 22.8 | nd | 23.0 |
| Sens 6 | 50% | 25% | 57% | 45% | nd | 58% | 10% | nd | 14% |
| Spec 6 | 90% | 90% | 90% | 90% | nd | 90% | 90% | nd | 90% |
| OR Quart 2 | >5.1 | >2.0 | >3.0 | 1.00 | nd | 2.0 | 0.66 | nd | 0.50 |
| p Value | <0.14 | <0.57 | <0.34 | 1.00 | nd | 0.57 | 0.66 | nd | 0.57 |
| 95% CI of | >0.59 | >0.18 | >0.31 | 0.14 | nd | 0.18 | 0.11 | nd | 0.045 |
| OR Quart2 | na | na | na | 7.1 | nd | 22 | 4.0 | nd | 5.5 |
| OR Quart 3 | >3.0 | >2.0 | >1.0 | 2.5 | nd | 3.0 | 0.33 | nd | 0.50 |
| p Value | <0.34 | <0.57 | <1.00 | 0.27 | nd | 0.34 | 0.34 | nd | 0.57 |
| 95% CI of | >0.31 | >0.18 | >0.062 | 0.48 | nd | 0.31 | 0.034 | nd | 0.045 |
| OR Quart3 | na | na | na | 13 | nd | 29 | 3.2 | nd | 5.5 |
| OR Quart 4 | >15 | >4.0 | >10 | 5.6 | nd | 14 | 1.3 | nd | 1.5 |
| p Value | <0.0098 | <0.21 | <0.026 | 0.025 | nd | 0.012 | 0.71 | nd | 0.66 |
| 95% CI of | >1.9 | >0.45 | >1.3 | 1.2 | nd | 1.8 | 0.30 | nd | 0.25 |
| OR Quart4 | na | na | na | 26 | nd | 100 | 6.0 | nd | 9.0 |

| | | | | | | | | | |
|---|---|---|---|---|---|---|---|---|---|
| **Cathepsin D** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 75900 | | 115000 | 75900 | | 114000 | 75900 | | 101000 |
| Average | 81800 | | 121000 | 81800 | | 117000 | 81800 | | 95500 |
| Stdev | 41500 | | 43500 | 41500 | | 43600 | 41500 | | 52800 |
| p(t-test) | | | 1.2E-5 | | | 1.4E-4 | | | 0.30 |
| Min | 656 | | 63100 | 656 | | 45000 | 656 | | 4320 |
| Max | 200000 | | 200000 | 200000 | | 200000 | 200000 | | 200000 |
| n (Samp) | 1273 | | 22 | 1273 | | 20 | 1273 | | 10 |
| n (Patient) | 451 | | 22 | 451 | | 20 | 451 | | 10 |
| | | | | | | | | | |

| sCr only | 0hr prior to AKI stage | | | 24hr prior to AKI stage | | | 24hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 78100 | | 99300 | nd | | nd | nd | | nd |
| Average | 84000 | | 109000 | nd | | nd | nd | | nd |
| Stdev | 43000 | | 32700 | nd | | nd | nd | | nd |
| p(t-test) | | | 0.10 | nd | | nd | nd | | nd |
| Min | 656 | | 71400 | nd | | nd | nd | | nd |
| Max | 200000 | | 151000 | nd | | nd | nd | | nd |
| n (Samp) | 1337 | | 8 | nd | | nd | nd | | nd |
| n (Patient) | 466 | | 8 | nd | | nd | nd | | nd |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 78500 | | 132000 | 78500 | | 117000 | 78500 | | 99800 |
| Average | 84100 | | 128000 | 84100 | | 123000 | 84100 | | 104000 |
| Stdev | 41200 | | 47600 | 41200 | | 46300 | 41200 | | 51000 |
| p(t-test) | | | 7.8E-5 | | | 4.2E-5 | | | 0.21 |
| Min | 2520 | | 63100 | 2520 | | 45000 | 2520 | | 54900 |
| Max | 200000 | | 200000 | 200000 | | 200000 | 200000 | | 200000 |
| n(Samp) | 1119 | | 14 | 1119 | | 19 | 1119 | | 7 |
| n (Patient) | 361 | | 14 | 361 | | 19 | 361 | | 7 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.75 | 0.69 | 0.76 | 0.73 | nd | 0.74 | 0.59 | nd | 0.61 |
| SE | 0.061 | 0.10 | 0.075 | 0.065 | nd | 0.066 | 0.095 | nd | 0.11 |
| p | 5.9E-5 | 0.063 | 6.5E-4 | 3.8E-4 | nd | 2.7E-4 | 0.34 | nd | 0.36 |
| nCohort 1 | 1273 | 1337 | 1119 | 1273 | nd | 1119 | 1273 | nd | 1119 |
| nCohort 2 | 22 | 8 | 14 | 20 | nd | 19 | 10 | nd | 7 |
| Cutoff 1 | 84700 | 89600 | 89600 | 90800 | nd | 90800 | 66900 | nd | 66900 |
| Sens 1 | 73% | 75% | 71% | 70% | nd | 74% | 70% | nd | 71% |
| Spec 1 | 58% | 60% | 61% | 64% | nd | 62% | 40% | nd | 37% |
| Cutoff 2 | 76300 | 73800 | 76300 | 84400 | nd | 84400 | 63400 | nd | 63400 |
| Sens 2 | 82% | 88% | 86% | 80% | nd | 84% | 80% | nd | 86% |
| Spec 2 | 50% | 46% | 48% | 58% | nd | 56% | 36% | nd | 33% |
| Cutoff 3 | 71400 | 71400 | 70900 | 73700 | nd | 65500 | 54900 | nd | 54900 |
| Sens 3 | 91% | 100% | 93% | 90% | nd | 95% | 90% | nd | 100% |
| Spec 3 | 45% | 43% | 42% | 47% | nd | 35% | 27% | nd | 24% |
| Cutoff 4 | 101000 | 104000 | 103000 | 101000 | nd | 103000 | 101000 | nd | 103000 |
| Sens 4 | 59% | 50% | 64% | 60% | nd | 63% | 50% | nd | 43% |
| Spec 4 | 70% | 70% | 70% | 70% | nd | 70% | 70% | nd | 70% |
| Cutoff 5 | 116000 | 119000 | 118000 | 116000 | nd | 118000 | 116000 | nd | 118000 |
| Sens 5 | 50% | 38% | 57% | 45% | nd | 47% | 30% | nd | 29% |
| Spec 5 | 80% | 80% | 80% | 80% | nd | 80% | 80% | nd | 80% |
| Cutoff 6 | 137000 | 144000 | 140000 | 137000 | nd | 140000 | 137000 | nd | 140000 |
| Sens 6 | 41% | 25% | 50% | 25% | nd | 26% | 10% | nd | 14% |
| Spec 6 | 90% | 90% | 90% | 90% | nd | 90% | 90% | nd | 90% |
| OR Quart 2 | >5.1 | >2.0 | >3.0 | 2.0 | nd | 2.0 | 3.0 | nd | 2.0 |
| p Value | <0.14 | <0.57 | <0.34 | 0.57 | nd | 0.57 | 0.34 | nd | 0.57 |
| 95% CI of | >0.59 | >0.18 | >0.31 | 0.18 | nd | 0.18 | 0.31 | nd | 0.18 |
| OR Quart2 | na | na | na | 22 | nd | 22 | 29 | nd | 22 |
| OR Quart 3 | >4.0 | >3.0 | >3.0 | 5.1 | nd | 5.1 | 3.0 | nd | 2.0 |
| p Value | <0.21 | <0.34 | <0.34 | 0.14 | nd | 0.14 | 0.34 | nd | 0.57 |
| 95% CI of | >0.45 | >0.31 | >0.31 | 0.59 | nd | 0.59 | 0.31 | nd | 0.18 |
| OR Quart3 | na | na | na | 44 | nd | 44 | 29 | nd | 22 |
| OR Quart 4 | >14 | >3.0 | >8.2 | 12 | nd | 11 | 3.0 | nd | 2.0 |
| p Value | <0.012 | <0.34 | <0.048 | 0.016 | nd | 0.020 | 0.34 | nd | 0.57 |
| 95% CI of | >1.8 | >0.31 | >1.0 | 1.6 | nd | 1.5 | 0.31 | nd | 0.18 |
| OR Quart4 | na | na | na | 96 | nd | 89 | 29 | nd | 22 |

| | | | | | | | | | |
|---|---|---|---|---|---|---|---|---|---|
| **C-X-C motif chemokine 13** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 0.0398 | | 6.51 | 0.0398 | | 13.3 | 0.0398 | | 2.67 |
| Average | 6.13 | | 110 | 6.13 | | 38.0 | 6.13 | | 52.4 |
| Stdev | 35.0 | | 392 | 35.0 | | 47.6 | 35.0 | | 138 |
| p(t-test) | | | 5.5E-15 | | | 6.6E-5 | | | 7.7E-5 |
| Min | 0.00269 | | 0.0104 | 0.00269 | | 0.00471 | 0.00269 | | 0.00471 |
| Max | 832 | | 1850 | 832 | | 131 | 832 | | 440 |
| n (Samp) | 1275 | | 22 | 1275 | | 20 | 1275 | | 10 |
| n (Patient) | 452 | | 22 | 452 | | 20 | 452 | | 10 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 0.145 | | 3.98 | nd | | nd | nd | | nd |
| Average | 11.2 | | 13.7 | nd | | nd | nd | | nd |
| Stdev | 83.4 | | 27.2 | nd | | nd | nd | | nd |
| p(t-test) | | | 0.93 | nd | | nd | nd | | nd |
| Min | 0.00269 | | 0.0174 | nd | | nd | nd | | nd |
| Max | 1930 | | 80.5 | nd | | nd | nd | | nd |
| n (Samp) | 1339 | | 8 | nd | | nd | nd | | nd |
| n (Patient) | 467 | | 8 | nd | | nd | nd | | nd |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 0.116 | | 20.2 | 0.116 | | 30.0 | 0.116 | | 2.90 |
| Average | 6.45 | | 169 | 6.45 | | 142 | 6.45 | | 74.4 |
| Stdev | 37.0 | | 510 | 37.0 | | 418 | 37.0 | | 163 |
| p(t-test) | | | 2.2E-19 | | | 3.4E-19 | | | 4.1E-6 |
| Min | 0.00269 | | 0.0104 | 0.00269 | | 0.00471 | 0.00269 | | 0.00821 |
| Max | 832 | | 1930 | 832 | | 1850 | 832 | | 440 |
| n (Samp) | 1122 | | 14 | 1122 | | 19 | 1122 | | 7 |
| n (Patient) | 362 | | 14 | 362 | | 19 | 362 | | 7 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.79 | 0.77 | 0.81 | 0.81 | nd | 0.84 | 0.59 | nd | 0.68 |
| SE | 0.058 | 0.098 | 0.071 | 0.059 | nd | 0.058 | 0.095 | nd | 0.11 |
| p | 8.4E-7 | 0.0051 | 1.5E-5 | 2.0E-7 | nd | 6.1E-9 | 0.33 | nd | 0.12 |
| nCohort 1 | 1275 | 1339 | 1122 | 1275 | nd | 1122 | 1275 | nd | 1122 |
| nCohort 2 | 22 | 8 | 14 | 20 | nd | 19 | 10 | nd | 7 |
| Cutoff 1 | 2.00 | 2.57 | 2.86 | 2.60 | nd | 3.17 | 0.0109 | nd | 2.43 |
| Sens 1 | 73% | 75% | 71% | 70% | nd | 74% | 70% | nd | 71% |
| Spec 1 | 74% | 75% | 78% | 77% | nd | 79% | 26% | nd | 76% |
| Cutoff 2 | 0.362 | 2.00 | 0.362 | 1.19 | nd | 1.19 | 0.00930 | nd | 0.00930 |
| Sens 2 | 82% | 88% | 86% | 80% | nd | 84% | 80% | nd | 86% |
| Spec 2 | 55% | 71% | 54% | 67% | nd | 66% | 25% | nd | 23% |
| Cutoff 3 | 0.0163 | 0.0163 | 0.0155 | 0.0193 | nd | 0.0174 | 0.00789 | nd | 0.00789 |
| Sens 3 | 91% | 100% | 93% | 90% | nd | 95% | 90% | nd | 100% |
| Spec 3 | 45% | 44% | 42% | 47% | nd | 44% | 18% | nd | 16% |
| Cutoff 4 | 1.51 | 1.76 | 1.54 | 1.51 | nd | 1.54 | 1.51 | nd | 1.54 |
| Sens 4 | 73% | 88% | 71% | 75% | nd | 79% | 60% | nd | 71% |
| Spec 4 | 70% | 70% | 70% | 70% | nd | 70% | 70% | nd | 70% |
| Cutoff 5 | 3.37 | 3.89 | 3.44 | 3.37 | nd | 3.44 | 3.37 | nd | 3.44 |
| Sens 5 | 55% | 50% | 64% | 60% | nd | 68% | 30% | nd | 43% |
| Spec 5 | 80% | 80% | 80% | 80% | nd | 80% | 80% | nd | 80% |
| Cutoff 6 | 9.59 | 12.3 | 9.51 | 9.59 | nd | 9.51 | 9.59 | nd | 9.51 |
| Sens 6 | 41% | 12% | 57% | 55% | nd | 63% | 20% | nd | 29% |
| Spec 6 | 90% | 90% | 90% | 90% | nd | 90% | 90% | nd | 90% |
| OR Quart 2 | >4.0 | >1.0 | 1.0 | 2.0 | nd | 1.0 | 1.0 | nd | 0 |
| p Value | <0.21 | <1.0 | 1.0 | 0.57 | nd | 1.0 | 1.0 | nd | na |
| 95% CI of | >0.45 | >0.062 | 0.062 | 0.18 | nd | 0.062 | 0.14 | nd | na |
| OR Quart2 | na | na | 16 | 22 | nd | 16 | 7.1 | nd | na |
| OR Quart 3 | >3.0 | >2.0 | 2.0 | 3.0 | nd | 3.0 | 0 | nd | 0 |
| p Value | <0.34 | <0.57 | 0.57 | 0.34 | nd | 0.34 | na | nd | na |
| 95% CI of | >0.31 | >0.18 | 0.18 | 0.31 | nd | 0.31 | na | nd | na |
| OR Quart3 | na | na | 22 | 29 | nd | 29 | na | nd | na |
| OR Quart 4 | >16 | >5.1 | 10 | 15 | nd | 15 | 3.0 | nd | 2.5 |
| p Value | <0.0079 | <0.14 | 0.026 | 0.0099 | nd | 0.0098 | 0.18 | nd | 0.27 |
| 95% CI of | >2.1 | >0.59 | 1.3 | 1.9 | nd | 1.9 | 0.61 | nd | 0.48 |
| OR Quart4 | na | na | 81 | 110 | nd | 110 | 15 | nd | 13 |

| | | | | | | | | | |
|---|---|---|---|---|---|---|---|---|---|
| **Insulin-like growth factor-binding protein 3** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 392 | | 551 | 392 | | 670 | 392 | | 609 |
| Average | 672 | | 1350 | 672 | | 1760 | 672 | | 684 |
| +Stdev | 1010 | | 1580 | 1010 | | 2850 | 1010 | | 546 |
| p(t-test) | | | 0.0012 | | | 7.6E-7 | | | 0.96 |
| Min | 0.0478 | | 88.0 | 0.0478 | | 98.3 | 0.0478 | | 0.311 |
| Max | 12500 | | 5660 | 12500 | | 12500 | 12500 | | 1550 |
| n (Samp) | 1385 | | 24 | 1385 | | 24 | 1385 | | 13 |
| n (Patient) | 484 | | 24 | 484 | | 24 | 484 | | 13 |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 410 | | 786 | 410 | | 1560 | 410 | | 862 |
| Average | 730 | | 1260 | 730 | | 2430 | 730 | | 931 |
| Stdev | 1190 | | 1440 | 1190 | | 2880 | 1190 | | 559 |
| p(t-test) | | | 0.21 | | | 5.4E-4 | | | 0.65 |
| Min | 0.0478 | | 88.0 | 0.0478 | | 455 | 0.0478 | | 267 |
| Max | 12500 | | 4040 | 12500 | | 8160 | 12500 | | 1880 |
| n (Samp) | 1452 | | 8 | 1452 | | 6 | 1452 | | 7 |
| n (Patient) | 500 | | 8 | 500 | | 6 | 500 | | 7 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 419 | | 993 | 419 | | 947 | 419 | | 609 |
| Average | 701 | | 2620 | 701 | | 2080 | 701 | | 753 |
| Stdev | 1080 | | 3340 | 1080 | | 3020 | 1080 | | 673 |
| p(t-test) | | | 1.0E-10 | | | 4.5E-8 | | | 0.90 |
| Min | 0.0478 | | 119 | 0.0478 | | 98.3 | 0.0478 | | 65.9 |
| Max | 12500 | | 12500 | 12500 | | 12500 | 12500 | | 1550 |
| n (Samp) | 1243 | | 15 | 1243 | | 21 | 1243 | | 7 |
| n (Patient) | 397 | | 15 | 397 | | 21 | 397 | | 7 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.63 | 0.61 | 0.71 | 0.66 | 0.82 | 0.69 | 0.56 | 0.70 | 0.55 |
| SE | 0.062 | 0.11 | 0.076 | 0.061 | 0.10 | 0.065 | 0.083 | 0.11 | 0.11 |
| p | 0.043 | 0.28 | 0.0067 | 0.0074 | 0.0021 | 0.0044 | 0.47 | 0.072 | 0.68 |
| nCohort 1 | 1385 | 1452 | 1243 | 1385 | 1452 | 1243 | 1385 | 1452 | 1243 |
| nCohort 2 | 24 | 8 | 15 | 24 | 6 | 21 | 13 | 7 | 7 |
| Cutoff 1 | 267 | 271 | 388 | 353 | 654 | 353 | 201 | 666 | 201 |
| Sens 1 | 71% | 75% | 73% | 71% | 83% | 71% | 77% | 71% | 71% |
| Spec 1 | 39% | 39% | 48% | 47% | 66% | 45% | 31% | 67% | 29% |
| Cutoff 2 | 185 | 108 | 259 | 245 | 654 | 313 | 77.5 | 395 | 77.5 |
| Sens 2 | 83% | 88% | 80% | 83% | 83% | 81% | 85% | 86% | 86% |
| Spec 2 | 30% | 20% | 36% | 37% | 66% | 41% | 15% | 49% | 13% |
| Cutoff 3 | 108 | 87.7 | 185 | 149 | 455 | 174 | 65.8 | 267 | 65.8 |
| Sens 3 | 92% | 100% | 93% | 92% | 100% | 90% | 92% | 100% | 100% |
| Spec 3 | 20% | 16% | 27% | 25% | 54% | 26% | 13% | 38% | 11% |
| Cutoff 4 | 726 | 751 | 750 | 726 | 751 | 750 | 726 | 751 | 750 |
| Sens 4 | 46% | 50% | 60% | 46% | 67% | 52% | 38% | 57% | 43% |
| Spec 4 | 70% | 70% | 70% | 70% | 70% | 70% | 70% | 70% | 70% |
| Cutoff 5 | 1020 | 1070 | 1040 | 1020 | 1070 | 1040 | 1020 | 1070 | 1040 |
| Sens 5 | 33% | 38% | 47% | 42% | 67% | 48% | 31% | 43% | 43% |
| Spec 5 | 80% | 80% | 80% | 80% | 80% | 80% | 80% | 80% | 80% |
| Cutoff 6 | 1470 | 1560 | 1470 | 1470 | 1560 | 1470 | 1470 | 1560 | 1470 |
| Sens 6 | 33% | 25% | 47% | 33% | 50% | 43% | 15% | 14% | 29% |
| Spec 6 | 90% | 90% | 90% | 90% | 90% | 90% | 90% | 90% | 90% |
| OR Quart 2 | 1.5 | 0.50 | 4.0 | 1.7 | >0 | 2.5 | 0.33 | >2.0 | 0.50 |
| p Value | 0.53 | 0.57 | 0.21 | 0.48 | <na | 0.27 | 0.34 | <0.57 | 0.57 |
| 95% CI of | 0.42 | 0.045 | 0.45 | 0.40 | >na | 0.49 | 0.034 | >0.18 | 0.045 |
| OR Quart2 | 5.4 | 5.5 | 36 | 7.1 | na | 13 | 3.2 | na | 5.5 |
| OR Quart 3 | 0.75 | 0.50 | 1.0 | 1.7 | >2.0 | 1.5 | 1.3 | >2.0 | 0.50 |
| p Value | 0.71 | 0.57 | 1.0 | 0.48 | <0.57 | 0.66 | 0.70 | <0.57 | 0.57 |
| 95% CI of | 0.17 | 0.045 | 0.062 | 0.40 | >0.18 | 0.25 | 0.30 | >0.18 | 0.045 |
| OR Quart3 | 3.4 | 5.5 | 16 | 7.1 | na | 9.1 | 6.0 | na | 5.5 |
| OR Quart 4 | 2.8 | 2.0 | 9.2 | 3.7 | >4.0 | 5.7 | 1.7 | >3.0 | 1.5 |
| p Value | 0.081 | 0.42 | 0.036 | 0.044 | <0.21 | 0.025 | 0.48 | <0.34 | 0.66 |
| 95% CI of | 0.88 | 0.37 | 1.2 | 1.0 | >0.45 | 1.2 | 0.40 | >0.31 | 0.25 |
| OR Quart4 | 8.9 | 11 | 73 | 14 | na | 26 | 7.0 | na | 9.0 |

| | | | | | | | | | |
|---|---|---|---|---|---|---|---|---|---|
| **Immunoglogulin G1** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 3300 | | 10100 | 3300 | | 13900 | 3300 | | 5060 |
| Average | 5860 | | 12600 | 5860 | | 16000 | 5860 | | 7690 |
| Stdev | 6930 | | 9230 | 6930 | | 16500 | 6930 | | 7590 |
| p(t-test) | | | 7.5E-6 | | | 3.8E-10 | | | 0.40 |
| Min | 3.36 | | 980 | 3.36 | | 1440 | 3.36 | | 55.9 |
| Max | 80000 | | 35700 | 80000 | | 80000 | 80000 | | 25300 |
| n (Samp) | 1268 | | 22 | 1268 | | 20 | 1268 | | 10 |
| n (Patient) | 449 | | 22 | 449 | | 20 | 449 | | 10 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 3480 | | 10000 | nd | | nd | nd | | nd |
| Average | 6270 | | 12600 | nd | | nd | nd | | nd |
| Stdev | 7490 | | 9250 | nd | | nd | nd | | nd |
| p(t-test) | | | 0.018 | nd | | nd | nd | | nd |
| Min | 3.36 | | 980 | nd | | nd | nd | | nd |
| Max | 80000 | | 28300 | nd | | nd | nd | | nd |
| n (Samp) | 1332 | | 8 | nd | | nd | nd | | nd |
| n (Patient) | 464 | | 8 | nd | | nd | nd | | nd |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 3420 | | 11700 | 3420 | | 14900 | 3420 | | 5570 |
| Average | 5990 | | 13400 | 5990 | | 18400 | 5990 | | 9330 |
| Stdev | 7020 | | 9250 | 7020 | | 17000 | 7020 | | 8180 |
| p(t-test) | | | 9.1E-5 | | | 2.9E-13 | | | 0.21 |
| Min | 3.36 | | 1880 | 3.36 | | 1440 | 3.36 | | 2380 |
| Max | 80000 | | 35700 | 80000 | | 80000 | 80000 | | 25300 |
| n(Samp) | 1114 | | 14 | 1114 | | 19 | 1114 | | 7 |
| n (Patient) | 359 | | 14 | 359 | | 19 | 359 | | 7 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.76 | 0.74 | 0.78 | 0.81 | nd | 0.84 | 0.60 | nd | 0.69 |
| SE | 0.060 | 0.10 | 0.074 | 0.059 | nd | 0.057 | 0.095 | nd | 0.11 |
| p | 1.2E-5 | 0.017 | 1.4E-4 | 1.5E-7 | nd | 2.0E-9 | 0.30 | nd | 0.086 |
| nCohort 1 | 1268 | 1332 | 1114 | 1268 | nd | 1114 | 1268 | nd | 1114 |
| nCohort 2 | 22 | 8 | 14 | 20 | nd | 19 | 10 | nd | 7 |
| Cutoff 1 | 6900 | 6900 | 8550 | 9500 | nd | 9500 | 3900 | nd | 4550 |
| Sens 1 | 73% | 75% | 71% | 70% | nd | 74% | 70% | nd | 71% |
| Spec 1 | 72% | 70% | 78% | 81% | nd | 81% | 56% | nd | 61% |
| Cutoff 2 | 5190 | 5190 | 4150 | 7030 | nd | 7030 | 2380 | nd | 3900 |
| Sens 2 | 82% | 88% | 86% | 80% | nd | 84% | 80% | nd | 86% |
| Spec 2 | 65% | 63% | 57% | 73% | nd | 72% | 37% | nd | 55% |
| Cutoff 3 | 1990 | 977 | 1990 | 4740 | nd | 4740 | 1740 | nd | 2380 |
| Sens 3 | 91% | 100% | 93% | 90% | nd | 95% | 90% | nd | 100% |
| Spec 3 | 31% | 12% | 30% | 63% | nd | 62% | 26% | nd | 36% |
| Cutoff 4 | 6240 | 6760 | 6490 | 6240 | nd | 6490 | 6240 | nd | 6490 |
| Sens 4 | 77% | 75% | 79% | 80% | nd | 84% | 40% | nd | 43% |
| Spec 4 | 70% | 70% | 70% | 70% | nd | 70% | 70% | nd | 70% |
| Cutoff 5 | 8950 | 9780 | 9100 | 8950 | nd | 9100 | 8950 | nd | 9100 |
| Sens 5 | 55% | 50% | 64% | 70% | nd | 74% | 30% | nd | 29% |
| Spec 5 | 80% | 80% | 80% | 80% | nd | 80% | 80% | nd | 80% |
| Cutoff 6 | 14200 | 15400 | 14300 | 14200 | nd | 14300 | 14200 | nd | 14300 |
| Sens 6 | 36% | 38% | 43% | 50% | nd | 58% | 20% | nd | 29% |
| Spec 6 | 90% | 90% | 90% | 90% | nd | 90% | 90% | nd | 90% |
| OR Quart 2 | 2.0 | 0 | >2.0 | 1.0 | nd | 0 | 2.0 | nd | >1.0 |
| p Value | 0.57 | na | <0.57 | 1.0 | nd | na | 0.57 | nd | <1.00 |
| 95% CI of | 0.18 | na | >0.18 | 0.062 | nd | na | 0.18 | nd | >0.062 |
| OR Quart2 | 22 | na | na | 16 | nd | na | 22 | nd | na |
| OR Quart 3 | 4.0 | 2.0 | >1.0 | 3.0 | nd | 3.0 | 3.0 | nd | >3.0 |
| p Value | 0.21 | 0.57 | <1.00 | 0.34 | nd | 0.34 | 0.34 | nd | <0.34 |
| 95% CI of | 0.45 | 0.18 | >0.062 | 0.31 | nd | 0.31 | 0.31 | nd | >0.31 |
| OR Quart3 | 36 | 22 | na | 29 | nd | 29 | 29 | nd | na |
| OR Quart 4 | 16 | 5.1 | >11 | 16 | nd | 16 | 4.0 | nd | >3.0 |
| p Value | 0.0080 | 0.14 | <0.020 | 0.0079 | nd | 0.0078 | 0.21 | nd | <0.34 |
| 95% CI of | 2.1 | 0.59 | >1.5 | 2.1 | nd | 2.1 | 0.45 | nd | >0.31 |
| OR Quart4 | 120 | 44 | na | 120 | nd | 120 | 36 | nd | na |

| | | | | | | | | | |
|---|---|---|---|---|---|---|---|---|---|
| **Immunoglogulin G2** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 9080 | | 24500 | 9080 | | 31300 | 9080 | | 11300 |
| Average | 18400 | | 51400 | 18400 | | 57600 | 18400 | | 42800 |
| Stdev | 31600 | | 63700 | 31600 | | 80400 | 31600 | | 73900 |
| p(t-test) | | | 2.5E-6 | | | 1.5E-7 | | | 0.017 |
| Min | 119 | | 2380 | 119 | | 3940 | 119 | | 25.4 |
| Max | 240000 | | 240000 | 240000 | | 240000 | 240000 | | 240000 |
| n (Samp) | 1268 | | 22 | 1268 | | 20 | 1268 | | 10 |
| n (Patient) | 449 | | 22 | 449 | | 20 | 449 | | 10 |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 9340 | | 24600 | nd | | nd | nd | | nd |
| Average | 20400 | | 55100 | nd | | nd | nd | | nd |
| Stdev | 36100 | | 62200 | nd | | nd | nd | | nd |
| p(t-test) | | | 0.0071 | nd | | nd | nd | | nd |
| Min | 119 | | 2380 | nd | | nd | nd | | nd |
| Max | 240000 | | 182000 | nd | | nd | nd | | nd |
| n (Samp) | 1332 | | 8 | nd | | nd | nd | | nd |
| n (Patient) | 464 | | 8 | nd | | nd | nd | | nd |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 9630 | | 24500 | 9630 | | 33000 | 9630 | | 13800 |
| Average | 18500 | | 63500 | 18500 | | 76200 | 18500 | | 52800 |
| Stdev | 30500 | | 81000 | 30500 | | 89800 | 30500 | | 86800 |
| p(t-test) | | | 1.5E-7 | | | 2.9E-14 | | | 0.0038 |
| Min | 25.4 | | 4460 | 25.4 | | 3940 | 25.4 | | 2320 |
| Max | 240000 | | 240000 | 240000 | | 240000 | 240000 | | 240000 |
| n (Samp) | 1114 | | 14 | 1114 | | 19 | 1114 | | 7 |
| n (Patient) | 359 | | 14 | 359 | | 19 | 359 | | 7 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.71 | 0.73 | 0.71 | 0.74 | nd | 0.77 | 0.57 | nd | 0.61 |
| SE | 0.063 | 0.10 | 0.078 | 0.064 | nd | 0.064 | 0.095 | nd | 0.11 |
| p | 0.0011 | 0.023 | 0.0064 | 1.5E-4 | nd | 1.9E-5 | 0.48 | nd | 0.32 |
| nCohort 1 | 1268 | 1332 | 1114 | 1268 | nd | 1114 | 1268 | nd | 1114 |
| nCohort 2 | 22 | 8 | 14 | 20 | nd | 19 | 10 | nd | 7 |
| Cutoff 1 | 11800 | 13700 | 13700 | 14100 | nd | 14100 | 8540 | nd | 8840 |
| Sens 1 | 73% | 75% | 71% | 70% | nd | 74% | 70% | nd | 71% |
| Spec 1 | 60% | 65% | 65% | 67% | nd | 66% | 47% | nd | 47% |
| Cutoff 2 | 5910 | 11800 | 5910 | 12400 | nd | 12400 | 6890 | nd | 8540 |
| Sens 2 | 82% | 88% | 86% | 80% | nd | 84% | 80% | nd | 86% |
| Spec 2 | 31% | 59% | 29% | 63% | nd | 61% | 37% | nd | 45% |
| Cutoff 3 | 4910 | 2370 | 5180 | 5300 | nd | 5300 | 2270 | nd | 2270 |
| Sens 3 | 91% | 100% | 93% | 90% | nd | 95% | 90% | nd | 100% |
| Spec 3 | 25% | 8% | 24% | 27% | nd | 25% | 9% | nd | 8% |
| Cutoff 4 | 15200 | 16200 | 16000 | 15200 | nd | 16000 | 15200 | nd | 16000 |
| Sens 4 | 59% | 62% | 57% | 60% | nd | 63% | 30% | nd | 29% |
| Spec 4 | 70% | 70% | 70% | 70% | nd | 70% | 70% | nd | 70% |
| Cutoff 5 | 21200 | 22500 | 21800 | 21200 | nd | 21800 | 21200 | nd | 21800 |
| Sens 5 | 59% | 62% | 57% | 55% | nd | 58% | 30% | nd | 29% |
| Spec 5 | 80% | 80% | 80% | 80% | nd | 80% | 80% | nd | 80% |
| Cutoff 6 | 37600 | 41400 | 37600 | 37600 | nd | 37600 | 37600 | nd | 37600 |
| Sens 6 | 36% | 38% | 43% | 30% | nd | 42% | 30% | nd | 29% |
| Spec 6 | 90% | 90% | 90% | 90% | nd | 90% | 90% | nd | 90% |
| OR Quart 2 | 0.66 | 0 | 0.50 | 1.0 | nd | 2.0 | 1.5 | nd | 2.0 |
| p Value | 0.65 | na | 0.57 | 1.0 | nd | 0.57 | 0.66 | nd | 0.57 |
| 95% CI of | 0.11 | na | 0.045 | 0.14 | nd | 0.18 | 0.25 | nd | 0.18 |
| OR Quart2 | 4.0 | na | 5.5 | 7.1 | nd | 22 | 9.0 | nd | 22 |
| OR Quart 3 | 1.3 | 2.0 | 1.5 | 2.0 | nd | 4.0 | 1.0 | nd | 2.0 |
| p Value | 0.70 | 0.57 | 0.66 | 0.42 | nd | 0.21 | 1.0 | nd | 0.57 |
| 95% CI of | 0.30 | 0.18 | 0.25 | 0.37 | nd | 0.45 | 0.14 | nd | 0.18 |
| OR Quart3 | 6.0 | 22 | 9.1 | 11 | nd | 36 | 7.1 | nd | 22 |
| OR Quart 4 | 4.5 | 5.1 | 4.1 | 6.2 | nd | 12 | 1.5 | nd | 2.0 |
| p Value | 0.021 | 0.14 | 0.077 | 0.018 | nd | 0.016 | 0.66 | nd | 0.57 |
| 95% CI of | 1.3 | 0.59 | 0.86 | 1.4 | nd | 1.6 | 0.25 | nd | 0.18 |
| OR Quart4 | 16 | 44 | 19 | 28 | nd | 96 | 9.0 | nd | 22 |

| | | | | | | | | | |
|---|---|---|---|---|---|---|---|---|---|
| **Interleukin-11** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 133 | | 222 | 133 | | 253 | 133 | | 93.2 |
| Average | 212 | | 502 | 212 | | 422 | 212 | | 327 |
| Stdev | 234 | | 693 | 234 | | 570 | 234 | | 645 |
| p(t-test) | | | 6.8E-8 | | | 1.8E-4 | | | 0.13 |
| Min | 0.0822 | | 48.0 | 0.0822 | | 49.5 | 0.0822 | | 14.5 |
| Max | 2260 | | 2900 | 2260 | | 2590 | 2260 | | 2140 |
| n (Samp) | 1275 | | 22 | 1275 | | 19 | 1275 | | 10 |
| n (Patient) | 452 | | 22 | 452 | | 19 | 452 | | 10 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 137 | | 124 | nd | | nd | nd | | nd |
| Average | 231 | | 276 | nd | | nd | nd | | nd |
| Stdev | 302 | | 348 | nd | | nd | nd | | nd |
| p(t-test) | | | 0.68 | nd | | nd | nd | | nd |
| Min | 0.0822 | | 48.0 | nd | | nd | nd | | nd |
| Max | 3780 | | 1040 | nd | | nd | nd | | nd |
| n (Samp) | 1338 | | 8 | nd | | nd | nd | | nd |
| n (Patient) | 467 | | 8 | nd | | nd | nd | | nd |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 142 | | 384 | 142 | | 255 | 142 | | 95.5 |
| Average | 220 | | 588 | 220 | | 490 | 220 | | 430 |
| Stdev | 238 | | 740 | 238 | | 623 | 238 | | 763 |
| p(t-test) | | | 5.4E-8 | | | 5.2E-6 | | | 0.023 |
| Min | 0.0822 | | 116 | 0.0822 | | 49.5 | 0.0822 | | 14.5 |
| Max | 2260 | | 2900 | 2260 | | 2590 | 2260 | | 2140 |
| n (Samp) | 1121 | | 14 | 1121 | | 18 | 1121 | | 7 |
| n (Patient) | 362 | | 14 | 362 | | 18 | 362 | | 7 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.68 | 0.51 | 0.75 | 0.69 | nd | 0.69 | 0.46 | nd | 0.50 |
| SE | 0.064 | 0.10 | 0.076 | 0.068 | nd | 0.070 | 0.094 | nd | 0.11 |
| p | 0.0046 | 0.93 | 0.0012 | 0.0043 | nd | 0.0068 | 0.63 | nd | 0.98 |
| nCohort 1 | 1275 | 1338 | 1121 | 1275 | nd | 1121 | 1275 | nd | 1121 |
| nCohort 2 | 22 | 8 | 14 | 19 | nd | 18 | 10 | nd | 7 |
| Cutoff 1 | 131 | 87.1 | 158 | 181 | nd | 179 | 88.1 | nd | 90.5 |
| Sens 1 | 73% | 75% | 71% | 74% | nd | 72% | 70% | nd | 71% |
| Spec 1 | 49% | 32% | 55% | 63% | nd | 60% | 33% | nd | 31% |
| Cutoff 2 | 115 | 76.3 | 131 | 135 | nd | 135 | 85.7 | nd | 85.7 |
| Sens 2 | 82% | 88% | 86% | 84% | nd | 83% | 80% | nd | 86% |
| Spec 2 | 44% | 26% | 46% | 51% | nd | 48% | 32% | nd | 29% |
| Cutoff 3 | 87.1 | 47.7 | 128 | 79.7 | nd | 79.7 | 66.2 | nd | 13.5 |
| Sens 3 | 91% | 100% | 93% | 95% | nd | 94% | 90% | nd | 100% |
| Spec 3 | 32% | 13% | 46% | 29% | nd | 27% | 23% | nd | 1% |
| Cutoff 4 | 226 | 235 | 235 | 226 | nd | 235 | 226 | nd | 235 |
| Sens 4 | 50% | 25% | 57% | 58% | nd | 61% | 30% | nd | 43% |
| Spec 4 | 70% | 70% | 70% | 70% | nd | 70% | 70% | nd | 70% |
| Cutoff 5 | 312 | 329 | 319 | 312 | nd | 319 | 312 | nd | 319 |
| Sens 5 | 45% | 25% | 57% | 32% | nd | 33% | 20% | nd | 29% |
| Spec 5 | 80% | 80% | 80% | 80% | nd | 80% | 80% | nd | 80% |
| Cutoff 6 | 469 | 503 | 474 | 469 | nd | 474 | 469 | nd | 474 |
| Sens 6 | 32% | 25% | 43% | 21% | nd | 28% | 10% | nd | 14% |
| Spec 6 | 90% | 90% | 90% | 90% | nd | 90% | 90% | nd | 90% |
| OR Quart 2 | 6.1 | 4.0 | >3.0 | 2.0 | nd | 4.0 | 0.50 | nd | 0.50 |
| p Value | 0.095 | 0.21 | <0.34 | 0.57 | nd | 0.21 | 0.57 | nd | 0.57 |
| 95% CI of | 0.73 | 0.45 | >0.31 | 0.18 | nd | 0.45 | 0.045 | nd | 0.045 |
| OR Quart2 | 51 | 36 | na | 22 | nd | 36 | 5.5 | nd | 5.5 |
| OR Quart 3 | 5.1 | 1.0 | >3.0 | 8.2 | nd | 5.1 | 2.5 | nd | 1.5 |
| p Value | 0.14 | 1.0 | <0.34 | 0.048 | nd | 0.14 | 0.27 | nd | 0.66 |
| 95% CI of | 0.59 | 0.062 | >0.31 | 1.0 | nd | 0.59 | 0.49 | nd | 0.25 |
| OR Quart3 | 44 | 16 | na | 66 | nd | 44 | 13 | nd | 9.1 |
| OR Quart 4 | 10 | 2.0 | >8.2 | 8.2 | nd | 8.2 | 1.0 | nd | 0.50 |
| p Value | 0.027 | 0.57 | <0.048 | 0.049 | nd | 0.048 | 1.00 | nd | 0.57 |
| 95% CI of | 1.3 | 0.18 | >1.0 | 1.0 | nd | 1.0 | 0.14 | nd | 0.045 |
| OR Quart4 | 81 | 22 | na | 66 | nd | 66 | 7.2 | nd | 5.5 |

| | | | | | | | | | |
|---|---|---|---|---|---|---|---|---|---|
| **Interleukin-2 receptor alpha chain** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 573 | | 935 | 573 | | 1390 | 573 | | 770 |
| Average | 1010 | | 1690 | 1010 | | 2110 | 1010 | | 1620 |
| Stdev | 1270 | | 1810 | 1270 | | 2430 | 1270 | | 2400 |
| p(t-test) | | | 0.014 | | | 1.6E-4 | | | 0.13 |
| Min | 0.0317 | | 0.118 | 0.0317 | | 50.9 | 0.0317 | | 0.115 |
| Max | 10400 | | 6080 | 10400 | | 9090 | 10400 | | 7740 |
| n (Samp) | 1277 | | 22 | 1277 | | 20 | 1277 | | 10 |
| n (Patient) | 452 | | 22 | 452 | | 20 | 452 | | 10 |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 609 | | 485 | nd | | nd | nd | | nd |
| Average | 1090 | | 1290 | nd | | nd | nd | | nd |
| Stdev | 1420 | | 2030 | nd | | nd | nd | | nd |
| p(t-test) | | | 0.70 | nd | | nd | nd | | nd |
| Min | 0.0317 | | 0.118 | nd | | nd | nd | | nd |
| Max | 10400 | | 6080 | nd | | nd | nd | | nd |
| n (Samp) | 1341 | | 8 | nd | | nd | nd | | nd |
| n (Patient) | 467 | | 8 | nd | | nd | nd | | nd |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 624 | | 1540 | 624 | | 1700 | 624 | | 819 |
| Average | 1050 | | 2130 | 1050 | | 2300 | 1050 | | 2120 |
| Stdev | 1280 | | 1940 | 1280 | | 2450 | 1280 | | 2740 |
| p(t-test) | | | 0.0020 | | | 3.7E-5 | | | 0.029 |
| Min | 0.0317 | | 54.4 | 0.0317 | | 50.9 | 0.0317 | | 19.9 |
| Max | 10400 | | 6080 | 10400 | | 9090 | 10400 | | 7740 |
| n(Samp) | 1124 | | 14 | 1124 | | 19 | 1124 | | 7 |
| n (Patient) | 362 | | 14 | 362 | | 19 | 362 | | 7 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.59 | 0.47 | 0.66 | 0.65 | nd | 0.68 | 0.52 | nd | 0.58 |
| SE | 0.064 | 0.10 | 0.080 | 0.067 | nd | 0.068 | 0.093 | nd | 0.11 |
| p | 0.16 | 0.78 | 0.052 | 0.021 | nd | 0.0078 | 0.86 | nd | 0.46 |
| nCohort 1 | 1277 | 1341 | 1124 | 1277 | nd | 1124 | 1277 | nd | 1124 |
| nCohort 2 | 22 | 8 | 14 | 20 | nd | 19 | 10 | nd | 7 |
| Cutoff 1 | 350 | 189 | 477 | 761 | nd | 761 | 227 | nd | 718 |
| Sens 1 | 73% | 75% | 71% | 70% | nd | 74% | 70% | nd | 71% |
| Spec 1 | 37% | 24% | 42% | 57% | nd | 56% | 28% | nd | 54% |
| Cutoff 2 | 189 | 60.5 | 277 | 385 | nd | 385 | 35.7 | nd | 35.7 |
| Sens 2 | 82% | 88% | 86% | 80% | nd | 84% | 80% | nd | 86% |
| Spec 2 | 25% | 12% | 30% | 39% | nd | 37% | 10% | nd | 9% |
| Cutoff 3 | 60.5 | 0.116 | 89.0 | 69.2 | nd | 68.0 | 18.6 | nd | 18.6 |
| Sens 3 | 91% | 100% | 93% | 90% | nd | 95% | 90% | nd | 100% |
| Spec 3 | 13% | 2% | 13% | 14% | nd | 12% | 8% | nd | 7% |
| Cutoff 4 | 1110 | 1160 | 1140 | 1110 | nd | 1140 | 1110 | nd | 1140 |
| Sens 4 | 45% | 38% | 57% | 55% | nd | 58% | 40% | nd | 43% |
| Spec 4 | 70% | 70% | 70% | 70% | nd | 70% | 70% | nd | 70% |
| Cutoff 5 | 1620 | 1760 | 1670 | 1620 | nd | 1670 | 1620 | nd | 1670 |
| Sens 5 | 36% | 12% | 50% | 45% | nd | 53% | 30% | nd | 43% |
| Spec 5 | 80% | 80% | 80% | 80% | nd | 80% | 80% | nd | 80% |
| Cutoff 6 | 2420 | 2620 | 2480 | 2420 | nd | 2480 | 2420 | nd | 2480 |
| Sens 6 | 32% | 12% | 43% | 30% | nd | 37% | 20% | nd | 29% |
| Spec 6 | 90% | 90% | 90% | 90% | nd | 90% | 90% | nd | 90% |
| OR Quart 2 | 1.00 | 0.50 | 1.5 | 0.50 | nd | 0.66 | 0.33 | nd | 0 |
| p Value | 1.00 | 0.57 | 0.66 | 0.42 | nd | 0.65 | 0.34 | nd | na |
| 95% CI of | 0.29 | 0.045 | 0.25 | 0.090 | nd | 0.11 | 0.034 | nd | na |
| OR Quart2 | 3.5 | 5.5 | 9.0 | 2.7 | nd | 4.0 | 3.2 | nd | na |
| OR Quart 3 | 0.80 | 1.0 | 1.0 | 1.0 | nd | 1.3 | 1.00 | nd | 1.00 |
| p Value | 0.73 | 1.00 | 1.0 | 1.0 | nd | 0.71 | 1.00 | nd | 1.00 |
| 95% CI of | 0.21 | 0.14 | 0.14 | 0.25 | nd | 0.30 | 0.20 | nd | 0.14 |
| OR Quart3 | 3.0 | 7.2 | 7.1 | 4.0 | nd | 6.0 | 5.0 | nd | 7.1 |
| OR Quart 4 | 1.6 | 1.5 | 3.6 | 2.5 | nd | 3.4 | 1.00 | nd | 1.5 |
| p Value | 0.41 | 0.65 | 0.12 | 0.12 | nd | 0.065 | 1.00 | nd | 0.66 |
| 95% CI of | 0.52 | 0.25 | 0.73 | 0.79 | nd | 0.93 | 0.20 | nd | 0.25 |
| OR Quart4 | 5.0 | 9.1 | 17 | 8.2 | nd | 13 | 5.0 | nd | 9.0 |

| | | | | | | | | | |
|---|---|---|---|---|---|---|---|---|---|
| **Neutrophil collagenase** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 3540 | | 13400 | 3540 | | 13400 | 3540 | | 3260 |
| Average | 15000 | | 81600 | 15000 | | 38600 | 15000 | | 11700 |
| Stdev | 41900 | | 159000 | 41900 | | 49400 | 41900 | | 18300 |
| p(t-test) | | | 4.3E-12 | | | 0.0066 | | | 0.78 |
| Min | 0.114 | | 0.260 | 0.114 | | 25.8 | 0.114 | | 212 |
| Max | 670000 | | 649000 | 670000 | | 166000 | 670000 | | 66900 |
| n (Samp) | 1382 | | 24 | 1382 | | 24 | 1382 | | 13 |
| n (Patient) | 485 | | 24 | 485 | | 24 | 485 | | 13 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 3770 | | 5520 | 3770 | | 14100 | 3770 | | 10800 |
| Average | 17100 | | 99700 | 17100 | | 97000 | 17100 | | 10600 |
| Stdev | 47900 | | 225000 | 47900 | | 160000 | 47900 | | 8750 |
| p(t-test) | | | 3.9E-6 | | | 6.4E-5 | | | 0.72 |
| Min | 0.114 | | 0.260 | 0.114 | | 25.8 | 0.114 | | 1120 |
| Max | 670000 | | 649000 | 670000 | | 401000 | 670000 | | 21900 |
| n (Samp) | 1449 | | 8 | 1449 | | 6 | 1449 | | 7 |
| n (Patient) | 501 | | 8 | 501 | | 6 | 501 | | 7 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 3590 | | 22500 | 3590 | | 20500 | 3590 | | 3260 |
| Average | 14600 | | 80500 | 14600 | | 61600 | 14600 | | 13500 |
| Stdev | 38300 | | 147000 | 38300 | | 82900 | 38300 | | 24100 |
| p(t-test) | | | 9.7E-10 | | | 7.0E-8 | | | 0.94 |
| Min | 0.114 | | 1660 | 0.114 | | 551 | 0.114 | | 576 |
| Max | 670000 | | 561000 | 670000 | | 327000 | 670000 | | 66900 |
| n (Samp) | 1240 | | 15 | 1240 | | 21 | 1240 | | 7 |
| n (Patient) | 398 | | 15 | 398 | | 21 | 398 | | 7 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.68 | 0.55 | 0.76 | 0.72 | 0.61 | 0.77 | 0.51 | 0.61 | 0.49 |
| SE | 0.061 | 0.11 | 0.072 | 0.060 | 0.12 | 0.061 | 0.081 | 0.11 | 0.11 |
| p | 0.0024 | 0.64 | 2.5E-4 | 3.1E-4 | 0.38 | 6.5E-6 | 0.90 | 0.34 | 0.95 |
| nCohort 1 | 1382 | 1449 | 1240 | 1382 | 1449 | 1240 | 1382 | 1449 | 1240 |
| nCohort 2 | 24 | 8 | 15 | 24 | 6 | 21 | 13 | 7 | 7 |
| Cutoff 1 | 5080 | 3020 | 8450 | 7290 | 978 | 10400 | 1120 | 2970 | 1430 |
| Sens 1 | 71% | 75% | 73% | 71% | 83% | 71% | 77% | 71% | 71% |
| Spec 1 | 58% | 45% | 69% | 65% | 22% | 74% | 25% | 44% | 29% |
| Cutoff 2 | 2970 | 127 | 5080 | 3090 | 978 | 7290 | 576 | 2660 | 576 |
| Sens 2 | 83% | 88% | 80% | 83% | 83% | 81% | 85% | 86% | 86% |
| Spec 2 | 46% | 3% | 58% | 46% | 22% | 65% | 15% | 42% | 15% |
| Cutoff 3 | 1650 | 0.159 | 2030 | 2450 | 23.8 | 2660 | 571 | 1120 | 571 |
| Sens 3 | 92% | 100% | 93% | 92% | 100% | 90% | 92% | 100% | 100% |
| Spec 3 | 34% | 0% | 36% | 42% | 1% | 43% | 15% | 24% | 15% |
| Cutoff 4 | 8980 | 9730 | 8740 | 8980 | 9730 | 8740 | 8980 | 9730 | 8740 |
| Sens 4 | 54% | 38% | 67% | 62% | 50% | 71% | 38% | 57% | 29% |
| Spec 4 | 70% | 70% | 70% | 70% | 70% | 70% | 70% | 70% | 70% |
| Cutoff 5 | 15600 | 17000 | 15100 | 15600 | 17000 | 15100 | 15600 | 17000 | 15100 |
| Sens 5 | 46% | 25% | 53% | 46% | 50% | 52% | 23% | 29% | 14% |
| Spec 5 | 80% | 80% | 80% | 80% | 80% | 80% | 80% | 80% | 80% |
| Cutoff 6 | 32700 | 36000 | 32700 | 32700 | 36000 | 32700 | 32700 | 36000 | 32700 |
| Sens 6 | 33% | 25% | 40% | 33% | 33% | 43% | 8% | 0% | 14% |
| Spec 6 | 90% | 90% | 90% | 90% | 90% | 90% | 90% | 90% | 90% |
| OR Quart 2 | 2.5 | 0.50 | >3.0 | 2.0 | 0 | 3.0 | 0.75 | 2.0 | 0.50 |
| p Value | 0.27 | 0.57 | <0.34 | 0.42 | na | 0.34 | 0.70 | 0.57 | 0.57 |
| 95% CI of | 0.48 | 0.045 | >0.31 | 0.36 | na | 0.31 | 0.17 | 0.18 | 0.045 |
| OR Quart2 | 13 | 5.5 | na | 11 | na | 29 | 3.4 | 22 | 5.5 |
| OR Quart 3 | 2.0 | 1.0 | >2.0 | 2.0 | 0.50 | 3.0 | 0.50 | 1.0 | 1.0 |
| p Value | 0.42 | 1.0 | <0.57 | 0.42 | 0.57 | 0.34 | 0.42 | 1.0 | 1.0 |
| 95% CI of | 0.37 | 0.14 | >0.18 | 0.37 | 0.045 | 0.31 | 0.090 | 0.062 | 0.14 |
| OR Quart3 | 11 | 7.1 | na | 11 | 5.5 | 29 | 2.7 | 16 | 7.1 |
| OR Quart 4 | 6.7 | 1.5 | >10 | 7.2 | 1.5 | 15 | 1.00 | 3.0 | 1.0 |
| p Value | 0.013 | 0.66 | <0.027 | 0.0092 | 0.66 | 0.0099 | 1.00 | 0.34 | 1.00 |
| 95% CI of | 1.5 | 0.25 | >1.3 | 1.6 | 0.25 | 1.9 | 0.25 | 0.31 | 0.14 |
| OR Quart4 | 30 | 9.0 | na | 32 | 9.0 | 110 | 4.0 | 29 | 7.2 |

| | | | | | | | | | |
|---|---|---|---|---|---|---|---|---|---|
| **Protransforming growth factor alpha** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 6.25 | | 13.8 | 6.25 | | 12.5 | 6.25 | | 13.5 |
| Average | 10.9 | | 20.9 | 10.9 | | 20.1 | 10.9 | | 14.9 |
| Stdev | 21.2 | | 26.1 | 21.2 | | 16.0 | 21.2 | | 12.1 |
| p(t-test) | | | 0.028 | | | 0.051 | | | 0.54 |
| Min | 0.00184 | | 0.781 | 0.00184 | | 3.26 | 0.00184 | | 0.00454 |
| Max | 361 | | 122 | 361 | | 50.4 | 361 | | 36.0 |
| n (Samp) | 1271 | | 22 | 1271 | | 20 | 1271 | | 10 |
| n (Patient) | 452 | | 22 | 452 | | 20 | 452 | | 10 |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 6.57 | | 13.7 | nd | | nd | nd | | nd |
| Average | 11.3 | | 12.4 | nd | | nd | nd | | nd |
| Stdev | 21.2 | | 5.27 | nd | | nd | nd | | nd |
| p(t-test) | | | 0.89 | nd | | nd | nd | | nd |
| Min | 0.00184 | | 1.41 | nd | | nd | nd | | nd |
| Max | 361 | | 18.0 | nd | | nd | nd | | nd |
| n (Samp) | 1335 | | 8 | nd | | nd | nd | | nd |
| n (Patient) | 467 | | 8 | nd | | nd | nd | | nd |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | 6.50 | | 16.0 | 6.50 | | 17.8 | 6.50 | | 14.4 |
| Average | 11.3 | | 21.8 | 11.3 | | 21.7 | 11.3 | | 17.6 |
| Stdev | 22.3 | | 19.7 | 22.3 | | 15.6 | 22.3 | | 13.1 |
| p(t-test) | | | 0.080 | | | 0.044 | | | 0.45 |
| Min | 0.00184 | | 0.781 | 0.00184 | | 3.26 | 0.00184 | | 0.564 |
| Max | 361 | | 63.1 | 361 | | 50.4 | 361 | | 36.0 |
| n (Samp) | 1118 | | 14 | 1118 | | 19 | 1118 | | 7 |
| n (Patient) | 362 | | 14 | 362 | | 19 | 362 | | 7 |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.72 | 0.70 | 0.73 | 0.74 | nd | 0.78 | 0.65 | nd | 0.70 |
| SE | 0.062 | 0.10 | 0.078 | 0.064 | nd | 0.064 | 0.095 | nd | 0.11 |
| p | 3.3E-4 | 0.061 | 0.0034 | 1.8E-4 | nd | 1.4E-5 | 0.12 | nd | 0.069 |
| nCohort 1 | 1271 | 1335 | 1118 | 1271 | nd | 1118 | 1271 | nd | 1118 |
| nCohort 2 | 22 | 8 | 14 | 20 | nd | 19 | 10 | nd | 7 |
| Cutoff 1 | 9.29 | 11.3 | 8.44 | 10.5 | nd | 11.3 | 9.53 | nd | 12.6 |
| Sens 1 | 73% | 75% | 71% | 70% | nd | 74% | 70% | nd | 71% |
| Spec 1 | 67% | 73% | 63% | 72% | nd | 74% | 68% | nd | 78% |
| Cutoff 2 | 6.62 | 9.29 | 6.53 | 6.37 | nd | 8.14 | 6.20 | nd | 6.20 |
| Sens 2 | 82% | 88% | 86% | 80% | nd | 84% | 80% | nd | 86% |
| Spec 2 | 52% | 65% | 50% | 51% | nd | 62% | 50% | nd | 49% |
| Cutoff 3 | 5.52 | 1.41 | 5.48 | 4.33 | nd | 4.33 | 0.530 | nd | 0.530 |
| Sens 3 | 91% | 100% | 93% | 90% | nd | 95% | 90% | nd | 100% |
| Spec 3 | 45% | 10% | 44% | 35% | nd | 34% | 5% | nd | 5% |
| Cutoff 4 | 9.95 | 10.5 | 10.1 | 9.95 | nd | 10.1 | 9.95 | nd | 10.1 |
| Sens 4 | 68% | 75% | 64% | 70% | nd | 79% | 60% | nd | 71% |
| Spec 4 | 70% | 70% | 70% | 70% | nd | 70% | 70% | nd | 70% |
| Cutoff 5 | 13.4 | 14.1 | 13.7 | 13.4 | nd | 13.7 | 13.4 | nd | 13.7 |
| Sens 5 | 55% | 50% | 57% | 45% | nd | 53% | 50% | nd | 57% |
| Spec 5 | 80% | 80% | 80% | 80% | nd | 80% | 80% | nd | 80% |
| Cutoff 6 | 21.9 | 23.3 | 23.2 | 21.9 | nd | 23.2 | 21.9 | nd | 23.2 |
| Sens 6 | 18% | 0% | 29% | 35% | nd | 37% | 20% | nd | 29% |
| Spec 6 | 90% | 90% | 90% | 90% | nd | 90% | 90% | nd | 90% |
| OR Quart 2 | 0.50 | 0 | 1.0 | >4.0 | nd | >3.0 | 0.50 | nd | 1.0 |
| p Value | 0.57 | na | 1.0 | <0.21 | nd | <0.34 | 0.57 | nd | 1.0 |
| 95% CI of | 0.045 | na | 0.062 | >0.45 | nd | >0.31 | 0.045 | nd | 0.062 |
| OR Quart2 | 5.5 | na | 16 | na | nd | na | 5.5 | nd | 16 |
| OR Quart 3 | 3.0 | 2.0 | 4.0 | >5.1 | nd | >4.1 | 0.50 | nd | 0 |
| p Value | 0.18 | 0.57 | 0.21 | <0.14 | nd | <0.21 | 0.57 | nd | na |
| 95% CI of | 0.61 | 0.18 | 0.45 | >0.59 | nd | >0.45 | 0.045 | nd | na |
| OR Quart3 | 15 | 22 | 36 | na | nd | na | 5.5 | nd | na |
| OR Quart 4 | 6.7 | 5.0 | 8.2 | >11 | nd | >12 | 3.0 | nd | 5.1 |
| p Value | 0.013 | 0.14 | 0.048 | <0.020 | nd | <0.016 | 0.18 | nd | 0.14 |
| 95% CI of | 1.5 | 0.59 | 1.0 | >1.5 | nd | >1.6 | 0.61 | nd | 0.59 |
| OR Quart4 | 30 | 43 | 66 | na | nd | na | 15 | nd | 44 |

| | | | | | | | | | |
|---|---|---|---|---|---|---|---|---|---|
| **CA 15-3** | | | | | | | | | |
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | 434 | | 434 | 434 | | 434 | 434 | | 434 |
| Average | 326 | | 423 | 326 | | 325 | 326 | | 308 |
| Stdev | 223 | | 180 | 223 | | 224 | 223 | | 191 |
| p(t-test) | | | 0.17 | | | 0.99 | | | 0.82 |
| Min | 1.17 | | 22.0 | 1.17 | | 39.8 | 1.17 | | 4.72 |
| Max | 784 | | 784 | 784 | | 784 | 784 | | 434 |
| n (Samp) | 558 | | 10 | 558 | | 15 | 558 | | 8 |
| n (Patient) | 262 | | 10 | 262 | | 15 | 262 | | 8 |
| | | | | | | | | | |

| sCr only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | nd | | nd | nd | | nd | 434 | | 434 |
| Average | nd | | nd | nd | | nd | 326 | | 406 |
| Stdev | nd | | nd | nd | | nd | 223 | | 68.4 |
| p(t-test) | nd | | nd | nd | | nd | | | 0.38 |
| Min | nd | | nd | nd | | nd | 1.17 | | 266 |
| Max | nd | | nd | nd | | nd | 784 | | 434 |
| n (Samp) | nd | | nd | nd | | nd | 575 | | 6 |
| n (Patient) | nd | | nd | nd | | nd | 269 | | 6 |
| | | | | | | | | | |

| UO only | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| Median | nd | | nd | 434 | | 434 | nd | | nd |
| Average | nd | | nd | 331 | | 349 | nd | | nd |
| Stdev | nd | | nd | 218 | | 235 | nd | | nd |
| p(t-test) | nd | | nd | | | 0.80 | nd | | nd |
| Min | nd | | nd | 1.17 | | 52.2 | nd | | nd |
| Max | nd | | nd | 784 | | 784 | nd | | nd |
| n (Samp) | nd | | nd | 533 | | 10 | nd | | nd |
| n (Patient) | nd | | nd | 235 | | 10 | nd | | nd |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.60 | nd | nd | 0.53 | nd | 0.55 | 0.47 | 0.60 | nd |
| SE | 0.096 | nd | nd | 0.077 | nd | 0.095 | 0.10 | 0.12 | nd |
| p | 0.28 | nd | nd | 0.71 | nd | 0.58 | 0.75 | 0.40 | nd |
| nCohort 1 | 558 | nd | nd | 558 | nd | 533 | 558 | 575 | nd |
| nCohort 2 | 10 | nd | nd | 15 | nd | 10 | 8 | 6 | nd |
| Cutoff 1 | 427 | nd | nd | 88.4 | nd | 239 | 265 | 427 | nd |
| Sens 1 | 80% | nd | nd | 73% | nd | 70% | 75% | 83% | nd |
| Spec 1 | 39% | nd | nd | 28% | nd | 33% | 36% | 39% | nd |
| Cutoff 2 | 427 | nd | nd | 62.5 | nd | 87.5 | 19.7 | 427 | nd |
| Sens 2 | 80% | nd | nd | 80% | nd | 80% | 88% | 83% | nd |
| Spec 2 | 39% | nd | nd | 24% | nd | 26% | 10% | 39% | nd |
| Cutoff 3 | 387 | nd | nd | 45.0 | nd | 62.5 | 4.68 | 265 | nd |
| Sens 3 | 90% | nd | nd | 93% | nd | 90% | 100% | 100% | nd |
| Spec 3 | 39% | nd | nd | 20% | nd | 23% | 1% | 36% | nd |
| Cutoff 4 | 434 | nd | nd | 434 | nd | 434 | 434 | 434 | nd |
| Sens 4 | 10% | nd | nd | 13% | nd | 20% | 0% | 0% | nd |
| Spec 4 | 92% | nd | nd | 92% | nd | 92% | 92% | 91% | nd |
| Cutoff 5 | 434 | nd | nd | 434 | nd | 434 | 434 | 434 | nd |
| Sens 5 | 10% | nd | nd | 13% | nd | 20% | 0% | 0% | nd |
| Spec 5 | 92% | nd | nd | 92% | nd | 92% | 92% | 91% | nd |
| Cutoff 6 | 434 | nd | nd | 434 | nd | 434 | 434 | 434 | nd |
| Sens 6 | 10% | nd | nd | 13% | nd | 20% | 0% | 0% | nd |
| Spec 6 | 92% | nd | nd | 92% | nd | 92% | 92% | 91% | nd |
| OR Quart 2 | 8.4 | nd | nd | 0.49 | nd | 0.99 | >0 | >6.3 | nd |
| p Value | 0.046 | nd | nd | 0.42 | nd | 0.99 | <na | <0.091 | nd |
| 95% CI of | 1.0 | nd | nd | 0.089 | nd | 0.14 | >na | >0.74 | nd |
| OR Quart2 | 68 | nd | nd | 2.7 | nd | 7.2 | na | na | nd |
| OR Quart 3 | 0 | nd | nd | 1.8 | nd | 2.0 | >6.3 | >0 | nd |
| p Value | na | nd | nd | 0.36 | nd | 0.42 | <0.091 | <na | nd |
| 95% CI of | na | nd | nd | 0.51 | nd | 0.36 | >0.74 | >na | nd |
| OR Quart3 | na | nd | nd | 6.2 | nd | 11 | na | na | nd |
| OR Quart 4 | 1.0 | nd | nd | 0.49 | nd | 0.99 | >2.0 | >0 | nd |
| p Value | 1.0 | nd | nd | 0.41 | nd | 0.99 | <0.56 | <na | nd |
| 95% CI of | 0.062 | nd | nd | 0.088 | nd | 0.14 | >0.18 | >na | nd |
| OR Quart4 | 16 | nd | nd | 2.7 | nd | 7.2 | na | na | nd |

**Table 10: Comparison of marker levels in EDTA samples collected from Cohort 1 (patients that did not progress beyond RIFLE stage 0, R, or I) and in EDTA samples collected from Cohort 2 (subjects who progress to RIFLE stage F) at 0, 24 hours, and 48 hours prior to the subject reaching RIFLE stage I.**

| **C-C motif chemokine 18** | | | | | | | | | |
|---|---|---|---|---|---|---|---|---|---|
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | nd | | nd | 182 | | 272 | nd | | nd |
| Average | nd | | nd | 245 | | 513 | nd | | nd |
| Stdev | nd | | nd | 205 | | 604 | nd | | nd |

| sCr or UO | 0hr prior to AKI stage | | | 24hr prior to AKI stage | | | 48hr prior to AKI stage | | |
|---|---|---|---|---|---|---|---|---|---|
| | Cohort 1 | | Cohort 2 | Cohort 1 | | Cohort 2 | Cohort 1 | | Cohort 2 |
| p(t-test) | nd | | nd | | | 0.0027 | nd | | nd |
| Min | nd | | nd | 31.7 | | 92.4 | nd | | nd |
| Max | nd | | nd | 2110 | | 1650 | nd | | nd |
| n (Samp) | nd | | nd | 367 | | 6 | nd | | nd |
| n (Patient) | nd | | nd | 197 | | 6 | nd | | nd |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | nd | nd | nd | 0.58 | nd | nd | nd | nd | nd |
| SE | nd | nd | nd | 0.12 | nd | nd | nd | nd | nd |
| p | nd | nd | nd | 0.51 | nd | nd | nd | nd | nd |
| nCohort 1 | nd | nd | nd | 367 | nd | nd | nd | nd | nd |
| nCohort 2 | nd | nd | nd | 6 | nd | nd | nd | nd | nd |
| Cutoff 1 | nd | nd | nd | 107 | nd | nd | nd | nd | nd |
| Sens 1 | nd | nd | nd | 83% | nd | nd | nd | nd | nd |
| Spec 1 | nd | nd | nd | 23% | nd | nd | nd | nd | nd |
| Cutoff 2 | nd | nd | nd | 107 | nd | nd | nd | nd | nd |
| Sens 2 | nd | nd | nd | 83% | nd | nd | nd | nd | nd |
| Spec 2 | nd | nd | nd | 23% | nd | nd | nd | nd | nd |
| Cutoff 3 | nd | nd | nd | 90.9 | nd | nd | nd | nd | nd |
| Sens 3 | nd | nd | nd | 100% | nd | nd | nd | nd | nd |
| Spec 3 | nd | nd | nd | 16% | nd | nd | nd | nd | nd |
| Cutoff 4 | nd | nd | nd | 278 | nd | nd | nd | nd | nd |
| Sens 4 | nd | nd | nd | 50% | nd | nd | nd | nd | nd |
| Spec 4 | nd | nd | nd | 70% | nd | nd | nd | nd | nd |
| Cutoff 5 | nd | nd | nd | 346 | nd | nd | nd | nd | nd |
| Sens 5 | nd | nd | nd | 50% | nd | nd | nd | nd | nd |
| Spec 5 | nd | nd | nd | 80% | nd | nd | nd | nd | nd |
| Cutoff 6 | nd | nd | nd | 491 | nd | nd | nd | nd | nd |
| Sens 6 | nd | nd | nd | 33% | nd | nd | nd | nd | nd |
| Spec 6 | nd | nd | nd | 90% | nd | nd | nd | nd | nd |
| OR Quart 2 | nd | nd | nd | 0.49 | nd | nd | nd | nd | nd |
| p Value | nd | nd | nd | 0.57 | nd | nd | nd | nd | nd |
| 95% CI of | nd | nd | nd | 0.044 | nd | nd | nd | nd | nd |
| OR Quart2 | nd | nd | nd | 5.6 | nd | nd | nd | nd | nd |
| OR Quart 3 | nd | nd | nd | 0 | nd | nd | nd | nd | nd |
| p Value | nd | nd | nd | na | nd | nd | nd | nd | nd |
| 95% CI of | nd | nd | nd | na | nd | nd | nd | nd | nd |
| OR Quart3 | nd | nd | nd | na | nd | nd | nd | nd | nd |
| OR Quart 4 | nd | nd | nd | 1.5 | nd | nd | nd | nd | nd |
| p Value | nd | nd | nd | 0.66 | nd | nd | nd | nd | nd |
| 95% CI of | nd | nd | nd | 0.24 | nd | nd | nd | nd | nd |
| OR Quart4 | nd | nd | nd | 9.2 | nd | nd | nd | nd | nd |

| **C-C motif chemokine 24** | | | | | | | | | |
|---|---|---|---|---|---|---|---|---|---|
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | nd | | nd | 254 | | 278 | nd | | nd |
| Average | nd | | nd | 388 | | 405 | nd | | nd |
| Stdev | nd | | nd | 385 | | 395 | nd | | nd |
| p(t-test) | nd | | nd | | | 0.92 | nd | | nd |
| Min | nd | | nd | 0.0260 | | 81.9 | nd | | nd |
| Max | nd | | nd | 2920 | | 1100 | nd | | nd |
| n (Samp) | nd | | nd | 297 | | 6 | nd | | nd |
| n (Patient) | nd | | nd | 166 | | 6 | nd | | nd |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | nd | nd | nd | 0.51 | nd | nd | nd | nd | nd |
| SE | nd | nd | nd | 0.12 | nd | nd | nd | nd | nd |
| p | nd | nd | nd | 0.93 | nd | nd | nd | nd | nd |
| nCohort 1 | nd | nd | nd | 297 | nd | nd | nd | nd | nd |
| nCohort 2 | nd | nd | nd | 6 | nd | nd | nd | nd | nd |
| Cutoff 1 | nd | nd | nd | 95.2 | nd | nd | nd | nd | nd |
| Sens 1 | nd | nd | nd | 83% | nd | nd | nd | nd | nd |
| Spec 1 | nd | nd | nd | 20% | nd | nd | nd | nd | nd |
| Cutoff 2 | nd | nd | nd | 95.2 | nd | nd | nd | nd | nd |
| Sens 2 | nd | nd | nd | 83% | nd | nd | nd | nd | nd |
| Spec 2 | nd | nd | nd | 20% | nd | nd | nd | nd | nd |
| Cutoff 3 | nd | nd | nd | 80.9 | nd | nd | nd | nd | nd |
| Sens 3 | nd | nd | nd | 100% | nd | nd | nd | nd | nd |
| Spec 3 | nd | nd | nd | 16% | nd | nd | nd | nd | nd |
| Cutoff 4 | nd | nd | nd | 475 | nd | nd | nd | nd | nd |
| Sens 4 | nd | nd | nd | 33% | nd | nd | nd | nd | nd |
| Spec 4 | nd | nd | nd | 70% | nd | nd | nd | nd | nd |
| Cutoff 5 | nd | nd | nd | 589 | nd | nd | nd | nd | nd |
| Sens 5 | nd | nd | nd | 33% | nd | nd | nd | nd | nd |
| Spec 5 | nd | nd | nd | 80% | nd | nd | nd | nd | nd |
| Cutoff 6 | nd | nd | nd | 900 | nd | nd | nd | nd | nd |
| Sens 6 | nd | nd | nd | 17% | nd | nd | nd | nd | nd |
| Spec 6 | nd | nd | nd | 90% | nd | nd | nd | nd | nd |
| OR Quart 2 | nd | nd | nd | 0.49 | nd | nd | nd | nd | nd |
| p Value | nd | nd | nd | 0.56 | nd | nd | nd | nd | nd |
| 95% CI of | nd | nd | nd | 0.043 | nd | nd | nd | nd | nd |
| OR Quart2 | nd | nd | nd | 5.5 | nd | nd | nd | nd | nd |
| OR Quart 3 | nd | nd | nd | 0.49 | nd | nd | nd | nd | nd |
| p Value | nd | nd | nd | 0.56 | nd | nd | nd | nd | nd |
| 95% CI of | nd | nd | nd | 0.043 | nd | nd | nd | nd | nd |
| OR Quart3 | nd | nd | nd | 5.5 | nd | nd | nd | nd | nd |
| OR Quart 4 | nd | nd | nd | 0.99 | nd | nd | nd | nd | nd |
| p Value | nd | nd | nd | 0.99 | nd | nd | nd | nd | nd |
| 95% CI of | nd | nd | nd | 0.14 | nd | nd | nd | nd | nd |
| OR Quart4 | nd | nd | nd | 7.2 | nd | nd | nd | nd | nd |

| **C-C motif chemokine 8** | | | | | | | | | |
|---|---|---|---|---|---|---|---|---|---|
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | nd | | nd | 13.4 | | 16.7 | nd | | nd |
| Average | nd | | nd | 17.8 | | 29.4 | nd | | nd |
| Stdev | nd | | nd | 23.5 | | 35.7 | nd | | nd |
| p(t-test) | nd | | nd | | | 0.24 | nd | | nd |
| Min | nd | | nd | 0.162 | | 6.24 | nd | | nd |
| Max | nd | | nd | 180 | | 101 | nd | | nd |
| n (Samp) | nd | | nd | 297 | | 6 | nd | | nd |
| n (Patient) | nd | | nd | 166 | | 6 | nd | | nd |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | nd | nd | nd | 0.62 | nd | nd | nd | nd | nd |
| SE | nd | nd | nd | 0.12 | nd | nd | nd | nd | nd |
| p | nd | nd | nd | 0.34 | nd | nd | nd | nd | nd |
| nCohort 1 | nd | nd | nd | 297 | nd | nd | nd | nd | nd |
| nCohort 2 | nd | nd | nd | 6 | nd | nd | nd | nd | nd |
| Cutoff 1 | nd | nd | nd | 11.5 | nd | nd | nd | nd | nd |
| Sens 1 | nd | nd | nd | 83% | nd | nd | nd | nd | nd |
| Spec 1 | nd | nd | nd | 39% | nd | nd | nd | nd | nd |
| Cutoff 2 | nd | nd | nd | 11.5 | nd | nd | nd | nd | nd |
| Sens 2 | nd | nd | nd | 83% | nd | nd | nd | nd | nd |
| Spec 2 | nd | nd | nd | 39% | nd | nd | nd | nd | nd |
| Cutoff 3 | nd | nd | nd | 5.84 | nd | nd | nd | nd | nd |
| Sens 3 | nd | nd | nd | 100% | nd | nd | nd | nd | nd |
| Spec 3 | nd | nd | nd | 17% | nd | nd | nd | nd | nd |
| Cutoff 4 | nd | nd | nd | 17.1 | nd | nd | nd | nd | nd |
| Sens 4 | nd | nd | nd | 50% | nd | nd | nd | nd | nd |
| Spec 4 | nd | nd | nd | 70% | nd | nd | nd | nd | nd |
| Cutoff 5 | nd | nd | nd | 20.1 | nd | nd | nd | nd | nd |
| Sens 5 | nd | nd | nd | 33% | nd | nd | nd | nd | nd |
| Spec 5 | nd | nd | nd | 80% | nd | nd | nd | nd | nd |
| Cutoff 6 | nd | nd | nd | 30.5 | nd | nd | nd | nd | nd |
| Sens 6 | nd | nd | nd | 17% | nd | nd | nd | nd | nd |
| Spec 6 | nd | nd | nd | 90% | nd | nd | nd | nd | nd |
| OR Quart 2 | nd | nd | nd | 0.99 | nd | nd | nd | nd | nd |
| p Value | nd | nd | nd | 0.99 | nd | nd | nd | nd | nd |
| 95% CI of | nd | nd | nd | 0.061 | nd | nd | nd | nd | nd |
| OR Quart2 | nd | nd | nd | 16 | nd | nd | nd | nd | nd |
| OR Quart 3 | nd | nd | nd | 0.99 | nd | nd | nd | nd | nd |
| p Value | nd | nd | nd | 0.99 | nd | nd | nd | nd | nd |
| 95% CI of | nd | nd | nd | 0.061 | nd | nd | nd | nd | nd |
| OR Quart3 | nd | nd | nd | 16 | nd | nd | nd | nd | nd |
| OR Quart 4 | nd | nd | nd | 3.0 | nd | nd | nd | nd | nd |
| p Value | nd | nd | nd | 0.34 | nd | nd | nd | nd | nd |
| 95% CI of | nd | nd | nd | 0.31 | nd | nd | nd | nd | nd |
| OR Quart4 | nd | nd | nd | 30 | nd | nd | nd | nd | nd |

| **Cathepsin D** | | | | | | | | | |
|---|---|---|---|---|---|---|---|---|---|
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | nd | | nd | 225000 | | 379000 | nd | | nd |
| Average | nd | | nd | 260000 | | 408000 | nd | | nd |
| Stdev | nd | | nd | 165000 | | 159000 | nd | | nd |
| p(t-test) | nd | | nd | | | 0.030 | nd | | nd |
| Min | nd | | nd | 34100 | | 230000 | nd | | nd |
| Max | nd | | nd | 1870000 | | 655000 | nd | | nd |
| n (Samp) | nd | | nd | 366 | | 6 | nd | | nd |
| n (Patient) | nd | | nd | 196 | | 6 | nd | | nd |

| | | | | | | | | | |
|---|---|---|---|---|---|---|---|---|---|
| | 0hr prior to AKI stage | | | 24hr prior to AKI stage | | | 48hr prior to AKI stage | | |
| | sCr or UO | sCr only | UO only | sCr or UO | sCr only | UO only | sCr or UO | sCr only | UO only |
| AUC | nd | nd | nd | 0.79 | nd | nd | nd | nd | nd |
| SE | nd | nd | nd | 0.11 | nd | nd | nd | nd | nd |
| p | nd | nd | nd | 0.0096 | nd | nd | nd | nd | nd |
| nCohort 1 | nd | nd | nd | 366 | nd | nd | nd | nd | nd |
| nCohort 2 | nd | nd | nd | 6 | nd | nd | nd | nd | nd |
| Cutoff 1 | nd | nd | nd | 295000 | nd | nd | nd | nd | nd |
| Sens 1 | nd | nd | nd | 83% | nd | nd | nd | nd | nd |
| Spec 1 | nd | nd | nd | 67% | nd | nd | nd | nd | nd |
| Cutoff 2 | nd | nd | nd | 295000 | nd | nd | nd | nd | nd |
| Sens 2 | nd | nd | nd | 83% | nd | nd | nd | nd | nd |
| Spec 2 | nd | nd | nd | 67% | nd | nd | nd | nd | nd |
| Cutoff 3 | nd | nd | nd | 229000 | nd | nd | nd | nd | nd |
| Sens 3 | nd | nd | nd | 100% | nd | nd | nd | nd | nd |
| Spec 3 | nd | nd | nd | 51% | nd | nd | nd | nd | nd |
| Cutoff 4 | nd | nd | nd | 306000 | nd | nd | nd | nd | nd |
| Sens 4 | nd | nd | nd | 67% | nd | nd | nd | nd | nd |
| Spec 4 | nd | nd | nd | 70% | nd | nd | nd | nd | nd |
| Cutoff 5 | nd | nd | nd | 357000 | nd | nd | nd | nd | nd |
| Sens 5 | nd | nd | nd | 50% | nd | nd | nd | nd | nd |
| Spec 5 | nd | nd | nd | 80% | nd | nd | nd | nd | nd |
| Cutoff 6 | nd | nd | nd | 449000 | nd | nd | nd | nd | nd |
| Sens 6 | nd | nd | nd | 50% | nd | nd | nd | nd | nd |
| Spec 6 | nd | nd | nd | 90% | nd | nd | nd | nd | nd |
| OR Quart 2 | nd | nd | nd | >0 | nd | nd | nd | nd | nd |
| p Value | nd | nd | nd | <na | nd | nd | nd | nd | nd |
| 95% CI of | nd | nd | nd | >na | nd | nd | nd | nd | nd |
| OR Quart2 | nd | nd | nd | na | nd | nd | nd | nd | nd |
| OR Quart 3 | nd | nd | nd | >3.1 | nd | nd | nd | nd | nd |
| p Value | nd | nd | nd | <0.33 | nd | nd | nd | nd | nd |
| 95% CI of | nd | nd | nd | >0.32 | nd | nd | nd | nd | nd |
| OR Quart3 | nd | nd | nd | na | nd | nd | nd | nd | nd |
| OR Quart 4 | nd | nd | nd | >3.1 | nd | nd | nd | nd | nd |
| p Value | nd | nd | nd | <0.33 | nd | nd | nd | nd | nd |
| 95% CI of | nd | nd | nd | >0.32 | nd | nd | nd | nd | nd |
| OR Quart4 | nd | nd | nd | na | nd | nd | nd | nd | nd |

| **C-X-C motif chemokine 13** | | | | | | | | | |
|---|---|---|---|---|---|---|---|---|---|
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | nd | | nd | 51.9 | | 73.9 | nd | | nd |
| Average | nd | | nd | 129 | | 89.7 | nd | | nd |
| Stdev | nd | | nd | 258 | | 39.2 | nd | | nd |
| p(t-test) | nd | | nd | | | 0.71 | nd | | nd |
| Min | nd | | nd | 9.90 | | 45.0 | nd | | nd |
| Max | nd | | nd | 2000 | | 141 | nd | | nd |
| n (Samp) | nd | | nd | 297 | | 6 | nd | | nd |
| n (Patient) | nd | | nd | 166 | | 6 | nd | | nd |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | nd | nd | nd | 0.64 | nd | nd | nd | nd | nd |
| SE | nd | nd | nd | 0.12 | nd | nd | nd | nd | nd |
| p | nd | nd | nd | 0.24 | nd | nd | nd | nd | nd |
| nCohort 1 | nd | nd | nd | 297 | nd | nd | nd | nd | nd |
| nCohort 2 | nd | nd | nd | 6 | nd | nd | nd | nd | nd |
| Cutoff 1 | nd | nd | nd | 67.5 | nd | nd | nd | nd | nd |
| Sens 1 | nd | nd | nd | 83% | nd | nd | nd | nd | nd |
| Spec 1 | nd | nd | nd | 58% | nd | nd | nd | nd | nd |
| Cutoff 2 | nd | nd | nd | 67.5 | nd | nd | nd | nd | nd |
| Sens 2 | nd | nd | nd | 83% | nd | nd | nd | nd | nd |
| Spec 2 | nd | nd | nd | 58% | nd | nd | nd | nd | nd |
| Cutoff 3 | nd | nd | nd | 44.8 | nd | nd | nd | nd | nd |
| Sens 3 | nd | nd | nd | 100% | nd | nd | nd | nd | nd |
| Spec 3 | nd | nd | nd | 44% | nd | nd | nd | nd | nd |
| Cutoff 4 | nd | nd | nd | 95.3 | nd | nd | nd | nd | nd |
| Sens 4 | nd | nd | nd | 33% | nd | nd | nd | nd | nd |
| Spec 4 | nd | nd | nd | 70% | nd | nd | nd | nd | nd |
| Cutoff 5 | nd | nd | nd | 134 | nd | nd | nd | nd | nd |
| Sens 5 | nd | nd | nd | 33% | nd | nd | nd | nd | nd |
| Spec 5 | nd | nd | nd | 80% | nd | nd | nd | nd | nd |
| Cutoff 6 | nd | nd | nd | 250 | nd | nd | nd | nd | nd |
| Sens 6 | nd | nd | nd | 0% | nd | nd | nd | nd | nd |
| Spec 6 | nd | nd | nd | 90% | nd | nd | nd | nd | nd |
| OR Quart 2 | nd | nd | nd | >1.0 | nd | nd | nd | nd | nd |
| p Value | nd | nd | nd | <1.0 | nd | nd | nd | nd | nd |
| 95% CI of | nd | nd | nd | >0.061 | nd | nd | nd | nd | nd |
| OR Quart2 | nd | nd | nd | na | nd | nd | nd | nd | nd |
| OR Quart 3 | nd | nd | nd | >3.1 | nd | nd | nd | nd | nd |
| p Value | nd | nd | nd | <0.33 | nd | nd | nd | nd | nd |
| 95% CI of | nd | nd | nd | >0.31 | nd | nd | nd | nd | nd |
| OR Quart3 | nd | nd | nd | na | nd | nd | nd | nd | nd |
| OR Quart 4 | nd | nd | nd | >2.0 | nd | nd | nd | nd | nd |
| p Value | nd | nd | nd | <0.57 | nd | nd | nd | nd | nd |
| 95% CI of | nd | nd | nd | >0.18 | nd | nd | nd | nd | nd |
| OR Quart4 | nd | nd | nd | na | nd | nd | nd | nd | nd |

| **Insulin-like growth factor-binding protein 3** | | | | | | | | | |
|---|---|---|---|---|---|---|---|---|---|
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | nd | | nd | 3150 | | 3200 | nd | | nd |
| Average | nd | | nd | 3500 | | 3580 | nd | | nd |
| Stdev | nd | | nd | 1590 | | 2040 | nd | | nd |
| p(t-test) | nd | | nd | | | 0.90 | nd | | nd |
| Min | nd | | nd | 651 | | 1380 | nd | | nd |
| Max | nd | | nd | 8600 | | 7360 | nd | | nd |
| n (Samp) | nd | | nd | 367 | | 6 | nd | | nd |
| n (Patient) | nd | | nd | 197 | | 6 | nd | | nd |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | nd | nd | nd | 0.50 | nd | nd | nd | nd | nd |
| SE | nd | nd | nd | 0.12 | nd | nd | nd | nd | nd |
| p | nd | nd | nd | 0.98 | nd | nd | nd | nd | nd |
| nCohort 1 | nd | nd | nd | 367 | nd | nd | nd | nd | nd |
| nCohort 2 | nd | nd | nd | 6 | nd | nd | nd | nd | nd |
| Cutoff 1 | nd | nd | nd | 2410 | nd | nd | nd | nd | nd |
| Sens 1 | nd | nd | nd | 83% | nd | nd | nd | nd | nd |
| Spec 1 | nd | nd | nd | 30% | nd | nd | nd | nd | nd |
| Cutoff 2 | nd | nd | nd | 2410 | nd | nd | nd | nd | nd |
| Sens 2 | nd | nd | nd | 83% | nd | nd | nd | nd | nd |
| Spec 2 | nd | nd | nd | 30% | nd | nd | nd | nd | nd |
| Cutoff 3 | nd | nd | nd | 1380 | nd | nd | nd | nd | nd |
| Sens 3 | nd | nd | nd | 100% | nd | nd | nd | nd | nd |
| Spec 3 | nd | nd | nd | 6% | nd | nd | nd | nd | nd |
| Cutoff 4 | nd | nd | nd | 4250 | nd | nd | nd | nd | nd |
| Sens 4 | nd | nd | nd | 17% | nd | nd | nd | nd | nd |
| Spec 4 | nd | nd | nd | 70% | nd | nd | nd | nd | nd |
| Cutoff 5 | nd | nd | nd | 4870 | nd | nd | nd | nd | nd |
| Sens 5 | nd | nd | nd | 17% | nd | nd | nd | nd | nd |
| Spec 5 | nd | nd | nd | 80% | nd | nd | nd | nd | nd |
| Cutoff 6 | nd | nd | nd | 5780 | nd | nd | nd | nd | nd |
| Sens 6 | nd | nd | nd | 17% | nd | nd | nd | nd | nd |
| Spec 6 | nd | nd | nd | 90% | nd | nd | nd | nd | nd |
| OR Quart 2 | nd | nd | nd | 2.0 | nd | nd | nd | nd | nd |
| p Value | nd | nd | nd | 0.56 | nd | nd | nd | nd | nd |
| 95% CI of | nd | nd | nd | 0.18 | nd | nd | nd | nd | nd |
| OR Quart2 | nd | nd | nd | 23 | nd | nd | nd | nd | nd |
| OR Quart 3 | nd | nd | nd | 2.0 | nd | nd | nd | nd | nd |
| p Value | nd | nd | nd | 0.56 | nd | nd | nd | nd | nd |
| 95% CI of | nd | nd | nd | 0.18 | nd | nd | nd | nd | nd |
| OR Quart3 | nd | nd | nd | 23 | nd | nd | nd | nd | nd |
| OR Quart 4 | nd | nd | nd | 1.0 | nd | nd | nd | nd | nd |
| p Value | nd | nd | nd | 0.99 | nd | nd | nd | nd | nd |
| 95% CI of | nd | nd | nd | 0.062 | nd | nd | nd | nd | nd |
| OR Quart4 | nd | nd | nd | 16 | nd | nd | nd | nd | nd |

| **Interleukin-11** | | | | | | | | | |
|---|---|---|---|---|---|---|---|---|---|
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | nd | | nd | 62.8 | | 83.7 | nd | | nd |
| Average | nd | | nd | 147 | | 102 | nd | | nd |
| Stdev | nd | | nd | 555 | | 53.4 | nd | | nd |
| p(t-test) | nd | | nd | | | 0.84 | nd | | nd |
| Min | nd | | nd | 0.359 | | 51.1 | nd | | nd |
| Max | nd | | nd | 6920 | | 171 | nd | | nd |
| n (Samp) | nd | | nd | 298 | | 6 | nd | | nd |
| n (Patient) | nd | | nd | 167 | | 6 | nd | | nd |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | nd | nd | nd | 0.63 | nd | nd | nd | nd | nd |
| SE | nd | nd | nd | 0.12 | nd | nd | nd | nd | nd |
| p | nd | nd | nd | 0.29 | nd | nd | nd | nd | nd |
| nCohort 1 | nd | nd | nd | 298 | nd | nd | nd | nd | nd |
| nCohort 2 | nd | nd | nd | 6 | nd | nd | nd | nd | nd |
| Cutoff 1 | nd | nd | nd | 51.1 | nd | nd | nd | nd | nd |
| Sens 1 | nd | nd | nd | 83% | nd | nd | nd | nd | nd |
| Spec 1 | nd | nd | nd | 46% | nd | nd | nd | nd | nd |
| Cutoff 2 | nd | nd | nd | 51.1 | nd | nd | nd | nd | nd |
| Sens 2 | nd | nd | nd | 83% | nd | nd | nd | nd | nd |
| Spec 2 | nd | nd | nd | 46% | nd | nd | nd | nd | nd |
| Cutoff 3 | nd | nd | nd | 47.4 | nd | nd | nd | nd | nd |
| Sens 3 | nd | nd | nd | 100% | nd | nd | nd | nd | nd |
| Spec 3 | nd | nd | nd | 46% | nd | nd | nd | nd | nd |
| Cutoff 4 | nd | nd | nd | 111 | nd | nd | nd | nd | nd |
| Sens 4 | nd | nd | nd | 33% | nd | nd | nd | nd | nd |
| Spec 4 | nd | nd | nd | 70% | nd | nd | nd | nd | nd |
| Cutoff 5 | nd | nd | nd | 158 | nd | nd | nd | nd | nd |
| Sens 5 | nd | nd | nd | 33% | nd | nd | nd | nd | nd |
| Spec 5 | nd | nd | nd | 81% | nd | nd | nd | nd | nd |
| Cutoff 6 | nd | nd | nd | 221 | nd | nd | nd | nd | nd |
| Sens 6 | nd | nd | nd | 0% | nd | nd | nd | nd | nd |
| Spec 6 | nd | nd | nd | 90% | nd | nd | nd | nd | nd |
| OR Quart 2 | nd | nd | nd | >2.1 | nd | nd | nd | nd | nd |
| p Value | nd | nd | nd | <0.56 | nd | nd | nd | nd | nd |
| 95% CI of | nd | nd | nd | >0.18 | nd | nd | nd | nd | nd |
| OR Quart2 | nd | nd | nd | na | nd | nd | nd | nd | nd |
| OR Quart 3 | nd | nd | nd | >2.1 | nd | nd | nd | nd | nd |
| p Value | nd | nd | nd | <0.56 | nd | nd | nd | nd | nd |
| 95% CI of | nd | nd | nd | >0.18 | nd | nd | nd | nd | nd |
| OR Quart3 | nd | nd | nd | na | nd | nd | nd | nd | nd |
| OR Quart 4 | nd | nd | nd | >2.1 | nd | nd | nd | nd | nd |
| +p Value | nd | nd | nd | <0.56 | nd | nd | nd | nd | nd |
| 95% CI of | nd | nd | nd | >0.18 | nd | nd | nd | nd | nd |
| OR Quart4 | nd | nd | nd | na | nd | nd | nd | nd | nd |

| **Interleukin-2 receptor alpha chain** | | | | | | | | | |
|---|---|---|---|---|---|---|---|---|---|
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | nd | | nd | 19.4 | | 28.9 | nd | | nd |
| Average | nd | | nd | 70.5 | | 48.1 | nd | | nd |
| Stdev | nd | | nd | 148 | | 56.2 | nd | | nd |
| p(t-test) | nd | | nd | | | 0.71 | nd | | nd |
| Min | nd | | nd | 0.0290 | | 0.0290 | nd | | nd |
| Max | nd | | nd | 1040 | | 154 | nd | | nd |
| n (Samp) | nd | | nd | 298 | | 6 | nd | | nd |
| n (Patient) | nd | | nd | 167 | | 6 | nd | | nd |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | nd | nd | nd | 0.54 | nd | nd | nd | nd | nd |
| SE | nd | nd | nd | 0.12 | nd | nd | nd | nd | nd |
| p | nd | nd | nd | 0.77 | nd | nd | nd | nd | nd |
| nCohort 1 | nd | nd | nd | 298 | nd | nd | nd | nd | nd |
| nCohort 2 | nd | nd | nd | 6 | nd | nd | nd | nd | nd |
| Cutoff 1 | nd | nd | nd | 11.9 | nd | nd | nd | nd | nd |
| Sens 1 | nd | nd | nd | 83% | nd | nd | nd | nd | nd |
| Spec 1 | nd | nd | nd | 43% | nd | nd | nd | nd | nd |
| Cutoff 2 | nd | nd | nd | 11.9 | nd | nd | nd | nd | nd |
| Sens 2 | nd | nd | nd | 83% | nd | nd | nd | nd | nd |
| Spec 2 | nd | nd | nd | 43% | nd | nd | nd | nd | nd |
| Cutoff 3 | nd | nd | nd | 0 | nd | nd | nd | nd | nd |
| Sens 3 | nd | nd | nd | 100% | nd | nd | nd | nd | nd |
| Spec 3 | nd | nd | nd | 0% | nd | nd | nd | nd | nd |
| Cutoff 4 | nd | nd | nd | 50.7 | nd | nd | nd | nd | nd |
| Sens 4 | nd | nd | nd | 33% | nd | nd | nd | nd | nd |
| Spec 4 | nd | nd | nd | 70% | nd | nd | nd | nd | nd |
| Cutoff 5 | nd | nd | nd | 92.7 | nd | nd | nd | nd | nd |
| Sens 5 | nd | nd | nd | 17% | nd | nd | nd | nd | nd |
| Spec 5 | nd | nd | nd | 80% | nd | nd | nd | nd | nd |
| Cutoff 6 | nd | nd | nd | 167 | nd | nd | nd | nd | nd |
| Sens 6 | nd | nd | nd | 0% | nd | nd | nd | nd | nd |
| Spec 6 | nd | nd | nd | 90% | nd | nd | nd | nd | nd |
| OR Quart 2 | nd | nd | nd | 1.0 | nd | nd | nd | nd | nd |
| p Value | nd | nd | nd | 1.0 | nd | nd | nd | nd | nd |
| 95% CI of | nd | nd | nd | 0.061 | nd | nd | nd | nd | nd |
| OR Quart2 | nd | nd | nd | 16 | nd | nd | nd | nd | nd |
| OR Quart 3 | nd | nd | nd | 3.1 | nd | nd | nd | nd | nd |
| p Value | nd | nd | nd | 0.33 | nd | nd | nd | nd | nd |
| 95% CI of | nd | nd | nd | 0.31 | nd | nd | nd | nd | nd |
| OR Quart3 | nd | nd | nd | 30 | nd | nd | nd | nd | nd |
| OR Quart 4 | nd | nd | nd | 1.0 | nd | nd | nd | nd | nd |
| p Value | nd | nd | nd | 1.0 | nd | nd | nd | nd | nd |
| 95% CI of | nd | nd | nd | 0.061 | nd | nd | nd | nd | nd |
| OR Quart4 | nd | nd | nd | 16 | nd | nd | nd | nd | nd |

| **Neutrophil collagenase** | | | | | | | | | |
|---|---|---|---|---|---|---|---|---|---|
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | nd | | nd | 1100 | | 5350 | nd | | nd |
| Average | nd | | nd | 3160 | | 14200 | nd | | nd |
| Stdev | nd | | nd | 6940 | | 17400 | nd | | nd |
| p(t-test) | nd | | nd | | | 2.3E-4 | nd | | nd |
| Min | nd | | nd | 40.0 | | 1.14 | nd | | nd |
| Max | nd | | nd | 55000 | | 38700 | nd | | nd |
| n (Samp) | nd | | nd | 366 | | 6 | nd | | nd |
| n (Patient) | nd | | nd | 197 | | 6 | nd | | nd |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | nd | nd | nd | 0.72 | nd | nd | nd | nd | nd |
| SE | nd | nd | nd | 0.12 | nd | nd | nd | nd | nd |
| p | nd | nd | nd | 0.059 | nd | nd | nd | nd | nd |
| nCohort 1 | nd | nd | nd | 366 | nd | nd | nd | nd | nd |
| nCohort 2 | nd | nd | nd | 6 | nd | nd | nd | nd | nd |
| Cutoff 1 | nd | nd | nd | 1500 | nd | nd | nd | nd | nd |
| Sens 1 | nd | nd | nd | 83% | nd | nd | nd | nd | nd |
| Spec 1 | nd | nd | nd | 64% | nd | nd | nd | nd | nd |
| Cutoff 2 | nd | nd | nd | 1500 | nd | nd | nd | nd | nd |
| Sens 2 | nd | nd | nd | 83% | nd | nd | nd | nd | nd |
| Spec 2 | nd | nd | nd | 64% | nd | nd | nd | nd | nd |
| Cutoff 3 | nd | nd | nd | 0 | nd | nd | nd | nd | nd |
| Sens 3 | nd | nd | nd | 100% | nd | nd | nd | nd | nd |
| Spec 3 | nd | nd | nd | 0% | nd | nd | nd | nd | nd |
| Cutoff 4 | nd | nd | nd | 1830 | nd | nd | nd | nd | nd |
| Sens 4 | nd | nd | nd | 67% | nd | nd | nd | nd | nd |
| Spec 4 | nd | nd | nd | 70% | nd | nd | nd | nd | nd |
| Cutoff 5 | nd | nd | nd | 3110 | nd | nd | nd | nd | nd |
| Sens 5 | nd | nd | nd | 67% | nd | nd | nd | nd | nd |
| Spec 5 | nd | nd | nd | 80% | nd | nd | nd | nd | nd |
| Cutoff 6 | nd | nd | nd | 6510 | nd | nd | nd | nd | nd |
| Sens 6 | nd | nd | nd | 50% | nd | nd | nd | nd | nd |
| Spec 6 | nd | nd | nd | 90% | nd | nd | nd | nd | nd |
| OR Quart 2 | nd | nd | nd | 0 | nd | nd | nd | nd | nd |
| p Value | nd | nd | nd | na | nd | nd | nd | nd | nd |
| 95% CI of | nd | nd | nd | na | nd | nd | nd | nd | nd |
| OR Quart2 | nd | nd | nd | na | nd | nd | nd | nd | nd |
| OR Quart 3 | nd | nd | nd | 1.0 | nd | nd | nd | nd | nd |
| p Value | nd | nd | nd | 1.0 | nd | nd | nd | nd | nd |
| 95% CI of | nd | nd | nd | 0.062 | nd | nd | nd | nd | nd |
| OR Quart3 | nd | nd | nd | 16 | nd | nd | nd | nd | nd |
| OR Quart 4 | nd | nd | nd | 4.1 | nd | nd | nd | nd | nd |
| p Value | nd | nd | nd | 0.21 | nd | nd | nd | nd | nd |
| 95% CI of | nd | nd | nd | 0.45 | nd | nd | nd | nd | nd |
| OR Quart4 | nd | nd | nd | 38 | nd | nd | nd | nd | nd |

| **Protransforming growth factor alpha** | | | | | | | | | |
|---|---|---|---|---|---|---|---|---|---|
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | nd | | nd | 1.45 | | 6.40 | nd | | nd |
| Average | nd | | nd | 7.21 | | 17.3 | nd | | nd |
| Stdev | nd | | nd | 27.5 | | 26.5 | nd | | nd |
| p(t-test) | nd | | nd | | | 0.37 | nd | | nd |
| Min | nd | | nd | 0.00228 | | 0.0105 | nd | | nd |
| Max | nd | | nd | 287 | | 68.3 | nd | | nd |
| n (Samp) | nd | | nd | 298 | | 6 | nd | | nd |
| n (Patient) | nd | | nd | 167 | | 6 | nd | | nd |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | nd | nd | nd | 0.60 | nd | nd | nd | nd | nd |
| SE | nd | nd | nd | 0.12 | nd | nd | nd | nd | nd |
| p | nd | nd | nd | 0.42 | nd | nd | nd | nd | nd |
| nCohort 1 | nd | nd | nd | 298 | nd | nd | nd | nd | nd |
| nCohort 2 | nd | nd | nd | 6 | nd | nd | nd | nd | nd |
| Cutoff 1 | nd | nd | nd | 0.190 | nd | nd | nd | nd | nd |
| Sens 1 | nd | nd | nd | 83% | nd | nd | nd | nd | nd |
| Spec 1 | nd | nd | nd | 23% | nd | nd | nd | nd | nd |
| Cutoff 2 | nd | nd | nd | 0.190 | nd | nd | nd | nd | nd |
| Sens 2 | nd | nd | nd | 83% | nd | nd | nd | nd | nd |
| Spec 2 | nd | nd | nd | 23% | nd | nd | nd | nd | nd |
| Cutoff 3 | nd | nd | nd | 0.00584 | nd | nd | nd | nd | nd |
| Sens 3 | nd | nd | nd | 100% | nd | nd | nd | nd | nd |
| Spec 3 | nd | nd | nd | 16% | nd | nd | nd | nd | nd |
| Cutoff 4 | nd | nd | nd | 3.12 | nd | nd | nd | nd | nd |
| Sens 4 | nd | nd | nd | 50% | nd | nd | nd | nd | nd |
| Spec 4 | nd | nd | nd | 70% | nd | nd | nd | nd | nd |
| Cutoff 5 | nd | nd | nd | 4.80 | nd | nd | nd | nd | nd |
| Sens 5 | nd | nd | nd | 50% | nd | nd | nd | nd | nd |
| Spec 5 | nd | nd | nd | 81% | nd | nd | nd | nd | nd |
| Cutoff 6 | nd | nd | nd | 9.07 | nd | nd | nd | nd | nd |
| Sens 6 | nd | nd | nd | 50% | nd | nd | nd | nd | nd |
| Spec 6 | nd | nd | nd | 90% | nd | nd | nd | nd | nd |
| OR Quart 2 | nd | nd | nd | 0.49 | nd | nd | nd | nd | nd |
| p Value | nd | nd | nd | 0.57 | nd | nd | nd | nd | nd |
| 95% CI of | nd | nd | nd | 0.044 | nd | nd | nd | nd | nd |
| OR Quart2 | nd | nd | nd | 5.6 | nd | nd | nd | nd | nd |
| OR Quart 3 | nd | nd | nd | 0 | nd | nd | nd | nd | nd |
| p Value | nd | nd | nd | na | nd | nd | nd | nd | nd |
| 95% CI of | nd | nd | nd | na | nd | nd | nd | nd | nd |
| OR Quart3 | nd | nd | nd | na | nd | nd | nd | nd | nd |
| OR Quart 4 | nd | nd | nd | 1.5 | nd | nd | nd | nd | nd |
| p Value | nd | nd | nd | 0.65 | nd | nd | nd | nd | nd |
| 95% CI of | nd | nd | nd | 0.25 | nd | nd | nd | nd | nd |
| OR Quart4 | nd | nd | nd | 9.4 | nd | nd | nd | nd | nd |

| **CA 15-3** | | | | | | | | | |
|---|---|---|---|---|---|---|---|---|---|
| sCr or UO | 0hr prior to AKI stage | | | 24hr prior to AKI stage | | | 48hr prior to AKI stage | | |
| | Cohort 1 | | Cohort 2 | Cohort 1 | | Cohort 2 | Cohort 1 | | Cohort 2 |
| Median | nd | | nd | 0.286 | | 0.833 | nd | | nd |
| Average | nd | | nd | 1.00 | | 1.13 | nd | | nd |
| Stdev | nd | | nd | 4.15 | | 0.884 | nd | | nd |
| p(t-test) | nd | | nd | | | 0.94 | nd | | nd |
| Min | nd | | nd | 0.00154 | | 0.249 | nd | | nd |
| Max | nd | | nd | 60.0 | | 2.69 | nd | | nd |
| n (Samp) | nd | | nd | 367 | | 6 | nd | | nd |
| n (Patient) | nd | | nd | 197 | | 6 | nd | | nd |
| | | | | | | | | | |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | nd | nd | nd | 0.80 | nd | nd | nd | nd | nd |
| SE | nd | nd | nd | 0.11 | nd | nd | nd | nd | nd |
| p | nd | nd | nd | 0.0065 | nd | nd | nd | nd | nd |
| nCohort 1 | nd | nd | nd | 367 | nd | nd | nd | nd | nd |
| nCohort 2 | nd | nd | nd | 6 | nd | nd | nd | nd | nd |
| Cutoff 1 | nd | nd | nd | 0.597 | nd | nd | nd | nd | nd |
| Sens 1 | nd | nd | nd | 83% | nd | nd | nd | nd | nd |
| Spec 1 | nd | nd | nd | 78% | nd | nd | nd | nd | nd |
| Cutoff 2 | nd | nd | nd | 0.597 | nd | nd | nd | nd | nd |
| Sens 2 | nd | nd | nd | 83% | nd | nd | nd | nd | nd |
| Spec 2 | nd | nd | nd | 78% | nd | nd | nd | nd | nd |
| Cutoff 3 | nd | nd | nd | 0.249 | nd | nd | nd | nd | nd |
| Sens 3 | nd | nd | nd | 100% | nd | nd | nd | nd | nd |
| Spec 3 | nd | nd | nd | 46% | nd | nd | nd | nd | nd |
| Cutoff 4 | nd | nd | nd | 0.466 | nd | nd | nd | nd | nd |
| Sens 4 | nd | nd | nd | 83% | nd | nd | nd | nd | nd |
| Spec 4 | nd | nd | nd | 70% | nd | nd | nd | nd | nd |
| Cutoff 5 | nd | nd | nd | 0.680 | nd | nd | nd | nd | nd |
| Sens 5 | nd | nd | nd | 67% | nd | nd | nd | nd | nd |
| Spec 5 | nd | nd | nd | 80% | nd | nd | nd | nd | nd |
| Cutoff 6 | nd | nd | nd | 1.26 | nd | nd | nd | nd | nd |
| Sens 6 | nd | nd | nd | 33% | nd | nd | nd | nd | nd |
| Spec 6 | nd | nd | nd | 90% | nd | nd | nd | nd | nd |
| OR Quart 2 | nd | nd | nd | >1.0 | nd | nd | nd | nd | nd |
| p Value | nd | nd | nd | <0.99 | nd | nd | nd | nd | nd |
| 95% CI of | nd | nd | nd | >0.062 | nd | nd | nd | nd | nd |
| OR Quart2 | nd | nd | nd | na | nd | nd | nd | nd | nd |
| OR Quart 3 | nd | nd | nd | >0 | nd | nd | nd | nd | nd |
| p Value | nd | nd | nd | <na | nd | nd | nd | nd | nd |
| 95% CI of | nd | nd | nd | >na | nd | nd | nd | nd | nd |
| OR Quart3 | nd | nd | nd | na | nd | nd | nd | nd | nd |
| OR Quart 4 | nd | nd | nd | >5.2 | nd | nd | nd | nd | nd |
| p Value | nd | nd | nd | <0.13 | nd | nd | nd | nd | nd |
| 95% CI of | nd | nd | nd | >0.60 | nd | nd | nd | nd | nd |
| OR Quart4 | nd | nd | nd | na | nd | nd | nd | nd | nd |

**Table 11: Comparison of marker levels in enroll urine samples collected from Cohort 1 (patients that did not progress beyond RIFLE stage 0 or R within 48hrs) and in enroll urine samples collected from Cohort 2 (subjects reaching RIFLE stage I or F within 48hrs). Enroll samples from patients already at RIFLE stage I or F were included in Cohort 2.**

| **C-C motif chemokine 18** | | | | | | |
|---|---|---|---|---|---|---|
| | sCr or UO | | sCr only | | UO only | |
| | Cohort 1 | Cohort 2 | Cohort 1 | Cohort 2 | Cohort 1 | Cohort 2 |
| Median | 0.460 | 1.04 | 0.486 | 2.03 | 0.488 | 1.04 |
| Average | 1.95 | 6.61 | 2.58 | 9.28 | 2.03 | 7.35 |
| Stdev | 5.90 | 12.7 | 7.41 | 14.4 | 5.85 | 13.5 |
| p(t-test) | | 2.7E-7 | | 2.0E-4 | | 3.3E-7 |
| Min | 3.13E-5 | 0.00268 | 3.13E-5 | 0.204 | 3.13E-5 | 0.00268 |
| Max | 40.0 | 40.0 | 40.0 | 40.0 | 40.0 | 40.0 |
| n (Samp) | 383 | 92 | 451 | 20 | 296 | 79 |
| n (Patient) | 383 | 92 | 451 | 20 | 296 | 79 |
| | | | | | | |

| | At Enrollment | | | | | |
|---|---|---|---|---|---|---|
| | sCr or UO | | sCr only | | UO only | |
| AUC | 0.66 | | 0.75 | | 0.64 | |
| SE | 0.034 | | 0.064 | | 0.037 | |
| p | 2.3E-6 | | 9.2E-5 | | 1.4E-4 | |
| nCohort 1 | 383 | | 451 | | 296 | |
| nCohort 2 | 92 | | 20 | | 79 | |
| Cutoff 1 | 0.402 | | 0.906 | | 0.362 | |
| Sens 1 | 71% | | 70% | | 71% | |
| Spec 1 | 48% | | 67% | | 43% | |
| Cutoff 2 | 0.274 | | 0.826 | | 0.264 | |
| Sens 2 | 80% | | 80% | | 81% | |
| Spec 2 | 39% | | 63% | | 33% | |
| Cutoff 3 | 0.147 | | 0.340 | | 0.141 | |
| Sens 3 | 90% | | 90% | | 91% | |
| Spec 3 | 24% | | 42% | | 19% | |
| Cutoff 4 | 0.928 | | 1.10 | | 1.01 | |
| Sens 4 | 53% | | 60% | | 52% | |
| Spec 4 | 70% | | 70% | | 70% | |
| Cutoff 5 | 1.61 | | 1.84 | | 1.81 | |
| Sens 5 | 41% | | 50% | | 39% | |
| Spec 5 | 80% | | 80% | | 80% | |
| Cutoff 6 | 3.46 | | 3.81 | | 3.59 | |
| Sens 6 | 26% | | 35% | | 25% | |
| Spec 6 | 90% | | 90% | | 90% | |
| OR Quart 2 | 2.3 | | >3.1 | | 1.7 | |
| p Value | 0.031 | | <0.34 | | 0.18 | |
| 95% CI of | 1.1 | | >0.31 | | 0.78 | |
| OR Quart2 | 5.0 | | na | | 3.8 | |
| OR Quart 3 | 1.8 | | >6.3 | | 1.3 | |
| p Value | 0.13 | | <0.092 | | 0.55 | |
| 95% CI of | 0.84 | | >0.74 | | 0.56 | |
| OR Quart3 | 4.1 | | na | | 2.9 | |
| OR Quart 4 | 4.7 | | >12 | | 3.7 | |
| p Value | 2.9E-5 | | <0.018 | | 6.0E-4 | |
| 95% CI of | 2.3 | | >1.5 | | 1.7 | |
| OR Quart4 | 9.8 | | na | | 7.7 | |

| | | | | | | |
|---|---|---|---|---|---|---|
| **C-C motif chemokine 24** | | | | | | |
| | sCr or UO | | sCr only | | UO only | |
| | Cohort 1 | Cohort 2 | Cohort 1 | Cohort 2 | Cohort 1 | Cohort 2 |
| Median | 10.6 | 36.2 | 13.9 | 79.4 | 11.4 | 36.1 |
| Average | 24.0 | 134 | 39.6 | 172 | 24.8 | 144 |
| Stdev | 47.0 | 357 | 147 | 418 | 49.4 | 384 |
| p(t-test) | | 1.1E-8 | | 5.4E-4 | | 3.3E-7 |
| Min | 0.0120 | 0.0347 | 0.0120 | 3.66 | 0.0120 | 0.0347 |
| Max | 554 | 2380 | 2380 | 1930 | 554 | 2380 |
| n (Samp) | 383 | 92 | 451 | 20 | 297 | 79 |
| n (Patient) | 383 | 92 | 451 | 20 | 297 | 79 |
| | | | | | | |

| | At Enrollment | | | | | |
|---|---|---|---|---|---|---|
| | sCr or UO | | sCr only | | UO only | |
| AUC | 0.76 | | 0.81 | | 0.75 | |
| SE | 0.031 | | 0.059 | | 0.034 | |
| p | 0 | | 1.4E-7 | | 2.5E-13 | |
| nCohort 1 | 383 | | 451 | | 297 | |
| nCohort 2 | 92 | | 20 | | 79 | |
| Cutoff 1 | 24.0 | | 27.3 | | 21.2 | |
| Sens 1 | 71% | | 70% | | 71% | |
| Spec 1 | 72% | | 71% | | 69% | |
| Cutoff 2 | 14.5 | | 27.0 | | 11.7 | |
| Sens 2 | 80% | | 80% | | 81% | |
| Spec 2 | 58% | | 71% | | 51% | |
| Cutoff 3 | 7.72 | | 20.4 | | 5.54 | |
| Sens 3 | 90% | | 90% | | 91% | |
| Spec 3 | 42% | | 61% | | 35% | |
| Cutoff 4 | 22.4 | | 26.6 | | 22.4 | |
| Sens 4 | 71% | | 80% | | 70% | |
| Spec 4 | 70% | | 70% | | 70% | |
| Cutoff 5 | 31.0 | | 38.5 | | 29.7 | |
| Sens 5 | 54% | | 60% | | 57% | |
| Spec 5 | 80% | | 80% | | 80% | |
| Cutoff 6 | 57.3 | | 67.2 | | 57.9 | |
| Sens 6 | 37% | | 55% | | 35% | |
| Spec 6 | 90% | | 90% | | 90% | |
| OR Quart 2 | 2.8 | | >2.0 | | 2.6 | |
| p Value | 0.061 | | <0.57 | | 0.084 | |
| 95% CI of | 0.96 | | >0.18 | | 0.88 | |
| OR Quart2 | 8.0 | | na | | 7.7 | |
| OR Quart 3 | 6.6 | | >6.3 | | 5.4 | |
| p Value | 1.9E-4 | | <0.092 | | 0.0011 | |
| 95% CI of | 2.5 | | >0.74 | | 2.0 | |
| OR Quart3 | 18 | | na | | 15 | |
| OR Quart 4 | 15 | | >13 | | 13 | |
| p Value | 5.1E-8 | | <0.014 | | 3.2E-7 | |
| 95% CI of | 5.6 | | >1.7 | | 4.9 | |
| OR Quart4 | 39 | | na | | 35 | |

| | | | | | | |
|---|---|---|---|---|---|---|
| **C-C motif chemokine 8** | | | | | | |
| | sCr or UO | | sCr only | | UO only | |
| | Cohort 1 | Cohort 2 | Cohort 1 | Cohort 2 | Cohort 1 | Cohort 2 |
| Median | 1.36 | 6.27 | 1.51 | 19.7 | 1.55 | 6.84 |
| Average | 11.6 | 106 | 14.8 | 376 | 13.1 | 74.4 |
| Stdev | 35.6 | 494 | 45.8 | 1020 | 39.9 | 345 |
| p(t-test) | | 2.2E-4 | | 3.3E-13 | | 0.0029 |
| Min | 0.0250 | 0.0250 | 0.0250 | 0.0701 | 0.0250 | 0.0250 |
| Max | 473 | 3670 | 492 | 3670 | 473 | 3020 |
| n (Samp) | 383 | 92 | 451 | 20 | 297 | 79 |
| n (Patient) | 383 | 92 | 451 | 20 | 297 | 79 |
| | | | | | | |

| | At Enrollment | | | | | |
|---|---|---|---|---|---|---|
| | sCr or UO | | sCr only | | UO only | |
| AUC | 0.64 | | 0.70 | | 0.63 | |
| SE | 0.034 | | 0.067 | | 0.037 | |
| p | 3.2E-5 | | 0.0024 | | 3.7E-4 | |
| nCohort 1 | 383 | | 451 | | 297 | |
| nCohort 2 | 92 | | 20 | | 79 | |
| Cutoff 1 | 0.491 | | 3.16 | | 0.491 | |
| Sens 1 | 71% | | 70% | | 71% | |
| Spec 1 | 38% | | 58% | | 35% | |
| Cutoff 2 | 0.240 | | 0.491 | | 0.240 | |
| Sens 2 | 80% | | 80% | | 82% | |
| Spec 2 | 35% | | 37% | | 31% | |
| Cutoff 3 | 0.127 | | 0.154 | | 0.127 | |
| Sens 3 | 91% | | 90% | | 91% | |
| Spec 3 | 27% | | 28% | | 23% | |
| Cutoff 4 | 7.03 | | 7.99 | | 7.18 | |
| Sens 4 | 48% | | 60% | | 49% | |
| Spec 4 | 70% | | 70% | | 70% | |
| Cutoff 5 | 12.0 | | 12.7 | | 12.2 | |
| Sens 5 | 36% | | 55% | | 34% | |
| Spec 5 | 80% | | 80% | | 80% | |
| Cutoff 6 | 24.5 | | 30.8 | | 24.5 | |
| Sens 6 | 27% | | 35% | | 28% | |
| Spec 6 | 90% | | 90% | | 91% | |
| OR Quart 2 | 3.4 | | 5.1 | | 1.8 | |
| p Value | 0.0031 | | 0.14 | | 0.16 | |
| 95% CI of | 1.5 | | 0.59 | | 0.79 | |
| OR Quart2 | 7.6 | | 45 | | 4.0 | |
| OR Quart 3 | 2.4 | | 2.0 | | 2.2 | |
| p Value | 0.035 | | 0.57 | | 0.056 | |
| 95% CI of | 1.1 | | 0.18 | | 0.98 | |
| OR Quart3 | 5.6 | | 22 | | 4.8 | |
| OR Quart 4 | 5.5 | | 13 | | 3.4 | |
| p Value | 2.1E-5 | | 0.014 | | 0.0019 | |
| 95% CI of | 2.5 | | 1.7 | | 1.6 | |
| OR Quart4 | 12 | | 100 | | 7.2 | |

| | | | | | | |
|---|---|---|---|---|---|---|
| **Cathepsin D** | | | | | | |
| | sCr or UO | | sCr only | | UO only | |
| | Cohort 1 | Cohort 2 | Cohort 1 | Cohort 2 | Cohort 1 | Cohort 2 |
| Median | 72700 | 105000 | 75800 | 130000 | 75500 | 104000 |
| Average | 76500 | 111000 | 81000 | 130000 | 80300 | 106000 |
| Stdev | 40100 | 42500 | 41700 | 40400 | 39500 | 41400 |
| p(t-test) | | 2.0E-12 | | 4.8E-7 | | 3.7E-7 |
| Min | 656 | 4320 | 656 | 55700 | 2520 | 4320 |
| Max | 200000 | 200000 | 200000 | 200000 | 200000 | 200000 |
| n (Samp) | 383 | 92 | 451 | 20 | 296 | 79 |
| n (Patient) | 383 | 92 | 451 | 20 | 296 | 79 |
| | | | | | | |

| | At Enrollment | | | | | |
|---|---|---|---|---|---|---|
| | sCr or UO | | sCr only | | UO only | |
| AUC | 0.72 | | 0.80 | | 0.68 | |
| SE | 0.032 | | 0.060 | | 0.036 | |
| p | 2.7E-12 | | 6.5E-7 | | 4.3E-7 | |
| nCohort 1 | 383 | | 451 | | 296 | |
| nCohort 2 | 92 | | 20 | | 79 | |
| Cutoff 1 | 84100 | | 108000 | | 78200 | |
| Sens 1 | 71% | | 70% | | 71% | |
| Spec 1 | 63% | | 76% | | 53% | |
| Cutoff 2 | 71300 | | 99200 | | 70400 | |
| Sens 2 | 80% | | 80% | | 81% | |
| Spec 2 | 49% | | 69% | | 46% | |
| Cutoff 3 | 63600 | | 84400 | | 62300 | |
| Sens 3 | 90% | | 90% | | 91% | |
| Spec 3 | 42% | | 59% | | 37% | |
| Cutoff 4 | 95800 | | 102000 | | 99200 | |
| Sens 4 | 60% | | 70% | | 54% | |
| Spec 4 | 70% | | 70% | | 70% | |
| Cutoff 5 | 109000 | | 116000 | | 115000 | |
| Sens 5 | 47% | | 65% | | 39% | |
| Spec 5 | 80% | | 80% | | 80% | |
| Cutoff 6 | 133000 | | 138000 | | 134000 | |
| Sens 6 | 27% | | 35% | | 24% | |
| Spec 6 | 90% | | 90% | | 90% | |
| OR Quart 2 | 5.4 | | >2.0 | | 4.8 | |
| p Value | 0.0029 | | <0.57 | | 0.0029 | |
| 95% CI of | 1.8 | | >0.18 | | 1.7 | |
| OR Quart2 | 16 | | na | | 13 | |
| OR Quart 3 | 8.0 | | >5.2 | | 5.1 | |
| p Value | 1.8E-4 | | <0.14 | | 0.0019 | |
| 95% CI of | 2.7 | | >0.60 | | 1.8 | |
| OR Quart3 | 24 | | na | | 14 | |
| OR Quart 4 | 16 | | >14 | | 9.5 | |
| p Value | 3.1E-7 | | <0.011 | | 9.2E-6 | |
| 95% CI of | 5.6 | | >1.9 | | 3.5 | |
| OR Quart4 | 47 | | na | | 26 | |

| | | | | | | |
|---|---|---|---|---|---|---|
| **C-X-C motif chemokine 13** | | | | | | |
| | sCr or UO | | sCr only | | UO only | |
| | Cohort 1 | Cohort 2 | Cohort 1 | Cohort 2 | Cohort 1 | Cohort 2 |
| Median | 0.0733 | 2.45 | 0.236 | 7.12 | 0.156 | 2.43 |
| Average | 6.47 | 46.6 | 9.11 | 131 | 7.64 | 51.3 |
| Stdev | 37.8 | 201 | 42.6 | 413 | 42.7 | 216 |
| p(t-test) | | 2.8E-4 | | 1.8E-8 | | 0.0012 |
| Min | 0.00269 | 0.00269 | 0.00269 | 0.00442 | 0.00269 | 0.00269 |
| Max | 652 | 1850 | 652 | 1850 | 652 | 1850 |
| n (Samp) | 383 | 92 | 451 | 20 | 297 | 79 |
| n (Patient) | 383 | 92 | 451 | 20 | 297 | 79 |
| | | | | | | |

| | At Enrollment | | | | | |
|---|---|---|---|---|---|---|
| | sCr or UO | | sCr only | | UO only | |
| AUC | 0.69 | | 0.74 | | 0.69 | |
| SE | 0.033 | | 0.065 | | 0.036 | |
| p | 3.5E-9 | | 2.0E-4 | | 1.8E-7 | |
| nCohort 1 | 383 | | 451 | | 297 | |
| nCohort 2 | 92 | | 20 | | 79 | |
| Cutoff 1 | 0.236 | | 2.65 | | 0.236 | |
| Sens 1 | 71% | | 70% | | 71% | |
| Spec 1 | 53% | | 76% | | 52% | |
| Cutoff 2 | 0.0151 | | 0.244 | | 0.0151 | |
| Sens 2 | 82% | | 80% | | 81% | |
| Spec 2 | 40% | | 50% | | 37% | |
| Cutoff 3 | 0.0109 | | 0.0160 | | 0.0109 | |
| Sens 3 | 90% | | 90% | | 91% | |
| Spec 3 | 33% | | 39% | | 28% | |
| Cutoff 4 | 1.19 | | 1.65 | | 1.14 | |
| Sens 4 | 58% | | 75% | | 57% | |
| Spec 4 | 70% | | 70% | | 70% | |
| Cutoff 5 | 3.20 | | 4.17 | | 3.20 | |
| Sens 5 | 43% | | 60% | | 43% | |
| Spec 5 | 80% | | 80% | | 80% | |
| Cutoff 6 | 9.95 | | 15.0 | | 12.8 | |
| Sens 6 | 30% | | 40% | | 28% | |
| Spec 6 | 90% | | 90% | | 90% | |
| OR Quart 2 | 2.4 | | 1.5 | | 2.2 | |
| p Value | 0.035 | | 0.66 | | 0.086 | |
| 95% CI of | 1.1 | | 0.25 | | 0.89 | |
| OR Quart2 | 5.6 | | 9.1 | | 5.4 | |
| OR Quart 3 | 2.7 | | 0.99 | | 3.1 | |
| p Value | 0.016 | | 0.99 | | 0.011 | |
| 95% CI of | 1.2 | | 0.14 | | 1.3 | |
| OR Quart3 | 6.3 | | 7.2 | | 7.4 | |
| OR Quart 4 | 6.4 | | 7.1 | | 6.1 | |
| p Value | 3.1E-6 | | 0.011 | | 2.4E-5 | |
| 95% CI of | 2.9 | | 1.6 | | 2.6 | |
| OR Quart4 | 14 | | 32 | | 14 | |

| | | | | | | |
|---|---|---|---|---|---|---|
| **Insulin-like growth factor-binding protein 3** | | | | | | |
| | sCr or UO | | sCr only | | UO only | |
| | Cohort 1 | Cohort 2 | Cohort 1 | Cohort 2 | Cohort 1 | Cohort 2 |
| Median | 339 | 706 | 370 | 1120 | 364 | 793 |
| Average | 575 | 1170 | 652 | 1490 | 607 | 1230 |
| Stdev | 735 | 1640 | 939 | 1810 | 762 | 1740 |
| p(t-test) | | 7.8E-8 | | 1.7E-4 | | 1.4E-6 |
| Min | 3.84 | 0.311 | 0.311 | 44.1 | 3.84 | 0.311 |
| Max | 7920 | 12500 | 12500 | 8160 | 7920 | 12500 |
| n (Samp) | 412 | 96 | 482 | 21 | 325 | 83 |
| n (Patient) | 412 | 96 | 482 | 21 | 325 | 83 |
| | | | | | | |

| | At Enrollment | | | | | |
|---|---|---|---|---|---|---|
| | sCr or UO | | sCr only | | UO only | |
| AUC | 0.68 | | 0.68 | | 0.66 | |
| SE | 0.032 | | 0.066 | | 0.035 | |
| p | 5.3E-8 | | 0.0050 | | 3.1E-6 | |
| nCohort 1 | 412 | | 482 | | 325 | |
| nCohort 2 | 96 | | 21 | | 83 | |
| Cutoff 1 | 371 | | 425 | | 369 | |
| Sens 1 | 71% | | 71% | | 71% | |
| Spec 1 | 54% | | 54% | | 50% | |
| Cutoff 2 | 293 | | 269 | | 293 | |
| Sens 2 | 80% | | 81% | | 81% | |
| Spec 2 | 45% | | 40% | | 42% | |
| Cutoff 3 | 114 | | 108 | | 125 | |
| Sens 3 | 91% | | 90% | | 90% | |
| Spec 3 | 26% | | 23% | | 23% | |
| Cutoff 4 | 667 | | 736 | | 703 | |
| Sens 4 | 52% | | 52% | | 52% | |
| Spec 4 | 70% | | 70% | | 70% | |
| Cutoff 5 | 1010 | | 1070 | | 1030 | |
| Sens 5 | 41% | | 52% | | 40% | |
| Spec 5 | 80% | | 80% | | 80% | |
| Cutoff 6 | 1350 | | 1470 | | 1370 | |
| Sens 6 | 28% | | 38% | | 29% | |
| Spec 6 | 90% | | 90% | | 90% | |
| OR Quart 2 | 1.7 | | 0.99 | | 2.1 | |
| p Value | 0.17 | | 0.99 | | 0.075 | |
| 95% CI of | 0.79 | | 0.20 | | 0.93 | |
| OR Quart2 | 3.9 | | 5.0 | | 4.8 | |
| OR Quart 3 | 2.8 | | 1.3 | | 2.2 | |
| p Value | 0.0063 | | 0.71 | | 0.052 | |
| 95% CI of | 1.3 | | 0.29 | | 0.99 | |
| OR Quart3 | 6.0 | | 6.1 | | 5.1 | |
| OR Quart 4 | 4.8 | | 3.9 | | 4.6 | |
| p Value | 1.9E-5 | | 0.041 | | 1.1E-4 | |
| 95% CI of | 2.4 | | 1.1 | | 2.1 | |
| OR Quart4 | 10.0 | | 14 | | 10.0 | |

| | | | | | | |
|---|---|---|---|---|---|---|
| **Immunoglogulin G1** | | | | | | |
| | sCr or UO | | sCr only | | UO only | |
| | Cohort 1 | Cohort 2 | Cohort 1 | Cohort 2 | Cohort 1 | Cohort 2 |
| Median | 3190 | 7960 | 3630 | 14900 | 3200 | 7950 |
| Average | 5790 | 11100 | 6510 | 14000 | 5780 | 11500 |
| Stdev | 7390 | 11900 | 8530 | 10300 | 7370 | 12400 |
| p(t-test) | | 9.1E-8 | | 1.7E-4 | | 2.8E-7 |
| Min | 59.7 | 55.9 | 55.9 | 177 | 59.7 | 55.9 |
| Max | 80000 | 80000 | 80000 | 28800 | 80000 | 80000 |
| n (Samp) | 379 | 92 | 447 | 20 | 292 | 79 |
| n (Patient) | 379 | 92 | 447 | 20 | 292 | 79 |
| | | | | | | |

| | At Enrollment | | | | | |
|---|---|---|---|---|---|---|
| | sCr or UO | | sCr only | | UO only | |
| AUC | 0.66 | | 0.70 | | 0.67 | |
| SE | 0.033 | | 0.066 | | 0.036 | |
| p | 9.5E-7 | | 0.0021 | | 1.4E-6 | |
| nCohort 1 | 379 | | 447 | | 292 | |
| nCohort 2 | 92 | | 20 | | 79 | |
| Cutoff 1 | 3420 | | 7950 | | 3630 | |
| Sens 1 | 71% | | 70% | | 71% | |
| Spec 1 | 53% | | 75% | | 53% | |
| Cutoff 2 | 1900 | | 2280 | | 2320 | |
| Sens 2 | 80% | | 80% | | 81% | |
| Spec 2 | 28% | | 34% | | 36% | |
| Cutoff 3 | 1420 | | 1250 | | 1440 | |
| Sens 3 | 90% | | 90% | | 91% | |
| Spec 3 | 21% | | 16% | | 20% | |
| Cutoff 4 | 6150 | | 6670 | | 6150 | |
| Sens 4 | 57% | | 70% | | 57% | |
| Spec 4 | 70% | | 70% | | 70% | |
| Cutoff 5 | 8420 | | 9330 | | 8540 | |
| Sens 5 | 47% | | 60% | | 46% | |
| Spec 5 | 80% | | 80% | | 80% | |
| Cutoff 6 | 13900 | | 16200 | | 13400 | |
| Sens 6 | 32% | | 40% | | 35% | |
| Spec 6 | 90% | | 90% | | 90% | |
| OR Quart 2 | 0.85 | | 0.99 | | 0.75 | |
| p Value | 0.68 | | 0.99 | | 0.50 | |
| 95% CI of | 0.39 | | 0.20 | | 0.32 | |
| OR Quart2 | 1.8 | | 5.0 | | 1.7 | |
| OR Quart 3 | 1.2 | | 0.32 | | 1.3 | |
| p Value | 0.60 | | 0.33 | | 0.46 | |
| 95% CI of | 0.59 | | 0.033 | | 0.62 | |
| OR Quart3 | 2.5 | | 3.2 | | 2.9 | |
| OR Quart 4 | 3.6 | | 4.7 | | 3.5 | |
| p Value | 9.8E-5 | | 0.018 | | 4.7E-4 | |
| 95% CI of | 1.9 | | 1.3 | | 1.7 | |
| OR Quart4 | 6.9 | | 17 | | 7.1 | |

| | | | | | | |
|---|---|---|---|---|---|---|
| **Immunoglogulin G2** | | | | | | |
| | sCr or UO | | sCr only | | UO only | |
| | Cohort 1 | Cohort 2 | Cohort 1 | Cohort 2 | Cohort 1 | Cohort 2 |
| Median | 8420 | 18400 | 9310 | 36400 | 9250 | 18300 |
| Average | 17300 | 46300 | 20800 | 69400 | 17000 | 48900 |
| Stdev | 30200 | 64200 | 37700 | 72400 | 28000 | 67700 |
| p(t-test) | | 4.4E-10 | | 1.3E-7 | | 7.9E-10 |
| Min | 119 | 25.4 | 25.4 | 2380 | 334 | 25.4 |
| Max | 240000 | 240000 | 240000 | 203000 | 240000 | 240000 |
| n (Samp) | 379 | 92 | 447 | 20 | 292 | 79 |
| n (Patient) | 379 | 92 | 447 | 20 | 292 | 79 |
| | | | | | | |

| | At Enrollment | | | | | |
|---|---|---|---|---|---|---|
| | sCr or UO | | sCr only | | UO only | |
| AUC | 0.69 | | 0.72 | | 0.69 | |
| SE | 0.033 | | 0.066 | | 0.036 | |
| p | 2.1E-8 | | 8.8E-4 | | 1.4E-7 | |
| nCohort 1 | 379 | | 447 | | 292 | |
| nCohort 2 | 92 | | 20 | | 79 | |
| Cutoff 1 | 8630 | | 13500 | | 8840 | |
| Sens 1 | 71% | | 70% | | 71% | |
| Spec 1 | 52% | | 64% | | 50% | |
| Cutoff 2 | 7050 | | 7680 | | 7950 | |
| Sens 2 | 80% | | 80% | | 81% | |
| Spec 2 | 44% | | 43% | | 46% | |
| Cutoff 3 | 3980 | | 4240 | | 3980 | |
| Sens 3 | 90% | | 90% | | 91% | |
| Spec 3 | 21% | | 21% | | 20% | |
| Cutoff 4 | 14400 | | 15900 | | 14500 | |
| Sens 4 | 57% | | 65% | | 57% | |
| Spec 4 | 70% | | 70% | | 70% | |
| Cutoff 5 | 19800 | | 22200 | | 19800 | |
| Sens 5 | 45% | | 55% | | 46% | |
| Spec 5 | 80% | | 80% | | 80% | |
| Cutoff 6 | 35300 | | 41300 | | 31100 | |
| Sens 6 | 30% | | 45% | | 33% | |
| Spec 6 | 90% | | 90% | | 90% | |
| OR Quart 2 | 1.5 | | 0.66 | | 1.5 | |
| p Value | 0.34 | | 0.65 | | 0.40 | |
| 95% CI of | 0.67 | | 0.11 | | 0.61 | |
| OR Quart2 | 3.2 | | 4.0 | | 3.5 | |
| OR Quart 3 | 2.0 | | 1.3 | | 2.1 | |
| p Value | 0.071 | | 0.71 | | 0.078 | |
| 95% CI of | 0.94 | | 0.29 | | 0.92 | |
| OR Quart3 | 4.3 | | 6.1 | | 4.8 | |
| OR Quart 4 | 4.7 | | 3.9 | | 5.2 | |
| p Value | 2.0E-5 | | 0.040 | | 3.4E-5 | |
| 95% CI of | 2.3 | | 1.1 | | 2.4 | |
| OR Quart4 | 9.5 | | 14 | | 11 | |

| | | | | | | |
|---|---|---|---|---|---|---|
| **Interleukin-11** | | | | | | |
| | sCr or UO | | sCr only | | UO only | |
| | Cohort 1 | Cohort 2 | Cohort 1 | Cohort 2 | Cohort 1 | Cohort 2 |
| Median | 122 | 203 | 129 | 258 | 139 | 196 |
| Average | 194 | 364 | 217 | 438 | 209 | 354 |
| Stdev | 247 | 441 | 288 | 499 | 260 | 424 |
| p(t-test) | | 9.3E-7 | | 0.0013 | | 1.9E-4 |
| Min | 0.154 | 28.3 | 0.154 | 41.6 | 2.83 | 28.3 |
| Max | 2260 | 2140 | 2260 | 2020 | 2260 | 2140 |
| n (Samp) | 385 | 91 | 452 | 20 | 298 | 78 |
| n (Patient) | 385 | 91 | 452 | 20 | 298 | 78 |
| | | | | | | |

| | At Enrollment | | | | | |
|---|---|---|---|---|---|---|
| | sCr or UO | | sCr only | | UO only | |
| AUC | 0.66 | | 0.71 | | 0.63 | |
| SE | 0.034 | | 0.066 | | 0.037 | |
| p | 1.2E-6 | | 0.0019 | | 5.6E-4 | |
| nCohort 1 | 385 | | 452 | | 298 | |
| nCohort 2 | 91 | | 20 | | 78 | |
| Cutoff 1 | 117 | | 201 | | 113 | |
| Sens 1 | 70% | | 70% | | 71% | |
| Spec 1 | 49% | | 70% | | 42% | |
| Cutoff 2 | 94.5 | | 179 | | 94.5 | |
| Sens 2 | 80% | | 80% | | 81% | |
| Spec 2 | 42% | | 65% | | 35% | |
| Cutoff 3 | 63.9 | | 56.7 | | 64.5 | |
| Sens 3 | 90% | | 90% | | 91% | |
| Spec 3 | 29% | | 23% | | 24% | |
| Cutoff 4 | 185 | | 203 | | 203 | |
| Sens 4 | 54% | | 65% | | 47% | |
| Spec 4 | 70% | | 70% | | 70% | |
| Cutoff 5 | 253 | | 286 | | 274 | |
| Sens 5 | 41% | | 40% | | 38% | |
| Spec 5 | 80% | | 80% | | 80% | |
| Cutoff 6 | 416 | | 479 | | 436 | |
| Sens 6 | 22% | | 25% | | 22% | |
| Spec 6 | 90% | | 90% | | 90% | |
| OR Quart 2 | 2.9 | | 0.33 | | 2.7 | |
| p Value | 0.010 | | 0.34 | | 0.015 | |
| 95% CI of | 1.3 | | 0.034 | | 1.2 | |
| OR Quart2 | 6.6 | | 3.2 | | 6.1 | |
| OR Quart 3 | 2.6 | | 1.7 | | 1.5 | |
| p Value | 0.022 | | 0.48 | | 0.38 | |
| 95% CI of | 1.1 | | 0.40 | | 0.62 | |
| OR Quart3 | 6.0 | | 7.3 | | 3.5 | |
| OR Quart 4 | 5.7 | | 3.9 | | 4.1 | |
| p Value | 1.2E-5 | | 0.039 | | 3.9E-4 | |
| 95% CI of | 2.6 | | 1.1 | | 1.9 | |
| OR Quart4 | 13 | | 15 | | 9.1 | |

| | | | | | | |
|---|---|---|---|---|---|---|
| **Interleukin-2 receptor alpha chain** | | | | | | |
| | sCr or UO | | sCr only | | UO only | |
| | Cohort 1 | Cohort 2 | Cohort 1 | Cohort 2 | Cohort 1 | Cohort 2 |
| Median | 518 | 937 | 566 | 1560 | 524 | 913 |
| Average | 922 | 1840 | 1040 | 2330 | 943 | 1910 |
| Stdev | 1120 | 2190 | 1310 | 2770 | 1100 | 2260 |
| p(t-test) | | 2.1E-8 | | 6.2E-5 | | 1.6E-7 |
| Min | 0.0339 | 0.0482 | 0.0339 | 0.0482 | 0.0339 | 0.115 |
| Max | 8370 | 10100 | 9660 | 10100 | 6540 | 10100 |
| n (Samp) | 385 | 92 | 453 | 20 | 298 | 79 |
| n (Patient) | 385 | 92 | 453 | 20 | 298 | 79 |
| | | | | | | |

| | At Enrollment | | | | | |
|---|---|---|---|---|---|---|
| | sCr or UO | | sCr only | | UO only | |
| AUC | 0.64 | | 0.61 | | 0.65 | |
| SE | 0.034 | | 0.068 | | 0.037 | |
| p | 3.4E-5 | | 0.096 | | 7.0E-5 | |
| nCohort 1 | 385 | | 453 | | 298 | |
| nCohort 2 | 92 | | 20 | | 79 | |
| Cutoff 1 | 467 | | 385 | | 484 | |
| Sens 1 | 71% | | 70% | | 71% | |
| Spec 1 | 48% | | 41% | | 48% | |
| Cutoff 2 | 325 | | 60.5 | | 333 | |
| Sens 2 | 80% | | 80% | | 81% | |
| Spec 2 | 41% | | 13% | | 39% | |
| Cutoff 3 | 91.3 | | 0.120 | | 152 | |
| Sens 3 | 90% | | 90% | | 91% | |
| Spec 3 | 17% | | 2% | | 23% | |
| Cutoff 4 | 1090 | | 1170 | | 1110 | |
| Sens 4 | 42% | | 60% | | 41% | |
| Spec 4 | 70% | | 70% | | 70% | |
| Cutoff 5 | 1600 | | 1690 | | 1660 | |
| Sens 5 | 36% | | 50% | | 33% | |
| Spec 5 | 80% | | 80% | | 80% | |
| Cutoff 6 | 2280 | | 2590 | | 2390 | |
| Sens 6 | 26% | | 30% | | 27% | |
| Spec 6 | 90% | | 90% | | 90% | |
| OR Quart 2 | 1.6 | | 0.39 | | 2.4 | |
| p Value | 0.24 | | 0.27 | | 0.058 | |
| 95% CI of | 0.73 | | 0.074 | | 0.97 | |
| OR Quart2 | 3.5 | | 2.0 | | 5.8 | |
| OR Quart 3 | 2.9 | | 0.59 | | 4.1 | |
| p Value | 0.0045 | | 0.48 | | 0.0012 | |
| 95% CI of | 1.4 | | 0.14 | | 1.7 | |
| OR Quart3 | 6.0 | | 2.5 | | 9.7 | |
| OR Quart 4 | 3.4 | | 2.1 | | 4.5 | |
| p Value | 8.9E-4 | | 0.20 | | 5.2E-4 | |
| 95% CI of | 1.6 | | 0.69 | | 1.9 | |
| OR Quart4 | 6.9 | | 6.3 | | 10 | |

| | | | | | | |
|---|---|---|---|---|---|---|
| **Neutrophil collagenase** | | | | | | |
| | sCr or UO | | sCr only | | UO only | |
| | Cohort 1 | Cohort 2 | Cohort 1 | Cohort 2 | Cohort 1 | Cohort 2 |
| Median | 3190 | 9770 | 3750 | 21900 | 3260 | 10400 |
| Average | 13100 | 49700 | 16000 | 113000 | 11100 | 46700 |
| Stdev | 44400 | 112000 | 52900 | 169000 | 27800 | 99400 |
| p(t-test) | | 4.0E-7 | | 5.7E-12 | | 2.6E-8 |
| Min | 0.114 | 25.8 | 0.114 | 25.8 | 0.114 | 198 |
| Max | 670000 | 670000 | 670000 | 649000 | 300000 | 670000 |
| n (Samp) | 412 | 96 | 482 | 21 | 326 | 83 |
| n (Patient) | 412 | 96 | 482 | 21 | 326 | 83 |

| | At Enrollment | | | | | |
|---|---|---|---|---|---|---|
| | sCr or UO | | sCr only | | UO only | |
| AUC | 0.71 | | 0.72 | | 0.72 | |
| SE | 0.032 | | 0.064 | | 0.034 | |
| p | 1.1E-10 | | 7.6E-4 | | 5.9E-11 | |
| nCohort 1 | 412 | | 482 | | 326 | |
| nCohort 2 | 96 | | 21 | | 83 | |
| Cutoff 1 | 4980 | | 8140 | | 5380 | |
| Sens 1 | 71% | | 71% | | 71% | |
| Spec 1 | 60% | | 68% | | 63% | |
| Cutoff 2 | 2810 | | 2810 | | 3170 | |
| Sens 2 | 80% | | 81% | | 81% | |
| Spec 2 | 48% | | 44% | | 49% | |
| Cutoff 3 | 1020 | | 191 | | 1630 | |
| Sens 3 | 91% | | 90% | | 90% | |
| Spec 3 | 27% | | 5% | | 35% | |
| Cutoff 4 | 7060 | | 8960 | | 6920 | |
| Sens 4 | 66% | | 67% | | 66% | |
| Spec 4 | 70% | | 70% | | 70% | |
| Cutoff 5 | 11900 | | 15600 | | 10700 | |
| Sens 5 | 46% | | 62% | | 49% | |
| Spec 5 | 80% | | 80% | | 80% | |
| Cutoff 6 | 22200 | | 30800 | | 21900 | |
| Sens 6 | 34% | | 48% | | 36% | |
| Spec 6 | 90% | | 90% | | 90% | |
| OR Quart 2 | 1.7 | | 0.24 | | 3.0 | |
| p Value | 0.22 | | 0.21 | | 0.030 | |
| 95% CI of | 0.73 | | 0.027 | | 1.1 | |
| OR Quart2 | 3.9 | | 2.2 | | 8.0 | |
| OR Quart 3 | 3.0 | | 0.74 | | 4.4 | |
| p Value | 0.0054 | | 0.69 | | 0.0022 | |
| 95% CI of | 1.4 | | 0.16 | | 1.7 | |
| OR Quart3 | 6.5 | | 3.4 | | 11 | |
| OR Quart 4 | 6.2 | | 3.5 | | 9.8 | |
| p Value | 1.4E-6 | | 0.033 | | 1.1E-6 | |
| 95% CI of | 3.0 | | 1.1 | | 3.9 | |
| OR Quart4 | 13 | | 11 | | 24 | |

| | | | | | | |
|---|---|---|---|---|---|---|
| **Protransforming growth factor alpha** | | | | | | |
| | sCr or UO | | sCr only | | UO only | |
| | Cohort 1 | Cohort 2 | Cohort 1 | Cohort 2 | Cohort 1 | Cohort 2 |
| Median | 6.28 | 12.5 | 6.98 | 15.7 | 6.62 | 12.3 |
| Average | 11.2 | 17.0 | 11.7 | 28.1 | 12.3 | 14.7 |
| Stdev | 24.0 | 18.0 | 22.5 | 32.1 | 26.8 | 10.2 |
| p(t-test) | | 0.030 | | 0.0019 | | 0.45 |
| Min | 0.00262 | 0.00454 | 0.00262 | 1.41 | 0.00262 | 0.00454 |
| Max | 361 | 122 | 361 | 122 | 361 | 50.4 |
| n (Samp) | 385 | 92 | 453 | 20 | 298 | 79 |
| n (Patient) | 385 | 92 | 453 | 20 | 298 | 79 |

| | At Enrollment | | | | | |
|---|---|---|---|---|---|---|
| | sCr or UO | | sCr only | | UO only | |
| AUC | 0.71 | | 0.74 | | 0.69 | |
| SE | 0.033 | | 0.065 | | 0.036 | |
| p | 3.0E-10 | | 2.9E-4 | | 1.5E-7 | |
| nCohort 1 | 385 | | 453 | | 298 | |
| nCohort 2 | 92 | | 20 | | 79 | |
| Cutoff 1 | 8.32 | | 11.3 | | 8.57 | |
| Sens 1 | 71% | | 70% | | 71% | |
| Spec 1 | 62% | | 69% | | 61% | |
| Cutoff 2 | 6.15 | | 6.78 | | 6.19 | |
| Sens 2 | 80% | | 80% | | 81% | |
| Spec 2 | 49% | | 49% | | 47% | |
| Cutoff 3 | 4.83 | | 4.92 | | 4.83 | |
| Sens 3 | 90% | | 90% | | 91% | |
| Spec 3 | 42% | | 37% | | 39% | |
| Cutoff 4 | 10.4 | | 11.6 | | 10.8 | |
| Sens 4 | 61% | | 65% | | 59% | |
| Spec 4 | 70% | | 70% | | 70% | |
| Cutoff 5 | 13.8 | | 15.2 | | 14.7 | |
| Sens 5 | 41% | | 60% | | 37% | |
| Spec 5 | 80% | | 80% | | 80% | |
| Cutoff 6 | 21.9 | | 23.8 | | 24.9 | |
| Sens 6 | 20% | | 35% | | 10% | |
| Spec 6 | 90% | | 90% | | 90% | |
| OR Quart 2 | 2.1 | | 4.1 | | 2.0 | |
| p Value | 0.12 | | 0.21 | | 0.16 | |
| 95% CI of | 0.83 | | 0.45 | | 0.76 | |
| OR Quart2 | 5.5 | | 37 | | 5.2 | |
| OR Quart 3 | 5.9 | | 2.0 | | 5.3 | |
| p Value | 5.9E-5 | | 0.57 | | 2.4E-4 | |
| 95% CI of | 2.5 | | 0.18 | | 2.2 | |
| OR Quart3 | 14 | | 23 | | 13 | |
| OR Quart 4 | 7.7 | | 14 | | 6.0 | |
| p Value | 2.8E-6 | | 0.011 | | 6.6E-5 | |
| 95% CI of | 3.3 | | 1.8 | | 2.5 | |
| OR Quart4 | 18 | | 110 | | 15 | |

| | | | | | | |
|---|---|---|---|---|---|---|
| **CA 15-3** | | | | | | |
| | sCr or UO | | sCr only | | UO only | |
| | Cohort 1 | Cohort 2 | Cohort 1 | Cohort 2 | Cohort 1 | Cohort 2 |
| Median | 434 | 434 | nd | nd | 434 | 434 |
| Average | 300 | 382 | nd | nd | 303 | 391 |
| Stdev | 250 | 216 | nd | nd | 251 | 213 |
| p(t-test) | | 0.070 | nd | nd | | 0.059 |
| Min | 2.21 | 4.72 | nd | nd | 2.21 | 4.72 |
| Max | 784 | 784 | nd | nd | 784 | 784 |
| n (Samp) | 169 | 36 | nd | nd | 150 | 34 |
| n (Patient) | 169 | 36 | nd | nd | 150 | 34 |

| | At Enrollment | | | | | |
|---|---|---|---|---|---|---|
| | sCr or UO | | sCr only | | UO only | |
| AUC | 0.62 | | nd | | 0.62 | |
| SE | 0.054 | | nd | | 0.056 | |
| p | 0.033 | | nd | | 0.028 | |
| nCohort 1 | 169 | | nd | | 150 | |
| nCohort 2 | 36 | | nd | | 34 | |
| Cutoff 1 | 427 | | nd | | 427 | |
| Sens 1 | 75% | | nd | | 76% | |
| Spec 1 | 49% | | nd | | 48% | |
| Cutoff 2 | 80.1 | | nd | | 80.1 | |
| Sens 2 | 81% | | nd | | 82% | |
| Spec 2 | 36% | | nd | | 37% | |
| Cutoff 3 | 36.1 | | nd | | 51.3 | |
| Sens 3 | 92% | | nd | | 91% | |
| Spec 3 | 24% | | nd | | 31% | |
| Cutoff 4 | 434 | | nd | | 434 | |
| Sens 4 | 14% | | nd | | 15% | |
| Spec 4 | 88% | | nd | | 88% | |
| Cutoff 5 | 434 | | nd | | 434 | |
| Sens 5 | 14% | | nd | | 15% | |
| Spec 5 | 88% | | nd | | 88% | |
| Cutoff 6 | 784 | | nd | | 784 | |
| Sens 6 | 0% | | nd | | 0% | |
| Spec 6 | 100% | | nd | | 100% | |
| OR Quart 2 | 1.3 | | nd | | 2.1 | |
| p Value | 0.73 | | nd | | 0.30 | |
| 95% CI of | 0.32 | | nd | | 0.50 | |
| OR Quart2 | 5.1 | | nd | | 9.2 | |
| OR Quart 3 | 8.9 | | nd | | 11 | |
| p Value | 2.2E-4 | | nd | | 3.2E-4 | |
| 95% CI of | 2.8 | | nd | | 3.0 | |
| OR Quart3 | 28 | | nd | | 41 | |
| OR Quart 4 | 1.2 | | nd | | 1.7 | |
| p Value | 0.75 | | nd | | 0.46 | |
| 95% CI of | 0.32 | | nd | | 0.39 | |
| OR Quart4 | 4.9 | | nd | | 7.8 | |

**Table 12: Comparison of marker levels in enroll EDTA samples collected from Cohort 1 (patients that did not progress beyond RIFLE stage 0 or R within 48hrs) and in enroll EDTA samples collected from Cohort 2 (subjects reaching RIFLE stage I or F within 48hrs). Enroll samples from patients already at stage I or F were included in Cohort 2.**

| **C-C motif chemokine 18** | | | | | | |
|---|---|---|---|---|---|---|
| | sCr or UO | | sCr only | | UO only | |
| | Cohort 1 | Cohort 2 | Cohort 1 | Cohort 2 | Cohort 1 | Cohort 2 |
| Median | 171 | 222 | nd | nd | 160 | 241 |

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Average | 238 | 264 | nd | nd | 230 | 269 |
| Stdev | 192 | 149 | nd | nd | 180 | 151 |
| p(t-test) | | 0.51 | nd | nd | | 0.32 |
| Min | 48.2 | 103 | nd | nd | 48.2 | 103 |
| Max | 1020 | 682 | nd | nd | 817 | 682 |
| n (Samp) | 109 | 28 | nd | nd | 98 | 25 |
| n (Patient) | 109 | 28 | nd | nd | 98 | 25 |
| | | | | | | |

| | At Enrollment | | | | | |
|---|---|---|---|---|---|---|
| | sCr or UO | | sCr only | | UO only | |
| AUC | 0.61 | | nd | | 0.63 | |
| SE | 0.062 | | nd | | 0.065 | |
| p | 0.078 | | nd | | 0.047 | |
| nCohort 1 | 109 | | nd | | 98 | |
| nCohort 2 | 28 | | nd | | 25 | |
| Cutoff 1 | 157 | | nd | | 158 | |
| Sens 1 | 71% | | nd | | 72% | |
| Spec 1 | 48% | | nd | | 50% | |
| Cutoff 2 | 150 | | nd | | 151 | |
| Sens 2 | 82% | | nd | | 80% | |
| Spec 2 | 45% | | nd | | 48% | |
| Cutoff 3 | 117 | | nd | | 117 | |
| Sens 3 | 93% | | nd | | 92% | |
| Spec 3 | 31% | | nd | | 31% | |
| Cutoff 4 | 264 | | nd | | 260 | |
| Sens 4 | 39% | | nd | | 44% | |
| Spec 4 | 71% | | nd | | 70% | |
| Cutoff 5 | 340 | | nd | | 321 | |
| Sens 5 | 25% | | nd | | 24% | |
| Spec 5 | 81% | | nd | | 81% | |
| Cutoff 6 | 548 | | nd | | 548 | |
| Sens 6 | 4% | | nd | | 4% | |
| Spec 6 | 91% | | nd | | 91% | |
| OR Quart 2 | 4.9 | | nd | | 3.4 | |
| p Value | 0.056 | | nd | | 0.16 | |
| 95% CI of | 0.96 | | nd | | 0.62 | |
| OR Quart2 | 25 | | nd | | 18 | |
| OR Quart 3 | 5.8 | | nd | | 5.7 | |
| p Value | 0.034 | | nd | | 0.036 | |
| 95% CI of | 1.1 | | nd | | 1.1 | |
| OR Quart3 | 29 | | nd | | 29 | |
| OR Quart 4 | 5.5 | | nd | | 4.9 | |
| p Value | 0.038 | | nd | | 0.059 | |
| 95% CI of | 1.1 | | nd | | 0.94 | |
| OR Quart4 | 28 | | nd | | 25 | |

| **C-C motif chemokine 24** | | | | | | |
|---|---|---|---|---|---|---|
| | sCr or UO | | sCr only | | UO only | |
| | Cohort 1 | Cohort 2 | Cohort 1 | Cohort 2 | Cohort 1 | Cohort 2 |
| Median | 268 | 224 | 257 | 509 | 275 | 179 |
| Average | 445 | 370 | 419 | 569 | 468 | 291 |
| Stdev | 477 | 370 | 455 | 465 | 495 | 298 |
| p(t-test) | | 0.46 | | 0.40 | | 0.11 |
| Min | 0.0260 | 12.4 | 0.0260 | 68.0 | 13.1 | 12.4 |
| Max | 2920 | 1290 | 2920 | 1290 | 2920 | 1100 |
| n (Samp) | 89 | 26 | 108 | 7 | 81 | 22 |
| n (Patient) | 89 | 26 | 108 | 7 | 81 | 22 |
| | | | | | | |

| | At Enrollment | | | | | |
|---|---|---|---|---|---|---|
| | sCr or UO | | sCr only | | UO only | |
| AUC | 0.44 | | 0.62 | | 0.37 | |
| SE | 0.065 | | 0.12 | | 0.070 | |
| p | 0.40 | | 0.29 | | 0.066 | |
| nCohort 1 | 89 | | 108 | | 81 | |
| nCohort 2 | 26 | | 7 | | 22 | |
| Cutoff 1 | 94.1 | | 279 | | 86.2 | |
| Sens 1 | 73% | | 71% | | 73% | |
| Spec 1 | 17% | | 56% | | 14% | |
| Cutoff 2 | 67.2 | | 169 | | 39.1 | |
| Sens 2 | 81% | | 86% | | 82% | |
| Spec 2 | 9% | | 36% | | 4% | |
| Cutoff 3 | 25.7 | | 67.2 | | 25.7 | |
| Sens 3 | 92% | | 100% | | 91% | |
| Spec 3 | 3% | | 12% | | 2% | |
| Cutoff 4 | 511 | | 490 | | 542 | |
| Sens 4 | 27% | | 57% | | 14% | |
| Spec 4 | 71% | | 70% | | 70% | |
| Cutoff 5 | 737 | | 668 | | 761 | |
| Sens 5 | 15% | | 29% | | 9% | |
| Spec 5 | 81% | | 81% | | 80% | |
| Cutoff 6 | 1110 | | 1080 | | 1110 | |
| Sens 6 | 4% | | 29% | | 0% | |
| Spec 6 | 91% | | 91% | | 90% | |
| OR Quart 2 | 1.5 | | 0.96 | | 1.8 | |
| p Value | 0.52 | | 0.98 | | 0.45 | |
| 95% CI of | 0.42 | | 0.057 | | 0.39 | |
| OR Quart2 | 5.5 | | 16 | | 8.6 | |
| OR Quart 3 | 1.0 | | 3.1 | | 1.8 | |
| p Value | 1.0 | | 0.34 | | 0.45 | |
| 95% CI of | 0.26 | | 0.30 | | 0.39 | |
| OR Quart3 | 3.9 | | 32 | | 8.6 | |
| OR Quart 4 | 2.3 | | 2.0 | | 4.3 | |
| p Value | 0.20 | | 0.58 | | 0.049 | |
| 95% CI of | 0.65 | | 0.17 | | 1.0 | |
| OR Quart4 | 7.9 | | 23 | | 18 | |

| **C-C motif chemokine 8** | | | | | | |
|---|---|---|---|---|---|---|
| | sCr or UO | | sCr only | | UO only | |
| | Cohort 1 | Cohort 2 | Cohort 1 | Cohort 2 | Cohort 1 | Cohort 2 |
| Median | 12.8 | 15.4 | 14.0 | 14.4 | 12.8 | 15.7 |
| Average | 21.9 | 51.7 | 21.2 | 143 | 22.4 | 57.6 |
| Stdev | 31.4 | 144 | 29.0 | 270 | 32.9 | 156 |
| p(t-test) | | 0.070 | | 1.3E-5 | | 0.060 |
| Min | 0.162 | 6.24 | 0.162 | 12.4 | 0.162 | 6.24 |
| Max | 177 | 740 | 177 | 740 | 177 | 740 |
| n (Samp) | 89 | 26 | 108 | 7 | 81 | 22 |
| n (Patient) | 89 | 26 | 108 | 7 | 81 | 22 |
| | | | | | | |

| | At Enrollment | | | | | |
|---|---|---|---|---|---|---|
| | sCr or UO | | sCr only | | UO only | |
| AUC | 0.60 | | 0.68 | | 0.61 | |
| SE | 0.065 | | 0.11 | | 0.070 | |
| p | 0.13 | | 0.11 | | 0.11 | |
| nCohort 1 | 89 | | 108 | | 81 | |
| nCohort 2 | 26 | | 7 | | 22 | |
| Cutoff 1 | 13.0 | | 13.7 | | 13.7 | |
| Sens 1 | 73% | | 71% | | 73% | |
| Spec 1 | 51% | | 47% | | 54% | |
| Cutoff 2 | 12.4 | | 13.0 | | 12.3 | |
| Sens 2 | 81% | | 86% | | 82% | |
| Spec 2 | 45% | | 47% | | 47% | |
| Cutoff 3 | 9.32 | | 12.3 | | 9.32 | |
| Sens 3 | 92% | | 100% | | 91% | |
| Spec 3 | 27% | | 41% | | 28% | |
| Cutoff 4 | 19.9 | | 18.9 | | 18.9 | |
| Sens 4 | 27% | | 43% | | 27% | |
| Spec 4 | 71% | | 70% | | 70% | |
| Cutoff 5 | 22.4 | | 22.4 | | 22.4 | |
| Sens 5 | 27% | | 43% | | 27% | |
| Spec 5 | 81% | | 81% | | 80% | |
| Cutoff 6 | 34.9 | | 33.4 | | 34.9 | |
| Sens 6 | 15% | | 43% | | 14% | |
| Spec 6 | 91% | | 91% | | 90% | |
| OR Quart 2 | 2.7 | | >3.2 | | 2.7 | |
| p Value | 0.19 | | <0.32 | | 0.26 | |
| 95% CI of | 0.61 | | >0.32 | | 0.48 | |
| OR Quart2 | 12 | | na | | 16 | |
| OR Quart 3 | 3.8 | | >1.0 | | 6.1 | |
| p Value | 0.071 | | <1.0 | | 0.032 | |
| 95% CI of | 0.89 | | >0.060 | | 1.2 | |
| OR Quart3 | 16 | | na | | 32 | |
| OR Quart 4 | 2.7 | | >3.2 | | 3.4 | |
| p Value | 0.19 | | <0.32 | | 0.16 | |
| 95% CI of | 0.61 | | >0.32 | | 0.62 | |
| OR Quart4 | 12 | | na | | 19 | |

| **Cathepsin D** | | | | | | |
|---|---|---|---|---|---|---|
| | sCr or UO | | sCr only | | UO only | |
| | Cohort 1 | Cohort 2 | Cohort 1 | Cohort 2 | Cohort 1 | Cohort 2 |
| Median | 234000 | 230000 | nd | nd | 240000 | 244000 |
| Average | 266000 | 284000 | nd | nd | 278000 | 279000 |
| Stdev | 146000 | 165000 | nd | nd | 153000 | 154000 |
| p(t-test) | | 0.55 | nd | nd | | 0.99 |
| Min | 34100 | 126000 | nd | nd | 54000 | 126000 |
| Max | 802000 | 655000 | nd | nd | 802000 | 618000 |
| n (Samp) | 109 | 28 | nd | nd | 98 | 25 |
| n (Patient) | 109 | 28 | nd | nd | 98 | 25 |
| | | | | | | |

| | At Enrollment | | | | | |
|---|---|---|---|---|---|---|
| | sCr or UO | | sCr only | | UO only | |
| AUC | 0.53 | | nd | | 0.50 | |
| SE | 0.062 | | nd | | 0.065 | |
| p | 0.66 | | nd | | 1.0 | |
| nCohort 1 | 109 | | nd | | 98 | |
| nCohort 2 | 28 | | nd | | 25 | |
| Cutoff 1 | 170000 | | nd | | 170000 | |
| Sens 1 | 71% | | nd | | 72% | |
| Spec 1 | 36% | | nd | | 34% | |
| Cutoff 2 | 144000 | | nd | | 144000 | |
| Sens 2 | 82% | | nd | | 80% | |
| Spec 2 | 21% | | nd | | 18% | |
| Cutoff 3 | 135000 | | nd | | 130000 | |
| Sens 3 | 93% | | nd | | 92% | |
| Spec 3 | 17% | | nd | | 14% | |
| Cutoff 4 | 324000 | | nd | | 333000 | |
| Sens 4 | 29% | | nd | | 28% | |
| Spec 4 | 71% | | nd | | 70% | |
| Cutoff 5 | 375000 | | nd | | 382000 | |
| Sens 5 | 25% | | nd | | 24% | |
| Spec 5 | 81% | | nd | | 81% | |
| Cutoff 6 | 471000 | | nd | | 489000 | |
| Sens 6 | 18% | | nd | | 16% | |
| Spec 6 | 91% | | nd | | 91% | |
| OR Quart 2 | 1.4 | | nd | | 0.82 | |
| p Value | 0.55 | | nd | | 0.76 | |
| 95% CI of | 0.44 | | nd | | 0.24 | |
| OR Quart2 | 4.7 | | nd | | 2.8 | |
| OR Quart 3 | 1.0 | | nd | | 0.82 | |
| p Value | 1.0 | | nd | | 0.76 | |
| 95% CI of | 0.29 | | nd | | 0.24 | |
| OR Quart3 | 3.5 | | nd | | 2.8 | |
| OR Quart 4 | 1.4 | | nd | | 0.86 | |
| p Value | 0.59 | | nd | | 0.81 | |
| 95% CI of | 0.42 | | nd | | 0.25 | |
| OR Quart4 | 4.5 | | nd | | 2.9 | |

| **C-X-C motif chemokine 13** | | | | | | |
|---|---|---|---|---|---|---|
| | sCr or UO | | sCr only | | UO only | |
| | Cohort 1 | Cohort 2 | Cohort 1 | Cohort 2 | Cohort 1 | Cohort 2 |
| Median | 55.0 | 53.1 | 53.1 | 54.5 | 55.6 | 53.1 |
| Average | 155 | 212 | 139 | 612 | 164 | 239 |
| Stdev | 292 | 530 | 268 | 949 | 304 | 573 |
| p(t-test) | | 0.48 | | 5.5E-4 | | 0.41 |
| Min | 9.90 | 15.1 | 9.90 | 15.1 | 9.90 | 15.6 |
| Max | 1790 | 2000 | 1790 | 2000 | 1790 | 2000 |
| n (Samp) | 89 | 26 | 108 | 7 | 81 | 22 |
| n (Patient) | 89 | 26 | 108 | 7 | 81 | 22 |
| | | | | | | |

| | At Enrollment | | | | | |
|---|---|---|---|---|---|---|
| | sCr or UO | | sCr only | | UO only | |
| AUC | 0.47 | | 0.57 | | 0.48 | |
| SE | 0.065 | | 0.12 | | 0.070 | |
| p | 0.67 | | 0.55 | | 0.76 | |
| nCohort 1 | 89 | | 108 | | 81 | |
| nCohort 2 | 26 | | 7 | | 22 | |
| Cutoff 1 | 34.2 | | 35.0 | | 34.2 | |
| Sens 1 | 73% | | 71% | | 73% | |
| Spec 1 | 33% | | 32% | | 32% | |
| Cutoff 2 | 28.5 | | 34.2 | | 28.5 | |
| Sens 2 | 81% | | 86% | | 82% | |
| Spec 2 | 25% | | 32% | | 23% | |
| Cutoff 3 | 15.6 | | 14.8 | | 18.1 | |
| Sens 3 | 92% | | 100% | | 91% | |
| Spec 3 | 6% | | 4% | | 12% | |
| Cutoff 4 | 114 | | 101 | | 116 | |
| Sens 4 | 19% | | 43% | | 18% | |
| Spec 4 | 71% | | 70% | | 70% | |
| Cutoff 5 | 194 | | 157 | | 194 | |
| Sens 5 | 12% | | 29% | | 14% | |
| Spec 5 | 81% | | 81% | | 80% | |
| Cutoff 6 | 271 | | 267 | | 271 | |
| Sens 6 | 12% | | 29% | | 14% | |
| Spec 6 | 91% | | 91% | | 90% | |
| OR Quart 2 | 1.8 | | 2.0 | | 1.6 | |
| p Value | 0.35 | | 0.58 | | 0.48 | |
| 95% CI of | 0.52 | | 0.17 | | 0.41 | |
| OR Quart2 | 6.5 | | 23 | | 6.7 | |
| OR Quart 3 | 1.5 | | 0.96 | | 2.0 | |
| p Value | 0.52 | | 0.98 | | 0.31 | |
| 95% CI of | 0.42 | | 0.057 | | 0.51 | |
| OR Quart3 | 5.5 | | 16 | | 8.0 | |
| OR Quart 4 | 1.3 | | 3.1 | | 1.4 | |
| p Value | 0.69 | | 0.34 | | 0.67 | |
| 95% CI of | 0.35 | | 0.30 | | 0.32 | |
| OR Quart4 | 4.9 | | 32 | | 5.8 | |

| **Insulin-like growth factor-binding protein 3** | | | | | | |
|---|---|---|---|---|---|---|
| | sCr or UO | | sCr only | | UO only | |
| | Cohort 1 | Cohort 2 | Cohort 1 | Cohort 2 | Cohort 1 | Cohort 2 |
| Median | 3010 | 3200 | nd | nd | 2940 | 2720 |
| Average | 3450 | 3420 | nd | nd | 3380 | 3240 |
| Stdev | 1530 | 1520 | nd | nd | 1500 | 1470 |
| p(t-test) | | 0.93 | nd | nd | | 0.67 |
| Min | 730 | 1120 | nd | nd | 730 | 1120 |
| Max | 7370 | 7100 | nd | nd | 7260 | 7100 |
| n (Samp) | 109 | 28 | nd | nd | 98 | 25 |
| n (Patient) | 109 | 28 | nd | nd | 98 | 25 |
| | | | | | | |

| | At Enrollment | | | | | |
|---|---|---|---|---|---|---|
| | sCr or UO | | sCr only | | UO only | |
| AUC | 0.50 | | nd | | 0.48 | |
| SE | 0.061 | | nd | | 0.065 | |
| p | 0.97 | | nd | | 0.70 | |
| nCohort 1 | 109 | | nd | | 98 | |
| nCohort 2 | 28 | | nd | | 25 | |
| Cutoff 1 | 2410 | | nd | | 2370 | |
| Sens 1 | 71% | | nd | | 72% | |
| Spec 1 | 32% | | nd | | 32% | |
| Cutoff 2 | 2050 | | nd | | 2050 | |
| Sens 2 | 82% | | nd | | 80% | |
| Spec 2 | 19% | | nd | | 20% | |
| Cutoff 3 | 1400 | | nd | | 1400 | |
| Sens 3 | 93% | | nd | | 92% | |
| Spec 3 | 6% | | nd | | 6% | |
| Cutoff 4 | 4410 | | nd | | 4350 | |
| Sens 4 | 29% | | nd | | 24% | |
| Spec 4 | 71% | | nd | | 70% | |
| Cutoff 5 | 4970 | | nd | | 4900 | |
| Sens 5 | 14% | | nd | | 12% | |
| Spec 5 | 81% | | nd | | 81% | |
| Cutoff 6 | 5550 | | nd | | 5520 | |
| Sens 6 | 7% | | nd | | 4% | |
| Spec 6 | 91% | | nd | | 91% | |
| OR Quart 2 | 1.2 | | nd | | 1.5 | |
| p Value | 0.72 | | nd | | 0.52 | |
| 95% CI of | 0.39 | | nd | | 0.42 | |
| OR Quart2 | 3.9 | | nd | | 5.4 | |
| OR Quart 3 | 0.86 | | nd | | 1.2 | |
| p Value | 0.80 | | nd | | 0.74 | |
| 95% CI of | 0.26 | | nd | | 0.34 | |
| OR Quart3 | 2.9 | | nd | | 4.6 | |
| OR Quart 4 | 1.0 | | nd | | 1.6 | |
| p Value | 0.95 | | nd | | 0.48 | |
| 95% CI of | 0.32 | | nd | | 0.44 | |
| OR Quart4 | 3.4 | | nd | | 5.7 | |

| **Immunoglogulin G1** | | | | | | |
|---|---|---|---|---|---|---|
| | sCr or UO | | sCr only | | UO only | |
| | Cohort 1 | Cohort 2 | Cohort 1 | Cohort 2 | Cohort 1 | Cohort 2 |
| Median | 8690000 | 1.04E7 | nd | nd | 8030000 | 1.04E7 |
| Average | 1.02E7 | 1.14E7 | nd | nd | 9810000 | 1.14E7 |
| Stdev | 5690000 | 5580000 | nd | nd | 5570000 | 5580000 |
| p(t-test) | | 0.44 | nd | nd | | 0.30 |
| Min | 3270000 | 3270000 | nd | nd | 3270000 | 3270000 |
| Max | 3.57E7 | 2.63E7 | nd | nd | 3.57E7 | 2.63E7 |
| n(Samp) | 81 | 17 | nd | nd | 74 | 17 |
| n (Patient) | 81 | 17 | nd | nd | 74 | 17 |
| | | | | | | |

| | At Enrollment | | | | | |
|---|---|---|---|---|---|---|
| | sCr or UO | | sCr only | | UO only | |
| AUC | 0.59 | | nd | | 0.61 | |
| SE | 0.079 | | nd | | 0.079 | |
| p | 0.24 | | nd | | 0.15 | |
| nCohort 1 | 81 | | nd | | 74 | |
| nCohort 2 | 17 | | nd | | 17 | |
| Cutoff 1 | 9280000 | | nd | | 9280000 | |
| Sens 1 | 71% | | nd | | 71% | |
| Spec 1 | 56% | | nd | | 59% | |
| Cutoff 2 | 7600000 | | nd | | 7600000 | |
| Sens 2 | 82% | | nd | | 82% | |
| Spec 2 | 43% | | nd | | 46% | |
| Cutoff 3 | 4390000 | | nd | | 4390000 | |
| Sens 3 | 94% | | nd | | 94% | |
| Spec 3 | 6% | | nd | | 7% | |
| Cutoff 4 | 1.11E7 | | nd | | 1.09E7 | |
| Sens 4 | 41% | | nd | | 47% | |
| Spec 4 | 70% | | nd | | 70% | |
| Cutoff 5 | 1.34E7 | | nd | | 1.32E7 | |
| Sens 5 | 18% | | nd | | 18% | |
| Spec 5 | 80% | | nd | | 81% | |
| Cutoff 6 | 1.65E7 | | nd | | 1.52E7 | |
| Sens 6 | 18% | | nd | | 18% | |
| Spec 6 | 90% | | nd | | 91% | |
| OR Quart 2 | 1.5 | | nd | | 1.5 | |
| p Value | 0.67 | | nd | | 0.67 | |
| 95% CI of | 0.23 | | nd | | 0.23 | |
| OR Quart2 | 9.9 | | nd | | 10.0 | |
| OR Quart 3 | 6.6 | | nd | | 5.3 | |
| p Value | 0.026 | | nd | | 0.052 | |
| 95% CI of | 1.2 | | nd | | 0.99 | |
| OR Quart3 | 35 | | nd | | 29 | |
| OR Quart 4 | 1.5 | | nd | | 2.1 | |
| p Value | 0.67 | | nd | | 0.42 | |
| 95% CI of | 0.23 | | nd | | 0.34 | |
| OR Quart4 | 9.9 | | nd | | 13 | |

| **Immunoglogulin G2** | | | | | | |
|---|---|---|---|---|---|---|
| | sCr or UO | | sCr only | | UO only | |
| | Cohort 1 | Cohort 2 | Cohort 1 | Cohort 2 | Cohort 1 | Cohort 2 |
| Median | 1.08E7 | 1.08E7 | nd | nd | 1.03E7 | 1.08E7 |
| Average | 1.38E7 | 1.11E7 | nd | nd | 1.32E7 | 1.11E7 |
| Stdev | 9680000 | 6680000 | nd | nd | 8830000 | 6680000 |
| p(t-test) | | 0.28 | nd | nd | | 0.37 |
| Min | 563000 | 2450000 | nd | nd | 1750000 | 2450000 |
| Max | 4.88E7 | 2.70E7 | nd | nd | 4.10E7 | 2.70E7 |
| n(Samp) | 81 | 17 | nd | nd | 74 | 17 |
| n (Patient) | 81 | 17 | nd | nd | 74 | 17 |
| | | | | | | |

| | At Enrollment | | | | | |
|---|---|---|---|---|---|---|
| | sCr or UO | | sCr only | | UO only | |
| AUC | 0.44 | | nd | | 0.46 | |
| SE | 0.079 | | nd | | 0.079 | |
| p | 0.45 | | nd | | 0.58 | |
| nCohort 1 | 81 | | nd | | 74 | |
| nCohort 2 | 17 | | nd | | 17 | |
| Cutoff 1 | 7850000 | | nd | | 7850000 | |
| Sens 1 | 71% | | nd | | 71% | |
| Spec 1 | 28% | | nd | | 30% | |
| Cutoff 2 | 1750000 | | nd | | 1750000 | |
| Sens 2 | 100% | | nd | | 100% | |
| Spec 2 | 7% | | nd | | 7% | |
| Cutoff 3 | 1750000 | | nd | | 1750000 | |
| Sens 3 | 100% | | nd | | 100% | |
| Spec 3 | 7% | | nd | | 7% | |
| Cutoff 4 | 1.64E7 | | nd | | 1.64E7 | |
| Sens 4 | 12% | | nd | | 12% | |
| Spec 4 | 70% | | nd | | 73% | |
| Cutoff 5 | 2.01E7 | | nd | | 1.84E7 | |
| Sens 5 | 6% | | nd | | 6% | |
| Spec 5 | 80% | | nd | | 81% | |
| Cutoff 6 | 2.66E7 | | nd | | 2.55E7 | |
| Sens 6 | 6% | | nd | | 6% | |
| Spec 6 | 90% | | nd | | 91% | |
| OR Quart 2 | 4.7 | | nd | | 4.6 | |
| p Value | 0.072 | | nd | | 0.079 | |
| 95% CI of | 0.87 | | nd | | 0.84 | |
| OR Quart2 | 26 | | nd | | 25 | |
| OR Quart 3 | 1.6 | | nd | | 2.2 | |
| p Value | 0.64 | | nd | | 0.39 | |
| 95% CI of | 0.24 | | nd | | 0.36 | |
| OR Quart3 | 10 | | nd | | 13 | |
| OR Quart 4 | 3.0 | | nd | | 2.3 | |
| p Value | 0.21 | | nd | | 0.36 | |
| 95% CI of | 0.53 | | nd | | 0.38 | |
| OR Quart4 | 17 | | nd | | 14 | |

| **Interleukin-11** | | | | | | |
|---|---|---|---|---|---|---|
| | sCr or UO | | sCr only | | UO only | |
| | Cohort 1 | Cohort 2 | Cohort 1 | Cohort 2 | Cohort 1 | Cohort 2 |
| Median | 61.5 | 89.1 | 62.2 | 111 | 61.5 | 97.6 |
| Average | 97.6 | 146 | 95.1 | 316 | 98.2 | 142 |
| Stdev | 111 | 226 | 105 | 390 | 113 | 225 |
| p(t-test) | | 0.14 | | 5.8E-5 | | 0.20 |
| Min | 0.359 | 0.480 | 0.359 | 0.920 | 0.359 | 0.480 |
| Max | 741 | 1060 | 741 | 1060 | 741 | 1060 |
| n (Samp) | 90 | 26 | 109 | 7 | 82 | 22 |
| n (Patient) | 90 | 26 | 109 | 7 | 82 | 22 |
| | | | | | | |

| | At Enrollment | | | | | |
|---|---|---|---|---|---|---|
| | sCr or UO | | sCr only | | UO only | |
| AUC | 0.53 | | 0.64 | | 0.54 | |
| SE | 0.065 | | 0.12 | | 0.070 | |
| p | 0.63 | | 0.22 | | 0.54 | |
| nCohort 1 | 90 | | 109 | | 82 | |
| nCohort 2 | 26 | | 7 | | 22 | |
| Cutoff 1 | 20.4 | | 47.4 | | 33.9 | |
| Sens 1 | 73% | | 71% | | 73% | |
| Spec 1 | 27% | | 46% | | 32% | |
| Cutoff 2 | 15.1 | | 20.4 | | 15.1 | |
| Sens 2 | 81% | | 86% | | 82% | |
| Spec 2 | 21% | | 28% | | 21% | |
| Cutoff 3 | 0.736 | | 0.736 | | 6.00 | |
| Sens 3 | 92% | | 100% | | 91% | |
| Spec 3 | 11% | | 11% | | 15% | |
| Cutoff 4 | 132 | | 132 | | 132 | |
| Sens 4 | 31% | | 43% | | 32% | |
| Spec 4 | 70% | | 71% | | 71% | |
| Cutoff 5 | 173 | | 171 | | 177 | |
| Sens 5 | 19% | | 43% | | 18% | |
| Spec 5 | 80% | | 81% | | 80% | |
| Cutoff 6 | 209 | | 209 | | 209 | |
| Sens 6 | 15% | | 43% | | 14% | |
| Spec 6 | 90% | | 91% | | 90% | |
| OR Quart 2 | 1.0 | | 2.1 | | 0.76 | |
| p Value | 1.0 | | 0.56 | | 0.71 | |
| 95% CI of | 0.28 | | 0.18 | | 0.18 | |
| OR Quart2 | 3.6 | | 24 | | 3.2 | |
| OR Quart 3 | 1.2 | | 1.0 | | 1.5 | |
| p Value | 0.75 | | 1.0 | | 0.51 | |
| 95% CI of | 0.35 | | 0.060 | | 0.42 | |
| OR Quart3 | 4.2 | | 17 | | 5.7 | |
| OR Quart 4 | 1.2 | | 3.2 | | 1.3 | |
| p Value | 0.75 | | 0.32 | | 0.73 | |
| 95% CI of | 0.35 | | 0.32 | | 0.33 | |
| OR Quart4 | 4.2 | | 33 | | 4.8 | |

| **Interleukin-2 receptor alpha chain** | | | | | | |
|---|---|---|---|---|---|---|
| | sCr or UO | | sCr only | | UO only | |
| | Cohort 1 | Cohort 2 | Cohort 1 | Cohort 2 | Cohort 1 | Cohort 2 |
| Median | 25.5 | 27.4 | 25.4 | 33.6 | 28.1 | 27.4 |
| Average | 69.7 | 82.6 | 70.8 | 102 | 74.9 | 93.3 |
| Stdev | 141 | 146 | 139 | 193 | 147 | 157 |
| p(t-test) | | 0.68 | | 0.58 | | 0.61 |
| Min | 0.0290 | 0.0461 | 0.0290 | 0.0569 | 0.0290 | 0.0461 |
| Max | 918 | 562 | 918 | 533 | 918 | 562 |
| n (Samp) | 90 | 26 | 109 | 7 | 82 | 22 |
| n (Patient) | 90 | 26 | 109 | 7 | 82 | 22 |
| | | | | | | |

| | At Enrollment | | | | | |
|---|---|---|---|---|---|---|
| | sCr or UO | | sCr only | | UO only | |
| AUC | 0.53 | | 0.51 | | 0.52 | |
| SE | 0.065 | | 0.11 | | 0.070 | |
| p | 0.69 | | 0.90 | | 0.73 | |
| nCohort 1 | 90 | | 109 | | 82 | |
| nCohort 2 | 26 | | 7 | | 22 | |
| Cutoff 1 | 5.04 | | 4.75 | | 10.6 | |
| Sens 1 | 73% | | 71% | | 73% | |
| Spec 1 | 30% | | 28% | | 32% | |
| Cutoff 2 | 0.0515 | | 0.0515 | | 0.0515 | |
| Sens 2 | 88% | | 100% | | 86% | |
| Spec 2 | 16% | | 16% | | 15% | |
| Cutoff 3 | 0.0359 | | 0.0515 | | 0.0359 | |
| Sens 3 | 100% | | 100% | | 100% | |
| Spec 3 | 9% | | 16% | | 7% | |
| Cutoff 4 | 50.7 | | 60.8 | | 52.5 | |
| Sens 4 | 42% | | 29% | | 45% | |
| Spec 4 | 70% | | 71% | | 71% | |
| Cutoff 5 | 93.9 | | 94.9 | | 104 | |
| Sens 5 | 19% | | 14% | | 18% | |
| Spec 5 | 80% | | 81% | | 80% | |
| Cutoff 6 | 153 | | 155 | | 155 | |
| Sens 6 | 15% | | 14% | | 14% | |
| Spec 6 | 90% | | 91% | | 90% | |
| OR Quart 2 | 1.2 | | 0.48 | | 1.3 | |
| p Value | 0.75 | | 0.56 | | 0.73 | |
| 95% CI of | 0.35 | | 0.041 | | 0.33 | |
| OR Quart2 | 4.2 | | 5.6 | | 4.8 | |
| OR Quart 3 | 1.0 | | 1.0 | | 1.0 | |
| p Value | 1.0 | | 1.0 | | 1.0 | |
| 95% CI of | 0.28 | | 0.13 | | 0.25 | |
| OR Quart3 | 3.6 | | 7.6 | | 4.0 | |
| OR Quart 4 | 1.2 | | 1.0 | | 1.3 | |
| p Value | 0.75 | | 1.0 | | 0.73 | |
| 95% CI of | 0.35 | | 0.13 | | 0.33 | |
| OR Quart4 | 4.2 | | 7.6 | | 4.8 | |

| **Neutrophil collagenase** | | | | | | |
|---|---|---|---|---|---|---|
| | sCr or UO | | sCr only | | UO only | |
| | Cohort 1 | Cohort 2 | Cohort 1 | Cohort 2 | Cohort 1 | Cohort 2 |
| Median | 1260 | 1780 | nd | nd | 1190 | 2410 |
| Average | 3150 | 8240 | nd | nd | 3130 | 9130 |
| Stdev | 5690 | 17400 | nd | nd | 5890 | 18200 |
| p(t-test) | | 0.011 | nd | nd | | 0.0067 |
| Min | 83.9 | 1.14 | nd | nd | 1.14 | 241 |
| Max | 44900 | 83900 | nd | nd | 44900 | 83900 |
| n (Samp) | 108 | 28 | nd | nd | 97 | 25 |
| n (Patient) | 108 | 28 | nd | nd | 97 | 25 |
| | | | | | | |

| | At Enrollment | | | | | |
|---|---|---|---|---|---|---|
| | sCr or UO | | sCr only | | UO only | |
| AUC | 0.58 | | nd | | 0.63 | |
| SE | 0.062 | | nd | | 0.065 | |
| p | 0.19 | | nd | | 0.041 | |
| nCohort 1 | 108 | | nd | | 97 | |
| nCohort 2 | 28 | | nd | | 25 | |
| Cutoff 1 | 1040 | | nd | | 1130 | |
| Sens 1 | 71% | | nd | | 72% | |
| Spec 1 | 41% | | nd | | 49% | |
| Cutoff 2 | 810 | | nd | | 994 | |
| Sens 2 | 82% | | nd | | 80% | |
| Spec 2 | 29% | | nd | | 41% | |
| Cutoff 3 | 261 | | nd | | 712 | |
| Sens 3 | 93% | | nd | | 92% | |
| Spec 3 | 6% | | nd | | 30% | |
| Cutoff 4 | 2370 | | nd | | 2340 | |
| Sens 4 | 46% | | nd | | 52% | |
| Spec 4 | 70% | | nd | | 70% | |
| Cutoff 5 | 3750 | | nd | | 3580 | |
| Sens 5 | 29% | | nd | | 32% | |
| Spec 5 | 81% | | nd | | 80% | |
| Cutoff 6 | 7620 | | nd | | 7370 | |
| Sens 6 | 21% | | nd | | 24% | |
| Spec 6 | 91% | | nd | | 91% | |
| OR Quart 2 | 2.7 | | nd | | 5.7 | |
| p Value | 0.13 | | nd | | 0.036 | |
| 95% CI of | 0.74 | | nd | | 1.1 | |
| OR Quart2 | 9.8 | | nd | | 29 | |
| OR Quart 3 | 1.6 | | nd | | 2.8 | |
| p Value | 0.50 | | nd | | 0.24 | |
| 95% CI of | 0.41 | | nd | | 0.50 | |
| OR Quart3 | 6.3 | | nd | | 16 | |
| OR Quart 4 | 2.7 | | nd | | 5.7 | |
| p Value | 0.13 | | nd | | 0.036 | |
| 95% CI of | 0.74 | | nd | | 1.1 | |
| OR Quart4 | 9.8 | | nd | | 29 | |

| **Protransforming growth factor alpha** | | | | | | |
|---|---|---|---|---|---|---|
| | sCr or UO | | sCr only | | UO only | |
| | Cohort 1 | Cohort 2 | Cohort 1 | Cohort 2 | Cohort 1 | Cohort 2 |
| Median | 1.47 | 4.03 | 1.70 | 7.33 | 1.40 | 4.03 |
| Average | 8.43 | 12.3 | 8.77 | 17.3 | 8.98 | 12.7 |
| Stdev | 32.1 | 18.1 | 30.0 | 22.4 | 33.6 | 19.0 |
| p(t-test) | | 0.56 | | 0.46 | | 0.62 |
| Min | 0.00228 | 0.00228 | 0.00228 | 0.227 | 0.00228 | 0.00228 |
| Max | 245 | 68.3 | 245 | 62.6 | 245 | 68.3 |
| n (Samp) | 90 | 26 | 109 | 7 | 82 | 22 |
| n (Patient) | 90 | 26 | 109 | 7 | 82 | 22 |
| | | | | | | |

| | At Enrollment | | | | | |
|---|---|---|---|---|---|---|
| | sCr or UO | | sCr only | | UO only | |
| AUC | 0.68 | | 0.76 | | 0.69 | |
| SE | 0.063 | | 0.11 | | 0.068 | |
| p | 0.0044 | | 0.018 | | 0.0058 | |
| nCohort 1 | 90 | | 109 | | 82 | |
| nCohort 2 | 26 | | 7 | | 22 | |
| Cutoff 1 | 1.78 | | 5.22 | | 1.78 | |
| Sens 1 | 73% | | 71% | | 73% | |
| Spec 1 | 56% | | 83% | | 59% | |
| Cutoff 2 | 0.885 | | 2.57 | | 0.885 | |
| Sens 2 | 81% | | 86% | | 82% | |
| Spec 2 | 39% | | 59% | | 41% | |
| Cutoff 3 | 0.141 | | 0.141 | | 0.783 | |
| Sens 3 | 92% | | 100% | | 91% | |
| Spec 3 | 21% | | 19% | | 39% | |
| Cutoff 4 | 2.99 | | 3.27 | | 2.93 | |
| Sens 4 | 50% | | 71% | | 50% | |
| Spec 4 | 70% | | 71% | | 71% | |
| Cutoff 5 | 4.00 | | 4.91 | | 4.23 | |
| Sens 5 | 50% | | 71% | | 50% | |
| Spec 5 | 80% | | 81% | | 80% | |
| Cutoff 6 | 5.64 | | 13.6 | | 6.65 | |
| Sens 6 | 42% | | 29% | | 41% | |
| Spec 6 | 90% | | 91% | | 90% | |
| OR Quart 2 | 1.8 | | 0 | | 2.2 | |
| p Value | 0.45 | | na | | 0.39 | |
| 95% CI of | 0.39 | | na | | 0.36 | |
| OR Quart2 | 8.4 | | na | | 13 | |
| OR Quart 3 | 1.8 | | 1.0 | | 2.9 | |
| p Value | 0.45 | | 1.0 | | 0.24 | |
| 95% CI of | 0.39 | | 0.060 | | 0.50 | |
| OR Quart3 | 8.4 | | 17 | | 16 | |
| OR Quart 4 | 7.0 | | 5.8 | | 8.8 | |
| p Value | 0.0063 | | 0.12 | | 0.0093 | |
| 95% CI of | 1.7 | | 0.64 | | 1.7 | |
| OR Quart4 | 29 | | 53 | | 45 | |

| **CA 15-3** | | | | | | |
|---|---|---|---|---|---|---|
| | sCr or UO | | sCr only | | UO only | |
| | Cohort 1 | Cohort 2 | Cohort 1 | Cohort 2 | Cohort 1 | Cohort 2 |
| Median | 0.309 | 0.529 | nd | nd | 0.324 | 0.506 |
| Average | 1.36 | 0.742 | nd | nd | 1.48 | 0.757 |
| Stdev | 5.87 | 0.707 | nd | nd | 6.18 | 0.748 |
| p(t-test) | | 0.58 | nd | nd | | 0.56 |
| Min | 0.0328 | 0.109 | nd | nd | 0.0328 | 0.109 |
| Max | 60.0 | 3.12 | nd | nd | 60.0 | 3.12 |
| n (Samp) | 109 | 28 | nd | nd | 98 | 25 |
| n (Patient) | 109 | 28 | nd | nd | 98 | 25 |
| | | | | | | |

| | At Enrollment | | | | | |
|---|---|---|---|---|---|---|
| | sCr or UO | | sCr only | | UO only | |
| AUC | 0.65 | | nd | | 0.62 | |
| SE | 0.061 | | nd | | 0.066 | |
| p | 0.017 | | nd | | 0.071 | |
| nCohort 1 | 109 | | nd | | 98 | |
| nCohort 2 | 28 | | nd | | 25 | |
| Cutoff 1 | 0.392 | | nd | | 0.385 | |
| Sens 1 | 71% | | nd | | 72% | |
| Spec 1 | 59% | | nd | | 55% | |
| Cutoff 2 | 0.208 | | nd | | 0.208 | |
| Sens 2 | 86% | | nd | | 84% | |
| Spec 2 | 35% | | nd | | 34% | |
| Cutoff 3 | 0.181 | | nd | | 0.181 | |
| Sens 3 | 93% | | nd | | 92% | |
| Spec 3 | 32% | | nd | | 31% | |
| Cutoff 4 | 0.510 | | nd | | 0.535 | |
| Sens 4 | 54% | | nd | | 44% | |
| Spec 4 | 71% | | nd | | 70% | |
| Cutoff 5 | 0.762 | | nd | | 0.864 | |
| Sens 5 | 29% | | nd | | 24% | |
| Spec 5 | 81% | | nd | | 81% | |
| Cutoff 6 | 1.91 | | nd | | 2.03 | |
| Sens 6 | 7% | | nd | | 8% | |
| Spec 6 | 91% | | nd | | 91% | |
| OR Quart 2 | 2.8 | | nd | | 2.7 | |
| p Value | 0.25 | | nd | | 0.26 | |
| 95% CI of | 0.50 | | nd | | 0.48 | |
| OR Quart2 | 15 | | nd | | 15 | |
| OR Quart 3 | 8.7 | | nd | | 6.7 | |
| p Value | 0.0077 | | nd | | 0.022 | |
| 95% CI of | 1.8 | | nd | | 1.3 | |
| OR Quart3 | 43 | | nd | | 34 | |
| OR Quart 4 | 5.5 | | nd | | 4.9 | |
| p Value | 0.038 | | nd | | 0.059 | |
| 95% CI of | 1.1 | | nd | | 0.94 | |
| OR Quart4 | 28 | | nd | | 25 | |

Thus, for example, in each instance herein any of the terms "comprising", "consisting essentially of" and "consisting of" may be replaced with either of the other two terms.

The present disclosure can be further characterized by the following not claimed aspects:
1. A method for evaluating renal status in a subject, comprising:
   performing one or more assays configured to detect one or more biomarkers selected from the group consisting of Cancer antigen CA 15-3, C-C Motif chemokine 18, C-C Motif chemokine 24, Cathepsin D, C-X-C Motif chemokine 13, C-C motif chemokine 8, Interleukin-2 receptor alpha chain, Insulin-like growth factor-binding protein 3, Interleukin-11, Matrix Metalloproteinase-8, Transforming growth factor alpha, IgG1, and IgG2 on a body fluid sample obtained from the subject to provide an assay result; and correlating the assay result(s) to the renal status of the subject.
2. A method according to aspect 1, wherein said correlation step comprises correlating the assay result(s) to one or more of risk stratification, diagnosis, staging, prognosis, classifying and monitoring of the renal status of the subject.
3. A method according to aspect 1, wherein said correlating step comprises assigning a likelihood of one or more future changes in renal status to the subject based on the assay result(s).
4. A method according to aspect 3, wherein said one or more future changes in renal status comprise one or more of a future injury to renal function, future reduced renal function, future improvement in renal function, and future acute renal failure (ARF).
5. A method according to one of aspects 1-4, wherein said assay results comprise at least 2, 3, 4, or 5 of:
   a measured concentration of Cancer antigen CA 15-3,
   a measured concentration of C-C Motif chemokine 18,
   a measured concentration of C-C Motif chemokine 24,
   a measured concentration of Cathepsin D,
   a measured concentration of C-X-C Motif chemokine 13,
   a measured concentration of C-C motif chemokine 8,
   a measured concentration of Interleukin-2 receptor alpha chain,
   a measured concentration of Insulin-like growth factor-binding protein 3,
   a measured concentration of Interleukin-11,
   a measured concentration of Matrix Metalloproteinase-8,
   a measured concentration of Transforming growth factor alpha,
   a measured concentration of IgG1, and
   a measured concentration of IgG2.
6. A method according to one of aspects 1-5, wherein a plurality of assay results are combined using a function that converts the plurality of assay results into a single composite result.
7. A method according to aspect 3, wherein said one or more future changes in renal status comprise a clinical outcome related to a renal injury suffered by the subject.
8. A method according to aspect 3, wherein the likelihood of one or more future changes in renal status is that an event of interest is more or less likely to occur within 30 days of the time at which the body fluid sample is obtained from the subject.
9. A method according to aspect 8, wherein the likelihood of one or more future changes in renal status is that an event of interest is more or less likely to occur within a period selected from the group consisting of 21 days, 14 days, 7 days, 5 days, 96 hours, 72 hours, 48 hours, 36 hours, 24 hours, and 12 hours.
10. A method according to one of aspects 1-5, wherein the subject is selected for evaluation of renal status based on the pre-existence in the subject of one or more known risk factors for prerenal, intrinsic renal, or postrenal ARF.
11. A method according to one of aspects 1-5, wherein the subject is selected for evaluation of renal status based on an existing diagnosis of one or more of congestive heart failure, preeclampsia, eclampsia, diabetes mellitus, hypertension, coronary artery disease, proteinuria, renal insufficiency, glomerular filtration below the normal range, cirrhosis, serum creatinine above the normal range, sepsis, injury to renal function, reduced renal function, or ARF, or based on undergoing or having undergone major vascular surgery, coronary artery bypass, or other cardiac surgery, or based on exposure to NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin.
12. A method according to one of aspects 1-5, wherein said correlating step comprises assigning a diagnosis of the occurrence or nonoccurrence of one or more of an injury to renal function, reduced renal function, or ARF to the subject based on the assay result(s).
13. A method according to one of aspects 1-5, wherein said correlating step comprises assessing whether or not renal function is improving or worsening in a subject who has suffered from an injury to renal function, reduced renal function, or ARF based on the assay result(s).
14. A method according to one of aspects 1-5, wherein said method is a method of diagnosing the occurrence or nonoccurrence of an injury to renal function in said subject.
15. A method according to one of aspects 1-5, wherein said method is a method of diagnosing the occurrence or nonoccurrence of reduced renal function in said subject.
16. A method according to one of aspects 1-5, wherein said method is a method of diagnosing the occurrence or nonoccurrence of acute renal failure in said subject.
17. A method according to one of aspects 1-5, wherein said method is a method of diagnosing the occurrence or nonoccurrence of a need for renal replacement therapy in said subject.
18. A method according to one of aspects 1-5, wherein said method is a method of diagnosing the occurrence or nonoccurrence of a need for renal transplantation in said subject.
19. A method according to one of aspects 1-5, wherein said method is a method of assigning a risk of the future occurrence or nonoccurrence of an injury to renal function in said subject.
20. A method according to one of aspects 1-5, wherein said method is a method of assigning a risk of the future occurrence or nonoccurrence of reduced renal function in said subject.
21. A method according to one of aspects 1-5, wherein said method is a method of assigning a risk of the future occurrence or nonoccurrence of acute renal failure in said subject.
22. A method according to one of aspects 1-5, wherein said method is a method of assigning a risk of the future occurrence or nonoccurrence of a need for renal replacement therapy in said subject.
23. A method according to one of aspects 1-5, wherein said method is a method of assigning a risk of the future occurrence or nonoccurrence of a need for renal transplantation in said subject.
24. A method according to one of aspects 1-5, wherein said one or more future changes in renal status comprise one or more of a future injury to renal function, future reduced renal function, future improvement in renal function, and future acute renal failure (ARF) within 72 hours of the time at which the body fluid sample is obtained.
25. A method according to one of aspects 1-5, wherein said one or more future changes in renal status comprise one or more of a future injury to renal function, future reduced renal function, future improvement in renal function, and future acute renal failure (ARF) within 48 hours of the time at which the body fluid sample is obtained.
26. A method according to one of aspects 1-5, wherein said one or more future changes in renal status comprise one or more of a future injury to renal function, future reduced renal function, future improvement in renal function, and future acute renal failure (ARF) within 24 hours of the time at which the body fluid sample is obtained.
27. A method according to one of aspects 1-5, wherein the subject is in RIFLE stage 0 or R.
28. A method according to aspect 27, wherein the subject is in RIFLE stage 0, and said correlating step comprises assigning a likelihood that the subject will reach RIFLE stage R, I or F within 72 hours.
29. A method according to aspect 28, wherein the subject is in RIFLE stage 0, and said correlating step comprises assigning a likelihood that the subject will reach RIFLE stage I or F within 72 hours.
30. A method according to aspect 28, wherein the subject is in RIFLE stage 0, and said correlating step comprises assigning a likelihood that the subject will reach RIFLE stage F within 72 hours.
31. A method according to aspect 27, wherein the subject is in RIFLE stage 0 or R, and said correlating step comprises assigning a likelihood that the subject will reach RIFLE stage I or F within 72 hours.
32. A method according to aspect 31, wherein the subject is in RIFLE stage 0 or R, and said correlating step comprises assigning a likelihood that the subject will reach RIFLE stage F within 72 hours.
33. A method according to aspect 27, wherein the subject is in RIFLE stage R, and said correlating step comprises assigning a likelihood that the subject will reach RIFLE stage I or F within 72 hours.
34. A method according to aspect 33, wherein the subject is in RIFLE stage R, and said correlating step comprises assigning a likelihood that the subject will reach RIFLE stage F within 72 hours.
35. A method according to one of aspects 1-5, wherein the subject is in RIFLE stage 0, R, or I, and said correlating step comprises assigning a likelihood that the subject will reach RIFLE stage F within 72 hours.
36. A method according to aspect 35, wherein the subject is in RIFLE stage I, and said correlating step comprises assigning a likelihood that the subject will reach RIFLE stage F within 72 hours.
37. A method according to aspect 28, wherein said correlating step comprises assigning a likelihood that the subject will reach RIFLE stage R, I or F within 48 hours.
38. A method according to aspect 29, wherein said correlating step comprises assigning a likelihood that the subject will reach RIFLE stage I or F within 48 hours.
39. A method according to aspect 30, wherein said correlating step comprises assigning a likelihood that the subject will reach RIFLE stage F within 48 hours.
40. A method according to aspect 31, wherein said correlating step comprises assigning a likelihood that the subject will reach RIFLE stage I or F within 48 hours.
41. A method according to aspect 32, wherein said correlating step comprises assigning a likelihood that the subject will reach RIFLE stage F within 48 hours.
42. A method according to aspect 33, wherein said correlating step comprises assigning a likelihood that the subject will reach RIFLE stage I or F within 48 hours.
43. A method according to aspect 34, wherein said correlating step comprises assigning a likelihood that the subject will reach RIFLE stage F within 48 hours.
44. A method according to aspect 35, wherein said correlating step comprises assigning a likelihood that the subject will reach RIFLE stage F within 48 hours.
45. A method according to aspect 36, wherein said correlating step comprises assigning a likelihood that the subject will reach RIFLE stage F within 48 hours.
46. A method according to aspect 28, wherein said correlating step comprises assigning a likelihood that the subject will reach RIFLE stage R, I or F within 24 hours.
47. A method according to aspect 29, wherein said correlating step comprises assigning a likelihood that the subject will reach RIFLE stage I or F within 24 hours.
48. A method according to aspect 30, wherein said correlating step comprises assigning a likelihood that the subject will reach RIFLE stage F within 24 hours.
49. A method according to aspect 31, wherein said correlating step comprises assigning a likelihood that the subject will reach RIFLE stage I or F within 24 hours.
50. A method according to aspect 32, wherein said correlating step comprises assigning a likelihood that the subject will reach RIFLE stage F within 24 hours.
51. A method according to aspect 33, wherein said correlating step comprises assigning a likelihood that the subject will reach RIFLE stage I or F within 24 hours.
52. A method according to aspect 34, wherein said correlating step comprises assigning a likelihood that the subject will reach RIFLE stage F within 24 hours.
53. A method according to aspect 35, wherein said correlating step comprises assigning a likelihood that the subject will reach RIFLE stage F within 24 hours.
54. A method according to aspect 36, wherein said correlating step comprises assigning a likelihood that the subject will reach RIFLE stage F within 24 hours.
55. A method according to one of aspects 1-5, wherein the subject is not in acute renal failure.
56. A method according to one of aspects 1-5, wherein the subject has not experienced a 1.5-fold or greater increase in serum creatinine over a baseline value determined prior to the time at which the body fluid sample is obtained.
57. A method according to one of aspects 1-5, wherein the subject has a urine output of at least 0.5 ml/kg/hr over the 6 hours preceding the time at which the body fluid sample is obtained.
58. A method according to one of aspects 1-5, wherein the subject has not experienced an increase of 0.3 mg/dL or greater in serum creatinine over a baseline value determined prior to the time at which the body fluid sample is obtained.
59. A method according to one of aspects 1-5, wherein the subject (i) has not experienced a 1.5-fold or greater increase in serum creatinine over a baseline value determined prior to the time at which the body fluid sample is obtained, (ii) has a urine output of at least 0.5 ml/kg/hr over the 6 hours preceding the time at which the body fluid sample is obtained, and (iii) has not experienced an increase of 0.3 mg/dL or greater in serum creatinine over a baseline value determined prior to the time at which the body fluid sample is obtained.
60. A method according to one of aspects 1-5, wherein the subject has not experienced a 1.5-fold or greater increase in serum creatinine over a baseline value determined prior to the time at which the body fluid sample is obtained.
61. A method according to one of aspects 1-5, wherein the subject has a urine output of at least 0.5 ml/kg/hr over the 6 hours preceding the time at which the body fluid sample is obtained.
62. A method according to one of aspects 1-5, wherein the subject (i) has not experienced a 1.5-fold or greater increase in serum creatinine over a baseline value determined prior to the time at which the body fluid sample is obtained, (ii) has a urine output of at least 0.5 ml/kg/hr over the 12 hours preceding the time at which the body fluid sample is obtained, and (iii) has not experienced an increase of 0.3 mg/dL or greater in serum creatinine over a baseline value determined prior to the time at which the body fluid sample is obtained.
63. A method according to one of aspects 1-5, wherein said correlating step comprises assigning one or more of: a likelihood that within 72 hours the subject will (i) experience a 1.5-fold or greater increase in serum creatinine (ii) have a urine output of less than 0.5 ml/kg/hr over a 6 hour period, or (iii) experience an increase of 0.3 mg/dL or greater in serum creatinine.
64. A method according to aspect 63, wherein said correlating step comprises assigning one or more of: a likelihood that within 48 hours the subject will (i) experience a 1.5-fold or greater increase in serum creatinine (ii) have a urine output of less than 0.5 ml/kg/hr over a 6 hour period, or (iii) experience an increase of 0.3 mg/dL or greater in serum creatinine.
65. A method according to aspect 63, wherein said correlating step comprises assigning one or more of: a likelihood that within 24 hours the subject will (i) experience a 1.5-fold or greater increase in serum creatinine (ii) have a urine output of less than 0.5 ml/kg/hr over a 6 hour period, or (iii) experience an increase of 0.3 mg/dL or greater in serum creatinine.
66. A method according to aspect 63, wherein said correlating step comprises assigning a likelihood that within 72 hours the subject will experience a 1.5-fold or greater increase in serum creatinine.
67. A method according to aspect 63, wherein said correlating step comprises assigning a likelihood that within 72 hours the subject will have a urine output of less than 0.5 ml/kg/hr over a 6 hour period.
68. A method according to aspect 63, wherein said correlating step comprises assigning a likelihood that within 72 hours the subject will experience an increase of 0.3 mg/dL or greater in serum creatinine.
69. A method according to aspect 63, wherein said correlating step comprises assigning a likelihood that within 48 hours the subject will experience a 1.5-fold or greater increase in serum creatinine.
70. A method according to aspect 63, wherein said correlating step comprises assigning a likelihood that within 48 hours the subject will have a urine output of less than 0.5 ml/kg/hr over a 6 hour period.
71. A method according to aspect 63, wherein said correlating step comprises assigning a likelihood that within 48 hours the subject will experience an increase of 0.3 mg/dL or greater in serum creatinine.
72. A method according to aspect 63, wherein said correlating step comprises assigning a likelihood that within 24 hours the subject will experience a 1.5-fold or greater increase in serum creatinine.
73. A method according to aspect 63, wherein said correlating step comprises assigning a likelihood that within 24 hours the subject will have a urine output of less than 0.5 ml/kg/hr over a 6 hour period.
74. A method according to aspect 63, wherein said correlating step comprises assigning a likelihood that within 24 hours the subject will experience an increase of 0.3 mg/dL or greater in serum creatinine.
75. A method according to one of aspects 1-5, wherein the subject has not experienced a 2-fold or greater increase in serum creatinine over a baseline value determined prior to the time at which the body fluid sample is obtained.
76. A method according to one of aspects 1-5, wherein the subject has a urine output of at least 0.5 ml/kg/hr over the 12 hours preceding the time at which the body fluid sample is obtained.
77. A method according to one of aspects 1-5, wherein the subject (i) has not experienced a 2-fold or greater increase in serum creatinine over a baseline value determined prior to the time at which the body fluid sample is obtained, (ii) has a urine output of at least 0.5 ml/kg/hr over the 2 hours preceding the time at which the body fluid sample is obtained, and (iii) has not experienced an increase of 0.3 mg/dL or greater in serum creatinine over a baseline value determined prior to the time at which the body fluid sample is obtained.
78. A method according to one of aspects 1-5, wherein the subject has not experienced a 3-fold or greater increase in serum creatinine over a baseline value determined prior to the time at which the body fluid sample is obtained.
79. A method according to one of aspects 1-5, wherein the subject has a urine output of at least 0.3 ml/kg/hr over the 24 hours preceding the time at which the body fluid sample is obtained, or anuria over the 12 hours preceding the time at which the body fluid sample is obtained.
80. A method according to one of aspects 1-5, wherein the subject (i) has not experienced a 3-fold or greater increase in serum creatinine over a baseline value determined prior to the time at which the body fluid sample is obtained, (ii) has a urine output of at least 0.3 ml/kg/hr over the 24 hours preceding the time at which the body fluid sample is obtained, or anuria over the 12 hours preceding the time at which the body fluid sample is obtained, and (iii) has not experienced an increase of 0.3 mg/dL or greater in serum creatinine over a baseline value determined prior to the time at which the body fluid sample is obtained.
81. A method according to one of aspects 1-5, wherein said correlating step comprises assigning one or more of: a likelihood that within 72 hours the subject will (i) experience a 2-fold or greater increase in serum creatinine (ii) have a urine output of less than 0.5 ml/kg/hr over a 12 hour period, or (iii) experience an increase of 0.3 mg/dL or greater in serum creatinine.
82. A method according to aspect 81, wherein said correlating step comprises assigning one or more of: a likelihood that within 48 hours the subject will (i) experience a 2-fold or greater increase in serum creatinine (ii) have a urine output of less than 0.5 ml/kg/hr over a 6 hour period, or (iii) experience an increase of 0.3 mg/dL or greater in serum creatinine.
83. A method according to aspect 81, wherein said correlating step comprises assigning one or more of: a likelihood that within 24 hours the subject will (i) experience a 2-fold or greater increase in serum creatinine, or (ii) have a urine output of less than 0.5 ml/kg/hr over a 6 hour period.
84. A method according to aspect 81, wherein said correlating step comprises assigning a likelihood that within 72 hours the subject will experience a 2-fold or greater increase in serum creatinine.
85. A method according to aspect 81, wherein said correlating step comprises assigning a likelihood that within 72 hours the subject will have a urine output of less than 0.5 ml/kg/hr over a 6 hour period.
86. A method according to aspect 81, wherein said correlating step comprises assigning a likelihood that within 48 hours the subject will experience a 2-fold or greater increase in serum creatinine.
87. A method according to aspect 81, wherein said correlating step comprises assigning a likelihood that within 48 hours the subject will have a urine output of less than 0.5 ml/kg/hr over a 6 hour period.
88. A method according to aspect 81, wherein said correlating step comprises assigning a likelihood that within 24 hours the subject will experience a 2-fold or greater increase in serum creatinine.
89. A method according to aspect 81, wherein said correlating step comprises assigning a likelihood that within 24 hours the subject will have a urine output of less than 0.5 ml/kg/hr over a 6 hour period.
90. A method according to one of aspects 1-5, wherein said correlating step comprises assigning one or more of: a likelihood that within 72 hours the subject will (i) experience a 3-fold or greater increase in serum creatinine, or (ii) have a urine output of less than 0.3 ml/kg/hr over a 24 hour period or anuria over a 12 hour period.
91. A method according to aspect 90, wherein said correlating step comprises assigning one or more of: a likelihood that within 48 hours the subject will (i) experience a 3-fold or greater increase in serum creatinine, or (ii) have a urine output of less than 0.3 ml/kg/hr over a 24 hour period or anuria over a 12 hour period.
92. A method according to aspect 90, wherein said correlating step comprises assigning one or more of: a likelihood that within 24 hours the subject will (i) experience a 3-fold or greater increase in serum creatinine, or (ii) have a urine output of less than 0.3 ml/kg/hr over a 24 hour period or anuria over a 12 hour period.
93. A method according to aspect 90, wherein said correlating step comprises assigning a likelihood that within 72 hours the subject will experience a 3-fold or greater increase in serum creatinine.
94. A method according to aspect 90, wherein said correlating step comprises assigning a likelihood that within 72 hours the subject will have a urine output of less than 0.3 ml/kg/hr over a 24 hour period or anuria over a 12 hour period.
95. A method according to aspect 90, wherein said correlating step comprises assigning a likelihood that within 48 hours the subject will experience a 3-fold or greater increase in serum creatinine.
96. A method according to aspect 90, wherein said correlating step comprises assigning a likelihood that within 48 hours the subject will have a urine output of less than 0.3 ml/kg/hr over a 24 hour period or anuria over a 12 hour period.
97. A method according to aspect 90, wherein said correlating step comprises assigning a likelihood that within 24 hours the subject will experience a 3-fold or greater increase in serum creatinine.
98. A method according to aspect 90, wherein said correlating step comprises assigning a likelihood that within 24 hours the subject will have a urine output of less than 0.3 ml/kg/hr over a 24 hour period or anuria over a 12 hour period.
99. A method according to one of aspects 1-98, wherein the body fluid sample is a urine sample.
100. A method according to one of aspects 1-99, wherein said method comprises performing assays that detect one, two or three, or more of Cancer antigen CA 15-3, C-C Motif chemokine 18, C-C Motif chemokine 24, Cathepsin D, C-X-C Motif chemokine 13, C-C motif chemokine 8, Interleukin-2 receptor alpha chain, Insulin-like growth factor-binding protein 3, Interleukin-11, Matrix Metalloproteinase-8, Transforming growth factor alpha, IgG1, and IgG2.
101. Measurement of one or more biomarkers selected from the group consisting of Cancer antigen CA 15-3, C-C Motif chemokine 18, C-C Motif chemokine 24, Cathepsin D, C-X-C Motif chemokine 13, C-C motif chemokine 8, Interleukin-2 receptor alpha chain, Insulin-like growth factor-binding protein 3, Interleukin-11, Matrix Metalloproteinase-8, Transforming growth factor alpha, IgG1, and IgG2 for the evaluation of renal injury.
102. Measurement of one or more biomarkers selected from the group consisting of Cancer antigen CA 15-3, C-C Motif chemokine 18, C-C Motif chemokine 24, Cathepsin D, C-X-C Motif chemokine 13, C-C motif chemokine 8, Interleukin-2 receptor alpha chain, Insulin-like growth factor-binding protein 3, Interleukin-11, Matrix Metalloproteinase-8, Transforming growth factor alpha, IgG1, and IgG2 for the evaluation of acute renal injury.
103. A kit, comprising:
   reagents for performing one or more assays configured to detect one or more kidney injury markers selected from the group consisting of Cancer antigen CA 15-3, C-C Motif chemokine 18, C-C Motif chemokine 24, Cathepsin D, C-X-C Motif chemokine 13, C-C motif chemokine 8, Interleukin-2 receptor alpha chain, Insulin-like growth factor-binding protein 3, Interleukin-11, Matrix Metalloproteinase-8, Transforming growth factor alpha, IgG1, and IgG2.
104. A kit according to aspect 103, wherein said reagents comprise one or more binding reagents, each of which specifically binds one of said of kidney injury markers.
105. A kit according to aspect 104, wherein a plurality of binding reagents are contained in a single assay device.
106. A kit according to aspect 103, wherein at least one of said assays is configured as a sandwich binding assay.
107. A kit according to aspect 103, wherein at least one of said assays is configured as a competitive binding assay.
108. A kit according to one of aspects 103-107, wherein said one or more assays comprise assays that detect one, two or three, or more of Cancer antigen CA 15-3, C-C Motif chemokine 18, C-C Motif chemokine 24, Cathepsin D, C-X-C Motif chemokine 13, C-C motif chemokine 8, Interleukin-2 receptor alpha chain, Insulin-like growth factor-binding protein 3, Interleukin-11, Matrix Metalloproteinase-8, Transforming growth factor alpha, IgG1, and IgG2.

## Claims

1. A method for evaluating renal status in a subject, comprising:
performing one or more assays that generate(s) a detectable signal indicative of the presence or amount of a physiologically relevant concentration of C-C Motif chemokine 24, and optionally one or more biomarkers selected from the group consisting of Cancer antigen CA 15-3, C-C Motif chemokine 18, Cathepsin D, C-X-C Motif chemokine 13, C-C motif chemokine 8, Interleukin-2 receptor alpha chain, Insulin-like growth factor-binding protein 3, Interleukin-11, Matrix Metalloproteinase-8, Transforming growth factor alpha, IgG1, and IgG2 on a body fluid sample that has been obtained from the subject to provide an assay result; and correlating the assay result(s) to the renal status of the subject,
and wherein the correlating step comprises comparing an assay result in the form of the C-C Motif chemokine 24 concentration to a predetermined threshold selected to be indicative of the occurrence or nonoccurrence of a change in renal status or the likelihood of one or more future changes in renal status.

2. A method according to claim 1, wherein said correlation step comprises correlating the assay result(s) to one or more of risk stratification, diagnosis, staging, prognosis, classifying and monitoring of the renal status of the subject.

3. A method according to claim 1, wherein said correlating step comprises assigning a likelihood of one or more future changes in renal status to the subject based on the assay result(s).

4. A method according to claim 3, wherein said one or more future changes in renal status comprise one or more of a future injury to renal function, future reduced renal function, future improvement in renal function, and future acute renal failure (ARF).

5. A method according to one of claims 1-4, wherein said assay results comprise a measured concentration of C-C Motif chemokine 24, and optionally further at least 1, 2, 3, 4, or 5 of:
a measured concentration of Cancer antigen CA 15-3,
a measured concentration of C-C Motif chemokine 18,
a measured concentration of Cathepsin D,
a measured concentration of C-X-C Motif chemokine 13,
a measured concentration of C-C motif chemokine 8,
a measured concentration of Interleukin-2 receptor alpha chain,
a measured concentration of Insulin-like growth factor-binding protein 3,
a measured concentration of Interleukin-11,
a measured concentration of Matrix Metalloproteinase-8,
a measured concentration of Transforming growth factor alpha,
a measured concentration of IgG1, and
a measured concentration of IgG2.

6. A method according to claim 3, wherein said one or more future changes in renal status comprise a clinical outcome related to a renal injury suffered by the subject, or
wherein the likelihood of one or more future changes in renal status is that an event of interest is more or less likely to occur within 30 days of the time at which the body fluid sample is obtained from the subject,
wherein the likelihood of one or more future changes in renal status preferably is that an event of interest is more or less likely to occur within a period selected from the group consisting of 21 days, 14 days, 7 days, 5 days, 96 hours, 72 hours, 48 hours, 36 hours, 24 hours, and 12 hours.

7. A method according to one of claims 1-5, wherein a plurality of assay results are combined using a function that converts the plurality of assay results into a single composite result, or
wherein the subject is selected for evaluation of renal status based on the pre-existence in the subject of one or more known risk factors for prerenal, intrinsic renal, or postrenal ARF, or
wherein the subject is selected for evaluation of renal status based on an existing diagnosis of one or more of congestive heart failure, preeclampsia, eclampsia, diabetes mellitus, hypertension, coronary artery disease, proteinuria, renal insufficiency, glomerular filtration below the normal range, cirrhosis, serum creatinine above the normal range, sepsis, injury to renal function, reduced renal function, or ARF, or based on undergoing or having undergone major vascular surgery, coronary artery bypass, or other cardiac surgery, or based on exposure to NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin, or
wherein said correlating step comprises assigning a diagnosis of the occurrence or nonoccurrence of one or more of an injury to renal function, reduced renal function, or ARF to the subject based on the assay result(s), or
wherein said correlating step comprises assessing whether or not renal function is improving or worsening in a subject who has suffered from an injury to renal function, reduced renal function, or ARF based on the assay result(s), or
wherein said method is a method of diagnosing the occurrence or nonoccurrence of an injury to renal function in said subject, or
wherein said method is a method of diagnosing the occurrence or nonoccurrence of reduced renal function in said subject, or
wherein said method is a method of diagnosing the occurrence or nonoccurrence of acute renal failure in said subject, or
wherein said method is a method of diagnosing the occurrence or nonoccurrence of a need for renal replacement therapy in said subject, or
wherein said method is a method of diagnosing the occurrence or nonoccurrence of a need for renal transplantation in said subject, or
wherein said method is a method of assigning a risk of the future occurrence or nonoccurrence of an injury to renal function in said subject, or
wherein said method is a method of assigning a risk of the future occurrence or nonoccurrence of reduced renal function in said subject, or
wherein said method is a method of assigning a risk of the future occurrence or nonoccurrence of acute renal failure in said subject, or
wherein said method is a method of assigning a risk of the future occurrence or nonoccurrence of a need for renal replacement therapy in said subject, or
wherein said method is a method of assigning a risk of the future occurrence or nonoccurrence of a need for renal transplantation in said subject, or
wherein said one or more future changes in renal status comprise one or more of a future injury to renal function, future reduced renal function, future improvement in renal function, and future acute renal failure (ARF) within 72 hours, within 48 hours or within 24 hours of the time at which the body fluid sample is obtained.

8. A method according to one of claims 1-5, wherein the subject is in RIFLE stage 0 or R.

9. A method according to claim 8, wherein the subject is in RIFLE stage 0, and said correlating step comprises assigning a likelihood that the subject will reach RIFLE stage R, I or F within 72 hours, within 48 hours or within 24 hours.

10. A method according to claim 9, wherein the subject is in RIFLE stage 0, and said correlating step comprises assigning a likelihood that the subject will reach RIFLE stage I or F within 72 hours, within 48 hours or within 24 hours,
wherein the subject is in RIFLE stage 0, and said correlating step comprises assigning a likelihood that the subject will reach RIFLE stage F within 72 hours, within 48 hours or within 24 hours,
wherein the subject is in RIFLE stage 0 or R, and said correlating step comprises assigning a likelihood that the subject will reach RIFLE stage I or F within 72 hours, within 48 hours or within 24 hours,
wherein the subject is in RIFLE stage 0 or R, and said correlating step comprises assigning a likelihood that the subject will reach RIFLE stage F within 72 hours, within 48 hours or within 24 hours,
wherein the subject is in RIFLE stage R, and said correlating step comprises assigning a likelihood that the subject will reach RIFLE stage I or F within 72 hours, within 48 hours or within 24 hours,
wherein the subject is in RIFLE stage R, and said correlating step comprises assigning a likelihood that the subject will reach RIFLE stage F within 72 hours, within 48 hours or within 24 hours,
wherein the subject is in RIFLE stage 0, R, or I, and said correlating step comprises assigning a likelihood that the subject will reach RIFLE stage F within 72 hours, within 48 hours or within 24 hours,
wherein the subject preferably is in RIFLE stage I, and said correlating step comprises assigning a likelihood that the subject will reach RIFLE stage F within 72 hours, within 48 hours or within 24 hours.

11. A method according to one of claims 1-5, wherein the subject is not in acute renal failure, or
wherein the subject has not experienced a 1.5-fold or greater increase in serum creatinine over a baseline value determined prior to the time at which the body fluid sample is obtained, or
wherein the subject has a urine output of at least 0.5 ml/kg/hr over the 6 hours preceding the time at which the body fluid sample is obtained, or
wherein the subject has not experienced an increase of 0.3 mg/dL or greater in serum creatinine over a baseline value determined prior to the time at which the body fluid sample is obtained, or
wherein the subject (i) has not experienced a 1.5-fold or greater increase in serum creatinine over a baseline value determined prior to the time at which the body fluid sample is obtained, (ii) has a urine output of at least 0.5 ml/kg/hr over the 6 hours preceding the time at which the body fluid sample is obtained, and (iii) has not experienced an increase of 0.3 mg/dL or greater in serum creatinine over a baseline value determined prior to the time at which the body fluid sample is obtained, or
wherein the subject has not experienced a 1.5-fold or greater increase in serum creatinine over a baseline value determined prior to the time at which the body fluid sample is obtained, or
wherein the subject has a urine output of at least 0.5 ml/kg/hr over the 6 hours preceding the time at which the body fluid sample is obtained, or
wherein the subject (i) has not experienced a 1.5-fold or greater increase in serum creatinine over a baseline value determined prior to the time at which the body fluid sample is obtained, (ii) has a urine output of at least 0.5 ml kg/hr over the 12 hours preceding the time at which the body fluid sample is obtained, and (iii) has not experienced an increase of 0.3 mg/dL or greater in serum creatinine over a baseline value determined prior to the time at which the body fluid sample is obtained, or
wherein said correlating step comprises assigning one or more of: a likelihood that within 72 hours the subject will (i) experience a 1.5-fold or greater increase in serum creatinine (ii) have a urine output of less than 0.5 ml kg/hr over a 6 hour period, or (iii) experience an increase of 0.3 mg/dL or greater in serum creatinine,
wherein said correlating step preferably comprises assigning one or more of: a likelihood that within 48 hours the subject will (i) experience a 1.5-fold or greater increase in serum creatinine (ii) have a urine output of less than 0.5 ml/kg/hr over a 6 hour period, or (iii) experience an increase of 0.3 mg/dL or greater in serum creatinine, or
wherein said correlating step preferably comprises assigning one or more of: a likelihood that within 24 hours the subject will (i) experience a 1.5-fold or greater increase in serum creatinine (ii) have a urine output of less than 0.5 ml/kg/hr over a 6 hour period, or (iii) experience an increase of 0.3 mg/dL or greater in serum creatinine, or
wherein said correlating step preferably comprises assigning a likelihood that within 72 hours the subject will experience a 1.5-fold or greater increase in serum creatinine, or
wherein said correlating step preferably comprises assigning a likelihood that within 72 hours the subject will have a urine output of less than 0.5 ml/kg/hr over a 6 hour period, or
wherein said correlating step preferably comprises assigning a likelihood that within 72 hours the subject will experience an increase of 0.3 mg/dL or greater in serum creatinine, or
wherein said correlating step preferably comprises assigning a likelihood that within 48 hours the subject will experience a 1.5-fold or greater increase in serum creatinine, or
wherein said correlating step preferably comprises assigning a likelihood that within 48 hours the subject will have a urine output of less than 0.5 ml/kg/hr over a 6 hour period, or
wherein said correlating step preferably comprises assigning a likelihood that within 48 hours the subject will experience an increase of 0.3 mg/dL or greater in serum creatinine, or
wherein said correlating step preferably comprises assigning a likelihood that within 24 hours the subject will experience a 1.5-fold or greater increase in serum creatinine, or
wherein said correlating step preferably comprises assigning a likelihood that within 24 hours the subject will have a urine output of less than 0.5 ml/kg/hr over a 6 hour period, or
wherein said correlating step preferably comprises assigning a likelihood that within 24 hours the subject will experience an increase of 0.3 mg/dL or greater in serum creatinine, or
wherein the subject has not experienced a 2-fold or greater increase in serum creatinine over a baseline value determined prior to the time at which the body fluid sample is obtained, or
wherein the subject has a urine output of at least 0.5 ml/kg/hr over the 12 hours preceding the time at which the body fluid sample is obtained, or
wherein the subject (i) has not experienced a 2-fold or greater increase in serum creatinine over a baseline value determined prior to the time at which the body fluid sample is obtained, (ii) has a urine output of at least 0.5 ml/kg/hr over the 2 hours preceding the time at which the body fluid sample is obtained, and (iii) has not experienced an increase of 0.3 mg/dL or greater in serum creatinine over a baseline value determined prior to the time at which the body fluid sample is obtained, or
wherein the subject has not experienced a 3-fold or greater increase in serum creatinine over a baseline value determined prior to the time at which the body fluid sample is obtained, or
wherein the subject has a urine output of at least 0.3 ml/kg/hr over the 24 hours preceding the time at which the body fluid sample is obtained, or anuria over the 12 hours preceding the time at which the body fluid sample is obtained, or
wherein the subject (i) has not experienced a 3-fold or greater increase in serum creatinine over a baseline value determined prior to the time at which the body fluid sample is obtained, (ii) has a urine output of at least 0.3 ml/kg/hr over the 24 hours preceding the time at which the body fluid sample is obtained, or anuria over the 12 hours preceding the time at which the body fluid sample is obtained, and (iii) has not experienced an increase of 0.3 mg/dL or greater in serum creatinine over a baseline value determined prior to the time at which the body fluid sample is obtained, or
wherein said correlating step comprises assigning one or more of: a likelihood that within 72 hours the subject will (i) experience a 2-fold or greater increase in serum creatinine (ii) have a urine output of less than 0.5 ml/kg/hr over a 12 hour period, or (iii) experience an increase of 0.3 mg/dL or greater in serum creatinine, wherein said correlating step preferably comprises assigning one or more of: a likelihood that within 48 hours the subject will (i) experience a 2-fold or greater increase in serum creatinine (ii) have a urine output of less than 0.5 ml/kg/hr over a 6 hour period, or (iii) experience an increase of 0.3 mg/dL or greater in serum creatinine, or
wherein said correlating step preferably comprises assigning one or more of: a likehhood that within 24 hours the subject will (i) experience a 2-fold or greater increase in serum creatinine, or (ii) have a urine output of less than 0.5 ml/kg/hr over a 6 hour period, or
wherein said correlating step preferably comprises assigning a likelihood that within 72 hours the subject will experience a 2-fold or greater increase in serum creatinine, or
wherein said correlating step preferably comprises assigning a likelihood that within 72 hours the subject will have a urine output of less than 0.5 ml kg/hr over a 6 hour period, or
wherein said correlating step preferably comprises assigning a likelihood that within 48 hours the subject will experience a 2-fold or greater increase in serum creatinine, or wherein said correlating step preferably comprises assigning a likelihood that within 48 hours the subject will have a urine output of less than 0.5 ml kg/hr over a 6 hour period, or
wherein said correlating step preferably comprises assigning a likelihood that within 24 hours the subject will experience a 2-fold or greater increase in serum creatinine, or
wherein said correlating step preferably comprises assigning a likelihood that within 24 hours the subject will have a urine output of less than 0.5 ml/kg/hr over a 6 hour period, or
wherein said correlating step comprises assigning one or more of: a likelihood that within 72 hours the subject will (i) experience a 3-fold or greater increase in serum creatinine, or (ii) have a urine output of less than 0.3 ml/kg/hr over a 24 hour period or anuria over a 12 hour period,
wherein said correlating step preferably comprises assigning one or more of: a likelihood that within 48 hours the subject will (i) experience a 3-fold or greater increase in serum creatinine, or (ii) have a urine output of less than 0.3 ml/kg/hr over a 24 hour period or anuria over a 12 hour period, or
wherein said correlating step preferably comprises assigning one or more of: a likelihood that within 24 hours the subject will (i) experience a 3-fold or greater increase in serum creatinine, or (ii) have a urine output of less than 0.3 ml/kg/hr over a 24 hour period or anuria over a 12 hour period, or
wherein said correlating step preferably comprises assigning a likelihood that within 72 hours the subject will experience a 3-fold or greater increase in serum creatinine, or
wherein said correlating step preferably comprises assigning a likelihood that within 72 hours the subject will have a urine output of less than 0.3 ml/kg/hr over a 24 hour period or anuria over a 12 hour period, or
wherein said correlating step preferably comprises assigning a likelihood that within 48 hours the subject will experience a 3-fold or greater increase in serum creatinine, or
wherein said correlating step preferably comprises assigning a likelihood that within 48 hours the subject will have a urine output of less than 0.3 ml kg/hr over a 24 hour period or anuria over a 12 hour period, or
wherein said correlating step preferably comprises assigning a likelihood that within 24 hours the subject will experience a 3-fold or greater increase in serum creatinine, or
wherein said correlating step preferably comprises assigning a likelihood that within 24 hours the subject will have a urine output of less than 0.3 ml/kg/hr over a 24 hour period or anuria over a 12 hour period.

12. A method according to one of claims 1-11, wherein the body fluid sample is a urine sample, and/or
wherein said method comprises performing assays that detect C-C Motif chemokine 24, and optionally further one, two or three, or more of Cancer antigen CA 15-3, C-C Motif chemokine 18, Cathepsin D, C-X-C Motif chemokine 13, C-C motif chemokine 8, Interleukin-2 receptor alpha chain, Insulin-like growth factor-binding protein 3, Interleukin-11, Matrix Metalloproteinase-8, Transforming growth factor alpha, IgGl and IgG2.

13. In vitro use of C-C Motif chemokine 24 and optionally one or more biomarkers selected from the group consisting of Cancer antigen CA 15-3, C-C Motif chemokine 18, Cathepsin D, C-X-C Motif chemokine 13, C-C motif chemokine 8, Interleukin-2 receptor alpha chain, Insulin-like growth factor-binding protein 3, Interleukin-11, Matrix Metalloproteinase-8, Transforming growth factor alpha, IgG 1, and IgG2 for the evaluation of renal injury, preferably for the evaluation of acute renal injury, said use comprising
comparing a measured concentration of C-C Motif chemokine 24 to a predetermined threshold selected to be indicative of the occurrence or nonoccurrence of a change in renal status or the likelihood of one or more future changes in renal status.

14. The use of claim 13 to be performed with a kit, the kit comprising:
reagents for performing one or more assays configured to detect C-C Motif chemokine 24, and optionally one or more kidney injury markers selected from the group consisting of Cancer antigen CA 15-3, C-C Motif chemokine 18, Cathepsin D, C-X-C Motif chemokine 13, C-C motif chemokine 8, Interleukin-2 receptor alpha chain, Insulin-like growth factor-binding protein 3, Interleukin-11, Matrix Metalloproteinase-8, Transforming growth factor alpha, IgGl, and IgG2,
and reagents comprising detectably labeled antibodies, wherein the detectably labeled antibody comprise antibody that detects the intended biomarker target(s) bound to a detectable label, and
wherein said assay(s) is/are configured as a sandwich binding assay.

15. The use according to claim 14, wherein said one or more assays comprise assays that detect C-C Motif chemokine 24, and optionally further one, two or three, or more of, Cancer antigen CA 15-3, C-C Motif chemokine 18, Cathepsin D, C-X-C Motif chemokine 13, C-C motif chemokine 8, Interleukin-2 receptor alpha chain, Insulin-like growth factor-binding protein 3, Interleukin-11, Matrix Metalloproteinase-8, Transforming growth factor alpha, IgGl and IgG2.

## Patentansprüche

1. Verfahren zum Evaluieren des Nierenstatus eines Subjektes, umfassend:
Durchführen eines oder mehrerer Assays(s), der ein nachweisbares Signal erzeugt, das für das Vorhandensein oder die Menge einer physiologisch relevanten Konzentration von C-C Motiv Chemokin 24 und wahlweise einen oder mehrere Biomarker, der aus der Gruppe ausgewählt wird, die aus Krebsantigen CA 15-3, C-C Motiv Chemokin 18, Cathepsin D, C-X-C Motif Chemokin 13, C-C Motif Chemokin 8, Interleukin-2 Rezeptor Alphakette, insulinähnlichem Wachstumsfaktor-Bindeprotein 3, Interleukin-11, Matrix-Metalloproteinase-8, transformierendem Wachstumsfaktor Alpha, IgGI und IgG2 besteht, indikativ ist, in einer Körperflüssigkeitsprobe, die von dem Subjekt erhalten worden ist, um ein Assayergebnis bereitzustellen; und Korrelieren des/der Assayergebnisse(s) mit dem Nierenstatus des Subjektes,
und wobei der Korrelationsschritt das Vergleichen eines Assayergebnisses in Form der C-C Motiv Chemokin 24-Konzentration mit einem vorher bestimmten Schwellenwert, der ausgewählt ist, um das Auftreten oder Nichtauftreten einer Veränderung in dem Nierenstatus oder die Wahrscheinlichkeit einer oder mehrerer künftiger Veränderungen in dem Nierenstatus, anzuzeigen.

2. Verfahren nach Anspruch 1, wobei der Korrelationsschritt das Korrelieren des/der Assayergebnis(se) mit einem oder mehreren aus Risikostratifizierung, Diagnose, Einstufen, Prognose, Klassifizieren und Überwachen des Nierenstatus des Subjektes umfasst.

3. Verfahren nach Anspruch 1, wobei der Korrelationsschritt das Zuweisen einer Wahrscheinlichkeit von einer oder mehreren künftigen Veränderungen des Nierenstatus des Subjektes, basierend auf dem/den Assayergebnis(sen), umfasst.

4. Verfahren nach Anspruch 3, wobei die eine oder die mehreren künftigen Veränderungen des Nierenstatus eines oder mehrere umfassen aus einer künftigen Verletzung der Nierenfunktion, künftige reduzierte Nierenfunktion, künftige Verbesserung der Nierenfunktion und künftiges akutes Nierenversagen (ARF).

5. Verfahren nach einem der Ansprüche 1-4, wobei die Assayergebnisse eine gemessene Konzentration von C-C Motiv Chemokin 24 umfassen und wahlweise weiterhin mindestens 1, 2, 3, 4 oder 5 aus:
einer gemessenen Konzentration des Krebsantigens CA 15-3,
einer gemessenen Konzentration von C-C Motiv Chemokin 18,
einer gemessenen Konzentration von Cathepsin D,
einer gemessenen Konzentration von C-X-C Motif Chemokin 13,
einer gemessenen Konzentration von C-C Motif Chemokin 8,
einer gemessenen Konzentration der Interleukin-2 Rezeptor Alphakette,
einer gemessenen Konzentration des insulinähnlichen Wachstumsfaktor-Bindeproteins 3,
einer gemessenen Konzentration von Interleukin-11,
einer gemessenen Konzentration der Matrix-Metalloproteinase-8,
einer gemessenen Konzentration des transformierenden Wachstumsfaktors Alpha,
einer gemessenen Konzentration von IgG1 und
einer gemessenen Konzentration von IgG2.

6. Verfahren nach Anspruch 3, wobei die eine oder die mehreren künftigen Veränderungen des Nierenstatus ein klinisches Ergebnis im Zusammenhang mit einer Nierenverletzung, die das Subjekt erlitten hat, umfassen, oder
wobei die Wahrscheinlichkeit von einer oder mehreren künftigen Veränderungen im Nierenstatus darin besteht, dass ein Ereignis von Interesse mehr oder weniger wahrscheinlich innerhalb von 30 Tagen nach dem Zeitpunkt eintritt, an dem die Körperflüssigkeitsprobe von dem Subjekt erhalten wurde,
wobei die Wahrscheinlichkeit von einer oder mehreren künftigen Veränderungen im Nierenstatus vorzugsweise darin besteht, dass ein Ereignis von Interesse mehr oder weniger wahrscheinlich innerhalb eines Zeitraums eintritt, der aus der Gruppe ausgewählt ist, die aus 21 Tagen, 14 Tagen, 7 Tagen, 5 Tagen, 96 Stunden, 72 Stunden, 48 Stunden, 36 Stunden, 24 Stunden und 12 Stunden besteht.

7. Verfahren nach einem der Ansprüche 1-5, wobei eine Vielzahl von Assayergebnissen unter Verwendung einer Funktion kombiniert werden, die die Vielzahl von Assayergebnissen in ein einziges zusammengesetztes Ergebnis umwandelt, oder
wobei das Subjekt zur Beurteilung des Nierenstatus basierend auf dem/den bei dem Subjekt bereits vorhandenen einen oder mehreren bekannten Risikofaktoren für prärenales, immanent renales oder postrenales ARF ausgewählt ist, oder
wobei das Subjekt zur Beurteilung des Nierenstatus ausgewählt ist, basierend auf einer vorhandenen Diagnose von einem oder mehreren aus kongestiver Herzinsuffizienz, Präeklampsie, Eklampsie, Diabetes mellitus, Hypertonie, koronarer Herzkrankheit, Proteinurie, Niereninsuffizienz, glomerulärer Filtration unterhalb des normalen Bereichs, Zirrhose, Serumkreatinin oberhalb des normalen Bereichs, Sepsis, Verletzung der Nierenfunktion, reduzierter Nierenfunktion oder ARF, oder basierend auf einem laufenden oder vorangegangenen größeren gefäßchirurgischen Eingriff, Koronararterien-Bypassoperation oder einer anderen Herzoperation, oder auf der Grundlage einer Exposition gegenüber NSAIDs, Cyclosporinen, Tacrolimus, Aminoglycosiden, Foscarnet, Ethylenglykol, Hämoglobin, Myoglobin, Ifosfamid, Schwermetallen, Methotrexat, röntgendichten Kontrastmitteln oder Streptozotocin, oder
wobei der Korrelationsschritt das Zuweisen einer Diagnose des Auftretens oder Nichtauftretens von einem oder mehreren aus einer Verletzung der Nierenfunktion, reduzierter Nierenfunktion oder ARF bei dem dem Subjekt, basierend auf dem/den Assayergebnis(sen), umfasst, oder
wobei der Korrelationsschritt das Beurteilen, basierend auf dem (den) Assayergebnis(sen), umfasst, ob sich die Nierenfunktion bei einem Subjekt, das eine Verletzung der Nierenfunktion, eine reduzierte Nierenfunktion oder ARF erlitten hat, verbessert oder verschlechtert, oder wobei das Verfahren ein Verfahren zur Diagnose des Auftretens oder Nichtauftretens einer Verletzung der Nierenfunktion bei dem Subjekt ist, oder
wobei das Verfahren ein Verfahren zur Diagnose des Auftretens oder Nichtauftretens einer reduzierten Nierenfunktion bei dem Subjekt ist, oder
wobei das Verfahren ein Verfahren zur Diagnose des Auftretens oder Nichtauftretens von akutem Nierenversagen bei dem Subjekt ist, oder
wobei das Verfahren ein Verfahren zur Diagnose des Auftretens oder Nichtauftretens eines Bedarfs für eine Nierenersatztherapie bei dem Subjekt ist, oder
wobei das Verfahren ein Verfahren zur Diagnose des Auftretens oder Nichtauftretens eines Bedarfs für eine Nierentransplantation bei dem Subjekt ist, oder
wobei das Verfahren ein Verfahren des Zuweisens eines Risikos für das künftige Auftreten oder Nichtauftreten einer Verletzung der Nierenfunktion bei dem Subjekt ist, oder
wobei das Verfahren ein Verfahren des Zuweisens eines Risikos für das künftige Auftreten oder Nichtauftreten einer reduzierten Nierenfunktion bei dem Subjekt ist, oder
wobei das Verfahren ein Verfahren des Zuweisens eines Risikos für das künftige Auftreten oder Nichtauftreten von akutem Nierenversagen bei dem Subjekt ist, oder
wobei das Verfahren ein Verfahren des Zuweisens eines Risikos für das künftige Auftreten oder Nichtauftreten eines Bedarfs für eine Nierenersatztherapie bei dem Subjekt ist, oder
wobei das Verfahren ein Verfahren des Zuweisens eines Risikos für das künftige Auftreten oder Nichtauftreten eines Bedarfs für eine Nierentransplantation bei dem Subjekt ist, oder
wobei eine oder mehrere künftige Veränderungen im Nierenstatus eine oder mehrere umfassen aus einer künftigen Verletzung der Nierenfunktion, einer künftigen reduzierten Nierenfunktion, einer künftigen Verbesserung der Nierenfunktion und künftiges akutes Nierenversagen (ARF) innerhalb von 72 Stunden, innerhalb von 48 Stunden oder innerhalb von 24 Stunden nach dem Zeitpunkt, an dem die Körperflüssigkeitsprobe von dem Subjekt erhalten wurde.

8. Verfahren nach einem der Ansprüche 1-5, wobei das Subjekt in RIFLE-Stadium 0 oder R ist.

9. Verfahren nach Anspruch 8, wobei das Subjekt in RIFLE-Stadium 0 ist und der Korrelationsschritt das Zuweisen einer Wahrscheinlichkeit umfasst, dass das Subjekt RIFLE-Stadium R, I oder F innerhalb von 72 Stunden, innerhalb von 48 Stunden oder innerhalb von 24 Stunden erreichen wird.

10. Verfahren nach Anspruch 9, wobei das Subjekt in RIFLE-Stadium 0 ist und der Korrelationsschritt das Zuweisen einer Wahrscheinlichkeit umfasst, dass das Subjekt RIFLE-Stadium I oder F innerhalb von 72 Stunden, innerhalb von 48 Stunden oder innerhalb von 24 Stunden erreichen wird,
wobei das Subjekt in RIFLE-Stadium 0 ist und der Korrelationsschritt das Zuweisen einer Wahrscheinlichkeit umfasst, dass das Subjekt RIFLE-Stadium F innerhalb von 72 Stunden, innerhalb von 48 Stunden oder innerhalb von 24 Stunden erreichen wird,
wobei das Subjekt in RIFLE-Stadium 0 oder R ist und der Korrelationsschritt das Zuweisen einer Wahrscheinlichkeit umfasst, dass das Subjekt RIFLE-Stadium I oder F innerhalb von 72 Stunden, innerhalb von 48 Stunden oder innerhalb von 24 Stunden erreichen wird,
wobei das Subjekt in RIFLE-Stadium 0 oder R ist und der Korrelationsschritt das Zuweisen einer Wahrscheinlichkeit umfasst, dass das Subjekt RIFLE-Stadium F innerhalb von 72 Stunden, innerhalb von 48 Stunden oder innerhalb von 24 Stunden erreichen wird,
wobei das Subjekt in RIFLE-Stadium R ist und der Korrelationsschritt das Zuweisen einer Wahrscheinlichkeit umfasst, dass das Subjekt RIFLE-Stadium oder I oder F innerhalb von 72 Stunden, innerhalb von 48 Stunden oder innerhalb von 24 Stunden erreichen wird,
wobei das Subjekt in RIFLE-Stadium R ist und der Korrelationsschritt das Zuweisen einer Wahrscheinlichkeit umfasst, dass das Subjekt RIFLE-Stadium F innerhalb von 72 Stunden, innerhalb von 48 Stunden oder innerhalb von 24 Stunden erreichen wird,
wobei das Subjekt in RIFLE-Stadium 0, R oder I ist und der Korrelationsschritt das Zuweisen einer Wahrscheinlichkeit umfasst, dass das Subjekt RIFLE-Stadium F innerhalb von 72 Stunden, innerhalb von 48 Stunden oder innerhalb von 24 Stunden erreichen wird,
wobei das Subjekt vorzugsweise in RIFLE-Stadium I ist und der Korrelationsschritt das Zuweisen einer Wahrscheinlichkeit umfasst, dass das Subjekt RIFLE-Stadium F innerhalb von 72 Stunden, innerhalb von 48 Stunden oder innerhalb von 24 Stunden erreichen wird.

11. Verfahren nach einem der Ansprüche 1-5, wobei das Subjekt kein akutes Nierenversagen hat, oder
wobei das Subjekt keine 1,5-fache oder höhere Zunahme des Serumkreatinins gegenüber einem Basiswert, der vor dem Zeitpunkt, an dem die Körperflüssigkeitsprobe erhalten wurde, bestimmt wurde, erfahren hat, oder
wobei das Subjekt eine Urinausscheidung von mindestens 0,5 ml/kg/Std. über 6 Stunden vor dem Zeitpunkt hat, an dem die Körperflüssigkeitsprobe erhalten wurde, oder
wobei das Subjekt keine Zunahme von 0,3 mg/dL oder mehr des Serumkreatinins gegenüber einem Basiswert, der vor dem Zeitpunkt, an dem die Körperflüssigkeitsprobe erhalten wurde, bestimmt wurde, erfahren hat, oder
wobei das Subjekt (i) keine 1,5-fache oder höhere Zunahme des Serumkreatinins gegenüber einem Basiswert, der vor dem Zeitpunkt, an dem die Körperflüssigkeitsprobe erhalten wurde, bestimmt wurde, erfahren hat, (ii) eine Urinausscheidung von mindestens 0,5 ml/kg/Std. über 6 Stunden vor dem Zeitpunkt hat, an dem die Körperflüssigkeitsprobe erhalten wurde, und (iii) keine Zunahme von 0,3 mg/dL oder mehr des Serumkreatinins gegenüber einem Basiswert, der vor dem Zeitpunkt, an dem die Körperflüssigkeitsprobe erhalten wurde, bestimmt wurde, erfahren hat, oder
wobei das Subjekt keine 1,5-fache oder höhere Zunahme des Serumkreatinins gegenüber einem Basiswert, der vor dem Zeitpunkt, an dem die Körperflüssigkeitsprobe erhalten wurde, bestimmt wurde, erfahren hat, oder
wobei das Subjekt eine Urinauscheidung von mindestens 0,5 ml/kg/Std. über 6 Stunden vor dem Zeitpunkt hat, an dem die Körperflüssigkeitsprobe erhalten wurde, oder
wobei das Subjekt (i) keine 1,5-fache oder höhere Zunahme des Serumkreatinins gegenüber einem Basiswert, der vor dem Zeitpunkt, an dem die Körperflüssigkeitsprobe erhalten wurde, bestimmt wurde, erfahren hat, (ii) eine Urinausscheidung von mindestens 0,5 ml/kg/Std. über 12 Stunden vor dem Zeitpunkt hat, an dem die Körperflüssigkeitsprobe erhalten wurde, und (iii) keine Zunahme von 0,3 mg/dL oder mehr des Serumkreatinins gegenüber einem Basiswert, der vor dem Zeitpunkt, an dem die Körperflüssigkeitsprobe erhalten wurde, bestimmt wurde, erfahren hat, oder
wobei der Korrelationsschritt das Zuweisen von einem oder mehreren umfasst aus: einer Wahrscheinlichkeit, dass das Subjekt innerhalb von 72 Stunden (i) eine 1,5-fache oder höhere Zunahme des Serumkreatinins erfahren wird (ii) eine Urinausscheidung von weniger als 0,5 ml/kg/Std. über einen Zeitraum von 6 Stunden haben wird, oder (iii) eine Zunahme von 0,3 mg/dL oder mehr des Serumkreatinins erfahren wird,
wobei der Korrelationsschritt vorzugsweise das Zuweisen von einem oder mehreren umfasst aus: einer Wahrscheinlichkeit, dass das Subjekt innerhalb von 48 Stunden (i) eine 1,5-fache oder höhere Zunahme des Serumkreatinins erfahren wird (ii) eine Urinausscheidung von weniger als 0,5 ml/kg/Std. über einen Zeitraum von 6 Stunden haben wird, oder (iii) eine Zunahme von 0,3 mg/dL oder mehr im Serumkreatinin erfahren wird, oder
wobei der Korrelationsschritt vorzugsweise das Zuweisen von einem oder mehreren umfasst aus: einer Wahrscheinlichkeit, dass das Subjekt innerhalb von 24 Stunden (i) eine 1,5-fache oder höhere Zunahme des Serumkreatinins erfahren wird (ii) eine Urinausscheidung von weniger als 0,5 ml/kg/Std. über einen Zeitraum von 6 Stunden haben wird, oder (iii) eine Zunahme von 0,3 mg/dL oder mehr des Serumkreatinins erfahren wird,
wobei der Korrelationsschritt vorzugsweise das Zuweisen einer Wahrscheinlichkeit umfasst, dass das Subjekt innerhalb von 72 Stunden eine 1,5-fache oder höhere Zunahme des Serumkreatinins erfahren wird, oder
wobei der Korrelationsschritt vorzugsweise das Zuweisen einer Wahrscheinlichkeit umfasst, dass das Subjekt innerhalb von 72 Stunden eine Urinausscheidung von weniger als 0,5 ml/kg/Std. über einen Zeitraum von 6 Stunden haben wird, oder
wobei der Korrelationsschritt vorzugsweise das Zuweisen einer Wahrscheinlichkeit umfasst, dass das Subjekt innerhalb von 72 Stunden eine Zunahme von 0,3 mg/dL oder mehr des Serumkreatinins erfahren wird, oder
wobei der Korrelationsschritt vorzugsweise das Zuweisen einer Wahrscheinlichkeit umfasst, dass das Subjekt innerhalb von 48 Stunden eine 1,5-fache oder höhere Zunahme des Serumkreatinins erfahren wird, oder
wobei der Korrelationsschritt vorzugsweise das Zuweisen einer Wahrscheinlichkeit umfasst, dass das Subjekt innerhalb von 48 Stunden eine Urinausscheidung von weniger als 0,5 ml/kg/Std. über einen Zeitraum von 6 Stunden haben wird, oder
wobei der Korrelationsschritt vorzugsweise das Zuweisen einer Wahrscheinlichkeit umfasst, dass das Subjekt innerhalb von 48 Stunden eine Zunahme von 0,3 mg/dL oder mehr des Serumkreatinins erfahren wird, oder
wobei der Korrelationsschritt vorzugsweise das Zuweisen einer Wahrscheinlichkeit umfasst, dass das Subjekt innerhalb von 24 Stunden eine 1,5-fache oder höhere Zunahme des Serumkreatinins erfahren wird, oder
wobei der Korrelationsschritt vorzugsweise das Zuweisen einer Wahrscheinlichkeit umfasst, dass das Subjekt innerhalb von 24 Stunden eine Urinausscheidung von weniger als 0,5 ml/kg/Std. über einen Zeitraum von 6 Stunden haben wird,
wobei der Korrelationsschritt vorzugsweise das Zuweisen einer Wahrscheinlichkeit umfasst, dass das Subjekt innerhalb von 24 Stunden eine Zunahme von 0,3 mg/dL oder mehr des Serumkreatinins erfahren wird, oder
wobei das Subjekt keine 2-fache oder höhere Zunahme des Serumkreatinins gegenüber einem Basiswert erfahren hat, der vor dem Zeitpunkt bestimmt wurde, an dem die Körperflüssigkeitsprobe erhalten wurde, oder
wobei das Subjekt eine Urinausscheidung von mindestens 0,5 ml/kg/Std. über 12 Stunden vor dem Zeitpunkt hat, an dem die Körperflüssigkeitsprobe erhalten wurde, oder
wobei das Subjekt (i) keine 2-fache oder höhere Zunahme des Serumkreatinins gegenüber einem Basiswert erfahren hat, der vor dem Zeitpunkt bestimmt wurde, an dem die Körperflüssigkeitsprobe erhalten wurde, (ii) eine Urinausscheidung von mindestens 0,5 ml/kg/Std. über 2 Stunden vor dem Zeitpunkt hat, an dem die Körperflüssigkeitsprobe erhalten wurde, und (iii) keine Zunahme von 0,3 mg/dL oder mehr des Serumkreatinins gegenüber einem Basiswert erfahren hat, der vor dem Zeitpunkt bestimmt wurde, an dem die Körperflüssigkeitsprobe erhalten wurde, oder
wobei das Subjekt keine 3-fache oder höhere Zunahme des Serumkreatinins gegenüber einem Basiswert erfahren hat, der vor dem Zeitpunkt bestimmt wurde, an dem die Körperflüssigkeitsprobe erhalten wurde, oder
wobei das Subjekt eine Urinausscheidung von mindestens 0,3 ml/kg/Std. über 24 Stunden vor dem Zeitpunkt hat, an dem die Körperflüssigkeitsprobe erhalten wurde, oder Anurie über 12 Stunden vor dem Zeitpunkt hat, an dem die Körperflüssigkeitsprobe erhalten wurde, oder
wobei das Subjekt (i) keine 3-fache oder höhere Zunahme des Serumkreatinins gegenüber einem Basiswert erfahren hat, der vor dem Zeitpunkt bestimmt wurde, an dem die Körperflüssigkeitsprobe erhalten wurde, (ii) eine Urinausscheidung von mindestens 0,3 ml/kg/Std. über 24 Stunden vor dem Zeitpunkt hat, an dem die Körperflüssigkeitsprobe erhalten wurde, oder Anurie über die 12 Stunden vor dem Zeitpunkt hat, an dem die Körperflüssigkeitsprobe erhalten wurde, und (iii) keine Zunahme von 0,3 mg/dL oder mehr des Serumkreatinins gegenüber einem Basiswert erfahren hat, der vor dem Zeitpunkt bestimmt wurde, an dem die Körperflüssigkeitsprobe erhalten wurde, oder
wobei der Korrelationsschritt das Zuweisen von einem oder mehreren umfasst aus: einer Wahrscheinlichkeit, dass das Subjekt innerhalb von 72 Stunden (i) eine 2-fache oder höhere Zunahme des Serumkreatinins erfahren wird (ii) eine Urinausscheidung von weniger als 0,5 ml/kg/Std. über einen 12-Stunden-Zeitraum haben wird, oder (iii) eine Zunahme von 0,3 mg/dL oder mehr des Serumkreatinins erfahren wird, wobei der Korrelationsschritt vorzugsweise das Zuweisen von einem oder mehreren umfasst aus: einer Wahrscheinlichkeit, dass das Subjekt innerhalb von 48 Stunden (i) eine 2-fache oder höhere Zunahme des Serumkreatinins erfahren wird (ii) eine Urinausscheidung von weniger als 0,5 ml/kg/Std. über einen 6-Stunden-Zeitraum haben wird, oder (iii) eine Zunahme von 0,3 mg/dL oder mehr des Serumkreatinins erfahren wird, oder
wobei der Korrelationsschritt vorzugsweise das Zuweisen von einem oder mehreren umfasst aus: einer Wahrscheinlichkeit, dass das Subjekt innerhalb von 24 Stunden (i) eine 2-fache oder höhere Zunahme des Serumkreatinins erfahren wird oder (ii) eine Urinausscheidung von weniger als 0,5 ml/kg/Std. über einen 6-Stunden-Zeitraum haben wird, oder
wobei der Korrelationsschritt vorzugsweise das Zuweisen einer Wahrscheinlichkeit umfasst, dass das Subjekt innerhalb von 72 Stunden eine 2-fache oder höhere Zunahme des Serumkreatinins erfahren wird, oder
wobei der Korrelationsschritt vorzugsweise das Zuweisen einer Wahrscheinlichkeit umfasst, dass das Subjekt innerhalb von 72 Stunden eine Urinausscheidung von weniger als 0,5 ml/kg/Std. über einen 6-Stunden-Zeitraum haben wird, oder
wobei der Korrelationsschritt vorzugsweise das Zuweisen einer Wahrscheinlichkeit umfasst, dass das Subjekt innerhalb von 48 Stunden eine 2-fache oder höhere Zunahme des Serumkreatinins erfahren wird, oder
wobei der Korrelationsschritt vorzugsweise das Zuweisen einer Wahrscheinlichkeit umfasst, dass das Subjekt innerhalb von 48 Stunden eine Urinausscheidung von weniger als 0,5 ml/kg/Std. über einen 6-Stunden-Zeitraum haben wird, oder
wobei der Korrelationsschritt vorzugsweise das Zuweisen einer Wahrscheinlichkeit umfasst, dass das Subjekt innerhalb von 24 Stunden eine 2-fache oder höhere Zunahme des Serumkreatinins erfahren wird, oder
wobei der Korrelationsschritt vorzugsweise das Zuweisen einer Wahrscheinlichkeit umfasst, dass das Subjekt innerhalb von 24 Stunden eine Urinausscheidung von weniger als 0,5 ml/kg/Std. über einen 6-Stunden-Zeitraum haben wird, oder
wobei der Korrelationsschritt das Zuweisen von einem oder mehreren umfasst aus: einer Wahrscheinlichkeit, dass das Subjekt innerhalb von 72 Stunden (i) eine 3-fache oder höhere Zunahme des Serumkreatinins erfahren wird oder (ii) eine Urinausscheidung von weniger als 0,3 ml/kg/Std. über einen 24-Stunden-Zeitraum oder Anurie über einen 12-Stunden-Zeitraum haben wird,
wobei der Korrelationsschritt vorzugsweise das Zuweisen von einem oder mehreren umfasst aus: einer Wahrscheinlichkeit, dass das Subjekt innerhalb von 48 Stunden (i) eine 3-fache oder höhere Zunahme des Serumkreatinins erfahren wird (ii) eine Urinausscheidung von weniger als 0,3 ml/kg/Std. über einen 24 Stunden-Zeitraum oder Anurie über einen 12 Stunden-Zeitraum haben wird, oder
wobei der Korrelationsschritt vorzugsweise das Zuweisen von einem oder mehreren umfasst aus: einer Wahrscheinlichkeit, dass das Subjekt innerhalb von 24 Stunden (i) eine 3-fache oder höhere Zunahme des Serumkreatinins erfahren wird (ii) eine Urinausscheidung von weniger als 0,3 ml/kg/Std. über einen 24 Stunden-Zeitraum oder Anurie über einen 12 Stunden-Zeitraum haben wird, oder
wobei der Korrelationsschritt vorzugsweise das Zuweisen einer Wahrscheinlichkeit umfasst, dass das Subjekt innerhalb von 72 Stunden eine 3-fache oder höhere Zunahme des Serumkreatinins erfahren wird, oder
wobei der Korrelationsschritt vorzugsweise das Zuweisen einer Wahrscheinlichkeit umfasst, dass das Subjekt innerhalb von 72 Stunden eine Urinausscheidung von weniger als 0,3 ml/kg/Std. über einen 24-Stunden-Zeitraum oder Anurie über einen 12-Stunden-Zeitraum haben wird, oder
wobei der Korrelationsschritt vorzugsweise das Zuweisen einer Wahrscheinlichkeit umfasst, dass das Subjekt innerhalb von 48 Stunden eine 3-fache oder höhere Zunahme des Serumkreatinins erfahren wird, oder
wobei der Korrelationsschritt vorzugsweise das Zuweisen einer Wahrscheinlichkeit umfasst, dass das Subjekt innerhalb von 48 Stunden eine Urinausscheidung von weniger als 0,3 ml/kg/Std. über einen 24-Stunden-Zeitraum oder Anurie über einen 12-Stunden-Zeitraum haben wird, oder
wobei der Korrelationsschritt vorzugsweise das Zuweisen einer Wahrscheinlichkeit umfasst, dass das Subjekt innerhalb von 24 Stunden eine 3-fache oder höhere Zunahme des Serumkreatinins erfahren wird, oder
wobei der Korrelationsschritt vorzugsweise das Zuweisen einer Wahrscheinlichkeit umfasst, dass das Subjekt innerhalb von 24 Stunden eine Urinausscheidung von weniger als 0,3 ml/kg/Std. über einen 24 Stunden-Zeitraum oder Anurie über einen 12 Stunden-Zeitraum haben wird.

12. Verfahren nach einem der Ansprüche 1-11, wobei die Körperflüssigkeitsprobe eine Urinprobe ist, und/oder
wobei das Verfahren das Durchführen von Assays umfasst, die C-C Motiv Chemokin 24 und wahlweise weiter ein, zwei drei oder mehr von nachweisen aus Krebsantigen CA 15-3, C-C Motiv Chemokin 18, Cathepsin D, C-X-C Motiv Chemokin 13, C-C Motiv Chemokin 8, Interleukin-2 Rezeptor Alphakette, insulinähnliches Wachstumsfaktor-Bindeprotein 3, Interleukin-11, Matrix-Metalloproteinase-8, transformierender Wachstumsfaktor Alpha, IgG1 und IgG2.

13. In vitro Verwendung von C-C Motiv Chemokin 24 und wahlweise einem oder mehreren Biomarkern, die aus der Gruppe ausgewählt sind, die aus Krebsantigen CA 15-3, C-C Motiv Chemokin 18, Cathepsin D, C-X-C Motiv Chemokin 13, C-C Motiv Chemokin 8, Interleukin-2 Rezeptor Alphakette, insulinähnlichem Wachstumsfaktor-Bindeprotein 3, Interleukin-11, Matrix-Metalloproteinase-8, transformierendem Wachstumsfaktor Alpha, IgGI und IgG2 besteht, zur Evaluierung von Nierenverletzung, vorzugsweise zur Evaluierung akuter Nierenverletzung, wobei die Verwendung umfasst:
Vergleichen einer gemessenen C-C Motiv Chemokin 24-Konzentration mit einem vorher bestimmten Schwellenwert, der ausgewählt ist, um das Auftreten oder Nichtauftreten einer Veränderung in dem Nierenstatus oder die Wahrscheinlichkeit einer oder mehrerer künftiger Veränderungen in dem Nierenstatus anzuzeigen.

14. Verwendung nach Anspruch 13, die mit einem Kit durchgeführt wird, wobei das Kit umfasst:
Reagenzien zur Durchführung von einem oder mehreren Assay(s), die dafür konfiguriert sind, C-C Motiv Chemokin 24, und wahlweise einen oder mehrere Nierenverletzungsmarker, die aus der Gruppe ausgewählt sind, die aus Krebsantigen CA 15-3, C-C Motiv Chemokin 18, Cathepsin D, C-X-C Motiv Chemokin 13, C-C Motiv Chemokin 8, Interleukin-2 Rezeptor Alphakette, insulinähnlichem Wachstumsfaktor-Bindeprotein 3, Interleukin-11, Matrix-Metalloproteinase-8, transformierendem Wachstumsfaktor Alpha, IgGI und IgG2 besteht, nachzuweisen
und Reagenzien, die detektierbar markierte Antikörper umfassen, wobei der detektierbar markierte Antikörper einen Antikörper umfasst, der die angestrebte(n) Biomarker-Target(s) detektiert, die an ein detektierbares Label gebunden sind, und
wobei das (die) Assay(s) als Sandwich-Bindungsassay konfiguriert ist (sind).

15. Verwendung nach Anspruch 14, wobei der eine oder die mehreren Assays Assays umfassen, die C-C Motiv Chemokin 24, und wahlweise weiterhin ein, zwei, drei oder mehr nachweist aus: Krebsantigen CA 15-3, C-C Motiv Chemokin 18, Cathepsin D, C-X-C Motiv Chemokin 13, C-C Motiv Chemokin 8, Interleukin-2 Rezeptor Alphakette, insulinähnliches Wachstumsfaktor-Bindeprotein 3, Interleukin-11, Matrix-Metalloproteinase-8, transformierender Wachstumsfaktor Alpha, IgGI und IgG2.

## Revendications

1. Procédé d'évaluation de l'état rénal chez un sujet, comportant:
la réalisation d'un ou de plusieurs analyse(s) qui génèrent un signal détectable indiquant la présence ou la quantité d'une concentration physiologiquement pertinent de C-C motif chimiokine 24, et facultativement un ou plusieurs marqueurs biologiques choisi parmi le groupe constitué de l'antigène de cancer CA 15-3, C-C motif chimiokine 18, la cathepsine D, C-X-C motif chimiokine 13, C-C motif chimiokine 8, la chaîne alpha des récepteurs de l'interleukine-2, la protéine de liaison 3 du facteur de croissance de type insuline, l'interleukine 11, la métalloprotéinase-8 matricielle, le facteur de croissance transformant alpha, IgGI et IgG2 sur un échantillon de fluide corporel qui a été obtenu du sujet pour fournir un résultat d'analyse; et la corrélation du/des résultat(s) d'analyse(s) à l'état rénal du sujet, et l'étape de corrélation comportant la comparaison d'un résultat d'analyse sous la forme de la concentration C-C motif chimiokine 24 avec un seuil prédéterminé choisi pour indiquer l'occurrence ou la non-occurrence d'un changement de l'état rénal ou la probabilité d'un ou de plusieurs changements futurs de l'état rénal.

2. Procédé selon la revendication 1, ladite étape de corrélation comportant la corrélation du (des) résultat(s) d'analyse(s) à un ou plusieurs parmi une stratification des risques, un diagnostic, une stadification, un pronostic, une classification et une surveillance de l'état rénal du sujet.

3. Procédé selon la revendication 1, ladite étape de corrélation comprenant l'assignation d'une probabilité d'un ou de plusieurs changements futurs de l'état rénal chez le sujet en fonction du/des résultat(s) d'analyse(s).

4. Procédé selon la revendication 3, ledit un ou plusieurs changements futurs de l'état rénal comportant un ou plusieurs parmi une lésion future de la fonction rénale, une fonction rénale réduite future, une amélioration future de la fonction rénale et une insuffisance rénale aiguë future (IRA).

5. Procédé selon l'une quelconque des revendications 1-4, lesdits résultats d'analyse comportant une concentration mesurée de C-C motif chimiokine 24, et facultativement en outre au moins 1, 2, 3, 4 ou 5 d'une:
concentration mesurée de l'antigène de cancer CA 15-3,
concentration mesurée de C-C motif chimiokine 18,
concentration mesurée de la cathepsine D,
concentration mesurée de C-X-C motif chimiokine 13,
concentration mesurée de C-C motif chimiokine 8,
concentration mesurée de la chaîne alpha des récepteurs de l'interleukine-2,
concentration mesurée de la protéine de liaison 3 du facteur de croissance de type insuline,
concentration mesurée de l'interleukine 11,
concentration mesurée de la métalloprotéinase-8 matricielle,
concentration mesurée de le facteur de croissance transformant alpha,
concentration mesurée de IgGI et
concentration mesurée de IgG2.

6. Procédé selon la revendication 3, ledit un ou plusieurs changements futurs de l'état rénal comportant un résultat clinique lié à une lésion rénale soufferte par le sujet, ou
la probabilité d'un ou de plusieurs changements futurs de l'état rénal étant qu'un événement d'intérêt est plus ou moins probable dans les 30 jours du moment auquel l'échantillon de fluide corporel a été obtenu du sujet,
la probabilité d'un ou de plusieurs changements futurs de l'état rénal étant de préférence qu'un événement d'intérêt est plus ou moins probable dans un délai choisi parmi le groupe comportant 21 jours, 14 jours, 7 jours, 5 jours, 96 heures, 72 heures, 48 heures, 36 heures, 24 heures et 12 heures.

7. Procédé selon l'une quelconque des revendications 1-5, une pluralité de résultats d'analyse étant combiné en utilisant une fonction qui convertit la pluralité de résultats d'analyse dans un seul résultat composite, ou
le sujet étant sélectionné pour l'évaluation de l'état rénal en fonction de la préexistence chez le sujet d'un ou de plusieurs facteurs de risque connus pour une IRA pré-rénale, rénale intrinsèque, ou post-rénale, ou
le sujet étant sélectionné pour l'évaluation de l'état rénal en fonction d'un diagnostic existant d'un ou de plusieurs parmi une insuffisance cardiaque congestive, une pré-éclampsie, une éclampsie, un diabète sucré, une hypertension, une coronaropathie, une protéinurie, une insuffisance rénale, une filtration glomérulaire inférieure à la plage normale, une cirrhose, la créatinine sérique supérieure à la plage normale, une sepsie, une lésion de la fonction rénale, une fonction rénale réduite, ou une IRA, ou en fonction du fait de subir ou d'avoir subi une intervention chirurgicale vasculaire majeure, un pontage aorto-coronarien, ou une autre intervention cardiaque, ou en fonction de l'exposition à des AINS, à des cyclosporines, au tacrolimus, à des aminoglycosides, au foscarnet, à l'éthylèneglycol, à l'hémoglobine, à la myoglobine, à l'ifosfamide, à des métaux lourds, au méthotrexate, à des agents de contraste radio-opaques, ou à la streptozotocine, ou
ladite étape de corrélation comprenant l'assignation d'un diagnostic de l'occurrence ou de la non-occurrence d'un ou de plusieurs parmi une lésion de la fonction rénale, une fonction rénale réduite ou une IRA chez le sujet en fonction du (des) résultat(s) d'analyse, ou
ladite étape de corrélation comprenant l'évaluation si ou non la fonction rénale s'améliore ou s'aggrave chez un sujet qui a souffert d'une lésion de la fonction rénale, d'une fonction rénale réduite ou d'une IRA en fonction du (des) résultat(s) de l'analyse, ou
ledit procédé étant un procédé de diagnostic de l'occurrence ou de la non-occurrence d'une lésion de la fonction rénale chez ledit sujet, ou
ledit procédé étant un procédé de diagnostic de l'occurrence ou de la non-occurrence de la fonction rénal réduite chez ledit sujet, ou
ledit procédé étant un procédé de diagnostic de l'occurrence ou de la non-occurrence de l'insuffisance rénale aiguë chez ledit sujet, ou
ledit procédé étant un procédé de diagnostic de l'occurrence ou de la non-occurrence d'un besoin de traitement rénal substitutif chez ledit sujet, ou
ledit procédé étant un procédé de diagnostic de l'occurrence ou de la non-occurrence d'un besoin de transplantation rénale chez ledit sujet, ou
ledit procédé étant un procédé de l'assignation d'un risque de l'occurrence ou de non-occurrence future d'une lésion de la fonction rénale chez ledit sujet, ou
ledit procédé étant un procédé de l'assignation d'un risque de l'occurrence ou de non-occurrence future de la fonction rénale réduite chez ledit sujet, ou
ledit procédé étant un procédé de l'assignation d'un risque de l'occurrence ou de non-occurrence future de l'insuffisance rénale aiguë chez ledit sujet, ou
ledit procédé étant un procédé de l'assignation d'un risque de l'occurrence ou de non-occurrence future d'un besoin de traitement rénal substitutif chez ledit sujet, ou
ledit procédé étant un procédé de l'assignation d'un risque de l'occurrence ou de non-occurrence future d'un besoin de transplantation rénale chez ledit sujet, ou
ledit un ou plusieurs changements futurs de l'état rénal comprenant une ou plusieurs d'une lésion future de la fonction rénale, d'une fonction rénale réduite future, d'une amélioration future de la fonction rénale, et d'une insuffisance rénale aiguë (IRA) future dans les 72 heures, dans les 48 heures ou dans les 24 heures du moment auquel l'échantillon de fluide corporel est obtenu.

8. Procédé selon l'une quelconque des revendications 1-5, le sujet étant dans le stade 0 ou R de RIFLE.

9. Procédé selon la revendication 8, le sujet étant au stade 0 de RIFLE, et ladite étape de corrélation comportant l'assignation d'une probabilité que le sujet atteindra le stade R, I ou F de RIFLE dans les 72 heures, dans les 48 heures ou dans les 24 heures.

10. Procédé selon la revendication 9, le sujet étant dans le stade 0 de RIFLE, et ladite étape de corrélation comprenant l'assignation d'une probabilité que le sujet atteindra le stade I ou F de RIFLE dans les 72 heures, dans les 48 heures ou dans les 24 heures,
le sujet étant dans le stade 0 de RIFLE, et ladite étape de corrélation comprenant l'assignation d'une probabilité que le sujet atteindra le stade F de RIFLE dans les 72 heures, dans les 48 heures ou dans les 24 heures,
le sujet étant dans le stade 0 ou R de RIFLE, et ladite étape de corrélation comprenant l'assignation d'une probabilité que le sujet atteindra le stade I ou F de RIFLE dans les 72 heures, dans les 48 heures ou dans les 24 heures,
le sujet étant dans le stade 0 ou R de RIFLE, et ladite étape de corrélation comprenant l'assignation d'une probabilité que le sujet atteindra le stade F de RIFLE dans les 72 heures, dans les 48 heures ou dans les 24 heures,
le sujet étant dans le stade R de RIFLE, et ladite étape de corrélation comprenant l'assignation d'une probabilité que le sujet atteindra le stade I ou F de RIFLE dans les 72 heures, dans les 48 heures ou dans les 24 heures,
le sujet étant dans le stade R de RIFLE, et ladite étape de corrélation comprenant l'assignation d'une probabilité que le sujet atteindra le stade F de RIFLE dans les 72 heures, dans les 48 heures ou dans les 24 heures,
le sujet étant dans le stade 0, R ou I de RIFLE, et ladite étape de corrélation comprenant l'assignation d'une probabilité que le sujet atteindra le stade F de RIFLE dans les 72 heures, dans les 48 heures ou dans les 24 heures,
le sujet étant de préférence dans le stade I de RIFLE, et ladite étape de corrélation comprenant l'assignation d'une probabilité que le sujet atteindra le stade F de RIFLE dans les 72 heures, dans les 48 heures ou dans les 24 heures.

11. Procédé selon l'une quelconque des revendications 1-5, le sujet n'étant pas dans une insuffisance rénale aiguë, ou
le sujet n'ayant pas connu une augmentation de 1,5 fois ou plus de la créatinine sérique par rapport à une valeur de référence déterminée avant le moment auquel l'échantillon de fluide corporel est obtenu, ou
le sujet ayant une production d'urine d'au moins 0,5 ml/kg/heure sur les 6 heures qui précèdent le moment auquel l'échantillon de fluide corporel est obtenu, ou
le sujet n'ayant pas connu une augmentation de 0,3 mg/dL ou plus de la créatinine sérique par rapport à une valeur de référence déterminée avant le moment auquel l'échantillon de fluide corporel est obtenu, ou
le sujet (i) n'ayant pas connu une augmentation de 1,5 fois ou plus de la créatinine sérique par rapport à une valeur de référence déterminée avant le moment auquel l'échantillon de fluide corporel est obtenu, (ii) ayant une production d'urine d'au moins 0,5 ml/kg/heure sur les 6 heures qui précèdent le moment auquel l'échantillon de fluide corporel est obtenu, et (iii) n'ayant pas connu une augmentation de 0,3 mg/dL ou plus de la créatinine sérique par rapport à une valeur de référence déterminée avant le moment auquel l'échantillon de fluide corporel est obtenu, ou
le sujet n'ayant pas connu une augmentation de 1,5 fois ou plus de la créatinine sérique par rapport à une valeur de référence déterminée avant le moment auquel l'échantillon de fluide corporel est obtenu, ou le sujet ayant une production d'urine d'au moins 0,5 ml/kg/heure sur les 6 heures qui précèdent le moment auquel l'échantillon de fluide corporel est obtenu, ou
le sujet (i) n'ayant pas connu une augmentation de 1,5 fois ou plus de la créatinine sérique par rapport à une valeur de référence déterminée avant le moment auquel l'échantillon de fluide corporel est obtenu, (ii) ayant une production d'urine d'au moins 0,5 ml/kg/heure sur les 12 heures qui précèdent le moment auquel l'échantillon de fluide corporel est obtenu, et (iii) n'ayant pas connu une augmentation de 0,3 mg/dL ou plus de la créatinine sérique par rapport à une valeur de référence déterminée avant le moment auquel l'échantillon de fluide corporel est obtenu, ou
ladite étape de corrélation comportant l'assignation d'un ou de plusieurs parmi: une probabilité que dans les 72 heures le sujet (i) connaîtra une augmentation de 1,5 fois ou plus de la créatinine sérique (ii) aura une production d'urine de moins de 0,5 ml/kg/heure sur une période de 6 heures, ou (iii) connaîtra une augmentation de 0,3 mg/dL ou plus de la créatinine sérique,
ladite étape de corrélation comportant de préférence l'assignation d'un ou de plusieurs parmi: une probabilité que dans les 48 heures le sujet (i) connaîtra une augmentation de 1,5 fois ou plus de la créatinine sérique (ii) aura une production d'urine de moins de 0,5 ml/kg/heure sur une période de 6 heures, ou (iii) connaîtra une augmentation de 0,3 mg/dL ou plus de la créatinine sérique, ou
ladite étape de corrélation comportant de préférence l'assignation d'un ou de plusieurs parmi: une probabilité que dans les 24 heures le sujet (i) connaîtra une augmentation de 1,5 fois ou plus de la créatinine sérique (ii) aura une production d'urine de moins de 0,5 ml/kg/heure sur une période de 6 heures, ou (iii) connaîtra une augmentation de 0,3 mg/dL ou plus de la créatinine sérique, ou
ladite étape de corrélation comportant de préférence l'assignation d'une probabilité que dans les 72 heures le sujet connaîtra une augmentation de 1,5 fois ou plus de la créatinine sérique, ou
ladite étape de corrélation comportant de préférence l'assignation d'une probabilité que dans les 72 heures le sujet aura une production d'urine de moins de 0,5 ml/kg/heure sur une période de 6 heures, ou
ladite étape de corrélation comportant de préférence une probabilité que dans les 72 heures le sujet connaîtra une augmentation de 0,3 mg/dL ou plus de la créatinine sérique, ou
ladite étape de corrélation comportant de préférence l'assignation d'une probabilité que dans les 48 heures le sujet connaîtra une augmentation de 1,5 fois ou plus de la créatinine sérique, ou
ladite étape de corrélation comportant de préférence l'assignation d'une probabilité que dans les 48 heures le sujet aura une production d'urine de moins de 0,5 ml/kg/heure sur une période de 6 heures, ou
ladite étape de corrélation comportant de préférence l'assignation d'une probabilité que dans les 48 heures le sujet connaîtra une augmentation de 0,3 mg/dL ou plus de la créatinine sérique, ou
ladite étape de corrélation comportant de préférence l'assignation d'une probabilité que dans les 24 heures le sujet connaîtra une augmentation de 1,5 fois ou plus de la créatinine sérique, ou
ladite étape de corrélation comportant de préférence l'assignation d'une probabilité que dans les 24 heures le sujet aura une production d'urine de moins de 0,5 ml/kg/heure sur une période de 6 heures, ou
ladite étape de corrélation comportant de préférence l'assignation d'une probabilité que dans les 24 heures le sujet connaîtra une augmentation de 0,3 mg/dL ou plus de la créatinine sérique, ou
le sujet n'ayant pas connu une augmentation de 2 fois ou plus de la créatinine sérique par rapport à une valeur de référence déterminée avant le moment auquel l'échantillon de fluide corporel est obtenu, ou
le sujet ayant une production d'urine d'au moins 0,5 ml/kg/heure sur les 12 heures qui précèdent le moment auquel l'échantillon de fluide corporel est obtenu, ou
le sujet (i) n'ayant pas connu une augmentation de 2 fois ou plus de la créatinine sérique par rapport à une valeur de référence déterminée avant le moment auquel l'échantillon de fluide corporel est obtenu, (ii) ayant une production d'urine d'au moins 0,5 ml/kg/heure sur les 2 heures qui précèdent le moment auquel l'échantillon de fluide corporel est obtenu, et (iii) n'ayant pas connu une augmentation de 0,3 mg/dL ou plus de la créatinine sérique par rapport à une valeur de référence déterminée avant le moment auquel l'échantillon de fluide corporel est obtenu, ou
le sujet n'ayant pas connu une augmentation de 3 fois ou plus de la créatinine sérique par rapport à une valeur de référence déterminée avant le moment auquel l'échantillon de fluide corporel est obtenu, ou
le sujet ayant une production d'urine d'au moins 0,3 ml/kg/heure sur les 24 heures qui précèdent le moment auquel l'échantillon de fluide corporel est obtenu, ou de l'anurie sur les 12 heures qui précèdent le moment auquel l'échantillon de fluide corporel est obtenu, ou
le sujet (i) n'ayant pas connu une augmentation de 3 fois ou plus de la créatinine sérique par rapport à une valeur de référence déterminée avant le moment auquel l'échantillon de fluide corporel est obtenu, (ii) ayant une production d'urine d'au moins 0,3 ml/kg/heure sur les 24 heures qui précèdent le moment auquel l'échantillon de fluide corporel est obtenu, ou de l'anurie sur les 12 heures qui précèdent le moment auquel l'échantillon de fluide corporel est obtenu et (iii) n'ayant pas connu une augmentation de 0,3 mg/dL ou plus de la créatinine sérique par rapport à une valeur de référence déterminée avant le moment auquel l'échantillon de fluide corporel est obtenu, ou
ladite étape de corrélation comportant l'assignation d'un ou de plusieurs parmi: une probabilité que dans les 72 heures le sujet (i) connaîtra une augmentation de 2 fois ou plus de la créatinine sérique (ii) aura une production d'urine de moins de 0,5 ml/kg/heure sur une période de 12 heures, ou (iii) connaîtra une augmentation de 0,3 mg/dL ou plus de la créatinine sérique, ladite étape de corrélation comportant de préférence l'assignation d'un ou de plusieurs parmi: une probabilité que dans les 48 heures le sujet (i) connaîtra une augmentation de 2 fois ou plus de la créatinine sérique (ii) aura une production d'urine de moins de 0,5 ml/kg/heure sur une période de 6 heures, ou (iii) connaîtra une augmentation de 0,3 mg/dL ou plus de la créatinine sérique, ou
ladite étape de corrélation comportant de préférence l'assignation d'un ou de plusieurs parmi: une probabilité que dans les 24 heures le sujet (i) connaîtra une augmentation de 2 fois ou plus de la créatinine sérique, ou (ii) aura une production d'urine de moins de 0,5 ml/kg/heure sur une période de 6 heures, ou
ladite étape de corrélation comportant de préférence l'assignation d'une probabilité que dans les 72 heures le sujet connaîtra une augmentation de 2 fois ou plus de la créatinine sérique, ou
ladite étape de corrélation comportant de préférence l'assignation d'une probabilité que dans les 72 heures le sujet aura une production d'urine de moins de 0,5 ml/kg/heure sur une période de 6 heures, ou
ladite étape de corrélation comportant de préférence l'assignation d'une probabilité que dans les 48 heures le sujet connaîtra une augmentation de 2 fois ou plus de la créatinine sérique, ou
ladite étape de corrélation comportant de préférence l'assignation d'une probabilité que dans les 48 heures le sujet aura une production d'urine de moins de 0,5 ml/kg/heure sur une période de 6 heures, ou
ladite étape de corrélation comportant de préférence l'assignation d'une probabilité que dans les 24 heures le sujet connaîtra une augmentation de 2 fois ou plus de la créatinine sérique, ou
ladite étape de corrélation comportant de préférence l'assignation d'une probabilité que dans les 24 heures le sujet aura une production d'urine de moins de 0,5 ml/kg/heure sur une période de 6 heures, ou
ladite étape de corrélation comportant l'assignation d'un ou de plusieurs parmi: une probabilité que dans les 72 heures le sujet (i) connaîtra une augmentation de 3 fois ou plus de la créatinine sérique, ou (ii) aura une production d'urine de moins de 0,3 ml/kg/heure sur une période de 24 heures, ou de l'anurie sur une période de 12 heures,
ladite étape de corrélation comportant de préférence l'assignation d'un ou de plusieurs parmi: une probabilité que dans les 48 heures le sujet (i) connaîtra une augmentation de 3 fois ou plus de la créatinine sérique, ou (ii) aura une production d'urine de moins de 0,3 ml/kg/heure sur une période de 24 heures, ou de l'anurie sur une période de 12 heures, ou
ladite étape de corrélation comportant de préférence l'assignation d'un ou de plusieurs parmi: une probabilité que dans les 24 heures le sujet (i) connaîtra une augmentation de 3 fois ou plus de la créatinine sérique, ou (ii) aura une production d'urine de moins de 0,3 ml/kg/heure sur une période de 24 heures, ou de l'anurie sur une période de 12 heures, ou
ladite étape de corrélation comportant de préférence l'assignation d'une probabilité que dans les 72 heures le sujet connaîtra une augmentation de 3 fois ou plus de la créatinine sérique, ou
ladite étape de corrélation comportant de préférence l'assignation d'une probabilité que dans les 72 heures le sujet aura une production d'urine de moins de 0,3 ml/kg/heure sur une période de 24 heures, ou de l'anurie sur une période de 12 heures, ou
ladite étape de corrélation comportant de préférence l'assignation d'une probabilité que dans les 48 heures le sujet connaîtra une augmentation de 3 fois ou plus de la créatinine sérique, ou
ladite étape de corrélation comportant de préférence l'assignation d'une probabilité que dans les 48 heures le sujet aura une production d'urine de moins de 0,3 ml/kg/heure sur une période de 24 heures, ou de l'anurie sur une période de 12 heures, ou
ladite étape de corrélation comportant de préférence l'assignation d'une probabilité que dans les 24 heures le sujet connaîtra une augmentation de 3 fois ou plus de la créatinine sérique, ou
ladite étape de corrélation comportant de préférence l'assignation d'une probabilité que dans les 24 heures le sujet aura une production d'urine de moins de 0,3 ml/kg/heure sur une période de 24 heures, ou de l'anurie sur une période de 12 heures.

12. Procédé selon l'une quelconque des revendications 1-11, l'échantillon de fluide corporel étant un échantillon d'urine, et/ou
ledit procédé comportant la réalisation des analyses qui détectent la C-C motif chimiokine 24, et facultativement en outre un, deux ou trois ou plusieurs de l'antigène de cancer CA 15-3, C-C motif chimiokine 18, la cathepsine D, C-X-C motif chimiokine 13, C-C motif chimiokine 8, la chaîne alpha des récepteurs de l'interleukine-2, la protéine de liaison 3 du facteur de croissance de type insuline, l'interleukine 11, la métalloprotéinase-8 matricielle, le facteur de croissance transformant alpha, IgGI et IgG2.

13. Utilisation *in vitro* de C-C motif chimiokine 24 et facultativement un ou plusieurs marqueurs biologiques choisi parmi le groupe constitué de l'antigène de cancer CA 15-3, C-C motif chimiokine 18, la cathepsine D, C-X-C motif chimiokine 13, C-C motif chimiokine 8, la chaîne alpha des récepteurs de l'interleukine-2, la protéine de liaison 3 du facteur de croissance de type insuline, l'interleukine 11, la métalloprotéinase-8 matricielle, le facteur de croissance transformant alpha, IgGI et IgG2 pour l'évaluation de la lésion rénale, de préférence pour l'évaluation de la lésion rénale aiguë, l'utilisation comportant
la comparaison d'une concentration mesurée de C-C motif chimiokine 24 à un seuil prédéterminé choisi pour indiquer l'occurrence ou la non-occurrence d'un changement de l'état rénal ou la probabilité d'un ou de plusieurs changements futurs de l'état rénal.

14. Utilisation selon la revendication 13 devant être réalisé au moyen d'un kit, le kit comportant:
des réactifs pour la réalisation d'une ou de plusieurs analyse(s) configurée(s) pour détecter la C-C motif chimiokine 24, et facultativement un ou plusieurs marqueurs de la lésion rénale choisi parmi le groupe constitué de l'antigène de cancer CA 15-3, C-C motif chimiokine 18, la cathepsine D, C-X-C motif chimiokine 13, C-C motif chimiokine 8, la chaîne alpha des récepteurs de l'interleukine-2, la protéine de liaison 3 du facteur de croissance de type insuline, l'interleukine 11, la métalloprotéinase-8 matricielle, le facteur de croissance transformant alpha, IgGI et IgG2,
et des réactifs comportant des anticorps marqués de manière détectable, l'anticorps marqué de manière détectable comportant un anticorps détectant la (les) cible(s) visée(s) des marqueurs biologiques liée(s) à un marqueur détectable, et
ladite (lesdites) analyse(s) étant configuré en tant qu'analyse de liaison «en sandwich».

15. Utilisation selon la revendication 14, ladite une ou plusieurs analyses comportant des analyses qui détectent C-C motif chimiokine 24 et facultativement en outre un, deux ou trois ou plusieurs de l'antigène de cancer CA 15-3, C-C motif chimiokine 18, la cathepsine D, C-X-C motif chimiokine 13, C-C motif chimiokine 8, la chaîne alpha des récepteurs de l'interleukine-2, la protéine de liaison 3 du facteur de croissance de type insuline, l'interleukine 11, la métalloprotéinase-8 matricielle, le facteur de croissance transformant alpha, IgGI et IgG2.


### SEQUENCE LISTING

<110> ASTUTE MEDICAL, INC.
<120> METHODS AND COMPOSITIONS FOR DIAGNOSIS AND PROGNOSIS OF RENAL INJURY AND RENAL FAILURE
<130> BLG14661PCTEPD1
<140> Divisional Application of EP 11 798 515.0
   <141> 2011-06-23
<150> 61/364,300
   <151> 2010-07-14
<150> 61/364,304
   <151> 2010-07-14
<150> 61/357,956
   <151> 2010-06-23
<150> 61/357,965
   <151> 2010-06-23
<160> 13
<170> PatentIn version 3.5
<210> 1
   <211> 99
   <212> PRT
   <213> Homo sapiens
<400> 1
   Met Lys Val Ser Ala Ala Leu Leu Cys Leu Leu Leu Met Ala Ala Thr
   1               5                   10                  15
   Phe Ser Pro Gln Gly Leu Ala Gln Pro Asp Ser Val Ser Ile Pro Ile
               20                  25                  30
   Thr Cys Cys Phe Asn Val Ile Asn Arg Lys Ile Pro Ile Gln Arg Leu
           35                  40                  45
   Glu Ser Tyr Thr Arg Ile Thr Asn Ile Gln Cys Pro Lys Glu Ala Val
       50                  55                  60
   Ile Phe Lys Thr Lys Arg Gly Lys Glu Val Cys Ala Asp Pro Lys Glu
   65                  70                  75                  80
   Arg Trp Val Arg Asp Ser Met Lys His Leu Asp Gln Ile Phe Gln Asn
                   85                  90                  95
   Leu Lys Pro
<210> 2
   <211> 272
   <212> PRT
   <213> Homo sapiens
<400> 2
   Met Asp Ser Tyr Leu Leu Met Trp Gly Leu Leu Thr Phe Ile Met Val
   1               5                   10                  15
   Pro Gly Cys Gln Ala Glu Leu Cys Asp Asp Asp Pro Pro Glu Ile Pro
               20                  25                  30
   His Ala Thr Phe Lys Ala Met Ala Tyr Lys Glu Gly Thr Met Leu Asn
           35                  40                  45
   Cys Glu Cys Lys Arg Gly Phe Arg Arg Ile Lys Ser Gly Ser Leu Tyr
       50                  55                  60
   Met Leu Cys Thr Gly Asn Ser Ser His Ser Ser Trp Asp Asn Gln Cys
   65                  70                  75                  80
   Gln Cys Thr Ser Ser Ala Thr Arg Asn Thr Thr Lys Gln Val Thr Pro
                   85                  90                  95
   Gln Pro Glu Glu Gln Lys Glu Arg Lys Thr Thr Glu Met Gln Ser Pro
               100                 105                 110
   Met Gln Pro Val Asp Gln Ala Ser Leu Pro Gly His Cys Arg Glu Pro
           115                 120                 125
   Pro Pro Trp Glu Asn Glu Ala Thr Glu Arg Ile Tyr His Phe Val Val
       130                 135                 140
   Gly Gln Met Val Tyr Tyr Gln Cys Val Gln Gly Tyr Arg Ala Leu His
   145                 150                 155                 160
   Arg Gly Pro Ala Glu Ser Val Cys Lys Met Thr His Gly Lys Thr Arg
                   165                 170                 175
   Trp Thr Gln Pro Gln Leu Ile Cys Thr Gly Glu Met Glu Thr Ser Gln
               180                 185                 190
   Phe Pro Gly Glu Glu Lys Pro Gln Ala Ser Pro Glu Gly Arg Pro Glu
           195                 200                 205
   Ser Glu Thr Ser Cys Leu Val Thr Thr Thr Asp Phe Gln Ile Gln Thr
       210                 215                 220
   Glu Met Ala Ala Thr Met Glu Thr Ser Ile Phe Thr Thr Glu Tyr Gln
   225                 230                 235                 240
   Val Ala Val Ala Gly Cys Val Phe Leu Leu Ile Ser Val Leu Leu Leu
                   245                 250                 255
   Ser Gly Leu Thr Trp Gln Arg Arg Gln Arg Lys Ser Arg Arg Thr Ile
               260                 265                 270
<210> 3
   <211> 291
   <212> PRT
   <213> Homo sapiens
<400> 3
   Met Gln Arg Ala Arg Pro Thr Leu Trp Ala Ala Ala Leu Thr Leu Leu
   1               5                   10                  15
   Val Leu Leu Arg Gly Pro Pro Val Ala Arg Ala Gly Ala Ser Ser Ala
               20                  25                  30
   Gly Leu Gly Pro Val Val Arg Cys Glu Pro Cys Asp Ala Arg Ala Leu
           35                  40                  45
   Ala Gln Cys Ala Pro Pro Pro Ala Val Cys Ala Glu Leu Val Arg Glu
       50                  55                  60
   Pro Gly Cys Gly Cys Cys Leu Thr Cys Ala Leu Ser Glu Gly Gln Pro
   65                  70                  75                  80
   Cys Gly Ile Tyr Thr Glu Arg Cys Gly Ser Gly Leu Arg Cys Gln Pro
                   85                  90                  95
   Ser Pro Asp Glu Ala Arg Pro Leu Gln Ala Leu Leu Asp Gly Arg Gly
               100                 105                 110
   Leu Cys Val Asn Ala Ser Ala Val Ser Arg Leu Arg Ala Tyr Leu Leu
           115                 120                 125
   Pro Ala Pro Pro Ala Pro Gly Asn Ala Ser Glu Ser Glu Glu Asp Arg
       130                 135                 140
   Ser Ala Gly Ser Val Glu Ser Pro Ser Val Ser Ser Thr His Arg Val
   145                 150                 155                 160
   Ser Asp Pro Lys Phe His Pro Leu His Ser Lys Ile Ile Ile Ile Lys
                   165                 170                 175
   Lys Gly His Ala Lys Asp Ser Gln Arg Tyr Lys Val Asp Tyr Glu Ser
               180                 185                 190
   Gln Ser Thr Asp Thr Gln Asn Phe Ser Ser Glu Ser Lys Arg Glu Thr
   195 200 205
   Glu Tyr Gly Pro Cys Arg Arg Glu Met Glu Asp Thr Leu Asn His Leu
   210 215 220
   Lys Phe Leu Asn Val Leu Ser Pro Arg Gly Val His Ile Pro Asn Cys
   225 230 235 240
   Asp Lys Lys Gly Phe Tyr Lys Lys Lys Gln Cys Arg Pro Ser Lys Gly
   245 250 255
   Arg Lys Arg Gly Phe Cys Trp Cys Val Asp Lys Tyr Gly Gln Pro Leu
   260 265 270
   Pro Gly Tyr Thr Thr Lys Gly Lys Glu Asp Val His Cys Tyr Ser Met
   275 280 285
   Gln Ser Lys
   290
<210> 4
   <211> 199
   <212> PRT
   <213> Homo sapiens
<400> 4
   Met Asn Cys Val Cys Arg Leu Val Leu Val Val Leu Ser Leu Trp Pro
   1 5 10 15
   Asp Thr Ala Val Ala Pro Gly Pro Pro Pro Gly Pro Pro Arg Val Ser
   20 25 30
   Pro Asp Pro Arg Ala Glu Leu Asp Ser Thr Val Leu Leu Thr Arg Ser
   35 40 45
   Leu Leu Ala Asp Thr Arg Gln Leu Ala Ala Gln Leu Arg Asp Lys Phe
   50 55 60
   Pro Ala Asp Gly Asp His Asn Leu Asp Ser Leu Pro Thr Leu Ala Met
   65 70 75 80
   Ser Ala Gly Ala Leu Gly Ala Leu Gln Leu Pro Gly Val Leu Thr Arg
   85 90 95
   Leu Arg Ala Asp Leu Leu Ser Tyr Leu Arg His Val Gln Trp Leu Arg
   100 105 110
   Arg Ala Gly Gly Ser Ser Leu Lys Thr Leu Glu Pro Glu Leu Gly Thr
           115                 120                 125
   Leu Gln Ala Arg Leu Asp Arg Leu Leu Arg Arg Leu Gln Leu Leu Met
       130                 135                 140
   Ser Arg Leu Ala Leu Pro Gln Pro Pro Pro Asp Pro Pro Ala Pro Pro
   145                 150                 155                 160
   Leu Ala Pro Pro Ser Ser Ala Trp Gly Gly Ile Arg Ala Ala His Ala
                   165                 170                 175
   Ile Leu Gly Gly Leu His Leu Thr Leu Asp Trp Ala Val Arg Gly Leu
               180                 185                 190
   Leu Leu Leu Lys Thr Arg Leu
           195
<210> 5
   <211> 467
   <212> PRT
   <213> Homo sapiens
<400> 5
<210> 6
   <211> 160
   <212> PRT
   <213> Homo sapiens
<400> 6
   Met Val Pro Ser Ala Gly Gln Leu Ala Leu Phe Ala Leu Gly Ile Val
   1 5 10 15
   Leu Ala Ala Cys Gln Ala Leu Glu Asn Ser Thr Ser Pro Leu Ser Ala
   20 25 30
   Asp Pro Pro Val Ala Ala Ala Val Val Ser His Phe Asn Asp Cys Pro
   35 40 45
   Asp Ser His Thr Gln Phe Cys Phe His Gly Thr Cys Arg Phe Leu Val
   50 55 60
   Gln Glu Asp Lys Pro Ala Cys Val Cys His Ser Gly Tyr Val Gly Ala
   65 70 75 80
   Arg Cys Glu His Ala Asp Leu Leu Ala Val Val Ala Ala Ser Gln Lys
   85 90 95
   Lys Gln Ala Ile Thr Ala Leu Val Val Val Ser Ile Val Ala Leu Ala
   100 105 110
   Val Leu Ile Ile Thr Cys Val Leu Ile His Cys Cys Gln Val Arg Lys
   115 120 125
   His Cys Glu Trp Cys Arg Ala Leu Ile Cys Arg His Glu Lys Pro Ser
   130 135 140
   Ala Leu Leu Lys Gly Arg Thr Ala Cys Cys His Ser Glu Thr Val Val
   145 150 155 160
<210> 7
   <211> 22152
   <212> PRT
   <213> Homo sapiens
<220>
   <221> MOD_RES
   <222> (13877)..(13878)
   <223> Any amino acid
<220>
   <221> MOD_RES
   <222> (13880)..(13880)
   <223> Any amino acid
<220>
   <221> MOD_RES
   <222> (13887)..(13887)
   <223> Any amino acid
<220>
   <221> MOD_RES
   <222> (13890)..(13891)
   <223> Any amino acid
<220>
   <221> MOD_RES
   <222> (13893)..(13893)
   <223> Any amino acid
<220>
   <221> MOD_RES
   <222> (13903)..(13903)
   <223> Any amino acid
<220>
   <221> MOD_RES
   <222> (13913)..(13914)
   <223> Any amino acid
<220>
   <221> MOD_RES
   <222> (13916)..(13916)
   <223> Any amino acid
<220>
   <221> MOD_RES
   <222> (13928)..(13929)
   <223> Any amino acid
<220>
   <221> MOD_RES
   <222> (13938)..(13938)
   <223> Any amino acid
<220>
   <221> MOD_RES
   <222> (13940)..(13941)
   <223> Any amino acid
<220>
   <221> MOD_RES
   <222> (14569)..(14571)
   <223> Any amino acid
<220>
   <221> MOD_RES
   <222> (14575)..(14575)
   <223> Any amino acid
<220>
   <221> MOD_RES
   <222> (14579)..(14579)
   <223> Any amino acid
<220>
   <221> MOD_RES
   <222> (14581)..(14581)
   <223> Any amino acid
<220>
   <221> MOD_RES
   <222> (14587)..(14591)
   <223> Any amino acid
<220>
   <221> MOD_RES
   <222> (14593)..(14594)
   <223> Any amino acid
<220>
   <221> MOD_RES
   <222> (14725)..(14727)
   <223> Any amino acid
<220>
   <221> MOD_RES
   <222> (14731)..(14731)
   <223> Any amino acid
<220>
   <221> MOD_RES
   <222> (14735)..(14735)
   <223> Any amino acid
<220>
   <221> MOD_RES
   <222> (14737)..(14737)
   <223> Any amino acid
<220>
   <221> MOD_RES
   <222> (14743)..(14747)
   <223> Any amino acid
<220>
   <221> MOD_RES
   <222> (14749)..(14750)
   <223> Any amino acid
<220>
   <221> MOD_RES
   <222> (15661)..(15663)
   <223> Any amino acid
<220>
   <221> MOD_RES
   <222> (15667)..(15667)
   <223> Any amino acid
<220>
   <221> MOD_RES
   <222> (15671)..(15671)
   <223> Any amino acid
<220>
   <221> MOD_RES
   <222> (15673)..(15673)
   <223> Any amino acid
<220>
   <221> MOD_RES
   <222> (15679)..(15683)
   <223> Any amino acid
<220>
   <221> MOD_RES
   <222> (15685)..(15686)
   <223> Any amino acid
<220>
   <221> MOD_RES
   <222> (15972)..(15974)
   <223> Any amino acid
<220>
   <221> MOD_RES
   <222> (15978)..(15978)
   <223> Any amino acid
<220>
   <221> MOD_RES
   <222> (15982)..(15982)
   <223> Any amino acid
<220>
   <221> MOD_RES
   <222> (15984)..(15984)
   <223> Any amino acid
<220>
   <221> MOD_RES
   <222> (15990)..(15994)
   <223> Any amino acid
<220>
   <221> MOD_RES
   <222> (15996)..(15997)
   <223> Any amino acid
<220>
   <221> MOD_RES
   <222> (16008)..(16008)
   <223> Any amino acid
<220>
   <221> MOD_RES
   <222> (16015)..(16015)
   <223> Any amino acid
<220>
   <221> MOD_RES
   <222> (16017)..(16017)
   <223> Any amino acid
<220>
   <221> MOD_RES
   <222> (16021)..(16021)
   <223> Any amino acid
<220>
   <221> MOD_RES
   <222> (16025)..(16025)
   <223> Any amino acid
<220>
   <221> MOD_RES
   <222> (16034)..(16034)
   <223> Any amino acid
<220>
   <221> MOD_RES
   <222> (16037)..(16037)
   <223> Any amino acid
<220>
   <221> MOD_RES
   <222> (16040)..(16040)
   <223> Any amino acid
<220>
   <221> MOD_RES
   <222> (16046)..(16046)
   <223> Any amino acid
<220>
   <221> MOD_RES
   <222> (16051)..(16051)
   <223> Any amino acid
<220>
   <221> MOD_RES
   <222> (16053)..(16055)
   <223> Any amino acid
<220>
   <221> MOD_RES
   <222> (16058)..(16058)
   <223> Any amino acid
<220>
   <221> MOD_RES
   <222> (16062)..(16063)
   <223> Any amino acid
<220>
   <221> MOD_RES
   <222> (16065)..(16065)
   <223> Any amino acid
<220>
   <221> MOD_RES
   <222> (16072)..(16072)
   <223> Any amino acid
<220>
   <221> MOD_RES
   <222> (16075)..(16076)
   <223> Any amino acid
<220>
   <221> MOD_RES
   <222> (16078)..(16078)
   <223> Any amino acid
<220>
   <221> MOD_RES
   <222> (16088)..(16088)
   <223> Any amino acid
<220>
   <221> MOD_RES
   <222> (16268)..(16269)
   <223> Any amino acid
<220>
   <221> MOD_RES
   <222> (16278)..(16278)
   <223> Any amino acid
<220>
   <221> MOD_RES
   <222> (16280)..(16281)
   <223> Any amino acid
<220>
   <221> MOD_RES
   <222> (16373)..(16374)
   <223> Any amino acid
<220>
   <221> MOD_RES
   <222> (16376)..(16376)
   <223> Any amino acid
<220>
   <221> MOD_RES
   <222> (16383)..(16383)
   <223> Any amino acid
<220>
   <221> MOD_RES
   <222> (16386)..(16387)
   <223> Any amino acid
<220>
   <221> MOD_RES
   <222> (16389)..(16389)
   <223> Any amino acid
<220>
   <221> MOD_RES
   <222> (16399)..(16399)
   <223> Any amino acid
<220>
   <221> MOD_RES
   <222> (16409)..(16410)
   <223> Any amino acid
<220>
   <221> MOD_RES
   <222> (16412)..(16412)
   <223> Any amino acid
<220>
   <221> MOD_RES
   <222> (16424)..(16425)
   <223> Any amino acid
<220>
   <221> MOD_RES
   <222> (16434)..(16434)
   <223> Any amino acid
<220>
   <221> MOD_RES
   <222> (16436)..(16437)
   <223> Any amino acid
<220>
   <221> MOD_RES
   <222> (16439)..(16441)
   <223> Any amino acid
<220>
   <221> MOD_RES
   <222> (16445)..(16445)
   <223> Any amino acid
<220>
   <221> MOD_RES
   <222> (16449)..(16449)
   <223> Any amino acid
<220>
   <221> MOD_RES
   <222> (16451)..(16451)
   <223> Any amino acid
<220>
   <221> MOD_RES
   <222> (16457)..(16461)
   <223> Any amino acid
<220>
   <221> MOD_RES
   <222> (16463)..(16464)
   <223> Any amino acid
<220>
   <221> MOD_RES
   <222> (16841)..(16842)
   <223> Any amino acid
<220>
   <221> MOD_RES
   <222> (16844)..(16844)
   <223> Any amino acid
<220>
   <221> MOD_RES
   <222> (16851)..(16851)
   <223> Any amino acid
<220>
   <221> MOD_RES
   <222> (16854)..(16855)
   <223> Any amino acid
<220>
   <221> MOD_RES
   <222> (16857)..(16857)
   <223> Any amino acid
<220>
   <221> MOD_RES
   <222> (16867)..(16867)
   <223> Any amino acid
<220>
   <221> MOD_RES
   <222> (16877)..(16878)
   <223> Any amino acid
<220>
   <221> MOD_RES
   <222> (16880)..(16880)
   <223> Any amino acid
<220>
   <221> MOD_RES
   <222> (16892)..(16893)
   <223> Any amino acid
<220>
   <221> MOD_RES
   <222> (16902)..(16902)
   <223> Any amino acid
<220>
   <221> MOD_RES
   <222> (16904)..(16905)
   <223> Any amino acid
<220>
   <221> MOD_RES
   <222> (16907)..(16909)
   <223> Any amino acid
<220>
   <221> MOD_RES
   <222> (16913)..(16913)
   <223> Any amino acid
<220>
   <221> MOD_RES
   <222> (16917)..(16917)
   <223> Any amino acid
<220>
   <221> MOD_RES
   <222> (16919)..(16919)
   <223> Any amino acid
<220>
   <221> MOD_RES
   <222> (16925)..(16929)
   <223> Any amino acid
<220>
   <221> MOD_RES
   <222> (16931)..(16932)
   <223> Any amino acid
<220>
   <221> MOD_RES
   <222> (17465)..(17466)
   <223> Any amino acid
<220>
   <221> MOD_RES
   <222> (17468)..(17468)
   <223> Any amino acid
<220>
   <221> MOD_RES
   <222> (17475)..(17475)
   <223> Any amino acid
<220>
   <221> MOD_RES
   <222> (17478)..(17479)
   <223> Any amino acid
<220>
   <221> MOD_RES
   <222> (17481)..(17481)
   <223> Any amino acid
<220>
   <221> MOD_RES
   <222> (17491)..(17491)
   <223> Any amino acid
<220>
   <221> MOD_RES
   <222> (17501)..(17502)
   <223> Any amino acid
<220>
   <221> MOD_RES
   <222> (17504)..(17504)
   <223> Any amino acid
<220>
   <221> MOD_RES
   <222> (17516)..(17517)
   <223> Any amino acid
<220>
   <221> MOD_RES
   <222> (17526)..(17526)
   <223> Any amino acid
<220>
   <221> MOD_RES
   <222> (17528)..(17529)
   <223> Any amino acid
<220>
   <221> MOD_RES
   <222> (17531)..(17533)
   <223> Any amino acid
<220>
   <221> MOD_RES
   <222> (17537)..(17537)
   <223> Any amino acid
<220>
   <221> MOD_RES
   <222> (17541)..(17541)
   <223> Any amino acid
<220>
   <221> MOD_RES
   <222> (17543)..(17543)
   <223> Any amino acid
<220>
   <221> MOD_RES
   <222> (17549)..(17553)
   <223> Any amino acid
<220>
   <221> MOD_RES
   <222> (17555)..(17556)
   <223> Any amino acid
<220>
   <221> MOD_RES
   <222> (17567)..(17567)
   <223> Any amino acid
<220>
   <221> MOD_RES
   <222> (17574)..(17574)
   <223> Any amino acid
<220>
   <221> MOD_RES
   <222> (17576)..(17576)
   <223> Any amino acid
<220>
   <221> MOD_RES
   <222> (17580)..(17580)
   <223> Any amino acid
<220>
   <221> MOD_RES
   <222> (17584)..(17584)
   <223> Any amino acid
<220>
   <221> MOD_RES
   <222> (17593)..(17593)
   <223> Any amino acid
<220>
   <221> MOD_RES
   <222> (17596)..(17596)
   <223> Any amino acid
<220>
   <221> MOD_RES
   <222> (17599)..(17599)
   <223> Any amino acid
<220>
   <221> MOD_RES
   <222> (17605)..(17605)
   <223> Any amino acid
<220>
   <221> MOD_RES
   <222> (17610)..(17610)
   <223> Any amino acid
<220>
   <221> MOD_RES
   <222> (17612)..(17614)
   <223> Any amino acid
<220>
   <221> MOD_RES
   <222> (17617)..(17617)
   <223> Any amino acid
<220>
   <221> MOD_RES
   <222> (17621)..(17622)
   <223> Any amino acid
<220>
   <221> MOD_RES
   <222> (17624)..(17624)
   <223> Any amino acid
<220>
   <221> MOD_RES
   <222> (17631)..(17631)
   <223> Any amino acid
<220>
   <221> MOD_RES
   <222> (17777)..(17778)
   <223> Any amino acid
<220>
   <221> MOD_RES
   <222> (17780)..(17780)
   <223> Any amino acid
<220>
   <221> MOD_RES
   <222> (17787)..(17787)
   <223> Any amino acid
<220>
   <221> MOD_RES
   <222> (17790)..(17791)
   <223> Any amino acid
<220>
   <221> MOD_RES
   <222> (17793)..(17793)
   <223> Any amino acid
<220>
   <221> MOD_RES
   <222> (17803)..(17803)
   <223> Any amino acid
<220>
   <221> MOD_RES
   <222> (17813)..(17814)
   <223> Any amino acid
<220>
   <221> MOD_RES
   <222> (17816)..(17816)
   <223> Any amino acid
<220>
   <221> MOD_RES
   <222> (17828)..(17829)
   <223> Any amino acid
<220>
   <221> MOD_RES
   <222> (17838)..(17838)
   <223> Any amino acid
<220>
   <221> MOD_RES
   <222> (17840)..(17841)
   <223> Any amino acid
<220>
   <221> MOD_RES
   <222> (17843)..(17845)
   <223> Any amino acid
<220>
   <221> MOD_RES
   <222> (17849)..(17849)
   <223> Any amino acid
<220>
   <221> MOD_RES
   <222> (17853)..(17853)
   <223> Any amino acid
<220>
   <221> MOD_RES
   <222> (17855)..(17855)
   <223> Any amino acid
<220>
   <221> MOD_RES
   <222> (17861)..(17865)
   <223> Any amino acid
<220>
   <221> MOD_RES
   <222> (17867)..(17868)
   <223> Any amino acid
<220>
   <221> MOD_RES
   <222> (17879)..(17879)
   <223> Any amino acid
<220>
   <221> MOD_RES
   <222> (17886)..(17886)
   <223> Any amino acid
<220>
   <221> MOD_RES
   <222> (17888)..(17888)
   <223> Any amino acid
<220>
   <221> MOD_RES
   <222> (17892)..(17892)
   <223> Any amino acid
<220>
   <221> MOD_RES
   <222> (17896)..(17896)
   <223> Any amino acid
<220>
   <221> MOD_RES
   <222> (17905)..(17905)
   <223> Any amino acid
<220>
   <221> MOD_RES
   <222> (17908)..(17908)
   <223> Any amino acid
<220>
   <221> MOD_RES
   <222> (17911)..(17911)
   <223> Any amino acid
<220>
   <221> MOD_RES
   <222> (17917)..(17917)
   <223> Any amino acid
<220>
   <221> MOD_RES
   <222> (17922)..(17922)
   <223> Any amino acid
<220>
   <221> MOD_RES
   <222> (17924)..(17926)
   <223> Any amino acid
<220>
   <221> MOD_RES
   <222> (17929)..(17929)
   <223> Any amino acid
<220>
   <221> MOD_RES
   <222> (17933)..(17934)
   <223> Any amino acid
<220>
   <221> MOD_RES
   <222> (17936)..(17936)
   <223> Any amino acid
<220>
   <221> MOD_RES
   <222> (17943)..(17943)
   <223> Any amino acid
<220>
   <221> MOD_RES
   <222> (17946)..(17947)
   <223> Any amino acid
<220>
   <221> MOD_RES
   <222> (17949)..(17949)
   <223> Any amino acid
<220>
   <221> MOD_RES
   <222> (17959)..(17959)
   <223> Any amino acid
<220>
   <221> MOD_RES
   <222> (18089)..(18090)
   <223> Any amino acid
<220>
   <221> MOD_RES
   <222> (18092)..(18092)
   <223> Any amino acid
<220>
   <221> MOD_RES
   <222> (18099)..(18099)
   <223> Any amino acid
<220>
   <221> MOD_RES
   <222> (18102)..(18103)
   <223> Any amino acid
<220>
   <221> MOD_RES
   <222> (18105)..(18105)
   <223> Any amino acid
<220>
   <221> MOD_RES
   <222> (18115)..(18115)
   <223> Any amino acid
<220>
   <221> MOD_RES
   <222> (18125)..(18126)
   <223> Any amino acid
<220>
   <221> MOD_RES
   <222> (18128)..(18128)
   <223> Any amino acid
<220>
   <221> MOD_RES
   <222> (18140)..(18141)
   <223> Any amino acid
<220>
   <221> MOD_RES
   <222> (18150)..(18150)
   <223> Any amino acid
<220>
   <221> MOD_RES
   <222> (18152)..(18153)
   <223> Any amino acid
<220>
   <221> MOD_RES
   <222> (18155)..(18157)
   <223> Any amino acid
<220>
   <221> MOD_RES
   <222> (18161)..(18161)
   <223> Any amino acid
<220>
   <221> MOD_RES
   <222> (18165)..(18165)
   <223> Any amino acid
<220>
   <221> MOD_RES
   <222> (18167)..(18167)
   <223> Any amino acid
<220>
   <221> MOD_RES
   <222> (18173)..(18177)
   <223> Any amino acid
<220>
   <221> MOD_RES
   <222> (18179)..(18180)
   <223> Any amino acid
<220>
   <221> MOD_RES
   <222> (18191)..(18191)
   <223> Any amino acid
<220>
   <221> MOD_RES
   <222> (18198)..(18198)
   <223> Any amino acid
<220>
   <221> MOD_RES
   <222> (18200)..(18200)
   <223> Any amino acid
<220>
   <221> MOD_RES
   <222> (18204)..(18204)
   <223> Any amino acid
<220>
   <221> MOD_RES
   <222> (18208)..(18208)
   <223> Any amino acid
<220>
   <221> MOD_RES
   <222> (18217)..(18217)
   <223> Any amino acid
<220>
   <221> MOD_RES
   <222> (18220)..(18220)
   <223> Any amino acid
<220>
   <221> MOD_RES
   <222> (18223)..(18223)
   <223> Any amino acid
<220>
   <221> MOD_RES
   <222> (18229)..(18229)
   <223> Any amino acid
<220>
   <221> MOD_RES
   <222> (18234)..(18234)
   <223> Any amino acid
<220>
   <221> MOD_RES
   <222> (18236)..(18238)
   <223> Any amino acid
<220>
   <221> MOD_RES
   <222> (18241)..(18241)
   <223> Any amino acid
<220>
   <221> MOD_RES
   <222> (18245)..(18246)
   <223> Any amino acid
<220>
   <221> MOD_RES
   <222> (18248)..(18248)
   <223> Any amino acid
<220>
   <221> MOD_RES
   <222> (18255)..(18255)
   <223> Any amino acid
<220>
   <221> MOD_RES
   <222> (18258)..(18259)
   <223> Any amino acid
<220>
   <221> MOD_RES
   <222> (18261)..(18261)
   <223> Any amino acid
<220>
   <221> MOD_RES
   <222> (18271)..(18271)
   <223> Any amino acid
<220>
   <221> MOD_RES
   <222> (18401)..(18402)
   <223> Any amino acid
<220>
   <221> MOD_RES
   <222> (18404)..(18404)
   <223> Any amino acid
<220>
   <221> MOD_RES
   <222> (18411)..(18411)
   <223> Any amino acid
<220>
   <221> MOD_RES
   <222> (18414)..(18415)
   <223> Any amino acid
<220>
   <221> MOD_RES
   <222> (18417)..(18417)
   <223> Any amino acid
<220>
   <221> MOD_RES
   <222> (18427)..(18427)
   <223> Any amino acid
<220>
   <221> MOD_RES
   <222> (18437)..(18438)
   <223> Any amino acid
<220>
   <221> MOD_RES
   <222> (18440)..(18440)
   <223> Any amino acid
<220>
   <221> MOD_RES
   <222> (18452)..(18453)
   <223> Any amino acid
<220>
   <221> MOD_RES
   <222> (18462)..(18462)
   <223> Any amino acid
<220>
   <221> MOD_RES
   <222> (18464)..(18465)
   <223> Any amino acid
<220>
   <221> MOD_RES
   <222> (18467)..(18469)
   <223> Any amino acid
<220>
   <221> MOD_RES
   <222> (18473)..(18473)
   <223> Any amino acid
<220>
   <221> MOD_RES
   <222> (18477)..(18477)
   <223> Any amino acid
<220>
   <221> MOD_RES
   <222> (18479)..(18479)
   <223> Any amino acid
<220>
   <221> MOD_RES
   <222> (18485)..(18489)
   <223> Any amino acid
<220>
   <221> MOD_RES
   <222> (18491)..(18492)
   <223> Any amino acid
<220>
   <221> MOD_RES
   <222> (18503)..(18503)
   <223> Any amino acid
<220>
   <221> MOD_RES
   <222> (18510)..(18510)
   <223> Any amino acid
<220>
   <221> MOD_RES
   <222> (18512)..(18512)
   <223> Any amino acid
<220>
   <221> MOD_RES
   <222> (18516)..(18516)
   <223> Any amino acid
<220>
   <221> MOD_RES
   <222> (18520)..(18520)
   <223> Any amino acid
<220>
   <221> MOD_RES
   <222> (18529)..(18529)
   <223> Any amino acid
<220>
   <221> MOD_RES
   <222> (18532)..(18532)
   <223> Any amino acid
<220>
   <221> MOD_RES
   <222> (18535)..(18535)
   <223> Any amino acid
<220>
   <221> MOD_RES
   <222> (18541)..(18541)
   <223> Any amino acid
<220>
   <221> MOD_RES
   <222> (18546)..(18546)
   <223> Any amino acid
<220>
   <221> MOD_RES
   <222> (18548)..(18550)
   <223> Any amino acid
<220>
   <221> MOD_RES
   <222> (18553)..(18553)
   <223> Any amino acid
<220>
   <221> MOD_RES
   <222> (18557)..(18558)
   <223> Any amino acid
<220>
   <221> MOD_RES
   <222> (18560)..(18560)
   <223> Any amino acid
<220>
   <221> MOD_RES
   <222> (18567)..(18567)
   <223> Any amino acid
<220>
   <221> MOD_RES
   <222> (18570)..(18571)
   <223> Any amino acid
<220>
   <221> MOD_RES
   <222> (18573)..(18573)
   <223> Any amino acid
<220>
   <221> MOD_RES
   <222> (18583)..(18583)
   <223> Any amino acid
<220>
   <221> MOD_RES
   <222> (18713)..(18714)
   <223> Any amino acid
<220>
   <221> MOD_RES
   <222> (18716)..(18716)
   <223> Any amino acid
<220>
   <221> MOD_RES
   <222> (18723)..(18723)
   <223> Any amino acid
<220>
   <221> MOD_RES
   <222> (18726)..(18727)
   <223> Any amino acid
<220>
   <221> MOD_RES
   <222> (18729)..(18729)
   <223> Any amino acid
<220>
   <221> MOD_RES
   <222> (18739)..(18739)
   <223> Any amino acid
<220>
   <221> MOD_RES
   <222> (18749)..(18750)
   <223> Any amino acid
<220>
   <221> MOD_RES
   <222> (18752)..(18752)
   <223> Any amino acid
<220>
   <221> MOD_RES
   <222> (18764)..(18765)
   <223> Any amino acid
<220>
   <221> MOD_RES
   <222> (18774)..(18774)
   <223> Any amino acid
<220>
   <221> MOD_RES
   <222> (18776)..(18777)
   <223> Any amino acid
<220>
   <221> MOD_RES
   <222> (18779)..(18781)
   <223> Any amino acid
<220>
   <221> MOD_RES
   <222> (18785)..(18785)
   <223> Any amino acid
<220>
   <221> MOD_RES
   <222> (18789)..(18789)
   <223> Any amino acid
<220>
   <221> MOD_RES
   <222> (18791)..(18791)
   <223> Any amino acid
<220>
   <221> MOD_RES
   <222> (18797)..(18801)
   <223> Any amino acid
<220>
   <221> MOD_RES
   <222> (18803)..(18804)
   <223> Any amino acid
<220>
   <221> MOD_RES
   <222> (18815)..(18815)
   <223> Any amino acid
<220>
   <221> MOD_RES
   <222> (18822)..(18822)
   <223> Any amino acid
<220>
   <221> MOD_RES
   <222> (18824)..(18824)
   <223> Any amino acid
<220>
   <221> MOD_RES
   <222> (18828)..(18828)
   <223> Any amino acid
<220>
   <221> MOD_RES
   <222> (18832)..(18832)
   <223> Any amino acid
<220>
   <221> MOD_RES
   <222> (18841)..(18841)
   <223> Any amino acid
<220>
   <221> MOD_RES
   <222> (18844)..(18844)
   <223> Any amino acid
<220>
   <221> MOD_RES
   <222> (18847)..(18847)
   <223> Any amino acid
<220>
   <221> MOD_RES
   <222> (18853)..(18853)
   <223> Any amino acid
<220>
   <221> MOD_RES
   <222> (18858)..(18858)
   <223> Any amino acid
<220>
   <221> MOD_RES
   <222> (18860)..(18862)
   <223> Any amino acid
<220>
   <221> MOD_RES
   <222> (18865)..(18865)
   <223> Any amino acid
<220>
   <221> MOD_RES
   <222> (18869)..(18870)
   <223> Any amino acid
<220>
   <221> MOD_RES
   <222> (18872)..(18872)
   <223> Any amino acid
<220>
   <221> MOD_RES
   <222> (18879)..(18879)
   <223> Any amino acid
<220>
   <221> MOD_RES
   <222> (18882)..(18883)
   <223> Any amino acid
<220>
   <221> MOD_RES
   <222> (18885)..(18885)
   <223> Any amino acid
<220>
   <221> MOD_RES
   <222> (18895)..(18895)
   <223> Any amino acid
<220>
   <221> MOD_RES
   <222> (19091)..(19093)
   <223> Any amino acid
<220>
   <221> MOD_RES
   <222> (19097)..(19097)
   <223> Any amino acid
<220>
   <221> MOD_RES
   <222> (19101)..(19101)
   <223> Any amino acid
<220>
   <221> MOD_RES
   <222> (19103)..(19103)
   <223> Any amino acid
<220>
   <221> MOD_RES
   <222> (19109)..(19113)
   <223> Any amino acid
<220>
   <221> MOD_RES
   <222> (19115)..(19116)
   <223> Any amino acid
<220>
   <221> MOD_RES
   <222> (19127)..(19127)
   <223> Any amino acid
<220>
   <221> MOD_RES
   <222> (19134)..(19134)
   <223> Any amino acid
<220>
   <221> MOD_RES
   <222> (19136)..(19136)
   <223> Any amino acid
<220>
   <221> MOD_RES
   <222> (19140)..(19140)
   <223> Any amino acid
<220>
   <221> MOD_RES
   <222> (19144)..(19144)
   <223> Any amino acid
<220>
   <221> MOD_RES
   <222> (19153)..(19153)
   <223> Any amino acid
<220>
   <221> MOD_RES
   <222> (19156)..(19156)
   <223> Any amino acid
<220>
   <221> MOD_RES
   <222> (19159)..(19159)
   <223> Any amino acid
<220>
   <221> MOD_RES
   <222> (19165)..(19165)
   <223> Any amino acid
<220>
   <221> MOD_RES
   <222> (19170)..(19170)
   <223> Any amino acid
<220>
   <221> MOD_RES
   <222> (19172)..(19174)
   <223> Any amino acid
<220>
   <221> MOD_RES
   <222> (19177)..(19177)
   <223> Any amino acid
<220>
   <221> MOD_RES
   <222> (19181)..(19182)
   <223> Any amino acid
<220>
   <221> MOD_RES
   <222> (19184)..(19184)
   <223> Any amino acid
<220>
   <221> MOD_RES
   <222> (19191)..(19191)
   <223> Any amino acid
<220>
   <221> MOD_RES
   <222> (19194)..(19195)
   <223> Any amino acid
<220>
   <221> MOD_RES
   <222> (19197)..(19197)
   <223> Any amino acid
<220>
   <221> MOD_RES
   <222> (19207)..(19207)
   <223> Any amino acid
<220>
   <221> MOD_RES
   <222> (19337)..(19338)
   <223> Any amino acid
<220>
   <221> MOD_RES
   <222> (19340)..(19340)
   <223> Any amino acid
<220>
   <221> MOD_RES
   <222> (19347)..(19347)
   <223> Any amino acid
<220>
   <221> MOD_RES
   <222> (19350)..(19351)
   <223> Any amino acid
<220>
   <221> MOD_RES
   <222> (19353)..(19353)
   <223> Any amino acid
<220>
   <221> MOD_RES
   <222> (19363)..(19363)
   <223> Any amino acid
<220>
   <221> MOD_RES
   <222> (19373)..(19374)
   <223> Any amino acid
<220>
   <221> MOD_RES
   <222> (19376)..(19376)
   <223> Any amino acid
<220>
   <221> MOD_RES
   <222> (19388)..(19389)
   <223> Any amino acid
<220>
   <221> MOD_RES
   <222> (19398)..(19398)
   <223> Any amino acid
<220>
   <221> MOD_RES
   <222> (19400)..(19401)
   <223> Any amino acid
<220>
   <221> MOD_RES
   <222> (19403)..(19405)
   <223> Any amino acid
<220>
   <221> MOD_RES
   <222> (19409)..(19409)
   <223> Any amino acid
<220>
   <221> MOD_RES
   <222> (19413)..(19413)
   <223> Any amino acid
<220>
   <221> MOD_RES
   <222> (19415)..(19415)
   <223> Any amino acid
<220>
   <221> MOD_RES
   <222> (19421)..(19425)
   <223> Any amino acid
<220>
   <221> MOD_RES
   <222> (19427)..(19428)
   <223> Any amino acid
<220>
   <221> MOD_RES
   <222> (19439)..(19439)
   <223> Any amino acid
<220>
   <221> MOD_RES
   <222> (19446)..(19446)
   <223> Any amino acid
<220>
   <221> MOD_RES
   <222> (19448)..(19448)
   <223> Any amino acid
<220>
   <221> MOD_RES
   <222> (19452)..(19452)
   <223> Any amino acid
<220>
   <221> MOD_RES
   <222> (19456)..(19456)
   <223> Any amino acid
<220>
   <221> MOD_RES
   <222> (19465)..(19465)
   <223> Any amino acid
<220>
   <221> MOD_RES
   <222> (19468)..(19468)
   <223> Any amino acid
<220>
   <221> MOD_RES
   <222> (19471)..(19471)
   <223> Any amino acid
<220>
   <221> MOD_RES
   <222> (19477)..(19477)
   <223> Any amino acid
<220>
   <221> MOD_RES
   <222> (19482)..(19482)
   <223> Any amino acid
<220>
   <221> MOD_RES
   <222> (19484)..(19486)
   <223> Any amino acid
<220>
   <221> MOD_RES
   <222> (19489)..(19489)
   <223> Any amino acid
<220>
   <221> MOD_RES
   <222> (19493)..(19494)
   <223> Any amino acid
<220>
   <221> MOD_RES
   <222> (19496)..(19496)
   <223> Any amino acid
<220>
   <221> MOD_RES
   <222> (19503)..(19503)
   <223> Any amino acid
<220>
   <221> MOD_RES
   <222> (19506)..(19507)
   <223> Any amino acid
<220>
   <221> MOD_RES
   <222> (19509)..(19509)
   <223> Any amino acid
<220>
   <221> MOD_RES
   <222> (19519)..(19519)
   <223> Any amino acid
<220>
   <221> MOD_RES
   <222> (19649)..(19650)
   <223> Any amino acid
<220>
   <221> MOD_RES
   <222> (19652)..(19652)
   <223> Any amino acid
<220>
   <221> MOD_RES
   <222> (19659)..(19659)
   <223> Any amino acid
<220>
   <221> MOD_RES
   <222> (19662)..(19663)
   <223> Any amino acid
<220>
   <221> MOD_RES
   <222> (19665)..(19665)
   <223> Any amino acid
<220>
   <221> MOD_RES
   <222> (19675)..(19675)
   <223> Any amino acid
<220>
   <221> MOD_RES
   <222> (19685)..(19686)
   <223> Any amino acid
<220>
   <221> MOD_RES
   <222> (19688)..(19688)
   <223> Any amino acid
<220>
   <221> MOD_RES
   <222> (19700)..(19701)
   <223> Any amino acid
<220>
   <221> MOD_RES
   <222> (19710)..(19710)
   <223> Any amino acid
<220>
   <221> MOD_RES
   <222> (19712)..(19713)
   <223> Any amino acid
<220>
   <221> MOD_RES
   <222> (19715)..(19717)
   <223> Any amino acid
<220>
   <221> MOD_RES
   <222> (19721)..(19721)
   <223> Any amino acid
<220>
   <221> MOD_RES
   <222> (19725)..(19725)
   <223> Any amino acid
<220>
   <221> MOD_RES
   <222> (19727)..(19727)
   <223> Any amino acid
<220>
   <221> MOD_RES
   <222> (19733)..(19737)
   <223> Any amino acid
<220>
   <221> MOD_RES
   <222> (19739)..(19740)
   <223> Any amino acid
<220>
   <221> MOD_RES
   <222> (19751)..(19751)
   <223> Any amino acid
<220>
   <221> MOD_RES
   <222> (19758)..(19758)
   <223> Any amino acid
<220>
   <221> MOD_RES
   <222> (19760)..(19760)
   <223> Any amino acid
<220>
   <221> MOD_RES
   <222> (19764)..(19764)
   <223> Any amino acid
<220>
   <221> MOD_RES
   <222> (19768)..(19768)
   <223> Any amino acid
<220>
   <221> MOD_RES
   <222> (19777)..(19777)
   <223> Any amino acid
<220>
   <221> MOD_RES
   <222> (19780)..(19780)
   <223> Any amino acid
<220>
   <221> MOD_RES
   <222> (19783)..(19783)
   <223> Any amino acid
<220>
   <221> MOD_RES
   <222> (19789)..(19789)
   <223> Any amino acid
<220>
   <221> MOD_RES
   <222> (19794)..(19794)
   <223> Any amino acid
<220>
   <221> MOD_RES
   <222> (19796)..(19798)
   <223> Any amino acid
<220>
   <221> MOD_RES
   <222> (19801)..(19801)
   <223> Any amino acid
<220>
   <221> MOD_RES
   <222> (19805)..(19806)
   <223> Any amino acid
<220>
   <221> MOD_RES
   <222> (19808)..(19808)
   <223> Any amino acid
<220>
   <221> MOD_RES
   <222> (19815)..(19815)
   <223> Any amino acid
<220>
   <221> MOD_RES
   <222> (19818)..(19819)
   <223> Any amino acid
<220>
   <221> MOD_RES
   <222> (19821)..(19821)
   <223> Any amino acid
<220>
   <221> MOD_RES
   <222> (19831)..(19831)
   <223> Any amino acid
<220>
   <221> MOD_RES
   <222> (19960)..(19961)
   <223> Any amino acid
<220>
   <221> MOD_RES
   <222> (19963)..(19963)
   <223> Any amino acid
<220>
   <221> MOD_RES
   <222> (19970)..(19970)
   <223> Any amino acid
<220>
   <221> MOD_RES
   <222> (19973)..(19974)
   <223> Any amino acid
<220>
   <221> MOD_RES
   <222> (19976)..(19976)
   <223> Any amino acid
<220>
   <221> MOD_RES
   <222> (19986)..(19986)
   <223> Any amino acid
<220>
   <221> MOD_RES
   <222> (19996)..(19997)
   <223> Any amino acid
<220>
   <221> MOD_RES
   <222> (19999)..(19999)
   <223> Any amino acid
<220>
   <221> MOD_RES
   <222> (20011)..(20012)
   <223> Any amino acid
<220>
   <221> MOD_RES
   <222> (20021)..(20021)
   <223> Any amino acid
<220>
   <221> MOD_RES
   <222> (20023)..(20024)
   <223> Any amino acid
<220>
   <221> MOD_RES
   <222> (20026)..(20028)
   <223> Any amino acid
<220>
   <221> MOD_RES
   <222> (20032)..(20032)
   <223> Any amino acid
<220>
   <221> MOD_RES
   <222> (20036)..(20036)
   <223> Any amino acid
<220>
   <221> MOD_RES
   <222> (20038)..(20038)
   <223> Any amino acid
<220>
   <221> MOD_RES
   <222> (20044)..(20048)
   <223> Any amino acid
<220>
   <221> MOD_RES
   <222> (20050)..(20051)
   <223> Any amino acid
<220>
   <221> MOD_RES
   <222> (20062)..(20062)
   <223> Any amino acid
<220>
   <221> MOD_RES
   <222> (20069)..(20069)
   <223> Any amino acid
<220>
   <221> MOD_RES
   <222> (20071)..(20071)
   <223> Any amino acid
<220>
   <221> MOD_RES
   <222> (20075)..(20075)
   <223> Any amino acid
<220>
   <221> MOD_RES
   <222> (20079)..(20079)
   <223> Any amino acid
<220>
   <221> MOD_RES
   <222> (20088)..(20088)
   <223> Any amino acid
<220>
   <221> MOD_RES
   <222> (20091)..(20091)
   <223> Any amino acid
<220>
   <221> MOD_RES
   <222> (20094)..(20094)
   <223> Any amino acid
<220>
   <221> MOD_RES
   <222> (20100)..(20100)
   <223> Any amino acid
<220>
   <221> MOD_RES
   <222> (20105)..(20105)
   <223> Any amino acid
<220>
   <221> MOD_RES
   <222> (20107)..(20109)
   <223> Any amino acid
<220>
   <221> MOD_RES
   <222> (20112)..(20112)
   <223> Any amino acid
<220>
   <221> MOD_RES
   <222> (20116)..(20117)
   <223> Any amino acid
<220>
   <221> MOD_RES
   <222> (20119)..(20119)
   <223> Any amino acid
<220>
   <221> MOD_RES
   <222> (20126)..(20126)
   <223> Any amino acid
<220>
   <221> MOD_RES
   <222> (20129)..(20130)
   <223> Any amino acid
<220>
   <221> MOD_RES
   <222> (20132)..(20132)
   <223> Any amino acid
<220>
   <221> MOD_RES
   <222> (20142)..(20142)
   <223> Any amino acid
<220>
   <221> MOD_RES
   <222> (20272)..(20273)
   <223> Any amino acid
<220>
   <221> MOD_RES
   <222> (20275)..(20275)
   <223> Any amino acid
<220>
   <221> MOD_RES
   <222> (20282)..(20282)
   <223> Any amino acid
<220>
   <221> MOD_RES
   <222> (20285)..(20286)
   <223> Any amino acid
<220>
   <221> MOD_RES
   <222> (20288)..(20288)
   <223> Any amino acid
<220>
   <221> MOD_RES
   <222> (20298)..(20298)
   <223> Any amino acid
<220>
   <221> MOD_RES
   <222> (20308)..(20309)
   <223> Any amino acid
<220>
   <221> MOD_RES
   <222> (20311)..(20311)
   <223> Any amino acid
<220>
   <221> MOD_RES
   <222> (20323)..(20324)
   <223> Any amino acid
<220>
   <221> MOD_RES
   <222> (20333)..(20333)
   <223> Any amino acid
<220>
   <221> MOD_RES
   <222> (20335)..(20336)
   <223> Any amino acid
<220>
   <221> MOD_RES
   <222> (20806)..(20808)
   <223> Any amino acid
<220>
   <221> MOD_RES
   <222> (20812)..(20812)
   <223> Any amino acid
<220>
   <221> MOD_RES
   <222> (20816)..(20816)
   <223> Any amino acid
<220>
   <221> MOD_RES
   <222> (20818)..(20818)
   <223> Any amino acid
<220>
   <221> MOD_RES
   <222> (20824)..(20828)
   <223> Any amino acid
<220>
   <221> MOD_RES
   <222> (20830)..(20831)
   <223> Any amino acid
<400> 7
   Met Leu Lys Pro Ser Gly Leu Pro Gly Ser Ser Ser Pro Thr Arg Ser
   1               5                   10                  15
   Leu Met Thr Gly Ser Arg Ser Thr Lys Ala Thr Pro Glu Met Asp Ser
               20                  25                  30
   Gly Leu Thr Gly Ala Thr Leu Ser Pro Lys Thr Ser Thr Gly Ala Ile
           35                  40                  45
   Val Val Thr Glu His Thr Leu Pro Phe Thr Ser Pro Asp Lys Thr Leu
       50                  55                  60
   Ala Ser Pro Thr Ser Ser Val Val Gly Arg Thr Thr Gln Ser Leu Gly
   65                  70                  75                  80
   Val Met Ser Ser Ala Leu Pro Glu Ser Thr Ser Arg Gly Met Thr His
                   85                  90                  95
   Ser Glu Gln Arg Thr Ser Pro Ser Leu Ser Pro Gln Val Asn Gly Thr
               100                 105                 110
   Pro Ser Arg Asn Tyr Pro Ala Thr Ser Met Val Ser Gly Leu Ser Ser
           115                 120                 125
   Pro Arg Thr Arg Thr Ser Ser Thr Glu Gly Asn Phe Thr Lys Glu Ala
       130                 135                 140
   Ser Thr Tyr Thr Leu Thr Val Glu Thr Thr Ser Gly Pro Val Thr Glu
   145                 150                 155                 160
   Lys Tyr Thr Val Pro Thr Glu Thr Ser Thr Thr Glu Gly Asp Ser Thr
                   165                 170                 175
   Glu Thr Pro Trp Asp Thr Arg Tyr Ile Pro Val Lys Ile Thr Ser Pro
               180                 185                 190
   Met Lys Thr Phe Ala Asp Ser Thr Ala Ser Lys Glu Asn Ala Pro Val
           195                 200                 205
   Ser Met Thr Pro Ala Glu Thr Thr Val Thr Asp Ser His Thr Pro Gly
       210                 215                 220
   Arg Thr Asn Pro Ser Phe Gly Thr Leu Tyr Ser Ser Phe Leu Asp Leu
   225                 230                 235                 240
   Ser Pro Lys Gly Thr Pro Asn Ser Arg Gly Glu Thr Ser Leu Glu Leu
                   245                 250                 255
   Ile Leu Ser Thr Thr Gly Tyr Pro Phe Ser Ser Pro Glu Pro Gly Ser
               260                 265                 270
   Ala Gly His Ser Arg Ile Ser Thr Ser Ala Pro Leu Ser Ser Ser Ala
           275                 280                 285
   Ser Val Leu Asp Asn Lys Ile Ser Glu Thr Ser Ile Phe Ser Gly Gln
       290                 295                 300
   Ser Leu Thr Ser Pro Leu Ser Pro Gly Val Pro Glu Ala Arg Ala Ser
   305                 310                 315                 320
   Thr Met Pro Asn Ser Ala Ile Pro Phe Ser Met Thr Leu Ser Asn Ala
                   325                 330                 335
   Glu Thr Ser Ala Glu Arg Val Arg Ser Thr Ile Ser Ser Leu Gly Thr
               340                 345                 350
   Pro Ser Ile Ser Thr Lys Gln Thr Ala Glu Thr Ile Leu Thr Phe His
   355 360 365
   Ala Phe Ala Glu Thr Met Asp Ile Pro Ser Thr His Ile Ala Lys Thr
   370 375 380
   Leu Ala Ser Glu Trp Leu Gly Ser Pro Gly Thr Leu Gly Gly Thr Ser
   385 390 395 400
   Thr Ser Ala Leu Thr Thr Thr Ser Pro Ser Thr Thr Leu Val Ser Glu
   405 410 415
   Glu Thr Asn Thr His His Ser Thr Ser Gly Lys Glu Thr Glu Gly Thr
   420 425 430
   Leu Asn Thr Ser Met Thr Pro Leu Glu Thr Ser Ala Pro Gly Glu Glu
   435 440 445
   Ser Glu Met Thr Ala Thr Leu Val Pro Thr Leu Gly Phe Thr Thr Leu
   450 455 460
   Asp Ser Lys Ile Arg Ser Pro Ser Gln Val Ser Ser Ser His Pro Thr
   465 470 475 480
   Arg Glu Leu Arg Thr Thr Gly Ser Thr Ser Gly Arg Gln Ser Ser Ser
   485 490 495
   Thr Ala Ala His Gly Ser Ser Asp Ile Leu Arg Ala Thr Thr Ser Ser
   500 505 510
   Thr Ser Lys Ala Ser Ser Trp Thr Ser Glu Ser Thr Ala Gln Gln Phe
   515 520 525
   Ser Glu Pro Gln His Thr Gln Trp Val Glu Thr Ser Pro Ser Met Lys
   530 535 540
   Thr Glu Arg Pro Pro Ala Ser Thr Ser Val Ala Ala Pro Ile Thr Thr
   545 550 555 560
   Ser Val Pro Ser Val Val Ser Gly Phe Thr Thr Leu Lys Thr Ser Ser
   565 570 575
   Thr Lys Gly Ile Trp Leu Glu Glu Thr Ser Ala Asp Thr Leu Ile Gly
   580 585 590
   Glu Ser Thr Ala Gly Pro Thr Thr His Gln Phe Ala Val Pro Thr Gly
   595 600 605
   Ile Ser Met Thr Gly Gly Ser Ser Thr Arg Gly Ser Gln Gly Thr Thr
       610                 615                 620
   His Leu Leu Thr Arg Ala Thr Ala Ser Ser Glu Thr Ser Ala Asp Leu
   625                 630                 635                 640
   Thr Leu Ala Thr Asn Gly Val Pro Val Ser Val Ser Pro Ala Val Ser
                   645                 650                 655
   Lys Thr Ala Ala Gly Ser Ser Pro Pro Gly Gly Thr Lys Pro Ser Tyr
               660                 665                 670
   Thr Met Val Ser Ser Val Ile Pro Glu Thr Ser Ser Leu Gln Ser Ser
           675                 680                 685
   Ala Phe Arg Glu Gly Thr Ser Leu Gly Leu Thr Pro Leu Asn Thr Arg
       690                 695                 700
   His Pro Phe Ser Ser Pro Glu Pro Asp Ser Ala Gly His Thr Lys Ile
   705                 710                 715                 720
   Ser Thr Ser Ile Pro Leu Leu Ser Ser Ala Ser Val Leu Glu Asp Lys
                   725                 730                 735
   Val Ser Ala Thr Ser Thr Phe Ser His His Lys Ala Thr Ser Ser Ile
               740                 745                 750
   Thr Thr Gly Thr Pro Glu Ile Ser Thr Lys Thr Lys Pro Ser Ser Ala
           755                 760                 765
   Val Leu Ser Ser Met Thr Leu Ser Asn Ala Ala Thr Ser Pro Glu Arg
       770                 775                 780
   Val Arg Asn Ala Thr Ser Pro Leu Thr His Pro Ser Pro Ser Gly Glu
   785                 790                 795                 800
   Glu Thr Ala Gly Ser Val Leu Thr Leu Ser Thr Ser Ala Glu Thr Thr
                   805                 810                 815
   Asp Ser Pro Asn Ile His Pro Thr Gly Thr Leu Thr Ser Glu Ser Ser
               820                 825                 830
   Glu Ser Pro Ser Thr Leu Ser Leu Pro Ser Val Ser Gly Val Lys Thr
           835                 840                 845
   Thr Phe Ser Ser Ser Thr Pro Ser Thr His Leu Phe Thr Ser Gly Glu
       850                 855                 860
   Glu Thr Glu Glu Thr Ser Asn Pro Ser Val Ser Gln Pro Glu Thr Ser
   865                 870                 875                 880
   Val Ser Arg Val Arg Thr Thr Leu Ala Ser Thr Ser Val Pro Thr Pro
                   885                 890                 895
   Val Phe Pro Thr Met Asp Thr Trp Pro Thr Arg Ser Ala Gln Phe Ser
               900                 905                 910
   Ser Ser His Leu Val Ser Glu Leu Arg Ala Thr Ser Ser Thr Ser Val
           915                 920                 925
   Thr Asn Ser Thr Gly Ser Ala Leu Pro Lys Ile Ser His Leu Thr Gly
       930                 935                 940
   Thr Ala Thr Met Ser Gln Thr Asn Arg Asp Thr Phe Asn Asp Ser Ala
   945                 950                 955                 960
   Ala Pro Gln Ser Thr Thr Trp Pro Glu Thr Ser Pro Arg Phe Lys Thr
                   965                 970                 975
   Gly Leu Pro Ser Ala Thr Thr Thr Val Ser Thr Ser Ala Thr Ser Leu
               980                 985                 990
   Ser Ala Thr Val Met Val Ser Lys  Phe Thr Ser Pro Ala  Thr Ser Ser
           995                 1000                 1005
   Met Glu  Ala Thr Ser Ile Arg  Glu Pro Ser Thr Thr  Ile Leu Thr
       1010                 1015                 1020
   Thr Glu  Thr Thr Asn Gly Pro  Gly Ser Met Ala Val  Ala Ser Thr
       1025                 1030                 1035
   Asn Ile  Pro Ile Gly Lys Gly  Tyr Ile Thr Glu Gly  Arg Leu Asp
       1040                 1045                 1050
   Thr Ser  His Leu Pro Ile Gly  Thr Thr Ala Ser Ser  Glu Thr Ser
       1055                 1060                 1065
   Met Asp  Phe Thr Met Ala Lys  Glu Ser Val Ser Met  Ser Val Ser
       1070                 1075                 1080
   Pro Ser  Gln Ser Met Asp Ala  Ala Gly Ser Ser Thr  Pro Gly Arg
       1085                 1090                 1095
   Thr Ser  Gln Phe Val Asp Thr  Phe Ser Asp Asp Val  Tyr His Leu
   1100 1105 1110
   Thr Ser Arg Glu Ile Thr Ile Pro Arg Asp Gly Thr Ser Ser Ala
   1115 1120 1125
   Leu Thr Pro Gln Met Thr Ala Thr His Pro Pro Ser Pro Asp Pro
   1130 1135 1140
   Gly Ser Ala Arg Ser Thr Trp Leu Gly Ile Leu Ser Ser Ser Pro
   1145 1150 1155
   Ser Ser Pro Thr Pro Lys Val Thr Met Ser Ser Thr Phe Ser Thr
   1160 1165 1170
   Gln Arg Val Thr Thr Ser Met Ile Met Asp Thr Val Glu Thr Ser
   1175 1180 1185
   Arg Trp Asn Met Pro Asn Leu Pro Ser Thr Thr Ser Leu Thr Pro
   1190 1195 1200
   Ser Asn Ile Pro Thr Ser Gly Ala Ile Gly Lys Ser Thr Leu Val
   1205 1210 1215
   Pro Leu Asp Thr Pro Ser Pro Ala Thr Ser Leu Glu Ala Ser Glu
   1220 1225 1230
   Gly Gly Leu Pro Thr Leu Ser Thr Tyr Pro Glu Ser Thr Asn Thr
   1235 1240 1245
   Pro Ser Ile His Leu Gly Ala His Ala Ser Ser Glu Ser Pro Ser
   1250 1255 1260
   Thr Ile Lys Leu Thr Met Ala Ser Val Val Lys Pro Gly Ser Tyr
   1265 1270 1275
   Thr Pro Leu Thr Phe Pro Ser Ile Glu Thr His Ile His Val Ser
   1280 1285 1290
   Thr Ala Arg Met Ala Tyr Ser Ser Gly Ser Ser Pro Glu Met Thr
   1295 1300 1305
   Ala Pro Gly Glu Thr Asn Thr Gly Ser Thr Trp Asp Pro Thr Thr
   1310 1315 1320
   Tyr Ile Thr Thr Thr Asp Pro Lys Asp Thr Ser Ser Ala Gln Val
   1325 1330 1335
   Ser Thr Pro His Ser Val Arg Thr Leu Arg Thr Thr Glu Asn His
   1340 1345 1350
   Pro Lys Thr Glu Ser Ala Thr Pro Ala Ala Tyr Ser Gly Ser Pro
   1355 1360 1365
   Lys Ile Ser Ser Ser Pro Asn Leu Thr Ser Pro Ala Thr Lys Ala
   1370 1375 1380
   Trp Thr Ile Thr Asp Thr Thr Glu His Ser Thr Gln Leu His Tyr
   1385 1390 1395
   Thr Lys Leu Ala Glu Lys Ser Ser Gly Phe Glu Thr Gln Ser Ala
   1400 1405 1410
   Pro Gly Pro Val Ser Val Val Ile Pro Thr Ser Pro Thr Ile Gly
   1415 1420 1425
   Ser Ser Thr Leu Glu Leu Thr Ser Asp Val Pro Gly Glu Pro Leu
   1430 1435 1440
   Val Leu Ala Pro Ser Glu Gln Thr Thr Ile Thr Leu Pro Met Ala
   1445 1450 1455
   Thr Trp Leu Ser Thr Ser Leu Thr Glu Glu Met Ala Ser Thr Asp
   1460 1465 1470
   Leu Asp Ile Ser Ser Pro Ser Ser Pro Met Ser Thr Phe Ala Ile
   1475 1480 1485
   Phe Pro Pro Met Ser Thr Pro Ser His Glu Leu Ser Lys Ser Glu
   1490 1495 1500
   Ala Asp Thr Ser Ala Ile Arg Asn Thr Asp Ser Thr Thr Leu Asp
   1505 1510 1515
   Gln His Leu Gly Ile Arg Ser Leu Gly Arg Thr Gly Asp Leu Thr
   1520 1525 1530
   Thr Val Pro Ile Thr Pro Leu Thr Thr Thr Trp Thr Ser Val Ile
   1535 1540 1545
   Glu His Ser Thr Gln Ala Gln Asp Thr Leu Ser Ala Thr Met Ser
   1550 1555 1560
   Pro Thr His Val Thr Gln Ser Leu Lys Asp Gln Thr Ser Ile Pro
   1565 1570 1575
   Ala Ser Ala Ser Pro Ser His Leu Thr Glu Val Tyr Pro Glu Leu
   1580 1585 1590
   Gly Thr Gln Gly Arg Ser Ser Ser Glu Ala Thr Thr Phe Trp Lys
   1595 1600 1605
   Pro Ser Thr Asp Thr Leu Ser Arg Glu Ile Glu Thr Gly Pro Thr
   1610 1615 1620
   Asn Ile Gln Ser Thr Pro Pro Met Asp Asn Thr Thr Thr Gly Ser
   1625 1630 1635
   Ser Ser Ser Gly Val Thr Leu Gly Ile Ala His Leu Pro Ile Gly
   1640 1645 1650
   Thr Ser Ser Pro Ala Glu Thr Ser Thr Asn Met Ala Leu Glu Arg
   1655 1660 1665
   Arg Ser Ser Thr Ala Thr Val Ser Met Ala Gly Thr Met Gly Leu
   1670 1675 1680
   Leu Val Thr Ser Ala Pro Gly Arg Ser Ile Ser Gln Ser Leu Gly
   1685 1690 1695
   Arg Val Ser Ser Val Leu Ser Glu Ser Thr Thr Glu Gly Val Thr
   1700 1705 1710
   Asp Ser Ser Lys Gly Ser Ser Pro Arg Leu Asn Thr Gln Gly Asn
   1715 1720 1725
   Thr Ala Leu Ser Ser Ser Leu Glu Pro Ser Tyr Ala Glu Gly Ser
   1730 1735 1740
   Gln Met Ser Thr Ser Ile Pro Leu Thr Ser Ser Pro Thr Thr Pro
   1745 1750 1755
   Asp Val Glu Phe Ile Gly Gly Ser Thr Phe Trp Thr Lys Glu Val
   1760 1765 1770
   Thr Thr Val Met Thr Ser Asp Ile Ser Lys Ser Ser Ala Arg Thr
   1775 1780 1785
   Glu Ser Ser Ser Ala Thr Leu Met Ser Thr Ala Leu Gly Ser Thr
   1790 1795 1800
   Glu Asn Thr Gly Lys Glu Lys Leu Arg Thr Ala Ser Met Asp Leu
   1805 1810 1815
   Pro Ser Pro Thr Pro Ser Met Glu Val Thr Pro Trp Ile Ser Leu
   1820 1825 1830
   Thr Leu Ser Asn Ala Pro Asn Thr Thr Asp Ser Leu Asp Leu Ser
   1835 1840 1845
   His Gly Val His Thr Ser Ser Ala Gly Thr Leu Ala Thr Asp Arg
   1850 1855 1860
   Ser Leu Asn Thr Gly Val Thr Arg Ala Ser Arg Leu Glu Asn Gly
   1865 1870 1875
   Ser Asp Thr Ser Ser Lys Ser Leu Ser Met Gly Asn Ser Thr His
   1880 1885 1890
   Thr Ser Met Thr Asp Thr Glu Lys Ser Glu Val Ser Ser Ser Ile
   1895 1900 1905
   His Pro Arg Pro Glu Thr Ser Ala Pro Gly Ala Glu Thr Thr Leu
   1910 1915 1920
   Thr Ser Thr Pro Gly Asn Arg Ala Ile Ser Leu Thr Leu Pro Phe
   1925 1930 1935
   Ser Ser Ile Pro Val Glu Glu Val Ile Ser Thr Gly Ile Thr Ser
   1940 1945 1950
   Gly Pro Asp Ile Asn Ser Ala Pro Met Thr His Ser Pro Ile Thr
   1955 1960 1965
   Pro Pro Thr Ile Val Trp Thr Ser Thr Gly Thr Ile Glu Gln Ser
   1970 1975 1980
   Thr Gln Pro Leu His Ala Val Ser Ser Glu Lys Val Ser Val Gln
   1985 1990 1995
   Thr Gln Ser Thr Pro Tyr Val Asn Ser Val Ala Val Ser Ala Ser
   2000 2005 2010
   Pro Thr His Glu Asn Ser Val Ser Ser Gly Ser Ser Thr Ser Ser
   2015 2020 2025
   Pro Tyr Ser Ser Ala Ser Leu Glu Ser Leu Asp Ser Thr Ile Ser
   2030 2035 2040
   Arg Arg Asn Ala Ile Thr Ser Trp Leu Trp Asp Leu Thr Thr Ser
   2045 2050 2055
   Leu Pro Thr Thr Thr Trp Pro Ser Thr Ser Leu Ser Glu Ala Leu
   2060 2065 2070
   Ser Ser Gly His Ser Gly Val Ser Asn Pro Ser Ser Thr Thr Thr
   2075 2080 2085
   Glu Phe Pro Leu Phe Ser Ala Ala Ser Thr Ser Ala Ala Lys Gln
   2090 2095 2100
   Arg Asn Pro Glu Thr Glu Thr His Gly Pro Gln Asn Thr Ala Ala
   2105 2110 2115
   Ser Thr Leu Asn Thr Asp Ala Ser Ser Val Thr Gly Leu Ser Glu
   2120 2125 2130
   Thr Pro Val Gly Ala Ser Ile Ser Ser Glu Val Pro Leu Pro Met
   2135 2140 2145
   Ala Ile Thr Ser Arg Ser Asp Val Ser Gly Leu Thr Ser Glu Ser
   2150 2155 2160
   Thr Ala Asn Pro Ser Leu Gly Thr Ala Ser Ser Ala Gly Thr Lys
   2165 2170 2175
   Leu Thr Arg Thr Ile Ser Leu Pro Thr Ser Glu Ser Leu Val Ser
   2180 2185 2190
   Phe Arg Met Asn Lys Asp Pro Trp Thr Val Ser Ile Pro Leu Gly
   2195 2200 2205
   Ser His Pro Thr Thr Asn Thr Glu Thr Ser Ile Pro Val Asn Ser
   2210 2215 2220
   Ala Gly Pro Pro Gly Leu Ser Thr Val Ala Ser Asp Val Ile Asp
   2225 2230 2235
   Thr Pro Ser Asp Gly Ala Glu Ser Ile Pro Thr Val Ser Phe Ser
   2240 2245 2250
   Pro Ser Pro Asp Thr Glu Val Thr Thr Ile Ser His Phe Pro Glu
   2255 2260 2265
   Lys Thr Thr His Ser Phe Arg Thr Ile Ser Ser Leu Thr His Glu
   2270 2275 2280
   Leu Thr Ser Arg Val Thr Pro Ile Pro Gly Asp Trp Met Ser Ser
   2285 2290 2295
   Ala Met Ser Thr Lys Pro Thr Gly Ala Ser Pro Ser Ile Thr Leu
   2300 2305 2310
   Gly Glu Arg Arg Thr Ile Thr Ser Ala Ala Pro Thr Thr Ser Pro
   2315 2320 2325
   Ile Val Leu Thr Ala Ser Phe Thr Glu Thr Ser Thr Val Ser Leu
   2330 2335 2340
   Asp Asn Glu Thr Thr Val Lys Thr Ser Asp Ile Leu Asp Ala Arg
   2345 2350 2355
   Lys Thr Asn Glu Leu Pro Ser Asp Ser Ser Ser Ser Ser Asp Leu
   2360 2365 2370
   Ile Asn Thr Ser Ile Ala Ser Ser Thr Met Asp Val Thr Lys Thr
   2375 2380 2385
   Ala Ser Ile Ser Pro Thr Ser Ile Ser Gly Met Thr Ala Ser Ser
   2390 2395 2400
   Ser Pro Ser Leu Phe Ser Ser Asp Arg Pro Gln Val Pro Thr Ser
   2405 2410 2415
   Thr Thr Glu Thr Asn Thr Ala Thr Ser Pro Ser Val Ser Ser Asn
   2420 2425 2430
   Thr Tyr Ser Leu Asp Gly Gly Ser Asn Val Gly Gly Thr Pro Ser
   2435 2440 2445
   Thr Leu Pro Pro Phe Thr Ile Thr His Pro Val Glu Thr Ser Ser
   2450 2455 2460
   Ala Leu Leu Ala Trp Ser Arg Pro Val Arg Thr Phe Ser Thr Met
   2465 2470 2475
   Val Ser Thr Asp Thr Ala Ser Gly Glu Asn Pro Thr Ser Ser Asn
   2480 2485 2490
   Ser Val Val Thr Ser Val Pro Ala Pro Gly Thr Trp Ala Ser Val
   2495 2500 2505
   Gly Ser Thr Thr Asp Leu Pro Ala Met Gly Phe Leu Lys Thr Ser
   2510 2515 2520
   Pro Ala Gly Glu Ala His Ser Leu Leu Ala Ser Thr Ile Glu Pro
   2525 2530 2535
   Ala Thr Ala Phe Thr Pro His Leu Ser Ala Ala Val Val Thr Gly
   2540 2545 2550
   Ser Ser Ala Thr Ser Glu Ala Ser Leu Leu Thr Thr Ser Glu Ser
   2555 2560 2565
   Lys Ala Ile His Ser Ser Pro Gln Thr Pro Thr Thr Pro Thr Ser
   2570 2575 2580
   Gly Ala Asn Trp Glu Thr Ser Ala Thr Pro Glu Ser Leu Leu Val
   2585 2590 2595
   Val Thr Glu Thr Ser Asp Thr Thr Leu Thr Ser Lys Ile Leu Val
   2600 2605 2610
   Thr Asp Thr Ile Leu Phe Ser Thr Val Ser Thr Pro Pro Ser Lys
   2615 2620 2625
   Phe Pro Ser Thr Gly Thr Leu Ser Gly Ala Ser Phe Pro Thr Leu
   2630 2635 2640
   Leu Pro Asp Thr Pro Ala Ile Pro Leu Thr Ala Thr Glu Pro Thr
   2645 2650 2655
   Ser Ser Leu Ala Thr Ser Phe Asp Ser Thr Pro Leu Val Thr Ile
   2660 2665 2670
   Ala Ser Asp Ser Leu Gly Thr Val Pro Glu Thr Thr Leu Thr Met
   2675 2680 2685
   Ser Glu Thr Ser Asn Gly Asp Ala Leu Val Leu Lys Thr Val Ser
   2690 2695 2700
   Asn Pro Asp Arg Ser Ile Pro Gly Ile Thr Ile Gln Gly Val Thr
   2705 2710 2715
   Glu Ser Pro Leu His Pro Ser Ser Thr Ser Pro Ser Lys Ile Val
   2720 2725 2730
   Ala Pro Arg Asn Thr Thr Tyr Glu Gly Ser Ile Thr Val Ala Leu
   2735 2740 2745
   Ser Thr Leu Pro Ala Gly Thr Thr Gly Ser Leu Val Phe Ser Gln
   2750 2755 2760
   Ser Ser Glu Asn Ser Glu Thr Thr Ala Leu Val Asp Ser Ser Ala
   2765 2770 2775
   Gly Leu Glu Arg Ala Ser Val Met Pro Leu Thr Thr Gly Ser Gln
   2780 2785 2790
   Gly Met Ala Ser Ser Gly Gly Ile Arg Ser Gly Ser Thr His Ser
   2795 2800 2805
   Thr Gly Thr Lys Thr Phe Ser Ser Leu Pro Leu Thr Met Asn Pro
   2810 2815 2820
   Gly Glu Val Thr Ala Met Ser Glu Ile Thr Thr Asn Arg Leu Thr
   2825 2830 2835
   Ala Thr Gln Ser Thr Ala Pro Lys Gly Ile Pro Val Lys Pro Thr
   2840 2845 2850
   Ser Ala Glu Ser Gly Leu Leu Thr Pro Val Ser Ala Ser Ser Ser
   2855 2860 2865
   Pro Ser Lys Ala Phe Ala Ser Leu Thr Thr Ala Pro Pro Ser Thr
   2870 2875 2880
   Trp Gly Ile Pro Gln Ser Thr Leu Thr Phe Glu Phe Ser Glu Val
   2885 2890 2895
   Pro Ser Leu Asp Thr Lys Ser Ala Ser Leu Pro Thr Pro Gly Gln
   2900 2905 2910
   Ser Leu Asn Thr Ile Pro Asp Ser Asp Ala Ser Thr Ala Ser Ser
   2915 2920 2925
   Ser Leu Ser Lys Ser Pro Glu Lys Asn Pro Arg Ala Arg Met Met
   2930 2935 2940
   Thr Ser Thr Lys Ala Ile Ser Ala Ser Ser Phe Gln Ser Thr Gly
   2945 2950 2955
   Phe Thr Glu Thr Pro Glu Gly Ser Ala Ser Pro Ser Met Ala Gly
   2960 2965 2970
   His Glu Pro Arg Val Pro Thr Ser Gly Thr Gly Asp Pro Arg Tyr
   2975 2980 2985
   Ala Ser Glu Ser Met Ser Tyr Pro Asp Pro Ser Lys Ala Ser Ser
   2990 2995 3000
   Ala Met Thr Ser Thr Ser Leu Ala Ser Lys Leu Thr Thr Leu Phe
   3005 3010 3015
   Ser Thr Gly Gln Ala Ala Arg Ser Gly Ser Ser Ser Ser Pro Ile
   3020 3025 3030
   Ser Leu Ser Thr Glu Lys Glu Thr Ser Phe Leu Ser Pro Thr Ala
   3035 3040 3045
   Ser Thr Ser Arg Lys Thr Ser Leu Phe Leu Gly Pro Ser Met Ala
   3050 3055 3060
   Arg Gln Pro Asn Ile Leu Val His Leu Gln Thr Ser Ala Leu Thr
   3065 3070 3075
   Leu Ser Pro Thr Ser Thr Leu Asn Met Ser Gln Glu Glu Pro Pro
   3080 3085 3090
   Glu Leu Thr Ser Ser Gln Thr Ile Ala Glu Glu Glu Gly Thr Thr
   3095 3100 3105
   Ala Glu Thr Gln Thr Leu Thr Phe Thr Pro Ser Glu Thr Pro Thr
   3110 3115 3120
   Ser Leu Leu Pro Val Ser Ser Pro Thr Glu Pro Thr Ala Arg Arg
   3125 3130 3135
   Lys Ser Ser Pro Glu Thr Trp Ala Ser Ser Ile Ser Val Pro Ala
   3140 3145 3150
   Lys Thr Ser Leu Val Glu Thr Thr Asp Gly Thr Leu Val Thr Thr
   3155 3160 3165
   Ile Lys Met Ser Ser Gln Ala Ala Gln Gly Asn Ser Thr Trp Pro
   3170 3175 3180
   Ala Pro Ala Glu Glu Thr Gly Thr Ser Pro Ala Gly Thr Ser Pro
   3185 3190 3195
   Gly Ser Pro Glu Val Ser Thr Thr Leu Lys Ile Met Ser Ser Lys
   3200 3205 3210
   Glu Pro Ser Ile Ser Pro Glu Ile Arg Ser Thr Val Arg Asn Ser
   3215 3220 3225
   Pro Trp Lys Thr Pro Glu Thr Thr Val Pro Met Glu Thr Thr Val
       3230                3235                3240
   Glu Pro Val Thr Leu Gln Ser Thr Ala Leu Gly Ser Gly Ser Thr
       3245                3250                3255
   Ser Ile Ser His Leu Pro Thr Gly Thr Thr Ser Pro Thr Lys Ser
       3260                3265                3270
   Pro Thr Glu Asn Met Leu Ala Thr Glu Arg Val Ser Leu Ser Pro
       3275                3280                3285
   Ser Pro Pro Glu Ala Trp Thr Asn Leu Tyr Ser Gly Thr Pro Gly
       3290                3295                3300
   Gly Thr Arg Gln Ser Leu Ala Thr Met Ser Ser Val Ser Leu Glu
       3305                3310                3315
   Ser Pro Thr Ala Arg Ser Ile Thr Gly Thr Gly Gln Gln Ser Ser
       3320                3325                3330
   Pro Glu Leu Val Ser Lys Thr Thr Gly Met Glu Phe Ser Met Trp
       3335                3340                3345
   His Gly Ser Thr Gly Gly Thr Thr Gly Asp Thr His Val Ser Leu
       3350                3355                3360
   Ser Thr Ser Ser Asn Ile Leu Glu Asp Pro Val Thr Ser Pro Asn
       3365                3370                3375
   Ser Val Ser Ser Leu Thr Asp Lys Ser Lys His Lys Thr Glu Thr
       3380                3385                3390
   Trp Val Ser Thr Thr Ala Ile Pro Ser Thr Val Leu Asn Asn Lys
       3395                3400                3405
   Ile Met Ala Ala Glu Gln Gln Thr Ser Arg Ser Val Asp Glu Ala
       3410                3415                3420
   Tyr Ser Ser Thr Ser Ser Trp Ser Asp Gln Thr Ser Gly Ser Asp
       3425                3430                3435
   Ile Thr Leu Gly Ala Ser Pro Asp Val Thr Asn Thr Leu Tyr Ile
       3440                3445                3450
   Thr Ser Thr Ala Gln Thr Thr Ser Leu Val Ser Leu Pro Ser Gly
       3455                3460                3465
   Asp Gln Gly Ile Thr Ser Leu Thr Asn Pro Ser Gly Gly Lys Thr
   3470 3475 3480
   Ser Ser Ala Ser Ser Val Thr Ser Pro Ser Ile Gly Leu Glu Thr
   3485 3490 3495
   Leu Arg Ala Asn Val Ser Ala Val Lys Ser Asp Ile Ala Pro Thr
   3500 3505 3510
   Ala Gly His Leu Ser Gln Thr Ser Ser Pro Ala Glu Val Ser Ile
   3515 3520 3525
   Leu Asp Val Thr Thr Ala Pro Thr Pro Gly Ile Ser Thr Thr Ile
   3530 3535 3540
   Thr Thr Met Gly Thr Asn Ser Ile Ser Thr Thr Thr Pro Asn Pro
   3545 3550 3555
   Glu Val Gly Met Ser Thr Met Asp Ser Thr Pro Ala Thr Glu Arg
   3560 3565 3570
   Arg Thr Thr Ser Thr Glu His Pro Ser Thr Trp Ser Ser Thr Ala
   3575 3580 3585
   Ala Ser Asp Ser Trp Thr Val Thr Asp Met Thr Ser Asn Leu Lys
   3590 3595 3600
   Val Ala Arg Ser Pro Gly Thr Ile Ser Thr Met His Thr Thr Ser
   3605 3610 3615
   Phe Leu Ala Ser Ser Thr Glu Leu Asp Ser Met Ser Thr Pro His
   3620 3625 3630
   Gly Arg Ile Thr Val Ile Gly Thr Ser Leu Val Thr Pro Ser Ser
   3635 3640 3645
   Asp Ala Ser Ala Val Lys Thr Glu Thr Ser Thr Ser Glu Arg Thr
   3650 3655 3660
   Leu Ser Pro Ser Asp Thr Thr Ala Ser Thr Pro Ile Ser Thr Phe
   3665 3670 3675
   Ser Arg Val Gln Arg Met Ser Ile Ser Val Pro Asp Ile Leu Ser
   3680 3685 3690
   Thr Ser Trp Thr Pro Ser Ser Thr Glu Ala Glu Asp Val Pro Val
   3695 3700 3705
   Ser Met Val Ser Thr Asp His Ala Ser Thr Lys Thr Asp Pro Asn
   3710 3715 3720
   Thr Pro Leu Ser Thr Phe Leu Phe Asp Ser Leu Ser Thr Leu Asp
   3725 3730 3735
   Trp Asp Thr Gly Arg Ser Leu Ser Ser Ala Thr Ala Thr Thr Ser
   3740 3745 3750
   Ala Pro Gln Gly Ala Thr Thr Pro Gln Glu Leu Thr Leu Glu Thr
   3755 3760 3765
   Met Ile Ser Pro Ala Thr Ser Gln Leu Pro Phe Ser Ile Gly His
   3770 3775 3780
   Ile Thr Ser Ala Val Thr Pro Ala Ala Met Ala Arg Ser Ser Gly
   3785 3790 3795
   Val Thr Phe Ser Arg Pro Asp Pro Thr Ser Lys Lys Ala Glu Gln
   3800 3805 3810
   Thr Ser Thr Gln Leu Pro Thr Thr Thr Ser Ala His Pro Gly Gln
   3815 3820 3825
   Val Pro Arg Ser Ala Ala Thr Thr Leu Asp Val Ile Pro His Thr
   3830 3835 3840
   Ala Lys Thr Pro Asp Ala Thr Phe Gln Arg Gln Gly Gln Thr Ala
   3845 3850 3855
   Leu Thr Thr Glu Ala Arg Ala Thr Ser Asp Ser Trp Asn Glu Lys
   3860 3865 3870
   Glu Lys Ser Thr Pro Ser Ala Pro Trp Ile Thr Glu Met Met Asn
   3875 3880 3885
   Ser Val Ser Glu Asp Thr Ile Lys Glu Val Thr Ser Ser Ser Ser
   3890 3895 3900
   Val Leu Lys Asp Pro Glu Tyr Ala Gly His Lys Leu Gly Ile Trp
   3905 3910 3915
   Asp Asp Phe Ile Pro Lys Phe Gly Lys Ala Ala His Met Arg Glu
   3920 3925 3930
   Leu Pro Leu Leu Ser Pro Pro Gln Asp Lys Glu Ala Ile His Pro
   3935 3940 3945
   Ser Thr Asn Thr Val Glu Thr Thr Gly Trp Val Thr Ser Ser Glu
   3950 3955 3960
   His Ala Ser His Ser Thr Ile Pro Ala His Ser Ala Ser Ser Lys
   3965 3970 3975
   Leu Thr Ser Pro Val Val Thr Thr Ser Thr Arg Glu Gln Ala Ile
   3980 3985 3990
   Val Ser Met Ser Thr Thr Thr Trp Pro Glu Ser Thr Arg Ala Arg
   3995 4000 4005
   Thr Glu Pro Asn Ser Phe Leu Thr Ile Glu Leu Arg Asp Val Ser
   4010 4015 4020
   Pro Tyr Met Asp Thr Ser Ser Thr Thr Gln Thr Ser Ile Ile Ser
   4025 4030 4035
   Ser Pro Gly Ser Thr Ala Ile Thr Lys Gly Pro Arg Thr Glu Ile
   4040 4045 4050
   Thr Ser Ser Lys Arg Ile Ser Ser Ser Phe Leu Ala Gln Ser Met
   4055 4060 4065
   Arg Ser Ser Asp Ser Pro Ser Glu Ala Ile Thr Arg Leu Ser Asn
   4070 4075 4080
   Phe Pro Ala Met Thr Glu Ser Gly Gly Met Ile Leu Ala Met Gln
   4085 4090 4095
   Thr Ser Pro Pro Gly Ala Thr Ser Leu Ser Ala Pro Thr Leu Asp
   4100 4105 4110
   Thr Ser Ala Thr Ala Ser Trp Thr Gly Thr Pro Leu Ala Thr Thr
   4115 4120 4125
   Gln Arg Phe Thr Tyr Ser Glu Lys Thr Thr Leu Phe Ser Lys Gly
   4130 4135 4140
   Pro Glu Asp Thr Ser Gln Pro Ser Pro Pro Ser Val Glu Glu Thr
   4145 4150 4155
   Ser Ser Ser Ser Ser Leu Val Pro Ile His Ala Thr Thr Ser Pro
   4160 4165 4170
   Ser Asn Ile Leu Leu Thr Ser Gln Gly His Ser Pro Ser Ser Thr
   4175 4180 4185
   Pro Pro Val Thr Ser Val Phe Leu Ser Glu Thr Ser Gly Leu Gly
   4190 4195 4200
   Lys Thr Thr Asp Met Ser Arg Ile Ser Leu Glu Pro Gly Thr Ser
   4205 4210 4215
   Leu Pro Pro Asn Leu Ser Ser Thr Ala Gly Glu Ala Leu Ser Thr
   4220 4225 4230
   Tyr Glu Ala Ser Arg Asp Thr Lys Ala Ile His His Ser Ala Asp
   4235 4240 4245
   Thr Ala Val Thr Asn Met Glu Ala Thr Ser Ser Glu Tyr Ser Pro
   4250 4255 4260
   Ile Pro Gly His Thr Lys Pro Ser Lys Ala Thr Ser Pro Leu Val
   4265 4270 4275
   Thr Ser His Ile Met Gly Asp Ile Thr Ser Ser Thr Ser Val Phe
   4280 4285 4290
   Gly Ser Ser Glu Thr Thr Glu Ile Glu Thr Val Ser Ser Val Asn
   4295 4300 4305
   Gln Gly Leu Gln Glu Arg Ser Thr Ser Gln Val Ala Ser Ser Ala
   4310 4315 4320
   Thr Glu Thr Ser Thr Val Ile Thr His Val Ser Ser Gly Asp Ala
   4325 4330 4335
   Thr Thr His Val Thr Lys Thr Gln Ala Thr Phe Ser Ser Gly Thr
   4340 4345 4350
   Ser Ile Ser Ser Pro His Gln Phe Ile Thr Ser Thr Asn Thr Phe
   4355 4360 4365
   Thr Asp Val Ser Thr Asn Pro Ser Thr Ser Leu Ile Met Thr Glu
   4370 4375 4380
   Ser Ser Gly Val Thr Ile Thr Thr Gln Thr Gly Pro Thr Gly Ala
   4385 4390 4395
   Ala Thr Gln Gly Pro Tyr Leu Leu Asp Thr Ser Thr Met Pro Tyr
   4400 4405 4410
   Leu Thr Glu Thr Pro Leu Ala Val Thr Pro Asp Phe Met Gln Ser
   4415 4420 4425
   Glu Lys Thr Thr Leu Ile Ser Lys Gly Pro Lys Asp Val Thr Trp
   4430 4435 4440
   Thr Ser Pro Pro Ser Val Ala Glu Thr Ser Tyr Pro Ser Ser Leu
   4445 4450 4455
   Thr Pro Phe Leu Val Thr Thr Ile Pro Pro Ala Thr Ser Thr Leu
   4460 4465 4470
   Gln Gly Gln His Thr Ser Ser Pro Val Ser Ala Thr Ser Val Leu
   4475 4480 4485
   Thr Ser Gly Leu Val Lys Thr Thr Asp Met Leu Asn Thr Ser Met
   4490 4495 4500
   Glu Pro Val Thr Asn Ser Pro Gln Asn Leu Asn Asn Pro Ser Asn
   4505 4510 4515
   Glu Ile Leu Ala Thr Leu Ala Ala Thr Thr Asp Ile Glu Thr Ile
   4520 4525 4530
   His Pro Ser Ile Asn Lys Ala Val Thr Asn Met Gly Thr Ala Ser
   4535 4540 4545
   Ser Ala His Val Leu His Ser Thr Leu Pro Val Ser Ser Glu Pro
   4550 4555 4560
   Ser Thr Ala Thr Ser Pro Met Val Pro Ala Ser Ser Met Gly Asp
   4565 4570 4575
   Ala Leu Ala Ser Ile Ser Ile Pro Gly Ser Glu Thr Thr Asp Ile
   4580 4585 4590
   Glu Gly Glu Pro Thr Ser Ser Leu Thr Ala Gly Arg Lys Glu Asn
   4595 4600 4605
   Ser Thr Leu Gln Glu Met Asn Ser Thr Thr Glu Ser Asn Ile Ile
   4610 4615 4620
   Leu Ser Asn Val Ser Val Gly Ala Ile Thr Glu Ala Thr Lys Met
   4625 4630 4635
   Glu Val Pro Ser Phe Asp Ala Thr Phe Ile Pro Thr Pro Ala Gln
   4640 4645 4650
   Ser Thr Lys Phe Pro Asp Ile Phe Ser Val Ala Ser Ser Arg Leu
   4655 4660 4665
   Ser Asn Ser Pro Pro Met Thr Ile Ser Thr His Met Thr Thr Thr
   4670 4675 4680
   Gln Thr Gly Ser Ser Gly Ala Thr Ser Lys Ile Pro Leu Ala Leu
   4685 4690 4695
   Asp Thr Ser Thr Leu Glu Thr Ser Ala Gly Thr Pro Ser Val Val
   4700 4705 4710
   Thr Glu Gly Phe Ala His Ser Lys Ile Thr Thr Ala Met Asn Asn
   4715 4720 4725
   Asp Val Lys Asp Val Ser Gln Thr Asn Pro Pro Phe Gln Asp Glu
   4730 4735 4740
   Ala Ser Ser Pro Ser Ser Gln Ala Pro Val Leu Val Thr Thr Leu
   4745 4750 4755
   Pro Ser Ser Val Ala Phe Thr Pro Gln Trp His Ser Thr Ser Ser
   4760 4765 4770
   Pro Val Ser Met Ser Ser Val Leu Thr Ser Ser Leu Val Lys Thr
   4775 4780 4785
   Ala Gly Lys Val Asp Thr Ser Leu Glu Thr Val Thr Ser Ser Pro
   4790 4795 4800
   Gln Ser Met Ser Asn Thr Leu Asp Asp Ile Ser Val Thr Ser Ala
   4805 4810 4815
   Ala Thr Thr Asp Ile Glu Thr Thr His Pro Ser Ile Asn Thr Val
   4820 4825 4830
   Val Thr Asn Val Gly Thr Thr Gly Ser Ala Phe Glu Ser His Ser
   4835 4840 4845
   Thr Val Ser Ala Tyr Pro Glu Pro Ser Lys Val Thr Ser Pro Asn
   4850 4855 4860
   Val Thr Thr Ser Thr Met Glu Asp Thr Thr Ile Ser Arg Ser Ile
   4865 4870 4875
   Pro Lys Ser Ser Lys Thr Thr Arg Thr Glu Thr Glu Thr Thr Ser
   4880 4885 4890
   Ser Leu Thr Pro Lys Leu Arg Glu Thr Ser Ile Ser Gln Glu Ile
   4895 4900 4905
   Thr Ser Ser Thr Glu Thr Ser Thr Val Pro Tyr Lys Glu Leu Thr
   4910 4915 4920
   Gly Ala Thr Thr Glu Val Ser Arg Thr Asp Val Thr Ser Ser Ser
   4925 4930 4935
   Ser Thr Ser Phe Pro Gly Pro Asp Gln Ser Thr Val Ser Leu Asp
   4940 4945 4950
   Ile Ser Thr Glu Thr Asn Thr Arg Leu Ser Thr Ser Pro Ile Met
   4955 4960 4965
   Thr Glu Ser Ala Glu Ile Thr Ile Thr Thr Gln Thr Gly Pro His
   4970 4975 4980
   Gly Ala Thr Ser Gln Asp Thr Phe Thr Met Asp Pro Ser Asn Thr
   4985 4990 4995
   Thr Pro Gln Ala Gly Ile His Ser Ala Met Thr His Gly Phe Ser
   5000 5005 5010
   Gln Leu Asp Val Thr Thr Leu Met Ser Arg Ile Pro Gln Asp Val
   5015 5020 5025
   Ser Trp Thr Ser Pro Pro Ser Val Asp Lys Thr Ser Ser Pro Ser
   5030 5035 5040
   Ser Phe Leu Ser Ser Pro Ala Met Thr Thr Pro Ser Leu Ile Ser
   5045 5050 5055
   Ser Thr Leu Pro Glu Asp Lys Leu Ser Ser Pro Met Thr Ser Leu
   5060 5065 5070
   Leu Thr Ser Gly Leu Val Lys Ile Thr Asp Ile Leu Arg Thr Arg
   5075 5080 5085
   Leu Glu Pro Val Thr Ser Ser Leu Pro Asn Phe Ser Ser Thr Ser
   5090 5095 5100
   Asp Lys Ile Leu Ala Thr Ser Lys Asp Ser Lys Asp Thr Lys Glu
   5105 5110 5115
   Ile Phe Pro Ser Ile Asn Thr Glu Glu Thr Asn Val Lys Ala Asn
   5120 5125 5130
   Asn Ser Gly His Glu Ser His Ser Pro Ala Leu Ala Asp Ser Glu
   5135 5140 5145
   Thr Pro Lys Ala Thr Thr Gln Met Val Ile Thr Thr Thr Val Gly
   5150 5155 5160
   Asp Pro Ala Pro Ser Thr Ser Met Pro Val His Gly Ser Ser Glu
   5165 5170 5175
   Thr Thr Asn Ile Lys Arg Glu Pro Thr Tyr Phe Leu Thr Pro Arg
   5180 5185 5190
   Leu Arg Glu Thr Ser Thr Ser Gln Glu Ser Ser Phe Pro Thr Asp
   5195 5200 5205
   Thr Ser Phe Leu Leu Ser Lys Val Pro Thr Gly Thr Ile Thr Glu
   5210 5215 5220
   Val Ser Ser Thr Gly Val Asn Ser Ser Ser Lys Ile Ser Thr Pro
   5225 5230 5235
   Asp His Asp Lys Ser Thr Val Pro Pro Asp Thr Phe Thr Gly Glu
   5240 5245 5250
   Ile Pro Arg Val Phe Thr Ser Ser Ile Lys Thr Lys Ser Ala Glu
   5255 5260 5265
   Met Thr Ile Thr Thr Gln Ala Ser Pro Pro Glu Ser Ala Ser His
   5270 5275 5280
   Ser Thr Leu Pro Leu Asp Thr Ser Thr Thr Leu Ser Gln Gly Gly
   5285 5290 5295
   Thr His Ser Thr Val Thr Gln Gly Phe Pro Tyr Ser Glu Val Thr
   5300 5305 5310
   Thr Leu Met Gly Met Gly Pro Gly Asn Val Ser Trp Met Thr Thr
   5315 5320 5325
   Pro Pro Val Glu Glu Thr Ser Ser Val Ser Ser Leu Met Ser Ser
   5330 5335 5340
   Pro Ala Met Thr Ser Pro Ser Pro Val Ser Ser Thr Ser Pro Gln
   5345 5350 5355
   Ser Ile Pro Ser Ser Pro Leu Pro Val Thr Ala Leu Pro Thr Ser
   5360 5365 5370
   Val Leu Val Thr Thr Thr Asp Val Leu Gly Thr Thr Ser Pro Glu
   5375 5380 5385
   Ser Val Thr Ser Ser Pro Pro Asn Leu Ser Ser Ile Thr His Glu
   5390 5395 5400
   Arg Pro Ala Thr Tyr Lys Asp Thr Ala His Thr Glu Ala Ala Met
   5405 5410 5415
   His His Ser Thr Asn Thr Ala Val Thr Asn Val Gly Thr Ser Gly
   5420 5425 5430
   Ser Gly His Lys Ser Gln Ser Ser Val Leu Ala Asp Ser Glu Thr
   5435 5440 5445
   Ser Lys Ala Thr Pro Leu Met Ser Thr Thr Ser Thr Leu Gly Asp
   5450 5455 5460
   Thr Ser Val Ser Thr Ser Thr Pro Asn Ile Ser Gln Thr Asn Gln
   5465 5470 5475
   Ile Gln Thr Glu Pro Thr Ala Ser Leu Ser Pro Arg Leu Arg Glu
   5480 5485 5490
   Ser Ser Thr Ser Glu Lys Thr Ser Ser Thr Thr Glu Thr Asn Thr
   5495 5500 5505
   Ala Phe Ser Tyr Val Pro Thr Gly Ala Ile Thr Gln Ala Ser Arg
   5510 5515 5520
   Thr Glu Ile Ser Ser Ser Arg Thr Ser Ile Ser Asp Leu Asp Arg
   5525 5530 5535
   Pro Thr Ile Ala Pro Asp Ile Ser Thr Gly Met Ile Thr Arg Leu
   5540 5545 5550
   Phe Thr Ser Pro Ile Met Thr Lys Ser Ala Glu Met Thr Val Thr
   5555 5560 5565
   Thr Gln Thr Thr Thr Pro Gly Ala Thr Ser Gln Gly Ile Leu Pro
   5570 5575 5580
   Trp Asp Thr Ser Thr Thr Leu Phe Gln Gly Gly Thr His Ser Thr
   5585 5590 5595
   Val Ser Gln Gly Phe Pro His Ser Glu Ile Thr Thr Leu Arg Ser
   5600 5605 5610
   Arg Thr Pro Gly Asp Val Ser Trp Met Thr Thr Pro Pro Val Glu
   5615 5620 5625
   Glu Thr Ser Ser Gly Phe Ser Leu Met Ser Pro Ser Met Thr Ser
   5630 5635 5640
   Pro Ser Pro Val Ser Ser Thr Ser Pro Glu Ser Ile Pro Ser Ser
   5645 5650 5655
   Pro Leu Pro Val Thr Ala Leu Leu Thr Ser Val Leu Val Thr Thr
   5660 5665 5670
   Thr Asn Val Leu Gly Thr Thr Ser Pro Glu Thr Val Thr Ser Ser
   5675 5680 5685
   Pro Pro Asn Leu Ser Ser Pro Thr Gln Glu Arg Leu Thr Thr Tyr
   5690 5695 5700
   Lys Asp Thr Ala His Thr Glu Ala Met His Ala Ser Met His Thr
   5705 5710 5715
   Asn Thr Ala Val Ala Asn Val Gly Thr Ser Ile Ser Gly His Glu
   5720 5725 5730
   Ser Gln Ser Ser Val Pro Ala Asp Ser His Thr Ser Lys Ala Thr
   5735 5740 5745
   Ser Pro Met Gly Ile Thr Phe Ala Met Gly Asp Thr Ser Val Ser
   5750 5755 5760
   Thr Ser Thr Pro Ala Phe Phe Glu Thr Arg Ile Gln Thr Glu Ser
   5765 5770 5775
   Thr Ser Ser Leu Ile Pro Gly Leu Arg Asp Thr Arg Thr Ser Glu
   5780 5785 5790
   Glu Ile Asn Thr Val Thr Glu Thr Ser Thr Val Leu Ser Glu Val
   5795 5800 5805
   Pro Thr Thr Thr Thr Thr Glu Val Ser Arg Thr Glu Val Ile Thr
   5810 5815 5820
   Ser Ser Arg Thr Thr Ile Ser Gly Pro Asp His Ser Lys Met Ser
   5825 5830 5835
   Pro Tyr Ile Ser Thr Glu Thr Ile Thr Arg Leu Ser Thr Phe Pro
   5840 5845 5850
   Phe Val Thr Gly Ser Thr Glu Met Ala Ile Thr Asn Gln Thr Gly
   5855 5860 5865
   Pro Ile Gly Thr Ile Ser Gln Ala Thr Leu Thr Leu Asp Thr Ser
   5870 5875 5880
   Ser Thr Ala Ser Trp Glu Gly Thr His Ser Pro Val Thr Gln Arg
   5885 5890 5895
   Phe Pro His Ser Glu Glu Thr Thr Thr Met Ser Arg Ser Thr Lys
   5900 5905 5910
   Gly Val Ser Trp Gln Ser Pro Pro Ser Val Glu Glu Thr Ser Ser
   5915 5920 5925
   Pro Ser Ser Pro Val Pro Leu Pro Ala Ile Thr Ser His Ser Ser
   5930 5935 5940
   Leu Tyr Ser Ala Val Ser Gly Ser Ser Pro Thr Ser Ala Leu Pro
   5945 5950 5955
   Val Thr Ser Leu Leu Thr Ser Gly Arg Arg Lys Thr Ile Asp Met
   5960 5965 5970
   Leu Asp Thr His Ser Glu Leu Val Thr Ser Ser Leu Pro Ser Ala
   5975 5980 5985
   Ser Ser Phe Ser Gly Glu Ile Leu Thr Ser Glu Ala Ser Thr Asn
   5990 5995 6000
   Thr Glu Thr Ile His Phe Ser Glu Asn Thr Ala Glu Thr Asn Met
   6005 6010 6015
   Gly Thr Thr Asn Ser Met His Lys Leu His Ser Ser Val Ser Ile
   6020 6025 6030
   His Ser Gln Pro Ser Gly His Thr Pro Pro Lys Val Thr Gly Ser
   6035 6040 6045
   Met Met Glu Asp Ala Ile Val Ser Thr Ser Thr Pro Gly Ser Pro
   6050 6055 6060
   Glu Thr Lys Asn Val Asp Arg Asp Ser Thr Ser Pro Leu Thr Pro
   6065 6070 6075
   Glu Leu Lys Glu Asp Ser Thr Ala Leu Val Met Asn Ser Thr Thr
   6080 6085 6090
   Glu Ser Asn Thr Val Phe Ser Ser Val Ser Leu Asp Ala Ala Thr
   6095 6100 6105
   Glu Val Ser Arg Ala Glu Val Thr Tyr Tyr Asp Pro Thr Phe Met
   6110 6115 6120
   Pro Ala Ser Ala Gln Ser Thr Lys Ser Pro Asp Ile Ser Pro Glu
   6125 6130 6135
   Ala Ser Ser Ser His Ser Asn Ser Pro Pro Leu Thr Ile Ser Thr
   6140 6145 6150
   His Lys Thr Ile Ala Thr Gln Thr Gly Pro Ser Gly Val Thr Ser
   6155 6160 6165
   Leu Gly Gln Leu Thr Leu Asp Thr Ser Thr Ile Ala Thr Ser Ala
   6170 6175 6180
   Gly Thr Pro Ser Ala Arg Thr Gln Asp Phe Val Asp Ser Glu Thr
   6185 6190 6195
   Thr Ser Val Met Asn Asn Asp Leu Asn Asp Val Leu Lys Thr Ser
   6200 6205 6210
   Pro Phe Ser Ala Glu Glu Ala Asn Ser Leu Ser Ser Gln Ala Pro
   6215 6220 6225
   Leu Leu Val Thr Thr Ser Pro Ser Pro Val Thr Ser Thr Leu Gln
   6230 6235 6240
   Glu His Ser Thr Ser Ser Leu Val Ser Val Thr Ser Val Pro Thr
   6245 6250 6255
   Pro Thr Leu Ala Lys Ile Thr Asp Met Asp Thr Asn Leu Glu Pro
   6260 6265 6270
   Val Thr Arg Ser Pro Gln Asn Leu Arg Asn Thr Leu Ala Thr Ser
   6275 6280 6285
   Glu Ala Thr Thr Asp Thr His Thr Met His Pro Ser Ile Asn Thr
   6290 6295 6300
   Ala Met Ala Asn Val Gly Thr Thr Ser Ser Pro Asn Glu Phe Tyr
   6305 6310 6315
   Phe Thr Val Ser Pro Asp Ser Asp Pro Tyr Lys Ala Thr Ser Ala
   6320 6325 6330
   Val Val Ile Thr Ser Thr Ser Gly Asp Ser Ile Val Ser Thr Ser
   6335 6340 6345
   Met Pro Arg Ser Ser Ala Met Lys Lys Ile Glu Ser Glu Thr Thr
   6350 6355 6360
   Phe Ser Leu Ile Phe Arg Leu Arg Glu Thr Ser Thr Ser Gln Lys
   6365 6370 6375
   Ile Gly Ser Ser Ser Asp Thr Ser Thr Val Phe Asp Lys Ala Phe
   6380 6385 6390
   Thr Ala Ala Thr Thr Glu Val Ser Arg Thr Glu Leu Thr Ser Ser
   6395 6400 6405
   Ser Arg Thr Ser Ile Gln Gly Thr Glu Lys Pro Thr Met Ser Pro
   6410 6415 6420
   Asp Thr Ser Thr Arg Ser Val Thr Met Leu Ser Thr Phe Ala Gly
   6425 6430 6435
   Leu Thr Lys Ser Glu Glu Arg Thr Ile Ala Thr Gln Thr Gly Pro
   6440 6445 6450
   His Arg Ala Thr Ser Gln Gly Thr Leu Thr Trp Asp Thr Ser Ile
   6455 6460 6465
   Thr Thr Ser Gln Ala Gly Thr His Ser Ala Met Thr His Gly Phe
   6470 6475 6480
   Ser Gln Leu Asp Leu Ser Thr Leu Thr Ser Arg Val Pro Glu Tyr
   6485 6490 6495
   Ile Ser Gly Thr Ser Pro Pro Ser Val Glu Lys Thr Ser Ser Ser
   6500 6505 6510
   Ser Ser Leu Leu Ser Leu Pro Ala Ile Thr Ser Pro Ser Pro Val
   6515 6520 6525
   Pro Thr Thr Leu Pro Glu Ser Arg Pro Ser Ser Pro Val His Leu
   6530 6535 6540
   Thr Ser Leu Pro Thr Ser Gly Leu Val Lys Thr Thr Asp Met Leu
   6545 6550 6555
   Ala Ser Val Ala Ser Leu Pro Pro Asn Leu Gly Ser Thr Ser His
   6560 6565 6570
   Lys Ile Pro Thr Thr Ser Glu Asp Ile Lys Asp Thr Glu Lys Met
   6575 6580 6585
   Tyr Pro Ser Thr Asn Ile Ala Val Thr Asn Val Gly Thr Thr Thr
   6590 6595 6600
   Ser Glu Lys Glu Ser Tyr Ser Ser Val Pro Ala Tyr Ser Glu Pro
   6605 6610 6615
   Pro Lys Val Thr Ser Pro Met Val Thr Ser Phe Asn Ile Arg Asp
   6620 6625 6630
   Thr Ile Val Ser Thr Ser Met Pro Gly Ser Ser Glu Ile Thr Arg
   6635 6640 6645
   Ile Glu Met Glu Ser Thr Phe Ser Val Ala His Gly Leu Lys Gly
   6650 6655 6660
   Thr Ser Thr Ser Gln Asp Pro Ile Val Ser Thr Glu Lys Ser Ala
   6665 6670 6675
   Val Leu His Lys Leu Thr Thr Gly Ala Thr Glu Thr Ser Arg Thr
   6680 6685 6690
   Glu Val Ala Ser Ser Arg Arg Thr Ser Ile Pro Gly Pro Asp His
   6695 6700 6705
   Ser Thr Glu Ser Pro Asp Ile Ser Thr Glu Val Ile Pro Ser Leu
   6710 6715 6720
   Pro Ile Ser Leu Gly Ile Thr Glu Ser Ser Asn Met Thr Ile Ile
   6725 6730 6735
   Thr Arg Thr Gly Pro Pro Leu Gly Ser Thr Ser Gln Gly Thr Phe
   6740 6745 6750
   Thr Leu Asp Thr Pro Thr Thr Ser Ser Arg Ala Gly Thr His Ser
   6755 6760 6765
   Met Ala Thr Gln Glu Phe Pro His Ser Glu Met Thr Thr Val Met
   6770 6775 6780
   Asn Lys Asp Pro Glu Ile Leu Ser Trp Thr Ile Pro Pro Ser Ile
   6785 6790 6795
   Glu Lys Thr Ser Phe Ser Ser Ser Leu Met Pro Ser Pro Ala Met
   6800 6805 6810
   Thr Ser Pro Pro Val Ser Ser Thr Leu Pro Lys Thr Ile His Thr
   6815 6820 6825
   Thr Pro Ser Pro Met Thr Ser Leu Leu Thr Pro Ser Leu Val Met
   6830 6835 6840
   Thr Thr Asp Thr Leu Gly Thr Ser Pro Glu Pro Thr Thr Ser Ser
   6845 6850 6855
   Pro Pro Asn Leu Ser Ser Thr Ser His Val Ile Leu Thr Thr Asp
   6860 6865 6870
   Glu Asp Thr Thr Ala Ile Glu Ala Met His Pro Ser Thr Ser Thr
   6875 6880 6885
   Ala Ala Thr Asn Val Glu Thr Thr Cys Ser Gly His Gly Ser Gln
   6890 6895 6900
   Ser Ser Val Leu Thr Asp Ser Glu Lys Thr Lys Ala Thr Ala Pro
   6905 6910 6915
   Met Asp Thr Thr Ser Thr Met Gly His Thr Thr Val Ser Thr Ser
   6920 6925 6930
   Met Ser Val Ser Ser Glu Thr Thr Lys Ile Lys Arg Glu Ser Thr
   6935 6940 6945
   Tyr Ser Leu Thr Pro Gly Leu Arg Glu Thr Ser Ile Ser Gln Asn
   6950 6955 6960
   Ala Ser Phe Ser Thr Asp Thr Ser Ile Val Leu Ser Glu Val Pro
   6965 6970 6975
   Thr Gly Thr Thr Ala Glu Val Ser Arg Thr Glu Val Thr Ser Ser
   6980 6985 6990
   Gly Arg Thr Ser Ile Pro Gly Pro Ser Gln Ser Thr Val Leu Pro
   6995 7000 7005
   Glu Ile  Ser Thr Arg Thr Met  Thr Arg Leu Phe Ala  Ser Pro Thr
       7010                 7015                 7020
   Met Thr  Glu Ser Ala Glu Met  Thr Ile Pro Thr Gln  Thr Gly Pro
       7025                 7030                 7035
   Ser Gly  Ser Thr Ser Gln Asp  Thr Leu Thr Leu Asp  Thr Ser Thr
       7040                 7045                 7050
   Thr Lys  Ser Gln Ala Lys Thr  His Ser Thr Leu Thr  Gln Arg Phe
       7055                 7060                 7065
   Pro His  Ser Glu Met Thr Thr  Leu Met Ser Arg Gly  Pro Gly Asp
       7070                 7075                 7080
   Met Ser  Trp Gln Ser Ser Pro  Ser Leu Glu Asn Pro  Ser Ser Leu
       7085                 7090                 7095
   Pro Ser  Leu Leu Ser Leu Pro  Ala Thr Thr Ser Pro  Pro Pro Ile
       7100                 7105                 7110
   Ser Ser  Thr Leu Pro Val Thr  Ile Ser Ser Ser Pro  Leu Pro Val
       7115                 7120                 7125
   Thr Ser  Leu Leu Thr Ser Ser  Pro Val Thr Thr Thr  Asp Met Leu
       7130                 7135                 7140
   His Thr  Ser Pro Glu Leu Val  Thr Ser Ser Pro Pro  Lys Leu Ser
       7145                 7150                 7155
   His Thr  Ser Asp Glu Arg Leu  Thr Thr Gly Lys Asp  Thr Thr Asn
       7160                 7165                 7170
   Thr Glu  Ala Val His Pro Ser  Thr Asn Thr Ala Ala  Ser Asn Val
       7175                 7180                 7185
   Glu Ile  Pro Ser Phe Gly His  Glu Ser Pro Ser Ser  Ala Leu Ala
       7190                 7195                 7200
   Asp Ser  Glu Thr Ser Lys Ala  Thr Ser Pro Met Phe  Ile Thr Ser
       7205                 7210                 7215
   Thr Gln  Glu Asp Thr Thr Val  Ala Ile Ser Thr Pro  His Phe Leu
       7220                 7225                 7230
   Glu Thr  Ser Arg Ile Gln Lys  Glu Ser Ile Ser Ser  Leu Ser Pro
       7235                 7240                 7245
   Lys Leu Arg Glu Thr Gly Ser Ser Val Glu Thr Ser Ser Ala Ile
   7250 7255 7260
   Glu Thr Ser Ala Val Leu Ser Glu Val Ser Ile Gly Ala Thr Thr
   7265 7270 7275
   Glu Ile Ser Arg Thr Glu Val Thr Ser Ser Ser Arg Thr Ser Ile
   7280 7285 7290
   Ser Gly Ser Ala Glu Ser Thr Met Leu Pro Glu Ile Ser Thr Thr
   7295 7300 7305
   Arg Lys Ile Ile Lys Phe Pro Thr Ser Pro Ile Leu Ala Glu Ser
   7310 7315 7320
   Ser Glu Met Thr Ile Lys Thr Gln Thr Ser Pro Pro Gly Ser Thr
   7325 7330 7335
   Ser Glu Ser Thr Phe Thr Leu Asp Thr Ser Thr Thr Pro Ser Leu
   7340 7345 7350
   Val Ile Thr His Ser Thr Met Thr Gln Arg Leu Pro His Ser Glu
   7355 7360 7365
   Ile Thr Thr Leu Val Ser Arg Gly Ala Gly Asp Val Pro Arg Pro
   7370 7375 7380
   Ser Ser Leu Pro Val Glu Glu Thr Ser Pro Pro Ser Ser Gln Leu
   7385 7390 7395
   Ser Leu Ser Ala Met Ile Ser Pro Ser Pro Val Ser Ser Thr Leu
   7400 7405 7410
   Pro Ala Ser Ser His Ser Ser Ser Ala Ser Val Thr Ser Pro Leu
   7415 7420 7425
   Thr Pro Gly Gln Val Lys Thr Thr Glu Val Leu Asp Ala Ser Ala
   7430 7435 7440
   Glu Pro Glu Thr Ser Ser Pro Pro Ser Leu Ser Ser Thr Ser Val
   7445 7450 7455
   Glu Ile Leu Ala Thr Ser Glu Val Thr Thr Asp Thr Glu Lys Ile
   7460 7465 7470
   His Pro Phe Pro Asn Thr Ala Val Thr Lys Val Gly Thr Ser Ser
   7475 7480 7485
   Ser Gly  His Glu Ser Pro Ser  Ser Val Leu Pro Asp  Ser Glu Thr
       7490                 7495                 7500
   Thr Lys  Ala Thr Ser Ala Met  Gly Thr Ile Ser Ile  Met Gly Asp
       7505                 7510                 7515
   Thr Ser  Val Ser Thr Leu Thr  Pro Ala Leu Ser Asn  Thr Arg Lys
       7520                 7525                 7530
   Ile Gln  Ser Glu Pro Ala Ser  Ser Leu Thr Thr Arg  Leu Arg Glu
       7535                 7540                 7545
   Thr Ser  Thr Ser Glu Glu Thr  Ser Leu Ala Thr Glu  Ala Asn Thr
       7550                 7555                 7560
   Val Leu  Ser Lys Val Ser Thr  Gly Ala Thr Thr Glu  Val Ser Arg
       7565                 7570                 7575
   Thr Glu  Ala Ile Ser Phe Ser  Arg Thr Ser Met Ser  Gly Pro Glu
       7580                 7585                 7590
   Gln Ser  Thr Met Ser Gln Asp  Ile Ser Ile Gly Thr  Ile Pro Arg
       7595                 7600                 7605
   Ile Ser  Ala Ser Ser Val Leu  Thr Glu Ser Ala Lys  Met Thr Ile
       7610                 7615                 7620
   Thr Thr  Gln Thr Gly Pro Ser  Glu Ser Thr Leu Glu  Ser Thr Leu
       7625                 7630                 7635
   Asn Leu  Asn Thr Ala Thr Thr  Pro Ser Trp Val Glu  Thr His Ser
       7640                 7645                 7650
   Ile Val  Ile Gln Gly Phe Pro  His Pro Glu Met Thr  Thr Ser Met
       7655                 7660                 7665
   Gly Arg  Gly Pro Gly Gly Val  Ser Trp Pro Ser Pro  Pro Phe Val
       7670                 7675                 7680
   Lys Glu  Thr Ser Pro Pro Ser  Ser Pro Leu Ser Leu  Pro Ala Val
       7685                 7690                 7695
   Thr Ser  Pro His Pro Val Ser  Thr Thr Phe Leu Ala  His Ile Pro
       7700                 7705                 7710
   Pro Ser  Pro Leu Pro Val Thr  Ser Leu Leu Thr Ser  Gly Pro Ala
   7715 7720 7725
   Thr Thr Thr Asp Ile Leu Gly Thr Ser Thr Glu Pro Gly Thr Ser
   7730 7735 7740
   Ser Ser Ser Ser Leu Ser Thr Thr Ser His Glu Arg Leu Thr Thr
   7745 7750 7755
   Tyr Lys Asp Thr Ala His Thr Glu Ala Val His Pro Ser Thr Asn
   7760 7765 7770
   Thr Gly Gly Thr Asn Val Ala Thr Thr Ser Ser Gly Tyr Lys Ser
   7775 7780 7785
   Gln Ser Ser Val Leu Ala Asp Ser Ser Pro Met Cys Thr Thr Ser
   7790 7795 7800
   Thr Met Gly Asp Thr Ser Val Leu Thr Ser Thr Pro Ala Phe Leu
   7805 7810 7815
   Glu Thr Arg Arg Ile Gln Thr Glu Leu Ala Ser Ser Leu Thr Pro
   7820 7825 7830
   Gly Leu Arg Glu Ser Ser Gly Ser Glu Gly Thr Ser Ser Gly Thr
   7835 7840 7845
   Lys Met Ser Thr Val Leu Ser Lys Val Pro Thr Gly Ala Thr Thr
   7850 7855 7860
   Glu Ile Ser Lys Glu Asp Val Thr Ser Ile Pro Gly Pro Ala Gln
   7865 7870 7875
   Ser Thr Ile Ser Pro Asp Ile Ser Thr Arg Thr Val Ser Trp Phe
   7880 7885 7890
   Ser Thr Ser Pro Val Met Thr Glu Ser Ala Glu Ile Thr Met Asn
   7895 7900 7905
   Thr His Thr Ser Pro Leu Gly Ala Thr Thr Gln Gly Thr Ser Thr
   7910 7915 7920
   Leu Ala Thr Ser Ser Thr Thr Ser Leu Thr Met Thr His Ser Thr
   7925 7930 7935
   Ile Ser Gln Gly Phe Ser His Ser Gln Met Ser Thr Leu Met Arg
   7940 7945 7950
   Arg Gly Pro Glu Asp Val Ser Trp Met Ser Pro Pro Leu Leu Glu
       7955                7960                7965
   Lys Thr Arg Pro Ser Phe Ser Leu Met Ser Ser Pro Ala Thr Thr
       7970                7975                7980
   Ser Pro Ser Pro Val Ser Ser Thr Leu Pro Glu Ser Ile Ser Ser
       7985                7990                7995
   Ser Pro Leu Pro Val Thr Ser Leu Leu Thr Ser Gly Leu Ala Lys
       8000                8005                8010
   Thr Thr Asp Met Leu His Lys Ser Ser Glu Pro Val Thr Asn Ser
       8015                8020                8025
   Pro Ala Asn Leu Ser Ser Thr Ser Val Glu Ile Leu Ala Thr Ser
       8030                8035                8040
   Glu Val Thr Thr Asp Thr Glu Lys Thr His Pro Ser Ser Asn Arg
       8045                8050                8055
   Thr Val Thr Asp Val Gly Thr Ser Ser Ser Gly His Glu Ser Thr
       8060                8065                8070
   Ser Phe Val Leu Ala Asp Ser Gln Thr Ser Lys Val Thr Ser Pro
       8075                8080                8085
   Met Val Ile Thr Ser Thr Met Glu Asp Thr Ser Val Ser Thr Ser
       8090                8095                8100
   Thr Pro Gly Phe Phe Glu Thr Ser Arg Ile Gln Thr Glu Pro Thr
       8105                8110                8115
   Ser Ser Leu Thr Leu Gly Leu Arg Lys Thr Ser Ser Ser Glu Gly
       8120                8125                8130
   Thr Ser Leu Ala Thr Glu Met Ser Thr Val Leu Ser Gly Val Pro
       8135                8140                8145
   Thr Gly Ala Thr Ala Glu Val Ser Arg Thr Glu Val Thr Ser Ser
       8150                8155                8160
   Ser Arg Thr Ser Ile Ser Gly Phe Ala Gln Leu Thr Val Ser Pro
       8165                8170                8175
   Glu Thr Ser Thr Glu Thr Ile Thr Arg Leu Pro Thr Ser Ser Ile
       8180                8185                8190
   Met Thr Glu Ser Ala Glu Met Met Ile Lys Thr Gln Thr Asp Pro
   8195 8200 8205
   Pro Gly Ser Thr Pro Glu Ser Thr His Thr Val Asp Ile Ser Thr
   8210 8215 8220
   Thr Pro Asn Trp Val Glu Thr His Ser Thr Val Thr Gln Arg Phe
   8225 8230 8235
   Ser His Ser Glu Met Thr Thr Leu Val Ser Arg Ser Pro Gly Asp
   8240 8245 8250
   Met Leu Trp Pro Ser Gln Ser Ser Val Glu Glu Thr Ser Ser Ala
   8255 8260 8265
   Ser Ser Leu Leu Ser Leu Pro Ala Thr Thr Ser Pro Ser Pro Val
   8270 8275 8280
   Ser Ser Thr Leu Val Glu Asp Phe Pro Ser Ala Ser Leu Pro Val
   8285 8290 8295
   Thr Ser Leu Leu Thr Pro Gly Leu Val Ile Thr Thr Asp Arg Met
   8300 8305 8310
   Gly Ile Ser Arg Glu Pro Gly Thr Ser Ser Thr Ser Asn Leu Ser
   8315 8320 8325
   Ser Thr Ser His Glu Arg Leu Thr Thr Leu Glu Asp Thr Val Asp
   8330 8335 8340
   Thr Glu Asp Met Gln Pro Ser Thr His Thr Ala Val Thr Asn Val
   8345 8350 8355
   Arg Thr Ser Ile Ser Gly His Glu Ser Gln Ser Ser Val Leu Ser
   8360 8365 8370
   Asp Ser Glu Thr Pro Lys Ala Thr Ser Pro Met Gly Thr Thr Tyr
   8375 8380 8385
   Thr Met Gly Glu Thr Ser Val Ser Ile Ser Thr Ser Asp Phe Phe
   8390 8395 8400
   Glu Thr Ser Arg Ile Gln Ile Glu Pro Thr Ser Ser Leu Thr Ser
   8405 8410 8415
   Gly Leu Arg Glu Thr Ser Ser Ser Glu Arg Ile Ser Ser Ala Thr
   8420 8425 8430
   Glu Gly Ser Thr Val Leu Ser Glu Val Pro Ser Gly Ala Thr Thr
   8435 8440 8445
   Glu Val Ser Arg Thr Glu Val Ile Ser Ser Arg Gly Thr Ser Met
   8450 8455 8460
   Ser Gly Pro Asp Gln Phe Thr Ile Ser Pro Asp Ile Ser Thr Glu
   8465 8470 8475
   Ala Ile Thr Arg Leu Ser Thr Ser Pro Ile Met Thr Glu Ser Ala
   8480 8485 8490
   Glu Ser Ala Ile Thr Ile Glu Thr Gly Ser Pro Gly Ala Thr Ser
   8495 8500 8505
   Glu Gly Thr Leu Thr Leu Asp Thr Ser Thr Thr Thr Phe Trp Ser
   8510 8515 8520
   Gly Thr His Ser Thr Ala Ser Pro Gly Phe Ser His Ser Glu Met
   8525 8530 8535
   Thr Thr Leu Met Ser Arg Thr Pro Gly Asp Val Pro Trp Pro Ser
   8540 8545 8550
   Leu Pro Ser Val Glu Glu Ala Ser Ser Val Ser Ser Ser Leu Ser
   8555 8560 8565
   Ser Pro Ala Met Thr Ser Thr Ser Phe Phe Ser Ala Leu Pro Glu
   8570 8575 8580
   Ser Ile Ser Ser Ser Pro His Pro Val Thr Ala Leu Leu Thr Leu
   8585 8590 8595
   Gly Pro Val Lys Thr Thr Asp Met Leu Arg Thr Ser Ser Glu Pro
   8600 8605 8610
   Glu Thr Ser Ser Pro Pro Asn Leu Ser Ser Thr Ser Ala Glu Ile
   8615 8620 8625
   Leu Ala Thr Ser Glu Val Thr Lys Asp Arg Glu Lys Ile His Pro
   8630 8635 8640
   Ser Ser Asn Thr Pro Val Val Asn Val Gly Thr Val Ile Tyr Lys
   8645 8650 8655
   His Leu Ser Pro Ser Ser Val Leu Ala Asp Leu Val Thr Thr Lys
   8660 8665 8670
   Pro Thr Ser Pro Met Ala Thr Thr Ser Thr Leu Gly Asn Thr Ser
   8675 8680 8685
   Val Ser Thr Ser Thr Pro Ala Phe Pro Glu Thr Met Met Thr Gln
   8690 8695 8700
   Pro Thr Ser Ser Leu Thr Ser Gly Leu Arg Glu Ile Ser Thr Ser
   8705 8710 8715
   Gln Glu Thr Ser Ser Ala Thr Glu Arg Ser Ala Ser Leu Ser Gly
   8720 8725 8730
   Met Pro Thr Gly Ala Thr Thr Lys Val Ser Arg Thr Glu Ala Leu
   8735 8740 8745
   Ser Leu Gly Arg Thr Ser Thr Pro Gly Pro Ala Gln Ser Thr Ile
   8750 8755 8760
   Ser Pro Glu Ile Ser Thr Glu Thr Ile Thr Arg Ile Ser Thr Pro
   8765 8770 8775
   Leu Thr Thr Thr Gly Ser Ala Glu Met Thr Ile Thr Pro Lys Thr
   8780 8785 8790
   Gly His Ser Gly Ala Ser Ser Gln Gly Thr Phe Thr Leu Asp Thr
   8795 8800 8805
   Ser Ser Arg Ala Ser Trp Pro Gly Thr His Ser Ala Ala Thr His
   8810 8815 8820
   Arg Ser Pro His Ser Gly Met Thr Thr Pro Met Ser Arg Gly Pro
   8825 8830 8835
   Glu Asp Val Ser Trp Pro Ser Arg Pro Ser Val Glu Lys Thr Ser
   8840 8845 8850
   Pro Pro Ser Ser Leu Val Ser Leu Ser Ala Val Thr Ser Pro Ser
   8855 8860 8865
   Pro Leu Tyr Ser Thr Pro Ser Glu Ser Ser His Ser Ser Pro Leu
   8870 8875 8880
   Arg Val Thr Ser Leu Phe Thr Pro Val Met Met Lys Thr Thr Asp
   8885 8890 8895
   Met Leu Asp Thr Ser Leu Glu Pro Val Thr Thr Ser Pro Pro Ser
   8900 8905 8910
   Met Asn Ile Thr Ser Asp Glu Ser Leu Ala Thr Ser Lys Ala Thr
   8915 8920 8925
   Met Glu Thr Glu Ala Ile Gln Leu Ser Glu Asn Thr Ala Val Thr
   8930 8935 8940
   Gln Met Gly Thr Ile Ser Ala Arg Gln Glu Phe Tyr Ser Ser Tyr
   8945 8950 8955
   Pro Gly Leu Pro Glu Pro Ser Lys Val Thr Ser Pro Val Val Thr
   8960 8965 8970
   Ser Ser Thr Ile Lys Asp Ile Val Ser Thr Thr Ile Pro Ala Ser
   8975 8980 8985
   Ser Glu Ile Thr Arg Ile Glu Met Glu Ser Thr Ser Thr Leu Thr
   8990 8995 9000
   Pro Thr Pro Arg Glu Thr Ser Thr Ser Gln Glu Ile His Ser Ala
   9005 9010 9015
   Thr Lys Pro Ser Thr Val Pro Tyr Lys Ala Leu Thr Ser Ala Thr
   9020 9025 9030
   Ile Glu Asp Ser Met Thr Gln Val Met Ser Ser Ser Arg Gly Pro
   9035 9040 9045
   Ser Pro Asp Gln Ser Thr Met Ser Gln Asp Ile Ser Ser Glu Val
   9050 9055 9060
   Ile Thr Arg Leu Ser Thr Ser Pro Ile Lys Ala Glu Ser Thr Glu
   9065 9070 9075
   Met Thr Ile Thr Thr Gln Thr Gly Ser Pro Gly Ala Thr Ser Arg
   9080 9085 9090
   Gly Thr Leu Thr Leu Asp Thr Ser Thr Thr Phe Met Ser Gly Thr
   9095 9100 9105
   His Ser Thr Ala Ser Gln Gly Phe Ser His Ser Gln Met Thr Ala
   9110 9115 9120
   Leu Met Ser Arg Thr Pro Gly Asp Val Pro Trp Leu Ser His Pro
   9125 9130 9135
   Ser Val Glu Glu Ala Ser Ser Ala Ser Phe Ser Leu Ser Ser Pro
   9140 9145 9150
   Val Met Thr Ser Ser Ser Pro Val Ser Ser Thr Leu Pro Asp Ser
   9155 9160 9165
   Ile His Ser Ser Ser Leu Pro Val Thr Ser Leu Leu Thr Ser Gly
   9170 9175 9180
   Leu Val Lys Thr Thr Glu Leu Leu Gly Thr Ser Ser Glu Pro Glu
   9185 9190 9195
   Thr Ser Ser Pro Pro Asn Leu Ser Ser Thr Ser Ala Glu Ile Leu
   9200 9205 9210
   Ala Thr Thr Glu Val Thr Thr Asp Thr Glu Lys Leu Glu Met Thr
   9215 9220 9225
   Asn Val Val Thr Ser Gly Tyr Thr His Glu Ser Pro Ser Ser Val
   9230 9235 9240
   Leu Ala Asp Ser Val Thr Thr Lys Ala Thr Ser Ser Met Gly Ile
   9245 9250 9255
   Thr Tyr Pro Thr Gly Asp Thr Asn Val Leu Thr Ser Thr Pro Ala
   9260 9265 9270
   Phe Ser Asp Thr Ser Arg Ile Gln Thr Lys Ser Lys Leu Ser Leu
   9275 9280 9285
   Thr Pro Gly Leu Met Glu Thr Ser Ile Ser Glu Glu Thr Ser Ser
   9290 9295 9300
   Ala Thr Glu Lys Ser Thr Val Leu Ser Ser Val Pro Thr Gly Ala
   9305 9310 9315
   Thr Thr Glu Val Ser Arg Thr Glu Ala Ile Ser Ser Ser Arg Thr
   9320 9325 9330
   Ser Ile Pro Gly Pro Ala Gln Ser Thr Met Ser Ser Asp Thr Ser
   9335 9340 9345
   Met Glu Thr Ile Thr Arg Ile Ser Thr Pro Leu Thr Arg Lys Glu
   9350 9355 9360
   Ser Thr Asp Met Ala Ile Thr Pro Lys Thr Gly Pro Ser Gly Ala
   9365 9370 9375
   Thr Ser Gln Gly Thr Phe Thr Leu Asp Ser Ser Ser Thr Ala Ser
       9380                9385                9390
   Trp Pro Gly Thr His Ser Ala Thr Thr Gln Arg Phe Pro Gln Ser
       9395                9400                9405
   Val Val Thr Thr Pro Met Ser Arg Gly Pro Glu Asp Val Ser Trp
       9410                9415                9420
   Pro Ser Pro Leu Ser Val Glu Lys Asn Ser Pro Pro Ser Ser Leu
       9425                9430                9435
   Val Ser Ser Ser Ser Val Thr Ser Pro Ser Pro Leu Tyr Ser Thr
       9440                9445                9450
   Pro Ser Gly Ser Ser His Ser Ser Pro Val Pro Val Thr Ser Leu
       9455                9460                9465
   Phe Thr Ser Ile Met Met Lys Ala Thr Asp Met Leu Asp Ala Ser
       9470                9475                9480
   Leu Glu Pro Glu Thr Thr Ser Ala Pro Asn Met Asn Ile Thr Ser
       9485                9490                9495
   Asp Glu Ser Leu Ala Thr Ser Lys Ala Thr Thr Glu Thr Glu Ala
       9500                9505                9510
   Ile His Val Phe Glu Asn Thr Ala Ala Ser His Val Glu Thr Thr
       9515                9520                9525
   Ser Ala Thr Glu Glu Leu Tyr Ser Ser Ser Pro Gly Phe Ser Glu
       9530                9535                9540
   Pro Thr Lys Val Ile Ser Pro Val Val Thr Ser Ser Ser Ile Arg
       9545                9550                9555
   Asp Asn Met Val Ser Thr Thr Met Pro Gly Ser Ser Gly Ile Thr
       9560                9565                9570
   Arg Ile Glu Ile Glu Ser Met Ser Ser Leu Thr Pro Gly Leu Arg
       9575                9580                9585
   Glu Thr Arg Thr Ser Gln Asp Ile Thr Ser Ser Thr Glu Thr Ser
       9590                9595                9600
   Thr Val Leu Tyr Lys Met Ser Ser Gly Ala Thr Pro Glu Val Ser
   9605 9610 9615
   Arg Thr Glu Val Met Pro Ser Ser Arg Thr Ser Ile Pro Gly Pro
   9620 9625 9630
   Ala Gln Ser Thr Met Ser Leu Asp Ile Ser Asp Glu Val Val Thr
   9635 9640 9645
   Arg Leu Ser Thr Ser Pro Ile Met Thr Glu Ser Ala Glu Ile Thr
   9650 9655 9660
   Ile Thr Thr Gln Thr Gly Tyr Ser Leu Ala Thr Ser Gln Val Thr
   9665 9670 9675
   Leu Pro Leu Gly Thr Ser Met Thr Phe Leu Ser Gly Thr His Ser
   9680 9685 9690
   Thr Met Ser Gln Gly Leu Ser His Ser Glu Met Thr Asn Leu Met
   9695 9700 9705
   Ser Arg Gly Pro Glu Ser Leu Ser Trp Thr Ser Pro Arg Phe Val
   9710 9715 9720
   Glu Thr Thr Arg Ser Ser Ser Ser Leu Thr Ser Leu Pro Leu Thr
   9725 9730 9735
   Thr Ser Leu Ser Pro Val Ser Ser Thr Leu Leu Asp Ser Ser Pro
   9740 9745 9750
   Ser Ser Pro Leu Pro Val Thr Ser Leu Ile Leu Pro Gly Leu Val
   9755 9760 9765
   Lys Thr Thr Glu Val Leu Asp Thr Ser Ser Glu Pro Lys Thr Ser
   9770 9775 9780
   Ser Ser Pro Asn Leu Ser Ser Thr Ser Val Glu Ile Pro Ala Thr
   9785 9790 9795
   Ser Glu Ile Met Thr Asp Thr Glu Lys Ile His Pro Ser Ser Asn
   9800 9805 9810
   Thr Ala Val Ala Lys Val Arg Thr Ser Ser Ser Val His Glu Ser
   9815 9820 9825
   His Ser Ser Val Leu Ala Asp Ser Glu Thr Thr Ile Thr Ile Pro
   9830 9835 9840
   Ser Met  Gly Ile Thr Ser Ala  Val Asp Asp Thr Thr  Val Phe Thr
       9845                 9850                 9855
   Ser Asn  Pro Ala Phe Ser Glu  Thr Arg Arg Ile Pro  Thr Glu Pro
       9860                 9865                 9870
   Thr Phe  Ser Leu Thr Pro Gly  Phe Arg Glu Thr Ser  Thr Ser Glu
       9875                 9880                 9885
   Glu Thr  Thr Ser Ile Thr Glu  Thr Ser Ala Val Leu  Tyr Gly Val
       9890                 9895                 9900
   Pro Thr  Ser Ala Thr Thr Glu  Val Ser Met Thr Glu  Ile Met Ser
       9905                 9910                 9915
   Ser Asn  Arg Thr His Ile Pro  Asp Ser Asp Gln Ser  Thr Met Ser
       9920                 9925                 9930
   Pro Asp  Ile Ile Thr Glu Val  Ile Thr Arg Leu Ser  Ser Ser Ser
       9935                 9940                 9945
   Met Met  Ser Glu Ser Thr Gln  Met Thr Ile Thr Thr  Gln Lys Ser
       9950                 9955                 9960
   Ser Pro  Gly Ala Thr Ala Gln  Ser Thr Leu Thr Leu  Ala Thr Thr
       9965                 9970                 9975
   Thr Ala  Pro Leu Ala Arg Thr  His Ser Thr Val Pro  Pro Arg Phe
       9980                 9985                 9990
   Leu His  Ser Glu Met Thr Thr   Leu Met Ser Arg Ser   Pro Glu Asn
       9995                 10000                 10005
   Pro Ser   Trp Lys Ser Ser Pro   Phe Val Glu Lys Thr   Ser Ser Ser
       10010                 10015                 10020
   Ser Ser   Leu Leu Ser Leu Pro   Val Thr Thr Ser Pro   Ser Val Ser
       10025                 10030                 10035
   Ser Thr   Leu Pro Gln Ser Ile   Pro Ser Ser Ser Phe   Ser Val Thr
       10040                 10045                 10050
   Ser Leu   Leu Thr Pro Gly Met   Val Lys Thr Thr Asp   Thr Ser Thr
       10055                 10060                 10065
   Glu Pro   Gly Thr Ser Leu Ser   Pro Asn Leu Ser Gly   Thr Ser Val
       10070                 10075                 10080
   Glu Ile   Leu Ala Ala Ser Glu   Val Thr Thr Asp Thr   Glu Lys Ile
       10085                 10090                 10095
   His Pro   Ser Ser Ser Met Ala   Val Thr Asn Val Gly   Thr Thr Ser
       10100                 10105                 10110
   Ser Gly   His Glu Leu Tyr Ser   Ser Val Ser Ile His   Ser Glu Pro
       10115                 10120                 10125
   Ser Lys   Ala Thr Tyr Pro Val   Gly Thr Pro Ser Ser   Met Ala Glu
       10130                 10135                 10140
   Thr Ser   Ile Ser Thr Ser Met   Pro Ala Asn Phe Glu   Thr Thr Gly
       10145                 10150                 10155
   Phe Glu   Ala Glu Pro Phe Ser   His Leu Thr Ser Gly   Phe Arg Lys
       10160                 10165                 10170
   Thr Asn   Met Ser Leu Asp Thr   Ser Ser Val Thr Pro   Thr Asn Thr
       10175                 10180                 10185
   Pro Ser   Ser Pro Gly Ser Thr   His Leu Leu Gln Ser   Ser Lys Thr
       10190                 10195                 10200
   Asp Phe   Thr Ser Ser Ala Lys   Thr Ser Ser Pro Asp   Trp Pro Pro
       10205                 10210                 10215
   Ala Ser   Gln Tyr Thr Glu Ile   Pro Val Asp Ile Ile   Thr Pro Phe
       10220                 10225                 10230
   Asn Ala   Ser Pro Ser Ile Thr   Glu Ser Thr Gly Ile   Thr Ser Phe
       10235                 10240                 10245
   Pro Glu   Ser Arg Phe Thr Met   Ser Val Thr Glu Ser   Thr His His
       10250                 10255                 10260
   Leu Ser   Thr Asp Leu Leu Pro   Ser Ala Glu Thr Ile   Ser Thr Gly
       10265                 10270                 10275
   Thr Val   Met Pro Ser Leu Ser   Glu Ala Met Thr Ser   Phe Ala Thr
       10280                 10285                 10290
   Thr Gly   Val Pro Arg Ala Ile   Ser Gly Ser Gly Ser   Pro Phe Ser
       10295                 10300                 10305
   Arg Thr   Glu Ser Gly Pro Gly   Asp Ala Thr Leu Ser   Thr Ile Ala
       10310                 10315                 10320
   Glu Ser Leu Pro Ser Ser Thr Pro Val Pro Phe Ser Ser Ser Thr
   10325 10330 10335
   Phe Thr Thr Thr Asp Ser Ser Thr Ile Pro Ala Leu His Glu Ile
   10340 10345 10350
   Thr Ser Ser Ser Ala Thr Pro Tyr Arg Val Asp Thr Ser Leu Gly
   10355 10360 10365
   Thr Glu Ser Ser Thr Thr Glu Gly Arg Leu Val Met Val Ser Thr
   10370 10375 10380
   Leu Asp Thr Ser Ser Gln Pro Gly Arg Thr Ser Ser Thr Pro Ile
   10385 10390 10395
   Leu Asp Thr Arg Met Thr Glu Ser Val Glu Leu Gly Thr Val Thr
   10400 10405 10410
   Ser Ala Tyr Gln Val Pro Ser Leu Ser Thr Arg Leu Thr Arg Thr
   10415 10420 10425
   Asp Gly Ile Met Glu His Ile Thr Lys Ile Pro Asn Glu Ala Ala
   10430 10435 10440
   His Arg Gly Thr Ile Arg Pro Val Lys Gly Pro Gln Thr Ser Thr
   10445 10450 10455
   Ser Pro Ala Ser Pro Lys Gly Leu His Thr Gly Gly Thr Lys Arg
   10460 10465 10470
   Met Glu Thr Thr Thr Thr Ala Leu Lys Thr Thr Thr Thr Ala Leu
   10475 10480 10485
   Lys Thr Thr Ser Arg Ala Thr Leu Thr Thr Ser Val Tyr Thr Pro
   10490 10495 10500
   Thr Leu Gly Thr Leu Thr Pro Leu Asn Ala Ser Arg Gln Met Ala
   10505 10510 10515
   Ser Thr Ile Leu Thr Glu Met Met Ile Thr Thr Pro Tyr Val Phe
   10520 10525 10530
   Pro Asp Val Pro Glu Thr Thr Ser Ser Leu Ala Thr Ser Leu Gly
   10535 10540 10545
   Ala Glu Thr Ser Thr Ala Leu Pro Arg Thr Thr Pro Ser Val Leu
   10550 10555 10560
   Asn Arg Glu Ser Glu Thr Thr Ala Ser Leu Val Ser Arg Ser Gly
   10565 10570 10575
   Ala Glu Arg Ser Pro Val Ile Gln Thr Leu Asp Val Ser Ser Ser
   10580 10585 10590
   Glu Pro Asp Thr Thr Ala Ser Trp Val Ile His Pro Ala Glu Thr
   10595 10600 10605
   Ile Pro Thr Val Ser Lys Thr Thr Pro Asn Phe Phe His Ser Glu
   10610 10615 10620
   Leu Asp Thr Val Ser Ser Thr Ala Thr Ser His Gly Ala Asp Val
   10625 10630 10635
   Ser Ser Ala Ile Pro Thr Asn Ile Ser Pro Ser Glu Leu Asp Ala
   10640 10645 10650
   Leu Thr Pro Leu Val Thr Ile Ser Gly Thr Asp Thr Ser Thr Thr
   10655 10660 10665
   Phe Pro Thr Leu Thr Lys Ser Pro His Glu Thr Glu Thr Arg Thr
   10670 10675 10680
   Thr Trp Leu Thr His Pro Ala Glu Thr Ser Ser Thr Ile Pro Arg
   10685 10690 10695
   Thr Ile Pro Asn Phe Ser His His Glu Ser Asp Ala Thr Pro Ser
   10700 10705 10710
   Ile Ala Thr Ser Pro Gly Ala Glu Thr Ser Ser Ala Ile Pro Ile
   10715 10720 10725
   Met Thr Val Ser Pro Gly Ala Glu Asp Leu Val Thr Ser Gln Val
   10730 10735 10740
   Thr Ser Ser Gly Thr Asp Arg Asn Met Thr Ile Pro Thr Leu Thr
   10745 10750 10755
   Leu Ser Pro Gly Glu Pro Lys Thr Ile Ala Ser Leu Val Thr His
   10760 10765 10770
   Pro Glu Ala Gln Thr Ser Ser Ala Ile Pro Thr Ser Thr Ile Ser
   10775 10780 10785
   Pro Ala Val Ser Arg Leu Val Thr Ser Met Val Thr Ser Leu Ala
   10790 10795 10800
   Ala Lys Thr Ser Thr Thr Asn Arg Ala Leu Thr Asn Ser Pro Gly
   10805 10810 10815
   Glu Pro Ala Thr Thr Val Ser Leu Val Thr His Pro Ala Gln Thr
   10820 10825 10830
   Ser Pro Thr Val Pro Trp Thr Thr Ser Ile Phe Phe His Ser Lys
   10835 10840 10845
   Ser Asp Thr Thr Pro Ser Met Thr Thr Ser His Gly Ala Glu Ser
   10850 10855 10860
   Ser Ser Ala Val Pro Thr Pro Thr Val Ser Thr Glu Val Pro Gly
   10865 10870 10875
   Val Val Thr Pro Leu Val Thr Ser Ser Arg Ala Val Ile Ser Thr
   10880 10885 10890
   Thr Ile Pro Ile Leu Thr Leu Ser Pro Gly Glu Pro Glu Thr Thr
   10895 10900 10905
   Pro Ser Met Ala Thr Ser His Gly Glu Glu Ala Ser Ser Ala Ile
   10910 10915 10920
   Pro Thr Pro Thr Val Ser Pro Gly Val Pro Gly Val Val Thr Ser
   10925 10930 10935
   Leu Val Thr Ser Ser Arg Ala Val Thr Ser Thr Thr Ile Pro Ile
   10940 10945 10950
   Leu Thr Phe Ser Leu Gly Glu Pro Glu Thr Thr Pro Ser Met Ala
   10955 10960 10965
   Thr Ser His Gly Thr Glu Ala Gly Ser Ala Val Pro Thr Val Leu
   10970 10975 10980
   Pro Glu Val Pro Gly Met Val Thr Ser Leu Val Ala Ser Ser Arg
   10985 10990 10995
   Ala Val Thr Ser Thr Thr Leu Pro Thr Leu Thr Leu Ser Pro Gly
   11000 11005 11010
   Glu Pro Glu Thr Thr Pro Ser Met Ala Thr Ser His Gly Ala Glu
   11015 11020 11025
   Ala Ser Ser Thr Val Pro Thr Val Ser Pro Glu Val Pro Gly Val
   11030 11035 11040
   Val Thr Ser Leu Val Thr Ser Ser Ser Gly Val Asn Ser Thr Ser
   11045 11050 11055
   Ile Pro Thr Leu Ile Leu Ser Pro Gly Glu Leu Glu Thr Thr Pro
   11060 11065 11070
   Ser Met Ala Thr Ser His Gly Ala Glu Ala Ser Ser Ala Val Pro
   11075 11080 11085
   Thr Pro Thr Val Ser Pro Gly Val Ser Gly Val Val Thr Pro Leu
   11090 11095 11100
   Val Thr Ser Ser Arg Ala Val Thr Ser Thr Thr Ile Pro Ile Leu
   11105 11110 11115
   Thr Leu Ser Ser Ser Glu Pro Glu Thr Thr Pro Ser Met Ala Thr
   11120 11125 11130
   Ser His Gly Val Glu Ala Ser Ser Ala Val Leu Thr Val Ser Pro
   11135 11140 11145
   Glu Val Pro Gly Met Val Thr Ser Leu Val Thr Ser Ser Arg Ala
   11150 11155 11160
   Val Thr Ser Thr Thr Ile Pro Thr Leu Thr Ile Ser Ser Asp Glu
   11165 11170 11175
   Pro Glu Thr Thr Thr Ser Leu Val Thr His Ser Glu Ala Lys Met
   11180 11185 11190
   Ile Ser Ala Ile Pro Thr Leu Ala Val Ser Pro Thr Val Gln Gly
   11195 11200 11205
   Leu Val Thr Ser Leu Val Thr Ser Ser Gly Ser Glu Thr Ser Ala
   11210 11215 11220
   Phe Ser Asn Leu Thr Val Ala Ser Ser Gln Pro Glu Thr Ile Asp
   11225 11230 11235
   Ser Trp Val Ala His Pro Gly Thr Glu Ala Ser Ser Val Val Pro
   11240 11245 11250
   Thr Leu Thr Val Ser Thr Gly Glu Pro Phe Thr Asn Ile Ser Leu
   11255 11260 11265
   Val Thr His Pro Ala Glu Ser Ser Ser Thr Leu Pro Arg Thr Thr
   11270 11275 11280
   Ser Arg Phe Ser His Ser Glu Leu Asp Thr Met Pro Ser Thr Val
   11285 11290 11295
   Thr Ser Pro Glu Ala Glu Ser Ser Ser Ala Ile Ser Thr Thr Ile
   11300 11305 11310
   Ser Pro Gly Ile Pro Gly Val Leu Thr Ser Leu Val Thr Ser Ser
   11315 11320 11325
   Gly Arg Asp Ile Ser Ala Thr Phe Pro Thr Val Pro Glu Ser Pro
   11330 11335 11340
   His Glu Ser Glu Ala Thr Ala Ser Trp Val Thr His Pro Ala Val
   11345 11350 11355
   Thr Ser Thr Thr Val Pro Arg Thr Thr Pro Asn Tyr Ser His Ser
   11360 11365 11370
   Glu Pro Asp Thr Thr Pro Ser Ile Ala Thr Ser Pro Gly Ala Glu
   11375 11380 11385
   Ala Thr Ser Asp Phe Pro Thr Ile Thr Val Ser Pro Asp Val Pro
   11390 11395 11400
   Asp Met Val Thr Ser Gln Val Thr Ser Ser Gly Thr Asp Thr Ser
   11405 11410 11415
   Ile Thr Ile Pro Thr Leu Thr Leu Ser Ser Gly Glu Pro Glu Thr
   11420 11425 11430
   Thr Thr Ser Phe Ile Thr Tyr Ser Glu Thr His Thr Ser Ser Ala
   11435 11440 11445
   Ile Pro Thr Leu Pro Val Ser Pro Gly Ala Ser Lys Met Leu Thr
   11450 11455 11460
   Ser Leu Val Ile Ser Ser Gly Thr Asp Ser Thr Thr Thr Phe Pro
   11465 11470 11475
   Thr Leu Thr Glu Thr Pro Tyr Glu Pro Glu Thr Thr Ala Ile Gln
   11480 11485 11490
   Leu Ile His Pro Ala Glu Thr Asn Thr Met Val Pro Lys Thr Thr
   11495 11500 11505
   Pro Lys Phe Ser His Ser Lys Ser Asp Thr Thr Leu Pro Val Ala
   11510 11515 11520
   Ile Thr Ser Pro Gly Pro Glu Ala Ser Ser Ala Val Ser Thr Thr
   11525 11530 11535
   Thr Ile Ser Pro Asp Met Ser Asp Leu Val Thr Ser Leu Val Pro
   11540 11545 11550
   Ser Ser Gly Thr Asp Thr Ser Thr Thr Phe Pro Thr Leu Ser Glu
   11555 11560 11565
   Thr Pro Tyr Glu Pro Glu Thr Thr Val Thr Trp Leu Thr His Pro
   11570 11575 11580
   Ala Glu Thr Ser Thr Thr Val Ser Gly Thr Ile Pro Asn Phe Ser
   11585 11590 11595
   His Arg Gly Ser Asp Thr Ala Pro Ser Met Val Thr Ser Pro Gly
   11600 11605 11610
   Val Asp Thr Arg Ser Gly Val Pro Thr Thr Thr Ile Pro Pro Ser
   11615 11620 11625
   Ile Pro Gly Val Val Thr Ser Gln Val Thr Ser Ser Ala Thr Asp
   11630 11635 11640
   Thr Ser Thr Ala Ile Pro Thr Leu Thr Pro Ser Pro Gly Glu Pro
   11645 11650 11655
   Glu Thr Thr Ala Ser Ser Ala Thr His Pro Gly Thr Gln Thr Gly
   11660 11665 11670
   Phe Thr Val Pro Ile Arg Thr Val Pro Ser Ser Glu Pro Asp Thr
   11675 11680 11685
   Met Ala Ser Trp Val Thr His Pro Pro Gln Thr Ser Thr Pro Val
   11690 11695 11700
   Ser Arg Thr Thr Ser Ser Phe Ser His Ser Ser Pro Asp Ala Thr
   11705 11710 11715
   Pro Val Met Ala Thr Ser Pro Arg Thr Glu Ala Ser Ser Ala Val
   11720 11725 11730
   Leu Thr Thr Ile Ser Pro Gly Ala Pro Glu Met Val Thr Ser Gln
       11735               11740               11745
   Ile Thr Ser Ser Gly Ala Ala Thr Ser Thr Thr Val Pro Thr Leu
       11750               11755               11760
   Thr His Ser Pro Gly Met Pro Glu Thr Thr Ala Leu Leu Ser Thr
       11765               11770               11775
   His Pro Arg Thr Gly Thr Ser Lys Thr Phe Pro Ala Ser Thr Val
       11780               11785               11790
   Phe Pro Gln Val Ser Glu Thr Thr Ala Ser Leu Thr Ile Arg Pro
       11795               11800               11805
   Gly Ala Glu Thr Ser Thr Ala Leu Pro Thr Gln Thr Thr Ser Ser
       11810               11815               11820
   Leu Phe Thr Leu Leu Val Thr Gly Thr Ser Arg Val Asp Leu Ser
       11825               11830               11835
   Pro Thr Ala Ser Pro Gly Val Ser Ala Lys Thr Ala Pro Leu Ser
       11840               11845               11850
   Thr His Pro Gly Thr Glu Thr Ser Thr Met Ile Pro Thr Ser Thr
       11855               11860               11865
   Leu Ser Leu Gly Leu Leu Glu Thr Thr Gly Leu Leu Ala Thr Ser
       11870               11875               11880
   Ser Ser Ala Glu Thr Ser Thr Ser Thr Leu Thr Leu Thr Val Ser
       11885               11890               11895
   Pro Ala Val Ser Gly Leu Ser Ser Ala Ser Ile Thr Thr Asp Lys
       11900               11905               11910
   Pro Gln Thr Val Thr Ser Trp Asn Thr Glu Thr Ser Pro Ser Val
       11915               11920               11925
   Thr Ser Val Gly Pro Pro Glu Phe Ser Arg Thr Val Thr Gly Thr
       11930               11935               11940
   Thr Met Thr Leu Ile Pro Ser Glu Met Pro Thr Pro Pro Lys Thr
       11945               11950               11955
   Ser His Gly Glu Gly Val Ser Pro Thr Thr Ile Leu Arg Thr Thr
       11960               11965               11970
   Met Val Glu Ala Thr Asn Leu Ala Thr Thr Gly Ser Ser Pro Thr
   11975 11980 11985
   Val Ala Lys Thr Thr Thr Thr Phe Asn Thr Leu Ala Gly Ser Leu
   11990 11995 12000
   Phe Thr Pro Leu Thr Thr Pro Gly Met Ser Thr Leu Ala Ser Glu
   12005 12010 12015
   Ser Val Thr Ser Arg Thr Ser Tyr Asn His Arg Ser Trp Ile Ser
   12020 12025 12030
   Thr Thr Ser Ser Tyr Asn Arg Arg Tyr Trp Thr Pro Ala Thr Ser
   12035 12040 12045
   Thr Pro Val Thr Ser Thr Phe Ser Pro Gly Ile Ser Thr Ser Ser
   12050 12055 12060
   Ile Pro Ser Ser Thr Ala Ala Thr Val Pro Phe Met Val Pro Phe
   12065 12070 12075
   Thr Leu Asn Phe Thr Ile Thr Asn Leu Gln Tyr Glu Glu Asp Met
   12080 12085 12090
   Arg His Pro Gly Ser Arg Lys Phe Asn Ala Thr Glu Arg Glu Leu
   12095 12100 12105
   Gln Gly Leu Leu Lys Pro Leu Phe Arg Asn Ser Ser Leu Glu Tyr
   12110 12115 12120
   Leu Tyr Ser Gly Cys Arg Leu Ala Ser Leu Arg Pro Glu Lys Asp
   12125 12130 12135
   Ser Ser Ala Met Ala Val Asp Ala Ile Cys Thr His Arg Pro Asp
   12140 12145 12150
   Pro Glu Asp Leu Gly Leu Asp Arg Glu Arg Leu Tyr Trp Glu Leu
   12155 12160 12165
   Ser Asn Leu Thr Asn Gly Ile Gln Glu Leu Gly Pro Tyr Thr Leu
   12170 12175 12180
   Asp Arg Asn Ser Leu Tyr Val Asn Gly Phe Thr His Arg Ser Ser
   12185 12190 12195
   Met Pro Thr Thr Ser Thr Pro Gly Thr Ser Thr Val Asp Val Gly
   12200 12205 12210
   Thr Ser Gly Thr Pro Ser Ser Ser Pro Ser Pro Thr Ala Ala Gly
       12215               12220               12225
   Pro Leu Leu Met Pro Phe Thr Leu Asn Phe Thr Ile Thr Asn Leu
       12230               12235               12240
   Gln Tyr Glu Glu Asp Met Arg Arg Thr Gly Ser Arg Lys Phe Asn
       12245               12250               12255
   Thr Met Glu Ser Val Leu Gln Gly Leu Leu Lys Pro Leu Phe Lys
       12260               12265               12270
   Asn Thr Ser Val Gly Pro Leu Tyr Ser Gly Cys Arg Leu Thr Leu
       12275               12280               12285
   Leu Arg Pro Glu Lys Asp Gly Ala Ala Thr Gly Val Asp Ala Ile
       12290               12295               12300
   Cys Thr His Arg Leu Asp Pro Lys Ser Pro Gly Leu Asn Arg Glu
       12305               12310               12315
   Gln Leu Tyr Trp Glu Leu Ser Lys Leu Thr Asn Asp Ile Glu Glu
       12320               12325               12330
   Leu Gly Pro Tyr Thr Leu Asp Arg Asn Ser Leu Tyr Val Asn Gly
       12335               12340               12345
   Phe Thr His Gln Ser Ser Val Ser Thr Thr Ser Thr Pro Gly Thr
       12350               12355               12360
   Ser Thr Val Asp Leu Arg Thr Ser Gly Thr Pro Ser Ser Leu Ser
       12365               12370               12375
   Ser Pro Thr Ile Met Ala Ala Gly Pro Leu Leu Val Pro Phe Thr
       12380               12385               12390
   Leu Asn Phe Thr Ile Thr Asn Leu Gln Tyr Gly Glu Asp Met Gly
       12395               12400               12405
   His Pro Gly Ser Arg Lys Phe Asn Thr Thr Glu Arg Val Leu Gln
       12410               12415               12420
   Gly Leu Leu Gly Pro Ile Phe Lys Asn Thr Ser Val Gly Pro Leu
       12425               12430               12435
   Tyr Ser Gly Cys Arg Leu Thr Ser Leu Arg Ser Glu Lys Asp Gly
   12440 12445 12450
   Ala Ala Thr Gly Val Asp Ala Ile Cys Ile His His Leu Asp Pro
   12455 12460 12465
   Lys Ser Pro Gly Leu Asn Arg Glu Arg Leu Tyr Trp Glu Leu Ser
   12470 12475 12480
   Gln Leu Thr Asn Gly Ile Lys Glu Leu Gly Pro Tyr Thr Leu Asp
   12485 12490 12495
   Arg Asn Ser Leu Tyr Val Asn Gly Phe Thr His Arg Thr Ser Val
   12500 12505 12510
   Pro Thr Thr Ser Thr Pro Gly Thr Ser Thr Val Asp Leu Gly Thr
   12515 12520 12525
   Ser Gly Thr Pro Phe Ser Leu Pro Ser Pro Ala Thr Ala Gly Pro
   12530 12535 12540
   Leu Leu Val Leu Phe Thr Leu Asn Phe Thr Ile Thr Asn Leu Lys
   12545 12550 12555
   Tyr Glu Glu Asp Met His Arg Pro Gly Ser Arg Lys Phe Asn Thr
   12560 12565 12570
   Thr Glu Arg Val Leu Gln Thr Leu Leu Gly Pro Met Phe Lys Asn
   12575 12580 12585
   Thr Ser Val Gly Leu Leu Tyr Ser Gly Cys Arg Leu Thr Leu Leu
   12590 12595 12600
   Arg Ser Glu Lys Asp Gly Ala Ala Thr Gly Val Asp Ala Ile Cys
   12605 12610 12615
   Thr His Arg Leu Asp Pro Lys Ser Pro Gly Leu Asp Arg Glu Gln
   12620 12625 12630
   Leu Tyr Trp Glu Leu Ser Gln Leu Thr Asn Gly Ile Lys Glu Leu
   12635 12640 12645
   Gly Pro Tyr Thr Leu Asp Arg Asn Ser Leu Tyr Val Asn Gly Phe
   12650 12655 12660
   Thr His Trp Ile Pro Val Pro Thr Ser Ser Thr Pro Gly Thr Ser
   12665 12670 12675
   Thr Val Asp Leu Gly Ser Gly Thr Pro Ser Ser Leu Pro Ser Pro
   12680 12685 12690
   Thr Ala Ala Gly Pro Leu Leu Val Pro Phe Thr Leu Asn Phe Thr
   12695 12700 12705
   Ile Thr Asn Leu Gln Tyr Glu Glu Asp Met His His Pro Gly Ser
   12710 12715 12720
   Arg Lys Phe Asn Thr Thr Glu Arg Val Leu Gln Gly Leu Leu Gly
   12725 12730 12735
   Pro Met Phe Lys Asn Thr Ser Val Gly Leu Leu Tyr Ser Gly Cys
   12740 12745 12750
   Arg Leu Thr Leu Leu Arg Ser Glu Lys Asp Gly Ala Ala Thr Gly
   12755 12760 12765
   Val Asp Ala Ile Cys Thr His Arg Leu Asp Pro Lys Ser Pro Gly
   12770 12775 12780
   Val Asp Arg Glu Gln Leu Tyr Trp Glu Leu Ser Gln Leu Thr Asn
   12785 12790 12795
   Gly Ile Lys Glu Leu Gly Pro Tyr Thr Leu Asp Arg Asn Ser Leu
   12800 12805 12810
   Tyr Val Asn Gly Phe Thr His Gln Thr Ser Ala Pro Asn Thr Ser
   12815 12820 12825
   Thr Pro Gly Thr Ser Thr Val Asp Leu Gly Thr Ser Gly Thr Pro
   12830 12835 12840
   Ser Ser Leu Pro Ser Pro Thr Ser Ala Gly Pro Leu Leu Val Pro
   12845 12850 12855
   Phe Thr Leu Asn Phe Thr Ile Thr Asn Leu Gln Tyr Glu Glu Asp
   12860 12865 12870
   Met Arg His Pro Gly Ser Arg Lys Phe Asn Thr Thr Glu Arg Val
   12875 12880 12885
   Leu Gln Gly Leu Leu Lys Pro Leu Phe Lys Ser Thr Ser Val Gly
   12890 12895 12900
   Pro Leu Tyr Ser Gly Cys Arg Leu Thr Leu Leu Arg Ser Glu Lys
   12905 12910 12915
   Asp Gly Ala Ala Thr Gly Val Asp Ala Ile Cys Thr His Arg Leu
   12920 12925 12930
   Asp Pro Lys Ser Pro Gly Val Asp Arg Glu Gln Leu Tyr Trp Glu
   12935 12940 12945
   Leu Ser Gln Leu Thr Asn Gly Ile Lys Glu Leu Gly Pro Tyr Thr
   12950 12955 12960
   Leu Asp Arg Asn Ser Leu Tyr Val Asn Gly Phe Thr His Gln Thr
   12965 12970 12975
   Ser Ala Pro Asn Thr Ser Thr Pro Gly Thr Ser Thr Val Asp Leu
   12980 12985 12990
   Gly Thr Ser Gly Thr Pro Ser Ser Leu Pro Ser Pro Thr Ser Ala
   12995 13000 13005
   Gly Pro Leu Leu Val Pro Phe Thr Leu Asn Phe Thr Ile Thr Asn
   13010 13015 13020
   Leu Gln Tyr Glu Glu Asp Met His His Pro Gly Ser Arg Lys Phe
   13025 13030 13035
   Asn Thr Thr Glu Arg Val Leu Gln Gly Leu Leu Gly Pro Met Phe
   13040 13045 13050
   Lys Asn Thr Ser Val Gly Leu Leu Tyr Ser Gly Cys Arg Leu Thr
   13055 13060 13065
   Leu Leu Arg Pro Glu Lys Asn Gly Ala Ala Thr Gly Met Asp Ala
   13070 13075 13080
   Ile Cys Ser His Arg Leu Asp Pro Lys Ser Pro Gly Leu Asn Arg
   13085 13090 13095
   Glu Gln Leu Tyr Trp Glu Leu Ser Gln Leu Thr His Gly Ile Lys
   13100 13105 13110
   Glu Leu Gly Pro Tyr Thr Leu Asp Arg Asn Ser Leu Tyr Val Asn
   13115 13120 13125
   Gly Phe Thr His Arg Ser Ser Val Ala Pro Thr Ser Thr Pro Gly
   13130 13135 13140
   Thr Ser Thr Val Asp Leu Gly Thr Ser Gly Thr Pro Ser Ser Leu
   13145 13150 13155
   Pro Ser Pro Thr Thr Ala Val Pro Leu Leu Val Pro Phe Thr Leu
   13160 13165 13170
   Asn Phe Thr Ile Thr Asn Leu Gln Tyr Gly Glu Asp Met Arg His
   13175 13180 13185
   Pro Gly Ser Arg Lys Phe Asn Thr Thr Glu Arg Val Leu Gln Gly
   13190 13195 13200
   Leu Leu Gly Pro Leu Phe Lys Asn Ser Ser Val Gly Pro Leu Tyr
   13205 13210 13215
   Ser Gly Cys Arg Leu Ile Ser Leu Arg Ser Glu Lys Asp Gly Ala
   13220 13225 13230
   Ala Thr Gly Val Asp Ala Ile Cys Thr His His Leu Asn Pro Gln
   13235 13240 13245
   Ser Pro Gly Leu Asp Arg Glu Gln Leu Tyr Trp Gln Leu Ser Gln
   13250 13255 13260
   Met Thr Asn Gly Ile Lys Glu Leu Gly Pro Tyr Thr Leu Asp Arg
   13265 13270 13275
   Asn Ser Leu Tyr Val Asn Gly Phe Thr His Arg Ser Ser Gly Leu
   13280 13285 13290
   Thr Thr Ser Thr Pro Trp Thr Ser Thr Val Asp Leu Gly Thr Ser
   13295 13300 13305
   Gly Thr Pro Ser Pro Val Pro Ser Pro Thr Thr Ala Gly Pro Leu
   13310 13315 13320
   Leu Val Pro Phe Thr Leu Asn Phe Thr Ile Thr Asn Leu Gln Tyr
   13325 13330 13335
   Glu Glu Asp Met His Arg Pro Gly Ser Arg Lys Phe Asn Thr Thr
   13340 13345 13350
   Glu Arg Val Leu Gln Gly Leu Leu Ser Pro Ile Phe Lys Asn Ser
   13355 13360 13365
   Ser Val Gly Pro Leu Tyr Ser Gly Cys Arg Leu Thr Ser Leu Arg
   13370 13375 13380
   Pro Glu Lys Asp Gly Ala Ala Thr Gly Met Asp Ala Val Cys Leu
   13385 13390 13395
   Tyr His Pro Asn Pro Lys Arg Pro Gly Leu Asp Arg Glu Gln Leu
   13400 13405 13410
   Tyr Trp Glu Leu Ser Gln Leu Thr His Asn Ile Thr Glu Leu Gly
   13415 13420 13425
   Pro Tyr Ser Leu Asp Arg Asp Ser Leu Tyr Val Asn Gly Phe Thr
   13430 13435 13440
   His Gln Asn Ser Val Pro Thr Thr Ser Thr Pro Gly Thr Ser Thr
   13445 13450 13455
   Val Tyr Trp Ala Thr Thr Gly Thr Pro Ser Ser Phe Pro Gly His
   13460 13465 13470
   Thr Glu Pro Gly Pro Leu Leu Ile Pro Phe Thr Phe Asn Phe Thr
   13475 13480 13485
   Ile Thr Asn Leu His Tyr Glu Glu Asn Met Gln His Pro Gly Ser
   13490 13495 13500
   Arg Lys Phe Asn Thr Thr Glu Arg Val Leu Gln Gly Leu Leu Lys
   13505 13510 13515
   Pro Leu Phe Lys Asn Thr Ser Val Gly Pro Leu Tyr Ser Gly Cys
   13520 13525 13530
   Arg Leu Thr Ser Leu Arg Pro Glu Lys Asp Gly Ala Ala Thr Gly
   13535 13540 13545
   Met Asp Ala Val Cys Leu Tyr His Pro Asn Pro Lys Arg Pro Gly
   13550 13555 13560
   Leu Asp Arg Glu Gln Leu Tyr Trp Glu Leu Ser Gln Leu Thr His
   13565 13570 13575
   Asn Ile Thr Glu Leu Gly Pro Tyr Ser Leu Asp Arg Asp Ser Leu
   13580 13585 13590
   Tyr Val Asn Gly Phe Thr His Gln Asn Ser Val Pro Thr Thr Ser
   13595 13600 13605
   Thr Pro Gly Thr Ser Thr Val Tyr Trp Ala Thr Thr Gly Thr Pro
   13610 13615 13620
   Ser Ser Phe Pro Gly His Thr Glu Pro Gly Pro Leu Leu Ile Pro
   13625 13630 13635
   Phe Thr Phe Asn Phe Thr Ile Thr Asn Leu His Tyr Glu Glu Asn
   13640 13645 13650
   Met Gln His Pro Gly Ser Arg Lys Phe Asn Thr Thr Glu Arg Val
   13655 13660 13665
   Leu Gln Gly Leu Leu Lys Pro Leu Phe Lys Asn Thr Ser Val Gly
   13670 13675 13680
   Pro Leu Tyr Ser Gly Cys Arg Leu Thr Leu Leu Arg Pro Glu Lys
   13685 13690 13695
   His Glu Ala Ala Thr Gly Val Asp Thr Ile Cys Thr His Arg Val
   13700 13705 13710
   Asp Pro Ile Gly Pro Gly Leu Asp Arg Glu Arg Leu Tyr Trp Glu
   13715 13720 13725
   Leu Ser Gln Leu Thr Asn Ser Ile Thr Glu Leu Gly Pro Tyr Thr
   13730 13735 13740
   Leu Asp Arg Asp Ser Leu Tyr Val Asn Gly Phe Asn Pro Arg Ser
   13745 13750 13755
   Ser Val Pro Thr Thr Ser Thr Pro Gly Thr Ser Thr Val His Leu
   13760 13765 13770
   Ala Thr Ser Gly Thr Pro Ser Ser Leu Pro Gly His Thr Ala Pro
   13775 13780 13785
   Val Pro Leu Leu Ile Pro Phe Thr Leu Asn Phe Thr Ile Thr Asn
   13790 13795 13800
   Leu His Tyr Glu Glu Asn Met Gln His Pro Gly Ser Arg Lys Phe
   13805 13810 13815
   Asn Thr Thr Glu Arg Val Leu Gln Gly Leu Leu Lys Pro Leu Phe
   13820 13825 13830
   Lys Asn Thr Ser Val Gly Pro Leu Tyr Ser Gly Cys Arg Leu Thr
   13835 13840 13845
   Leu Leu Arg Pro Glu Lys His Glu Ala Ala Thr Gly Val Asp Thr
   13850 13855 13860
   Ile Cys Thr His Arg Val Asp Pro Ile Gly Pro Gly Leu Xaa Xaa
       13865               13870               13875
   Glu Xaa Leu Tyr Trp Glu Leu Ser Xaa Leu Thr Xaa Xaa Ile Xaa
       13880               13885               13890
   Glu Leu Gly Pro Tyr Thr Leu Asp Arg Xaa Ser Leu Tyr Val Asn
       13895               13900               13905
   Gly Phe Thr His Xaa Xaa Ser Xaa Pro Thr Thr Ser Thr Pro Gly
       13910               13915               13920
   Thr Ser Thr Val Xaa Xaa Gly Thr Ser Gly Thr Pro Ser Ser Xaa
       13925               13930               13935
   Pro Xaa Xaa Thr Ser Ala Gly Pro Leu Leu Val Pro Phe Thr Leu
       13940               13945               13950
   Asn Phe Thr Ile Thr Asn Leu Gln Tyr Glu Glu Asp Met His His
       13955               13960               13965
   Pro Gly Ser Arg Lys Phe Asn Thr Thr Glu Arg Val Leu Gln Gly
       13970               13975               13980
   Leu Leu Gly Pro Met Phe Lys Asn Thr Ser Val Gly Leu Leu Tyr
       13985               13990               13995
   Ser Gly Cys Arg Leu Thr Leu Leu Arg Pro Glu Lys Asn Gly Ala
       14000               14005               14010
   Ala Thr Gly Met Asp Ala Ile Cys Ser His Arg Leu Asp Pro Lys
       14015               14020               14025
   Ser Pro Gly Leu Asp Arg Glu Gln Leu Tyr Trp Glu Leu Ser Gln
       14030               14035               14040
   Leu Thr His Gly Ile Lys Glu Leu Gly Pro Tyr Thr Leu Asp Arg
       14045               14050               14055
   Asn Ser Leu Tyr Val Asn Gly Phe Thr His Arg Ser Ser Val Ala
       14060               14065               14070
   Pro Thr Ser Thr Pro Gly Thr Ser Thr Val Asp Leu Gly Thr Ser
       14075               14080               14085
   Gly Thr Pro Ser Ser Leu Pro Ser Pro Thr Thr Ala Val Pro Leu
       14090               14095               14100
   Leu Val Pro Phe Thr Leu Asn Phe Thr Ile Thr Asn Leu Gln Tyr
   14105 14110 14115
   Gly Glu Asp Met Arg His Pro Gly Ser Arg Lys Phe Asn Thr Thr
   14120 14125 14130
   Glu Arg Val Leu Gln Gly Leu Leu Gly Pro Leu Phe Lys Asn Ser
   14135 14140 14145
   Ser Val Gly Pro Leu Tyr Ser Gly Cys Arg Leu Ile Ser Leu Arg
   14150 14155 14160
   Ser Glu Lys Asp Gly Ala Ala Thr Gly Val Asp Ala Ile Cys Thr
   14165 14170 14175
   His His Leu Asn Pro Gln Ser Pro Gly Leu Asp Arg Glu Gln Leu
   14180 14185 14190
   Tyr Trp Gln Leu Ser Gln Met Thr Asn Gly Ile Lys Glu Leu Gly
   14195 14200 14205
   Pro Tyr Thr Leu Asp Arg Asn Ser Leu Tyr Val Asn Gly Phe Thr
   14210 14215 14220
   His Arg Ser Ser Gly Leu Thr Thr Ser Thr Pro Trp Thr Ser Thr
   14225 14230 14235
   Val Asp Leu Gly Thr Ser Gly Thr Pro Ser Pro Val Pro Ser Pro
   14240 14245 14250
   Thr Thr Ala Gly Pro Leu Leu Val Pro Phe Thr Leu Asn Phe Thr
   14255 14260 14265
   Ile Thr Asn Leu Gln Tyr Glu Glu Asp Met His Arg Pro Gly Ser
   14270 14275 14280
   Arg Lys Phe Asn Ala Thr Glu Arg Val Leu Gln Gly Leu Leu Ser
   14285 14290 14295
   Pro Ile Phe Lys Asn Ser Ser Val Gly Pro Leu Tyr Ser Gly Cys
   14300 14305 14310
   Arg Leu Thr Ser Leu Arg Pro Glu Lys Asp Gly Ala Ala Thr Gly
   14315 14320 14325
   Met Asp Ala Val Cys Leu Tyr His Pro Asn Pro Lys Arg Pro Gly
   14330 14335 14340
   Leu Asp Arg Glu Gln Leu Tyr Trp Glu Leu Ser Gln Leu Thr His
   14345 14350 14355
   Asn Ile Thr Glu Leu Gly Pro Tyr Ser Leu Asp Arg Asp Ser Leu
   14360 14365 14370
   Tyr Val Asn Gly Phe Thr His Gln Ser Ser Met Thr Thr Thr Arg
   14375 14380 14385
   Thr Pro Asp Thr Ser Thr Met His Leu Ala Thr Ser Arg Thr Pro
   14390 14395 14400
   Ala Ser Leu Ser Gly Pro Thr Thr Ala Ser Pro Leu Leu Val Leu
   14405 14410 14415
   Phe Thr Ile Asn Cys Thr Ile Thr Asn Leu Gln Tyr Glu Glu Asp
   14420 14425 14430
   Met Arg Arg Thr Gly Ser Arg Lys Phe Asn Thr Met Glu Ser Val
   14435 14440 14445
   Leu Gln Gly Leu Leu Lys Pro Leu Phe Lys Asn Thr Ser Val Gly
   14450 14455 14460
   Pro Leu Tyr Ser Gly Cys Arg Leu Thr Leu Leu Arg Pro Lys Lys
   14465 14470 14475
   Asp Gly Ala Ala Thr Gly Val Asp Ala Ile Cys Thr His Arg Leu
   14480 14485 14490
   Asp Pro Lys Ser Pro Gly Leu Asn Arg Glu Gln Leu Tyr Trp Glu
   14495 14500 14505
   Leu Ser Lys Leu Thr Asn Asp Ile Glu Glu Leu Gly Pro Tyr Thr
   14510 14515 14520
   Leu Asp Arg Asn Ser Leu Tyr Val Asn Gly Phe Thr His Gln Ser
   14525 14530 14535
   Ser Val Ser Thr Thr Ser Thr Pro Gly Thr Ser Thr Val Asp Leu
   14540 14545 14550
   Arg Thr Ser Gly Thr Pro Ser Ser Leu Ser Ser Pro Thr Ile Met
   14555 14560 14565
   Xaa Xaa Xaa Pro Leu Leu Xaa Pro Phe Thr Xaa Asn Xaa Thr Ile
   14570 14575 14580
   Thr Asn Leu Xaa Xaa Xaa Xaa Xaa Met Xaa Xaa Pro Gly Ser Arg
   14585 14590 14595
   Lys Phe Asn Thr Thr Glu Arg Val Leu Gln Gly Leu Leu Arg Pro
   14600 14605 14610
   Leu Phe Lys Asn Thr Ser Val Ser Ser Leu Tyr Ser Gly Cys Arg
   14615 14620 14625
   Leu Thr Leu Leu Arg Pro Glu Lys Asp Gly Ala Ala Thr Arg Val
   14630 14635 14640
   Asp Ala Ala Cys Thr Tyr Arg Pro Asp Pro Lys Ser Pro Gly Leu
   14645 14650 14655
   Asp Arg Glu Gln Leu Tyr Trp Glu Leu Ser Gln Leu Thr His Ser
   14660 14665 14670
   Ile Thr Glu Leu Gly Pro Tyr Thr Leu Asp Arg Val Ser Leu Tyr
   14675 14680 14685
   Val Asn Gly Phe Asn Pro Arg Ser Ser Val Pro Thr Thr Ser Thr
   14690 14695 14700
   Pro Gly Thr Ser Thr Val His Leu Ala Thr Ser Gly Thr Pro Ser
   14705 14710 14715
   Ser Leu Pro Gly His Thr Xaa Xaa Xaa Pro Leu Leu Xaa Pro Phe
   14720 14725 14730
   Thr Xaa Asn Xaa Thr Ile Thr Asn Leu Xaa Xaa Xaa Xaa Xaa Met
   14735 14740 14745
   Xaa Xaa Pro Gly Ser Arg Lys Phe Asn Thr Thr Glu Arg Val Leu
   14750 14755 14760
   Gln Gly Leu Leu Lys Pro Leu Phe Arg Asn Ser Ser Leu Glu Tyr
   14765 14770 14775
   Leu Tyr Ser Gly Cys Arg Leu Ala Ser Leu Arg Pro Glu Lys Asp
   14780 14785 14790
   Ser Ser Ala Met Ala Val Asp Ala Ile Cys Thr His Arg Pro Asp
   14795 14800 14805
   Pro Glu Asp Leu Gly Leu Asp Arg Glu Arg Leu Tyr Trp Glu Leu
   14810 14815 14820
   Ser Asn Leu Thr Asn Gly Ile Gln Glu Leu Gly Pro Tyr Thr Leu
   14825 14830 14835
   Asp Arg Asn Ser Leu Tyr Val Asn Gly Phe Thr His Arg Ser Ser
   14840 14845 14850
   Gly Leu Thr Thr Ser Thr Pro Trp Thr Ser Thr Val Asp Leu Gly
   14855 14860 14865
   Thr Ser Gly Thr Pro Ser Pro Val Pro Ser Pro Thr Thr Ala Gly
   14870 14875 14880
   Pro Leu Leu Val Pro Phe Thr Leu Asn Phe Thr Ile Thr Asn Leu
   14885 14890 14895
   Gln Tyr Glu Glu Asp Met His Arg Pro Gly Ser Arg Arg Phe Asn
   14900 14905 14910
   Thr Thr Glu Arg Val Leu Gln Gly Leu Leu Thr Pro Leu Phe Lys
   14915 14920 14925
   Asn Thr Ser Val Gly Pro Leu Tyr Ser Gly Cys Arg Leu Thr Leu
   14930 14935 14940
   Leu Arg Pro Glu Lys Gln Glu Ala Ala Thr Gly Val Asp Thr Ile
   14945 14950 14955
   Cys Thr His Arg Val Asp Pro Ile Gly Pro Gly Leu Asp Arg Glu
   14960 14965 14970
   Arg Leu Tyr Trp Glu Leu Ser Gln Leu Thr Asn Ser Ile Thr Glu
   14975 14980 14985
   Leu Gly Pro Tyr Thr Leu Asp Arg Asp Ser Leu Tyr Val Asn Gly
   14990 14995 15000
   Phe Asn Pro Trp Ser Ser Val Pro Thr Thr Ser Thr Pro Gly Thr
   15005 15010 15015
   Ser Thr Val His Leu Ala Thr Ser Gly Thr Pro Ser Ser Leu Pro
   15020 15025 15030
   Gly His Thr Ala Pro Val Pro Leu Leu Ile Pro Phe Thr Leu Asn
   15035 15040 15045
   Phe Thr Ile Thr Asp Leu His Tyr Glu Glu Asn Met Gln His Pro
   15050 15055 15060
   Gly Ser Arg Lys Phe Asn Thr Thr Glu Arg Val Leu Gln Gly Leu
   15065 15070 15075
   Leu Lys Pro Leu Phe Lys Ser Thr Ser Val Gly Pro Leu Tyr Ser
   15080 15085 15090
   Gly Cys Arg Leu Thr Leu Leu Arg Pro Glu Lys His Gly Ala Ala
   15095 15100 15105
   Thr Gly Val Asp Ala Ile Cys Thr Leu Arg Leu Asp Pro Thr Gly
   15110 15115 15120
   Pro Gly Leu Asp Arg Glu Arg Leu Tyr Trp Glu Leu Ser Gln Leu
   15125 15130 15135
   Thr Asn Ser Val Thr Glu Leu Gly Pro Tyr Thr Leu Asp Arg Asp
   15140 15145 15150
   Ser Leu Tyr Val Asn Gly Phe Thr His Arg Ser Ser Val Pro Thr
   15155 15160 15165
   Thr Ser Ile Pro Gly Thr Ser Ala Val His Leu Glu Thr Ser Gly
   15170 15175 15180
   Thr Pro Ala Ser Leu Pro Gly His Thr Ala Pro Gly Pro Leu Leu
   15185 15190 15195
   Val Pro Phe Thr Leu Asn Phe Thr Ile Thr Asn Leu Gln Tyr Glu
   15200 15205 15210
   Glu Asp Met Arg His Pro Gly Ser Arg Lys Phe Ser Thr Thr Glu
   15215 15220 15225
   Arg Val Leu Gln Gly Leu Leu Lys Pro Leu Phe Lys Asn Thr Ser
   15230 15235 15240
   Val Ser Ser Leu Tyr Ser Gly Cys Arg Leu Thr Leu Leu Arg Pro
   15245 15250 15255
   Glu Lys Asp Gly Ala Ala Thr Arg Val Asp Ala Val Cys Thr His
   15260 15265 15270
   Arg Pro Asp Pro Lys Ser Pro Gly Leu Asp Arg Glu Arg Leu Tyr
   15275 15280 15285
   Trp Lys Leu Ser Gln Leu Thr His Gly Ile Thr Glu Leu Gly Pro
   15290 15295 15300
   Tyr Thr Leu Asp Arg His Ser Leu Tyr Val Asn Gly Phe Thr His
   15305 15310 15315
   Gln Ser Ser Met Thr Thr Thr Arg Thr Pro Asp Thr Ser Thr Met
   15320 15325 15330
   His Leu Ala Thr Ser Arg Thr Pro Ala Ser Leu Ser Gly Pro Thr
   15335 15340 15345
   Thr Ala Ser Pro Leu Leu Val Leu Phe Thr Ile Asn Phe Thr Ile
   15350 15355 15360
   Thr Asn Leu Arg Tyr Glu Glu Asn Met His His Pro Gly Ser Arg
   15365 15370 15375
   Lys Phe Asn Thr Thr Glu Arg Val Leu Gln Gly Leu Leu Arg Pro
   15380 15385 15390
   Val Phe Lys Asn Thr Ser Val Gly Pro Leu Tyr Ser Gly Cys Arg
   15395 15400 15405
   Leu Thr Thr Leu Arg Pro Lys Lys Asp Gly Ala Ala Thr Lys Val
   15410 15415 15420
   Asp Ala Ile Cys Thr Tyr Arg Pro Asp Pro Lys Ser Pro Gly Leu
   15425 15430 15435
   Asp Arg Glu Gln Leu Tyr Trp Glu Leu Ser Gln Leu Thr His Ser
   15440 15445 15450
   Ile Thr Glu Leu Gly Pro Tyr Thr Gln Asp Arg Asp Ser Leu Tyr
   15455 15460 15465
   Val Asn Gly Phe Thr His Arg Ser Ser Val Pro Thr Thr Ser Ile
   15470 15475 15480
   Pro Gly Thr Ser Ala Val His Leu Glu Thr Ser Gly Thr Pro Ala
   15485 15490 15495
   Ser Leu Pro Gly His Thr Ala Pro Gly Pro Leu Leu Val Pro Phe
   15500 15505 15510
   Thr Leu Asn Phe Thr Ile Thr Asn Leu Gln Tyr Glu Glu Asp Met
   15515 15520 15525
   Arg His Pro Gly Ser Arg Lys Phe Asn Thr Thr Glu Arg Val Leu
   15530 15535 15540
   Gln Gly Leu Leu Lys Pro Leu Phe Lys Ser Thr Ser Val Gly Pro
   15545 15550 15555
   Leu Tyr Ser Gly Cys Arg Leu Thr Leu Leu Arg Pro Glu Lys Arg
   15560 15565 15570
   Gly Ala Ala Thr Gly Val Asp Thr Ile Cys Thr His Arg Leu Asp
   15575 15580 15585
   Pro Leu Asn Pro Gly Leu Asp Arg Glu Gln Leu Tyr Trp Glu Leu
   15590 15595 15600
   Ser Lys Leu Thr Arg Gly Ile Ile Glu Leu Gly Pro Tyr Leu Leu
   15605 15610 15615
   Asp Arg Gly Ser Leu Tyr Val Asn Gly Phe Thr His Arg Thr Ser
   15620 15625 15630
   Val Pro Thr Thr Ser Thr Pro Gly Thr Ser Thr Val Asp Leu Gly
   15635 15640 15645
   Thr Ser Gly Thr Pro Phe Ser Leu Pro Ser Pro Ala Xaa Xaa Xaa
   15650 15655 15660
   Pro Leu Leu Xaa Pro Phe Thr Xaa Asn Xaa Thr Ile Thr Asn Leu
   15665 15670 15675
   Xaa Xaa Xaa Xaa Xaa Met Xaa Xaa Pro Gly Ser Arg Lys Phe Asn
   15680 15685 15690
   Thr Thr Glu Arg Val Leu Gln Thr Leu Leu Gly Pro Met Phe Lys
   15695 15700 15705
   Asn Thr Ser Val Gly Leu Leu Tyr Ser Gly Cys Arg Leu Thr Leu
   15710 15715 15720
   Leu Arg Ser Glu Lys Asp Gly Ala Ala Thr Gly Val Asp Ala Ile
   15725 15730 15735
   Cys Thr His Arg Leu Asp Pro Lys Ser Pro Gly Val Asp Arg Glu
   15740 15745 15750
   Gln Leu Tyr Trp Glu Leu Ser Gln Leu Thr Asn Gly Ile Lys Glu
   15755 15760 15765
   Leu Gly Pro Tyr Thr Leu Asp Arg Asn Ser Leu Tyr Val Asn Gly
   15770 15775 15780
   Phe Thr His Trp Ile Pro Val Pro Thr Ser Ser Thr Pro Gly Thr
   15785 15790 15795
   Ser Thr Val Asp Leu Gly Ser Gly Thr Pro Ser Ser Leu Pro Ser
   15800 15805 15810
   Pro Thr Thr Ala Gly Pro Leu Leu Val Pro Phe Thr Leu Asn Phe
   15815 15820 15825
   Thr Ile Thr Asn Leu Lys Tyr Glu Glu Asp Met His Cys Pro Gly
   15830 15835 15840
   Ser Arg Lys Phe Asn Thr Thr Glu Arg Val Leu Gln Ser Leu Leu
   15845 15850 15855
   Gly Pro Met Phe Lys Asn Thr Ser Val Gly Pro Leu Tyr Ser Gly
   15860 15865 15870
   Cys Arg Leu Thr Leu Leu Arg Ser Glu Lys Asp Gly Ala Ala Thr
   15875 15880 15885
   Gly Val Asp Ala Ile Cys Thr His Arg Leu Asp Pro Lys Ser Pro
   15890 15895 15900
   Gly Val Asp Arg Glu Gln Leu Tyr Trp Glu Leu Ser Gln Leu Thr
   15905 15910 15915
   Asn Gly Ile Lys Glu Leu Gly Pro Tyr Thr Leu Asp Arg Asn Ser
   15920 15925 15930
   Leu Tyr Val Asn Gly Phe Thr His Gln Thr Ser Ala Pro Asn Thr
   15935 15940 15945
   Ser Thr Pro Gly Thr Ser Thr Val Asp Leu Gly Thr Ser Gly Thr
   15950 15955 15960
   Pro Ser Ser Leu Pro Ser Pro Thr Xaa Xaa Xaa Pro Leu Leu Xaa
   15965 15970 15975
   Pro Phe Thr Xaa Asn Xaa Thr Ile Thr Asn Leu Xaa Xaa Xaa Xaa
   15980 15985 15990
   Xaa Met Xaa Xaa Pro Gly Ser Arg Lys Phe Asn Thr Thr Glu Xaa
   15995 16000 16005
   Val Leu Gln Gly Leu Leu Xaa Pro Xaa Phe Lys Asn Xaa Ser Val
   16010 16015 16020
   Gly Xaa Leu Tyr Ser Gly Cys Arg Leu Thr Xaa Leu Arg Xaa Glu
   16025 16030 16035
   Lys Xaa Gly Ala Ala Thr Gly Xaa Asp Ala Ile Cys Xaa His Xaa
   16040 16045 16050
   Xaa Xaa Pro Lys Xaa Pro Gly Leu Xaa Xaa Glu Xaa Leu Tyr Trp
   16055 16060 16065
   Glu Leu Ser Xaa Leu Thr Xaa Xaa Ile Xaa Glu Leu Gly Pro Tyr
   16070 16075 16080
   Thr Leu Asp Arg Xaa Ser Leu Tyr Val Asn Gly Phe Thr His Trp
   16085 16090 16095
   Ile Pro Val Pro Thr Ser Ser Thr Pro Gly Thr Ser Thr Val Asp
   16100 16105 16110
   Leu Gly Ser Gly Thr Pro Ser Ser Leu Pro Ser Pro Thr Thr Ala
   16115 16120 16125
   Gly Pro Leu Leu Val Pro Phe Thr Leu Asn Phe Thr Ile Thr Asn
   16130 16135 16140
   Leu Lys Tyr Glu Glu Asp Met His Cys Pro Gly Ser Arg Lys Phe
   16145 16150 16155
   Asn Thr Thr Glu Arg Val Leu Gln Ser Leu Leu Gly Pro Met Phe
   16160 16165 16170
   Lys Asn Thr Ser Val Gly Pro Leu Tyr Ser Gly Cys Arg Leu Thr
   16175 16180 16185
   Ser Leu Arg Ser Glu Lys Asp Gly Ala Ala Thr Gly Val Asp Ala
   16190 16195 16200
   Ile Cys Thr His Arg Val Asp Pro Lys Ser Pro Gly Val Asp Arg
   16205 16210 16215
   Glu Gln Leu Tyr Trp Glu Leu Ser Gln Leu Thr Asn Gly Ile Lys
   16220 16225 16230
   Glu Leu Gly Pro Tyr Thr Leu Asp Arg Asn Ser Leu Tyr Val Asn
   16235 16240 16245
   Gly Phe Thr His Gln Thr Ser Ala Pro Asn Thr Ser Thr Pro Gly
   16250 16255 16260
   Thr Ser Thr Val Xaa Xaa Gly Thr Ser Gly Thr Pro Ser Ser Xaa
   16265 16270 16275
   Pro Xaa Xaa Thr Ser Ala Gly Pro Leu Leu Val Pro Phe Thr Leu
   16280 16285 16290
   Asn Phe Thr Ile Thr Asn Leu Gln Tyr Glu Glu Asp Met His His
   16295 16300 16305
   Pro Gly Ser Arg Lys Phe Asn Thr Thr Glu Arg Val Leu Gln Gly
   16310 16315 16320
   Leu Leu Gly Pro Met Phe Lys Asn Thr Ser Val Gly Leu Leu Tyr
   16325 16330 16335
   Ser Gly Cys Arg Leu Thr Leu Leu Arg Pro Glu Lys Asn Gly Ala
   16340 16345 16350
   Thr Thr Gly Met Asp Ala Ile Cys Thr His Arg Leu Asp Pro Lys
   16355 16360 16365
   Ser Pro Gly Leu Xaa Xaa Glu Xaa Leu Tyr Trp Glu Leu Ser Xaa
   16370 16375 16380
   Leu Thr Xaa Xaa Ile Xaa Glu Leu Gly Pro Tyr Thr Leu Asp Arg
   16385 16390 16395
   Xaa Ser Leu Tyr Val Asn Gly Phe Thr His Xaa Xaa Ser Xaa Pro
   16400 16405 16410
   Thr Thr Ser Thr Pro Gly Thr Ser Thr Val Xaa Xaa Gly Thr Ser
   16415 16420 16425
   Gly Thr Pro Ser Ser Xaa Pro Xaa Xaa Thr Xaa Xaa Xaa Pro Leu
   16430 16435 16440
   Leu Xaa Pro Phe Thr Xaa Asn Xaa Thr Ile Thr Asn Leu Xaa Xaa
   16445 16450 16455
   Xaa Xaa Xaa Met Xaa Xaa Pro Gly Ser Arg Lys Phe Asn Thr Thr
   16460 16465 16470
   Glu Arg Val Leu Gln Gly Leu Leu Lys Pro Leu Phe Arg Asn Ser
   16475 16480 16485
   Ser Leu Glu Tyr Leu Tyr Ser Gly Cys Arg Leu Ala Ser Leu Arg
   16490 16495 16500
   Pro Glu Lys Asp Ser Ser Ala Met Ala Val Asp Ala Ile Cys Thr
   16505 16510 16515
   His Arg Pro Asp Pro Glu Asp Leu Gly Leu Asp Arg Glu Arg Leu
   16520 16525 16530
   Tyr Trp Glu Leu Ser Asn Leu Thr Asn Gly Ile Gln Glu Leu Gly
   16535 16540 16545
   Pro Tyr Thr Leu Asp Arg Asn Ser Leu Tyr Val Asn Gly Phe Thr
   16550 16555 16560
   His Arg Ser Ser Met Pro Thr Thr Ser Thr Pro Gly Thr Ser Thr
   16565 16570 16575
   Val Asp Val Gly Thr Ser Gly Thr Pro Ser Ser Ser Pro Ser Pro
   16580 16585 16590
   Thr Thr Ala Gly Pro Leu Leu Ile Pro Phe Thr Leu Asn Phe Thr
   16595 16600 16605
   Ile Thr Asn Leu Gln Tyr Gly Glu Asp Met Gly His Pro Gly Ser
   16610 16615 16620
   Arg Lys Phe Asn Thr Thr Glu Arg Val Leu Gln Gly Leu Leu Gly
   16625 16630 16635
   Pro Ile Phe Lys Asn Thr Ser Val Gly Pro Leu Tyr Ser Gly Cys
   16640 16645 16650
   Arg Leu Thr Ser Leu Arg Ser Glu Lys Asp Gly Ala Ala Thr Gly
   16655 16660 16665
   Val Asp Ala Ile Cys Ile His His Leu Asp Pro Lys Ser Pro Gly
   16670 16675 16680
   Leu Asn Arg Glu Arg Leu Tyr Trp Glu Leu Ser Gln Leu Thr Asn
   16685 16690 16695
   Gly Ile Lys Glu Leu Gly Pro Tyr Thr Leu Asp Arg Asn Ser Leu
   16700 16705 16710
   Tyr Val Asn Gly Phe Thr His Arg Thr Ser Val Pro Thr Thr Ser
   16715 16720 16725
   Thr Pro Gly Thr Ser Thr Val Asp Leu Gly Thr Ser Gly Thr Pro
   16730 16735 16740
   Phe Ser Leu Pro Ser Pro Ala Thr Ala Gly Pro Leu Leu Val Leu
   16745 16750 16755
   Phe Thr Leu Asn Phe Thr Ile Thr Asn Leu Lys Tyr Glu Glu Asp
   16760 16765 16770
   Met His Arg Pro Gly Ser Arg Lys Phe Asn Thr Thr Glu Arg Val
   16775 16780 16785
   Leu Gln Thr Leu Leu Gly Pro Met Phe Lys Asn Thr Ser Val Gly
   16790 16795 16800
   Leu Leu Tyr Ser Gly Cys Arg Leu Thr Leu Leu Arg Ser Glu Lys
   16805 16810 16815
   Asp Gly Ala Ala Thr Gly Val Asp Ala Ile Cys Thr His Arg Leu
   16820 16825 16830
   Asp Pro Lys Ser Pro Gly Leu Xaa Xaa Glu Xaa Leu Tyr Trp Glu
   16835 16840 16845
   Leu Ser Xaa Leu Thr Xaa Xaa Ile Xaa Glu Leu Gly Pro Tyr Thr
   16850 16855 16860
   Leu Asp Arg Xaa Ser Leu Tyr Val Asn Gly Phe Thr His Xaa Xaa
   16865 16870 16875
   Ser Xaa Pro Thr Thr Ser Thr Pro Gly Thr Ser Thr Val Xaa Xaa
   16880 16885 16890
   Gly Thr Ser Gly Thr Pro Ser Ser Xaa Pro Xaa Xaa Thr Xaa Xaa
   16895 16900 16905
   Xaa Pro Leu Leu Xaa Pro Phe Thr Xaa Asn Xaa Thr Ile Thr Asn
   16910 16915 16920
   Leu Xaa Xaa Xaa Xaa Xaa Met Xaa Xaa Pro Gly Ser Arg Lys Phe
   16925 16930 16935
   Asn Thr Thr Glu Arg Val Leu Gln Gly Leu Leu Arg Pro Val Phe
   16940 16945 16950
   Lys Asn Thr Ser Val Gly Pro Leu Tyr Ser Gly Cys Arg Leu Thr
   16955 16960 16965
   Leu Leu Arg Pro Lys Lys Asp Gly Ala Ala Thr Lys Val Asp Ala
   16970 16975 16980
   Ile Cys Thr Tyr Arg Pro Asp Pro Lys Ser Pro Gly Leu Asp Arg
   16985 16990 16995
   Glu Gln Leu Tyr Trp Glu Leu Ser Gln Leu Thr His Ser Ile Thr
   17000 17005 17010
   Glu Leu Gly Pro Tyr Thr Gln Asp Arg Asp Ser Leu Tyr Val Asn
   17015 17020 17025
   Gly Phe Thr His Arg Ser Ser Val Pro Thr Thr Ser Ile Pro Gly
   17030 17035 17040
   Thr Ser Ala Val His Leu Glu Thr Thr Gly Thr Pro Ser Ser Phe
   17045 17050 17055
   Pro Gly His Thr Glu Pro Gly Pro Leu Leu Ile Pro Phe Thr Phe
   17060 17065 17070
   Asn Phe Thr Ile Thr Asn Leu Arg Tyr Glu Glu Asn Met Gln His
   17075 17080 17085
   Pro Gly Ser Arg Lys Phe Asn Thr Thr Glu Arg Val Leu Gln Gly
   17090 17095 17100
   Leu Leu Thr Pro Leu Phe Lys Asn Thr Ser Val Gly Pro Leu Tyr
   17105 17110 17115
   Ser Gly Cys Arg Leu Thr Leu Leu Arg Pro Glu Lys Gln Glu Ala
   17120 17125 17130
   Ala Thr Gly Val Asp Thr Ile Cys Thr His Arg Val Asp Pro Ile
   17135 17140 17145
   Gly Pro Gly Leu Asp Arg Glu Arg Leu Tyr Trp Glu Leu Ser Gln
   17150 17155 17160
   Leu Thr Asn Ser Ile Thr Glu Leu Gly Pro Tyr Thr Leu Asp Arg
   17165 17170 17175
   Asp Ser Leu Tyr Val Asp Gly Phe Asn Pro Trp Ser Ser Val Pro
   17180 17185 17190
   Thr Thr Ser Thr Pro Gly Thr Ser Thr Val His Leu Ala Thr Ser
   17195 17200 17205
   Gly Thr Pro Ser Pro Leu Pro Gly His Thr Ala Pro Val Pro Leu
   17210 17215 17220
   Leu Ile Pro Phe Thr Leu Asn Phe Thr Ile Thr Asp Leu His Tyr
   17225 17230 17235
   Glu Glu Asn Met Gln His Pro Gly Ser Arg Lys Phe Asn Thr Thr
   17240 17245 17250
   Glu Arg Val Leu Gln Gly Leu Leu Lys Pro Leu Phe Lys Ser Thr
   17255 17260 17265
   Ser Val Gly Pro Leu Tyr Ser Gly Cys Arg Leu Thr Leu Leu Arg
   17270 17275 17280
   Pro Glu Lys His Gly Ala Ala Thr Gly Val Asp Ala Ile Cys Thr
   17285 17290 17295
   Leu Arg Leu Asp Pro Thr Gly Pro Gly Leu Asp Arg Glu Arg Leu
   17300 17305 17310
   Tyr Trp Glu Leu Ser Gln Leu Thr Asn Ser Ile Thr Glu Leu Gly
   17315 17320 17325
   Pro Tyr Thr Leu Asp Arg Asp Ser Leu Tyr Val Asn Gly Phe Asn
   17330 17335 17340
   Pro Trp Ser Ser Val Pro Thr Thr Ser Thr Pro Gly Thr Ser Thr
   17345 17350 17355
   Val His Leu Ala Thr Ser Gly Thr Pro Ser Ser Leu Pro Gly His
   17360 17365 17370
   Thr Thr Ala Gly Pro Leu Leu Val Pro Phe Thr Leu Asn Phe Thr
   17375 17380 17385
   Ile Thr Asn Leu Lys Tyr Glu Glu Asp Met His Cys Pro Gly Ser
   17390 17395 17400
   Arg Lys Phe Asn Thr Thr Glu Arg Val Leu Gln Ser Leu His Gly
   17405 17410 17415
   Pro Met Phe Lys Asn Thr Ser Val Gly Pro Leu Tyr Ser Gly Cys
   17420 17425 17430
   Arg Leu Thr Leu Leu Arg Ser Glu Lys Asp Gly Ala Ala Thr Gly
   17435 17440 17445
   Val Asp Ala Ile Cys Thr His Arg Leu Asp Pro Lys Ser Pro Gly
   17450 17455 17460
   Leu Xaa Xaa Glu Xaa Leu Tyr Trp Glu Leu Ser Xaa Leu Thr Xaa
   17465 17470 17475
   Xaa Ile Xaa Glu Leu Gly Pro Tyr Thr Leu Asp Arg Xaa Ser Leu
   17480 17485 17490
   Tyr Val Asn Gly Phe Thr His Xaa Xaa Ser Xaa Pro Thr Thr Ser
   17495 17500 17505
   Thr Pro Gly Thr Ser Thr Val Xaa Xaa Gly Thr Ser Gly Thr Pro
   17510 17515 17520
   Ser Ser Xaa Pro Xaa Xaa Thr Xaa Xaa Xaa Pro Leu Leu Xaa Pro
   17525 17530 17535
   Phe Thr Xaa Asn Xaa Thr Ile Thr Asn Leu Xaa Xaa Xaa Xaa Xaa
   17540 17545 17550
   Met Xaa Xaa Pro Gly Ser Arg Lys Phe Asn Thr Thr Glu Xaa Val
   17555 17560 17565
   Leu Gln Gly Leu Leu Xaa Pro Xaa Phe Lys Asn Xaa Ser Val Gly
   17570 17575 17580
   Xaa Leu Tyr Ser Gly Cys Arg Leu Thr Xaa Leu Arg Xaa Glu Lys
   17585 17590 17595
   Xaa Gly Ala Ala Thr Gly Xaa Asp Ala Ile Cys Xaa His Xaa Xaa
   17600 17605 17610
   Xaa Pro Lys Xaa Pro Gly Leu Xaa Xaa Glu Xaa Leu Tyr Trp Glu
   17615 17620 17625
   Leu Ser Xaa Leu Thr Asn Ser Ile Thr Glu Leu Gly Pro Tyr Thr
   17630 17635 17640
   Leu Asp Arg Asp Ser Leu Tyr Val Asn Gly Phe Thr His Arg Ser
   17645 17650 17655
   Ser Met Pro Thr Thr Ser Ile Pro Gly Thr Ser Ala Val His Leu
   17660 17665 17670
   Glu Thr Ser Gly Thr Pro Ala Ser Leu Pro Gly His Thr Ala Pro
   17675 17680 17685
   Gly Pro Leu Leu Val Pro Phe Thr Leu Asn Phe Thr Ile Thr Asn
   17690 17695 17700
   Leu Gln Tyr Glu Glu Asp Met Arg His Pro Gly Ser Arg Lys Phe
   17705 17710 17715
   Asn Thr Thr Glu Arg Val Leu Gln Gly Leu Leu Lys Pro Leu Phe
   17720 17725 17730
   Lys Ser Thr Ser Val Gly Pro Leu Tyr Ser Gly Cys Arg Leu Thr
   17735 17740 17745
   Leu Leu Arg Pro Glu Lys Arg Gly Ala Ala Thr Gly Val Asp Thr
   17750 17755 17760
   Ile Cys Thr His Arg Leu Asp Pro Leu Asn Pro Gly Leu Xaa Xaa
   17765 17770 17775
   Glu Xaa Leu Tyr Trp Glu Leu Ser Xaa Leu Thr Xaa Xaa Ile Xaa
   17780 17785 17790
   Glu Leu Gly Pro Tyr Thr Leu Asp Arg Xaa Ser Leu Tyr Val Asn
   17795 17800 17805
   Gly Phe Thr His Xaa Xaa Ser Xaa Pro Thr Thr Ser Thr Pro Gly
   17810 17815 17820
   Thr Ser Thr Val Xaa Xaa Gly Thr Ser Gly Thr Pro Ser Ser Xaa
   17825 17830 17835
   Pro Xaa Xaa Thr Xaa Xaa Xaa Pro Leu Leu Xaa Pro Phe Thr Xaa
   17840 17845 17850
   Asn Xaa Thr Ile Thr Asn Leu Xaa Xaa Xaa Xaa Xaa Met Xaa Xaa
   17855 17860 17865
   Pro Gly Ser Arg Lys Phe Asn Thr Thr Glu Xaa Val Leu Gln Gly
   17870 17875 17880
   Leu Leu   Xaa Pro Xaa Phe Lys   Asn Xaa Ser Val Gly   Xaa Leu Tyr
       17885                 17890                 17895
   Ser Gly   Cys Arg Leu Thr Xaa   Leu Arg Xaa Glu Lys   Xaa Gly Ala
       17900                 17905                 17910
   Ala Thr   Gly Xaa Asp Ala Ile   Cys Xaa His Xaa Xaa   Xaa Pro Lys
       17915                 17920                 17925
   Xaa Pro   Gly Leu Xaa Xaa Glu   Xaa Leu Tyr Trp Glu   Leu Ser Xaa
       17930                 17935                 17940
   Leu Thr   Xaa Xaa Ile Xaa Glu   Leu Gly Pro Tyr Thr   Leu Asp Arg
       17945                 17950                 17955
   Xaa Ser   Leu Tyr Val Asn Gly   Phe His Pro Arg Ser   Ser Val Pro
       17960                 17965                 17970
   Thr Thr   Ser Thr Pro Gly Thr   Ser Thr Val His Leu   Ala Thr Ser
       17975                 17980                 17985
   Gly Thr   Pro Ser Ser Leu Pro   Gly His Thr Ala Pro   Val Pro Leu
       17990                 17995                 18000
   Leu Ile   Pro Phe Thr Leu Asn   Phe Thr Ile Thr Asn   Leu His Tyr
       18005                 18010                 18015
   Glu Glu   Asn Met Gln His Pro   Gly Ser Arg Lys Phe   Asn Thr Thr
       18020                 18025                 18030
   Glu Arg   Val Leu Gln Gly Leu   Leu Gly Pro Met Phe   Lys Asn Thr
       18035                 18040                 18045
   Ser Val   Gly Leu Leu Tyr Ser   Gly Cys Arg Leu Thr   Leu Leu Arg
       18050                 18055                 18060
   Pro Glu   Lys Asn Gly Ala Ala   Thr Gly Met Asp Ala   Ile Cys Ser
       18065                 18070                 18075
   His Arg   Leu Asp Pro Lys Ser   Pro Gly Leu Xaa Xaa   Glu Xaa Leu
       18080                 18085                 18090
   Tyr Trp   Glu Leu Ser Xaa Leu   Thr Xaa Xaa Ile Xaa   Glu Leu Gly
       18095                 18100                 18105
   Pro Tyr   Thr Leu Asp Arg Xaa   Ser Leu Tyr Val Asn   Gly Phe Thr
   18110 18115 18120
   His Xaa Xaa Ser Xaa Pro Thr Thr Ser Thr Pro Gly Thr Ser Thr
   18125 18130 18135
   Val Xaa Xaa Gly Thr Ser Gly Thr Pro Ser Ser Xaa Pro Xaa Xaa
   18140 18145 18150
   Thr Xaa Xaa Xaa Pro Leu Leu Xaa Pro Phe Thr Xaa Asn Xaa Thr
   18155 18160 18165
   Ile Thr Asn Leu Xaa Xaa Xaa Xaa Xaa Met Xaa Xaa Pro Gly Ser
   18170 18175 18180
   Arg Lys Phe Asn Thr Thr Glu Xaa Val Leu Gln Gly Leu Leu Xaa
   18185 18190 18195
   Pro Xaa Phe Lys Asn Xaa Ser Val Gly Xaa Leu Tyr Ser Gly Cys
   18200 18205 18210
   Arg Leu Thr Xaa Leu Arg Xaa Glu Lys Xaa Gly Ala Ala Thr Gly
   18215 18220 18225
   Xaa Asp Ala Ile Cys Xaa His Xaa Xaa Xaa Pro Lys Xaa Pro Gly
   18230 18235 18240
   Leu Xaa Xaa Glu Xaa Leu Tyr Trp Glu Leu Ser Xaa Leu Thr Xaa
   18245 18250 18255
   Xaa Ile Xaa Glu Leu Gly Pro Tyr Thr Leu Asp Arg Xaa Ser Leu
   18260 18265 18270
   Tyr Val Asn Gly Phe Thr His Gln Asn Ser Val Pro Thr Thr Ser
   18275 18280 18285
   Thr Pro Gly Thr Ser Thr Val Tyr Trp Ala Thr Thr Gly Thr Pro
   18290 18295 18300
   Ser Ser Phe Pro Gly His Thr Glu Pro Gly Pro Leu Leu Ile Pro
   18305 18310 18315
   Phe Thr Phe Asn Phe Thr Ile Thr Asn Leu His Tyr Glu Glu Asn
   18320 18325 18330
   Met Gln His Pro Gly Ser Arg Lys Phe Asn Thr Thr Glu Arg Val
   18335 18340 18345
   Leu Gln Gly Leu Leu Thr Pro Leu Phe Lys Asn Thr Ser Val Gly
   18350 18355 18360
   Pro Leu Tyr Ser Gly Cys Arg Leu Thr Leu Leu Arg Pro Glu Lys
   18365 18370 18375
   Gln Glu Ala Ala Thr Gly Val Asp Thr Ile Cys Thr His Arg Val
   18380 18385 18390
   Asp Pro Ile Gly Pro Gly Leu Xaa Xaa Glu Xaa Leu Tyr Trp Glu
   18395 18400 18405
   Leu Ser Xaa Leu Thr Xaa Xaa Ile Xaa Glu Leu Gly Pro Tyr Thr
   18410 18415 18420
   Leu Asp Arg Xaa Ser Leu Tyr Val Asn Gly Phe Thr His Xaa Xaa
   18425 18430 18435
   Ser Xaa Pro Thr Thr Ser Thr Pro Gly Thr Ser Thr Val Xaa Xaa
   18440 18445 18450
   Gly Thr Ser Gly Thr Pro Ser Ser Xaa Pro Xaa Xaa Thr Xaa Xaa
   18455 18460 18465
   Xaa Pro Leu Leu Xaa Pro Phe Thr Xaa Asn Xaa Thr Ile Thr Asn
   18470 18475 18480
   Leu Xaa Xaa Xaa Xaa Xaa Met Xaa Xaa Pro Gly Ser Arg Lys Phe
   18485 18490 18495
   Asn Thr Thr Glu Xaa Val Leu Gln Gly Leu Leu Xaa Pro Xaa Phe
   18500 18505 18510
   Lys Asn Xaa Ser Val Gly Xaa Leu Tyr Ser Gly Cys Arg Leu Thr
   18515 18520 18525
   Xaa Leu Arg Xaa Glu Lys Xaa Gly Ala Ala Thr Gly Xaa Asp Ala
   18530 18535 18540
   Ile Cys Xaa His Xaa Xaa Xaa Pro Lys Xaa Pro Gly Leu Xaa Xaa
   18545 18550 18555
   Glu Xaa Leu Tyr Trp Glu Leu Ser Xaa Leu Thr Xaa Xaa Ile Xaa
   18560 18565 18570
   Glu Leu Gly Pro Tyr Thr Leu Asp Arg Xaa Ser Leu Tyr Val Asn
   18575 18580 18585
   Gly Phe Thr His Arg Ser Ser Val Pro Thr Thr Ser Ser Pro Gly
       18590                   18595                   18600
   Thr Ser Thr Val His Leu Ala Thr Ser Gly Thr Pro Ser Ser Leu
       18605                   18610                   18615
   Pro Gly His Thr Ala Pro Val Pro Leu Leu Ile Pro Phe Thr Leu
       18620                   18625                   18630
   Asn Phe Thr Ile Thr Asn Leu His Tyr Glu Glu Asn Met Gln His
       18635                   18640                   18645
   Pro Gly Ser Arg Lys Phe Asn Thr Thr Glu Arg Val Leu Gln Gly
       18650                   18655                   18660
   Leu Leu Lys Pro Leu Phe Lys Ser Thr Ser Val Gly Pro Leu Tyr
       18665                   18670                   18675
   Ser Gly Cys Arg Leu Thr Leu Leu Arg Pro Glu Lys His Gly Ala
       18680                   18685                   18690
   Ala Thr Gly Val Asp Ala Ile Cys Thr Leu Arg Leu Asp Pro Thr
       18695                   18700                   18705
   Gly Pro Gly Leu Xaa Xaa Glu Xaa Leu Tyr Trp Glu Leu Ser Xaa
       18710                   18715                   18720
   Leu Thr Xaa Xaa Ile Xaa Glu Leu Gly Pro Tyr Thr Leu Asp Arg
       18725                   18730                   18735
   Xaa Ser Leu Tyr Val Asn Gly Phe Thr His Xaa Xaa Ser Xaa Pro
       18740                   18745                   18750
   Thr Thr Ser Thr Pro Gly Thr Ser Thr Val Xaa Xaa Gly Thr Ser
       18755                   18760                   18765
   Gly Thr Pro Ser Ser Xaa Pro Xaa Xaa Thr Xaa Xaa Xaa Pro Leu
       18770                   18775                   18780
   Leu Xaa Pro Phe Thr Xaa Asn Xaa Thr Ile Thr Asn Leu Xaa Xaa
       18785                   18790                   18795
   Xaa Xaa Xaa Met Xaa Xaa Pro Gly Ser Arg Lys Phe Asn Thr Thr
       18800                   18805                   18810
   Glu Xaa Val Leu Gln Gly Leu Leu Xaa Pro Xaa Phe Lys Asn Xaa
       18815                   18820                   18825
   Ser Val Gly Xaa Leu Tyr Ser Gly Cys Arg Leu Thr Xaa Leu Arg
   18830 18835 18840
   Xaa Glu Lys Xaa Gly Ala Ala Thr Gly Xaa Asp Ala Ile Cys Xaa
   18845 18850 18855
   His Xaa Xaa Xaa Pro Lys Xaa Pro Gly Leu Xaa Xaa Glu Xaa Leu
   18860 18865 18870
   Tyr Trp Glu Leu Ser Xaa Leu Thr Xaa Xaa Ile Xaa Glu Leu Gly
   18875 18880 18885
   Pro Tyr Thr Leu Asp Arg Xaa Ser Leu Tyr Val Asn Gly Phe Thr
   18890 18895 18900
   His Arg Thr Ser Val Pro Thr Thr Ser Thr Pro Gly Thr Ser Thr
   18905 18910 18915
   Val His Leu Ala Thr Ser Gly Thr Pro Ser Ser Leu Pro Gly His
   18920 18925 18930
   Thr Ala Pro Val Pro Leu Leu Ile Pro Phe Thr Leu Asn Phe Thr
   18935 18940 18945
   Ile Thr Asn Leu Gln Tyr Glu Glu Asp Met His Arg Pro Gly Ser
   18950 18955 18960
   Arg Lys Phe Asn Thr Thr Glu Arg Val Leu Gln Gly Leu Leu Ser
   18965 18970 18975
   Pro Ile Phe Lys Asn Ser Ser Val Gly Pro Leu Tyr Ser Gly Cys
   18980 18985 18990
   Arg Leu Thr Ser Leu Arg Pro Glu Lys Asp Gly Ala Ala Thr Gly
   18995 19000 19005
   Met Asp Ala Val Cys Leu Tyr His Pro Asn Pro Lys Arg Pro Gly
   19010 19015 19020
   Leu Asp Arg Glu Gln Leu Tyr Cys Glu Leu Ser Gln Leu Thr His
   19025 19030 19035
   Asn Ile Thr Glu Leu Gly Pro Tyr Ser Leu Asp Arg Asp Ser Leu
   19040 19045 19050
   Tyr Val Asn Gly Phe Thr His Gln Asn Ser Val Pro Thr Thr Ser
   19055 19060 19065
   Thr Pro Gly Thr Ser Thr Val Tyr Trp Ala Thr Thr Gly Thr Pro
   19070 19075 19080
   Ser Ser Phe Pro Gly His Thr Xaa Xaa Xaa Pro Leu Leu Xaa Pro
   19085 19090 19095
   Phe Thr Xaa Asn Xaa Thr Ile Thr Asn Leu Xaa Xaa Xaa Xaa Xaa
   19100 19105 19110
   Met Xaa Xaa Pro Gly Ser Arg Lys Phe Asn Thr Thr Glu Xaa Val
   19115 19120 19125
   Leu Gln Gly Leu Leu Xaa Pro Xaa Phe Lys Asn Xaa Ser Val Gly
   19130 19135 19140
   Xaa Leu Tyr Ser Gly Cys Arg Leu Thr Xaa Leu Arg Xaa Glu Lys
   19145 19150 19155
   Xaa Gly Ala Ala Thr Gly Xaa Asp Ala Ile Cys Xaa His Xaa Xaa
   19160 19165 19170
   Xaa Pro Lys Xaa Pro Gly Leu Xaa Xaa Glu Xaa Leu Tyr Trp Glu
   19175 19180 19185
   Leu Ser Xaa Leu Thr Xaa Xaa Ile Xaa Glu Leu Gly Pro Tyr Thr
   19190 19195 19200
   Leu Asp Arg Xaa Ser Leu Tyr Val Asn Gly Phe Thr His Trp Ser
   19205 19210 19215
   Ser Gly Leu Thr Thr Ser Thr Pro Trp Thr Ser Thr Val Asp Leu
   19220 19225 19230
   Gly Thr Ser Gly Thr Pro Ser Pro Val Pro Ser Pro Thr Thr Ala
   19235 19240 19245
   Gly Pro Leu Leu Val Pro Phe Thr Leu Asn Phe Thr Ile Thr Asn
   19250 19255 19260
   Leu Gln Tyr Glu Glu Asp Met His Arg Pro Gly Ser Arg Lys Phe
   19265 19270 19275
   Asn Ala Thr Glu Arg Val Leu Gln Gly Leu Leu Ser Pro Ile Phe
   19280 19285 19290
   Lys Asn Thr Ser Val Gly Pro Leu Tyr Ser Gly Cys Arg Leu Thr
   19295 19300 19305
   Leu Leu Arg Pro Glu Lys Gln Glu Ala Ala Thr Gly Val Asp Thr
   19310 19315 19320
   Ile Cys Thr His Arg Val Asp Pro Ile Gly Pro Gly Leu Xaa Xaa
   19325 19330 19335
   Glu Xaa Leu Tyr Trp Glu Leu Ser Xaa Leu Thr Xaa Xaa Ile Xaa
   19340 19345 19350
   Glu Leu Gly Pro Tyr Thr Leu Asp Arg Xaa Ser Leu Tyr Val Asn
   19355 19360 19365
   Gly Phe Thr His Xaa Xaa Ser Xaa Pro Thr Thr Ser Thr Pro Gly
   19370 19375 19380
   Thr Ser Thr Val Xaa Xaa Gly Thr Ser Gly Thr Pro Ser Ser Xaa
   19385 19390 19395
   Pro Xaa Xaa Thr Xaa Xaa Xaa Pro Leu Leu Xaa Pro Phe Thr Xaa
   19400 19405 19410
   Asn Xaa Thr Ile Thr Asn Leu Xaa Xaa Xaa Xaa Xaa Met Xaa Xaa
   19415 19420 19425
   Pro Gly Ser Arg Lys Phe Asn Thr Thr Glu Xaa Val Leu Gln Gly
   19430 19435 19440
   Leu Leu Xaa Pro Xaa Phe Lys Asn Xaa Ser Val Gly Xaa Leu Tyr
   19445 19450 19455
   Ser Gly Cys Arg Leu Thr Xaa Leu Arg Xaa Glu Lys Xaa Gly Ala
   19460 19465 19470
   Ala Thr Gly Xaa Asp Ala Ile Cys Xaa His Xaa Xaa Xaa Pro Lys
   19475 19480 19485
   Xaa Pro Gly Leu Xaa Xaa Glu Xaa Leu Tyr Trp Glu Leu Ser Xaa
   19490 19495 19500
   Leu Thr Xaa Xaa Ile Xaa Glu Leu Gly Pro Tyr Thr Leu Asp Arg
   19505 19510 19515
   Xaa Ser Leu Tyr Val Asn Gly Phe Thr His Arg Ser Phe Gly Leu
   19520 19525 19530
   Thr Thr Ser Thr Pro Trp Thr Ser Thr Val Asp Leu Gly Thr Ser
   19535 19540 19545
   Gly Thr Pro Ser Pro Val Pro Ser Pro Thr Thr Ala Gly Pro Leu
   19550 19555 19560
   Leu Val Pro Phe Thr Leu Asn Phe Thr Ile Thr Asn Leu Gln Tyr
   19565 19570 19575
   Glu Glu Asp Met His Arg Pro Gly Ser Arg Lys Phe Asn Thr Thr
   19580 19585 19590
   Glu Arg Val Leu Gln Gly Leu Leu Thr Pro Leu Phe Arg Asn Thr
   19595 19600 19605
   Ser Val Ser Ser Leu Tyr Ser Gly Cys Arg Leu Thr Leu Leu Arg
   19610 19615 19620
   Pro Glu Lys Asp Gly Ala Ala Thr Arg Val Asp Ala Val Cys Thr
   19625 19630 19635
   His Arg Pro Asp Pro Lys Ser Pro Gly Leu Xaa Xaa Glu Xaa Leu
   19640 19645 19650
   Tyr Trp Glu Leu Ser Xaa Leu Thr Xaa Xaa Ile Xaa Glu Leu Gly
   19655 19660 19665
   Pro Tyr Thr Leu Asp Arg Xaa Ser Leu Tyr Val Asn Gly Phe Thr
   19670 19675 19680
   His Xaa Xaa Ser Xaa Pro Thr Thr Ser Thr Pro Gly Thr Ser Thr
   19685 19690 19695
   Val Xaa Xaa Gly Thr Ser Gly Thr Pro Ser Ser Xaa Pro Xaa Xaa
   19700 19705 19710
   Thr Xaa Xaa Xaa Pro Leu Leu Xaa Pro Phe Thr Xaa Asn Xaa Thr
   19715 19720 19725
   Ile Thr Asn Leu Xaa Xaa Xaa Xaa Xaa Met Xaa Xaa Pro Gly Ser
   19730 19735 19740
   Arg Lys Phe Asn Thr Thr Glu Xaa Val Leu Gln Gly Leu Leu Xaa
   19745 19750 19755
   Pro Xaa Phe Lys Asn Xaa Ser Val Gly Xaa Leu Tyr Ser Gly Cys
   19760 19765 19770
   Arg Leu Thr Xaa Leu Arg Xaa Glu Lys Xaa Gly Ala Ala Thr Gly
   19775 19780 19785
   Xaa Asp Ala Ile Cys Xaa His Xaa Xaa Xaa Pro Lys Xaa Pro Gly
   19790 19795 19800
   Leu Xaa Xaa Glu Xaa Leu Tyr Trp Glu Leu Ser Xaa Leu Thr Xaa
   19805 19810 19815
   Xaa Ile Xaa Glu Leu Gly Pro Tyr Thr Leu Asp Arg Xaa Ser Leu
   19820 19825 19830
   Tyr Val Asn Gly Phe Thr His Trp Ile Pro Val Pro Thr Ser Ser
   19835 19840 19845
   Thr Pro Gly Thr Ser Thr Val Asp Leu Gly Ser Gly Thr Pro Ser
   19850 19855 19860
   Ser Leu Pro Ser Pro Thr Thr Ala Gly Pro Leu Leu Val Pro Phe
   19865 19870 19875
   Thr Leu Asn Phe Thr Ile Thr Asn Leu Gln Tyr Gly Glu Asp Met
   19880 19885 19890
   Gly His Pro Gly Ser Arg Lys Phe Asn Thr Thr Glu Arg Val Leu
   19895 19900 19905
   Gln Gly Leu Leu Gly Pro Ile Phe Lys Asn Thr Ser Val Gly Pro
   19910 19915 19920
   Leu Tyr Ser Gly Cys Arg Leu Thr Ser Leu Arg Ser Glu Lys Asp
   19925 19930 19935
   Gly Ala Ala Thr Gly Val Asp Ala Ile Cys Ile His His Leu Asp
   19940 19945 19950
   Pro Lys Ser Pro Gly Leu Xaa Xaa Glu Xaa Leu Tyr Trp Glu Leu
   19955 19960 19965
   Ser Xaa Leu Thr Xaa Xaa Ile Xaa Glu Leu Gly Pro Tyr Thr Leu
   19970 19975 19980
   Asp Arg Xaa Ser Leu Tyr Val Asn Gly Phe Thr His Xaa Xaa Ser
   19985 19990 19995
   Xaa Pro Thr Thr Ser Thr Pro Gly Thr Ser Thr Val Xaa Xaa Gly
   20000 20005 20010
   Thr Ser Gly Thr Pro Ser Ser Xaa Pro Xaa Xaa Thr Xaa Xaa Xaa
   20015 20020 20025
   Pro Leu Leu Xaa Pro Phe Thr Xaa Asn Xaa Thr Ile Thr Asn Leu
   20030 20035 20040
   Xaa Xaa Xaa Xaa Xaa Met Xaa Xaa Pro Gly Ser Arg Lys Phe Asn
   20045 20050 20055
   Thr Thr Glu Xaa Val Leu Gln Gly Leu Leu Xaa Pro Xaa Phe Lys
   20060 20065 20070
   Asn Xaa Ser Val Gly Xaa Leu Tyr Ser Gly Cys Arg Leu Thr Xaa
   20075 20080 20085
   Leu Arg Xaa Glu Lys Xaa Gly Ala Ala Thr Gly Xaa Asp Ala Ile
   20090 20095 20100
   Cys Xaa His Xaa Xaa Xaa Pro Lys Xaa Pro Gly Leu Xaa Xaa Glu
   20105 20110 20115
   Xaa Leu Tyr Trp Glu Leu Ser Xaa Leu Thr Xaa Xaa Ile Xaa Glu
   20120 20125 20130
   Leu Gly Pro Tyr Thr Leu Asp Arg Xaa Ser Leu Tyr Val Asn Gly
   20135 20140 20145
   Phe Thr His Gln Thr Phe Ala Pro Asn Thr Ser Thr Pro Gly Thr
   20150 20155 20160
   Ser Thr Val Asp Leu Gly Thr Ser Gly Thr Pro Ser Ser Leu Pro
   20165 20170 20175
   Ser Pro Thr Ser Ala Gly Pro Leu Leu Val Pro Phe Thr Leu Asn
   20180 20185 20190
   Phe Thr Ile Thr Asn Leu Gln Tyr Glu Glu Asp Met His His Pro
   20195 20200 20205
   Gly Ser Arg Lys Phe Asn Thr Thr Glu Arg Val Leu Gln Gly Leu
   20210 20215 20220
   Leu Gly Pro Met Phe Lys Asn Thr Ser Val Gly Leu Leu Tyr Ser
   20225 20230 20235
   Gly Cys Arg Leu Thr Leu Leu Arg Pro Glu Lys Asn Gly Ala Ala
   20240 20245 20250
   Thr Arg Val Asp Ala Val Cys Thr His Arg Pro Asp Pro Lys Ser
   20255 20260 20265
   Pro Gly Leu Xaa Xaa Glu Xaa Leu Tyr Trp Glu Leu Ser Xaa Leu
   20270 20275 20280
   Thr Xaa Xaa Ile Xaa Glu Leu Gly Pro Tyr Thr Leu Asp Arg Xaa
   20285 20290 20295
   Ser Leu Tyr Val Asn Gly Phe Thr His Xaa Xaa Ser Xaa Pro Thr
   20300 20305 20310
   Thr Ser Thr Pro Gly Thr Ser Thr Val Xaa Xaa Gly Thr Ser Gly
   20315 20320 20325
   Thr Pro Ser Ser Xaa Pro Xaa Xaa Thr Ala Pro Val Pro Leu Leu
   20330 20335 20340
   Ile Pro Phe Thr Leu Asn Phe Thr Ile Thr Asn Leu His Tyr Glu
   20345 20350 20355
   Glu Asn Met Gln His Pro Gly Ser Arg Lys Phe Asn Thr Thr Glu
   20360 20365 20370
   Arg Val Leu Gln Gly Leu Leu Lys Pro Leu Phe Lys Ser Thr Ser
   20375 20380 20385
   Val Gly Pro Leu Tyr Ser Gly Cys Arg Leu Thr Leu Leu Arg Pro
   20390 20395 20400
   Glu Lys His Gly Ala Ala Thr Gly Val Asp Ala Ile Cys Thr Leu
   20405 20410 20415
   Arg Leu Asp Pro Thr Gly Pro Gly Leu Asp Arg Glu Arg Leu Tyr
   20420 20425 20430
   Trp Glu Leu Ser Gln Leu Thr Asn Ser Val Thr Glu Leu Gly Pro
   20435 20440 20445
   Tyr Thr Leu Asp Arg Asp Ser Leu Tyr Val Asn Gly Phe Thr Gln
   20450 20455 20460
   Arg Ser Ser Val Pro Thr Thr Ser Ile Pro Gly Thr Ser Ala Val
   20465 20470 20475
   His Leu Glu Thr Ser Gly Thr Pro Ala Ser Leu Pro Gly His Thr
   20480 20485 20490
   Ala Pro Gly Pro Leu Leu Val Pro Phe Thr Leu Asn Phe Thr Ile
   20495 20500 20505
   Thr Asn Leu Gln Tyr Glu Val Asp Met Arg His Pro Gly Ser Arg
   20510 20515 20520
   Lys Phe Asn Thr Thr Glu Arg Val Leu Gln Gly Leu Leu Lys Pro
   20525 20530 20535
   Leu Phe Lys Ser Thr Ser Val Gly Pro Leu Tyr Ser Gly Cys Arg
   20540 20545 20550
   Leu Thr Leu Leu Arg Pro Glu Lys Arg Gly Ala Ala Thr Gly Val
   20555 20560 20565
   Asp Thr Ile Cys Thr His Arg Leu Asp Pro Leu Asn Pro Gly Leu
   20570 20575 20580
   Asp Arg Glu Gln Leu Tyr Trp Glu Leu Ser Lys Leu Thr Arg Gly
   20585 20590 20595
   Ile Ile Glu Leu Gly Pro Tyr Leu Leu Asp Arg Gly Ser Leu Tyr
   20600 20605 20610
   Val Asn Gly Phe Thr His Arg Asn Phe Val Pro Ile Thr Ser Thr
   20615 20620 20625
   Pro Gly Thr Ser Thr Val His Leu Gly Thr Ser Glu Thr Pro Ser
   20630 20635 20640
   Ser Leu Pro Arg Pro Ile Val Pro Gly Pro Leu Leu Val Pro Phe
   20645 20650 20655
   Thr Leu Asn Phe Thr Ile Thr Asn Leu Gln Tyr Glu Glu Ala Met
   20660 20665 20670
   Arg His Pro Gly Ser Arg Lys Phe Asn Thr Thr Glu Arg Val Leu
   20675 20680 20685
   Gln Gly Leu Leu Arg Pro Leu Phe Lys Asn Thr Ser Ile Gly Pro
   20690 20695 20700
   Leu Tyr Ser Ser Cys Arg Leu Thr Leu Leu Arg Pro Glu Lys Asp
   20705 20710 20715
   Lys Ala Ala Thr Arg Val Asp Ala Ile Cys Thr His His Pro Asp
   20720 20725 20730
   Pro Gln Ser Pro Gly Leu Asn Arg Glu Gln Leu Tyr Trp Glu Leu
   20735 20740 20745
   Ser Gln Leu Thr His Gly Ile Thr Glu Leu Gly Pro Tyr Thr Leu
   20750 20755 20760
   Asp Arg Asp Ser Leu Tyr Val Asp Gly Phe Thr His Trp Ser Pro
   20765 20770 20775
   Ile Pro Thr Thr Ser Thr Pro Gly Thr Ser Ile Val Asn Leu Gly
   20780 20785 20790
   Thr Ser Gly Ile Pro Pro Ser Leu Pro Glu Thr Thr Xaa Xaa Xaa
   20795 20800 20805
   Pro Leu Leu Xaa Pro Phe Thr Xaa Asn Xaa Thr Ile Thr Asn Leu
   20810 20815 20820
   Xaa Xaa Xaa Xaa Xaa Met Xaa Xaa Pro Gly Ser Arg Lys Phe Asn
   20825 20830 20835
   Thr Thr Glu Arg Val Leu Gln Gly Leu Leu Lys Pro Leu Phe Lys
   20840 20845 20850
   Ser Thr Ser Val Gly Pro Leu Tyr Ser Gly Cys Arg Leu Thr Leu
   20855 20860 20865
   Leu Arg Pro Glu Lys Asp Gly Val Ala Thr Arg Val Asp Ala Ile
   20870 20875 20880
   Cys Thr His Arg Pro Asp Pro Lys Ile Pro Gly Leu Asp Arg Gln
   20885 20890 20895
   Gln Leu Tyr Trp Glu Leu Ser Gln Leu Thr His Ser Ile Thr Glu
   20900 20905 20910
   Leu Gly Pro Tyr Thr Leu Asp Arg Asp Ser Leu Tyr Val Asn Gly
   20915 20920 20925
   Phe Thr Gln Arg Ser Ser Val Pro Thr Thr Ser Thr Pro Gly Thr
   20930 20935 20940
   Phe Thr Val Gln Pro Glu Thr Ser Glu Thr Pro Ser Ser Leu Pro
   20945 20950 20955
   Gly Pro Thr Ala Thr Gly Pro Val Leu Leu Pro Phe Thr Leu Asn
   20960 20965 20970
   Phe Thr Ile Thr Asn Leu Gln Tyr Glu Glu Asp Met His Arg Pro
   20975 20980 20985
   Gly Ser Arg Lys Phe Asn Thr Thr Glu Arg Val Leu Gln Gly Leu
   20990 20995 21000
   Leu Met Pro Leu Phe Lys Asn Thr Ser Val Ser Ser Leu Tyr Ser
   21005 21010 21015
   Gly Cys Arg Leu Thr Leu Leu Arg Pro Glu Lys Asp Gly Ala Ala
   21020 21025 21030
   Thr Arg Val Asp Ala Val Cys Thr His Arg Pro Asp Pro Lys Ser
   21035 21040 21045
   Pro Gly Leu Asp Arg Glu Arg Leu Tyr Trp Lys Leu Ser Gln Leu
   21050 21055 21060
   Thr His Gly Ile Thr Glu Leu Gly Pro Tyr Thr Leu Asp Arg His
   21065 21070 21075
   Ser Leu Tyr Val Asn Gly Phe Thr His Gln Ser Ser Met Thr Thr
   21080 21085 21090
   Thr Arg Thr Pro Asp Thr Ser Thr Met His Leu Ala Thr Ser Arg
   21095 21100 21105
   Thr Pro Ala Ser Leu Ser Gly Pro Thr Thr Ala Ser Pro Leu Leu
   21110 21115 21120
   Val Leu Phe Thr Ile Asn Phe Thr Ile Thr Asn Leu Arg Tyr Glu
   21125 21130 21135
   Glu Asn Met His His Pro Gly Ser Arg Lys Phe Asn Thr Thr Glu
   21140 21145 21150
   Arg Val Leu Gln Gly Leu Leu Arg Pro Val Phe Lys Asn Thr Ser
   21155 21160 21165
   Val Gly Pro Leu Tyr Ser Gly Cys Arg Leu Thr Leu Leu Arg Pro
   21170 21175 21180
   Lys Lys Asp Gly Ala Ala Thr Lys Val Asp Ala Ile Cys Thr Tyr
   21185 21190 21195
   Arg Pro Asp Pro Lys Ser Pro Gly Leu Asp Arg Glu Gln Leu Tyr
   21200 21205 21210
   Trp Glu Leu Ser Gln Leu Thr His Ser Ile Thr Glu Leu Gly Pro
   21215 21220 21225
   Tyr Thr Leu Asp Arg Asp Ser Leu Tyr Val Asn Gly Phe Thr Gln
   21230 21235 21240
   Arg Ser Ser Val Pro Thr Thr Ser Ile Pro Gly Thr Pro Thr Val
   21245 21250 21255
   Asp Leu Gly Thr Ser Gly Thr Pro Val Ser Lys Pro Gly Pro Ser
   21260 21265 21270
   Ala Ala Ser Pro Leu Leu Val Leu Phe Thr Leu Asn Phe Thr Ile
   21275 21280 21285
   Thr Asn Leu Arg Tyr Glu Glu Asn Met Gln His Pro Gly Ser Arg
   21290 21295 21300
   Lys Phe Asn Thr Thr Glu Arg Val Leu Gln Gly Leu Leu Arg Ser
   21305 21310 21315
   Leu Phe Lys Ser Thr Ser Val Gly Pro Leu Tyr Ser Gly Cys Arg
   21320 21325 21330
   Leu Thr Leu Leu Arg Pro Glu Lys Asp Gly Thr Ala Thr Gly Val
   21335 21340 21345
   Asp Ala Ile Cys Thr His His Pro Asp Pro Lys Ser Pro Arg Leu
   21350 21355 21360
   Asp Arg Glu Gln Leu Tyr Trp Glu Leu Ser Gln Leu Thr His Asn
   21365 21370 21375
   Ile Thr Glu Leu Gly His Tyr Ala Leu Asp Asn Asp Ser Leu Phe
   21380 21385 21390
   Val Asn Gly Phe Thr His Arg Ser Ser Val Ser Thr Thr Ser Thr
   21395 21400 21405
   Pro Gly Thr Pro Thr Val Tyr Leu Gly Ala Ser Lys Thr Pro Ala
   21410 21415 21420
   Ser Ile Phe Gly Pro Ser Ala Ala Ser His Leu Leu Ile Leu Phe
   21425 21430 21435
   Thr Leu Asn Phe Thr Ile Thr Asn Leu Arg Tyr Glu Glu Asn Met
   21440 21445 21450
   Trp Pro Gly Ser Arg Lys Phe Asn Thr Thr Glu Arg Val Leu Gln
   21455 21460 21465
   Gly Leu Leu Arg Pro Leu Phe Lys Asn Thr Ser Val Gly Pro Leu
   21470 21475 21480
   Tyr Ser Gly Ser Arg Leu Thr Leu Leu Arg Pro Glu Lys Asp Gly
   21485 21490 21495
   Glu Ala Thr Gly Val Asp Ala Ile Cys Thr His Arg Pro Asp Pro
   21500 21505 21510
   Thr Gly Pro Gly Leu Asp Arg Glu Gln Leu Tyr Leu Glu Leu Ser
   21515 21520 21525
   Gln Leu Thr His Ser Ile Thr Glu Leu Gly Pro Tyr Thr Leu Asp
   21530 21535 21540
   Arg Asp Ser Leu Tyr Val Asn Gly Phe Thr His Arg Ser Ser Val
   21545 21550 21555
   Pro Thr Thr Ser Thr Gly Val Val Ser Glu Glu Pro Phe Thr Leu
   21560 21565 21570
   Asn Phe Thr Ile Asn Asn Leu Arg Tyr Met Ala Asp Met Gly Gln
   21575 21580 21585
   Pro Gly Ser Leu Lys Phe Asn Ile Thr Asp Asn Val Met Lys His
   21590 21595 21600
   Leu Leu Ser Pro Leu Phe Gln Arg Ser Ser Leu Gly Ala Arg Tyr
   21605 21610 21615
   Thr Gly Cys Arg Val Ile Ala Leu Arg Ser Val Lys Asn Gly Ala
   21620 21625 21630
   Glu Thr Arg Val Asp Leu Leu Cys Thr Tyr Leu Gln Pro Leu Ser
   21635 21640 21645
   Gly Pro Gly Leu Pro Ile Lys Gln Val Phe His Glu Leu Ser Gln
   21650 21655 21660
   Gln Thr His Gly Ile Thr Arg Leu Gly Pro Tyr Ser Leu Asp Lys
   21665 21670 21675
   Asp Ser Leu Tyr Leu Asn Gly Tyr Asn Glu Pro Gly Leu Asp Glu
   21680 21685 21690
   Pro Pro Thr Thr Pro Lys Pro Ala Thr Thr Phe Leu Pro Pro Leu
   21695 21700 21705
   Ser Glu Ala Thr Thr Ala Met Gly Tyr His Leu Lys Thr Leu Thr
   21710 21715 21720
   Leu Asn Phe Thr Ile Ser Asn Leu Gln Tyr Ser Pro Asp Met Gly
   21725 21730 21735
   Lys Gly Ser Ala Thr Phe Asn Ser Thr Glu Gly Val Leu Gln His
   21740 21745 21750
   Leu Leu Arg Pro Leu Phe Gln Lys Ser Ser Met Gly Pro Phe Tyr
   21755 21760 21765
   Leu Gly Cys Gln Leu Ile Ser Leu Arg Pro Glu Lys Asp Gly Ala
   21770 21775 21780
   Ala Thr Gly Val Asp Thr Thr Cys Thr Tyr His Pro Asp Pro Val
   21785 21790 21795
   Gly Pro Gly Leu Asp Ile Gln Gln Leu Tyr Trp Glu Leu Ser Gln
   21800 21805 21810
   Leu Thr His Gly Val Thr Gln Leu Gly Phe Tyr Val Leu Asp Arg
   21815 21820 21825
   Asp Ser Leu Phe Ile Asn Gly Tyr Ala Pro Gln Asn Leu Ser Ile
   21830 21835 21840
   Arg Gly Glu Tyr Gln Ile Asn Phe His Ile Val Asn Trp Asn Leu
   21845 21850 21855
   Ser Asn Pro Asp Pro Thr Ser Ser Glu Tyr Ile Thr Leu Leu Arg
   21860 21865 21870
   Asp Ile Gln Asp Lys Val Thr Thr Leu Tyr Lys Gly Ser Gln Leu
   21875 21880 21885
   His Asp Thr Phe Arg Phe Cys Leu Val Thr Asn Leu Thr Met Asp
   21890 21895 21900
   Ser Val Leu Val Thr Val Lys Ala Leu Phe Ser Ser Asn Leu Asp
   21905 21910 21915
   Pro Ser Leu Val Glu Gln Val Phe Leu Asp Lys Thr Leu Asn Ala
   21920 21925 21930
   Ser Phe His Trp Leu Gly Ser Thr Tyr Gln Leu Val Asp Ile His
   21935 21940 21945
   Val Thr Glu Met Glu Ser Ser Val Tyr Gln Pro Thr Ser Ser Ser
   21950 21955 21960
   Ser Thr Gln His Phe Tyr Leu Asn Phe Thr Ile Thr Asn Leu Pro
   21965 21970 21975
   Tyr Ser Gln Asp Lys Ala Gln Pro Gly Thr Thr Asn Tyr Gln Arg
   21980 21985 21990
   Asn Lys Arg Asn Ile Glu Asp Ala Leu Asn Gln Leu Phe Arg Asn
   21995 22000 22005
   Ser Ser Ile Lys Ser Tyr Phe Ser Asp Cys Gln Val Ser Thr Phe
   22010 22015 22020
   Arg Ser Val Pro Asn Arg His His Thr Gly Val Asp Ser Leu Cys
   22025 22030 22035
   Asn Phe Ser Pro Leu Ala Arg Arg Val Asp Arg Val Ala Ile Tyr
   22040 22045 22050
   Glu Glu Phe Leu Arg Met Thr Arg Asn Gly Thr Gln Leu Gln Asn
   22055 22060 22065
   Phe Thr Leu Asp Arg Ser Ser Val Leu Val Asp Gly Tyr Ser Pro
   22070 22075 22080
   Asn Arg Asn Glu Pro Leu Thr Gly Asn Ser Asp Leu Pro Phe Trp
   22085 22090 22095
   Ala Val Ile Leu Ile Gly Leu Ala Gly Leu Leu Gly Leu Ile Thr
   22100 22105 22110
   Cys Leu Ile Cys Gly Val Leu Val Thr Thr Arg Arg Arg Lys Lys
   22115 22120 22125
   Glu Gly Glu Tyr Asn Val Gln Gln Gln Cys Pro Gly Tyr Tyr Gln
   22130 22135 22140
   Ser His Leu Asp Leu Glu Asp Leu Gln
   22145 22150
<210> 8
   <211> 89
   <212> PRT
   <213> Homo sapiens
<400> 8
   Met Lys Gly Leu Ala Ala Ala Leu Leu Val Leu Val Cys Thr Met Ala
   1 5 10 15
   Leu Cys Ser Cys Ala Gln Val Gly Thr Asn Lys Glu Leu Cys Cys Leu
   20 25 30
   Val Tyr Thr Ser Trp Gln Ile Pro Gln Lys Phe Ile Val Asp Tyr Ser
   35 40 45
   Glu Thr Ser Pro Gln Cys Pro Lys Pro Gly Val Ile Leu Leu Thr Lys
   50 55 60
   Arg Gly Arg Gln Ile Cys Ala Asp Pro Asn Lys Lys Trp Val Gln Lys
   65 70 75 80
   Tyr Ile Ser Asp Leu Lys Leu Asn Ala
   85
<210> 9
   <211> 119
   <212> PRT
   <213> Homo sapiens
<400> 9
   Met Ala Gly Leu Met Thr Ile Val Thr Ser Leu Leu Phe Leu Gly Val
   1               5                   10                  15
   Cys Ala His His Ile Ile Pro Thr Gly Ser Val Val Ile Pro Ser Pro
               20                  25                  30
   Cys Cys Met Phe Phe Val Ser Lys Arg Ile Pro Glu Asn Arg Val Val
           35                  40                  45
   Ser Tyr Gln Leu Ser Ser Arg Ser Thr Cys Leu Lys Ala Gly Val Ile
       50                  55                  60
   Phe Thr Thr Lys Lys Gly Gln Gln Phe Cys Gly Asp Pro Lys Gln Glu
   65                  70                  75                  80
   Trp Val Gln Arg Tyr Met Lys Asn Leu Asp Ala Lys Gln Lys Lys Ala
                   85                  90                  95
   Ser Pro Arg Ala Arg Ala Val Ala Val Lys Gly Pro Val Gln Arg Tyr
               100                 105                 110
   Pro Gly Asn Gln Thr Thr Cys
           115
<210> 10
   <211> 119
   <212> PRT
   <213> Homo sapiens
<400> 10
   Met Ala Gly Leu Met Thr Ile Val Thr Ser Leu Leu Phe Leu Gly Val
   1               5                   10                  15
   Cys Ala His His Ile Ile Pro Thr Gly Ser Val Val Ile Pro Ser Pro
               20                  25                  30
   Cys Cys Met Phe Phe Val Ser Lys Arg Ile Pro Glu Asn Arg Val Val
           35                  40                  45
   Ser Tyr Gln Leu Ser Ser Arg Ser Thr Cys Leu Lys Ala Gly Val Ile
       50                  55                  60
   Phe Thr Thr Lys Lys Gly Gln Gln Phe Cys Gly Asp Pro Lys Gln Glu
   65                  70                  75                  80
   Trp Val Gln Arg Tyr Met Lys Asn Leu Asp Ala Lys Gln Lys Lys Ala
                   85                  90                  95
   Ser Pro Arg Ala Arg Ala Val Ala Val Lys Gly Pro Val Gln Arg Tyr
               100                 105                 110
   Pro Gly Asn Gln Thr Thr Cys
           115
<210> 11
   <211> 109
   <212> PRT
   <213> Homo sapiens
<400> 11
   Met Lys Phe Ile Ser Thr Ser Leu Leu Leu Met Leu Leu Val Ser Ser
   1               5                   10                  15
   Leu Ser Pro Val Gln Gly Val Leu Glu Val Tyr Tyr Thr Ser Leu Arg
               20                  25                  30
   Cys Arg Cys Val Gln Glu Ser Ser Val Phe Ile Pro Arg Arg Phe Ile
           35                  40                  45
   Asp Arg Ile Gln Ile Leu Pro Arg Gly Asn Gly Cys Pro Arg Lys Glu
       50                  55                  60
   Ile Ile Val Trp Lys Lys Asn Lys Ser Ile Val Cys Val Asp Pro Gln
   65                  70                  75                  80
   Ala Glu Trp Ile Gln Arg Met Met Glu Val Leu Arg Lys Arg Ser Ser
                   85                  90                  95
   Ser Thr Leu Pro Val Pro Val Phe Lys Arg Lys Ile Pro
               100                 105
<210> 12
   <211> 4
   <212> PRT
   <213> Homo sapiens
<400> 12
   Ala Thr Leu Gly
   1
<210> 13
   <211> 5
   <212> PRT
   <213> Homo sapiens
<400> 13
   Gly Cys Arg Leu Tyr
   1               5